# EUROPEAN PATENT APPLICATION

(11) **EP 3 699 280 A2**
(43) Date of publication of application: **26.08.2020**
(21) Application number: 20167427.2
(22) Date of filing: 27.02.2017
(51) Int. Cl.: C12N 15/113, C12N 9/22, C12N 15/82

(54) **NOVEL CAS9 SYSTEMS AND METHODS OF USE**

(30) Priority: 11.03.2016 US 201662306904 P
(62) Divisional of application: 17711047.5
(71) Applicant: Pioneer Hi-Bred International, Inc., Johnston, Iowa 50131-1014 (US)
(72) Inventor: CIGAN, Andrew Mark, Madison, WI 53704 (US); KING, Matthew G., Johnston, IA 50131-0552 (US); LIN, Haining, Johnston, IA 50131-0552 (US); YOUNG, Joshua K., Johnston, IA 50131-0552 (US)
(74) Representative: J A Kemp LLP

(57) **Abstract**

Compositions and methods are provided for novel Cas9 systems, including, but not limiting to, novel guide polynucleotide/Cas9 endonucleases complexes, single or dual guide RNAs, guide RNA elements, and Cas9 endonucleases. The present disclosure also describes methods for genome modification of a target sequence in the genome of a cell, for gene editing, and for inserting a polynucleotide of interest into the genome of a cell. Also provided are nucleic acid constructs and cells having an altered target site or altered polynucleotide of interest produced by the methods described herein.

SEQUENCE LISTING

<110>  Pioneer Hi-Bred International Inc.
       Cigan, Andrew  Mark
       King, Matt
       Lin, Helen
       Young, Joshua K

<120>  NOVEL CAS9 SYSTEMS AND METHODS OF USE

<130>  BB2398 PCT

<150>  62/306904
<151>  2016-03-11

<160>  207

<170>  PatentIn version 3.5

<210>  1
<211>  7342
<212>  DNA
<213>  Bacillus cereus

<220>
<221>  misc_feature
<222>  (5412)..(5467)
<223>  n is a, c, g, or t

<400>  1
acgaaggatc attttaaaaa aactgaacaa tcataatcaa caatactgtt aaaatcggac      60
cactaaggct aaataatgtc gaaatgaatt tagagttatt atattgcgac ataaaaaaga     120
taaccgaacc caaaatagta ataataggta caatgattgc tctcatagca aggaaataga     180
gtggaacggc gaaaatactt cctaatacga tgaaaaaaaa tgtacaaaga tgccaactta     240
ttattgttct ttttcgaaaa atataaagta aaaaccaccc aaagatacaa caaacataat     300
taaataaggt aacaaaccaa agaatactct gaaaactcga tgttcttaaa tcagtaaaat     360
caatcgtagg acttaataca tatataaaca aaataattag attagcaaaa aacagggata     420
atcctgctcc agacaagtat gatcgcattt aaatcataac ccccttacat taatattaat     480
tattatacca taaaatgggt tttgtttaat ggttcttaat cttactaacg tgtaaatttt     540
gaaatgttag gtttttaaat aagagtataa acatcgggca attggcagat gaactaaggg     600
tagctaggga acaggagaaa aagaacgaag gctaggttat taacgtgtca taattttcaa     660
taataaatat ataaaataaa tatataaaat aaatatataa aataaatata taaatgagat     720
tgacttcgat gatatatgga tataaaatga aagtatcata tcatatcaag ttttggtaag     780
ttgctatagt aagggcaaca gacccgaggc gttggggatc gcctagcccg tttttacggg     840
ctctccctat attcaaaata atgacaaacg agcaccttgg agaatttatt tccgaggtgc     900
ttttttttct aggagggata gaatggcata cacaatggga atagatgtgg ggattgcttc     960
gtgtggatgg gctattgtag atcttgaaag acaacgaata atagatctag gtgttcggac    1020
ttttgagaaa gcagagaatc cgaaaaatgg agaggctctt gctgtaccaa ggagagaagc    1080
tagatcgagc cgtaggagat tacggagaaa aaaacatcgc attgaaagat taaagcatat    1140
gtttattcgg aatggactgg cggttgatat tcaacatctt gagcagcaga cgttacgtag    1200
ccaaaatgaa atagatgtat ggcaattacg agtagatggt ttggatcgaa tgctaactca    1260
aaaagagtgg cttcgtgtat taattcatct tgcacaacgt cgtggttttc aatcaaatcg    1320
aaaaacagat ggttcaagtg aagatgggca ggttcttgta aatgtaacgg agaatgacag    1380
attaatggaa gagaaagatt atcgaaccgt agcggaaatg atggtaaaag atgaaaaatt    1440
ctctgaccat aagcgaaata aaaatggaaa ttatcatgga gtagtgagca gagcttcttt    1500
attagttgaa atacatacat tatttgaatc ccaaagacag caccataatt ttttggcatc    1560
gaaagatttc gagctggaat atgttaatat ttggtcttca caacggcctg ttgcaacaaa    1620
agatcaaata gaaaaaatga ttggtacatg tactttctta ccgaaagaaa agagaactcc    1680
aaaggcatct tggcattttc aatattttat gctcttgcaa acgatcaatc atatccgtat    1740
aacaaatata caaggcacga gatcattgaa taaagaagaa attgaacaag tggtcaatat    1800
ggcacttacc aaatcaaagg tatcttacca tgatattaga aaagtccttg atttatcaga    1860
agaatatcaa tttgttggct tggattatgg aaaagagaat gaaaaaaaga aagttgaaag    1920
taaggaaacg atcatcaaat tagatgatta ccataagtta aataaaattt ttaatgaagt    1980

ggaattagct aaaggagaag cttggaaagc tgttgattat gatacagtag cgtatgctct 2040
cactttcttt aaagatgatg aagatattag agattatttg caaaataaat ataaagatag 2100
taaaaatcgt cttgttaaga acgtagctaa taaagaatac acaaatgaat tggtagaaaa 2160
agtgagtacg ctatcttttc gtaaagtggg gcatttatca ttgaaagcct tacgaaagat 2220
catcccgttt ttggagcaag gcatgaccta tgataaagcg tgccaagcgg ctggttttaa 2280
ctttcaaggt atatcaaaaa agaagagatc tgttgtcttg ccggtcattg accaaatttc 2340
caacccagta gtaaatcgtg ctcttactca aacgcgtaaa gtaattaatg ctctaatcaa 2400
aaaatatggt tcacctgaaa ccatacacat tgaaacagca agagagttat caaagagatt 2460
tgatgaacga aagaatatta ctaaggatta taaggagaat agagacaaga acgaacgtgc 2520
aaaaaaacac ttatcggaat tagggattat taatcctact ggtcttgata tagtaaagta 2580
caagctgtgg tgtgagcaac aagggcgctg tatgtacagc aaccaaccca tctcctttga 2640
aagactgaga gaatcaggtt ataccgaggt agatcacatt attccataca gccgaagtat 2700
gaatgatagt tataacaatc gcgtattggt tatgactagg gaaaatcgtg aaaagagtaa 2760
tcaaactcct tatgaatata tgggtaatga tacgcaaaga tgggatgagt ttgaacaaag 2820
agtcacaaca aatccacaaa taaaaaagga gaaacgacag aacctcttgc taaaaggatt 2880
cactaatcgt cgggaattag aaatgttaga aagaaactta aatgatacac gttatataac 2940
taagtatctt tcacatttta ttagtacgaa tttggaattc tctcctagtg ataaaaagaa 3000
aaaggtagtt aatacaagcg gtcgtatcac ttcccattta agaagtagat ggggattaga 3060
aaaaaatcgt gagcaaaatg atttacacca tgcaatggac gcggttgtaa ttgctgtcac 3120
ctcagactcg tttattcaac aggtaacgaa ctactataaa cggaaagaaa gaagagagtt 3180
gaatagggat gataagttcc ctctaccttg gaagtttttt agagaagaag tcatagcaag 3240
attaagccct aatccaaaag aacaaattga agcattgccc aaccattttt atagcgaaga 3300
tgaacttgct gatcttcaac cgatttttgt gtcaagaatg cccaagcgta gtataactgg 3360
agaggcgcat caggcacagt ttcgccgagt tgtgggtaaa acgaaagagg gtaaaaatat 3420
tactgcaaag aaaactgctt tagttgatat tagctatgat aaaaatggtg attttaatat 3480
gtatggaaga gaaacagatc ctgctactta tgaagcaatt aaagaaagat atctcgaatt 3540
tggaggaaat gctaaaaaag cattttcaac ggatttacat aaaccgaaaa aagatggcac 3600
caaagggcca ctgataaaat ctgtgagaat aatggaaaat aaaacattgg tacatcccgt 3660
aaataaagga aaaggtgttg tgtataacag ctctattgta aggacagatg tatttcaaag 3720
aaaagagaaa tattatttac tacctgtgta cgtaacagat gtaaccaaag ggaaactacc 3780
aaataaggtg attgttgcta aaaagggata cgatgattgg attgaggttg atgatagctt 3840
cacattttta tacagtctat atccgaatga tttaatattc atcagacaaa atccaaaaaa 3900
gaaaatatca ttaaaaaaac gtattgagag tcattctatt tctgatagta aagaggttca 3960
agaaattctt gcctattata aaggggttga tagttcaact gctgctatag agtttattac 4020
tcatgatggt agttactatg caaaaggtgt tggtgtccaa aatctagatt gttttgaaaa 4080
atatcaagtt gatactctgg gtaattattc caaagtgaaa ggagaaaaac gacttgagtt 4140
ggagacatct gataataacg acaaaggcaa agatgtcaat tcgattaaat caactagtcg 4200
ttaaacaaga agaagaggtt accatacctc ttgaagatat cgcatctatt gtggttgaag 4260
aaaatcaagt tgtgttaaca gctgctctat taagtaaatg cagtgaatat ggaattggtt 4320
tgtatacttg cgatgaaaag catttaccca atggtttgtt acttcccttt cagagtcatt 4380
caaggcaatt aagggtactt gaaacccaat ataacctttc aaagccattt aaaaaaagag 4440
tgtggcaaaa aataattaca actaagataa tgaatcaagc attgtgctta tcttatttgg 4500
ataaaaccgg agaaagtgaa ctgcttgcaa ttcgcaatac cgttgattca ggtgactcta 4560
cgaatagaga ggcatatgct gctaaagttt attttcaaca tttgtttggg aaacggttta 4620
ttcgaaggga agaccatata ataaaccact ccctcaatta tggttattcg atccttagag 4680
gaaatgtagc tagagtttta gtacagtatg gattctttcc atgtctagga attgaccatc 4740
acaatgaaaa taacgcattt aatttagctg atgattttat ggaggttttt cgcccattgg 4800
ttgatttatt tgtcgtgaaa cattttcatg aggagatgga atttacaccg aagtggagag 4860
caggattgta taatgtagtg aatcatcaag ttgaactaga aggtgaaaga tattcctgta 4920
atcgagctat tgaagaaatg attaaaagct acgttacagc atgcagaaca aatatgtatg 4980
agagactgag aacaccaaca ttactccctt tgaagattca ttcatatgag taaatatatg 5040
cgaatacttg tcttttttga tctaccggtg aaaaggaagc aggatcgtaa ggcttactgt 5100
gcatttagaa agtttttaat agcagatgga tatgatatgc tccagttttc agtctattct 5160
cgaatttgta atggcaatga aggaacacat aagcatttgg aaaggctgtc acgtaatttg 5220
ccctcaaagg gttcggtacg atcgttgatt attactgaaa agcaatatga aaagatgaaa 5280
ttttttgttg ggaaaccaaa aataaaagaa gagaaagtaa acagtgtgca actaacattt 5340
ttctgaaaaa gaaaattatt aataggaaga aaggagtttt tcttaccttt ttaaaatacc 5400
aaaagccttg annnnnnnnn nnnnnnnnnn nnnnnnnnnn nnnnnnnnnn nnnnnnnnnn 5460
nnnnnnnctt tcaaggcttt ttgggcacgt atcatatcat atcgagtttt agtaaggaac 5520
tatagcagaa acggattagt caatttgtcg atatgatcat atcatatcga gttttagtaa 5580
ggaactatag ctgttctgat ggaatgaact ggactttcgt catcatatca tatcgagttt 5640
tagtaaggaa ctatagcctt tatgggcggt cactgccggg atattcaatc atatcatatc 5700
gagttttagt aaggaactat agctttaatc attgcagttg ctaagaaaat tctatcatat 5760

catatcgagt tttagtaagg aactatagca gtcgtataca agtatggact ctcgaaatga 5820
tcatatcata tcgagtttta gtaaggaact atagcatgga tttttagttt gattcccgat 5880
atgaaatcat atcatatcga gttttagtaa ggaactatag cttggggtca cgtataatca 5940
aaccacgatc aatcatatca tatcgagttt tagtaaggaa ctatagcaga atcagcaaga 6000
tcactacgaa agcgaaaatc atatcatatc gagttttagt aaggaactat agctttcttt 6060
gctcccttct ccattcttgg tgaatcatat catatcgagt tttagtaagg aactatagca 6120
acgaaacaac cggggtgtca tctatattta tcatatcata tcgagtttta gtaaggaact 6180
atagcgcgag aggatgagga cggactttat cgtttatcat atcatatcga gttttagtaa 6240
ggaactatag cttacgatgc tatttcgatg ggggatggtt tatcatatca tatcgagttt 6300
tagtaaggaa ctatagccat gacgttagac cagttgaaag atttttgatc atatcatatc 6360
gagttttagt aaggaactat agcaatcgtg ctggattgga tcgctggata tcgatcatat 6420
catatcgagt tttagtaagg aactatagca cggtgccagc agggaaagcg gccagaagaa 6480
tcatatcata tcgagtttta gtaaggaact atagcaactc cagccctatg caaaggctgt 6540
atgggatcat atcatatcga gttttagtaa ggaactatag cacagttaga tatatactgt 6600
caggtatctt tatcatatca tatcgagttt tagtaaggaa ctatagctta gattgctcta 6660
actgatttag ctgaagcatc atatcatatc gagttttagt aaggaactat ggtctattgg 6720
gtattatcca aatcactatc ctactcaaac cccagacgga tagtcttttt tattaataaa 6780
catgtaattt tacccattgc ttaatctcct catcatttaa taaaatagga acacacattc 6840
gtatttaggg agatgtttta atggctagta aaattcttga cccacttgta acgaagttta 6900
ttttgccaga acatgcagaa atgttacgtc agtatcatga ggataagaag ctgatcgaga 6960
agccgttcat tgaagaggat gagctggccg agttttgcta caggatatct gactcacgtc 7020
agtatgacta tgcattaacc attagttggt ggaaagaggc aaaagaaggt agaggcgtga 7080
tagaatccgc ttggggatgg gtagataagt ttgattctca atttaaacag attaagctaa 7140
aaaacgatga ggatttctgg tggattcctg tagaagatgt agtgagtgtg gaagcctaag 7200
cagagaatct tataactgca tatagtgtat tgcctgtggg aacagggata cagcaaacac 7260
cccactatta atagcctagt ggggtgtttg catttaacat tcaatagcgt tgcaagcggt 7320
tcggcttatt cattgcatca at 7342

<210> 2
<211> 7342
<212> DNA
<213> Brevibacillus laterosporus

<220>
<221> misc_feature
<222> (5412)..(5467)
<223> n is a, c, g, or t

<400> 2
acgaaggatc attttaaaaa aactgaacaa tcataatcaa caatactgtt aaaatcggac 60
cactaaggct aaataatgtc gaaatgaatt tagagttatt atattgcgac ataaaaaaga 120
taaccgaacc caaaatagta ataataggta caatgattgc tctcatagca aggaaataga 180
gtggaacggc gaaaatactt cctaatacga tgaaaaaaaa tgtacaaaga tgccaactta 240
ttattgttct ttttcgaaaa atataaagta aaaaccaccc aaagatacaa caaacataat 300
taaataaggt aacaaaccaa agaatactct gaaaactcga tgttcttaaa tcagtaaaat 360
caatcgtagg acttaataca tatataaaca aaataattag attagcaaaa aacagggata 420
atcctgctcc agacaagtat gatcgcattt aaatcataac ccccttacat taatattaat 480
tattatacca taaaatgggt tttgtttaat ggttcttaat cttactaacg tgtaaatttt 540
gaaatgttag gtttttaaat aagagtataa acatcgggca attggcagat gaactaaggg 600
tagctaggga acaggagaaa aagaacgaag gctaggttat taacgtgtca taattttcaa 660
taataaatat ataaaataaa tatataaaat aaatatataa aataaatata taaatgagat 720
tgacttcgat gatatatgga tataaaatga aagtatcata tcatatcaag ttttggtaag 780
ttgctatagt aagggcaaca gacccgaggc gttggggatc gcctagcccg tttttacggg 840
ctctccctat attcaaaata atgacaaacg agcaccttgg agaatttatt tccgaggtgc 900
ttttttttct aggagggata gaatggcata cacaatggga atagatgtgg ggattgcttc 960
gtgtggatgg gctattgtag atcttgaaag acaacgaata atagatctag gtgttcggac 1020
ttttgagaaa gcagagaatc cgaaaaatgg agaggctctt gctgtaccaa ggagagaagc 1080
tagatcgagc cgtaggagat tacggagaaa aaaacatcgc attgaaagat taaagcatat 1140
gtttattcgg aatggactgg cggttgatat tcaacatctt gagcagcaga cgttacgtag 1200
ccaaaatgaa atagatgtat ggcaattacg agtagatggt ttggatcgaa tgctaactca 1260
aaaagagtgg cttcgtgtat taattcatct tgcacaacgt cgtggttttc aatcaaatcg 1320
aaaaacagat ggttcaagtg aagatgggca ggttcttgta aatgtaacgg agaatgacag 1380
attaatggaa gagaaagatt atcgaaccgt agcggaaatg atggtaaaag atgaaaaatt 1440

ctctgaccat aagcgaaata aaaatggaaa ttatcatgga gtagtgagca gagcttcttt 1500
attagttgaa atacatacat tatttgaatc ccaaagacag caccataatt ttttggcatc 1560
gaaagatttc gagctggaat atgttaatat ttggtcttca caacggcctg ttgcaacaaa 1620
agatcaaata gaaaaaatga ttggtacatg tactttctta ccgaaagaaa agagaactcc 1680
aaaggcatct tggcattttc aatattttat gctcttgcaa acgatcaatc atatccgtat 1740
aacaaatata caaggcacga gatcattgaa taaagaagaa attgaacaag tggtcaatat 1800
ggcacttacc aaatcaaagg tatcttacca tgatattaga aaagtccttg atttatcaga 1860
agaatatcaa tttgttggct tggattatgg aaaagagaat gaaaaaaaga aagttgaaag 1920
taaggaaacg atcatcaaat tagatgatta ccataagtta aataaaattt ttaatgaagt 1980
ggaattagct aaaggagaag cttggaaagc tgttgattat gatacagtag cgtatgctct 2040
cactttcttt aaagatgatg aagatattag agattatttg caaaataaat ataaagatag 2100
taaaaatcgt cttgttaaga acgtagctaa taaagaatac acaaatgaat tggtagaaaa 2160
agtgagtacg ctatcttttc gtaaagtggg gcatttatca ttgaaagcct tacgaaagat 2220
catcccgttt ttggagcaag gcatgaccta tgataaagcg tgccaagcgg ctggttttaa 2280
ctttcaaggt atatcaaaaa agaagagatc tgttgtcttg ccggtcattg accaaatttc 2340
caacccagta gtaaatcgtg ctcttactca aacgcgtaaa gtaattaatg ctctaatcaa 2400
aaaatatggt tcacctgaaa ccatacacat tgaaacagca agagagttat caaagagatt 2460
tgatgaacga aagaatatta ctaaggatta taaggagaat agagacaaga acgaacgtgc 2520
aaaaaaacac ttatcggaat tagggattat taatcctact ggtcttgata tagtaaagta 2580
caagctgtgg tgtgagcaac aagggcgctg tatgtacagc aaccaaccca tctcctttga 2640
aagactgaga gaatcaggtt ataccgaggt agatcacatt attccataca gccgaagtat 2700
gaatgatagt tataacaatc gcgtattggt tatgactagg gaaaatcgtg aaaagagtaa 2760
tcaaactcct tatgaatata tgggtaatga tacgcaaaga tgggatgagt ttgaacaaag 2820
agtcacaaca aatccacaaa taaaaaagga gaaacgacag aacctcttgc taaaaggatt 2880
cactaatcgt cgggaattag aaatgttaga aagaaactta aatgatacac gttatataac 2940
taagtatctt tcacatttta ttagtacgaa tttggaattc tctcctagtg ataaaaagaa 3000
aaaggtagtt aatacaagcg gtcgtatcac ttcccattta agaagtagat ggggattaga 3060
aaaaaatcgt gagcaaaatg atttacacca tgcaatggac gcggttgtaa ttgctgtcac 3120
ctcagactcg tttattcaac aggtaacgaa ctactataaa cggaaagaaa gaagagagtt 3180
gaatagggat gataagttcc ctctaccttg gaagtttttt agagaagaag tcatagcaag 3240
attaagccct aatccaaaag aacaaattga agcattgccc aaccattttt atagcgaaga 3300
tgaacttgct gatcttcaac cgatttttgt gtcaagaatg cccaagcgta gtataactgg 3360
agaggcgcat caggcacagt ttcgccgagt tgtgggtaaa acgaaagagg gtaaaaatat 3420
tactgcaaag aaaactgctt tagttgatat tagctatgat aaaaatggtg attttaatat 3480
gtatggaaga gaaacagatc ctgctactta tgaagcaatt aaagaaagat atctcgaatt 3540
tggaggaaat gctaaaaaag cattttcaac ggatttacat aaaccgaaaa aagatggcac 3600
caaaggccca ctgataaaat ctgtgagaat aatggaaaat aaaacattgg tacatcccgt 3660
aaataaagga aaaggtgttg tgtataacag ctctattgta aggacagatg tatttcaaag 3720
aaaagagaaa tattatttac tacctgtgta cgtaacagat gtaaccaaag ggaaactacc 3780
aaataaggtg attgttgcta aaaagggata cgatgattgg attgaggttg atgatagctt 3840
cacattttta tacagtctat atccgaatga tttaatattc atcagacaaa atccaaaaaa 3900
gaaaatatca ttaaaaaaac gtattgagag tcattctatt tctgatagta aagaggttca 3960
agaaattctt gcctattata aaggggttga tagttcaact gctgctatag agtttattac 4020
tcatgatggt agttactatg caaaaggtgt tggtgtccaa aatctagatt gttttgaaaa 4080
atatcaagtt gatactctgg gtaattattc caaagtgaaa ggagaaaaac gacttgagtt 4140
ggagacatct gataataacg acaaaggcaa agatgtcaat tcgattaaat caactagtcg 4200
ttaaacaaga agaagaggtt accatacctc ttgaagatat cgcatctatt gtggttgaag 4260
aaaatcaagt tgtgttaaca gctgctctat taagtaaatg cagtgaatat ggaattggtt 4320
tgtatacttg cgatgaaaag catttaccca atggtttgtt acttcccttt cagagtcatt 4380
caaggcaatt aagggtactt gaaacccaat ataacctttc aaagccattt aaaaaaagag 4440
tgtggcaaaa aataattaca actaagataa tgaatcaagc attgtgctta tcttatttgg 4500
ataaaaccgg agaaagtgaa ctgcttgcaa ttcgcaatac cgttgattca ggtgactcta 4560
cgaatagaga ggcatatgct gctaaagttt attttcaaca tttgtttggg aaacggttta 4620
ttcgaaggga agaccatata ataaaccact ccctcaatta tggttattcg atccttagag 4680
gaaatgtagc tagagtttta gtacagtatg gattctttcc atgtctagga attgaccatc 4740
acaatgaaaa taacgcattt aatttagctg atgattttat ggaggttttt cgcccattgg 4800
ttgatttatt tgtcgtgaaa cattttcatg aggagatgga atttacaccg aagtggagag 4860
caggattgta taatgtagtg aatcatcaag ttgaactaga aggtgaaaga tattcctgta 4920
atcgagctat tgaagaaatg attaaaagct acgttacagc atgcagaaca aatatgtatg 4980
agagactgag aacaccaaca ttactccctt tgaagattca ttcatatgag taaatatatg 5040
cgaatacttg tcttttttga tctaccggtg aaaaggaagc aggatcgtaa ggcttactgt 5100
gcatttagaa agtttttaat agcagatgga tatgatatgc tccagttttc agtctattct 5160
cgaatttgta atggcaatga aggaacacat aagcatttgg aaaggctgtc acgtaatttg 5220

ccctcaaagg gttcggtacg atcgttgatt attactgaaa agcaatatga aaagatgaaa 5280
ttttttgttg ggaaaccaaa aataaaagaa gagaaagtaa acagtgtgca actaacattt 5340
ttctgaaaaa gaaaattatt aataggaaga aaggagtttt tcttaccttt ttaaaatacc 5400
aaaagccttg annnnnnnnn nnnnnnnnnn nnnnnnnnnn nnnnnnnnnn nnnnnnnnnn 5460
nnnnnnnctt tcaaggcttt ttgggcacgt atcatatcat atcgagtttt agtaaggaac 5520
tatagcagaa acggattagt caatttgtcg atatgatcat atcatatcga gttttagtaa 5580
ggaactatag ctgttctgat ggaatgaact ggactttcgt catcatatca tatcgagttt 5640
tagtaaggaa ctatagcctt tatgggcggt cactgccggg atattcaatc atatcatatc 5700
gagttttagt aaggaactat agctttaatc attgcagttg ctaagaaaat tctatcatat 5760
catatcgagt tttagtaagg aactatagca gtcgtataca agtatggact ctcgaaatga 5820
tcatatcata tcgagtttta gtaaggaact atagcatgga tttttagttt gattcccgat 5880
atgaaatcat atcatatcga gttttagtaa ggaactatag cttggggtca cgtataatca 5940
aaccacgatc aatcatatca tatcgagttt tagtaaggaa ctatagcaga atcagcaaga 6000
tcactacgaa agcgaaaatc atatcatatc gagttttagt aaggaactat agctttcttt 6060
gctcccttct ccattcttgg tgaatcatat catatcgagt tttagtaagg aactatagca 6120
acgaaacaac cggggtgtca tctatattta tcatatcata tcgagtttta gtaaggaact 6180
atagcgcgag aggatgagga cggactttat cgtttatcat atcatatcga gttttagtaa 6240
ggaactatag cttacgatgc tatttcgatg ggggatggtt tatcatatca tatcgagttt 6300
tagtaaggaa ctatagccat gacgttagac cagttgaaag atttttgatc atatcatatc 6360
gagttttagt aaggaactat agcaatcgtg ctggattgga tcgctggata tcgatcatat 6420
catatcgagt tttagtaagg aactatagca cggtgccagc agggaaagcg gccagaagaa 6480
tcatatcata tcgagtttta gtaaggaact atagcaactc cagccctatg caaaggctgt 6540
atgggatcat atcatatcga gttttagtaa ggaactatag cacagttaga tatatactgt 6600
caggtatctt tatcatatca tatcgagttt tagtaaggaa ctatagctta gattgctcta 6660
actgatttag ctgaagcatc atatcatatc gagttttagt aaggaactat ggtctattgg 6720
gtattatcca aatcactatc ctactcaaac cccagacgga tagtcttttt tattaataaa 6780
catgtaattt tacccattgc ttaatctcct catcatttaa taaaatagga acacacattc 6840
gtatttaggg agatgtttta atggctagta aaattcttga cccacttgta acgaagttta 6900
ttttgccaga acatgcagaa atgttacgtc agtatcatga ggataagaag ctgatcgaga 6960
agccgttcat tgaagaggat gagctggccg agttttgcta caggatatct gactcacgtc 7020
agtatgacta tgcattaacc attagttggt ggaaagaggc aaaagaaggt agaggcgtga 7080
tagaatccgc ttggggatgg gtagataagt ttgattctca atttaaacag attaagctaa 7140
aaaacgatga ggatttctgg tggattcctg tagaagatgt agtgagtgtg gaagcctaag 7200
cagagaatct tataactgca tatagtgtat tgcctgtggg aacagggata cagcaaacac 7260
cccactatta atagcctagt ggggtgtttg catttaacat tcaatagcgt tgcaagcggt 7320
tcggcttatt cattgcatca at 7342

<210> 3
<211> 5877
<212> DNA
<213> Artificial Sequence

<220>
<223> Bacilllus species seqeunce

<400> 3
gcccaaaatg aagttaggtt catacataaa ttgtttggaa ttgcatcata atatgtcatt 60
gaacagttca tgtaggtttc aatgtctcta tcaaatgatt tttgcaccag aacccttttt 120
tatgttatac ccatttccac tgaaatttca agaaaaaatt gttataatac gagacattat 180
taatgattat tttaatggtt tttactctaa atcacagaga atattatacc agattcttgt 240
tattccttct gaagactacc catatttctg tgttttttat ttgaaaaatc tgatattttt 300
gttatgatag aggtatcatg gtacagcaat ggctttgatg tttctatgat aagggctttg 360
gcccgtggcg taggggatcg cctgcccatt ttaatgggct tctccccatc tatttaatga 420
gagttttaca accttggcta ttcttaaata gctaaggttt ttttgtatac atataaatta 480
aaagaggtga gattatgaga gagttggatt atcgcatcgg attagatatt ggaacgaatt 540
ctattggctg gggtgttatt gaattatctt ggaacaaaga tagagaacaa tatgagaaag 600
cgagaattgt cgacaagggt gttcgtatgt ttgataaggc tgaaatacct aagactggtg 660
cttctcttgc tgaaccgagg cgtatagcgc gctcatcacg tagaaggtta aatcgcaaaa 720
gccagagaaa aaaagatata cgtaatttac ttgttcaaca tgaaattatt agccaaaaag 780
aattagcttc gttatatcct ctgtcaaaaa gttcaatgga tatttgggat attcgtttgg 840
atggattaga tcgcttgtta gaccgttttg aatgggctcg actattaatt catttagcac 900
aaaaacgcgg ttttaaatca aatcgaaagt ctgaactgaa agatgtggag acaggaaaag 960
tattatcaag tattcaagca aatgaaaaac gattatctct gtaccgtaca gtgggagaga 1020

tgtggatgaa gaatatggat tttagtaaat atggcaaaag gcgtaattct cctaatgagt 1080
atgtattttc ggttagccgt gcagatttag aaaaggaaat tgtgactcta tttgaagcgc 1140
aaagaaaatt tcattcatca tatgcatcgg ttgatttaca aaaaacatat ttacgaattt 1200
gggcacacca actccctttt gcttctggga atgcaattgt aaataaagta ggatattgtt 1260
cattattaaa aggtaaagaa aagagagttc cgaaagcaac atatactttt caatatttca 1320
atacactaga tcaaataaac cgaacacgat tagggcctga tttccaacca tttacgaagg 1380
aacagagaga cattatttta gataaaatgt ttcaacggac agattattat aaaaaaaaga 1440
caatacccga agttacttac tatgatattc ggaaatggtt agcactagat gaaacaattc 1500
aatttaaagg acttacctat gacccaaacg aagagctgaa aaaaatagaa ttgaaaccct 1560
ttattaattt aaagccattt tatgaaatta aaaaggtagt tactaattac gcccaaaaaa 1620
caaatgaggt attctcaaca ttagactatg atacagttgc atatgcttta acagtttata 1680
aaacggacaa agatattaga tcttatttga agagatctaa taatttatca aaacgttgct 1740
atgatgatca attaatagaa gagctattaa ctctctccta tacaaagttt ggtcatttat 1800
catttaaagc aattaatcat gtactgccaa ttatgcaaga gggaaggact tatcaggaag 1860
caataaacca attaggatat gataccacta atcttaaaaa agaaaacaga agtatgttct 1920
tgcctcttat tccagatgag ataacaaatc caattgttaa aagagcgcta actcaagcac 1980
gtaaagttgt aaatgctatt attagaagat atggttctcc aaattctgtt catattgaac 2040
tagctcgtga gctttctaaa agtcatgacg agagaaagaa aataatgaca gctcatgatg 2100
aaaattataa gaaaaataaa ggagctatat caattttgat cgagaatgga attttaaatc 2160
cgacaggata tgatattgtg cgttataagt tatggaaaga gcaaggagaa cgatgtgctt 2220
attcgttaaa aaagattcct gctaatacgt tttttaatga attgaaaaaa gagcgaagtg 2280
gccctccggt tctagaggta gatcacattt taccgtatag tcagagtttt attgatagtt 2340
accataataa agtattagtt tatggggatg agaatcaaaa aaagggaaat cgaattccat 2400
atactttttt ttcagaagaa aataaggagt gggaaagctt tgaaagctac gtaagatcga 2460
atagtttttt ttctaaaaag aagcgcggat atttattgaa aaaagcttac ttgccaagag 2520
agagtaacct gattaaggag cgtcatttaa atgatacccg atatgctagt agctatttga 2580
aaaatttcat tgagaaaaat ttaaaattta aagaagctga aggtattaca cgaaaaaaac 2640
atgtacagac ggttaacggt gtaattacag cccatctgcg aaaaagatgg ggcttagaaa 2700
aagataggca ggaaacatat ttgcatcatg caatggacgc tattattgtt gcttgtacag 2760
atcatcatat ggtcactaaa gtaacggagt actatcaaat aaaagaaagt aataagtcaa 2820
taaagaaacc gtattttcct ttgccatgga tgggctttag agaggaaatt ttatcacatt 2880
tagaaagtca gccaattgct agaaaaatta gtgaggaact taaaattgga tatcaatcat 2940
ctgattatat acttgtatcg cgaatgccaa aaagatctgt cactggatca gctcatgacc 3000
agacagtcat gaaaaaaggc ggtatcgata aaaaaggaaa gactattatc ataaagcgtg 3060
tgcacttaaa ggatattaag tttgatgaga atggcgattt taaaatggtt ggaaaagaac 3120
aagatttagc aacttacgaa gcgataaagc aaagatattt agagtatgga aaagaatcga 3180
aaaaagcatt tgaaacacct ttatacaagc ctagtaaaaa aggaaaagga aacctcatta 3240
aaaaaattaa agtagaagtg caaaccaaat cttttgttcg agaagttaat gggggcgtag 3300
ctcaaaatgg tgatttagta agaatagatc tgtttgaaaa ggataatagg tattatatgg 3360
tacctattta cgtagtagat actgttcgtt ccgaactacc aaataaagct gtaaaaagca 3420
gtaaaggcta tgagcaatgg ctaacaatag ataacagctt tacgttcaaa ttcagtttat 3480
attcttatga tttagtacga cttgtaaaag gtgatgaaga tcgtttccta tactttagta 3540
ctcttgatat taattcggac cgtcttaatt ttaaagatgt aaataagcca tcaaagcagg 3600
ctgaatatcg ttatagtctt aaaacaattg agaatttaga aaaatatgag gttggtgttt 3660
taggtgattt aaggttagtg agacaagaaa cacgtaaaat tttttaatag ataaaaatta 3720
aagagatttt aacacggtaa gtaacattag tttgtttacc gtattttttt gtcttttttc 3780
gtctagaata ctagataata agtattacta ggagggattt aaatgggatg gcggcatgtt 3840
atgattacaa aaaatgctcg tttatctatt aagcattctc aattagttgt acaacaagaa 3900
gaaatagcaa caatcccact acaagatatt gcttctattc ttattgaaga tttagctgtt 3960
acgcttactg caagtgtact tagtaaatgt gccgagtata aaattagttt aattgcgtgt 4020
aattctaaga aacttccaaa tggtattttg actagttttt aacagcattc tcgccaattg 4080
acagttttga atatgcagtt aaatcttgca aaacctctta aaaaaaggat atggcaacaa 4140
attatttgcc gaaaaattat gaatcaagca ctttgtttag aatacttgaa ttgtggaggc 4200
gcatatgaat tgaataaaat tgtaaagacc gtagaatctg gagatacctc taatcgtgaa 4260
gcatatgcag caaagcaata ttttcgatat ttatttagtg attcttttac acgtcgttcc 4320
gaaaatccta taaaccggtt gttgaattat ggatacgcta ttataagaag tgcagtagca 4380
cgttcattaa ctaattatgg ttttttcaca tgtttaggaa ttcgtcataa taatcagtta 4440
aattcgttca atttagcaga cgatttcatg gaagtatttc gtcctatagt agatcaatac 4500
gtaattcaac aagacccaga aaactgggat ttctctgtac gtgcaggtct tgtgaattta 4560
ttaaacatag atgttaacat aaatggtgag ttgcgttcag ttacaacggc tattgatgat 4620
atggtaaaaa gttttatagc ttgttgtcgt caagaaacaa cttcttacct taaactacca 4680
gaattgctcc ctattagggt gcatcaatat gagtaaatat atgagaatct tattgttttt 4740
tgatttacct gtaaagacga aaacggaaag acgggattat gttcggttta gaaattattt 4800

actaaatgag gggtttgaca tgatacagtt ctctgtatat tgtcgtttat gtaatggtca 4860
tgaatctatg gagcgtcaat taggcaagat aaaaacaaga cttccgtcta aagggtctat 4920
cagagcatta cctgtaaccg aaaaacaatt tgagcggatg aggttattat taggagaaat 4980
aacaacaaac gaaagaagag tgaatattaa tcaattaaca ctgttctaat atgaacttac 5040
catgtataga aaaattttat catggaaaaa ctagtatgtt tgtgtataag gtaattatga 5100
aacggcaata ctgttaaata actatagttg ttcaaggact ctgttttatg aaaaacaaca 5160
aaatggagga taactcactc tatataggtt aaaaaataaa aaacagaaag cctgtaagca 5220
ttgatattac aggctttttt gaatacgtat cataatacag caatggcttt aatggaacta 5280
tgaccgtaaa gtagcttata gcaaacggac agtaatcata atacagcaat ggctttaatg 5340
gaactatgac aaaaaggcta ctcttaaatg tgtagtcttt atcataatac agcaatggct 5400
ttaatggaac tatgacgacc tttggcaggg aagttgcgag gaactgatca taatacagca 5460
atggctttaa tggaactatg accgatgcat caagtttaag gcgatcggat aaatcataat 5520
acagcaacgg ctttaatgga actatgacaa tttgttccaa tgatggaatg catatatgat 5580
caaaatgtct ctttaatttt acggagtaac tcaattcctt ttttatgtta taacaaatca 5640
tacgtattct tcaagcctga gtatattact ttgaaggtgt agcatgtgat ctgtccctta 5700
tctcagcaca aaactgttcc atctgttgtt ctgtgaattt ttcttctttt atatccccaa 5760
acgttgctga accaaggaat acttcatcat cacagttgat atcccaggac tcaaaaggtt 5820
tctctccttt tgaaactttc ttagtgaatc gcatccattc gccatcaatg tatacta 5877

<210> 4
<211> 5684
<212> DNA
<213> Bacillus cereus

<400> 4
agcttcaagc tttgcgtggt gagaattaag gttatcatag catagcgatg tttcttttgg 60
aactatagca gtttttcatc actatctgat actttcttaa tatcatagca tagcgatgtt 120
tcttttggaa ctatagccta gatggcttat ccctttcaga aggcttgatc atagcatagc 180
gatgtttctt ttggaactat agcaacatga cttggttgca ttacgaaagt ttcatcatag 240
catagcgatg tttcttgtgg aactatagca tcagctaaca tgcgtaagta atccctaata 300
tcatagcata gccatgtttc ttgtggaatt atagctcaga cttatcttcc ttcttcattt 360
cttgtatcac atcatagcga tgttttctat ggaattataa tctcacattg tacgtaatgc 420
aaaacataaa aattaaatca tagcatagcg atttttcttt tggaattata gtacttccct 480
aagtcttgca ggaaacatat taatcacatc atagcgatgt ttttttggaa ttatagctaa 540
actccaattg gagaactcca tttatttatc acatcgtagc gatgattctt ttggaattat 600
agattacagc ggtaaaatat tacatataaa attcatataa ctattattcc catttgcttc 660
atttttataa ttatacgttt atctaggtat aaagtttatt tttggtgtca ataatgaaac 720
tataaggatc atatcatagc gatgtttctt aagttattat agtaagggca atgacccgtg 780
gcgtttgggg atcgccttat cctgtatgga tattctcccc atgttaaaag cacctacgca 840
taacgctatg gtgcttttat tttgtaaaac atgaaggagg ttgttaattt gaaatacgta 900
ttaggattgg atattggaat cgcatcttgt ggttgggctg taataaatca agaaaaacat 960
agaattgaag atttaggggt acgtattttt gataaagctg agcaccctaa agatggaaaa 1020
tccttagcag caccaagacg tgaatctcgc tctactagga aaacacttcg tcgtaaaaaa 1080
catagaatgc aacgtatcaa agtactttta gttaagtatg gtttattatc tacgactgag 1140
attgaaaatt tatatgaaag tgctagtgaa attgatgttt ggaacctacg tttagatgct 1200
ctagaacgca agttaaatca aaaggaattt gcaagagtcc ttattcacct tgctaaaaga 1260
agaggattta aatctaacat gaaagaaaca attctctcag aaaacggaca aatcttagaa 1320
agtatttcag aaaatcaaaa gattatggag caacaaaatt atagaactgt tggagaaatg 1380
attgtaaaag ataacaagtt tgaaaaccat aaaagaaaca aagatggtac ttatatcggg 1440
actgttactc gtcaacaact tcaagatgaa atacatatga ttttcaatgc acaacgtttg 1500
tttataaatg accttgcaac agaggaattt gaaagtagct acttagaaat ttgggcatct 1560
caacgtccct atgcttctaa agatcaaatt gaaaaaatga ttggttactg taccttagaa 1620
ccaaaagaaa aaagagtccc taaagcttca tggtcatttc aatattttgt agcgctacaa 1680
accataaaca acctacgact gattagtaaa gacaggatag aagaactctc ttttgaagaa 1740
aagaaacaaa ttatgaatct agcacttgaa aaacctattg ttaaatacat tgatatccga 1800
aaattattat ccatttctaa tgaacttcat tttaacaatc tattatattc agctgataca 1860
gtagatacaa cggtggaaaa taaaaaatgt atagaactca aagaatatca ctctattaat 1920
aaggtatata aacaaattta tggaaagaac gccttaaatt tattacctat agattacgat 1980
acaattgcat acggactaac tatcttcaaa gacgataaag atatcctaga atacttacaa 2040
aataaatata taaatacaaa aggaaaaact ataagtaatt taacaaaaaa gacatacgat 2100
gacacgttta ttcaagcatt attgactttg aacttcacaa aatggacaca tttatcattt 2160
aaagccttaa aaaatatcat tcctttttta gaagagggaa tgtcatatga taaagcttgt 2220
gataaggcag gatataactt taagggaaca tcatttacaa aaaaaacaaa gtacttgcct 2280

gttattccac agaatacaaa tcctgttgta catcgtgctc tttcacaaac aagaaaggtc 2340
ataaatgcaa ttataaataa atacggttct ccagtttcaa ttcatattga aaccgcaaga 2400
gagttatcta aaacattcca ggaaagaaaa gaaatagata gcatgtacca ggataactcc 2460
aaaaagaatg aacacgccat taataagcta aaagaattag gtaacattaa tccctcggga 2520
ataaatattg tcaaatttaa gctttggaag gaacaagacg gtagatgtat gtattccgga 2580
gaatatattg aaccacatcg attatttgaa gagggatata ccgaagtaga tcacatacta 2640
ccctatagtc gtagtttaga tgacagctat aacaataaaa cattagcttt aggaatagaa 2700
aatcaacgaa aagggaacaa aactccttat gagtacatgg ggaacacctc aatttggtac 2760
aaatttgaaa cgagggtaca gtcgaataaa aaaattaaca aaaagaaaca acaaaaattg 2820
cttctacaat acttttcgca tgcaagagag caagaattta tagagcgaaa tctaaatgat 2880
acccgttacg ccacaatata tatatcagcg tttattcaac aacacctcat tttctctgaa 2940
agcttcagta aaaaaagagt tcatacagtt agcggaatta taacatctca tgtacgtagt 3000
agatggggat ttaacaagga tcgtaaagaa agtcatatcc atcacgcatt agatgcagtg 3060
attgttgctg tcacatctga tcaaatgatt caaagagtaa caaattacta taaactaaaa 3120
gaattaaata gaaagcttca agccaaaggg atactgtttc ccgaaccttg ggaggggttt 3180
cgtttagaat tagaagcaag aatttctcca aatacccagc aatatttaaa gggaatttgt 3240
tctaaaaatt acgccgatat caatctatcc gaaatcaagc ctatctttgt ttcaagaatg 3300
cctaagcgat ctattactgg tgaattacat caagagacaa ttcgaaaatt tattggttat 3360
aacgaaaaag ggaaagtatt aaccgcaata aaaacaaagt tagaagatat tccttttgat 3420
gccaatggtg attttcctat gtatggaaaa gaaactgatt tgtatacgta taatgccatc 3480
aaagaacgtt atttaagtca taaaaaagat aaacgtaaat catttcaaga atccttatac 3540
aaacccacaa aaacaggaga aattgggcca cttataaaat ccattaaaat aattgataca 3600
agaaccattg tcaatcctgt taaccaaggt aaaggtgtcg tatataatag taaaattgct 3660
cgtactgatg tattcaaaaa agatgataag tactatttaa ttcccattta tacaattgac 3720
cttatgaaaa atgttttacc tcaaaaagca attacagcag gaaaaggata tgaagattgg 3780
ataaccattg atcatagctt tactttttta tttagccttt ttcctaatga cctaataaaa 3840
attgtcccct caaaaaataa agaaattaaa gctaggtcaa cttcatcaaa aaaagaaata 3900
ttactaccat cactcacagg atattttaag aaggttcata gtggaacagc aggcattact 3960
gttgaatccc acgatggtag gtttatagca gatataggct caaagcaatt attattattt 4020
gaaaaacacc aggtagatgt actgggacat tacacaaaaa taaaggagga aaaacgtatt 4080
gggatgtcga catgtaatga taacaaaaag agctcagctt tcggttcgct ataatcaact 4140
ggttataaag caagacgata aagtaagtat tcctttagag gatattgcct ctataattat 4200
agaagataat tcggtaacta tttcagccta tacacttagt aaattaagtg attataacat 4260
cgggttggtt acatgtaatg aaaaacactt accaaacggt atactaacat cttttcaaaa 4320
gcatacaaga cacgcaaaag ttcttcaaac acagtataac ttatccaagc ctttcagaaa 4380
aagaatatgg caaaaaatta ttatgaagaa gattatgaat caggctaagt gccttgaaat 4440
cttagaaaaa gagggatata aagaaatttt atcaatatgt aaaaccatag aatcaggaga 4500
tgtgaccaac agagaagcat atgcagcaaa actatatttc aaatatttat ttagtgcaca 4560
atttacaaga agaacagaaa acatatataa ctattcgtta aattatggat attcaataat 4620
acgtggaatg gttgcaagaa cattagttag ttatggtttc acaccctctc ttggaattaa 4680
acatcacaat gagttaaata attttaattt ggcagatgat tttatggaag tatttcgacc 4740
tatagttgat ttattcgtag cttcccatat agattattct acagaattta gtaaagaagt 4800
tcgaagaaac ttatacaaca tttcaaatta tgaaatacaa atcgacagta aatatcatag 4860
tattcaacaa gcaattgaag taatggtgaa aagttatgta tccgcttgtc gtacggaaga 4920
ttattgttta ataaaacttc ctaatttaat caaattaaga atgcattcat atgagtaatt 4980
tcatgcggat attagtcttt tttgacttac ctgtcaagca aaaaagtgaa cgaaaagaat 5040
atatgcgatt ccgtaaattc ctactcaatg atggatatga catgctacaa ttttcaattt 5100
actgtagaat ttgtaatggg aacgaaggta caaaaaaata tttagagcgc ttaaaatgca 5160
atgttcccga aaaaggttct attcgcacat taacgattac agataagcaa tatgctaaaa 5220
tggagttttt agtaggaaaa ccaacaccaa cagagaaaaa aataacttct gctcaactta 5280
cattatttta aaaagaaaaa gttggaacaa aaaacaaata taaaacctct tttttaaact 5340
taaaaacctt gtctatccct ttaatgacaa ggtttttttg acatctacca tatcatagcg 5400
atgtttctta tggaactata gctttattct atcgttcatc ataacaatgt aaaccatatc 5460
atagcgatgt ttcttatgga actatagcaa cacgtaatag aatcataagt gcagttacta 5520
ccatatcata gcgatgtttc ttatggaact atagctcgtc gcccataaac aaacttattc 5580
tttctgttat attatgataa tttttcttat gaaacagtat cctattagcc ttatttcacg 5640
aaaacccaaa tttcatagca gtattttcaa ataaaattac atct 5684

<210> 5
<211> 8221
<212> DNA
<213> Lactobacillus fermentum

<400> 5
acgaccaacc gatcacgcct aagcaagtcg ggtccgtctt tgccctcggc acggtggcgg 60
tcctggtcgg ggcgatgacc aacctcggcg ttaactccag cggccaaacc gttacggtgg 120
ccgcccaacg ctcccagcta tcgacccagt acggggccaa gccagttgac ctggcgccgg 180
gcaagcaaat cagccaggtc gtcaagatca acggggcagc cagtgacgtc tccgtgcaga 240
tccacaccaa gtcacacgtt acggtgaccc tggtagaact aaaaactggt gacgaatacg 300
tcttcaagcg ccacgccgac gagtacgatt accaagggaa cgtgccagcc ggtacctacc 360
agttcgtgat caagaaccaa accgccgtgg accaagatgt tgacatcgcc cacgtcccgt 420
actaccggat ggcgccgtac ccgatggacg accagggaac ggtccactgg gacacgtcat 480
tgatctactg gttccgtgac gttcgctccg ccaattaaat agaattagaa aaccccagcc 540
gttcgggaaa ccgagggctg ggtttttggg tgggaagagg agaaaatttt gaattaggag 600
ggatttttgg gcgggcgaca gaatatgtcc gaggggtggg atactatgga gttagtcggc 660
aaacgcaaaa agaggtcgca aaatagtcgc tgatccttcg ttcaaattta tcaaaataac 720
tactgaaagc gattgcgttt gctataatct atcttgacta cgatattaag gagtggatta 780
cgtgaaagag tatcacattg gcttggacat tgggacaagc tcaattggct gggcggtaac 840
ggatagccaa tttaaactga tgcgtatcaa ggggaagacg gcaatcgggg tgcggttgtt 900
tgaagaaggg aagacggctg cagaacgtcg aaccttccgg acaacgcggc ggcggttgaa 960
gcggcgaaaa tggcgcttgc actacttaga taaaattttt gccccgcact tacaagaagt 1020
ggatgaaaat ttcttacgcc gtctgaagca atcaaatatc cacccggaag atccagcgaa 1080
gaatcaagcc ttcattggta agctcttatt tccggatcta ctgaagaaga atgaacgtgg 1140
ttacccaaca ctgattaaga tgcgcgatga attgccagtt gagcaacgcg cccactaccc 1200
agtaacgaac atctacaagt tacgggaggc catgattaac gaagatcgcc aattcgatct 1260
gcgggaagtt tacctggccg ttcaccacat cgtgaagtac cggggtcact tcttaaataa 1320
cgctagcgtt gataagttca aagtggggag aatcgatttt gacaaatcct ttaacgtttt 1380
gaacgaagcc tacgaggagc ttcaaaacgg cgaaggttca ttcacgattg agcctagcaa 1440
ggttgaaaaa attggacaac tgttgctaga tacgaagatg cgtaagttgg accgccaaaa 1500
agcggttgcc aaattgttag aggtgaaggt cgctgacaaa gaggagacca agcgcaacaa 1560
gcaaattgca acggcgatga gtaagttagt tcttggctac aaggcggact ttgcaacggt 1620
agcgatggcg aatggcaacg aatggaagat cgatttaagt tctgaaacca gtgaggacga 1680
gattgaaaaa ttccgggaag aattgagtga tgctcaaaat gatatcttaa cggaaatcac 1740
tagcctgttc tcgcagatca tgctgaatga aatcgtgcca aatgggatgt cgatttctga 1800
atcgatgatg gatcgttatt ggatgcacga acgtcaatta gcggaagtta aggaatactt 1860
ggcgacccaa ccagctagcg cgcggaaaga ttttgaccaa gtgtataaca aatacattgg 1920
gcaagcgcca aaggaaaagg gctttgacct tgaaaaggga ctcaagaaga ttttatccaa 1980
aaaggaaaat tggaaagaaa tcgatgagtt actcaaggca ggtgatttct taccaaagca 2040
acggacatca gccaatgggg tcatcccaca ccagatgcac caacaagaat tggaccgaat 2100
tattgaaaag caagctaagt actacccgtg gctagcaacg gaaaacccag ccactggtga 2160
acgtgatcgt caccaagcga agtatgaact tgatcagcta gtttccttcc ggattcctta 2220
ctatgttgga ccgctagtaa cgcctgaggt tcaaaaggcg acatctggcg ctaagtttgc 2280
ttgggcaaag cggaaagaag atggggagat taccccttgg aacctgtggg ataagattga 2340
ccgtgctgaa tcagctgaag cctttatcaa gcggatgacg gttaaggata cgtatctatt 2400
aaacgaggat gtcctgcctg ctaacagctt gctataccaa aagtacaacg tcttaaacga 2460
actaaacaac gttcgagtga atggacgccg cctatcagtc ggaattaaac aagatattta 2520
cactgagctc ttcaagaaga aaaagacggt gaaggcaggt gacgtggcca gcctagtgat 2580
ggcgaagaca cgaggagtta acaagccaag tgttgaaggt ttatccgatc cgaagaagtt 2640
caattcaaat ttggcaacct accttgatct aaagtcaatt gtgggtgaca aggttgatga 2700
taaccgttat caaatggatc tcgaaaacat tatcgagtgg cgttccgtct ttgaagatgg 2760
ggaaattttt gcggacaagt taactgaagt ggaatggctg acggatgagc aacgttctgc 2820
ccttgttaag aaacggtata agggctgggg acgtttatcc aagaagctac tgacgggaat 2880
cgttgacgag aacggccaac ggatcattga tctgatgtgg aatactgatc aaaacttcat 2940
gcagattgtc aaccagcctg tctttaaaga acagattgac caactgaacc aaaaggccat 3000
caccaatgat gggatgactt tgcgggagcg ggtcgagagc gttctggatg acgcctatac 3060
ttcaccgcag aacaagaagg ccatctggca agttgttcgg gtggtggaag atattgttaa 3120
agcggtcggg aatgcaccaa agtcgatttc aattgaattt gcccgtaatg aaggcaacaa 3180
gggagaaata acccggagcc ggcgtactca attgcagaaa ttatttgaag atcaggccca 3240
tgaattagtt aaggatacga gcttgactga ggaattggag aaggcaccgg atctttccga 3300
tcgttattac ttctacttca cccaaggtgg taaggatatg tacaccggtg atccaatcaa 3360
cttcgatgaa atttctacca agtatgatat tgaccacatt ttgccacaat ccttcgtgaa 3420
ggatgattcc ttagataatc gggtgttagt aagtcgggcg gagaataata agaagtctga 3480
ccgggtgcca gctaagcttt acgcagccaa gatgaaacca tactggaacc aacttttgaa 3540
gcagggattg atcacgcaac ggaagtttga aaacctgacg atggacgttg atcaaaatat 3600
taagtatcgg tcacttggat tcgtgaagcg ccaattagtt gaaacacgcc aagttatcaa 3660
gttgacggct aacatcttgg gtagtatgta ccaagaagcc gggacggaca tcattgagac 3720

tcgggctggt ttaactaagc aattacgcga ggaattcgat ttaccaaagg ttcgtgaagt 3780
aaacgactat caccacgcag ttgatgctta cctgacaacg tttgctgggc aatatttgaa 3840
ccgccgttat ccgaaattac gctccttctt cgtatacggt gagtacatga agttcaagca 3900
tgggtcagat ctgaagctac gtaactttaa cttcttccat gaattgatgg aaggcgacaa 3960
gagccaaggt aaggttgtcg atcaacaaac tggtgagctg attacgacaa gggatgaagt 4020
agccaagagc tttgatcgat tattgaacat gaagtatatg ctggtatcga aggaagttca 4080
cgaccggagt gaccaactgt atggagcaac gattgtaaca gcaaaggaat ctggtaagct 4140
cacttcacca attgagatca agaaaaatcg acctgttgat ttgtatggtg cttatacgaa 4200
tggaacgtca gctttcatga cgatcattaa gtttactgga aacaaaccta agtataaggt 4260
gattggggtt ccaacaacat cggcggtcgg tttaaaacga gttgggaaac caggcagtga 4320
atcatataac caagaattac atcgaatcat taaatctaat ccgaaggtaa agaaagattt 4380
tgagattgta gttccacatg tttcgtatag tcagttgata gttgatggtg actgcaaatt 4440
taccctagct agtgatactt atcagcatcc ggcaactcag ctagttcttt ctaaagaatc 4500
gatggaaata attgcagatg attttaaatt tgtgaaagaa aatccggcaa ctgcggatga 4560
acaacttgtt agggtttttg atgaaattgt taatcaaatg aaccattact ttactatctt 4620
tgaccaacgt agtaatcgtc aaaaggtaac gaaggctcga gataagtttg tatcgttacc 4680
aacggaatca gagtatgagg gtgctaagaa aacgcaagta gggaagacag aagtcattac 4740
gaacttactg atgggtctac atgccaatgc tgcacaaggt gatttgaaag tattaggatt 4800
atcaaccttt gggtttttcc agagttcagg tggactgaat ttaagtgaag atgcaatgat 4860
cgtataccag tcaccaaccg gtttgtttga acgtcgcatt tgtttgaaag acatttaaga 4920
gtaaagtaaa aaggagccga ttaaatcgac tcctttttat tatatgtatg gccagaataa 4980
catggctcaa aagtaataac tcgtggaaaa ccactcgttt gatcttgact atgagtgtta 5040
gatcagtagt ctcgaatgtc gggagctacc cgacaagtta tattatatgg aattcggttg 5100
aatttgacaa ggaggaattt taaaaatggg atggcgttca ataatcattg gtcaacatgc 5160
aaagattacc cactctgctc atatgatggt tgtgcagact aaagatggga ttaatgaaat 5220
tccgatggat gacattgcaa aagtactagt tgaaacaact caggcgatga tctcttcaga 5280
tttcatgagt gcatgcctac aacggaacat taaagtaatc tttgtcgatc gaaagtacca 5340
accgattggg gagacggttg gttatcagcc agtagcgagg gattatgaat tgttggaacg 5400
ccaattctta tggcccttgg agcgaaggga acaactttgg actaggattg cccacgccaa 5460
aatcgagagc caaataaagg tggcccggct taatggatgc ccggttcaag aactagaaag 5520
tgagttagaa gccattgaag taaatgatat ttctaaccgt gaggctgtgg ttgctcgaaa 5580
gtatttccca ttgctgttcg gaaaagattt tagccgccga caggagaatc aatacaacgc 5640
cgctctggat tatggatatg cggttttatt agcggagaca aaccaatcga ttacggaaaa 5700
tggctatctg atggaactag gaatccatca tcaaaatcga gaaaacaggt ttaatttggg 5760
ttcagatttg atggaaccat ttcgaccagt gatcgactac tgggttacag gacagaaatt 5820
tgcggaattc aatcgtcaag tccgagttgg cttagtagat gcaatgcata cggtgatcaa 5880
gtataacggg attgaaatgc tgactcaaaa tgcagtgagg cagtatgtgc gggaatgtct 5940
tgattacttg agtggtgagc gggatgaaat tgaagtaaag gtggagttta aagatgaggt 6000
accgaatcat gcgcttgatg gtcatgtttg atttgcccac ggacactagt cagcaacgaa 6060
agcagtatcg tcagttccgc aagaagctat taaatgaagg atttattatg atccagtact 6120
ctgtttacgt tcgtgtatgt acgacaagga gtgcggctga gtttttggag agaaggctta 6180
agaattattt accagaccaa ggagtcatcc aatcgttaat gttaacggaa aagcaataca 6240
gtgacatgca tttcttgctt ggtgatgagg ttaaagaagt tagaaattca tcaaagagga 6300
cgattgtact atgaaattgc aataccatgg ccatcaacca gttgaactga tgcctggaaa 6360
agtagtgatt ttgcaaatag agaacccaag cgtcaacacc gaattaattc aggggttgca 6420
gggatataat gattgcttaa ggatgattga cgatgagtac caagagctta atttgagtaa 6480
ggtaatgaag tgggaaggag accttgttct taatccaacc aagcttgata gctaccaaac 6540
agcacttgat aagatgattg tcaaaaatct tagcattcag aaacgagatc ggatgaatga 6600
ccttgctcgt gaggtgttct cagagatgca agattctttg ttcgaattcg acttaccgtt 6660
ggaggtgcgg tatgacgaga gcctcttgag attatacaaa tatgcgaaaa ttaaatattt 6720
gacgcaagta ataaggcaac cttatggtat aattgaaacc gatctaaaat tacatctcga 6780
attaaaagac tgtcaggttc ttgggtattg taatgtcgct aattacttat tgcctgagca 6840
gatacaggaa ttcgctactc ttgttgagaa aaccgaattg gcagttttat tagtggaatt 6900
ctcagaaaag agagaaactc tacaacaact caaacaggat atttaccgga ttgatcggga 6960
cttcgtggac tggcacgaat agatcgttag tttttgatga gaaaataacg gtcttggatg 7020
agtgtcagat cagtagttcc gagtacatcg attacaggtc aacagagttc cgatgagtct 7080
tggatgagtg tcagatcagt agttccgagt acagctaacg tccaccgagt gggcactgtt 7140
ttgtcttgga tgagtgtcag atcagtagtt ccgagtaccg gtactgccca gctgtcagcg 7200
taggttgggt cttggatgag tgtcagatca gtagttccga gtaccgattg taccgaaata 7260
cgacctttgc atttgtcttg gatgagtgtc agatcagtag ttccgagtac tgatctttta 7320
aaaatttgat ggtttctttc gtcttggatg agtgtcagat cagtagttcc gagtacgtga 7380
ccgttaagga tcagccgtcc acgcttgtct tggatgagtg tcagatcagt agttccgagt 7440
acttgctacc agtcttagcc acgccggtgg aggtcttgga tgagtgtcag atcagtagtt 7500

ccgagtacgt gacggttggc ccagagggat tgacaacggt cttggatgag tgtcagatca 7560
gtagttccga gtacaggttt ttagtggaaa actgtactta tcgcgtcttg gatgagtgtc 7620
agatcagtag ttccgagtac tgtttgcttt taactggcca acttggtcgc gtcttggatg 7680
agtgtcagat cagtagttcc gagtacacta agctagcgat tttccacagt gcattggtct 7740
tggatgagtg tcagatcagt agttccgagt acatttcaaa gagtctcagc gcctctgata 7800
cggtcttgga tgagtgtcag atcagtagtt ccgagtaccg attgatagcg tgagttgtcg 7860
gtcccgtagt cttggatgag tgtcagatca gtagttccga gtaccacaat tggttaatgc 7920
aactcacacg atccgtcttg gatgagtgtc agatcagtag tcttataatt atagactaag 7980
ggcgttgctg tttttagcaa tgcctttttt agaagtgatg ggggaaagat aaatattccc 8040
acgttggtaa tctgacggga actttagcgg gcgatgtgct aggatagggc caaaatgatt 8100
ggaagtgttt accatgcaaa ttgatcccga acggattcgc cccgaactgc accggatcgg 8160
caaggtgggg tacgcgatca acctgatggc cagcccgcac gcctgcaacg aatgggacga 8220
a 8221

<210> 6
<211> 8221
<212> DNA
<213> Enterococcus faecalis

<400> 6
taaaaataaa ccagtaacgc taataactcc tataaaataa aaaaactttt tcaaaatatt 60
ccctccaatt aaatttatgt attaatttta tcattagtga tagtaaacat aaagaaataa 120
aaaattcaaa gtatcaataa acattgctga tactctgaat tatagctctg cccagacttt 180
tttagttgtg gaaaagttgt gattttttat ttttttattc cataaacctt atgatttgaa 240
gggattaaaa ggtttgctat ttatcgttta gcgaagtaaa gtaaaaaagc tgttttcgat 300
tttcgaaaac agcttttttt tgcaattttt gccttttaaa aacccaaaat cagaaccgtc 360
aatttactga aaatttcttg caaatcaaag gatcccacga tacgataaat aggagaaaag 420
gcaatgagtg aaacacttat tgttaactat ttaatcaaag gacgtgtgaa gatgaacgta 480
gaagaattag ttcaaaaagc aaaagattta attggagaag gcaacgtgga acatgcgcaa 540
caattcattg aagaacataa agatgaatta ggcgaacact atcataaaat tacagcgatg 600
ttagctgata caacgaccga tggtttgttt gataaaatta aaggattgtt tcaatagtcg 660
taattaaaaa ggcaaaacac agttaaccac gcactgtgtt ttgccttttt tgccatgtaa 720
agtttgacaa catcaacaaa tagtaaaaga tattaccata caataaatat agattcgtta 780
ttttaagaaa agtaaaggaa ttaaggtaag agattcttaa atagataatg ttatttttgt 840
tatgtatata attctattag tttacaaaaa ctataaataa cccttttttt aagaaaataa 900
gaatggtaaa ctttaagtag cggtattcag tttgattaat ttggaagcga ttactttctg 960
gctgcaaaca aaaaaaagcg ccgaaacagc gccactttac agttgattac ggttaaaacc 1020
ttattttaac ttgctatgtt gtttagaata gtcccaaaag aacttaacta aattatagca 1080
tagaataaaa atcattcaag tagtaattaa aaaaaaggag tggaaataaa atgaagaaag 1140
actacgttat cggtttggat atagggacta attctgtcgg ttgggcagtt atgacagaag 1200
actatcagtt ggtgaagaaa aaaatgccta tttatggaaa cactgaaaaa aagaaaatca 1260
agaaaaattt ttggggtgtg cgtttatttg aagaagggca tacagccgaa gatcgccgat 1320
taaaaagaac agcacgacga agaattagtc gtcgacggaa tcgtttacgc tacttacaag 1380
ctttttttga agaagcgatg acagctttgg atgaaaactt ttttgctcgt ttacaagaga 1440
gttttttagt gcctgaagat aagaagtggc acagacatcc gatttttgct aagttggaag 1500
atgaagtagc ttaccatgaa acgtatccga caatctacca tttacgcaaa aaattagcag 1560
attcatctga gcaagcagat ttacgactaa tttatttggc gttggcatat attgtcaaat 1620
atcgtggaca ttttttaatt gaagggaaat taagtacaga aaatatttct gttaaagaaa 1680
aatttcaaca atttatgatc atttataacc agacctttgt gaatggagag ggtcgcttag 1740
ttagtgcgcc attacctgaa tctgtcttga ttgaggaaga gttgactgaa aaagcttctc 1800
ggactaaaaa atctgaaaaa gtcttacaac aatttcctca agaaaaagct aatggcttat 1860
ttggtcagtt cttaaagcta atggtaggga ataaggctga ctttaaaaaa gtttttggtt 1920
tggaagaaga agccaaaata acctacgcta gtgaaagcta tgaagaagat ttagaaggca 1980
ttttagcaaa ggttggggat gaatatagtg acgtgttttt agctgctaaa aatgtgtacg 2040
atgcagttga attatcaacg attttagcag attcggataa aaaaagttac gcgaaattat 2100
cttctagcat gattgtccgt tttacagaac atcaagaaga tttaaaaaaa ttcaaacgat 2160
ttattcgtga aaattgtcca gacgaatatg ataatctctt taaaaatgaa cagaaagacg 2220
gctatgcagg ctacattgca cacgcaggta aggtttcaca gcttaaattt taccagtatg 2280
ttaagaaaat cattcaagat attgctggag cagaatattt tttagaaaaa attgctcaag 2340
aaaacttttt aagaaagcaa cggacctttg ataatggggt gattcctcat caaattcatt 2400
tggctgagtt acaagcaatc attcatcgtc aagcggccta ttatccattt ttaaaagaaa 2460
atcaagaaaa aattgaacaa ctggttacat ttcgaattcc ttattatgtc ggaccattat 2520
caaaaggaga tgcaagtacc ttcgcttggc taaaacgtca aagtgaggaa ccaattcgac 2580

cttggaacct tcaagaaacc gttgatttgg accaatcagc gacagccttt attgaacgaa 2640
tgaccaattt tgatacgtat ttaccttctg aaaaagtttt accgaaacat agtttgttat 2700
atgaaaagtt tatggtattt aatgaattga ccaagatttc ttatacggat gatcgaggca 2760
tcaaagccaa tttttcaggt aaggaaaaag aaaaaatctt tgattatctg tttaagacgc 2820
gtcgcaaagt taagaaaaag gatatcattc aattctatcg aaacgaatat aatacggaga 2880
ttgtcacgct tagtggactt gaagaagacc aatttaatgc tagttttagc acgtatcagg 2940
atttactgaa atgtggtttg acacgagcag aattagacca cccagataat gccgaaaaat 3000
tggaagatat cataaaaatt ttaactattt ttgaagatcg gcaacgaatt cggacgcaac 3060
tttctacatt caaagggcag ttctcagcag aagtactaaa aaaattagaa cgcaagcact 3120
acactggctg gggaagattg tcgaagaaat taatcaatgg tatctacgat aaagaatcgg 3180
gtaaaacaat tttggactat ttagttaaag atgatggggt ctcaaaacac tataatcgca 3240
attttatgca actgattaat gattcacaat tatcttttaa aaatgctatt caaaaagcac 3300
agtccagtga acatgaagaa acattaagtg aaactgtcaa tgaattagct ggtagcccag 3360
caataaaaaa aggaatttat caaagtttaa aaattgtcga tgaactagtc gcgattatgg 3420
gttatgcgcc taagcggatt gttgtcgaaa tggcacgtga aaatcaaacg actagcactg 3480
gcaagagaag atccatccaa cgcttgaaaa tagttgaaaa agcaatggcc gaaatcggca 3540
gcaatttatt aaaagaacaa ccaacgacta atgaacagtt acgagatacc cgtcttttcc 3600
tttactatat gcaaaatggt aaagatatgt acacgggaga tgaattatcg cttcatcgtt 3660
tatctcacta tgatattgat catattatcc cacaaagttt tatgaaagac gattcattgg 3720
ataacctagt tttagtgggc tctactgaaa atcgagggaa atccgatgat gtacctagca 3780
aggaagttgt taaggatatg aaagcttatt gggagaaatt atatgctgct ggcttaatta 3840
gtcaacggaa attccaacgt ctgaccaaag gggagcaagg tggcttgact ctcgaagaca 3900
aagcgcattt tatccaacga caattagtag aaactcgtca aattaccaaa aatgttgcag 3960
ggatcttaga tcaacgctac aatgccaagt caaaagagaa aaaagtccaa atcatcacct 4020
tgaaagctag tttaacgagc caatttcgtt cgatttttgg cttgtataaa gttcgtgaag 4080
tgaatgatta tcaccatgga caagatgctt atttaaactg tgtggtcgca accacgttat 4140
taaaagttta tcctaattta gcaccagaat ttgtttacgg agaatatcct aagttccaga 4200
cctttaaaga aaataaagcc acggcgaagg ctataattta tacaaatttg ctgcgctttt 4260
tcacggaaga tgaaccacgg ttcacgaagg atggcgaaat tctttggagt aacagttatt 4320
taaaaactat caaaaaagaa ttaaattacc atcaaatgaa tattgtcaaa aaagtagaag 4380
tgcaaaaagg cggtttttca aaagagtcga ttaaacccaa aggaccatcg aataaattga 4440
ttcctgtcaa aaatggtttg gatccccaaa aatatggcgg ttttgatagt cctatagttg 4500
catatacagt gctattcacg catgaaaaag gtaaaaaacc tcttattaaa caggagatac 4560
tgggcattac gattatggaa aaaacaaggt ttgaacaaaa tcctattctt tttttagagg 4620
agaaaggctt cctacggcct cgtgtattaa tgaaattgcc taagtataca ctgtatgaat 4680
ttccagaggg acgccggcgc ttattagcca gcgctaaaga agcgcaaaaa gggaaccaga 4740
tggttttacc tgaacattta cttacattac tgtaccatgc gaaacaatgt ttgttaccaa 4800
atcaatcaga aagtttggct tatgtagaac aacatcaacc agaatttcaa gagattttag 4860
agagagtggt tgactttgct gaagtgcata cattagccaa gtcaaaagtc cagcaaattg 4920
tgaaactttt tgaagcaaat caaacagcag atgtgaaaga gattgcggca tcctttatcc 4980
aacttatgca gtttaatgcg atgggcgcac caagtacgtt taaatttttc caaaaagaca 5040
ttgagcgagc acgctacacg tcaatcaaag aaatttttga tgccactatc atttaccaat 5100
cgactacggg actgtatgaa acacgcagaa aggttgtcga ctagatgggc tggcgaacgg 5160
tagtggttaa caagcattca aaattatcct ataaaaacaa tcatcttgtt tttaaagcaa 5220
ttgatcacca agaattaatt catttgtctg agattgatgt tttgttgttg gaaacaacgg 5280
acatttcgtt aaccacgatg ctattaaaac gattaattga tgaaaaaata ttagtgttat 5340
tttgcgatga taagcgtttg ccaattggta aaattttgcc cttttatgga cggcatgata 5400
gtagcttgca attgacacgc cagttggctt ggacggaaga acgaaaaggt caagtttgga 5460
cagcgatcat tgcacaaaaa atcaccaatc agagtttaca cttagcgcaa cgagactata 5520
gccaaaaggc tgctgctttg ttagctatgc gcgctgaatt acgtctgttt gatccagcca 5580
atcgagaagg tcatgcagct cgaagttact ttaatactct cttcggcaat gattttacac 5640
gtgaacaaga aaatgacatt aatgctggtt taaactatgg ttatacgttg ttattaagta 5700
tttttgctcg agagttagtc cagacaggtt gttttacaca actaggacta aaacatgcca 5760
accaatttaa tgactttaac ttagcaagtg atcttatgga gccttttcgt ccattggtag 5820
atcaaatcat ttatgagaat agaaaagaag catttccgat aatgaaacgt aagttatttg 5880
ctttgtttat gaatacgtat atgtacaaga aaaaacaaat gtttttaacc aatattgcta 5940
ccgattatac aaaacatgta gtaaaagttc tgaatcaaga ggaggaagga gttccagagt 6000
ttgggatatg agttatcgat atatgcgtct attattaatg tttgatatgc caacggacac 6060
agccagtgac cggaaagcgt atcggaagtt ccggaaattt ttgattaatg aaggatttat 6120
tatgcatcaa ttttcggttt acagcaaaat tttgttgaat gatactgcta ataaagcaat 6180
gttggctcgc ttaaagcaga ataatccgca aagaggacta attacactat taaatgtaac 6240
agaaaaacaa ttttctcgaa tgatttattt gcatggagaa caagacaatc gagttgctaa 6300
ttcagatgaa cggattgtct ttttaggaga agaatagatg agagttaatt tttctttatt 6360

ggaagaaccc attgaaattg aaaaagccac ctttttaacg attaaagacg ttcaaacctt 6420
tgcccatcta gtgaagttaa tttatcaata tgatggggaa aatgagttaa aactatttga 6480
tgcgcaacaa aaagggctga aaccaacgga attatttgtt gttacggata ttttaggcta 6540
tgacgttaat tcggctgcca cgctgaaatt aatctatggg gatttagaag cacaattaaa 6600
tgataaacca gaagtcaaat caatgattga gaagttaact ggaacaatta gtcaattaat 6660
tggctatgaa ttgttggaac atgaaatgga tttggaagaa gatggcatca ctgtgcagga 6720
acttttcaaa gctcttggaa tcaaaatcga aacaacgagt gatacgattt ttgaaaaagt 6780
tatggaaatt acacaagtac atcgttattt atcaaagaaa aaattattga tttttattaa 6840
tgcgtgtacg tatttgacag aggatgaagt gcaacaagtg gtagaatata tctctttaaa 6900
taatgtggat gtcctgtttt tagaacaaag ggtggtccag aacagattcc aatatatttt 6960
ggacgaaaac ttttatttga gttatgaaaa agcttaaatt gttactgatt agtggttcat 7020
tctaaactga aatctagcta tggataagtg atgcgagtac ggaactttgg aaaaaaataa 7080
ttctccgagg ttttagagtc atgttgttta gaatggtacc aaaacaagtg caactagcta 7140
gaattgaatc aaactgtttt agagtcatgt tgtttagaat ggtaccaaaa cccacctgtt 7200
gcatatatag cattcacaag tgttttagag tcatgttgtt tagaatggta ccaaaacatt 7260
taagattaga tatgaacaac ttacgttgtt ttagagtcat gttgtttaga atggtaccaa 7320
aactcaatcc attcagcaac tatattgtgt ttagttttag agtcatgttg tttagaatgg 7380
taccaaaaca tgtactcatt ctaatgtata agaagtgcag ttttagagtc atgttgttta 7440
gaatggtacc aaaacgtgaa cttttttctg ttggtgcgtt gtttcgtttt agagtcatgt 7500
tgtttagaat ggtaccaaaa ctaaacagtc taccatgtaa ctagtgaatg cgttttagag 7560
tcatgttgtt tagaatggta ccaaaactta tagaagtaca gtcaccatca gcgacctgtt 7620
ttagagtcat gttgtttaga atggtaccaa aacagggctg caggagttaa tgttacaatc 7680
tcagttttag agtcatgttg tttagaatgg taccaaaaca tcatctcatc gactaaatat 7740
gctttgtatg ttttagagtc atgttgttta gaatggtacc aaaacccact ttattaattg 7800
cttgtcaaca cgtctgtttt agagtcatgt tgtttagaat ggtaccaaaa cgtatctacg 7860
tgcataatca tatcgactca tgttttagag tcatgttgtt tagaatggta ccaaaaccaa 7920
tgtaaatgct cattatgatt tacatatgtt ttagagtcat gttgtttagt tttcgcagat 7980
acgatttgat tgatgtaaaa atatcgttaa tatgtataaa tatggttatt atataaaaaa 8040
tataagtaat aaattgaagc tttgctaaag caagtgatgc gattacgaaa ttatttaatt 8100
ttagagtcac gttatttatt actttacgaa tagagaatac gattatctat aaatcaagaa 8160
ttaatcccca atttattgaa aatgacactc cctgttttat taatatttca tgcttttgtt 8220
g 8221

<210> 7
<211> 8221
<212> DNA
<213> Bacillus cereus

<220>
<221> misc_feature
<222> (7114)..(7123)
<223> n is a, c, g, or t

<220>
<221> misc_feature
<222> (7176)..(7289)
<223> n is a, c, g, or t

<400> 7
agtttacgaa gaccatacag gcgaaaaagg ccaagaacaa atcattggtg gcggtactta 60
tggccgctta ttaaaacgtg gtgttgccta tggcgcaatg ttccctggct acacagacac 120
aatgcaccaa gccaacgaat ttatggaatt agatgactta ttccgagcag ctgctattta 180
tgcagatgcg atttatcgtt tagcgaagta aagtaaaaaa gctgttttcg attttcgaaa 240
acagtttttt tttgcaattt ttgcctttta aaaacccaaa atcagaaccg tcaatttact 300
gaaaatttct tgcaaatcaa aggatcccac gatacgataa ataggagaaa acgcaatgag 360
tgaaacactt attgttaact atttaatcaa aggacgtgtg aagatgaacg tagaagaatt 420
agttcaaaaa gcaaaagatt taattggaga aggcaacgtg gaacatgcgc aacaattcat 480
tgaagaacat aaagatgaat taggcgaaca ctatcataaa attacagcgc tgttagctga 540
tacaacgacc gatggtttgt ttgataaaat taaaggatta tttcaatagt cgtaattaaa 600
aaggcaaaac acagttaacc acgcactgtg ttttgccttt tttgccatgt aaagtttgac 660
aacatcaaca aatagtaaaa gatattacca tacaataaat atagattcgt tattttaagg 720
aaagtaaaga aattaaggta agagattctt aaatagataa tgttattttt gttacatata 780
taactatatt agttaaaaaa actataaata accctttttt taagaaaata agaatggtaa 840

actttaagta gtgttattca gtttgattga tttggaagcg attactttta aatttcaaac 900
aaaaaaagcg ccgaaacagc gccactttac agttgattac ggttaaaacc ttattttaac 960
ttgctatgtt gtttagaata gtcccaaaag aacttaacta aattatagca tagaataaaa 1020
atcattcaag tagtaattta aaaaaaggag tggaaataaa atgaagaaag attacgttat 1080
tggtctggat ataggaacta attctgtcgg ctgggcagtt atgacagaag actatcagtt 1140
ggtgaagaaa aaaatgccta tttatggaaa cactgaaaaa aagaaaatta agaaaaattt 1200
ttggggtgtg cgtttatttg aagaagggca tacagccgaa gatcgccgat taaaaagaac 1260
agcacgacga agaattagtc gtcgacggaa tcgtttacgc tacttacaag ctttttttga 1320
agaagcgatg acagatttgg atgaaaactt ttttgctcgt ttacatgaga gttttttagt 1380
gcctgaagat aagaagtggc acagacatcc gatttttgct aagttggaag atgaagtagc 1440
ttaccatgaa acgtatccga caatctacca tttacgcaaa aaattagcag attcatctga 1500
gcaagcagat ttacgactaa tttatttggc gttggcccat attgtcaaat atcgtggaca 1560
ttttttaatt gaaggaaaac taagtacaga aaatacttct gttaaagaac aatttcaaca 1620
atttatggtc atttataacc aaacctttgt gaatggggag agtcgcttag ttagtgcgcc 1680
tttacctgaa tctgtcttga ttgaggaaga gttaactgaa aaagcttctc ggactaaaaa 1740
atctgaaaaa gtcttacaac aatttcctca agaaaaagct aatggcttat ttggtcagtt 1800
tttaaagcta atggtaggga ataaagctga ctttaaaaaa gtttttgctt tggaagaaga 1860
agccaaaata acctacgcta gtgaaagtta tgaagaagat ttagaaggca ttttagcaaa 1920
gattggggat gaatatagtg acgtgttttt agctgctaaa aatgtgtacg atgcagttga 1980
attatcaacg attttagcag attcggataa aaaaagtcac gcgaaattat cttctagcat 2040
gattgtccgt tttacagaac atcaagaaga tttaaaaaaa ttcaaacggt ttattcgtga 2100
aaattgtcca gacgaatatg ataatctctt taaaaatgaa cagaaagacg gctatgcagg 2160
ttacattgca cacgcaggta aggtttcaca gcttaaattt taccagtatg ttaagaaaat 2220
cattcaagat attgctggag cagaatattt tttagaaaaa attgctcaag aaaacttttt 2280
aagaaagcaa cggacctttg ataatggggt gattcctcat caaattcatt tggctgagtt 2340
acaagcaatc attcatcgtc aagcggccta ttatccattt ttaaaagaaa atcaagaaaa 2400
aattgaacaa ctggttacat ttcgaattcc ttattatgtc ggaccattat caaaaggaga 2460
tgcaagtacc ttcgcttggt taaaacgtca aaatgagaaa ccaattcgac cttggaacct 2520
tcaagaaacc gttgatttgg accaatcagc gacagccttt attgaacgaa tgaccaattt 2580
tgatacgtat ttaccttctg aaaaagtttt accgaaacat agtttgttat atgaaaagtt 2640
tatggtattt aatgaattga ccaagatttc ttatacggat gatcgaggaa tcaaagccaa 2700
tttttcaggt aaggaaaaag aaaaaatctt tgattatctg tttaagacgc gtcgaaaagt 2760
taagaaaaag gatataattc aattctatcg aaacgaatat aatacggaga ttgtcacgct 2820
tagtggactt gaagaagacc aatttaatgc tagttttagc acgtatcagg atttactgaa 2880
atgtggtttg acaagagcag aattagacca cccagataat gccgaaaaat tggaagatat 2940
cataaaaatt ttaactattt ttgaagatcg gcaacgaatt cggacgcaac tttccacatt 3000
caaagggcag ttctcagcag aagtactaaa aaaattagaa cgcaagcact acactggctg 3060
gggaagattg tcgaagaaat taatcaatgg tatctacgat aaagaatcgg gtaaaacaat 3120
tttggactat ttagttaaag atgatggggt ctcaaaacac tataatcgca attttatgca 3180
actgattaat gattcacaat tatcttttaa aaatgcgatt caaaaagcac agtccagtga 3240
acatgaagaa acattaagtg aaactgtcaa tgaattagct ggtagcccag caataaaaaa 3300
aggaatttat caaagtttaa aaattgtcga tgaactagtc gcgattatgg gttatgcgcc 3360
taagcggatt gttgtcgaaa tggcacgtga aaatcaaacg actagcactg gcaagagaag 3420
atccatccaa cgcttgaaaa tagttgaaaa agcaatggcc gaaatcggca gcaatttatt 3480
aaaagaacaa ccaacgacta atgaacagtt acgagatacc cgtcttttcc tttactatat 3540
gcaaaatggt aaagatatgt acacgggaga tgaattatcg cttcatcgtt tatctcacta 3600
tgatattgat catattatcc cacaaagttt tatgaaagac gattcattgg ataacctagt 3660
tttagtgggc tctactgaaa atcgagggaa atccgatgat gtacctagca aggaagttgt 3720
taaggatatg aaagcttatt gggagaaatt atatgctgct ggcttaatta gtcaacggaa 3780
attccaacgt ctgaccaagg gggagcaagg tggcttgact ctcgaagaca aagcgcattt 3840
tatccaacgg caattagtag aaactcgtca aattaccaaa aatgttgcag ggatcttaga 3900
tcaacgctac aatgccaagt caaaagagaa aaaagtccaa atcatcacct tgaaagctag 3960
tttaacgagc caatttcgtt cgatttttgg cttgtataaa gtccgtgaag tgaatgatta 4020
tcaccatgga caagatgctt atttaaactg tgtggtcgca accacgttat taaaagtttg 4080
tcctaattta gcaccagaat ttgtttacgg agaatatcct aagttccaga cctttaaaga 4140
aaataaagcc acggcgaagg ctataattta tacaaatttg ctgcgctttt tcacggaaga 4200
tgaaccacgg ttcacgaagg atggcgaaat tctttggagt aacagttatt taaaaactat 4260
caaaaaagaa ttaaattacc atcaaatgaa tattgtcaaa aaagtagaag tgcaaaaagg 4320
cggtttttca aaagagtcga ttaaacccaa aggaccatcg aataaattga ttcctgtcaa 4380
aaatggtttg gatccccaaa aatatggcgg ttttgatagt cctgtagttg cttatacagt 4440
gctattcacg catgaaaaag gtaaaaaacc tcttattaaa caggagatac tgggcattac 4500
gattatggaa aaaacaaggt ttgaacaaaa tcctattctt tttttagagg agaaaggctt 4560
cctgcggcct cgtgtattaa tgaaattgcc taagtataca ctgtatgaat ttccagaggg 4620

gcgccggcgc ttattagcta gtgctaaaga agcgcaaaaa gggaaccaga tggttttacc 4680
cgaacattta cttacattac tgtaccatgc gaaacaatgt ttgttaccaa atcaatcaga 4740
aagtttggct tatgtagaac aacatcaacc agaatttcaa gagattttag agagagtggt 4800
tgactttgct gaagtgcata cattagccaa gtcaaaagtc cagcaaattg tgaaactttt 4860
tgaagcaaat caaacagcag atgtgaaaga gattgcggca tcctttatcc aacttatgca 4920
gtttaatgcg atgggcgcac caagtacgtt taaatttttc caaaaagata ttgagcgagc 4980
acgctacacg tcaatcaaag aaatttttga tgccactatc atttaccaat cgactacggg 5040
cctgtatgaa acacgcagaa aggttgtcga ctagatgggc tggcgaacgg tagtggttaa 5100
taagcattca aaattatcct ataaaaacaa tcatcttgtt tttaaagcca ttgatcacca 5160
agaattaatt catttgtctg agattgatgt tttgttgttg gaaacaacgg acatttcgtt 5220
aaccacgatg ctattaaaac gattaattga tgaaaaaata ttagtgttat tttgcgatga 5280
taagcgtttg ccaattggta aaattttgcc cttttatgga cggcatgata gtagcttgca 5340
attgacacgt cagttggctt ggacggaaga acgaaaaggt caagtttgga cagcgatcat 5400
tgcacaaaaa atcaccaatc agagtttaca cttagcgcaa cgagactatg gccaaaaggc 5460
tgctgctttg ttagctatgc gcgctgaatt acgtctgttt gatccagcca atcgagaagg 5520
tcatgcagct cgaagttatt ttaatactct cttcggcaat gattttacac gtgaacaaga 5580
aaatgacatt aatgctggtt taaactatgg ttatacgttg ttattaagta tttttgctcg 5640
agagttagtc cagacaggtt gttttacaca actaggacta aaacatgcca accaatttaa 5700
tgactttaac ttagcaagtg atcttatgga gccttttcgt ccattggtag atcaaatcat 5760
ttatgagaat agaaaagaag cctttccgat aatgaaacgt aagttatttg ctttgtttat 5820
gaatacgtat atgtacaaga aaaaacaaat gtttttaacc aatattgcta ccgattatac 5880
aaaacatgta gtaaaagttc tgaatcaaga ggaggaagga gttccagagt ttgggatatg 5940
agttatcgat atatgcgtct attattaatg tttgatatgc caacggacac agccagtgac 6000
cggaaagcgt atcggaagtt ccggaaattt ttgattaatg aaggatttat tatgcatcaa 6060
ttttcggttt acagcaaaat tttgttgaat gatactgcta ataaagcaat gttggctcgc 6120
ttaaagcaga ataatccgca aagaggacta attacactat taaatgtaac agaaaaacaa 6180
ttttctcgaa tgatttattt acatggagaa caagacaatc gagttgctaa ttcagatgaa 6240
cggattgtct ttttaggaga agaatagatg agagttaatt tttctttatt ggaagaaccc 6300
attgaaattg aaaaagccac ctttttaacg attaaagacg ttcaaacctt tgcccatcta 6360
gtgaagttaa tttatcaata tgatggggaa aatgagttaa aactatttga tacgcaacaa 6420
aaagggctga aaccaacgga attatttgtt gttacggata ttttaggcta tgatgttaat 6480
tcggctgcca cgctgaaatt aatctatggg gatttagaag cacaattaaa tgataaacca 6540
gaagtcaaat caatgattga gaagttaact ggaacaatta gtcaattaat tggctatgaa 6600
ttgttggaac atgaaatgga tttggaagaa gatggcatca ctgtgcagga acttttcaaa 6660
gctcttggaa tcaaaatcga aacaacgagt gatacgattt ttgaaaaagt tatggaaatt 6720
acacaagtac atcgttattt atcaaagaaa aaattattga tttttattaa tgcgtgtacg 6780
tatttgacag aggatgaagt gcaacaagtg gtagaatata tctctttaaa taatgtggat 6840
gtcctgtttt tagaacaaag ggtggtccag aacagattcc aatatatttt ggacgaaaac 6900
ttttatttga gttatgaaaa agcttaaatt gttattgatt agtggttcgt tctaaactga 6960
aatctagcta tggataagtg atgcgagtac ggaactttgg agaaaaaata attctccgag 7020
gttttagagt catgttgttt agaatggtac caaaactgcc ctctgcttta ttatgtgtgt 7080
gagagggttt tagagtcatg ttgtttagaa tggnnnnnnn nnntgtttgt ttgttggaat 7140
atagtcttgg tggttttaga gtcatgttgt ttagannnnn nnnnnnnnnn nnnnnnnnnn 7200
nnnnnnnnnn nnnnnnnnnn nnnnnnnnnn nnnnnnnnnn nnnnnnnnnn nnnnnnnnnn 7260
nnnnnnnnnn nnnnnnnnnn nnnnnnnnnt aacaaagaaa aagcatgatt tattgattgt 7320
tttagagtca tgttgtttag aatggtacca aaacaaacaa aatatataac gaagattgct 7380
tagagtttta gagtcatgtt gtttagaatg gtaccaaaac ggatcccaca cctcaacccc 7440
aaatggtgtt gttttagagt catgttgttt agaatggtac caaaacttga gactgcgtat 7500
aattgtgagt atggtggttt tagagtcatg ttgtttagaa tggtaccaaa actgaaactt 7560
ccaaggcatc cctttactag ttgttttaga gtcatgttgt ttagaatggt accaaaactt 7620
gtttcacctc gtttctgtta ttaagtgtgt tttagagtca tgttgtttag aatggtacca 7680
aaacttgaga ctgcgtataa ttgtgagtat ggtggtttta gagtcatgtt gtttagaatg 7740
gtaccaaaac agaagttcct tacaacgtat caacttaccg gttttagagt catgttgttt 7800
agaatggtac caaaacactc tatatcgtgc attcttttcc aatgttgttt tagagtcatg 7860
ttgtttagtt ttcgcagata cgatttgatt gatgtaaaaa tatcgttaat atgtataaat 7920
gtgattgtta tataaaaaat ataagtaata aattgaagct ttgctagagc aagtgatgcg 7980
attacgaaat tatttaattt tagagtcacg ttatttatta ctttgcgaat agagaatacg 8040
attatctata aatcaagaac taatccccaa tttattgaaa atgacactcc ctgttttatt 8100
aatatttcat gcttttgttg tactatttga tgatacaaaa aagtaaaact gaggacccaa 8160
ttaaagtcaa gttaaccaca ttgaaagata aacctttcaa tgtggttaaa gatgcttaat 8220
g 8221

<210> 8

<211>  8091
<212>  DNA
<213>  Enterococcus faecalis

<400>  8
ggcgcaatgt tccctggcta cacagacaca atgcaccaag ccaacgaatt tatggaatta      60
gatgacttat tccgagcagc tgctatttat gcagatgcga tttatcgttt agcgaagtaa     120
agtaaaaaag ctgttttcga ttttcgaaaa cagctttttt tttgcaattt ttgcctttta     180
aaaacccaaa atcagaactg tcaatttact gaaaatttct tgcaaatcaa gggatcccac     240
gatacgataa ataggagaaa acgcaatgag tgaaacactt attgttaact atttaatcaa     300
aggacgtgtg aagatgaacg tagaagaatt agttcaaaaa gcaaaagatt taattggaga     360
aggcaacgtg gaacatgcgc aacaattcat tgaagaacat aaagatgaat taggcgaaca     420
ctatcataaa attacagcgc tgttagctga tacaacgacc gatggtttgt ttgataaaat     480
taaaggattg tttcaataat cgtaattaaa aaggcaaaac acagttaacc acgcactgtg     540
ttttgccttt tttgccatgt aaagtttgac aacatcaaca aatagtaaaa gatattacca     600
tacaataaat atagattcgt tattttaagg aaagtaaaga aattaaggta agagattctt     660
aaatagataa tgttattttt gttatgtata taattctatt agtttacaaa aactataaat     720
accccttttt ttaagaaaat aagaatggta aactttaagt agcggtattc agtttgatca     780
atttggaagc gattactttc tgattgtaaa caaaaaaagc gccgaaacag cgccacttta     840
cagttgatta cggttgaaac cttattttaa cttgctatgt tgtttagaat agtcccaaaa     900
gaacttaact aaattatagc atagaataaa aatcattcaa gtagtaattt aaaaaaagga     960
gtggaaataa aatgaagaaa gactacgtta tcggtttgga tatagggact aattctgtcg    1020
gttgggcagt tatgacagaa gactatcagt tggtgaagaa aaaaatgcct atttatggaa    1080
acactgaaaa aaagaaaatc aagaaaaatt tttggggtgt gcgtttattt gaagaagggc    1140
atacagctga agatcgccga ttaaaaagaa cagcacgacg aagaattagt cgtcgacgga    1200
atcgtttacg ctacttacaa gctttttttg aagaagcgat gacagatttg gatgaaaact    1260
tttttgctcg tttacaagag agttttttag tgcctgaaga taagaagtgg cacagacatc    1320
cgatttttgc taagttggaa gatgaagtag cttaccatga aacgtatccg acaatctacc    1380
atttacgcaa aaaattagca gattcatctg agcaagcaga tttacgacta atttatttgg    1440
cgttggccca tattgtcaaa tatcgtggac attttttaat tgaaggaaaa ctaagtacag    1500
aaaatacttc tgttaaagaa caatttcaac aatttatggt catttataac caaacctttg    1560
tgaatggaga gagtcgctta gttagtgcgc cattacctga atctgtcttg attgaggaag    1620
agttaactga aaaagcttct cggactaaaa aatctgaaaa agtcttacaa caatttcctc    1680
aagaaaaagc taatggctta tttggtcagt tcttaaagct aatggtaggg aataaagctg    1740
actttaaaaa agtttttggt ttggaagaag aagccaaaat aacctacgct agtgaaagct    1800
atgaagaaga tttagaaggc attttagcaa aggttgggga tgagtatagt gacgtgtttt    1860
tagctgctaa aaatgtgtac gatgcagttg aattatcaac gattttagca gattcggata    1920
aaaaaagtca cgcgaaatta tcttctagca tgattgtccg ttttacagaa caccaagaag    1980
atttaaaaaa attcaaacga tttattcgtg aaaattgtcc agacgaatat gataatctct    2040
ttaaaaatga acagaaagac ggttatgcag gctacattgc gcacgcaggt aaggtttcac    2100
agcttaaatt ttaccagtat gttaagaaaa tcattcaaga tattgctgga gcagaatatt    2160
ttttagaaaa aattgctcaa gaaaactttt taagaaagca acggaccttt gataatgggg    2220
tgattcctca tcaaattcat ttggctgagt tacaagcaat cattcatcgt caagcggcct    2280
attatccatt tttaaaagaa aatcaagaaa aaattgaaca actggttaca tttcgaattc    2340
cttattatgt cggaccatta tcaaaaggag atgcaagtac cttcgcttgg ttaaaacgtc    2400
aaagtgagga accaattcga ccttggaacc ttcaagaaac cgttgatttg gaccaatcag    2460
cgacagcctt tattgaacga atgaccaatt ttgatacgta tttaccttct gaaaaagttt    2520
taccgaaaca tagtttgtta tatgaaaagt ttatggtatt taatgaattg accaagattt    2580
cttatacgga tgatcgagga atcaaagcca atttttcagg taaggaaaaa gaaaaaatct    2640
ttgattatct gtttaagacg cgtcgaaaag ttaagaaaaa ggatataatt caattctatc    2700
gaaacgaata taatacggag attgtcacgc ttagtggact tgaagaagac caatttaatg    2760
ctagttttag cacgtatcag gatttactga aatgtggttt gacaagagca gaattagacc    2820
acccagataa tgccgaaaaa ttggaagata tcataaaaat tttaactatt tttgaagatc    2880
ggcaacgaat tcggacgcaa ctttccacat tcaaaggcca gttctcagca gaagtactaa    2940
aaaaattaga acgcaagcac tacactggct ggggaagatt gtcgaagaaa ttaatcaatg    3000
gtatctacga taaagaatcg ggtaaaacaa ttttggacta tttagttaaa gatgatgggg    3060
tctcaaaaca ctataatcgc aattttatgc aactgattaa tgattcacaa ttatctttta    3120
aaaatgctat tcaaaaagca cagtccagtg aacatgaaga aacattaagt gaaactgtca    3180
atgaattagc tggtagccca gcaataaaaa aaggaattta tcaaagttta aaaattgtcg    3240
atgaactagt cgcgattatg ggttatgcgc ctaagcggat tgttgtcgaa atggcacgtg    3300
aaaatcaaac gactagcact ggcaaaagaa gatccatcca acgcttgaaa atagttgaaa    3360
aagcaatggc cgaaatcggc agcaatttat taaaagaaca accaacgacc aatgaacagt    3420
tacgagatac ccgtcttttc ctttactata tgcaaaatgg taaagatatg tacacgggag    3480

atgaattatc gcttcatcgt ttatctcact atgatattga tcatattatc ccacaaagtt 3540
ttatgaaaga cgattcattg gataacctag ttttagtggg ctctactgaa aatcgaggga 3600
aatctgatga cgtcccaagt aagaaagttg ttaagaagat gaaagcttat tgggagaaat 3660
tatatgctgc tggcttaatt agtcaacgga aattccaacg tctgaccaag ggggagcaag 3720
gtggcttgac tctcgaagac aaagcgcatt ttatccaacg gcaattagta gaaactcgtc 3780
aaattaccaa aaatgttgca gggatcttag atcaacgcta caatgccaag tcaaaagaga 3840
aaaaagtcca aatcatcacc ttgaaagcta gtttaacgag ccaatttcgt tcgatttttg 3900
gcttgtataa agtccgtgaa gtgaatgatt atcaccatgg acaagatgct tatttaaact 3960
gtgtggtcgc aaccacgtta ttaaaagttt accctaattt agcaccagaa tttgtttacg 4020
gagaatatcc taaattccag gcctttaaag aaaataaagc cacggcgaag actataattt 4080
atacaaattt gatgcgcttt ttcacggaag atgaaccacg gttcacgaag gatagcgaaa 4140
ttctttggag taacagttat ttgaaaacta tcaaaaaaga attaaattac catcaaatga 4200
atattgtcaa aaaagtagaa gtgcaaaaag gcggtttttc aaaagagtcg attaaaccca 4260
aaggaccatc gaataaattg attcctgtca aaaatggttt ggatccccaa aaatatggcg 4320
gttttgatag tcctgtagtt gcttatacag tgctattcac gcatgaaaaa ggtaaaaaac 4380
ctcttattaa acaggagata ctgggcatta cgattatgga aaaaacaagg tttgaacaaa 4440
atcctattct ttttttagag gagaaaggct tcctgcggcc tcgtgtatta atgaaattgc 4500
ctaagtatac actgtatgaa tttccagagg ggcgccggcg cttattagct agtgctaaag 4560
aagcgcaaaa agggaaccag atggttttac ctgaacattt acttacatta ctgtaccatg 4620
cgaaacaatg tttattacca aatcaatcag aaagtttggc ttatgtagaa caacatcaac 4680
cagaatttca agagatttta gagagagtgg ttgactttgc tgaagtgcat acattagcca 4740
agtcaaaagt ccagcaaatt gtgaaacttt ttgaagcaaa tcaaacagca gatgtgaaag 4800
agattgcggc atcctttatc caacttatgc agtttaatgc gatgggcgca ccaagtacgt 4860
ttaaattttt ccaaaaagat attgagcgag cacgctacac gtcaatcaaa gaaatttttg 4920
atgccactat catttaccaa tcgactacgg gcctgtatga aacacgcaga aaggttgtcg 4980
actagatggg ctggcgaacg gtagtggtta ataagcattc aaaattatcc tataaaaaca 5040
atcatcttgt ttttaaagcc attgatcacc aagaattaat tcatttgtct gagattgatg 5100
ttttgttgtt ggaaacaacg gacatttcgt taaccacgat gctattaaaa cgattaattg 5160
atgaaaaaat attagtgtta ttttgcgatg ataagcgttt gccaattggt aaaattttgc 5220
ccttttatgg acggcatgat agtagcttgc aattgacacg tcagttggct tggacggaag 5280
aacgaaaagg tcaagtttgg acagcgatca ttgcacaaaa aatcaccaat cagagtttac 5340
acttagcgca acgagactat ggccaaaagg ctgctgcttt gttagctatg cgcgctgaat 5400
tacgtctgtt tgatccagcc aatcgagaag gtcatgcagc tcgaagttat tttaatactc 5460
tctttggcaa tgattttaca cgtgaacaag aaaatgacat taatgctggt ttaaattatg 5520
gttatacgtt gttattaagt atttttgctc gagagttagt ccagacaggt tgttttacac 5580
aactaggact aaaacatgcc aaccaattta atgactttaa cttagcaagt gatcttatgg 5640
agccttttcg tccattggta gatcaaatca tttatgagaa tagaaaagaa gcatttccga 5700
taatgaaacg taagttattt gctttgttta tgaatacgta tatgtacaag aaaaaacaaa 5760
tgtttttaac caatattgct accgattata caaaacatgt agtaaaagtt ctgaatcaag 5820
aggaggaagg agttccagag tttgggatat gagttatcga tatatgcgtc tattattaat 5880
gtttgatatg ccaacggaca cagccagtga ccggaaagcg tatcggaagt tccggaaatt 5940
tttgattaat gaaggattta ttatgcatca attttcggtt tacagcaaaa ttttgttgaa 6000
tgatactgct aataaagcaa tgttggctcg cttaaagcag aataatccgc aaagaggact 6060
aattacacta ttaaatgtaa cagaaaaaca attttctcga atgatttatt tgcatggaga 6120
acaagacaat cgagttgcta attcagatga acggattgtc tttttaggag aagaatagat 6180
gagagttaat ttttctttat tggaagaacc cattgaaatt gaaaaagcca cctttttaac 6240
gattaaagac gttcaaacct ttgcccatct agtgaagtta atttatcaat atgatgggga 6300
aaatgagttg aaactatttg atgcgcaaca aaaagggctg aaaccaacag aattatttgt 6360
tgttacggat attttaggct atgacgttaa ttcggctgcc acgctgaaat taatctatgg 6420
ggatttagaa gcacaattaa atgataaacc agaagtcaaa tcaatgattg agaagttaac 6480
tggaacaatt agtcaattaa ttggctatga attgttggaa catgaaatgg atttggaaga 6540
agatggcatc actgtgcagg aacttttcaa agctcttgga atcaaaatcg aaacaacgag 6600
tgatacgatt tttgaaaaag ttatggaaat tacacaagta catcgttatt tatcaaagaa 6660
aaaattattg atttttatta atgcgtgtac gtatttgaca gaggatgaag tgcaacaagt 6720
ggtagaatat atctctttaa ataatgtgga tgtcctgttt ttagaacaaa gagtggtcca 6780
gaacagattc caatatattt tggacgaaaa cttttatttg agttatgaaa aagcttaaat 6840
tgttattgat tagtggttca ttctaaactg aaatctagct atggataagt gatgcgagta 6900
cggaactttg gagaaaaaat aattctccga ggttttagag tcatgttgtt tagaatggta 6960
ccaaaactaa gcagcttatg ccacttactg gcgtgacgtt ttagagtcat gttgtttaga 7020
atggtaccaa aacttgtgtt tcggtttcag gaacttcggc atcgttttag agtcatgttg 7080
tttagaatgg taccaaaaca cactgctaaa cgacttggca aagagttatg ttttagagtc 7140
atgttgttta gaatggtacc aaaaccgtac tcacaaacaa cgttgcgaca tattagtttt 7200
agagtcatgt tgtttagaat ggtaccaaaa ctttgctgat aaaaatatta taacagaaat 7260

ggttttagag tcatgttgtt tagaatggca ccaaaaccta tagaatggta gtaggtaccg 7320
acgctaagtt ttagagtcat gttgtttaga atggtaccaa aacttaaacg ttctattccc 7380
attcgttccg actgttttag agtcatgttg tttagttttc gcagatacga tttgattgat 7440
gtaaaaatat cgttaatatg tataaatgtg attgttatat aaaaaatata agtaataaat 7500
tgaagctttg ctagagcaag tgacgcgatt acgaaattat ttaattttag agtcacgtta 7560
tttattactt tacgaataga gaatacgatt atctataaat caagaactaa tccccaattt 7620
attgaaaatg acactccctg ttttattaat atttcatgct tttgttgtac tatttgatga 7680
tacaaaaaag taaaactgag gacccaatta aagtcaagtt aaccacattg aaagataaac 7740
ctttcaatgt ggttaaagat gcttaatgag attcatgaaa catagagagc ggattaattt 7800
cctatgttct ttatttgtat attttcttgg gaatcattat tgattgtgga ttaaaaagat 7860
aatccgctcc ccgtttcacc acaggcatat tcgtaatagc taaaatgaag agggctaaga 7920
agaaaagtgc caagattcgg aaagaaaacc attctttaaa gagtgtgtct aattgtaaac 7980
cactttccaa cagaataatt aagggcatat gcaagagata agggtagagg gtatcttttc 8040
cgagggcgct taaaggtaac tttcgtttcg gcaataatgc aaaccaagaa a 8091

<210> 9
<211> 7816
<212> DNA
<213> Artificial Sequence

<220>
<223> CRISPR-Cas locus 14

<400> 9
cataaaaaaa cctccttttg gtttgtgaac tgtttctact tcaaagcata aagcgtttag 60
aaaaataagc caaatgatag ggtaaggaag caacctggat ttttgacagt ttagaggaag 120
tgtctgtttt tcacaataaa gtgtaagaaa atgttaaatg aatcaaagaa gaattagtga 180
cgatcactga ggcaaaaaag gggaaagaga taatggctga aaaaaataga accaacattt 240
gctgatttct tgaaaaaact tcgtagtttc tggtttagag agaaagaata tgtttcatcg 300
actaatagat acaagtaatt gaatctcatt tttgttattt aaaaaaaggt ggtttgatta 360
tttaaaatat tcttccagtg gataatcaac taggatcagt cattttggtt ttttttatta 420
ttatgatgat tttgagaaaa gagtgtgata ggatgaatga aaagggtatc atagttgttt 480
ttgtatagtt tttagacgga tgaagtcaaa aaaagcgccg agacagcgct tgctcctaga 540
agctgatgac ggacaaagcc ttattttaac ttgctatatt gaattgaatg ctaccaatgg 600
ttttattata gcatagattg tcagacttac aacaatattt ttgattttgt ttttttatca 660
tgttgagagg tgaggattta agtatggaaa aagagtatac gattggatta gatattggga 720
caaattcggt tggatgggca gtgttgacgg atgattatcg attagtcgca agaaaaatga 780
gtattcaagg agactcgaat agaaagaaaa taaaaaagaa tttttggggt gcacgtttat 840
tcgaagaagg taaaactgca caatttcgac ggatcaaacg aacgaaccgt cgtaggatcg 900
cacgaagacg tcaaagagtt ttagcattac aagatatttt tgcagaagag atacataaaa 960
aagatcccaa tttttttgct cggctagaag aaggagatcg tgtggaagca gacaaacgat 1020
ttgctaagtt tcctgttttt gctacattgt cggaggaaaa aaattatcat cgtcaatatc 1080
cgaccatcta ccatttgagg catgatctag caaactcaaa agaacaagca gatattcgtt 1140
tagtgtactt agcgattgcc cattgcttga agtacagagg gcactttttg tttgaaggag 1200
aattagatac tgaaaatacc tcggtaaccg aaaattatca gcaattttta caagcatacc 1260
aacagttctt tccagaacct ataggagatt tggatgatgc tgtccctatt ttgacagagc 1320
ggctatctaa ggcaaaacga gtggaaaaag tattagctta ttatccatct gaaaaaagta 1380
ctggaaattt tgctcaattc cttaaactaa tggtcggaaa tcaagcaaat tttaaaaaga 1440
cgtttgattt agaagaagaa atgaagttga attttactcg tgattgttat gaagaggatt 1500
taaatgaact gctagaaaaa acaagtgacg attatgcaga attattttta aaagcgaagg 1560
gtgtgtatga tgcgatttta ctttcgcaaa tcctttctaa gtcagatgat gaaacgaaag 1620
ctaagttgtc tgcgaatatg aagttgcgct ttgaagagca ccaacgagac cttaaacaat 1680
taaaagaact agtccgtaga gacttaccta aaaagtacga tgatttcttt aagaatcgca 1740
gtaagaatgg atacgctggg tatgtcaagg ggaaagcgac acaagaagat ttttataaat 1800
ttctaagaac agaactagct ggattagagg aaagccaatc aatcatggaa aaaattgatc 1860
tagagatcta tctattgaaa caacgtacct ttgcaaatgg ggtgattcct catcaaatcc 1920
atttagttga aatgagagaa attatggatc ggcagaaacg tttttatcct tttctaaaag 1980
gggcacaagg aaaaatcgag aaactactga cgtttcgaat cccatattac gtaggaccgt 2040
tggcacaaga aggtcaaagt cctttcgctt ggatcaaacg taagagtccg agccaaatca 2100
ctccatggaa ttttgcagaa gtggttgata aagaaaattc agccattgaa tttattgagc 2160
gaatgacaaa tcaagatacg tatttaccaa aggaaaaagt tttaccaaaa caaagtttga 2220
tttatcaaag attcatgatc ttcaatgaat tgacaaaagt cagttatacg gatgaacgtg 2280
gcaaatctca ctatttttca agtgaacaaa aacgcaagat ttttaacgag ttgttcaagc 2340

aacatccaag agtaacagaa aaacaattga ggaaattttt agagttgaac gagcagatcg 2400
attcaactga aatcaaagga attgaaacgt cattcaatgc ttcttatagc acctatcatg 2460
atttgttgaa attatcagat cagatggata cattattaga tgatcccgat atgacaacga 2520
tgtttgaaga aattatcaaa attttgacga tttttgaaga cagagaaatg attagggaac 2580
aactgaaacc gtacgaaact gtcttaggtc ttccagctat caagaaatta gcaaaaaaac 2640
attatactgg ttggggacgg ctatcggaaa aaatgatcca aggaatgcga gagaagcagt 2700
cccggaaaac cattcttgat tatttgatcg atgatgatga ttttccttgc aatcgtaatc 2760
gaaatttcat gcaactaatc aatgatgatc atctttcgtt taaagaaacg attgctaatg 2820
aattgatcat gagcgactca aacgtattgc ttgatcaagt gaaggctatc ccaggaagtc 2880
ctgcggtcaa aaaaggattt tggcaaagta tcaaaatcgt cgaagaaatc attggtatca 2940
tcggtaaggc acctaagaac atagtgattg aaatggcacg tgagaatcaa agaacaagcc 3000
gttcaagacc ccgcttaaaa gccttagaag aagcattgaa gaatatcgac agtccattgt 3060
tgaaagacta tccaacagat aatcaagcgc tacaaaaaga tcgactgtat ttatattatt 3120
tgcaaaatgg aaaagatatg tatacaggtg aaccactaga gattcatcgt ttatcagaat 3180
atgatattga ccatattatt cctagaagtt ttattgttga taattcttta gataataagg 3240
tcctagtttc atcaaaagtc aatcgtggaa agttagataa tgccccggac ccacttgttg 3300
tcaaaagaat gcgatcacat tgggaaaagc tacatcaagc aaaattgatc agtgacaaaa 3360
aactagccaa tcttacaaaa caaaatctga ccgaggcaga caaggcaaga tttatccaac 3420
gccagttagt cgaaacaaga cagataacga aacatgttgc caatctgtta catcagcatt 3480
ttaatttacc agaagaagta agtgcgactg agaaaacaag tatcattacc ttgaaatcaa 3540
cgttgacaag ccaattccgt cagatgttcg atatttataa ggtgagagag atcaatcatc 3600
accaccatgc ccacgacgct tatttaaacg gagtagtcgc aatgacttta ctaaaaaaat 3660
atcctaagtt ggcaccagaa tttgtctacg gaagttatat caaaggggat atcaaccaga 3720
tcaataaagc aactgcaaaa aaagaattct attctaatat catgaaattt tttgaaagtg 3780
aagaaatcat ttgtgatgag caaggtgaag tgatttggaa taaaaaacgt gatctctcaa 3840
caatcaagaa gacgattggc gcacaccaag tgaatatcgt taagaaagta gaaaaacaaa 3900
aaggtggatt ctataaggaa acaattaatt caaaagcgaa tccagaaaaa ctaattccaa 3960
gaaaagcatc attagatcct ttgaaatatg gtggctatgg tagcccactt gtggcatata 4020
cggtgatttt tatctttgaa aaaggaaaac agaagaaggt aaccaaagga atcgaaggaa 4080
ttaccgttat ggaacaattg agattcgaac aagacccacg agagtttcta aaacaaaaag 4140
gctatgaggg cgtaaaacaa tggctgatat tgcctaaata tatattattc gaagcacaag 4200
gtggatatcg gagaatgatt gcgagtcatc aagaaacgca aaaagctaac tcattgattt 4260
tacctgaaaa tctagttaca ttgctttatc atgcaagaca ttacgatgaa atcaatcata 4320
aagtgagttt tgattatgtc aatgcgcata aagaagggtt taatgacatt tttgatttta 4380
tctctgactt tggggtgcgt tatattttgg ctcctcaaca tcttgaaaaa ataaaagttg 4440
cctatgaaaa aaataaagag gtagatctga aagaaatgat agatgctatt ttatctttat 4500
tgaaatttac gttatttggt gcttctgttg agtttaagtt ttttgatatt aagatattaa 4560
aaagatataa gagtttgacc gacatttggg aagcaaccat tatctaccaa tcggttactg 4620
gattatatga aaggcgagtg gaggtaagga agttatggga tggagaacgc ttgtaatcaa 4680
tacacattca aaattgacct atacaaacaa tcatttgatt tttaggaacg cagaaagaac 4740
agaaaaaatc cacttgtcag aaatcgatgt tctattatta gaaactacgg atatcacgat 4800
tactacaatg ttgctcaagc gtttagtgga tgaaaaagtc cttgtcttgt tctgtgatga 4860
caaacgatta cctactgcaa aattgcagcc attttatagt cggcatgata gcaatttaca 4920
attgactcgg caaatcgaat ggaagaaaga tgacaaagcg agtgtttgga ctgagatcat 4980
cacacagaaa atcaacaatc agcaacgttt tttagaaaag cgtttattta ctgaaaaagc 5040
agaagcaatt gctcagttga ctgccagttt acaattacta gatccaacca atcgagaagg 5100
ccatgcagcc agaatttatt tccaaacatt gtttggaaat aaattcacta gagaagcaga 5160
caatgcaatc aatgctggat tagattatgg atatacttta ttgatgagtc tatttgctcg 5220
agaaatcgtc aagaatggtt gcatgactca atttgggttg aaacatgcca atcagtttaa 5280
tgactttaac ttagccagtg atttaatgga gccgtttcgc ccattagtcg atcaaatcgt 5340
gtacgaggta cgtgagaaag aatttccaat aatcaaacgc agattattcg atctgttctt 5400
aacgaattat tcttatgaga aaaaagagat gttcctgaca aatatcgttg cagattatac 5460
aaagaaagta atcaaagcat taaataaaga agcgaaagga gttccacact tcggaatatg 5520
agttatcgct atatgagaat gatattaatg ttcgatatgc caactgaaac cgtcgatgac 5580
cgcaaagctt atcgtcgttt tcgaaaattt ttgatcagtg aaggcttcat catgcatcag 5640
ttttccgtct atagtaaact cctgcttaac aataccgcca atcaggtaat gatcggtcgt 5700
ttaaaaggga ataatcctaa gaaagggttg atcacgatcc ttacagtaac agaaaagcaa 5760
tttggtcgaa tgatttattt gactggtgaa aaagatcaaa gtatcgggaa tctagatgac 5820
cgagtgatct ttttaggaga ggagttcaat gatgaatctt aatttttcat tacttgatca 5880
gccaatgaaa tttgagcatg cgaatttttt tatcatagaa gacgtgacgg tgtttgctaa 5940
aatgatccaa cagctgtaca cctataaaga aaatggtgaa gtgaagttat tcaatgaacg 6000
gcatgaaacg attaaagaaa gcgaattgat gatgataaca gatattttgg gctttgacat 6060
caattcagcc gcgacactga aattgattta ttcagattta gaaagacaat tgaacgaaaa 6120

gccagaagtg aagtcgatga tcactaaatt gacagatacg atcagtgaac tgattgagta 6180
tgaattatta gaccatgaga tggacttgga aagtgatgag atcacgattt tagaattatt 6240
caaggcgtta ggagtcaaaa tagaaacgac gagtgatacg atttatgaaa aaataatcga 6300
gattttacaa gtatttaagt atctgacaaa aaagaaacta ttaatcttta tcaatgtttg 6360
ttcgtattta acgttgaaag aaatagaaga aatacgatct tatatttctt tatgtaacat 6420
ggatgttctt tttatcgaac caagaaaaat tgcaggaatc acacaaacaa tcttagataa 6480
tgattatttt ttaatagatg gtagtatggt agaataaaag aaaataagta ttcaattctg 6540
aagttttgct atgaacgaat cgcgcgatta cgaaactttg tcagaaaaaa ttttttctac 6600
gaggttttag agctatgttg aattgaatgc ttccaaaacc atccatttcg tcgatatggt 6660
catcgccctg ttttagagct atgttgaatt gaatgcttcc aaaacagatt gatacaaatc 6720
tcaaaacatc tgcaagtttt agagctatgt tgaattgaat gcttccaaaa cgtagagaag 6780
cggtgctacc tcttaaacca tgttttagag ctatgttgaa ttgaatgctt ccaaaacaac 6840
cagaagcgaa taagcgtcct atcatctgtt ttagagctat gttgaattga atgcttccaa 6900
aacagacgcc cgaatgttta tcaccacaac ggagttttag agctatgttg aattgaatgc 6960
ttccaaaact tgcaacacaa aggtaacata ggtgttgtgg ttttagagct atgttgaatt 7020
gaatgcttcc aaaaccatac tgttgtgcat cgaatgcacg ttggagtttt agagctatgt 7080
tgaattgaat gcttccaaaa ctagaattga acacatgtat ctagatattg agttttagag 7140
ctatgttgaa ttgaatgctt ccaaaacaat aaatggacga ataataatcc ttagagagtt 7200
ttagagctag gttgaattga atgcttccaa aacaacatac ttcattaatg atcctaaaaa 7260
gtcgttttag agctatgttg aattgtctgc cattactagc tatactggat ctcagtaaaa 7320
agatgatagt gggattcact aataaaaagt acagagtcag aagaaaacac agacacttgt 7380
gtttcttttt tttgctcaaa ctggacgaaa ctctttactc ttagtaggat aggagtagaa 7440
gaaaaagtgg ggtgtaaaga tgtttagtga aacgttggtc catttgttgg aatgggtgac 7500
tgcgattttg aatatttctt cgattttagt cttgttggtt ggaatttttt tagttttaaa 7560
aaatttgatg ttggaacgtc cattgattaa agattatgcg acacgtaatc ggcgcaatgc 7620
ggatattcga aaactactag ctagttacat tttgttgagt ttagaagtac tgatcgtggc 7680
agatctcatc gaatctgtca ttaaaccaac ttggacagac attttaaaat tagcattgat 7740
cattatcatt cgcacgatca tctcttattt cttaaattta gaaattgcga gtcatgtatc 7800
gcatgaaaga agggaa 7816

<210> 10
<211> 8221
<212> DNA
<213> Enterococcus faecalis

<400> 10
attgcgaaac aagcagctaa attcccaagt gtttttaaag ccgattatgg tggtgtaaac 60
tcagacaaaa tctttaaaat cgctcaaacc gttcgaactt ggggcttagc agctgctgca 120
ttacttttat tcgttgcagt gttcttaatt tccaacacca ttcggattac gattttatct 180
cgtcaaaaag aaatccaaat tatgcgtctt gtaggagcga aaaacagttt cattcgttgg 240
ccgttcttct tagaaggtgc ttggattggg ttgattggcg caatcgtgcc agtcatcatt 300
atgacgttag gctaccatca agtttataac atgtttaacc cgcagttatt gcgttcgaat 360
tattcattaa ttcgtccaga agattttatc tggaaagtta atctattaat gattgcaaca 420
ggaatgatta ttggatcact aggttcagtt atttcaatgc gtcgtttctt gaaaatttaa 480
aaaagtataa ttttgcggac aaaaaccttg ccatctaagt gatttatcac atagagattg 540
ctaagatgag agaaatgttt ttgttcagta ggctatctta aaagttaaaa aatgacacat 600
aagttgactc gacttatgtg tcatttttta gttaataatc aacacaccaa tccatcggaa 660
aatctgtagt agattttgta tacgggagga cctatctgat ggtaaaattt attgccatat 720
gcttaaaacg tatatattat ttaagcataa gcagataacc gtagattaaa attgtggaaa 780
ttgttatttt ttctttctaa atcatgataa attaaagggg taatagttat cataaatttc 840
tgaaaattat ttattgaaaa ttgtatccct ttacaaaaaa tattgtataa ttaaattatt 900
aattgtcatt gcttaaagga ggttctcatg tacagtatag gtttagattt agggattagt 960
agtgttggtt ggtcagtaat tgatgaacgg acaggcaatg ttattgattt aggtgtccgt 1020
ttatttagtg cgaaaaatag tgaaaaaaat ttagaacgac gcacaaatcg aggtggacgt 1080
cgtttaatta gacggaaaac gaatcgctta aaagatgcaa aaaaaatact tgcggctgtt 1140
ggattttatg aagataaatc attaaaaaat agttgcccgt accaattaag agtaaaagga 1200
ttgacagaac cattaagcaa aggagaaatt tataaagtga ctcttcacat tttaaaaaaa 1260
cgtggaatta gttacttaga cgaagatgat acggaagcag cgaaagaaag tcaagattat 1320
aaggaacaag ttagggaaaa tgctcagttg ttaacaaaat atacgccggg tcaaatccaa 1380
ttacagcggc tgaaagaaaa taatcgagtg aaaacaggaa tcaatgctca aggtaattat 1440
cagttaaatg tttttaaagt ttctgcttat gcaaatgaat tggcaaccat tcttaaaacc 1500
caacaagcct tttatcctaa cgaattgaca gatgattgga ttgcattatt tgtacagcct 1560
ggtattgcag aggaagctgg attaatttat cgcaaacgac cgtattatca tggaccagga 1620

aatgaagcca ataacagtcc ttatggtcgt tggtctgatt ttaaaaaaac aggccaacca 1680
gcaacaaata tttttgataa attgattggc aaagactttc aaggtgaact acgagcaagc 1740
ggcttaagtc tatctgcaca acaatataat ttactcaatg atttaaccaa tttaaaaatt 1800
gatggagaaa taccactttc gctagaacaa aaagaataca ttttagcaga attaatgacc 1860
aaagaattta ctcgttttgg cgttaatgat gtggttaaat tgctgggtgt aaaaaaagaa 1920
cgattaagtg gttggcgttt ggataaaaaa ggtaaaccag aaattcatac attaaaagcc 1980
tatcgcaatt ggcggaaaat ttttgctgaa gcagggatcg atttggctac gttaccaaca 2040
gaaacgattg actgtttagc gaaagtttta accttaaata ccgaacgcga agggattgaa 2100
aatacattag cttttgaact gcctaagtta gcagaaccag taaagtcact agttttagat 2160
cattacaaag aattaagcca aagcattagc acgcaagcgt ggcatcgttt ttctttaaaa 2220
acattacact tattaattcc agaactaatg aacgctacct cagaacaaaa tacactctta 2280
gaacagtttc agttaaagtc aggtgttaga acacggtata gtgaatataa taaattacca 2340
acaaaagatg tcttggcaga aatttataat ccaacggtga acaaaacagt gagtcaagcc 2400
ttcaaagtta tggatgcttt gttggaaaaa tatggcaaag agcaaattcg ttacatcact 2460
attgaaatgc ctagggacga taacgaagaa gatgaaaaaa aacgaataaa agaattacat 2520
gcaaagaata gtcaacgaaa gaatgacagc caaagttatt ttatgcaaaa gtctggatgg 2580
agccaagaaa aatttcaaac gaccattcaa aaaaatcgtc gttttttagc taagttatta 2640
tactattacg aacaagatgg catatgtgcc tatacaggac tgtctatttc tcctgaatta 2700
ttggttagtg attcaacaga aattgatcat attattccaa tttcgattag cttagatgat 2760
tctattaata ataaagtact ggttttaagt aaagctaatc aagtcaaagg acaacaaaca 2820
ccatatgatg catggatgga cggttctttc aaaaaaacta acgggaaatt tagcaactgg 2880
gatgactatc aaaaatgggt ggaaagtcgc cattttagtc ataaaaaaga aaataattta 2940
ttggaaacgc ggaatatttt tgacagtgag caagtcgaaa aatttttagc tagaaatctg 3000
aatgatacgc gttatgccag tcgtttagtt ttaaatacct tacagagttt ttttgcgaat 3060
cagggaacga aagttcgagt agtgaacggc agttttactc atactttacg taaaaaatgg 3120
ggtgccgact tagataaaac tcgtgaaaca catcatcatc atgccgttga tgcaacattg 3180
tgtgcagtca ctccgtttgt caaggttagt cgttatcact atgcagtgaa ggaagaaact 3240
ggtgaaaaag tcatgcgtga aatcgatttc gaaactggtg aaattgtcga tgaaatgtct 3300
taccaggaat tcaaaaaatc aaagaagtat gaacgaaaaa cctatcaggt aaaatggcct 3360
aattttcgtg agcaattgaa gccagtaaac ctccatccaa gaattaaatt ttctcaccaa 3420
gtggatcgta aagctaaccg caaactcagt gatgccacta tttattctgt tcgagagaaa 3480
acggaagtca aaacgctgaa aagtggtaaa caaaaaataa cgactgacga gtacacaatc 3540
ggtaaaatca aagacattta cacggtagat ggttgggaag ccttcaagaa aaaacaagat 3600
aaacttttaa tgaaagatct tgacgaaaag acctatgaac gattgttgtc tattgcagaa 3660
acaactcctg attttcaaga agtggaagaa aaaaatggta aggtaaagcg tgttaaacgt 3720
tcaccgtttg ctgtttactg tgaagaaaat gatattccag cgattcggaa atatgcgaaa 3780
aaaaacaatg gaccacttat tcgtagtttg aagtattatg acggtaaatt gaataaacat 3840
attaatatca ccaaggattc acaaggacga cctgttgaaa aaactaaaaa tggtcgcaaa 3900
gttacactac aaagtttaaa accatatcgc tacgatattt atcaagattt agaaactaag 3960
gcttattata cagtacaact atattattct gacttacgtt ttgtggaagg taagtatgga 4020
atcacagaaa aagaatacat gaaaaaagtc gccgaacaga caaagggaca agtggtgcgt 4080
ttctgttttt ctttacaaaa aaatgatggt ttggaaattg aatggaaaga tagccaacgc 4140
tatgatgtgc gtttttataa tttccaaagt gctaatagta ttaactttaa agggttagaa 4200
caagaaatga tgccagcaga aaatcaattt aagcaaaaac cttataataa tggagccatc 4260
aatttaaata tcgctaaata tggcaaagaa ggcaaaaagt tacgaaaatt caatacagat 4320
attttaggaa agaaacatta tctgtattat gaaaaagaac caaagaatat tattaaataa 4380
aaaagaagaa acgaaaagaa aagggttgca tgattagcgt ttttgtagta atctttaatt 4440
gtagtcgacg gactaccgcg tttgacgaaa cgtgtcttta ataattttac tgataagaaa 4500
ttattgagaa tctacaaaaa taaggcattt tgccgaattt accgccctac atatgtaggg 4560
cggttttttt attaaaaagg aggaaatgtt gttgggattt cgaacagttt tcgtaaaaaa 4620
tggcgagaaa ttaaaagtaa agttagataa tttagaagtg ataaaagaag ggaatacata 4680
tgtcattcct ttgacagata ttgaaagtgt aattttagaa ggcgaccaga cattcatcac 4740
tacacggtta ctagcgaagt tcgcacaaca tcatattgat accgtgattt gtgataatac 4800
gttcatgcct gtaggtgttt ttcttggtat tggacagtat catcgcagtg cgaaacgagc 4860
aatctggcaa agtaattgga cagaagaaca taaacaagta gcgtggtgcg aaattgtaac 4920
acaaaaaatt cagaatcaaa tcgcagttgc gaaatattta ggaacagatt ccgaacgagt 4980
ggaagtatta gaaaaactta gtgaaggaat tttaccaggg gatacaacga atcgtgaagg 5040
acatgttgca aaagtatatt ttcattcatt gtatggtgtg ggttttaccc gtgaagaaga 5100
gtgcttacca aatgcctgta tgaattatgg ctatgcagtt attcgtgcac aaatggcccg 5160
ttgcgtggta gcgttaggat tattaccgat gttagggatt ttccataaaa atgaatataa 5220
ttcgtttaac ctaattgatg acttaatgga accttttcgt ccattaatgg attggtatat 5280
tcatcaaacg atattgaaaa agaatgaaaa gtatttaacc tatcatagtc gtttaacctt 5340
agtggaattt ttacatcaaa agattaaggt gaaaaacaaa aaaatttata tgaaccaagc 5400

aatgtctgaa tatgtagctg cttttgtgaa agcaatggaa acaggtgatt ttgaaaacgt 5460
tcagaaaatt catttggtca atatgttgga gtgtgagggg aagtgagttt aggagtcgtg 5520
cgattaatgt gtttatttga tttacccttt gaaacgaaac aggagcaaaa agaataccga 5580
atttttagaa aacatttact ggaaaacgga tttattatgt tgcagtattc tatttattat 5640
cggagtttgc ccaatcgtag tgcgttaaaa aaatatgaaa caattctaaa gagaaaagta 5700
ccacgttcag ggaacgttcg attgttgtat gtttcagaaa ttcagtttca agatatgcta 5760
ttattggcgg gcagtcgtag tcatcaagaa gaagtagttg gcgttcaccg attggtggtg 5820
atttaatgaa acaaattcgt ttagaatatc agtatcaaca atttattgaa ttctctttgg 5880
aagattatgt atttttttat ggaggagaaa accaatggcg tcgtaaaata ttacgaacat 5940
taaaacggtt tgcgaaacaa aaagcgttaa gtgacttaga agaatcagtc tatggagata 6000
acggtattga aattttttta gaagaaaaaa agttaaaggc aaagagtatt gatttttact 6060
ttttagagga caatacaagt atttttcaac aaatgaattt tatcaaaggt acgttacttc 6120
accaggaatt actttattta caagatgatt ttgatattac aaatcaaatg agcaatttaa 6180
atgatcagtt gttaaaacta gaaaatttaa tgaatgataa attaaataac tatttaacga 6240
atattgaacc gcatttttta gaaatgaatt ttcaggatat tttgaagtat tcgttgtcat 6300
taaactattt agaaaatggc caatcgttgc ctttagaaat gatggataca agggaattgt 6360
tggctgaatt tatttatctc ttgcaacgga gtatcagccg aagagaacag cctgtctggc 6420
ttgttattac aaatccagaa agttttttgg agacggaatc aatttattat ctctttcagc 6480
agctaaagaa attggctcaa gaaacaaagc agttgaagtt ttttattttt agtaatagaa 6540
gcttgccttt accgtatacg gaggaagatg ttgagaaaac gattttgtta tatgataagt 6600
atcagcagtt gccagttttt gatgtttttc gtcaaagtat tgagcgacat tatccagatc 6660
aactctcttg gacaaatcaa caactaattg atgcttttta tcgagtctgt cattttgttg 6720
gtgatcagta tacaaaaaat tatcttttac ctaaagatat gatattatta aaattgttga 6780
aggaattgtt agacgacaat agtgagtgtg tcgagacgtc gatggaaaag ctcactgtat 6840
tggaagaaca atattttcgc gaacggttat tgcggaaaga gtgagtctgg aattttaacc 6900
gcgtaaagtt atatttttta tcaaaaggat tattaatgag atttcaaatt ttaaatttga 6960
ggtttttgta ctctcaataa tttcttatca gtaaaacttg aaacacataa tcattaaccg 7020
ctttttggtt tttatactct caataatttc ttatcagtaa aacaaaattc ttctaacgtt 7080
tgcgctgaaa tatgtttttg tactctcaat aatttcttat cagtaaaact gtttattctg 7140
cggtttctcg ccaaattaag tttttgtact ctcaataatt tcttatcagt aaaaccactt 7200
catatacctt ctaaacgagt tattagtttt tgtactctca ataatttctt atcagtaaaa 7260
cggatgtacc agttattctt ggtctttcaa agtttttgta ctctcaataa tttcttatca 7320
gtaaaactgt tcctcttgat ggtgctgacg gcaaccagtt tttgtactct caataatttc 7380
ttatcagtaa aactgtctct ttgatggctt cctttttaac cagtttttgt actctcaata 7440
atttcttatc agtaaaacaa aaaccagatt tagaaactat taaagatagt ttttgtactc 7500
tcaataattt cttatcagta aaacgctttt tctcctgcaa actcttcgat ctgagttttt 7560
gtactctcaa taatttctta tcagtaaaac caactagttc agtaatttcg ccttcacttt 7620
gtttttgtac tctcaataat ttcttatcag taaaacaaac gataccttat accattgaat 7680
ataatggttt ttgtactctc aataatttct tatcagtaaa accgttccat tgctttaaac 7740
tagtatcgga aggtttttgt actctcaata atttcttatc agtaaaacag ttcgcttatt 7800
cccatcatgg cacagaccgt ttttgtactc tcaataattt cttatcagta aaactggaac 7860
atgagagtgg tttaaaccct gctagttttt gtactctcaa taatttctta tcagtaaaac 7920
gcacctagaa cgacgtcaat cggttggttg gtttttgtac tctcaataat ttcttatcag 7980
taaaacgcaa aatgcccgct gctatctatt caacgagttt ttgtactctc aataatttct 8040
tatcagtaaa actaaagatg gcaatcctaa gccaaggctt gagtttttgt actctcaata 8100
atttcttatc agtaaaacac gtgtcgtcgt aagttgttgc agtccatggt ttttgtactc 8160
tcaataattt cttaccagta aaacaataag gaagtaggtt ggatgtacaa actcgctttt 8220
a 8221

<210> 11
<211> 8221
<212> DNA
<213> Artificial sequence

<220>
<223> CRISPR-Cas Locus 16

<400> 11
aagtagaccc ggcttcagta actgaaaaag ataaaaaacc tgcagatctt gaagcacact 60
tgaaaaatcc tgacttcttc gatacagcaa aaaatccaac ttctgacttt aaaattacca 120
gcgtaacaga tcttaaagaa gcaccgaaag atgcagtagc tggagctaac aaaacagtaa 180
gcggaaacct taccctatcc ggaaaaacaa tgaacgttac gttcccggct aaagtagacg 240
ttgcagaagg tacagcagct attcaggcta aatttacagt gaacagagca gactggggga 300

tcaaattcgg aacttctgaa gcagaccctg cagaatggat gatcagcaaa gacatcgaaa 360
tcgctgtaga cgtaaaagct aaaaaataat attgttagat attcaattct ttgagctgcc 420
ttttttaagg tggctttttt ttgtgcttta aatttttgta cttaaaaacc tttaaatggg 480
aaaaggcgag ttaccccgcc ttgaatgttt tcacaacgga ataatcctta ttgtgaattg 540
ctttcggtta caaatctaaa aattattttt ttaactgttt tatggtttcc cataaaatga 600
ttactttttt tctctttatt tttatgcttt atgaaaacca aatagcaatg actaaaaata 660
tactaggatt agacttagga acgaactcaa taggatgggc attgataaaa caggattttg 720
aaaacaaaca aggtgaaatt cttgggatgg gaagcagaat tatacctatg tctcaggata 780
ttttggggga atttggaaaa ggaaattctg tttctcaaac tgcttctcgg actagctata 840
gaggtgtgag aagattacgt gaaagatttc ttttgcgaag ggaaagatta catagagttt 900
taaatgttct gaacttttta cccgcacatt atgcttctca gatagatttt gaaaagaggt 960
ttgggaaatt caaagaagaa actgagccta agttggttta tgacgtgaat gaatttattt 1020
ttaaaaattc gtttgaagaa atgctttatg attttagaaa gtatcagcca cagcttttag 1080
aaaatgataa aaaagttccc tatgactgga cgatttatta tttacgtaac aaagccttaa 1140
cccaaaaaat agagaaagaa gaacttgctt ggattctttt aaattttaat cagaagcgtg 1200
gttattatca attacgtggg gaagattttg aagaagaaaa agataaaact tttgtacgcc 1260
taaaagttga taaaattgtt gattcgggag acactataaa gggcaaaata ttatatgatg 1320
tttatttcga aaatggctgg aaatatgata aacagattgt aaaaacggaa gattggatag 1380
aacgtgtaaa agaatttatt gttacagaat cattcttaaa aaacggcgat ataaaaagaa 1440
cttttaaagc tgttgattct gaaaaggatt ggattgctat caaaactaaa acagaacaag 1500
aaattgataa ttcgaataaa accgttggga catacattta tgaaacactt cttcgaaatc 1560
caaaacaaaa aattaaagga aaattggttc gtacgattga aagaaaattc tataaagatg 1620
agctgaaaca gattctggaa aaacaaaaag aatttcatct tgaattaaag aatgatgatt 1680
tatatagtga ttgtattcgt gaattgtatc gaaataatga agcgcatcaa ttgacgttga 1740
gtagaaaaga ttttgttcat cttttgatgg aggatattat tttttatcaa agacctttaa 1800
gaagtcataa atcttctatt tcaaactgta ctttggaatt tagaaaatat aaagatgaaa 1860
atggagcaga gcatattcaa tatttaaaag cgattccaaa atcaaatcca tattatcagg 1920
aattcagaat ctggcaatgg atttttaatc tcaatattta taagagagat gacgagcaaa 1980
atcctgttac aaaaaagttt ttaaacacca cccaagattt tgaaaatctg tttgaatttc 2040
tgaataacag aaaagaaatt gaccaaaagg ctttactcaa acatcttaaa ataaatgaga 2100
aaacgcatcg ttggaagtat gtagaagaca aaaagtatcc ttgcaacgaa accaaaacaa 2160
tgatttcatc aaggttggat aaagttgaaa atattctgaa aaacttcctg acaagagaaa 2220
ttgaacagaa gatttggcat attatctatt ctgtcaacga taaaattgaa tatgaaaaag 2280
ctttaaaatc ttttgccaat aaaaataatc tggacgaagt ttctttcttt gaagctttta 2340
aaaaattccc accttttaaa agcgaatatg gttctttttc tgaaaaagca ataaagaaac 2400
tgttgccttt aatgcgattg ggaaaatatt ggaattatgc tgatattgat aaatattcaa 2460
aagacagaat tcagaaaatt atcaatggag aatatgatga aaatatcaaa gacaaggtaa 2520
gggaaaaagc agttcatctt acgactgaaa atgattttca aggtcagcaa ttgtggctgg 2580
ctcaatatat tgtttacgga aggcattctg aagcttcaat gattggaaaa tggaattctg 2640
ccgatgattt ggaagagttt ttaaaagagt ttaagcaaca ttcgcttcga aatccgattg 2700
ttgagcaggt cattaccgaa actttgcgcg ttgtaaaaga tatttggttg aaatacggaa 2760
atggagcgaa agatttcttt aacgaaattc atattgagtt gggaagagaa atgaaacttc 2820
ctgcagatga tagaaaaaaa ctaacgaacc aaatttctga aaacgaaaat accaatcttc 2880
gtatcaaggc tttgctggct gaaatgataa acgaccattc tgttgaaaat gttcgtccgt 2940
tttcgcctat gcaacaggaa attttgaaaa tttatgaaga tggagtattg aattcaggta 3000
ttgaaattga agatgaatat ttgaaaatca gcaaaaccgc acaaccttct ccgtctgatt 3060
tgaagagata caaactttgg ttggaacaga aatacaaatc gccttacaca ggacaaatta 3120
ttcctttaaa taaattgttt acgccagaat atgaaattga acatatcata cctcaaagcc 3180
gatattttga tgacagcttt agtaataaaa ttatttgtga atcagccgtt aataaattga 3240
aggataatta tatcggactt ggatttatta agcaattcgg agggactatt attgagcttg 3300
gttttggaaa aaatgtaaag gtttttgaag tcgaagaata tgaagatttt gtcaaaaaac 3360
attatgcaaa taatcgaagt aagagaaata aacttttatt agaagaaatt cctgaaaaaa 3420
tgattgaacg tcagttgaat gatacacgtc atatcagtaa gtatatttca ggtgtgcttt 3480
ccaatattgt aagagtagaa gatggaacag atgaaggtgt aaattctaaa aatattgttc 3540
ccggaaacgg aaaaatcaca actcaattaa aacaggattg gggactgaat gatgtttgga 3600
atgatttgat actgccacgt tttgaaagaa tgaatcaact aaccaattca acagatttta 3660
cagcttggaa tgaaaatcat caaaagtatt tgccaactgt tccgattgag ttttcaaaag 3720
gattttcaaa gaaaagaatc gaccatcgtc atcacgcttt ggatgctttg gtgattgctt 3780
gtgctacaaa agaccacgta aatttattga ataatcaatc tgcaaaatcg gagacgaaac 3840
gttacgattt gaaaaagaga ttgatgaagt ttgaaaaagt tgtttacaat catacacaaa 3900
caggagagaa aatcgaaaga gatgttccga aaaagttttt gaaaccttgg gaaaacttta 3960
cggttgaagc taaaaacagt ttggagaaaa taattgtaag ctttaagcaa aatctcagag 4020
taatcaataa agctacaaat tattatgaaa aatatgtgga gaaagatggc ttgaaaacga 4080

aagaaagggt agagcagacc ggaacgaact gggcgataag aaaatcattg cacgaagaaa 4140
ccgtttcagg gaaaattaac ttgccttggg tggaaacaag caatggagaa tttattactg 4200
ccacaagaaa gtctttggac acaagcttca atttggaaaa aatcaaaaaa attacggata 4260
tcggaattca gaaaattctc agaaattatc ttgaggcaaa agatggcaat cccgaattgg 4320
ctttttcgcc agaaggaatt gaagatctga ataaaaatgt tgaaaagtat aatgatggta 4380
aatcacatca gccaattatc aaagttcgtg tttatgaaaa aggcaaagga aggtttgtct 4440
tagggcaaac aggaaataaa attgataaat acgttcaggg ttcccctaac ttatttttcg 4500
caatatataa agatgataat ggtaaaaaaa gttatgaaag tattcgttta gatattgcga 4560
ttgaaagatt aaagcaaggt ttgcaagcag ttccagaaac aaatcaaaat ggatttgcat 4620
tgtatcaaag cctgtctcct ttagatttag tttacattcc cacagaggat gaagttgaat 4680
caccacatct aattgatttt agtaaattag ataagataaa attggaaagg atttataatg 4740
taaatgactt ctcaggatat acaatttact tcagccaaaa ttcatttgct aaaaacattt 4800
atcctaaaga gatggatatg acctggaacg aaaaaaaaca aaaaccatca ggttcgtttg 4860
attctaaaac agcttcatac aataaaaaat cgattaaaga tatttttatt aaaatgaaaa 4920
ttgaccgttt aggaaatatt tcaaaagcgt aattatgata acccgctcca tctacatcgg 4980
caatcccgct tatctcaaac taaaagatga gcagatgaaa atcctctgtc cggaaaccaa 5040
atcggaaaag ggtagtatac ctgtggaaga tttgggacta ttgatgttgg atcattttca 5100
gattacgatt tcacataatc tgattcaaaa aatgatggga aataatgtgg tggtggtaag 5160
ttgtgatact catcatttgc cacacggaat tatgttgccg ttgtatgggc atacagagca 5220
ttcggatcgg gtaaaagacc agttggaagc cagtgaacct ctgaaaaaac aactttggaa 5280
acagacggtg gaatgtaaga ttgaaaatca gaaaaacgtt ttaatgaagt tggggaatta 5340
ttatgaacct atgattgaat atcaaaaaaa tgtaaagtcc ggcgatatca ccaatatgga 5400
aggtattgcg gcgcaacatt actggaaata tttaatcagt cttgattttc ttcgacaacg 5460
ttttggagat tcacccaatc agttttttaa ttttggatat gcagttctca gaagtattgt 5520
agcaagagct attgtggaaa caggactgct tccggtgttg ggtatttttc ataaaaataa 5580
atacaatccg tattgtcttg tggatgattt aatggaacct tatcgaccgt ttgtggattt 5640
gctggttatg cagtggctga aaattaatcc tgggactgaa gaactgacaa aagaatttaa 5700
agcatatatt cttcagattg cgaccaaaga tgtattgatt gataataaaa caagaccttt 5760
gttagtagct gtaaaaacaa cagcttcttc gctttataaa tgttatacag gcgaaaaacg 5820
attaatttcc taccctgaat tgagatgaat tccgaaaggt ttaatgcata ccgaattatg 5880
tgggttttag tattatacga tttaccgact gaaactaaag ctaatatgaa agatgcaaac 5940
cgctttcgta agagtttaat tgatgatggt tttacattat ttcagttttc gatgtatgtc 6000
cgccattgtc cgagtcggga aaatgctgaa gtgcatatca aaagagttaa gtttatgcta 6060
ccaaaagctg gtaaagtcgc tattatgtgc attacggata agcaatttgg agatattgaa 6120
attttctttg ccagaaataa agaagaacca cctccaacct ttcagcaact ggaattattc 6180
tgatttttta aatcctaaat tataaataaa cccactgtaa ttcagtgggt taaattttga 6240
ggttgtgctg tatctcaaag atacggataa atgaaagcaa ttcacaacag ttactgctgt 6300
tgatcaaaat tcttctcagt tgtgctgtat ctcaaagata cggataaatg aaagcaattc 6360
acaactaaaa tgggcgcaat tcacttgata agagtgttgt gctgtatctc aaagatacgg 6420
ataaatgaaa gcaattcaca acaccaaaag cttgatacga ttatacaact cagttgtgct 6480
gtatctcaaa gatacggata aatgaaagca attcacaacc tttgcagtcc atatacaccg 6540
atccgtaaag ttgtgctgta tctcaaagat acggataaat gaaagcaatt cacaacgaaa 6600
ttggacgcta tgtaataatc atccatagtt gtgctgtatc tcaaagatac ggataaatga 6660
aagcaattca caacagaaac tctaaagctt ctttttgatt atcggttgtg ctgtatctca 6720
aagatacgga taaatgaaag caattcacaa ctgatacgtt ctagcagtag cggttaatgt 6780
agttgtgctg tatctcaaag atacggataa atgaaagcaa ttcacaacaa gataaaaaaa 6840
taacagaaca ggattatagt tgtgctgtat ctcaaagata cggataaatg aaagcaattc 6900
acaacaagat aaaaaaataa cagaacagga ttatagttgt gctgtatctc aaagatacgg 6960
ataaatgaaa gcaattcaca actcattatg ctccatatta ttctcctagt tagttgtgct 7020
gtatctcaaa gatacggata aatgaaagca attcacaaca agataaaaaa ataacagaac 7080
aggattatag ttgtgctgta tctcaaagat acggataaat gaaagcaatt cacaacctta 7140
taatgatagt tctataaatt atataggttg tgctgtatct caaagatacg gataaatgaa 7200
agcaattcac aacttagtag cataaataac agctaattga gcagttgtgc tgtatctcaa 7260
agatacggat aaatgaaagc aattcacaac aaagttatta tttatttata tataagaaat 7320
gttgtgctgt atctcaaaga tacggataaa tgaaagcaat tcacaacaaa ttggagatag 7380
tgatgaggat tccttatgtt gtgctgtatc tcaaagatac ggataaatga aagcaattca 7440
caacaagagg ctccggaagt atcaggatac atatgttgtg ctgtatctca aagatacgga 7500
taaatgaaag caattcacaa ctcaattaat atgaaattga aattactatt tgttgtgctg 7560
tatctcaaag atacggataa atgaaagcaa ttcacaacca ttaatctgaa attgaaatta 7620
ctatttcagt tgtgctgtat ctcaaagata cggataaatg aaagcaattc acaacaattc 7680
tcaaactcat atccagcagt aggatgttgt gctgtatctc aaagatacgg ataaatgaaa 7740
gcaattcaca accgttatga aaaagtcagt taaaaaaaat atagttgtgc tgtatctcaa 7800
agatacggat aaatgaaagc aattcacaac tctgaaacgc cataattaat ttatcaggct 7860

gttgtgctgt atctcaaaga tacggataaa tgaaagcaat tcacaacccg agaacctatc 7920
caaagaagat ttgtaatgtt gtgctgtatc tcaaagatac ggataaatga aagcaattca 7980
caactcgaga aataatattc tggtttcact tggagttgtg ctgtatctca aagatacgga 8040
taaatgaaag caattcacaa cccgaaaatg gtataaagtt acgttatttt agttgtgctg 8100
tatctcaaag atacggataa atgaaagcaa ttcacaacta atttcgtacg aagtttatac 8160
tcgtttaggt tgtgctgtat ctcaaagata cggataaatg aaagcaattc acaacctacg 8220
t 8221

<210> 12
<211> 7304
<212> DNA
<213> Artificial sequence

<220>
<223> CRISPR-Cas Locus 17

<400> 12
tttgaagcca gaaacaggat gtcattattt ttctggctca taataattct ggctaaaggt 60
tccagccctt tctccttagc tgtatcagct ttggtttttt ttcgcttttt gaaaggcaga 120
taaaggtctt ccagctcctg tatatcaaag ctttcttcaa ttctttgctg aagttcaggc 180
gtcaaagcat tctgttcttc tatcgatttt aaaatggatt cctttctctt aacgatctct 240
tcaaattgct tactgatctt tgaaatctgt tcaatctgta tttcatcaag gtttccggtt 300
ttatcttttc ggtaacggga aataaaagga atggtgcagt cttctgctaa taattgtaag 360
gtattgttga tgctcttttc agaaatattg agctgcttct gtataaattc tacggtcgtc 420
attgttctgt tttcggatcg ctaaaatagg gttttaaaac gaaaaaaggc gagttgcccc 480
gccttgaatg ttttcacaac ggaataatcc ttattgtgaa ttgctttcaa aatttatatg 540
acaaatatag aagaaaaaat atttaataca attatgggaa tccgtaattt gaatgctttt 600
tttatttata ttttaggtga tattcaaaac ataactgtga tgattaaaaa taaacttggc 660
ttagacttgg gaacgaactc cataggatgg gcattgataa gacaggattt tgaaaacaaa 720
tacggagaaa ttcttgggat gggaagcaga attattccga tgtcacaaga tattctgggc 780
gattttggaa aagggaattc tgtttcgcaa accgcagaac gaacaaaata tagaagtgtg 840
agaagattac gtgaacgatt tttattgagg agagaacgac tacacagact tttacatctt 900
ttaaattttc ttccacaaca ttatgcttca caaattgatt ttgaaaaaag attcggaaag 960
tttaaagtgg aaaccgaacc aaaactggct tggaaaaata ttgaaggaaa attttcattc 1020
cttttccaaa actctttcaa tgaaatgctt gaagatttta atgcaaatgg acaggattta 1080
aaaattcctt acgactggac aatttatttt ctccgaaaaa aagcactttc acaaaaaatt 1140
gaaaaggaag aattggcgtg gattctttta aactttaatc aaaaacgtgg atattatcaa 1200
ttgcgtggtg aggaagagga agagaatcct aataaacagg ttgagtttta ttctttaaaa 1260
gtcatagacg ttttagcaga tgaacctcaa aaaggaaaat ctgatatttg gtattctttg 1320
attttagaga atggatggat ttacagacgt tcaagtaaaa ttccattatt tgactggaaa 1380
gataaaacaa gagattttat tgtaaccacg gacttgaatg atgatggaag cattaaaaaa 1440
gataaagacg gaaatgaaaa aagaagtttc cgtgcgccgg gtgagaatga ttggactttg 1500
gtaaaaaaga aaaccgagca ggaaatcgag caatctcaca aaaccgttgg aacctacatt 1560
tacgaagcac ttcttgataa tcccaaacaa aaaataaagg gaaaactgat tcgtaccatt 1620
gaaagaaaat tttacaaaga agaactcaga cagattttag aaaagcaaaa ggaatttcat 1680
caggaattac aaagtgatga tttgtataat gattgtattc gtgagttgta ccgaaacaac 1740
gaatcacatc aacttatttt gagcaagaaa gattttgttc atcttttgat ggaggatctt 1800
attttctacc aaagaccgct gagaagccag aaatcttctg tttctaactg taccttggag 1860
ttcagaaagt ataaagatga aaatggagca gaacatatac aatatttaaa agccattccg 1920
aaatctaatc cttattatca ggaattccgc ctttggcaat ggatctacaa tttaaacatt 1980
tatagaaaag atgatgaagc gaatgttacc aaagattttt taaatacgac ccaagattta 2040
gaaaatctgt ttgagttttt aaatatcaga aaagaaattg accaaaaagg attactaaaa 2100
cattttaaac tcaatgaaaa aaaacatcgt tggaattttg tagaagaaaa gaaatatcct 2160
tgcaacgaaa ctaaaacgat gatttcttct cgattggata aagtggataa tatttctgat 2220
gatttcctga caagggacat tgagcagaaa atttggcaca tcatttattc cgttaatgat 2280
aaagtagagt acgagaaagc cttaaaatct tttgcgagaa agtatcatct ggatgaaagt 2340
tctttttttg aggctttcag aaaatttccg ccatttaaaa gtgaatatgg ttccttttcg 2400
gaaaaagcaa ttaagaaatt gttggctttg atgcgattag gaaaatattg gaactatgca 2460
gatgttgata aattttcaaa agacagaatt cagaaaatca ttaacggaga atatgatgaa 2520
aatattaaag ataaagtgag agaaaaagca ataaatctca catcagaaaa tgattttcag 2580
gggttacaat tgtggttggc tcaatatata atatatggaa ggcattcaga agcatctatg 2640
attggaaaat ggaattctgc ggatgattta gaaatgtttt taaaagattt taaacaacat 2700
tcgcttcgca atccgattgt agaacaggtg attacagaaa ctcttcgtgt ggtaaaagat 2760

atttggttaa aatatggaaa tggagcaaaa gattttttca atgaaattca tattgagttg 2820
ggaagagaaa tgaagcttcc tgcgggtgat cgaaaaaaac tcacgaatca aattaccgaa 2880
aacgaaaaca ccaatctccg catcaaagct ctattggcag aaatgatgaa tgatcattca 2940
gtagaaaatg ttcgtccgtt ttcgcctatg caacaggaaa ttttgaaaat ctatgaagac 3000
ggcgttttaa attcaggtat tgagattgaa gatgaatatt tgaaaataag caaaacggca 3060
cagccttctt cttcagattt aaaaagatat aaactttggc tggaacaaaa atataaatca 3120
ccttataccg ggcaaattat tccattaaat aaattattca caccggaata cgaaattgaa 3180
cacattattc cgcaaagccg atattttgat gacagtttta gcaataaagt aatttgcgag 3240
tcggcggtta ataaattgaa agataattat atcggacttg gatttattaa gcagtttgga 3300
ggaactatta ttgagcttgg tttcgggaaa agtgtaaagg tatttaatat agaagagtat 3360
gaagattttg taaagaaaca ttatgcaaac aatagaggca aaagaaacaa gcttttgatg 3420
gaagatattc ctggaaaaat gattgaacgg caattgaacg atacccgcta tatcagtaaa 3480
tatgtttccg gaattctttc caatattgtt cgggtagaag acggttcaga cgaaggggtt 3540
aattctaaaa acatcgttcc gggaaatgga aaaatcacta cccaactgaa acaagattgg 3600
gggttgaatg atgtttggaa tgatataatt ttaccacgtt ttgaaagaat gaatcaactt 3660
actaattcaa aagattttac cgcctggaat gaaaatcatc aaaagttttt acctaccgtt 3720
ccaattgagt tttccaaagg attttcaaag aaaagaattg accatcgtca tcatgcttta 3780
gatgctttgg tgattgcctg tgccacaaaa gatcacgtga atttattaaa taatcaatcg 3840
gcaaaatcgg ataccaaacg atacgatttg aaaaagaaat tgatgaagtt tgaaaaagta 3900
gtttattatc atacgcaaac aggggagaaa atagaaaggg aaataccgaa acaattttta 3960
aaaccgtggg aaaactttac agttgacgca aaacataatc tggaatctat tattgtaagt 4020
tttaagcaaa accttcgtgt tattaataaa gcaactaatt attacgaaaa gtatgttgaa 4080
aaagacggta caaaaaataa ggaaagagta gagcagaccg gaacaaactg ggcgattaga 4140
aagccaatgc ataaggatac ggtttccgga aaagtagatt taccttgggt gaaagttcca 4200
aaaggaaaaa ttttaacagc aacgaggaaa agccttgata tttcatttga tttaaaatca 4260
atagcatcta ttactgatac agggattcag aaaattctca gaaattatct gaaattcaaa 4320
ggcagtccgg aacttgcatt ttctccagaa ggaatcgaag atctgaataa gaatattgag 4380
aagtacaatg atggaaaatc tcatcaaccg atcaataaag taagagtttt tgaactggga 4440
agtaaatttc aggtaggaca aaccggaaat aaaaaagata aatatgtaga agctgcaaaa 4500
ggaaccaatc tgttttttgc tgtttatgaa aatgaaaata gaaacagaag ttatgaaacc 4560
attcctttga atgaggttat agaaagacaa aagcaaggtt tatctgtggt tgacttaaaa 4620
ggaacaattg atttttttct ttgcccgaat gatttagttt atattccttc agaagatgaa 4680
cttgaaaata gtagcaacat tgacttcaaa aattttaaag aagaacaaaa caaaaaaatt 4740
tataaagtag ttagttttag tggaagtcag ttgtttttta taagacagga aattgccata 4800
tcaattgtaa ataaagcaga attttccacc ttgaataaaa tggagcgtag tattgatggg 4860
ttaatgatta aggaaaattg tatcaaatta aaagtagacc gtttgggaaa tatttctaaa 4920
gcatagtatt atgatcaccc gctccatcta catcggcaat cccgcttatc tcaaactcaa 4980
agatgagcag atgaaaatcc tctgtccgga taccaaaacg gaaaagggga gtgttcctgt 5040
ggaagatttg ggattactca tgctggatca ttatcagatt accatttcgc acaatttgat 5100
tcagaaaatg atgggaaata atgtggtgat cattagttgt gatgcacatc atttacccca 5160
cggaattatg cttccgctgt atggtcatac tgaacattcc gacagggtaa aagatcagct 5220
ggaagcaagt gaaccgctga aaaaacagct ttggaaacaa acggtggaat gtaaaattga 5280
aaaccagaaa aatgttctga tgaaactggg aaactattat gagcctatga ttcaatatca 5340
aaaaaatgta ctgtccggag atacaaccaa tatggaaggt attgcagctc agcattactg 5400
gaagtatcta atcagcctgg attttttaag acagcgtttt ggagattctc ccaatcagtt 5460
tttcaatttc ggttatgctg ttctcagaag tattgtggca agagccattg ttgagacggg 5520
actgctgccc gttcttggta tttttcataa aaataaatac aatccgtatt gccttgcgga 5580
tgatttaatg gaaccttacc gtccgtttgt tgatttactg gtgatgcagt ggctgaagat 5640
tcatcctgat acagaagaac tgtcgaaaga atttaaggct catattcttc agattgcaac 5700
caaagacgtg cgtattgatg acaaaacgag accattgctg gttgcggtaa aaactacggc 5760
ttcgtcgctt tacaaatgtt atataggaga aaagcggctg atctcttatc ctcaactgtt 5820
atgaattccg aaaggtttaa tgcataccga attatgtggg ttttagtatt atatgatctc 5880
ccgactgaga ccaaagctaa tatgaaagat gcgaaccgct tccggaaagg acttattgat 5940
gatggtttta ccctgtttca gttctctatg tatgtacgtc attgtccgag ccgcgaaaat 6000
gctgaagttc atattaaaag agtgaaattc atgcttccaa aagccgggaa agttgctatt 6060
atgtgcatta cagacaaaca attcggggat attgaaattt tctttgccag aaataaagaa 6120
gagcctcctc caacctttca gcaactcgaa ttattttaaa ttgcaaattc taattataaa 6180
aataatctac tgattttcag tagattattt tttgaggttg tgactaatca cgaagataat 6240
aaattttgaa agcaattcac aacgataaag gacacgtaca tgtaggtgat gtggttgtga 6300
ctaatcacga agataataaa ttttgaaagc aattcacaac ttacgtttac gtacggggaa 6360
gattcgttac gttgtgacta atcacgaaga taataaattt tgaaagcaat tcacaacaag 6420
atgcagcgat tattcttaaa gaaatgggtt gtgactaatc acgaagataa taaattttga 6480
aagcaattca caactactat tcgtagacgt gcgtgggaac atgagttgtg actaatcacg 6540

aagataataa attttgaaag caattcacaa ctcagagagt tgagaacatg tataataaat 6600
cgttgtgact aatcacgaag ataataaatt ttgaaagcaa ttcacaactc ttatgtagct 6660
aatcgtaaac agcaaactgt tgtgactaat cacgaagata ataaattttg aaagcaattc 6720
acaacattat ttctttatgt atgcacagac cgccggttgt gactaatcac gaagataata 6780
aattttgaaa gcaattcaca actataggaa tcatcaggag ccaatgtaaa aagttgtgac 6840
taatcacgaa gataataaat tttgaaagca attcacaacc atcacccttt catgaaatgc 6900
taatataatg ttgtgactaa tcacgaagat agtaaatttt ttatttttgc cttgaaacag 6960
tattcctgaa attttaaatg tataaggttc aggctgtttt gattctcaat acagatggaa 7020
tctataccgc aattgggaag tatgattgca aataattgaa gattttcaca tttcttgata 7080
tatgataaga tcatacaaat ctggtttttc tatttttgta cccgaaaaca acaaaatttc 7140
aataaacatt tttaaaacag ttcaatatga aaaaaattag cgtaattgca ttagtaggag 7200
taggattgct tttggcatca tgtgataaag gaaaaacagc agaaactgca gctaccgctc 7260
aggaacaaac cgtagcagaa agcaaaggtg aaacactggc agta 7304

<210> 13
<211> 8221
<212> DNA
<213> Artificial sequence

<220>
<223> CRISPR-Cas Locus 18

<400> 13
tgtgctgcga taatctggat gataagatgg aatgtattta atgtatccaa actgctgaag 60
ttgcttaaaa tatttatgat aactggttat tgtagtgata tgcgaaaact ccataagctt 120
gctcctgctc actctgataa tccatttttc attttgcttg caagcaaggc gggcaattgc 180
agttaacaaa gcaatatgcc aaacattcaa ttttggatca ctttgaattt catcaaggaa 240
atcaaaccag tattgtatat cattcctttt cataatcaaa tagatgttga aggtcttcac 300
aattgtaata atacgagccc aataatttct tgaatcgaat tttgccagtg atcctgaggt 360
tttgtaatgt cgaagcagaa atattcagtt ttctgcgaat taccttactc cgcatccagc 420
caaattcttc atcctctctt gaattagcta gcttagattt tagtaggttc tcaatatccg 480
aaactattag gtttctcaat gctgttaaat catttttcgt tattgtatcc atttttataa 540
attttgattt aattcaaaat ttaaactttg gacttacaga tatcctaaaa gaggaagtca 600
caggcataat ttttctttgt atttcttatt gcctgtgcag gaaatttaga aaagtgagct 660
ttaagtagtc atggaagaac aattctcgaa ccattttgtt gaagatttaa ataatcttaa 720
aggtttgatg tattaaagat gataattcta ataaaaatgc cccgaaggta gtcgaggcgc 780
taaatgtttt cacaacggaa taatccttat tgtgaattgc tttcaaaatt attgatacaa 840
atataaaaga aaaaaaataa ctgtatttat gggaatccat aatttgaata ttttttttat 900
tactattttt atgtggtatc aaaattaata gcgatgaaaa atatacttgg actggacttg 960
ggaacaaact ctattgggtg ggctttagta aaacaggatt ttgaaaacaa acaaggtgaa 1020
attcttggaa tggggaccag gataattccg atgtctcaag atattttggg cgattttggt 1080
aaagggaatt ctgtttcgca aactgcggaa cgtacaaaat acagaagtgt aagaagatta 1140
cgtgaacgat ttcttctacg aagggagagg cttcacaggg ttttaaacat tttgaattcc 1200
cttccagaac actatgcttc acagattgat tttgaaaaac gatttgggaa gttcaaagtt 1260
gaaactgaac caaaattggc ctggaaaaat attgaaggac atttttcatt tctgttccaa 1320
aactctttca acgagatgct tgaagatttt aaaacgaacg gacaggattt aaaaataccg 1380
tacgactgga cgatttatta tctccgtaaa aaggcacttt ctcaaaaaat tgaaaaagag 1440
gagttggcct ggattctttt gaattttaac caaaaacgtg gatattatca attgcgtggg 1500
gaagaagagg aagaaaatcc taataaaatt gttgaatttt attctttaaa agttgtggat 1560
gttctagcag atgagccaca aaaaagcaaa tcagatatat ggtattcttt ggttttagaa 1620
aatggatgga tttacagaag ggcaagtaaa atatcgttgt ttgattggaa agataaaaca 1680
agagatttta ttgtaacaac tgatttgaat gatgatggaa gtgttaaaac agacaaagac 1740
ggaaatgaaa aaagaagttt ccgggcaccg ggtgagaacg attggacttt ggtaaagaag 1800
aaaacagagc aggaaatcga gcagtctcat aaaaccgttg gaatatatat ttacgaaact 1860
ctccttaata atcccaaaca aaaaatcaaa gggaaactag ttcgtacgat tgaaagaaaa 1920
ttctataaag aagaactcag gcagatatta gaaaagcaaa agcagttcca tcaggaatta 1980
caaaatgatg atttatataa ttgttgtatt cgcgagttgt acagaaataa tgaagcccat 2040
caactgactt taagtaaaaa agattttgta catcttttta tggaggatat tgttttctac 2100
caaagaccgt tgagaagcca gaagtcttct atttccaact gtactttgga attcagaaaa 2160
tacaaagatg aaaatggagc agaacataca caatatttaa aagccattcc aaaatcgaat 2220
ccatattatc aggagtttcg tctctggcaa tggattttta atcttaatct atataaaaaa 2280
gataatgatg aaaacgttat caaagaattt ttaaccacta cccaggatat cgaaaatctg 2340
tttgagtttt taaataacag aaaagagatt gatcaaaaag ctctattgaa acattttaag 2400

ctcaatgaga aaacacatcg ttggaatttt gtagaggata aaaaataccc ctgcaatgaa 2460
acgaagacga tgatttcaac tcgattggaa aaagtagaaa atatttccga tgattttctg 2520
accagggaaa tcgaacaaca gatttggcat attatctatt ctgtcaatga taaaatagaa 2580
tacgaaaaag ccttaaaaac ctttgcatca aaacatcatc tagacgaaag ttcttttttc 2640
gaggctttca gaaaatttcc accatttaaa agcgaatatg gttcctattc ggaaaaagca 2700
attaagaaat tgttgcctat aatgcgattg ggcaaatatt gggattatgc caatattgat 2760
cagtcttcaa aagacaggat taagaagatc attaatggag aatatgatga aaatataaaa 2820
gataaggtaa gagaaaaggc agtccatctt acgaatgaaa atgatttcca gggtttacaa 2880
ttgtggctgg ctcaatatat tgtatatgga agacattcgg aagcatcaat aacagcgaaa 2940
tggaattcag ctgatgattt ggaagaattt ttaaaagatt ttaaacacca ctcgcttcgc 3000
aatccaattg tagagcaggt cattactgag actttacgtg ttgtgaaaga tatttggcta 3060
aaatacggaa aaggagcaaa agacttcttc aatgaaattc atatagagtt gggaagggaa 3120
atgaaacttc cggctgaaga tagaaagaaa cttacgagcc aaattattga aaatgaaaat 3180
acgaatctcc gcatcaaagc tctattagct gaaatgatga atgatcatgc tgtagaaaat 3240
gttcgtccgt tttcacccca gcaacaagaa attctgaaaa tttatgagga cggtgtttta 3300
aattcaggca ttgaaattga agatgaatat ttaaaaataa gcaaaacggc gcaaccttct 3360
ccttcagatt taaaacggta taaactttgg ctggaacaga aatacaaatc gccttatacc 3420
gggcaaatga ttccactaaa taaattgttt acgccagaat atgaaattga acatattatc 3480
ccacaaagtc gatactttga tgatagtttt agtaataaaa ttatttgtga atcggcagtt 3540
aacaaattga aggataatta tatcggactt ggatttatta agcagtttgg aggaactatt 3600
attgaacttg gtttgggtaa aagcgtgaag gtttttgata ttgatcaata cgaagatttt 3660
gttaagaagc actacgccaa taaccgaagt aaaagaaata aacttctttt agaagatatt 3720
cctgaaaaaa tgattgaacg tcagatcaat gatactcgtt atatcagtaa atatatttcc 3780
ggtatacttt ctaatattgt tcgcttagaa gatggttcgg atgaaggagt aaattctaaa 3840
aacattgttc ccggaaatgg aaaaattact acccaattga aaaaggactg ggggttgaat 3900
gatgtttgga atgacttgat tttaccccgt tttaaaagaa tgaatcagtt gaccaattcg 3960
acggatttta ccgcctggaa cgaaaatttt caaaagtttt tgccgaccgt tccaattgag 4020
tattcgaaag gtttttctaa aaaaaggatc gaccatcgtc atcatgctct agacgctttg 4080
gtaattgctt gtgctacaaa ggatcacgta aatttattaa ataatcaatc ggcaaaatcg 4140
gaaaccaaac gctatgattt gaaaaagaaa ttaatgaagt ttgaaagagt tgtttatttc 4200
catacgcaaa caggggagaa gatcgaaaga gaagtgccaa aacaattttt aaaaccctgg 4260
aaaaatttta ctgttgatgc tatgcataat ttggaaacgg ttattgtaag ttttaaacaa 4320
aaccttaggg tcattaataa agcaacgaat tattacgaaa aatatgtaga gaaagatggt 4380
gtaagaaata agacaagaat aaagcaaaag ggaataaatt gggcgattag aaaaccaatg 4440
cataaagata cggtttccgg taaagtagat ttaccttggg taaaagtccc aaaaggaaaa 4500
attttaacag caacaaggaa aagccttgac gcttcattcg atttaaaatc aataggctcc 4560
attactgata cagggattca aaaaattctc aaaaattatt taatgcataa agacggaaat 4620
acggaattgg ctttttcacc cgaaggaatt gaagatttga ataaaagtat tgagaagtat 4680
aatgatggta aacctcatca gcccatcaac aaagtaaggg tttttgaact gggtagtaaa 4740
tttcaggtag gacaagccgg aaataaaaaa gataaatacg tagaagccgc aaaaggaacc 4800
aatttgtttt ttggagtgta cgaagataaa aacggcaaga gaagctatga aaccattcct 4860
ttgaatgagg tgattgaaag gcaaaagcaa ggtttacctg tggttgactt aagaagtaca 4920
aatgatttct atttatgccc gaatgatttg gtttatattc tatctgatga tgagcggggt 4980
aatatgacaa atatcgattt tgaaagcctt tctgatgaac aagttaaaag gatatataaa 5040
tttgttagtt gtacaggagg tgagggacat tttattcctt acgcaaatgc gacagaaata 5100
ataaaaaatg aaaatggtac aaatagtaaa agtgagagaa tgcagaattt ctatgatggg 5160
agttgtatgt ttgataaaaa cgccaagccc attatgataa aagaaaactg tgtaaaactt 5220
aaagtcgacc gtttagggaa cattgcaaag atataattat gattacccgt tcaatctaca 5280
tcggtaatcc cgcttatctt aagctcaaag atgagcagct gaaaatcctc tgtccagaaa 5340
ccaaaatgga aaaggggagt gttcctgtgg aagatttggg tttgcttatg ctggatcatt 5400
atcagattac catttcgcat aatttgattc agaagatgat gggaaataat gtggtggtca 5460
ttagttgtga tgcgcatcat ttacctctcg gaattatgct tccactgtat ggtcatactg 5520
aacattctga ccgggtaaaa gatcagcttg aagccagtga accgctgaaa aagcaacttt 5580
ggaaacagac cgtggaatgt aagatcgaaa atcagaaaaa tgttctgatg aagttgggaa 5640
actattatga accaatggtt gaataccaaa caaatgtaaa gtctggggat ttaagcaata 5700
tggaaggtat tgcagctcag cattattgga aatatctgat cagccttgat tttttgagac 5760
agcgtttcgg agaatcaccc aatcagttct ttaatttcgg gtatgcggtt ctcagaagta 5820
ttgttgcaag ggctattgtt gaaacaggat tattgcccgt attgggaatt tttcataaaa 5880
ataaatataa tccttactgc cttgccgatg atctgatgga accttatcgt ccgtttattg 5940
atttactagt aatgcagtgg ctgaaaattt atcctgatgc agaagaactg gcgaaagaat 6000
ttaaggctca tattctgcag attgcaacca aagatgtgcg tattgatgat aaaacaaggc 6060
cattgctggt tgctgtaaaa atgacggctt cgtcgcttta caaatgctat acaggagaaa 6120
aaagactgat ttcttatcct gaactgttat gaacgccgaa aggtttaacg cataccggat 6180

tatgtgggtt ttagtattat atgatctccc gactgagacc aaagcgaata tgaaagatgc 6240
taaccgcttt cggaaagcgc tcattgatga tggtttcacc ttgtttcagt tctcaatgta 6300
tgtacgccat tgtccgagcc gggaaaatgc tgaggttcat attaaaagag ttaaatttat 6360
acttccaaaa gccgggaaag tggccattat gtgtatcacg gacaaacaat ttggagatat 6420
tgaaatcttc tttgccagga ataaagaaga gccacctcca acctttcagc agcttgaatt 6480
attctaaatt ttaaattcta aatataaaaa taatctactg aaaaccagta gattattttt 6540
tgaggctgtg actaatcacg aagataataa tttttgaaag caattcacaa cattccgctt 6600
aatccatttt taaatgactc agctgtgact aatcacgaag ataataattt ttgaaagcaa 6660
ttcacaacac atctttcttg tgtcaattgc accagttggc tgtgactaat cacgaagata 6720
ataatttttg aaagcaattc acaacaacca aagctactgc agttctaaga acatcgctgt 6780
gactaatcac gaagataata atttttgaaa gcaattcaca accgtaagac tgattagtac 6840
tcattgcaat tcgctgtgac taatcacgaa gataataatt tttgaaagca attcacaaca 6900
cgtattacca caatggaaat ttgaaaaatg ctgtgactaa tcacgaagat aataattttt 6960
gaaagcaatt cacaactgtc ccaagcatgg tttctcctaa ttttttgctg tgactaatca 7020
cgaagataat aatttttgaa agcaattcac aacagcatag agcattgtct aaatggaact 7080
ttagctgtga ctaatcacga agataataat ttttgaaagc aattcacaac cagcatcagc 7140
attatctgga ggattctctg gctgtgacta atcacgaaga taataatttt tgaaagcaat 7200
tcacaactaa caggacctat tggtttggtt acagttggct gtgactaatc acgaagataa 7260
taatttttga aagcaattca caacaaggac acattcctaa ctataatcca cagggctgtg 7320
actaatcacg aagataataa tttttgaaag caattcacaa ctgtctgata tgcttactac 7380
ttcacctctt agctgtgact aatcacgaag ataataattt ttgaaagcaa ttcacaacta 7440
ggtcctgaag catcaaatcc ggcattcgct gtgactaatc acgaagataa taatttttga 7500
aagcaattca caacgcgtat gaattataga catacatttc attcgctgtg actaatcacg 7560
aagataataa tttttgaaag caattcacaa cttgcatagt ttgcaggaaa ctaagtcttt 7620
agctgtgact aatcacgaag ataataattt ttgaaagcaa ttcacatgaa ttagtcaata 7680
cttaatctga cagttataat caaattaaat aactttaaaa cagattaagt attatgacaa 7740
cacaacaaaa aatcatcaaa aacaagttag gcgtacttga attagcacaa catttaggaa 7800
atgtatcgaa agcttgtaaa gtaatgggct attcccgaga cagtttttat cgattcaaag 7860
aactgtatga gcaaggaggt gaattagcat tacaggaaat ctccagaaga aagccagtat 7920
taaagaatcg tgtagatgaa gtcattgaaa aggctgttgt tgatatagct attgaaaacc 7980
ctgctttggg gcagcttaga gtaagtaatg aacttaaaaa gaaaggtttc attgtatcac 8040
caggtggagt cagaagtatt tggttaagac acgacctaca tacgtttaaa ctaagattaa 8100
aagccctaga agccaaatcc gctcaagatg gtgtagtcct tactgaatct caactttcag 8160
cactagaaag ggctaaggaa gagaaaaaag ctcatggaga aattgaaact catcatcctg 8220
g 8221

<210> 14
<211> 8221
<212> DNA
<213> Artificial sequence

<220>
<223> CRISPR-Cas Locus 19

<400> 14
tatcagggct ctcatcctca ttattgccta tccatacatt tttaccgtca ttggctaaaa 60
aaatagtaca ggcatttatt ggaatgaaag tacctgctat caaaagctag atatatatta 120
ttttggtttt cattacttta aagataattt tttttaataa atgcttgaca atcttgttgt 180
gattttttat aataaaagaa gctgattacc cattttaaaa ttttagaaag tagaataaaa 240
aacaaaaacc atccttaaaa aaggatggtt tgtagtcatg aaagaacaat tctcgaacca 300
ttttgttgag gatttaaata agcttaaacg tttgatgtat taaagatgat aattctaata 360
aaaatgcccc gaaggtagtc gaggcgctaa atgttttcac aacggaataa tccttattgt 420
gaattgcttt caaaattatt gatacaaata taaaagaaaa aaattaactg tatttatggg 480
aatccataat ttgaatattt tttttattac tatttttatg tggtatcaaa ttaatagcga 540
tgaaaaatat acttggactg gacttgggaa caaactctat tgggtgggcg ttggtaaaac 600
aggattttga aaacaaacaa ggtgaaattc ttggaatggg agtcaggata attccgatgt 660
ctcaagatat tttgggcgat tttggtaaag ggaattctgt ttcgcaaacc gcggaacgta 720
caaaatatag aagtgtgaga cgattacgtg aacgatttct tctacgaagg gagaggcttc 780
acagggtttt aaatattttg aatttccttc cagaacacta tgcttcacag attgattttg 840
aaaaacgatt tgggaagttc aaagttgaaa ctgaaccaaa attggcctgg aaaaatattg 900
aaggacagtt ttcatttctg ttccaaaact ctttcaacga gatgcttgaa gattttaaag 960
cgaacggtca ggatttaaga ataccgtacg actggacgat ttattatctt cgtaaaaaag 1020
cactttatca aaaaattgaa aaagaggagc tggcctggat tcttttgaat tttaaccaaa 1080

aacgtggata ttatcaattg cgtggggaag aagaggaaga aaatcctaat aaacttgttg 1140
aattttattc tttaaaagtt gtagatgttc ttgcagatga gccgcaaaaa agcaaatcag 1200
atatttggta ttctttggtt ttagaaaatg gatggattta cagaagggca agcaaaatat 1260
tgttgtttga ttggaaagat aaaacaagag attttattgt aacaactgat ttgaatgatg 1320
atggaagtgt taaaacagac aaagaaggaa atgaaaaaag aagtttcagg gcaccgggtg 1380
agaacgattg gactttggta aagaagaaaa ccgagcagaa aatcgagcag tctcataaaa 1440
ccgttggaat gtatatatac gaaacgctac ttgctaatcc taaacaaaaa atcaaaggaa 1500
aattagttcg tacgattgaa agaaaatttt acaaagaaga gcttagacag atattagaga 1560
agcaaaagga gtttcatcag gaattacaaa atgatgactt atataatgat tgtattcgtg 1620
agttgtacag aaacaatgaa gctcaccaac tgactttaag caaaaaagat tttatacatc 1680
tttttataaa ggatattgtt ttctaccaaa gaccgttgag aagccagaag tcttctattt 1740
tcaactgtac tttggaattc agaaaataca aagatgaaaa tggagcagaa catacgcaat 1800
atttaaaagc aattccaaaa tcgaatccat attatcagga atttcgtctt tggcaatgga 1860
tttttaatct taacctatat aaaaaagata atgatgaaaa cgttatcaaa gaatttttaa 1920
ccactaccca agatgtcgaa aatctgtttg agtttttaaa caacagaaaa gaagttgatc 1980
aaaaaacttt attgaaacat tttaagctca atgagaaaac acatcgctgg aattttgtag 2040
aggataaaaa atacccctgc aacgaaacaa agacgatgat ttcaacccga ttgcagaaag 2100
tagaaaatat ttccggggat tttctgacga gggaaatcga acaaaagatt tggcatatta 2160
tatattctgt caatgataaa attgaatacg aaaaagcctt aaaatccttt gcgttaaaac 2220
atcaacttga caaaagttct tttttcgagg ctttcagaaa atttccacca tttaaaagcg 2280
aatatggttc cttttcggaa aaagcaatta agaaattgtt gcctttaatg cgattgggta 2340
agtattggga ttatgccaat attgataagt tttcaaaaga caggattcag aaaatcatta 2400
atggagaata tgatgaaaat ataaaagata gggtaagaga aaaggcggtc catcttagga 2460
gtgaagatga tttccagggt ttacaattgt ggctggccca atatattgta tatggaagac 2520
attcggaagc atcaataaca gggaaatgga attcagctga tgacttggaa gaatttttga 2580
aagattttaa acaccacttg cttcgcaatc caattgtaga gcaggtcatt acagaaactt 2640
tacgtgttgt aaaagatatt tggctaaaat atggaaaagg agcaaaagat ttcttcaatg 2700
aaattcatat agagttggga agggaaatga aacttccagc tgacgataga aagaaactca 2760
caaaccagat tactgaaaac gaaaatacga atctccgcat taaagctcta ttaacagaaa 2820
tgatgaatga tcatgctgta gaaaatgttc gtccgttttc gccccagcaa caagaaattc 2880
tgaaaattta tgaggacggc gttttaaatt caggcattga aattgaagat gaatatttaa 2940
aaataagcaa aacggcgcaa ccttttcctt cagatttaaa acggtataaa ctttggctgg 3000
aacagaaata caaatcgcct tatactgggc aaattattcc attaaataaa ttgtttacac 3060
cagaatatga aattgaacac attattccac aaagccgata ctttgatgac agttttagta 3120
ataaaattat ttgtgaatcg gcggttaaca aattgaaaga taattatatc ggacttggat 3180
ttattaagca gtttggagga actattattg aacttggttt cgggaaaagc gtaaaggttt 3240
ttgatattga tgaatatgaa gattttgtca agaagcacta cgccaataac cgaagtaaaa 3300
gaaataagct tctcttagaa gatattcctg aaaaaatgat tgaacgtcag atcaatgata 3360
ctcgttatat cagtaaatat atttccggta tactttctaa tattgttcgc ttagaagatg 3420
gttcggatga aggagtaaat tctaaaaaca ttgttcccgg aaatggaaaa attactaccc 3480
aattgaaaaa agactggggg ttaaatgatg tttggaatga cttgatttta ccccgtttta 3540
aaagaatgaa tcagctgacc aattcgacag attttaccgc ctggaacgaa aattttcaaa 3600
agtttttgcc gaccgttcca attgaatatt ccaaaggttt ttctaaaaaa agaatcgacc 3660
atcgtcatca tgctttagac gctttggtaa ttgcttgtgc tacaaaggac cacgtaaatt 3720
tattaaataa tcaatcggca aaatcggaaa ccaaacgcta cgatttgaaa aagaaattaa 3780
tgaagtttga aagagttgtt tatctccata tgcaaacagg ggagaaaatc gaaagagaag 3840
tgccaaaaca atttttaaaa ccttgggaaa attttactgt agatgttaag tataatttgg 3900
acatggttat tgtaagtttt aagcaaaacc ttagggtcat taataaagca acgaattatt 3960
acgaaaaata tgtagagaag gatggtgcaa gaaataagac aagagtagag caaaagggaa 4020
taaattgggc tattagaaaa ccaatgcata aagatacggt ttccggtaaa gtagatttac 4080
cttgggtaaa agttccaaaa ggaaaaattt taacagcgac gaggaaaagc cttgatgctt 4140
cattcgattt aaaatcaatt gcctccatta ctgatacagg gattcaaaaa attctcaaaa 4200
attatttaat gcttaaagat ggaaaccctg aattggcttt ttcacccgaa ggaattgaag 4260
atttgaataa aagtattgag aagtacaatg atggcaaacc tcatcaaccc atcaataaag 4320
taagggtttt tgaactgggt agtaaatttc agataggaca agccggaaat aaaaaagata 4380
aatacgtaga agccgcaaaa ggaaccaatt tgttttttgc agtgtacgaa gatgaaaacg 4440
gcaagagaag ctatgaaact attcctttga atgaggtgat tgaaaggcaa aagcacggtt 4500
tacctgtggt tgatttaaca agtacaaatg atttctattt atgcccgaat gatttggttt 4560
atattctatc tgatgatgag cgggataata tgacaaataa cgattttgaa aacctttctg 4620
acgaacaagt taaaaggata tacaaatttg ttagttgtac aggaggtgag ggcattttta 4680
ttccttacgc aagtgcgaca gaaataataa aaaatgaaaa cggtacaaat agtaaaagcg 4740
agagaataca gaatttctat gatggtagtt gtatgcttga taaaaacagc aagcccatta 4800
tgataaagga aaactgtgta aaacttaaag tcgaccgttt aggtaacatt tcaaagatat 4860

aattatgatt acccgttcaa tctacatcgg taatcccgct tatctcaagc tgaaagatga 4920
acagatgaaa attctttgtc cggaaaccaa aacggaaaag gggagtgttc ctgtggaaga 4980
tttgggctta ctgatgttgg atcattatca gattactatt tcgcataatc tcattcagaa 5040
aatgatggga aataacgtgg tggtcatcag ttgtgacgct catcatttgc cccatggaat 5100
aatgcttcca ctgtatggtc atactgaaca ttccgacagg gtaaaagatc agttggaagc 5160
aagtgaacca ttgaaaaaac agctttggaa gcaaaccgtg gaatgcaaaa ttgaaaatca 5220
gaaaaatgtt ctgatgaaat tggaaaacta ttatgaacca atggttgaat accaaagaaa 5280
tgtaaagtct ggggatttaa ccaatatgga aggtattgca gctcagcatt actggaaata 5340
tctgatcagc cttgattttt tgagacagcg tttcggagaa tcacccaatc agttctttaa 5400
tttcgggtat gcggttctta gaagtattgt cgccagggct attgttgaaa caggattatt 5460
gcctgtattg gggatttttc ataaaaataa atataatcct tactgtcttg ccgatgatat 5520
aatggaacct tatcgtccgt ttgttgattt actcgtgatg caatggctga aaattcatcc 5580
taataccgaa gaactgtcga aagaatttag ggctcatatt cttcagattg caaccaaaga 5640
tgtacatatt gatgacaaaa caagaccgtt gcttgttgct gtaaaaatga cggcttcatc 5700
gctttacaaa tgctacacag gagaaaaaag attgatctct tatcctgaac tgttatgaac 5760
gccgaaaggt ttaacgcata ccggattatg tgggttttag tattatatga tctcccgact 5820
gagaccaaag cgaatatgaa agatgctaat cgctttcgga aggcgctcat tgatgatggt 5880
ttcaccttgt ttcagttctc aatgtatgta cgccattgtc cgagccggga aaatgctgag 5940
gttcatatta aaagagttaa atttatgctt ccaaaagctg ggaaagttgc cattatgtgt 6000
atcacggaca aacaatttgg agatattgaa atcttttttg ccaggaataa agaagagcca 6060
cctccaacct ttcagcagct tgaattattc taaattttaa attctaaata taaaaataat 6120
ctactggttt tcagtagatt attttttgag gctgtgacta atcacgaaga taataatttt 6180
tgaaagcaat tcacaacttg ttcctgactg aattctatcg atccggtgct gtgactaatc 6240
acgaagataa taatttttga aagcaattca caacaatctc ttctagtggt atccactttt 6300
ttcagctgtg actaatcacg aagataataa tttttgaaag caattcacaa cgctaaactt 6360
cctatcaagc agatcagaac cgctgtgact aatcacgaag ataataattt ttgaaagcaa 6420
ttcacaacgt agaaaaatca tatcctgcag ttggttgggc tgtgactaat cacgaagata 6480
ataatttttg aaagcaattc acaacaaccg atattgatat cggttttttt gtgctgctgt 6540
gactaatcac gaagataata atttttgaaa gcaattcaca acgacagaaa agataatcct 6600
gctcagattg atggctgtga ctaatcacga agataataat ttttgaaagc aattcacaac 6660
taacaattaa aactactatg tcctccatct gctgtgacta atcacgaaga taataatttt 6720
tgaaagcaat tcacaactct tttcatcgtt attaaatttt attttaggct gtgactaatc 6780
acgaagataa taatttttga aagcaattca caacagaata tttcggtgga tcttttcctg 6840
gttggctgtg actaatcacg aagataataa tttttgaaag caattcacaa ctatcgtaaa 6900
cactacaaga tgcattctta tgctgtgact aatcacgaag ataataattt ttgaaagcaa 6960
ttcacaacgt atctttaccc tatggaaaac atggaagagc tgtgactaat cacgaagata 7020
ataatttttg aaagcaattc acaacttcat ctgctttaaa cagattataa cctttgctgt 7080
gactaatcac gaagataata atttttgaaa gcaattcaca actaactttc aatttaggca 7140
taataacaaa ttgctgtgac taatcacgaa gataataatt tttgaaagca attcacaaca 7200
gaaatttcaa taatggatac aatcctttag ctgtgactaa tcacgaagat aataattttt 7260
gaaagcaatt cacaactatc cttagcctta gcagaaagat cagctggctg tgactaatca 7320
cgaagataat aatttttgaa agcaattcac aacgaaattg gatatggtgc tgtaatgaat 7380
tttgctgtga ctaatcacga agataataat ttttgaaagc aattcacaac acatttgaga 7440
agacattaat ggctttgaag gctgtgacta atcacgaaga taataatttt tgaaagcaat 7500
tcacaactac tatccccaaa ttcatcatta gcagcccgct gtgactaatc acgaagataa 7560
taatttttga aagcaattca caacacctat cgttaaggga attagcgact gcacgctgtg 7620
actaatcacg aagataataa tttttgaaag caattcacaa ctttagttct aattgtaaat 7680
gcttcattat tgctgtgact aatcacgaag ataataattt ttgaaagcaa ttcacaacta 7740
cgactacggt gactcttatt catatcacgc tgtgactaat cacgaagata ataatttttg 7800
aaagcaattc acaacgcgac tgcatgatgg caaaacgaaa gcagagctgt gactaatcac 7860
gaagataata atttttgaaa gcaattcaca acagtatgga aaagagaata ccaatttatc 7920
aagctgtgac taatcacgaa gataataatt tttgaaagca attcacaact atcgaggaca 7980
cttgactttt ctgaagtggg ctgtgattaa tcactaagat aataaatttt gaaggcaatt 8040
cacaacatat aagataacaa tttaattata gagttttaag aagttcgcct tttagttgaa 8100
catatattca agcactacta aaaccataca agagatatta caaataaatt tcttaaaaat 8160
caattttcca caattaccta aagaagtaag gagtgcattc gcaatttttg aaaatagttc 8220
c 8221

<210> 15
<211> 6813
<212> DNA
<213> Artificial sequence

<220>
<223>  CRISPR-Cas Locus 20

<400>  15
aataaaaaag caacctgctg taaattagct ggttgctttt gtttggttgt agtcatgaaa      60
gaacaattct cgaaccattt tgttgaggat ttaaataagc ttaaacgttt gatgtattaa     120
agatgataat tcaaataaaa atgccccgaa ggtagtcgag gctctaaatg ttttcacaac     180
ggaataatcc ttattgtgaa ttgctttcaa aattattgat acaaatataa aagaaaaaaa     240
ttaactgtat ttatgggaat ccataatttg aatatttttt ttattactat ttttatgtgg     300
tatcaaatta atagcgatga aaaatatact tggactggac ttgggaacaa actctattgg     360
gtgggcgttg gtaaaacagg attttgaaaa caaacaaggt gaaattcttg gaatgggagc     420
caggataatt ccgatgtctc aagatatttt gggcgatttt ggtaaaggga attctgtttc     480
gcaaaccgcg gaacgtacaa aatatagaag tgtgagacga ttacgtgaac gatttcttct     540
acgaagggag aggcttcaca gggttttaaa tattttgaat ttccttccag aacactatgc     600
ttcacagatt gattttgaaa aacgatttgg gaagttcaaa gttgaaactg aaccaaaatt     660
ggcatggaaa aatattgaag gacagttttc atttctgttc caaaactctt tcaacgagat     720
gcttgaagat tttaaagcga acggtcagga tttaagaata ccgtacgact ggacgattta     780
ttatcttcgt aaaaaagcac tttatcaaaa aattgaaaaa gaggagctgg cctggattct     840
tttgaatttt aaccaaaaac gtggatatta tcaattgcgt ggggaagaag aggaagaaaa     900
tcctaataaa cttgttgaat tttattcttt aaaagttgta gatgttcttg cagatgagcc     960
gcaaaaaagc aaatcagata tttggtattc tttggtttta gaaaatggat ggatttacag    1020
aagggcaagc aaaatattgt tgtttgattg gaaagataaa acaagagatt ttattgtaac    1080
aactgatttg aatgatgatg gaagtgttaa aacagacaaa gaaggaaatg aaaaaagaag    1140
tttcagggca ccgggtgaga acgattggac tttggtaaag aagaaaaccg agcaggaaat    1200
cgagcagtct cataaaaccg ttggaatgta tatatacgaa acgctacttg ctaatcctaa    1260
acaaaaaatc aaaggaaaat tagttcgtac gattgaaaga aaattttaca aagaagagct    1320
tagacagata ttagagaagc aaaaggagtt tcatcaggaa ttacaaaatg atgacttata    1380
taatgattgt attcgtgagt tgtacagaaa caatgaagct caccaactga ctttaagcaa    1440
aaaagatttt atacatcttt ttataaagga tattgttttc taccaaagac cgttgagaag    1500
ccagaagtct tctatttcca actgtacttt ggaattcaga aaatacaaag atgaaaatag    1560
agcagaacat acgcaatatt taaaagcaat tccaaaatcg aatccatatt atcaggaatt    1620
tcgtctttgg caatggattt ttaatcttaa cctatataaa aaagataatg atgaaaacgt    1680
tatcaaagaa tttttaacca ctacccaaga tgtcgaaaat ctgtttgagt ttttaaacaa    1740
tagaaaagaa gttgatcaaa aaactttatt gaaacatttt aagctcaatg agaaaacaca    1800
tcgctggaat tttgtagagg ataaaaaata cccctgcaac gaaacaaaga cgatgatttc    1860
aacccgattg cagaaagtag aaaatatttc cggggatttt ctgacgaggg aaatcgaaca    1920
aaagatttgg catattatat attctgtcaa tgataaaatt gaatacgaaa aagccttaaa    1980
atcctttgcg ttaaaacatc aacttgacaa aagttctttt ttcgaggctt tcagaaaatt    2040
tccaccattt aaaagcgaat atggttcctt ttcggaaaaa gcaattaaga aattgttgcc    2100
tttaatgcga ttgggtaagt attgggatta tgccaatatt gataagtttt caaaagacag    2160
gattcagaaa atcattaatg gagaatatga tgaaaatata aaagataggg taagagaaaa    2220
ggcggtccat cttaggagtg aagatgattt ccagggttta caattgtggc tggcccaata    2280
tattgtatat ggaagacatt cggaagcatc aataacaggg aaatggaatt cagctgatga    2340
cttggaagaa tttttgaaag atttaaaaca ccacttgctt cgcaatccaa ttgtagagca    2400
ggtcattaca gaaactttac gtgttgtaaa agatatttgg ctaaaatatg gaaaaggagc    2460
aaaagatttc ttcaatgaaa ttcatataga gttgggaagg gaaatgaaac ttccagctga    2520
cgatagaaag aaactcacaa accagattac tgaaaacgaa aatacgaatc tccgcattaa    2580
agctctatta acagaaatga tgaatgatca tgctgtagaa aatgttcgtc cgttttcgcc    2640
ccagcaacaa gaaattctga aaatttatga ggacggcgtt ttaaattcag gcattgaaat    2700
tgaagatgaa tatttaaaaa taagcaaaac ggcgcaacct tctccttcag atttaaaacg    2760
gtataaactt tggctggaac agaaatacaa atcgccttat actgggcaaa ttattccatt    2820
aaataaattg tttacaccag aatatgaaat tgaacacatt attccacaaa gccgatactt    2880
tgatgacagt tttagtaata aaattatttg tgaatcggcg gttaacaaat tgaaggataa    2940
ttatatcgga cttggattta ttaagcagtt tggaggaact attattgaac ttggtttcgg    3000
gaaaagcgta aaggtttttg atattgatga atatgaagat tttgtcaaga agcactacgc    3060
caataaccga agtaaaagaa ataagcttct cttagaagat attcctgaaa aaatgattga    3120
acgtcagatc aatgatactc gttatatcag taaatatatt tccggtatac tttctaatat    3180
tgttcgctta gaagatggtt cggatgaagg agtaaattct aaaaacattg ttcccggaaa    3240
tggaaaaatt actacccaat tgaaaaaaga ctgggggtta aatgatgttt ggaatgactt    3300
gattttaccc cgttttaaaa gaatgaatca gctgaccaat tcgacagatt ttaccgcctg    3360
gaacgaaaat tttcaaaagt ttttgccgac cgttccaatt gaatattcca aaggtttttc    3420
taaaaaaaga atcgaccatc gtcatcatgc tttagacgct ttggtaattg cttgtgctac    3480
aaaggaccac gtaaatttat taaataatca atcggcaaaa tcggaaacca aacgctacga    3540

tttgaaaaag aaattaatga agtttgaaag agttgtttat ctccatgtgc aaacagggga 3600
gaaaatcgaa agagaagtgc caaaacaatt tttaaaacct tgggaaaatt ttactgtaga 3660
tgttaagcat aatttggaca cggttattgt aagttttaag caaaacctta gggttattaa 3720
taaagcaacg aattattacg aaaaatatgt agagaaggat ggtgcaagaa ataagactag 3780
agtagagcaa aagggaataa attgggctat tagaaaacca atgcataaag atacggtttc 3840
cggtaaagta gatttacctt gggtaaaagt tccaaaagga aaaattttaa cagcgacgag 3900
gaaaagcctt gatgcttcat tcgatttaaa atcaattgcc tccattactg atacagggat 3960
tcaaaaaatt atcaaaaatt atttaatgct taaagatgga aaccctgaat tggctttttc 4020
acccgaagga attgaagatt tgaataaaag tattgagaag tacaatgatg gcaaacctca 4080
tcaacccatc aataaagtaa gggtttttga actgggtagt aaatttcaga taggacaagc 4140
cggaaataaa aaagataaat acgtagaagc cgcaaaagga accaatttgt tttttgcagt 4200
gtacgaagat gaaaacggca agagaagcta tgaaactatt cctttgaatg aggtgattga 4260
aaggcaaaag cacggtttac ctgtggttga tttaacaagt acaaatgatt tctatttatg 4320
cccgaatgat ttggtttata ttctatctga tgatgagcgg gataatatga caaataacga 4380
ttttgaaagc ctttctgacg aacaagttaa aaggatatac aaatttgtta gttgtacagg 4440
aggtgagggg cattttattc cttacgcaag tgcgacagaa ataataaaaa atgaaaacgg 4500
tacaaatagt aaaagcgaga gaatgcagaa tttctatgat ggtagttgta tgcttgataa 4560
aaacagcaag cccattatga taaaggaaaa ctgtgtaaaa cttaaagtcg accgtttagg 4620
taacatttca aagatataat tatgattacc cgttcaatct acatcggtaa tcccgcttat 4680
ctcaagctga aagatgaaca gatgaaaatt ctttgtccgg aaaccaaaac ggaaaagggg 4740
agtgttcctg tggaagattt gggcttactg atgttggatc attatcagat tactatttcg 4800
cataatctta ttcagaaaat gatgggaaat aacgtggtgg tcatcagttg tgacgctcat 4860
catttgcccc atggaataat gcttccactg tatggtcata ctgaacattc cgacagggta 4920
aaagatcagt tggaagcaag tgaaccattg aaaaaacagc tttggaagca aaccgtggaa 4980
tgcaaaattg aaaatcagaa aaatgttctg atgaaattgg aaaactatta tgaaccaatg 5040
gttgaatacc aaagaaatgt aaagtctggg gatttaacca atatggaagg tattgcagct 5100
cagcattact ggaaatatct gatcagcctt gattttttga gacagcgttt cggagaatca 5160
cccaatcagt tctttaattt cgggtatgcg gttcttagaa gtattgtcgc cagggctatt 5220
gttgaaacag gattattgcc tgtattgggg atttttcata aaaataaata taatccttac 5280
tgtcttgccg atgatataat ggaaccttat cgtccgtttg ttgatttact cgtgatgcaa 5340
tggctgaaaa ttcatcctaa taccgaagaa ctgtcgaaag aatttagggc tcatattctt 5400
cagattgcaa ccaaagatgt acatattgat gacaaaacaa gaccgttgct tgttgctgta 5460
aaaatgacgg cttcatcgct ttacaaatgc tacacaggag aaaaaagatt gatctcttat 5520
cctgaactgt tatgaacgcc gaaaggttta acgcataccg gattatgtgg gttttagtat 5580
tatatgatct cccgactgag accaaagcga atatgaaaga tgctaatcgc tttcggaagg 5640
cgctcattga tgatggtttc accttgtttc agttctcaat gtatgtacgc cattgtccga 5700
gccgggaaaa tgctgaggtt catattaaaa gagttaaatt tatgcttcca aaagctggga 5760
aagttgccat tatgtgtatc acggacaaac aatttggaga tattgaaatc ttttttgcca 5820
ggaataaaga agagccacct ccaacctttc agcagcttga attattctaa attttaaatt 5880
ctaaatataa aaataatcta ctggttttca gtagattatt ttttgagggc tgtgactaat 5940
cacgaagata ataatttttg aaagcaattc acaacagccc cagcgggttt agctccgcct 6000
ttcttgctgt gactaatcac gaagataata atttttgaaa gcaattcaca accaaataga 6060
ttattttcta tagacagctt cagctgtgac taatcacgaa gataataatt tttgaaagca 6120
attcacaaca atttccggat taaactaaag cttttcatcg ctgtgactaa tcacgaagat 6180
aataattttt gaaagcaatt cacaactaat taattattgc tgtgactaat cacgaagata 6240
ataatttttg aaagcaattc acaacgcttt gccacaggaa gtggatatcc atttagctgt 6300
gactaatcac gaagataata atttttgaaa gcaattcaca actagcaata aactgttttt 6360
gaagatctcc ctgctgtgac taatcacgaa gataataatt tttgaaagca attcacaacc 6420
tggtacttgg aaggggtacg ggaaaaacgg ctgtgactaa tcacgaagat aataattttt 6480
gaaagcaatt cacaacggtg aatacaacat gctttctaat gatcttgctg tgactaatca 6540
cgaagataat aatttttgaa agcaattcac aacatttttt cttttttcta tcactggaga 6600
cctgctgtga ctaatcacga agataataat ttttgaaagc aattcacaac ctatgaacct 6660
gtaaatacta agtgtgccag gctgtgacta atcacgaaga taataatttt tgaaagcaat 6720
tcacaacaaa taaaattaag gtactccctt tgccctcgct gtgactaatc acgaagataa 6780
taatttttga aagcaattca caacaaatat ata 6813

<210> 16
<211> 7929
<212> DNA
<213> Artificial sequence

<220>
<223> CRISPR-Cas Locus 21

<400> 16
acagcagcag accatggagc agaagtgttt tttactaact tttcagacga tgacggattg 60
tatatagtaa tcggcattat ttcattgaaa tcgtcactga aatttcccat ccatgtagta 120
taagctctaa tgtaataaac atcttctttt aaatctgcgg gtaagctgat gcttccgctg 180
cctttaccat taatcagggg atagattttt ttgctgattt gtttttattt ttgatcgtac 240
aattcaacga aaacattggt agaaatcgtt gaagggctgt atccgtcaaa tacaaaagag 300
gtaaaccaaa gattttcacc ggcaacataa ttctttttac taagaagaag gtgtattttt 360
tcttgcggat agttttcctc aaatttttta agtgcttctt ctgctttgtt ctgagaaaaa 420
taaaatggag caataattag tgataatgca atcaatggct tttgaaacaa acagatattc 480
attatatggt tcgtgtttta atatatttca aaaatacatt tttttctatt gaaagagaga 540
taaatggtgc tattatttaa ataaaaatgc cccgcagata ggcgaggcgc taaatgtttt 600
cacaacggaa taatccttat tgtgaattgc tttcggatac aaatctaaaa ataatatttt 660
taatcagttt acggttttcc acatttatat gaattaaatt cttattaatt tagaaaactc 720
gattaaaata aataaccatg gcaaaaaata tacttggatt agatttagga accaactcga 780
ttggttgggc gttaatagaa gttgactaaa acaatattcc tatccggatt attgcaatgg 840
gttcacgtat aattccttta tcaacaagtg ataaagaaga atttcaaaga ggtttgtcaa 900
tcactaagaa tcaaagtaga acggtaagcc gtactcagag aaaagggtat gaccggaaac 960
aattgaagaa aagtaattta aagaaaattc taaataaata taatatcttc ctttcagaag 1020
aattattaaa acttccaatg cttgatttgt ggaaattgag aagtgattct gcaaacccaa 1080
atgaaaatat ttcgactgaa caattgggac gaattttata tatgttgaat caaaagcgtg 1140
gctataaatc tgcaagaagt gaagcaaatg ccgataaaaa agatacagat tatgttcagg 1200
cagtaaaagg gcgctacgca cagctgaaag aaagaaatca aacaatcgga caatattttc 1260
atgaagaatt gcataatgca aatgcaaaca attcttattt cagaataaaa gaagaagttt 1320
atccgagaga agcttatata gaagagtttg atacgattat taatgctcaa aaagataaac 1380
atgattttct tacagacgaa gtcgttcatc aattaagaaa tgaaatcatt tattttcaaa 1440
gaaaactaaa atctcaaaaa ggattgatca atatttgtga atttgaaggc tttgagaaaa 1500
cagtttttga caaggacaaa aacaaacaca aagtagtgtc tgtcggtccg aaagttgcgc 1560
cgaaaacatc tccattgttt cagctttgtc gtatttggga agttgtaaat acaattacct 1620
taaaaataaa gaatcctgaa ggaagcaaat ataaatgggg tgagaaaatt ccaaatttaa 1680
ttgagaaaga gattttagct gattatttac ttaaaaatga gagtttaagt tttacgaagc 1740
ttcttgaaat tttagaatta aataaaaatg atatttatgt aaataagcaa atattaaaag 1800
gtataaaagg caatgaaact tatgctgttc ttcagaatat tttaggagat aatgaacttt 1860
taaattttga tgtttcaatt attccatcca attacacttc aattttggtg gacaaaaaaa 1920
cgggagagat attggaagag agagcaggtc tcgaattaga tgcttcatta gaaaaagagc 1980
ctttatacca gctgtggcac accatttatt ccctaaaaga tttagatgaa tgcaaaaatg 2040
ctttaattaa aaggtttgga ttcgatgatg agattgccga aaagttgtct cgtatcaatt 2100
tcaacaaaca agcttttagc aataaatcga acaaatcgat gagaaaaatt cttccttttc 2160
tgatggaagg ctacgattat tctcaatctt gcagtttggc ggggtacaat cattctaatt 2220
cgttgacgaa agatgaaaga gaacagcagg taactttaaa cagattagaa cttcttcaaa 2280
aaaacagttt acgacagcct attgttgaga aaattctgaa tcagatgata aatgtggtga 2340
acgcaattct tgagcaatac ggaaaaccaa ctgaaattag agttgagctc gcacgagacc 2400
ttaagcaaag caaagacgag cgcaacgatg ccgatttaca aaattcaaaa aataaaaaac 2460
tcaatgaaga aattggtaaa cgattggctg aattgggact gcctgctacg aaacgatata 2520
ttcaaaaata taaattcatt tttccatcgc ttagcaaaaa tctaaaagat gctcgcgtta 2580
acaatcaatg tatttattgt ggagaaacat ttaatttaac ggaagcttta agtggtgatg 2640
cttttgatgt tgaccacatt gtcccgaaag ctttattgtt tgatgattca caaaccaata 2700
aagtattggt tcatagaaaa tgtaattcga caaaaaccaa tcaaactgcg tatgattaca 2760
ttgctacaaa aggtgacgaa caagttcgta tttattctga acgaattgat gattggttta 2820
aaagaggaat tatttcttac agtaaaatgc aaagattaaa ggtttcacat aaggaatatc 2880
ttgaaagaaa aaaaatagga aaagaaaccg aaactgataa gaagctttgg gaaaatttta 2940
tagacagaca gttgagagat actcaatata tttcccgaaa atcaagagag attctacaga 3000
aagtatgcaa caatgtaact acaacagaag gcggagttac agcaaaactt cgcaatttgt 3060
ggggttggga tgatgttttg atgaatttgc aaatgccaaa atataaagaa ttagggcaaa 3120
ctgtcatcaa agaatggaca agcgaccacg gaaaaagaaa acatcaaaaa gaagaaatag 3180
aaaattggac aaaaagagat gaccatcgtc atcacgccat tgatgctttg gtaattgcct 3240
gcacaagaca aggttttatt cagcggataa atactttgaa ttctagcgat actaaagatt 3300
taatgatgaa agaagttgaa aaagcaaaaa tcgaatttaa tgaaaagagt acccttttag 3360
aaaaatattt aaagactcaa aagccgttta ctacagaaca agtaatgaaa gaagctgata 3420
aaattttagt ttcatttaaa gcaggtaaaa aagtagcaac aataactaaa tttaaagcta 3480
caggaaaaaa taaagataca ggagtaattg ttccaagagg agcgttacat gaacaaaatg 3540
tttatggtaa gataaaagct attgaaaaag ataagtcttt aaagtatttg tttgaaaatt 3600
cagggaaaat tgtaaatcct aaaattaaaa atttagttga aataagactt tcggaaaatg 3660

aaaatagttc aaaaatggct ttatcaacat taaagaaaaa tcccattttc ctaaatgaag 3720
aaaaaactga tttactagaa aaggcatcat gttatgagta tgttactgtt agaaagtatc 3780
ctttgaacac aataaaagca agtcaggctg atgatattgt tgatgaaaga gtgaaatttt 3840
tagtgaaaga aagattggcc caatttggaa ataaagaaaa agaagctttt aaagatacac 3900
tttggttcaa cgaagaaaaa caaattccga ttattaccgt tcgtttattt gctcgtcctg 3960
atgctaataa tttacaggca atgaaaaagg atgaaaacgg taaagaaata ggatttgtag 4020
tgttgggcaa caatcatcat atcgccattt atgaggataa agacaacaag cttgtgcagc 4080
acagttgtac tttttggcat gcagtggaaa ggaaaaaata taacattcca ccagtaatta 4140
aaaattcctc agaaatttgg aatcaattat taaataaaga attcccagaa acttttttgg 4200
ataaattacc tgcggataat ctcgaattaa aatttagtat gcaacaaaat gaaatgttta 4260
ttctgggatt accacaagaa gaatttgaag aagcaataaa aaataatgat aaatctgttt 4320
tgagtaaaca catgtattta gtttggagta ttgctgacgg agattattgg tttagacatc 4380
atttagaaac aaaaaataca gagttaaaga aaatcaatgg tgcaaaagaa agcaaaagat 4440
ttttcagatt gcaaagtctt aaggctgtag aaaatttgac accaatcaag gtaagactga 4500
atcatttagg agaaataact aaaataggag aatactaacc catgataacc cgttacatct 4560
acatcggcaa tcccacctat ctgaaactca aagacaatca gatgaaaatc ctttgtccgg 4620
aaactaaaat cgaaaagggg agtgtgcctg tcgaagattt gggtttgctg atgttagacc 4680
attatcaaat cacgatttcc catcagctca ttcagaaaat gatgggaaat aatgttgtag 4740
ttgtaagttg cgattcccat catttgccac acggaattat gcttccattg tatggtcatt 4800
cggaacattc agatagggta aaagaccagt tagaagcaag tgaaccgctc aaaaagcaac 4860
tctggaaaca aactatcgaa tgtaaaattg agaaccaaaa agaagttctc cggcgattag 4920
gaaactatta cgaaccgatg cttgagtatc aaaacaatgt taaaagcggt gatattacca 4980
atatggaagg catcgccgca caacattact ggaaatatct catcagtttg gatttcctga 5040
gacagcgttt tggagattca cccaatcagt ttttcaattt cggttatgcc gttctaagaa 5100
gtattgtcgc aagagcaata gtcgaaacag gattgcttcc ggttctcgga attttccata 5160
aaaataaata caatccttat tgtcttgctg atgatttgat ggagccttac cgcccatttg 5220
tagatttgct agtcatgcaa tggcttgaaa tacatcccga aaccgaagaa ttaaccaaag 5280
agttcaaagc ccatatcctc caaattgcca caaaagatgt tcggattgat gacaaaacaa 5340
gacctttatt aatttctgta aaaacaactg caacctcgct ttataaatgc tacaccggcg 5400
aaaaaagaat gatttcctat ccagaattac agtaattttt ttatgggcag cttttccgcc 5460
ttccgctccc aatctttttt gcagacgatt tcacataaaa tagtatttga gagcaagcaa 5520
aaaaggattt ccgctcaagt cgggttgcaa ccattatacc gcaaaacaaa ccatgaaaaa 5580
tgaatgccga aaggtttaac gcttaccgaa ttatgtgggt tttagtatta tacgacttac 5640
caacggaaac caaagccaat atgaaagatg ccaaccgctt tcgtaaaggc ctaattgatg 5700
atggctttac attatttcaa ttttccatgt atgttcgcca ttgcccaagt cgcgaaaatg 5760
ctgaggtgca cattaagcgc acgaagttta atcttccaaa agcaggaaaa gtagccatca 5820
tgtgtattac cgataagcaa tttggcgaca ttgaaatatt ctttgccaga aataaagaag 5880
aaccgccacc caccttccaa caactcgaat tattctaaat tttaaatcct aaaaaacaac 5940
aaaacccatt gaataacaat gggttgaatt ttgaggttgt gttatatctc aaagatactc 6000
aaaaatgaaa gcaattcaca acattatcac gatgattttc aagcggtaaa agttgtgtta 6060
tatctcaaag atactcaaaa atgaaagcaa ttcacaactg tacaagctta cggagagata 6120
caagctttgt tgtgttatat ctcaaagata ctcaaaaatg aaagcaattc acaacttgta 6180
tatcgtgtgg cacaacaaaa aataagttgt gttatatctc aaagatactc aaaaatgaaa 6240
gcaattcaca actgaaggcg ggaacctata cgctagctaa cggttgtgtt atatctcaaa 6300
gatactcaaa aatgaaagca attcacaaca tcgggccgat ggggtacacg atggaacgag 6360
ttgtgttata tctcaaagat actcaaaaat gaaagcaatt cacaacatat tattaaccca 6420
aagggcggcc acgcgggttg tgttatatct caaagatact caaaaatgaa agcaattcac 6480
aacagattaa cgacgcgatc gctaaagctt acggttgtgt tatatctcaa agatactcaa 6540
aaatgaaagc aattcacaac aggagcgcaa acaattgcag tattaactgg gttgtgttat 6600
atctcaaaga tactcaaaaa tgaaagcaat tcacaacgaa gcccgcgaag aggatttaga 6660
tacacttgtt gtgttatatc tcaaagatac tcaaaaatga aagcaattca caacaaaacc 6720
tttatgatgg ctcaacagag ttttgttgtg ttatatctca aagatactca aaaatgaaag 6780
caattcacaa catgtcagca tcttgctcta atgcggcact tgttgtgtta tatctcaaag 6840
atactcaaaa atgaaagcaa ttcacaacga caagcggaat gatcttagta tctaaaaagt 6900
tgtgttatat ctcaaagata ctcaaaaatg aaagcaattc acaactattt agacaaaatg 6960
agggattacg cttcagttgt gttatatctc aaagatactc aaaaatgaaa gcaattcaca 7020
actcaaaaga tagtgggagg tttatttgaa acgttgtgtt atatctcaaa gatactcaaa 7080
aatgaaagca attcacaaca tctgtaatta ccaatagttt tatggatggg ttgtgttata 7140
tctcaaagat actcaaaaat gaaagcaatt cacaacagtc ttcaaacgct gatattaatt 7200
tatctagtgt tatatctcaa agatactcaa aaatgaaagc aattcataac gggcaaatct 7260
gtcatcattt cagttggact gttgtgtaat atctcaaaga tactcaaaat aaaagcaacg 7320
aaatagtgtc acatcccaaa gaatattcat aaaagatatt cctatttttg cgaatgattt 7380
actttctagt ggtgtcaaat attttgactt ttatagtttt cggagcagat aaatggtatg 7440

ccaaaaaaag caaaagaaga gtttcagaac tatttcttct gagtttgact tttttaggag 7500
gtactttggg agcattgtta gcgatgatga tttttagaca taaaacatca aaaaaatctt 7560
ttattcttaa acttcttatc gtaatttttg ttcagctaat tatatttgga ataatgaaga 7620
attctaaaat aaattttaac attttttaat accacaacaa tcggaatagg catcatattt 7680
tcttacatat ctatcataac accaaaaaat attattatgt caaagaaaat tgcaattcta 7740
gcaacaaacg gatttgagga aagtgaattg aaatctccaa aagaacattt ggaacaacag 7800
ggttggacgg ctcatattat tagtccagaa tcgggtacca taaaagcatg ggcagaaaaa 7860
gattggggaa aagaatacaa tgttgataaa acaattagtg aatcgagcgc atcagattat 7920
gatgctttg 7929

<210> 17
<211> 6931
<212> DNA
<213> Artificial sequence

<220>
<223> CRISPR-Cas Locus 22

<400> 17
gtataaatct tccagctcct gaatatcaaa gctttcttca attctttgtt ggagttcagg 60
tgttaaagcg ccctgttcat ctatagattt tacaatagat tcctttcttt tgacaatctc 120
ttcaaactgc ttgctgatct ttgagatctg ttcaatctga acttcatcca aattccctgt 180
tttatccttt cggtaacggg aaataaaagg aatagtgcag tcttctgcta ataattgtag 240
ggtattgttg atgctctttt cagagatatt aagctgcttc tgtataaatt ctacggtcgt 300
cattgttctg ttttcggatc gctaaaatag ggtttttaaa aagaaaaaag acgagttgcc 360
ccgccttgaa tgttttcaca acggaataat ccttattgtg aattgctttt caaaattatt 420
aatacaaata tagaagaaaa aaatttacca taactgtgga aatccgtaat tcagatattt 480
tttttatttc tatttttatg tagtatcaaa agttagaatg atgattaaaa atatacttgg 540
attagactta ggaaccaact caattggttg ggcattaata gaagttgatg aaaataatat 600
tcctatccga attgttgcaa tggggtctag aataattccc ttgtcaacaa gtgataaaga 660
ggagtttcaa agaggtttat caattacaaa gaatcaaagt agaacggtaa gtcgtaccca 720
gagaaaaggt tataatagga aacaattaaa gaaaaatgat ctaaagaaaa ttctaaataa 780
atacaatatt tttccatcag aagaattgtt aaaacttcca atgcttgatt tgtggaaatt 840
aagaagtgac tctgctaatc ccaatgaaaa tatttctgca gaacaattag gacgtattct 900
ttatatgctt aatcagaaaa gaggttataa atctgctaga agtgaggcta atgctgataa 960
aaaagataca gactatgttc aggaggtaaa aggccgttat gctcagttga aagagagaga 1020
gcagactatt gggcagtatt tttatgaagg gctttctacc gctttttcag acaaagataa 1080
tcggcagtat tttagaatca aagaagaagt ttatccaaga gaagcttacg tagaagagtt 1140
tgatactatc attaatgctc aaaaagataa gcatgatttt cttacagata aagtagttca 1200
tcaattaaga aatgaaatca tttattttca aaggaagctg aaatctcaga aaggattagt 1260
tagtatctgt gaatttgaag gttttgagaa aactgttttt gataaggaaa aaaacaaaaa 1320
taaagtggtg tttattgggc cgaaagtagc tcctaaaacg tctccattgt ttcagctttg 1380
ccggatttgg gaagtcgtca atacgattac tttaaaaata aaaaatccag aaggaagtaa 1440
atataaatgg ggtgaaaaaa ttccggattt aaaggagaaa gaaattttag cagattattt 1500
atttaaaaac gaaagtgtaa gttttagtaa acttctcgaa attttaggat taaaaaaaga 1560
aaatgtatat gtgaataagc aaatcctgaa aggtattaaa ggaaacgaaa catatgcagc 1620
tctttataat gttttaggcg ataatgaact tttgaatttt aatgtatcta tcattacttc 1680
aaatcatact gcgatattga tagataagaa aacaggcgaa attcttgaag aaagagctgg 1740
ccttgaactt gatgcatttt tggaaaaaga gccgttatat caactgtggc ataccattta 1800
ttccctgaaa gacttagatg aatgtaaaaa tgcgttaatt aaaaggtttg gtttcgaaga 1860
agacattgcc gaaaaattat cttgtatcga ttttaataaa caggctttcg gtaataaatc 1920
gaataaatcg atgagaaaaa ttcttccttt tctgatggag ggctatgatt attctcaatc 1980
ctgcagcttg gcgggctata atcattctaa ttctttaact aaagatgaaa gagaacaaca 2040
aattacttta gaccgattag aacttctttc caaaaatagt ttgagacaac ctattgttga 2100
gaaaattctg aaccagatga ttaatgtagt taatgcaatt cttgagcaat atggaaaacc 2160
atctgaaatt agagttgaac ttgcccgtca acttaaacaa agtaaggacg agcgaaatga 2220
aactgatttg caaaactcta aaaataaaaa gctcaatgag gaaattggaa aacgattagc 2280
ggaattggga ctgcctgtaa ccaagcgtta tattcaaaaa tataaattca tttttccatc 2340
acttagtaaa aacctgaaag atgctcacgt caataatcaa tgtatttatt gtggagaaac 2400
cttcaattta acagaagctt tgagtgggga tgcttatgat gttgaccata ttgtaccgaa 2460
aactttatta tttgatgact cacaaaccaa caaagttttg gttcatcgaa aatgtaactc 2520
gacaaaaact aatcaaactg cgtatgatta cattgctaca aaaggagatg aacaggttag 2580
ggtatactca gaacgggtgg atgactggtt caagagagga attatctctt acagtaaaat 2640

gcaaaggttg aaagtttcac acaaagaata tcttgaaaga aaaaaacttg gaaaagaaac 2700
agagacggat aagaagcttt gggaaaactt catcgacagg cagttgagag atactcaata 2760
tatttctcgc aaatcaaggg aaattttgca gaaagtgtgc aataatgtaa caacaacaga 2820
aggtggagtg actgcaaaac tgcgtaatgt atggggttgg gatgatgttt taatgaatct 2880
tcaactgcct aaatataaag aattagggca aactttcacc aaagaatgga caagcaaaca 2940
tggaaaaaga aaacaccaaa aagaagaaat cgaaaactgg acaaaaagag atgatcatcg 3000
ccatcacgcc attgatgctt tgataatagc ttgtacaaaa caaggcttta ttcaacgaat 3060
aaatactttg aattcgagtg agacaaaaga tttgatggta aaagaagttg agggagctaa 3120
aatagaattt aatgaaaagg ctacgctttt agaaaaatac ttaaaaactc agaaaccttt 3180
tgatacacat gaagtaatga aagaagctga taagatttta atttcattta aagcaggaaa 3240
aaaggtagca acaataacta aatttaaagc aacaggaaaa aataaagaga cgggggtaat 3300
tgttccgaga ggagctttac acgaacaatc tgtttatggt aaaataaaag taattgagaa 3360
tgataaacct ttaaaatact tgtttgaaaa ctcagataaa attgtaaatc caaaaattaa 3420
gaatcttgtt gaaacaaggc tcacagaaaa tgaaaataat tttaaaaagg ctttatcaac 3480
attaaagaaa aatccaatct tttcaatgaa agaaaaaacc gagatattag aaaaagcatc 3540
ttgctacaat gatgccactg ttttgaaata taagttacaa aatataaaag caagtcaggc 3600
cgatgatatt gttgatgaaa gagtgaaatc tttagtaaaa gaaagacttg ctcaatttgg 3660
aaataaagaa aaagaagctt tcaaggatat tctttggctt aatgaagaaa aacaaattcc 3720
gattataacg gttcgtttgt ttgcccgtcc agatgcagat aatttacagg ctattaaaaa 3780
agataaatat gggaatgaga taggattcgt tgtgacggga aataatcatc atatcgcaat 3840
ttatgaagat aaagacaata agcttgttca gcatacttgt acattttggc acgcaataga 3900
aagaaaaaaa tacaatattc ctgctgttat tggaaattca tcagatattt ggaaccaatt 3960
attagacaaa gaacttccaa tatcattttt gaataaatta ccgatggata agctcaattt 4020
aaaatttagt gtgcagcaaa atgaaatgtt tattctgggg ttatcacaag acgaatttga 4080
tgagacaatt aaaaataatg ataaatcttt attgagtaaa tatttgtatt tggtttggag 4140
tattgcagaa ggtgattatt ggtttagaca ccatctggaa acaaagaaca ctgagttaaa 4200
gaaaactgat ggagcaaagg aaagtaaaag atattatcga ttaagttcaa aaggttttat 4260
tgacttaaat ccaataaaag tcagactgaa tcatttagga gaaattacca aaataggaga 4320
ataataatta tgatcacccg ctccatctac ataggcaacc ccgcttatct taaacttaaa 4380
gatgagcaaa tgtatatttt ggagccttcc acaaaagaaa tgaaaggtaa agttccggtg 4440
gaagatatgg gtttgttgat gttggatcat ttccagatta ctatatcgca tcaattgatt 4500
cagaaaatga tgggaaataa tgtggtagtg gtgagctgcg atagccacca tctgccacac 4560
ggaattatgc ttccgctgta tgggcacact gaacattctg atcggatcaa ggatcagttg 4620
gaagccagtg aaccactcaa aaagcaactt tggaaacaaa ctgttgaatg taaaattgaa 4680
aaccagaaag aagttttaaa acgcttagga aattattacg acccgatgat tgattatcag 4740
aatagcgtga aagtggcgga tattaccaat atggaaggga ttgcagcaca acattactgg 4800
aagtatctca tcagtttgga ttttttaaga cagcgttttg gagattcacc caatcagttt 4860
ttcaattttg ggtattcagt tctaagaagc attgttgcaa gagcgattgt agagaccgga 4920
ttacttcctg ttctcggaat tttccataaa aataaataca atccttattg tcttgccgat 4980
gacctgatgg aaccttatcg cccttttgtt gatttattag taatgcaatg gcttgcactt 5040
aattcagaaa ctgaagaatt aactaaagaa tttaaagctt tcattttaca gattgcaacc 5100
aaagatgtga aaatagatga taaaaccagg ccattattgg ttgcagtaaa aacaacagct 5160
acatcactgt ataaatgtta tacgggagaa aaacgtctga tctcttatcc tgatctgata 5220
tgaatgccga aaggtttaat gcataccgaa ttatgtgggt tttagtatta tatgatttac 5280
caacagaaac taaagccaat atgaaagacg ccaaccgctt tcgtaaatca ttgcttgatg 5340
atggctttac cttatttcag ttttcaatgt acgtaagaca ctgtccaagt cgtgaaaacg 5400
ctgaagttca tattaaaaga gtaaaattta tgcttcccaa agccggaaaa gtggccatta 5460
tgtgtattac ggataaacag tttggagata ttgaaatatt cttcgccaga aataaagaag 5520
aaccaccgcc aactttccaa caactcgaat tattctaatt tttaaattct aaataaataa 5580
taaatccgtt gattttcaac ggattaaatt ttgaggctgt gactaatcac gaagataata 5640
atttttgaaa gcaattcaca acactgcatg aactgcttta aaaacaccat ttgctgtgac 5700
taatcacgaa gataataatt tttgaaagca attcacaacc tgttccaagg caggaagaaa 5760
aaacggcctg ctgtgactaa tcacgaagat aataattttt gaaagcaatt cacaacagtt 5820
taataagttc tttgcagcct gttttggctg tgactaatca cgaagataat aatttttgaa 5880
agcaattcac aacccttgac ttaatgcatt gttgtaaaga cgggctgtga ctaatcacga 5940
agataataat ttttgaaagc aattcacaac taaacaacaa gagtcaaagt cttgtgtgct 6000
gctgtgacta atcacgaaga taataatttt tgaaagcaat tcacaactct tgcgcgtaaa 6060
tattgactaa tacaacagct gtgactaatc acgaagataa taatttttga aagcaattca 6120
caacaaattg aattaggttt ggatatcgga ataggctgtg actaatcacg aagataataa 6180
tttttgaaag caattcacaa ctgtagaagt tagtaaccaa caacgtagtg cgctgtgact 6240
aatcacgaag ataataattt ttgaaagcaa ttcacaacca agatgtaaat gttcaatatg 6300
ttcagaaggc tgtgactaat cacgaagata ataatttttg aaagcaattc acaacagaag 6360
caatcttatc tgtatccgaa cttttgctgt gactaatcac gaagataata atttttgaaa 6420

gcaattcaca accccgacga tgtccttttg cctggatgaa ttgctgtgac taatcacgaa 6480
gataataatt ttatgctgtc aattttaaaa atatacaata aaaaccaaaa tttttcattt 6540
cttgatgtat gttaaggtca cagaaataaa gtttctttac ttttggaatc ttaaaataca 6600
aactttttta aacaattcaa tatgaaaaaa attagcgtaa ttgcattagt aggagtagga 6660
ttgcttgtag catcatgtaa taaaggaaaa tcagcagata ctgcagctac agctcaggag 6720
cagaccgttg cagaaagcaa aggagaagta ttggcggtag atgctgcaac ttctgtagta 6780
aactggaaag catatcacaa aggaggtatg gcacctcgtt ggggaacact tagcgtaaaa 6840
tcaggagata tcagtgttga tggaggtcag gtttctgcag gaaactttgt aatcgatatg 6900
aactctatca aagtagatcc tgcttcagta a 6931

<210> 18
<211> 8221
<212> DNA
<213> Artificial sequence

<220>
<223> CRISPR-Cas Locus 23

<400> 18
cataatggtt ttgttgcttt cgtacaaact cgaatcatgg taatcgctct aaatctctta 60
caatggattc aatctcgctt tcagtgatat tttcggagac gtaggcagaa cgtaaatagt 120
tttttaaatc ttccttaagc aacacttgtt cactaacgat tatgtccgta aaccgttggt 180
gttcctctaa tcatttggtt tatgaacatg aggttttaat gtaggatgct taggcttcgt 240
ttcggggtta actctccttc ttaaatcatc ttttccttct tctgtctttg gttttggtcc 300
tggtttaatt gcttgaattg tttccataat aaatactttt ttaaaaatta gttactaaac 360
ccatctttta gaagggcttt gagacaaata acagtattag ggttttaagt acattacgga 420
aaaccttttt acaagtaaat aattataaaa cttactttat ttactcattc taattcaagt 480
ggatctatta ataatagaag tatgctattt aattaaaaaa tagcaattac aattaggtgc 540
tattttattg cattcaattt gtgcttgcga aacatattaa ataaaatcca tagaaaaagg 600
cgagtctccc cgccttaaat gttttcacaa cggaataatc cttattgtga attgcttcaa 660
aattgtattg caataataca ataaaatatt attctataat tatggttttc cataaaactg 720
ttaattttct tttgattaaa tttaaaaatt aatttagata accagaaaat cattataaaa 780
aatagtttga aatgaaaaaa atattaggat tggacttggg gacaaatagt attggatggg 840
ctttgattga aattgataaa aataatattc ctattagcat tattgcaatg ggttctcgaa 900
taatacettt atcatctagt gataaagaag agtttcaaag aggtttgtca ataacaaaga 960
atcagagtag aacggttagc cgcactcaaa gaaaaggata tgaccggaaa caattaaaaa 1020
aaagtgatct aaagaaaatt ctaaataaat acaacatttt tccttcagaa gaattattaa 1080
aacttccaat gcttgatttg tggaaattaa ggagtgattc tgctaatcca aatgaagata 1140
tttctgccga acaactaggc cgtatattgt atatgctaaa tcagaaaaga ggctataaat 1200
ctgctagaag tgaagcgaat gctgataaaa aagataccga ttatgtacaa gctgtaaaag 1260
gacgttatgc tcatttgaag gataggactc agactattgg ccaatacttc tttgaagaat 1320
tagacaatgc aaatactaat aattcttatt ttagaataaa agaagaagtt tatcccaggg 1380
aagcttatac agaagaattt gacgccataa ttaatgctca aaaaggtaaa cacgactttc 1440
ttaccgatga agttgttcat caattaagga atgaaatcat ttattttcaa agaaaactga 1500
aatctcaaaa aggattagtg agtatttgtg aatttgaagg ttttgagaaa actattttcg 1560
ataaggaaaa agacaaaaat aaagtggtac ttgttggtcc aaaagttgct ccaaaaacct 1620
ctcctctatt tcaactttgc cgtatttggg aggttgtcaa tacaattaca ttaaaaataa 1680
aaaatccaga aggaagcaaa tacaaatggg gtgaaaaaat tccaaattta aaagagaaag 1740
aaattttagc agattatttg tttaaaaatg aaagcttaag ttttagtaag cttcttgaaa 1800
ttttagaatt aaaaaaagaa aatgtttatg tgaataagca aatccttaaa ggtcttaaag 1860
gaaacgaaac ttattctact atccatagta ttttaggaga taatgaactt ttaaattttg 1920
atgtgtctat cattacttca aatcatacat caatgttggt agacaaaaag acgggagaaa 1980
ttttagaaga aagagcgggt cttgaacttg atgcatcatt agagagagaa cccctatatc 2040
aattgtggca tacaatctat tcattgaaag atttagatga atgtaaaaat gccttaatca 2100
aaagatttgg ttttgatgat gaaattgcta aaaaattatc tcgtattgat tttaataaac 2160
aggctttcgg aaataaatcg aataaatcga tgaggaaaat tctacctttt ctgatggaag 2220
gctacgatta ttctcagtct tgcagtttgg ctggttataa tcattctaat tctttaacga 2280
aggatgaacg agaacaacta atcactttag accagttagc actccttcct aagaacagtc 2340
tcagacaacc aattgttgag aaaattctga atcagatgat taatgttgtt aacgcaattc 2400
ttgaacaata tggcaaaccc tctgaaatta gagttgaact cgcacgtgaa cttaaacaaa 2460
gtaaggacga gcgaaacgat acagatttac aaaattcaaa aaataaaaaa ctcaatgagg 2520
aaatcagaaa acgattggtt gaactgggat tacctgtaac taaacgatat attcaaaaat 2580
ataaatttat ttttccttct cttagtaaaa atctgaaaga tgctcacgtt aacaatcaat 2640

gtatttattg tggagaaaca tttaacttaa cggaagcttt gagtggtgat gcctacgatg 2700
ttgaccatat tgtgccgaaa gctctattat ttgatgattc tcagaccaat aaagttttag 2760
ttcacagaaa atgtaattca acaaaagaca aattaacagc atacgattat atttctacaa 2820
aaggaataga gcaagttaga gtatattctg aacgtgtgga tgattggttc aaaagaggta 2880
ttatttctta tagtaaaatg caaaggttga aagtttcaca cgaagaatat ctggaaagaa 2940
aaaagatagg aaaagaaaca gagactgata aaaaactttg ggaaaacttc atcgagagac 3000
aattgagaga tactcagtat atatctcgta aagcaagaga aattttacat aaattatgca 3060
acaatgtgac tacaacagaa ggcggagtta cagcaaaact tcgtaattta tggggttggg 3120
atgatgtttt aatgaatcta caaatgccta aatacaaaga attaggacaa accgtcatca 3180
aagaatgggc aagcgatcat ggaaaacgaa aacaccaaaa agaagaagtc gaaaactgga 3240
caaaaaggga tgaccaccgc catcacgcca ttgatgcttt ggtaattgct tgtacaaaac 3300
aaggttatat tcagcggata aatactttga attcaagtga taccagagat ttgatgataa 3360
aagaagtgga agaagcgaaa atagaattta atgaaaagtc aacactttta gaaaaatatt 3420
taaaaactca agagccgttt actactcaag aagtgatgaa agaggctgat aagattttaa 3480
tttcttttaa agctggcaaa aaagtagcaa caataactaa atataaagct acaggcaaaa 3540
acaaaaatac tggtgttctt gtcccaagag gagctttgca cgaacaaaac gtttatggca 3600
aaataaaagt gattgaaaag gataaatctt taaaatacct attcgaaaat tcagataaaa 3660
tagtaaactc tagtattaaa aaacttatag aagtaaggct tttggataat gaaaataatt 3720
ctaaattggc tttatcctct ttaaagaaaa acccgatttt tctaaatgaa gaaaaaaaag 3780
aagtattaga aaaagcgtct tgttatgagt atgtcactgt taaaaaatat cccttgaaca 3840
caataaaagc caaccaagct gatgatattg ttgatgaaag agtgaagttt ttagtaaaag 3900
aaagattatc tcaatttgga aataaagaaa aagaagcttt taaagatacg ctttggttta 3960
atgaagaaaa acaaattcct attattactg ttcgtttatt tgctcgtcca gatgcaaata 4020
atttgcaggc aataaaaaaa gataagaacg gtaaagaaat aggatttgta gtgacggaaa 4080
acaatcatca tattgcaatt tatgaagaca aagacaataa acttattcag cacagttgta 4140
cattttggca tgcagtagaa agaaaaaaat acaatgttcc aaccgttatt aaaaattcat 4200
ctgatgtttg gaatcaatta ttacacaaag aattaccaga atcattttta aataaattac 4260
ctgctgatta tctcaattta aaattcagta tgcaacaaaa tgaaatgttt attctgggct 4320
tatcacaaga agaatttgat caagcaatta aaaagaataa taagccctta ttgagtaaac 4380
atttgtattt agtctggagt atcacagatg gagattactt ttttagacat catttagaaa 4440
ctaaaaatac agaattgaag aaaacagacg gagcaaagga gggtaaaaga tattttcgat 4500
taagtacaaa aggcttacag atttaaatcc gataaaaagt tagattaaat catttaggag 4560
aaataactaa aataggagaa tattagttat gataaaacga acccttttct tcggtaatcc 4620
tgcttatctg agtaccaaga atgagcaaat tctaatttct tatccagaca aggaacagga 4680
gagtaaaaca gttgctattg aagatattgg agtaatcgtt ttggaaaacc aacaaattac 4740
cattactaat ggattattag aaaaattgac ccgtaataat gtagcattga ttaactgtga 4800
tcaacagcac ttgcctattg gtttattgat gcccttaagc ggacataccg aacaaacgga 4860
acgttttaaa aaccaaatca atgcatctgt cccgcttaaa aagaatttgt ggcaacaaac 4920
cattagttcc aaaatatcta accaagcagg actgctcaaa gaaaaaggaa ttccgtgccg 4980
aaaaatggaa ctatgggcaa aagaagttac ctcaggtgat gcactcaatc acgaatcgag 5040
ggcagccgta ttttattggc aaaacttgat tacagtagaa aatttcaccc gaggacaaaa 5100
aggaattcct cctaataatt tattgaatta tggctatgct attttacgag cgattacggc 5160
aagagcgatt gttagttctg gaatgttacc caccttagga attttccatc gcaacaaata 5220
caatgcttat tgtctagccg atgatattat ggaaccctat cgcccttatg tcgatttgat 5280
tgtttgccat atcatggaaa ctgaagattc ttacgacgaa ttaactatcg aaattaaaaa 5340
gcaattacta ggtattgcca ctattgatgt gtttatagat ggaaaaaata gcccgttaat 5400
ggtagccatg agccgcacaa ccaattcgct acacgaatgt tttgaaggaa cagctaggaa 5460
aattttatac cctttgtatg tatgatgaac attacagccg tttaaaccaa tatagaagtt 5520
tgtggatatt agtatttttt gatctgccca ccgaaacacg caaggagcgt aaaattgcta 5580
gtggatttcg caagaaatta ttagacgatg gtttttctat gtttcaattt tcgatctata 5640
tgcgtttttg tgccagtaga gaaaatgccg aagtacatag caaaagaatt aaaaatagtt 5700
taccggagca cggcaaaatt ggcgtgatgc aaatcaccga caaacaattt ggcatgatgg 5760
agctttttta tggtaaaaaa ccagtagaac cagaaaaacc ctctcaacaa ttggaattgt 5820
tttaattttg ctagaataaa aaaatcgatt agaacactat cgttctacct cttacccctt 5880
attctttttc catttctaca actacttata actgccaaca tatcaaccga ttaaatcgag 5940
gtatgttggg aatcataagt aaaaatacaa ttttgaaagc aattcacaac aaacaagaag 6000
gaattaacac agctatcgaa gttgggaatc ataagtaaaa atacaatttt gaaagcaatt 6060
cacaaccaag tacccatttt cgcgtagcaa gagccgttgg gaatcataag taaaaataca 6120
atttgaaag caattcacaa cgtgcaatga aaaaggtgga tgttcaggac tgttgggaat 6180
cataagtaaa aatacaattt tgaaagcaat tcacaactgg aatggaccaa ggattcaacg 6240
atcctacgtt gggaatcata agtaaaaata caattttgaa agcaattcac aacaggtgta 6300
gggttaactc cgacaggttt taggttggga atcataagta aaaatacaat tttgaaagca 6360
attcacaact acaaacaaat gattcaagtg aaaattttag ttgggaatca taagtaaaaa 6420

tacaattttg aaagcaattc acaacattct ttaaatactt tcttaatatc tcttagttgg 6480
gaatcataag taaaaataca attttgaaag caattcacaa cgtgcaatga aaaagggtga 6540
tgttcaggac tgttgggaat cataagtaaa aatacaattt tgaaagcaat tcacaacaaa 6600
gctactatac ttcacttttt tataatgttg ggaatcataa gtaaaaatac aattttgaaa 6660
gcaattcaca acagatgaaa gttctatttt gaaaaattat agttgggaat cataagtaaa 6720
aatacaattt tgaaagcaat tcacaactgt ctgagaattt aatcatttct tttcgaagtt 6780
gggaatcata agtaaaaata caattttgaa agcaattcac aactaaattt gaagcgttta 6840
aaaatacccc accgttggga atcataagta aaaatacaat tttgaaagca attcacaaca 6900
gaaactaagt gttttacttt tccgtcgagt tgggaatcat aagtaaaaat acaattttga 6960
aagcaattca caacgaaagc tcgtaatgtc gaaacagctg ttttgttggg aatcataagt 7020
aaaaatacaa ttttgaaagc aattcacaac ataagtttgt tgaacaatac caacgagaat 7080
gttgggaatc ataagtaaaa atacaatttt gaaagcaatt cacaactggg tatccatgaa 7140
agaaatgaat taatagttgg gaatcataag taaaaataca attttgaaag caattcacaa 7200
ctcatcagcg ggtctttctc ctgctgatag ggttgggaat cataagtaaa aatacaattt 7260
tgaaagcaat tcacaacatt ttagatattg ctaaaaaaaa tcctaaggtt gggaatcata 7320
agtaaaaata caattttgaa agcaattcac aactcttcgg gtgttggtct gtaaatttta 7380
agagttggga atcataagta aaaatacaat tttgaaagca attcacaaca caatcaaaac 7440
aaatcaatct ggcaacacag ttgggaatca taagtaaaaa tacaattttg aaagcaattc 7500
acaacagaaa ccgttaacgg tggaacaagt caaacgttgg gaatcataag taaaaataca 7560
attttgaaag caattcacaa caattttgtg aacatggttt attttatgca agttgggaat 7620
cataagtaaa aatacaattt tgaaagcaat tcacaaccat ccaaactgcg tttagccact 7680
tcggtatgtt gggaatcata agtaaaaata caattttgaa agcaattcac aactagttgt 7740
agatgtttta tcagcatcta catgttggga atcataagta aaaatacaat tttgaaagca 7800
attcacaaca cagcggcgtc gatgttgttg tttagttcag ttgggaatca taagtaaaaa 7860
tacaattttg aaagcaattc acaacataat atgataaaat atatcaaaaa gtgagttggg 7920
aatcataagt aaaaatacaa ttttgaaagc aattcacaac gaatggggtg taaatgattt 7980
ttttgcattg ttgggaatca taagtaaaaa tacaattttg aaagcaattc acaacgcaaa 8040
aagctgtgtg ttgttgatga agaaagttgg gaatcataag taaaaataca attttgaaag 8100
caattcacaa caaaatagct attgaaaaag cacgtgattc gttgggaatc ataagtaaaa 8160
atacaatttt gaaagcaatt cacaactaat tttaggatca tcttctaaag tgaattgttg 8220
g 8221

<210> 19
<211> 8221
<212> DNA
<213> Artificial sequence

<220>
<223> CRISPR-Cas Locus 24

<400> 19
ataaatacct gtcttctgat aaaatggagg gacgttttcc tggtacaaaa ggcaataatg 60
aagcggcatc ttacattaaa agatatttca aaaaatatgg tcttaaggaa ttcaacagaa 120
attactatca aacttttaaa ctatttgtaa aagaaggaat caataaagtg aaatcggata 180
gtgtcaatac tcaaaatgtt gtagggtatc tcgaaggttc tgatgaaaag ctaaaaaatg 240
aatatatagt aataggggct cattatgatc actggggctg gggaggacaa aattcgggaa 300
gtaaaaagaa agaagctttc gccattcata acggagcaga tgacaatgca tccggggttt 360
cggctttgtt gtgtatactt caggaattat caaatgctaa agtcaaacct aaaaggagta 420
ttatttttat atcttttagt ggggaagaag aaggcttatt gggttctaaa tattttgtgt 480
ctcatttgcc ggtagaaaaa aatgccgtga aagtaatgct gaatatggat atggttggaa 540
ggcttaatga ggagaagcaa atttacatgg gcggtgcagg aacatttccg aatggagtag 600
aattgatgaa gaaattgggt gaaaacagta ccctgaatcc ggtcatacat gctggtgaag 660
ttggaggctc agatcatgta tcattttata aagctgctat ttctgctata ggatttcata 720
caggaggcca cccgcaatat catacacccg aagacgatat agatttaata aatattcagg 780
gcggtgcttt ggtatctcaa tatatttaca atgctttagt agcaattgca aatgactcgc 840
aacaactgta ttttattaat caggattagt aattctcggg ttagatccta ctatacaaac 900
aatctattta cggtagatat tctactgatt tgtataaatg aaaagtccca ccgaagcagg 960
actaaagatt ttcatctacg gcatatagcc ttattgtgat aagattaaag attagaaatt 1020
ttaatcagtt acaaatatat ataaaaagga gattaagtgg aatatggtaa tccgtaattg 1080
tgttaatttt ttttatatta tatttcgttc atattaaatt ttaaaagata tggcgaaaat 1140
tttaggattg gatttaggga caaactcgat tgggtgggcg attgttgata aagatggaaa 1200
tgatttttcc cttgtagata agggagtgag aatattttcc gagggagtaa aatctgaaaa 1260
aggaattgaa agttctcgtg ctgcggaacg aactggttat agaagcgcaa gaaaaattaa 1320

ataccgcaga aaattaagaa agtacgaaac cttaaaagtt ctttcctcaa atggaatgtg 1380
tcctttgtcg gttgaagaag ttgaagactg gaaaaaatcc gggtttaaaa aatatcctct 1440
taatcctgaa tttttgaaat ggttacgaac ggatgaatac caacatataa acccatacta 1500
ttttagagat agagcaagca agcaaaaagt tgccttattt gaattgggac gtgctttata 1560
tcatattgct caaagaagag gttttttgag taatcgtcta gatcagtcag ctgaaggagt 1620
ttttgaagaa cataatcctc aaatccaaag tttgattgaa gatttggatg attcgaaatt 1680
aattcttgat gaattgaaaa attattatgt tgataaagga ataattgaag aagctgaaaa 1740
aggagttttt aaaaaagatt tagatgaagg tgagaaaaaa ctaaaatcac tttataattc 1800
gttagtagga ataattaaga agaatgaaaa caacgttgaa aaatgcaaag aggaatttat 1860
tgcaaggctt aataaaaaag aagatttagg aaaagtaaaa ggaaaaatca aagatatttc 1920
gcaagcaatg cttgatggta attttaaaac tttaggacaa tattttttca gtctttacaa 1980
taaaggaaaa ataagaaatc aatatacttc gagagaagaa cattatttgg aagagtttat 2040
tactatttgt aaaattcagg gaatcgaagg aattgatgat aaagaaaaat tacctgaaaa 2100
gaagtttaca ggcttagcca aagatttgta caaagccatt ttctttcagc gtcccttaaa 2160
atctcaaaaa ggactgattg gaaagtgttc ctttgagaaa agcaaatcac gttgtgccgt 2220
ttcgcatccc gattttgagg agtacagaat gtggacttac ttaaacacta tcaaaattgg 2280
aacacaaagc gaaaaaacat tacgtttttt aacgcaagat gaaaaattaa aattagttcc 2340
aaaattctac agaaaaaatg attttaattt tgaagtttta gccaaagaac ttgttgaaaa 2400
aggagcaagt ttcgggtttt ataaatcttc caaaaagaac gatttttttt attggtttaa 2460
ttacaaacca actgattcgg ttgcagcttg tcaagtttca gcatctttaa aaaatgtgat 2520
tggtgaagac tggaaaacaa aaatatttac ttataaaact ttaaatgcaa aagaagaaga 2580
agtaacaaaa tctgttgatt ataaagactt atggcactta ctttccgtag caacatcgga 2640
tatttatttg gaggaatatg ctaaagataa attagggttt gacgacaaaa atgccaaagc 2700
tttcagtaaa ataaaactca aaaaagattt cgctagtttg agttcatcag cgatcacgaa 2760
aattttgcct tatgtaaaag aaggactttt gtattctcac gctgttttta tggcaaatat 2820
tgaaaatatt gttgatgaaa atatttggaa taatcttgac caaagaaatt atattcagtc 2880
taaaatagcc gaaattattg agggctatac ttttgaaaaa acaggtttgg aagtagtcaa 2940
cagccttatt aaagaatata aaacagaaaa tgattacagc aaaaaagtat attattcaaa 3000
agaagctgaa ccagaatttg aaaaagattt aaagcaaaaa ttagtagttt tttataaatc 3060
aaatgacatt gaagatgaaa atataaaaca agctattttt aatgatttat attcagtttt 3120
aattgaccaa cttaaaaaat atgagtatat caaaatacaa cgattggatg agaaagtttt 3180
agagttttta aaaggtgaaa atgaagacgg agaagttttt tgcaataatc caaagcaatt 3240
aaaaaagctg tatcatccat cagatattga aatttttaaa aagcaaaagg tcaaagataa 3300
agatggtaaa gaaacgggac agcttatttt aggtagccct ttaacttctt caatcaaaaa 3360
tccgatggca atgcgtgctt tgcatcaatt gcgtaaagtt ttaaatactt tgattttgga 3420
aggtcaggta gatgaaagta caaggattca tattgaaatg gctcgtgagt taaacgatgc 3480
caaccgaaga aaaggaattc aggattatca aaaagagaat aaagatttca gagacaatgc 3540
tattaaagaa atcaaaaaac tgtatttgga cgaatgtggt aaagaagtag aaccaacgga 3600
agatgatatt ttaagatatc agctttggat tgaacaggac aaatgtgaga tttacgaaca 3660
aggcaagaat attagtattt gtgatataat aggaagtaat ccagcttatg atatagaaca 3720
tacaattcca agaagtattt cgcaagataa ttctcaaatg aataaaacgc tatgcagtca 3780
gcgatttaac agggatgtca aaaagcaaaa aatgccaatt gaattgagca atcatttgga 3840
aattttgcca agaattgcgc attggaaaga agaagccgac aacttatcaa gggaaattga 3900
gataattagc cgttccataa aagccgcatc gaccaaagaa attaaagata aaaaaatacg 3960
aagaagacat tatctgactt taaaacgtga ttatcttcaa gggaaatacg atcgatttat 4020
ttggaaagag ccaaaggttg ggttcaaaaa cagtcaaatt ccggatacag gaattatcac 4080
caaatatgcg caagcttatt taaaatcgta ttttaaaaga gtagaaagtg taaaaggcgg 4140
aatggttgcc gagttcagaa aacaatgggg aattcaggag agttttattg acgaaaacgg 4200
atttaaacaa tacaaagaaa aagatagaag taaacacacc catcatacca ttgatgccat 4260
tactattgct tgtatgacca aagacaaata tgatgttttg gctaaagcct ggacttctga 4320
agatgaacaa gataaactaa aggcaaaagc tctaatggca gatgccaagc cgtggaaaac 4380
ctttaaagaa gatttactca aaatagaaga tgaaatttta gtttcacatt acacgcccga 4440
taatgtaaaa aaacaatcta aaaagattat tagggttcgt ggcaaaaaac aatttattgc 4500
agaaattgag agagatgtca aaggtaaagc aatacaaaaa aaagatgcta atggaaaagt 4560
agtttacaag ttaaatgata aaggagaaaa gatcccaaga ttgcagcaag gtgatactat 4620
tcgaggctcg ttacaccaag atagtattta tggagcaatt aaaaatccat taaatactga 4680
tgaaatacgt tatgtgattc gtaaagattt agaaagtctg aaagcaagtg atatcgaaaa 4740
cattgtggat gaaactgtaa aagagaaagt aaaacaagcc gttacaaata aagttttgtt 4800
gctttcttcc aatgcccaac aaaaaaataa aattgccgag aatgaaaagg tttggatgaa 4860
taaagaaaaa caaatttcca ttaacaaagt tagaatttat gccaattcag ttaaaaaccc 4920
actggaaata aaagagcata gtttattgtc aaaatctcgt cacgaacaca agcaaagagt 4980
atatggtcag aatgatgaga attatgcaat ggcactttat gagttagatg ggaaaagaga 5040
atttgaattg attaataatt ttaatttggc taaactcata aagaatggac aaggatttta 5100

tcctttaaat aaagaaaaag aattaaaagg taaaaaagtt ttatatccga ttgcaaaaag 5160
taacagcaga gatgttgttt tgaaacgagg acaacaagtt gttttttttg ataaagaagt 5220
tgaaaagcca aaggatattt ctgaaatctt ggattttaaa agaagggttt atataattga 5280
aggtttgtcc attcaaagaa taaaatcggg gaataatgaa tttgaatatg gtgtaattat 5340
gttaagatat ttcaaagaag caaggaaatc agatgaaatc aaaaaagata attttaaacc 5400
agatggtgtt tttaaattag gagaaaataa accaacaaga aaaatgaatc acagccaatt 5460
taccgctttt gttgaaggta ttgattttaa agtttaccca tcaggaaagt ttgtcaaaat 5520
ttgaaaaaaa atattatgga tggatcaaaa ttaacaaaag aacaaatcgc aaaaattcaa 5580
gcaaaaattg ataaagcgga aaaaagcggt tttagtacca aaacaaaaga agagttatta 5640
gctgacatca agaaaaaata taatgtttag tttgtacttc cttcgtcgga tgacaaacaa 5700
atgcaaaatg ttctttgata taaagttttt caaactgtta aacatctcaa accacaactg 5760
aaagttcacg tccctgccgg gcggatgtca gaactttcag ttgtggtttg attaaagatt 5820
agaaaacacg atattgcagt attatacgtt aataggaacc gaaggttgtg gtttgattaa 5880
agattagaaa acacgatatt tagcaatgta taattaagta agtcagagtg gttgtggttt 5940
gattaaagat tagaaaacac gatattttta tatgcttcta tttccaaact caatacgttg 6000
tggtttgatt aaagattaga aaacacgata tttaaatgaa aaacacgaca aactacaact 6060
cagttgtggt ttgattaaag attagaaaac acgatattag atataatgtt ttgcttattg 6120
tctttcaagt tgtggtttga ttaaagatta gaaaacacga tatttttaat ctatttctat 6180
tttcttcaat atgagttgtg gtttgattaa agattagaaa acacgatatt aatgtggatt 6240
ttgaggaatt tgtctcttta gttgtggttt gattaaagat tagaaaacac gatattacaa 6300
atcagaagca ttaaacgcag aaacagttgt ggtttgatta aagattagaa aacacgatat 6360
ttcaatttga aaatgtgggt acgtttattg tgttgtggtt tgattaaaga ttagaaaaca 6420
cgatattagt aactgaaaaa ttgaataaag aaaatatgtt gtggtttgat taaagattag 6480
aaaacacgat atttcatttc acgcacgtat aaaggcgcac ttagttgtgg tttgattaaa 6540
gattagaaaa cacgatatta acaaacagga agcactaaat gcagaaacag ttgtggtttg 6600
attaaagatt agaaaacacg atattttaaa caaaaacaaa taactatgaa gtatgttgtg 6660
gtttgattaa agattagaaa acacgatatt tctattactc ctgcagctaa accacataag 6720
ttgtggtttg attaaagatt agaaaacacg atatttacaa atacgcacat cgagaggtgg 6780
cataagttgt ggtttgatta aagattagaa aacacgatat tatcttatag gtgaaagatt 6840
cgattcaaca ggttgtggtt tgattaaaga ttagaaaaca cgatatttct ttgtatccaa 6900
cgttaggggc gacattagtt gtggtttgat taaagattag aaaacacgat atttttattc 6960
gttacattaa catatgaaac taagttgtgg tttgattaaa gattagaaaa cacgatattt 7020
ttaaagtttt aacgtcgtca gtggtagatg ttgtggtttg attaaagatt agaaaacacg 7080
atattatttt ccccgttgtt actcttgttt ccatagttgt ggtttgatta aagattagaa 7140
aacacgatat tagactatgc aaccgtaaca gccaatttta ggttgtggtt tgattaaaga 7200
ttagaaaaca cgatattaca aagaaatgaa atcagaatgg gaactagttg tggtttgatt 7260
aaagattaga aaacacgata ttaacacaaa cgggatagga ttaaaaatgg agttgtggtt 7320
tgattaaaga ttagaaaaca cgatattttc tactactaac ataagccaat tccaatcgtt 7380
gtggtttgat taaagattag aaaacacgat attattgttt atacgttcaa cttctgattt 7440
gttgttgtgg tttgattaaa gattagaaaa cacgatatta acattaactt ccactaatac 7500
aggtacaggg ttgtggtttg attaaagatt agaaaacacg atattgttaa ttttattagc 7560
aaagaatgtt ttatagttgt ggtttgatta aagattagaa aacacgatat tcatgtacaa 7620
caaaaaagaa agcagctaat ggttgtggtt tgattaaaga ttagaaaaca cgatattcta 7680
cgaacttttt actcatagca agttctgttg tggtttgatt aaagattaga aaacacgata 7740
ttagtttcct cctgggttta taggaggaaa agttgtggtt tgattaaaga ttagaaaaca 7800
cgatattgaa ctttataaaa cagttcagta ccaagctgtt gtggtttgat taaagattag 7860
aaaacacgat atttttttca catttttaaa cagtgtttaa atggttgtgg tttgattaaa 7920
gattagaaaa cacgatatta tttataaacg aaacccctaa ctcaataggg ttgtggtttg 7980
attaaagatt agaaaacacg atattaaaac tgagtaactc caccgctttc acttgttgtg 8040
gtttgattaa agattagaaa acacgatatt cttaatgcag agcttacaac ttcgtttaaa 8100
gttgtggttt gattaaagat tagaaaacac gatattttta tagttcaatg tttcgccacc 8160
attaacgttg tggtttgatt aaagattaga aaacacgata ttaatgtctg aaatattagc 8220
t 8221

<210> 20
<211> 8221
<212> DNA
<213> Artificial sequence

<220>
<223> CRISPR-Cas Locus 25

<400> 20

agtgtaccga atacttgata gtgatggaaa acccatcggg gttcctatta aagccagtga 60
cttttataat aaaccgactc ttaaattttt agagcaaaaa tttctatccg ccggtagtgg 120
aagagtatcg atgaaaaaca ggataaagaa tgccgtggac ctggcgcttc ttaataaaag 180
catttcattt tccgacctgg gaaaactgct cgaaaaagaa gggattaata ttatttcaag 240
aaaaagtgct gccgggatga tttatggcat cacctatgtg gatcatacta caaaatgcgt 300
atttaatgga agcgttttgg gcaggcagta cagcgcaaaa gcagtacagg aacgctgtaa 360
tttaatgggt aaaactctgc ggggttccga taacgacata aacataaaat ccaaagctga 420
attaatggga agtatatcga ataaagattt atttgaaagt agtaaaactg atggctttga 480
tactgctctg gctaaagttt tggatgtatt gacccggatg gaatccgcgc aggcttacct 540
gcccaatcag cttaaagcaa aacgcaggaa gaaaaaaagg aaagggcagt cagataatca 600
ataaaaaatt aatatcatgg gaatgcagac aggagaaaat gaacaggcat tgagaaagat 660
attggatatg acaaggctca tagggattat tattctggta attcactttt actattattg 720
ttattcagcc ttcaagcagt ggcagctggt aagtgagttt accgataaaa ttttaggcgg 780
tatttatact tgtaattcag tccgatcagc tttcttacta gttctttttc cgtatagata 840
tcccgtgtag tttaatatat ttgactaatt ttattcaaaa atctaaataa ggtcctctgc 900
aaaacaatca gataccatac aactattcaa atctgtcttt agaggtcgta atgatgtatt 960
tgccgttcgt tgggaaaaag ctggaaagtc tgggtatatg ccagcatatc aatatgacct 1020
gtataattat cgacaacata caatgaatgg agttatggga tttttttggc gaaccgcctt 1080
tccttcccga actttgtttc agaattaagc cttatagatt ttgcaaatct caaggttata 1140
caaaacagtt atttcagtat aaaaaaagtc ccaccgaagc aggactaaag attttcatct 1200
acggcatgta gccttattgt gataagatta aagattagaa attttaatca gtcacaaata 1260
tagagaaaaa atattatggt agaaaaagga aaaccgtaat tgtcttaatt ttttttatag 1320
taaattccgt gcagattaaa tttaagaaat atggcaaaga ttttaggatt agatttaggg 1380
acgaactcga ttggttgggc gattgtagat aaagacgaaa ataatttttc attagtagat 1440
aaaggagtca gaatattttc tgaaggagtt aaatctgaaa aaggaattga aagttctcgt 1500
gctgctgaac gaacaggatt tagaagtgca aggaaaatta aatatcgaag aaagctaagg 1560
aaatacgaaa ctttaaaagt tctgtcatca aatggaatgt gtccactttc aattgaagaa 1620
gtggaagact ggaaaaaatc aggttttaaa caatatccac ttaatcccga attcctgaaa 1680
tggttaagaa ctgatgaggc tgaaaacatc aatccttatt tttttagaga taaagccagc 1740
aaacaaaaag ttcctttgtt tgaattagga cgagcattct atcatattgc tcagagaaga 1800
ggttttttga gtaatcgttt agatcaatct gcagaaggaa ttttagagga acattgccct 1860
aaaattgaaa caattgtaga agatttgaat tcttatgaag aagttttaga aaatttaaaa 1920
gattactttt tttttgaaac tggaattctt gatgctaatg aaaaaagtgg ttatgcaaaa 1980
gatttagatg aaggtgataa aaaattggtt tctttataca aatcacttca taccattgta 2040
aaaaagaatg atactaattt tgaaaaaggt aaagcagaaa ttattgaacg actcaacaaa 2100
aaagatgttt taggaaaagt aaaaggaaaa attaaagata tttctcaagc tatggtcgac 2160
ggaagtttta agactttagg gcaatacttt ttcagtttat atagcaaaga aaaaattaga 2220
aatcaatata cttcaagaga agaacattat ctcgaagaat tcataactat ttgtaaagtc 2280
caaggaatcg attccattaa cgagaaagaa caattgccag agaagaaatc cgatggttta 2340
gccaaagatt tatacaaagc catattcttt caaagaccat taaaatctca aaaaggattg 2400
attggtaaat gttcttttga aaagagtaaa tcccgctgtt ccgtttcgca tcctgatttt 2460
gaagaataca gaatgtggaa ttacattaat acaattaaaa ttggaacaca aagcgaaaaa 2520
acattgcgat ttcttacgca agaagaaaag ttaaaattag ttccaaaatt ctacagaaaa 2580
aacgatttta attttgaagt tttagccaaa gaattaattg aaaaagggac gagctttgga 2640
ttttataaat cttctaaaaa gaacgattat ttttattggt tcaattttaa accaaccgat 2700
tcggtttctg cttgtcaggt ttctgcatca ttaaaaaata ttattggaga agattggaaa 2760
acaaaaacat tcacttacca aactttaaat gttaaaaaag aagaagttac aaaaacagtt 2820
gattataaag atttatggca cttactttca gtagcgactt ctgatattta tttgaataaa 2880
tatgcaaaag ataaattagg ttttgaggat aaaaacgcaa aagcattcag taaaatcaag 2940
ttaaaaaaag attttggaaa tttaagtttg tcggcaatta ctaaaattct gccttacata 3000
aaagaaggat tgctctattc tcacgctgtt tttatggcaa atatagaaaa cattgttgat 3060
gaggaaattt ggaaagatga acatcaaaga aaatatattc aaacccaaat agcagaaatt 3120
gttgaaaatt atacttttga gaaaagtcaa ttagaggtaa ttaatggact tataaaagat 3180
agtaaactaa aagaagaata ttattctaaa gaagcagaat caatttataa aaaagattta 3240
gctactaaat tagttttgtt ttttaaatac aatgttattg aaaatgaatc agaccaaaaa 3300
atcatttttg aaaacttatt tccaattttt attgagcaat taaaaaaata tgagttcatc 3360
aaaatccaga gattagatga aaaagtttta gaatttctta aaggagaaaa tcaagatggt 3420
gaaatatttt gcagcaatcc aaaacaatta aaaaaactct atcatccatc ggatattgaa 3480
gtttttaaaa agaaaatcat taaagacgaa tttgggaatg aaaaagtagt tttaggtact 3540
cctttaacat ctgctattaa aaacccaatg gcaatgaggg ctttgcatca attacgtaaa 3600
gttttaaata ctttgatttt agaaggtcta gtagatgaaa atacactaat tcatattgaa 3660
atggctcgcg aattaaacga tgctaacaaa cgaaaaggaa ttcaggatta tcaaaacgaa 3720
aacaaaaagt tcagagaaga tgcaataaaa gaaatcaaaa aactttattt ggatgaatgc 3780

aaaaaagaag tagaacccac agaagatgat attttaagat accaattgtg gattgaacaa 3840
aacagacaag agatttacga acaaggaaaa aatattagta tttgtgatat aattggaagt 3900
aaccctgctt atgatattga acatacaatt ccaagaagcc gctctcaaga caattctcag 3960
atgaataaaa ccttgtgtag tcaacgattt aatagagaaa ttaaaggaaa taaaatgcca 4020
attgaattga ataatcattt ggaaatcttg ccaagaattg cgcattggaa agaagaagcc 4080
agtaatttat caagagaaat tgaaattata agtcgttcca taaaagccgc tgcaaccaaa 4140
gaagtaaaag ataaaaaaat aagaaggaga cattatctaa cattaaagcg agattatctt 4200
caaggaaaat atgaccgatt tattttggaa gaaccaagag ttggatttaa aaacagtcaa 4260
attcctgata cgggaattat tactaaatat tctcaggctt acttaaaatc gtattttaaa 4320
agagtcgaaa gcgtaaaagg cggcatggtt gccgaattta gaaagcagtg gggaattcaa 4380
gaaagtttta ttgacgaaaa tggatttaag cattataaaa taaaagacag aagcaagcat 4440
acacatcaca caattgatgc cataacaatt gcttgtatga ccaaagataa atatgacgtt 4500
ttggctcacg cttgggcatt agaagatgaa caaaataaaa aagaagccaa agctattata 4560
gaaaaagcta aaccttggaa aacctttaaa gaagatttgt taaaaatcga agaagaaatt 4620
ttagtctcac attatacacc cgacaatgta aaaaaacaat ctaagaaagt tattagaatt 4680
cggggtaaaa aacaacacgt tgccgaaatt gaaagagatg aaaaaggaaa ggcaattcct 4740
aaaaaagatg ctaacggtaa aattctttac aagttagata aagaaggaaa aaaacttcca 4800
agatatcaac aaggcgatgc cattagaggt tctttgcatc aggatagtat ttatggagca 4860
attaaaaacc ctttaaatac ggaagaaatt cgttatgtta ttcgtaaaga tttagaaagt 4920
ttaaaagcat ctgatattga aaatattgtt gatgaaactg taaaagaaaa aattaaagaa 4980
gcagttgcaa ataaagtctt gttattttct tcaaatgccc aacaaaagaa taaaattaga 5040
gagaatgaaa aggtttggat gaacgaagaa aagcaagttc caataaacaa agttagaatt 5100
tatgctaatt ctgtaaaaaa tcctttggag gtaaaagaac atagtttatt gtcgaaatca 5160
cgtcatgagc ataaacaaaa ggtatatggg caaaatgatg agaattattg tatggcaatt 5220
tatgaattag aaggcaaaag ggaatttgaa ttaattaata attttaattt agcaaagttg 5280
attaagggag gacaaggatt ttatcctttt tataaagaaa aggaggtaaa aggaaaagta 5340
gttcaagttc ctattgctaa aaggaatggt agagatttaa ttttaaaaag agggcaacaa 5400
gtaatttttt atgataaaga tgttgaaaat cccgatgatg tttttgattt agtggattta 5460
aaaggaagaa tttatattat tgaaggattg tcgattcaac gccaagttgt ttctggaaag 5520
ctttatgagt ttggtagtat tatgtttcgt tattttaagg aagccagaaa agcagatgag 5580
attaaaaaag atagttttaa acccgatggt acttttaaat caggagaaat aaaaccgact 5640
cgtaaaatga atcataatca atttaccgct tttgtagaag gaattgattt taaagttttg 5700
ccatcaggta agtttattaa aatttaaaaa tttattatgg atgattcaaa attaacaaaa 5760
gagcaaattc aatctatcca agcaaaaatg gatagagatg aaaaaagtaa acccgtaaag 5820
aaatgctaat cgaattttaa aagagataag ggttggttta aaaatttaaa aagttctttg 5880
atataaagtt tttcaatctc ttaaacatcc aaaccacaac agaaaattat ggcgtccgac 5940
catcagggac gcaagcgggc aagttgtggt ttgattaaag attagaaaac acgatatcct 6000
acgccaaaat acggttcaac agcttcgggt tgtggtttga ttaaagatta gaaaacacga 6060
tatcagtagt caggaaacgc cttaatttcg atacgttgtg gtttgattaa agattagaaa 6120
acacgatatc ttaaaaatca tacttccaga gcctacagta gttgtggttt gattaaagat 6180
tagaaaacac gatatcgatt ttgcaaaagc ataaactcct cctacagttg tggtttgatt 6240
aaagattaga aaacacgata tcaatggaga gcctggaacc ttaactcctg aagttgtggt 6300
ttgattaaag attagaaaac acgatatccg ctatatttat tttagaaact gaagcaaggt 6360
tgtggtttga ttaaagatta gaaaacacga tatcccttct ccggtgaacg ccagagctgc 6420
ggcagttgtg gtttgattaa agattagaaa acacgatatc atgtatgtat atgtatcacc 6480
tatcacctat gttgtggttt gattaaagat tagaaaacac gatatccaaa tcaaaggata 6540
tttattacct gatatcgttg tggtttgatt aaagattaga aaacacgata tctttttcat 6600
tattactgag cttaaaacat gggttgtggt ttgattaaag attagaaaac acgatatccg 6660
atgcagatat aaccagccaa caaagctggt tgtggtttga ttaaagatta gaaaacacga 6720
tatccacttg ttacaatatg cgaatgaggc gttagttgtg gtttgattaa agattagaaa 6780
acacgatatc aaaattcgtt ttcattccgc ttttagccgt gttgtggttt gattaaagat 6840
tagaaaacac gatatcccaa cggtaaatta atacgtaaaa catcatgttg tggtttgatt 6900
aaagattaga aaacacgata tcggagcata acgttaatat tgtaacgtat aagttgtggt 6960
ttgattaaag attagaaaac acgatatctt atggatatcg caatcgagga gactattggt 7020
tgtggtttga ttaaagatta gaaaacacga tatctttatg ccgtttcgtt ctgccatttc 7080
ccacgttgtg gtttgattaa agattagaaa acacgatatc tacaaataag ttttaaacgt 7140
gcaactttta gttgtggttt gattaaagat tagaaaacac gatatcttta ccatagtggg 7200
agccgggttt cgatacgttg tggtttgatt aaagattaga aaacacgata tctaagtctg 7260
agacgttaac aaccctgtag agttgtggtt tgattaaaga ttagaaaaca cgatatcatt 7320
taatatggat gaaatggtta ctgttaggtt gtggtttgat taaagattag aaaacacgat 7380
atccgttatt ttatttaata ataaacaaat atagttgtgg tttgattaaa gattagaaaa 7440
cacgatatca caaattgcct aaatataagc ctccagcggt tgtggtttga ttaaagatta 7500
gaaaacacga tatccaaaac tataataaac cacctgagtg taaagttgtg gtttgattaa 7560

agattagaaa acacgatatc aattgttgtc caaattgagc cgcttgtgtt gttgtggttt 7620
gattaaagat tagaaaacac gatatcggtc aactatttct gaacggatgt gttgatgttg 7680
tggtttgatt aaagattaga aaacacgata tccaaattac gttaaggagt ataaagacct 7740
tgttgtggtt tgattaaaga ttagaaaaca cgatatcatt gagcagtaaa cacaaaggag 7800
gaaatatgtt gtggtttgat taaagattag aaaacacgat atcaaacgga gaacaagtaa 7860
tcacaaaaag tgcgttgtgg tttgattaaa gattagaaaa cacgatatca aattataatt 7920
aaacggtaga gtacaaatgt tgtggtttga ttaaagatta gaaaacacga tatccagtca 7980
agttctttgc tagatagtgt aatagttgtg gtttgattaa agattagaaa acacgatatc 8040
ctatttagac ggtaaattcg gcacagtagt gttgtggttt gattaaagat tagaaaacac 8100
gatatcattt gctgtatcgt agcaaatgcc ttgcgggttg tggtttgatt aaagattaga 8160
aaacacgata tcaaaataaa taccatgaaa ccgctttatc tagttgtggt ttgattaaag 8220
a 8221

<210> 21
<211> 8221
<212> DNA
<213> Artificial sequence

<220>
<223> CRISPR-Cas Locus 26

<400> 21
aaaatagtca tctaatttta tgaatgggga gacaattaaa aggttttgtt ctgcttccca 60
aattatttca taaactgcat tttcaagatt ttttcctgta ataaatttcc ccatttaatt 120
aaattgtttt ttgcaggttg atattgaaat tatcctttat tggcataagc tttttactga 180
tttcaaaaat aggattttta tttcaaaaat agttagagat atttttagtt gaatgatact 240
ttttgtgtag gcaatgttac agatttggaa ggtcatgttt gtgttattag ccttctctct 300
gcggttgttt tgtttggtat tttaaggcag taatttcgat gatggaaggg ctgctagttt 360
gttttaatga ataaactatt aaatagttag ttagttagtt agttagttag ttagttagtt 420
agttagttag ttagttagtt cttgtgtttt ttcttcattt caatcagatt tcttttattg 480
gagatagatt tttttatttt gtttttgttt ttgttttggc cggcgaatgg ttggttggcc 540
ggcgaatggt tggttggttg gttggttgga aaaagagctt atttgaatgg aaagatgatt 600
tagcgttctt aaactatggt acagcagata atttgcctag gttatgacta atttaaattt 660
attgtatttc tgccaccagg gtaatttgtg catcctaaaa aattgtcata tgttcctgct 720
cacttaacca taaagctgct ttcgcagctg tagcatatga attgatcatc taaaatttcc 780
aggttcttat aggtctgatt gcagttagga taatgtgagc atcccaaaaa cttgacgctg 840
gtattttgat tttgtctggt gaccagcttg cctgttttgc agtctgctat aggaatagct 900
tttcttgttg aaaacgtaga agtttattaa taatttgaat aaaaaaagtc ccaccgaagc 960
aggactaaag atttttatct acggcatata gccttattgt gataagatta aagattagaa 1020
attttaatca gttacaaata tatataaaaa gtattatgat agaaaaagga aaaccgtaat 1080
tgtcttattt ttttttatag taaattccgt gcagattaaa tttaagaaat atggcaaaga 1140
ttttaggatt agatttagga actaactcga taggttgggc attaattgac gataaattaa 1200
ataaaatttt agggattgga agtagaattt ttccaatggg ggttgaaaat cttggtgatg 1260
gcgataacga aatgtctaag aatgcaagtc gaacaggagc aagaggagtt cgacgtcagt 1320
tttttagacg aagactaaga aagaaagtgt tactaaaatc actttctgaa aataatatgt 1380
gtccaataaa caccaaagat tttgaagatt ggaaacaaac taaacagttc cctaatgaaa 1440
aattagcaac ttggtttgct atgaatcctt atgaattgcg tcaaaaagca ttgacggaaa 1500
aattatcttt agaagaaata ggtagaattt tttatcatct aattcaaaga agaggatttt 1560
taagtaatag tagaaaagga ggaagcgatg atggagctat ttttaaagga aatgctaaag 1620
aaggaaaaat aggaattgat tatactttag aaagtatcga aggaaaaaca ttgggttcat 1680
atttatatga gatttatcca aaagaaaatc agccttttca atatggacta gaaagaatca 1740
gaaacagata tacaactcgt agaatgtata ttgacgaatt tgaaatgatt tggaataaac 1800
agtcagaatt tcattcggtt ttgaattatg atttaaaaac aattttaggg ggtagaaaac 1860
aagataatta cacagaagac ggtattttat tttatcaaag acctttgcgt tctcaaaaac 1920
atttagttgg aaactgttct tttgagcctt taaaaactaa atgccctata agtgctattc 1980
ctgtagaaat gtttagaatt tggcaatggg ttaatacagt tgaatataat gggaagaaaa 2040
tatctgaaga agaaaaaacc aaactagttg agcaattgct ttcattcgat aaaattgaat 2100
ttaaaaaact ccgaaaagct attgggaaag aaagtgcgga gtttaagttt aattacaaag 2160
acgatgataa aattgctggt acacatacta tctctaattt atccaataga aaattctttg 2220
gcaaaagatg gtttgatttt tcagataaag agcaagaaga tatttggcat gttctgtatt 2280
tttttgatag caaatcaaac ctaaaagaat atgctgttaa gaattggaat tttagtgaag 2340
aacaagcaat tgcaatttca aggtttaatg taaaagacgg ctatgctagt ttgagtcgaa 2400
aagctattgg aaatattttg ccttttttgc agcaagggta tacttatgat attgcagttg 2460

ttatgggcgg aattaaaaat gcttttggtg acaaatggca aaatgacaca gtagaaaata 2520
agctaaaaca actagatttg attgataata ttgaaggtat agttaggtct aaaatttcag 2580
gaggatttat agatacaatt aaggatttat tacgtaaaga atttgaattt aatgacaaac 2640
aattaaaaaa actttaccat cattctgctg caattgatac ggtagcatta ttacgaaaac 2700
tgccaactgg gaaacaggca gataaggata ttcaagctat tagaaatcca attgtaatta 2760
cagctttatt cgaattacga aaattggtca atgaattaat cgaagagcat gggcagcttg 2820
acgaaataaa agtagaaatg gcccgtgacc tgaaaatatc aaaatcgcag cgaaataaga 2880
tacgcaaaga tcaaaaacga ttggagagag aaaatgacag agtaaaaaag gaagtagaaa 2940
agtatgtaag agtttctcat gataacattc taagatttaa gctttgggag gaatgcaagc 3000
agacttgtcc ttatacaggt gttgtaattc cagtcacaaa actttttaca ggggaaatac 3060
aaatagagca tatccatcct tggagtcgtt cgttaaacga tagtttcaat aataaaacat 3120
tgtgctatgc ggatgaaaat agacgaaaag gagacaaaac accttttgaa ttttatggaa 3180
gcgatgagct ggattggatt gctagaaaag aacgtgcttt aaaattgttt tctgatacta 3240
aagagtatcc caatgcctat caaaaattca agcgatttgt acagcaaaaa tttgacgatg 3300
atttttcttc aagacaatta aatgatacac gatttataag taaagaagca tcgtcttatt 3360
taaaacaaat atgtgaaaag gtaaatgttt ctccaggtca aatgacagct aacttacgtc 3420
aaaaatgggg tttgaatacg gttttgaatg atgaaaatgc taaaactcgg gaagaccatc 3480
gtcatcatgc tattgatgct ttggttatgg catgtgggaa gacaagttat ttacaggaat 3540
tgtcaaaatg gaatcgttac aatagaaatt atgatttaaa agatttccct atgccttggg 3600
aaactttccg atttgatgct gagaaagcaa ttgataaaat attgatttcg cataaacgta 3660
tttctaatga tattacaact cgatatacta aagtggaaaa aaacggaaag acatatataa 3720
ataagggagt tgcagcacga gggcaattgc ataaggaaac tgtttttggt aaacgaaatt 3780
ttaatggaga tgaagcattt catgttagga aatcaattga tagtttaaca actgagaaac 3840
aattagaaaa agtggttgat gaagttatta aaaaattaat tcataagaag attaaggaat 3900
tgggaggatt cgtaaaaggt actatccctg ctaatacttt tttcaatgtt gatgaaaatg 3960
gagttaaaca accacaaatt ttcctgccaa ataaaaatgg tagtccagtt ccaattttaa 4020
aagtccgaat gaaagaaaat attagtggag ctgaaaaatt aaaagacaat gtcaatcaat 4080
gggtaaaccc tagaaataat catcacgtac ttatttataa ggatgaaaaa ggaattttaa 4140
gggaagaagt ggtgactttc tggactgttg ttgaaagaaa aagtaaagga tttccagtgt 4200
atcaattacc agttgaagga aaagagatag tgacagcttt gcatattaat gatatgtttt 4260
tgttaggatt aaatgaagaa gaaatcaatt gggagaatcc agattatgag attctaagag 4320
atagtttata tagagttcaa agtttgtctt caaaatatta tgagtttagg cagtctatta 4380
atgcagaatc tcaaaataaa atttcaccta tttatgaaca aattagagga tttggttcgg 4440
gtaaaaccgg ttgggattct tttaatccaa taaaagtaaa aatttcagtt tcaggaaaaa 4500
tttcaaaaat ataattttac tttaaaaaat agtgtaaatg ctatcaaatc aaaggagatg 4560
aaaagataga gattattacc attataggaa gacaagattt tgataatagt tctttgacat 4620
aattcaaatc ttaaacatcc aaaccacaac gaaaaggtat ggcattcgcc cagcaggaat 4680
gcggtgaaag cagttgtggt ttgattaaag attagaaaac acgatattcg atgatgatat 4740
tgttttaggt ccataccggt tgtggtttga ttaaagatta gaaaatacga tattcatcat 4800
agtatgagtt gtaagaagtt ccagttgtgg tttgattaaa gattagaaaa cacgatatta 4860
aaatccttta gcgccttcaa tttcaagctg ttgtggtttg attaaagatt agaaaacacg 4920
atattattat attcttataa cgatccgatt gatatgttgt ggtttgatta aagattagaa 4980
aacacgatat tatgatttca attgtttaat attgttgggt cgttgtggtt tgattaaaga 5040
ttagaaaaca cgatattcct ttgtcgttga ttgtttccaa agactcgttg tggtttgatt 5100
aaagattaga aaacacgata ttccatgtta tccgatatat tcgatgaaat agttgtggtt 5160
tgattaaaga ttagaaaaca cgatattcag gacttgttcc aaaagcatca tcgctggttg 5220
tggtttgatt aaagattaga aaacacgata ttgggcacag acagctgtta acgctagata 5280
tagttgtggt ttgattaaag attagaaaac acgatattga cattgtttac ctaacactaa 5340
agggaaatgt tgtggtttga ttaaagatta gaaaacacga tattacggtg aaagttatgc 5400
gaaataattt aggagttgtg gtttgattaa agattagaaa acacgatatt ttggtgaata 5460
aagacgatga atttgaaatg gttgtggttt gattaaagat tagaaaacac gatattgact 5520
tagtaaaaga ctctacgcca ttcgcagttg tggtttgatt aaagattaga aaacacgata 5580
ttttgatgat ggtaaatgat ggatcggggc ctgttgtggt ttgattaaag attagaaaac 5640
acgatattgc tctcaacaac ttcttcccat ttgactagtt gtggtttgat taaagattag 5700
aaaacacgat attaaagata gaagctatgt gtacaattgc agagttgtgg tttgattaaa 5760
gattagaaaa cacgatattt ctttataaat ttcaatcctt gtatccgtgt tgtggtttga 5820
ttaaagatta gaaaacacga tattgaagtc aatcggtgta atttctttct gttcgttgtg 5880
gtttgattaa agattagaaa acacgatatt ctaacgcatt tctcaacaga ttaccattag 5940
gttgtggttt gattaaagat tagaaaacac gatattataa ctaactttgg gtcaaatcta 6000
aagacagttg tggtttgatt aaagattaga aaacacgata ttacatcgta ataagagtta 6060
tacgaacttc cagttgtggt ttgattaaag attagaaaac acgatattcc aacagcgcca 6120
acagcaacag caggaacgtt gtggtttgat taaagattag aaaacacgat attttatttg 6180
agttgtcaac tcaatgaatt tagttgtggt ttgattaaag attagaaaac acgatatttt 6240

aattaattta gtttttcata ataatctcgt tgtggtttga ttaaagatta gaaaacacga 6300
tattccgaat ccagaattaa tgagaaaatg cgtgttgtgg tttgattaaa gattagaaaa 6360
cacgatatta gaatacttgg tttaatcgag ttatgctaag ttgtggtttg attaaagatt 6420
agaaaacacg atattgatat gtttttagct tttggattga ttgctgttgt ggtttgatta 6480
aagattagaa aacacgatat ttccatattc aagaggatat tcttttgatc cgttgtggtt 6540
tgattaaaga ttagaaaaca cgatatttaa agctaaaatt agtgaaagca ggacttggtt 6600
gtggtttgat taaagattag aaaacacgat attaaaagag ccaataataa tgacaaagga 6660
aaagttgtgg tttgattaaa gattagaaaa cacgatattt acaccgaaag cagtaaaaat 6720
ttagaaactg ttgtggtttg attaaagatt agaaaacacg atatttaaaa atttaattat 6780
gaaagggaat tatttgttgt ggtttgatta aagattagaa aacacgatat taagtgaact 6840
gtttgactta agaaatgctg tgttgtggtt tgattaaaga ttagaaaaca cgatattaag 6900
gcaatttaga ctaattgcga gtccagcgtt gtggtttgat taaagattag aaaacacgat 6960
atttggtcac ctttccaacg caaatgttta acagttgtgg tttgattaaa gattagaaaa 7020
cacgatatta agacgtttag tattggtgcc aagcggattg ttgtggtttg attaaagatt 7080
agaaaacacg atatttctat ctgaaatgat gatgacgctt tttagttgtg gtttgattaa 7140
agattagaaa acacgatatt aatgcatcag taagcatata tgatacttct gttgtggttt 7200
gattaaagat tagaaaacac gatattaaac atatccaaga tcattattaa ccctatgttg 7260
tggtttgatt aaagattaga aaacacgata ttttgataac aattaaaaag atagattcta 7320
aagttgtggt ttgattaaag attagaaaac acgatattac ggtctaacat catacaaata 7380
actacttagt tgtggtttga ttaaagatta gaaaacacga tattttgaat tatttcgttt 7440
atcagtccac tgtagttgtg gtttgattaa agattagaaa acacgatatt ggtaacataa 7500
aaacaaatac catcgttatt gttgtggttt gattaaagat tagaaaacac gatattcggt 7560
tcgtttatta acagtttaac aggcaagttg tggtttgatt aaagattaga aaacacgata 7620
tttttaaaaa ttgatggaac agttacgctt gcgttgtggt ttgattaaag attagaaaac 7680
acgatattga gtacagtagc tttaggtgca tacaatgcgt tgtggtttga ttaaagatta 7740
gaaaacacga tatttgattt ttaagattgt ttatctcacg ttccgttgtg gtttgattaa 7800
agattagaaa acacgatatt aatgcagaaa attcattgag aatgactgcg ttgtggtttg 7860
attaaagatt agaaaacacg atattcgaca acctgagtat ctaaaaccaa tacttgttgt 7920
ggtttgatta aagattagaa aacacgatat tagttggctt ttttccttct gatatataag 7980
tctttaggtg taaatatcgt tcagaaaaat gcctctttta tggcctgata acccataatt 8040
gaggcatttt ttctattgta gggtttatta gataaggtct tgaatgttga aactttgcta 8100
ataattataa aagatcagtt ctagaataat tctagttgtg taggctgagg tgctgatggt 8160
aaggctgctt ttccccaaaa atttaagata ttaccaaatt gcttatccgt tattcgaagt 8220
a 8221

<210> 22
<211> 5908
<212> DNA
<213> Artificial sequence

<220>
<223> CRISPR-Cas Locus 27

<400> 22
acgtgtggtg gcattcggca ccttcacctc gaaaggcaaa cgacgctcat cagcaatgtg 60
cagcatgagc aggcgaattg cgtcggagat ggacaagccc gtcgcttcca gcacatcggc 120
ggcgcgctcc ttggtagcgc tatcgatgcg agcgcgaacg taggtatcga cggtgctcat 180
ggcgatttcc ttggagcggt tcctataact ccatcgtggt catattgcgc ctatatatca 240
aggtatgtgc cagcctgatc catatcttaa cggccagagc agtatttcaa agcctctacg 300
caagctgcgc cgattcgctg tctcgccggc cgtctttgac ggcctgagtc agtagatggc 360
ttgcgttgcc ctccgagctc acataacgcc cagcagccga aacaaagaca gtttcgtggc 420
acgacctaca gaaaagaaaa acggtcaccg agatgaccgt ttgtatcggt tggcttcgta 480
ccgaggtact tagccttgtt gccgatagtc cccaccatcg gaatgactga tacgctttaa 540
aagataagtg acttgtatcg acagggcaag gccatggcat acaaattagg aatagatctg 600
ggcaccgcgt ccattggtgc tgtcgccatc cgcatcgaaa acaacgaacc acaaagcatt 660
gactggcatg cagtacgcat ttacagcgaa ccagttgtaa acactccgaa agggctgcaa 720
cccaaaaagg cggcccgccg tgacgcacgg caattgcgca aacagattga aaggcgagca 780
cgacggttgc gccgcattgc caatctgatt ccattgctgg gattgcataa agccgaagtg 840
aaaccggggc gccgtcagga tttgaccaca ttgcgcgcca tcgcagcgcg ccagcaggtc 900
gagctaaacg agtttgccct agtactgctg cgtctggcca aacgccgtgg ttacgccggt 960
ggatttcgcg attccaaaga cggtgtagtc aagagctcca gcgacaaact gagcgaggaa 1020
atgagacgtc ttgccgagac gcaaaacctg caggaaataa ctctgggtga atacctgctg 1080
tacagacagg aacacggact gcctgtctgg ctcaaagccg aacgtccagg tctggaaccg 1140

cgctatgccc tgcgcgaaat ggtaaccgcc gagttcgaac agctctggac aacccaggcc 1200
cgataccacc cgatcctggc ctctaatcat ctcggcaagc cacttaagca atggttcaat 1260
gaagcgctgt tctaccagcg gccactgaag tctcccgccc acatggtcgg caattgcctg 1320
cttgagccca atctgccacg tgccccacgc gctcacatgg cagcccaaca cttcagaatc 1380
gataaaacgc tccatgacat gcgctggggt agtgggcgac gggctgaacg cctgagcaag 1440
gcacaactca acgtgctgcg tcagatgctg aatgatccag ccaatgccga tggcaaggtc 1500
agcttcaaac acctgtatca agctttgagc gagacagggt tgagcgatcc acgaagtcgc 1560
actttgaatc tggagcgcag cagctgtgat caactgtccg gtaacaacac ggttgctcgt 1620
tggcgtgcgt tgggactgta cgaccaatgg ctgacgctag cccctcgcga ccagacaacc 1680
atcatcaact tgctggcgga cttgggcggg ccggaactgc tggttccgga cgactggcac 1740
cagaacatcc gcaagagcag cgccaaagct ggcgatacca acctttatcg tcgattccgg 1800
cccgaggtcg ttgatttcat caatgcgctt cgcgagtgca aggacaacaa tggcagccca 1860
gttttcaatc ggctgacatc tatgaagttc gagggcggac gcagcagcta ttccgtcaag 1920
gccttggaaa aattgaatac ctggttcaat gatccttggt ggcccgacga aggctcaggg 1980
gccgagcgtg gtgacgaaat cgatgaagac gcagcactac gggtctgcta tccacatctt 2040
tttacgcagc cccaagagct gctccaacga ctcgaggccg ctccgaccac gggcaacgac 2100
gtggttgacg gagccttgcg cgaattacgc aaggtcatca accaatgcat tgacgctttg 2160
ggtgagaggc cagaggaaat cgtcgtcgaa atggcacgag agatcggcaa gggcgtcgtt 2220
tggcggaaag agcaggaaaa ccgcatacgg cagaatgaac tcgcccgaag gaaaattgcc 2280
agcgaactgg aaaagcacgg actgagcgcc aacaacaccc ggatactgcg ctatcaactc 2340
tgggaagagc aggatttgca ctggtgcccc tactgcgccc atcccattaa tctggaagca 2400
gccacctcgg gcatgacaca catagagcac gtcctgccac gctcgctcac ccagatcggg 2460
cgcaaacgca gcgaactggt gctggcccac gccggttgca acaacctgaa agccgatcag 2520
acaccttggc aagcatttgg ccatgatgtc gagcgctggt ctgcagtaga acgcgctgcc 2580
gcccgttttc aagagctggg caagaagtct tacgccaagg accgcatcaa ggcgatgggc 2640
ttctaccgca aggcggaact tctcctgctg aaggattacg aaagcgaagt actgagcgac 2700
gaatccatag ccgacttcgc cgaccgccag ttacatcaga cctcctggat cgccaaggct 2760
gcagcccagt ggttgcgcac catcaccccg ctggtttccg tctctcgcgg tgaattcacc 2820
gccctactac gccatcaatg gcgactcgac accgtgatcg cagaagcgcg ccttgatgaa 2880
aaacttcctg ttctggatat cgatgcgtgc cccgtatctc cggaagcatt ccagcagttc 2940
cggaaacaat gggaaggcca tccaccagac aacctggccg aacggaccga gcgcaaactg 3000
gaaaaacgcc tcgaccatcg ccaccacggc atagatgcct tggtcatcgc gctcaccagc 3060
cgaagcctct acatgtctct ggcccggcgc tacgttgaag aaagcgaaag gcgcgcccgc 3120
ggcgagagag tccggcatga ttggtccatc gagccgacta tgcacaatct gcgcaaccaa 3180
gcgctgggta tggtccgtac ttgcaacctg acccacaagc ctgaccgcaa cccagccggc 3240
cgactattca aggacaccgc ctatggcgtc cactttccag aggagggcga tcgggtacag 3300
ctgacgctgc gcaaaccttt gaccagcctg gccgacgaaa agtctgccga gaagactcgc 3360
aagaacctgc acagcattgc cagcagtgag gtacgcctac tcgttctgaa agcattcgaa 3420
gcgcgcctca gtgtcggcca gaatccgaaa caggcgctct ccgaaccggt accctacccc 3480
gcctatggca ccgcaatctg gcgagtccgc tgcatgcaag gctacgcgga cgatgccgtc 3540
cgtattgtcc ataaaagccg tcagggcgaa cacgaaaagc acctgctcaa cgatggctat 3600
gcctgcttgg aaataaccgc tggggaaaat ggcaaacacc aagtacgcct tgtcactcag 3660
cgtgaagcac aaggccgcgg cttcatggat aaccggcagc aggcgaaacg catattcaag 3720
ggagatacgg tgatcgaccg gcgcgacgac cgtaaatacc tagtgggcta cttcaaggcc 3780
gagggctata ttttcctcct cccccacacg gacacccgca acttcgacaa gatcaaggag 3840
gcggactcgg gcaagaagaa agtttccttc aaccaagcgt accaacttga gcgcatctag 3900
ccatgtccga gcaccgggta ttgctcatcg aacatcaggc ctatctgagc atcgacctag 3960
ggcgtctgtg tatccgccga ccacagcagg agagcgtgtt cgtcctgccc gctgacattg 4020
ccgtgctctg cctgcaccac ccggccatca cggtaacggc ccacaccctg cagaccctga 4080
gccaggaagg tgcgatcgtc ttggtgacgg acgaccaaca tctaccgctg gcccaggtct 4140
accccctgct cgcccccatg cgtcagacac tgcggctgct ccaacagttc gagctgcaag 4200
agtcgccgtg gcgcggcatc gtctggcaac gcctagtgca agctcgcctg cggggcgaag 4260
ccgccgttct gcgcagtttg ggcaagaaag gcacgctttt cctagagcga ctggccaaca 4320
aagtgcaacc gggcgacagc gaccaatgtg aaggccaagg cgcccgccac tactggaaac 4380
acctgctgcc tgagggtttc cggcgcagca agcagggtgc caccgacctg ctgaatgctc 4440
gcctcaactt cggttatgcc gtgctgcgct cgctgatcgc gcgcaacctg gcttgtgccg 4500
gccttaatgg ttgcttgggc gtcggccact gcaaccagca gaaccccttc aatttagccg 4560
atgacttgat agagccttac cgcttccttg tcgaatggcg ggtcgcgcgg caactcgagg 4620
agcatgggga gcaacctttc gatgcggcta gccgcaagag cctgttgcag ttcatgtccg 4680
acaccatccc catgctgcta ggggactacc gcctgcctgc agcgatagct gccactgtcg 4740
acagctgggt gcagacactc gacgcaattc atgcgcaccg gcatccaggt acgataacgc 4800
taccggagta tgaacgatgg ccattaacgg atggcgggtc atgtgggtga tcgccgttta 4860
cgactgcccg atgaccagcg cagaagcccg gcacggctat gcgaaattcc gaaagtcact 4920

gctgcgggcc aacttcatcc aactgcagaa ctcgctctac gtccgccact ttccgagcat 4980
ggcgacggcc gaggcttgca ttgctcgttt gcggtacgac attcccgaac gggcgcaact 5040
ggcgttcttt cttgtcaccg acaagcagta cgctatgact cgggagtatt tcggcgaatg 5100
cagcgtacgg aaaaagccag aggccccgga gcaaatcgag ctgttctgaa aaaaagcgca 5160
accctaacaa tgggttgcgt gatcaagagg ttatcagtca ttccgatggt ggggtatcgg 5220
aacatccgca gctacctgcc gcaagacggc tggaggttat cagtcattcc gatggtgggg 5280
tatcggaaca cacggtgatc tttgccgaac cgactggcta ggttatcagt cattccgatg 5340
gtggggtatc ggaacattca ggacatccac gaagactacc cgggcaggtt atcagtcatt 5400
ccgatggtgg ggtatcggaa ctgcgcgcct acggcaatgc cgtcaactcg caggttatca 5460
gtcattccga tggtggggtt atcggaactt ggcctgatcg ctggtggcac cgaaggaaga 5520
aggagtacac atgaagcttt gtgcgcggaa aatgctgtaa caaaactcgg gtatcgacta 5580
tgcgaccgga tacctagttt tgagatttta cgtttccata ctgactactt atcggttacc 5640
tgagtgatta tttgcttgct atttattcgc ttgtttaaac gagcagccag agtagcgctg 5700
atagcaatac cttttctcga actcgatagt cggttaaaaa ccctcaagac attgattaca 5760
ttaaaaatta ttaaaacagc cacaaacaga ctcgcgaaga ccttgcctta taaacaagtc 5820
actaagtcaa ccaagaatac ttagcgactt aatttttgag ggttttcgat gcgagttatt 5880
ctcacttaga aagttttcgt ttttttag 5908

<210> 23
<211> 6265
<212> DNA
<213> Artificial sequence

<220>
<223> CRISPR-Cas Locus 28

<400> 23
catgtgacgt gtagaagtat atccaaatca ctcccgattt cctcagccca ttggcactgc 60
ctgaacactc tgtatggagc cgcgagggta agcaggcgtt gcgctggggg aatgttcact 120
gcgcgctgga agcggtcgaa gaataccctg gtcatttcaa acaaggccag actcggttta 180
agggtacgga ccttggttag tgacagctgt caaaagtcga cttcatagtc gccttcggca 240
atcctaatga tggctattat aaatttcgaa accagtctct tttttgcaaa ccgtgttgct 300
tcgacggtgc acggcggcca aaactgacac ccaaaaaaca aaaacggcca ccgaggtggc 360
cgtttgtatc ggttggcttc gtaccgaagt actcagcctt gttgccgata gtccccacca 420
tcggaataac tgatacgctt tttgaagata aagcacttca ggtgacaggg caagataatg 480
gcatacaaat taggtatcga tctgggcacc gcttccatcg gtgcggttgc cattcgtatt 540
gaaaataagg tgcctatcag cgttgactgg catgcagtgc gcattttcag cgagcccgtc 600
gtaaacactc agaaagggct cacgcccaaa aagacggccc gtcgtgaagc ccggcaattg 660
cgtaaacagg tcgaacgacg agcccgacgg ttgcgccgta ttgccaatct gattccattg 720
ctggggttgc agaaagccga agtaaagctt gggcgccgtc aggacttgac cacgttgcga 780
gctattgccg cgcgcgagaa ggtcgaacta catgagtttg ccctggtact gctgcgtatt 840
gccaaacgcc gcggttacgc cggtggattt cgcgattccg aaggcggcgt ggtcaagact 900
gccagtgaca aactgagcgt ggaaatgaga gctcttgccg agacgcgagg cctgcaacag 960
ataactctgg gcgaatactt actgcatcga cagcaacgag ggctgcctat ctggctcaaa 1020
gctgagcgtc caggcttgga accgctctat gctctgcggg agatggtaac cgccgaattc 1080
gaacaaatct ggacaatcca agccaaatgc catccgatcc tgaacagctg tcatctcgaa 1140
aaaccactta agcaatggtt ctatcaagca ctgttctatc agcgaccact gaaatctccc 1200
gcccacatgg tcggcaattg cctgcttgaa cccaatttgc cacgcgcccc acgcgctcaa 1260
atggcagccc aacgcttcag gatagaaaaa acgcttcacg atttacgctg gggtggcgga 1320
cgacaggctg aacgcctgaa cacagcgcaa ctcgacctac tgcgtcagac tctgagcgat 1380
ccaaccaatg ttgatagcaa ggtcagcttt aagcgcctgt accaagccct gaacgaggct 1440
gggttgaacg atccgcgaaa tcgcacatta aacctggagc gcagcagtcg cgatcagtta 1500
cttggcaata gcaccgttgc ccgttggcgc gcgctgggcc tgcaagatca atggctgatg 1560
ctggctcctc gcgaccagac atctgttatc aacctgctgg cagacttggg tggtccggaa 1620
atactgaccc cagacgactg gcaccagaac atccgtaaaa gcagtgccaa ggacgacaac 1680
accaaagcct atcgtcgatt ccggcaggaa gttattgatt tcatcgatgc gcttcgccgg 1740
tgcatagata gtaatggcca gccggttttc aatcgtcttt ccgctatgaa gttcgacggc 1800
ggacgcagta gctactcggt aaaggccttg gaaaagatga acgaatggtt caataacccg 1860
tggtggccca atgaaggtcc aggctcagag cgtcgcggcg agatcgacga agaagtggcg 1920
atacgggtgt gttacccaca tctttatacg caatctcaag agctgcaccc gcgactcgaa 1980
gccgctccga ccaccggcaa cgacgttgtc gatggagcat tacgcgaact gcgcaaggtc 2040
atcaaccaat gcattgacga gcaaggtgag aaaccacagg aaatcatcat cgaattggca 2100
agagaggtcg ggaaaggtgt ggctttgcgg aatgaacagg aaagtcgcat tcggaacaac 2160

gaactcgcca ggcggaaaat tgccagcgaa cttgaagggc acggtttgat tgccagcagc 2220
acccggatac tgcgatatca actctgggag gagcaagagc gacactggtg tccttattgc 2280
gcacttccca tcagcctgga agcagccatc tcaaacatga cgcacataga acacgtcctg 2340
ccccgttcgc tcacccaagt cggtcgaaaa cgcagtgaac tggtgctagc ccatgctggt 2400
tgcaatcatc tgaaagccga ccagacgcct tggcaagcct tcggccacga tgccgagcgc 2460
tggtctgcag tagaacgtgc tgccgcccga tttcaggagc tgggtaaaaa gtgctatttc 2520
aaagacaggg cgaaggcgat gggttgcttc cgaaaatcag cccttctcat gctgaaggat 2580
tatgaaagtg aagtattaaa cgacgaatcc atagccggat tcgccaaccg tcagttacac 2640
cagacatctt ggattgccaa ggttgcagcc caatggttac gtacgatttc accgctgata 2700
tccgtttccc gcggcgaatt caccgccctg ctgcgccatc aatggcgact agacaccgtt 2760
atcgcagaaa cgcgctatgg tgaaaaactt cccgttctgg atatcgatgg caatcccgtc 2820
catccggaag aatttcagca cttccggaaa caatggaagg gacatccacc ggacaacccc 2880
gccgaaagaa ccgagcgcaa actggaaaag cgcttcgacc accgccacca tggcattgat 2940
gccctggtca tcgcgctcac cagccgtagt ttgtacatgt ctcttgctcg gcgctatgta 3000
gaagaaagca aaaagcgcgc ccgcggcgag aaagtccggc gtgactggtc cattgagcca 3060
cctatgcaca atttgcgcgg ccaagctctg gatatgatcc gcgcgtgcaa cctgaccctc 3120
aagcccgacc gcaacgctgc cggccgactg tttgaggact acgcctatgg cgtccacttt 3180
tctgacgcaa gcgagcgggc acagcttacg cggcgcaaaa cattgataga tcttgcagac 3240
gaaaagtccg cagagaagac tcgcaaaaat ctccagagca tcgcaagccc tgaggtacgc 3300
cagatcgttg tggaagcgct ccaaatgcgc ctaagtgccg ggcaaaatcc gaaacaggcg 3360
ctctccaaac ccgtactcta ccccgcttac ggtacagaaa tttggagggt ccgttgctta 3420
caaggctacg cggaccatgc cgtgcgaatc gttcacaata gccgccaggg tgaacacgaa 3480
aagtgtctgc tcaacgatgg ctatgcctgc ctggagataa gggccgggga aaatggcaaa 3540
caccaagcac gtctcgtcac tcagcgggaa gcacagggtc gtgacttcat ggaaacccag 3600
cagcaggcga agcgcctatt caagggagat acggtgatcg acaggcgcga tgatcgtaaa 3660
taccgagtgg gttatttcac cgccgagggc aatattttcc tcatcccctt cgaggacact 3720
cgtaatttcg ataaaatcaa tgagcggaac tcgggcaaga aaaaggtttc cttcaaccaa 3780
gcgcatcgac ttgagcgagt atgacgatgt ccgagcacag agtactgctc attgaacatc 3840
aagcctatct gagtattaat ctagggcgcc tgtgcatccg ccgaccacag caggagaacg 3900
tatatgtcct gccctcggac attgctgtgc tctgcttgca ccatccggct ataacgttaa 3960
cggcccagac attgcagatc ctgagccaag aaggcgctat cgttttggca accgacgata 4020
aacatcaacc acttgcacag gtctaccccc tcctcgcccc catgcgtcaa acactgagac 4080
tgctgcaaca attcaaactg caagagtcgc catggcgggg catcctctgg caacgattag 4140
tgcaagcccg cctacgggga gaagccgccg ttctgcgcag cttgggtaag aaaggcgctc 4200
tatttcttga acgactggcc atcagggtac tcccggcgga cagctgccaa tgtgaaagcc 4260
aaggtgcccg tcactactgg aagcacctgc tgcctgaggg ttttcatcgc agcaagcagg 4320
gggctatcga tccgcttaat gtccgcctca acttcggcta tgccgttctg cgttcactga 4380
tcactcgcaa cttggcttgt gctggcctga atggttgctt gggcgtgggt cactgtaacc 4440
aacagaaccc cttcaatctg gccgatgacc tgattgaacc ttaccgcttc ctcgttgaat 4500
ggcgggtggc acgccaactc ggtgagaatg ctcagctacc attcgatgca tcgactcgca 4560
agcaactgct gcaattcatg tccgacgccg tccccatgcc gcgaggggac taccgtctgc 4620
ctgcagcaat agaggccaca gtcgacagtt gggtacaaac actcgacgca attcatgcca 4680
accggaatcc aggcacgata acgctaccgg agtatgatcg atggccatta acggatggcg 4740
ggtcatgtgg gtgatcgccg tttacgactg cccaatgaca agcccagaag cgcggcacga 4800
ctatgcgatt ttccgaaagt cactgctgcg ggccaacttt atccagctgc agaactcgct 4860
ctacgtccgt cactttccga gcatagcaac agccgacgct tgcatttctc gtctgcgttt 4920
aaatattccc gatcgtgcgc agctagcatt ctttcttgtc accgataagc agtacgcaat 4980
gactcaggag tattttggtg aacgcagcgt tcggaaaaaa ccagaagccc cagagcaaat 5040
cgagctgttc tgaacaaaaa aacgcaaccc tggcaagagg ttgcgtaatt aagaggttat 5100
cagtcattcc gatggtgggg tatcggaacg ctgcggcaaa gataacttcg ctgtcataaa 5160
aggttatcag tcattccgat ggtggggtat cggaacacgg ccatggtggg tccgatacgc 5220
tttagcaggt tatcagtcat tccgatggtg gggtatcgga accgcgcatc tacagcaccg 5280
acgacgggtt ccaggttatc agtcattccg atggtggggt atcggaactg gtcattaccc 5340
cggaagccgg cgagcaggag gttatcagtc attccgatgg tggggtatcg gaacattcag 5400
cagtacatta aaaccggcca acacaggtta tcagtcattc cgatggtggg gtatcggaac 5460
taatcatggg cggcggtggc ggcggtggcg aggttatcag tcattccgat ggtggggtat 5520
cggaacgagc gaatgcagcg gtcgcgggtg cggatgaggt tatcagtcat tccgatggtg 5580
gggtatcgga actgccaccg tggcgcctgt cgttcgaccc gtaggttatc agtcattccg 5640
atggtggggt atcggaacgt agccgggcac gtcctgccgg gcttcgggag gttatcagtc 5700
attccgatgg tggggtatcg gaacacacgg tgcagaccaa aacgtggccc gccaaggtta 5760
tcagtcattc cgatggtggg gtatcggaac aatggcagac cgggcggcgc gcgctatttt 5820
aggttatcag tcattccgat ggtggggtat cggaacatcg ttaacgacga cctcggcgtt 5880
ttcgataggt tatcagtcat tccgatagtg gggtatcgga acaacgcaga gaccagcgag 5940

cagcgtattc gcaggttatc agtcattccg atggcggggt atcgaaacac caaaatgtac 6000
gacctgaaaa cattaggtca tggaaacatc aagcactccc agcatcacct cttgatagac 6060
ttactctctg agctagaaca accctcctaa ctcagccggt ttccaattca taattaccag 6120
ttccccgctc acatcagcct ttccctgccg ctggttggtg gcgctgtaac ggatatccag 6180
tcgctcgaag tgaaaacctt cgaacactcg gcgaatgtcg gggtgatcat tgatgctgac 6240
catcaccctg cccttgcagc ggcgc 6265

<210> 24
<211> 3282
<212> DNA
<213> Bacillus cereus

<400> 24
atggcataca caatgggaat agatgtgggg attgcttcgt gtggatgggc tattgtagat 60
cttgaaagac aacgaataat agatctaggt gttcggactt ttgagaaagc agagaatccg 120
aaaaatggag aggctcttgc tgtaccaagg agagaagcta gatcgagccg taggagatta 180
cggagaaaaa aacatcgcat tgaaagatta aagcatatgt ttattcggaa tggactggcg 240
gttgatattc aacatcttga gcagcagacg ttacgtagcc aaaatgaaat agatgtatgg 300
caattacgag tagatggttt ggatcgaatg ctaactcaaa aagagtggct tcgtgtatta 360
attcatcttg cacaacgtcg tggttttcaa tcaaatcgaa aaacagatgg ttcaagtgaa 420
gatgggcagg ttcttgtaaa tgtaacggag aatgacagat taatggaaga gaaagattat 480
cgaaccgtag cggaaatgat ggtaaaagat gaaaaattct ctgaccataa gcgaaataaa 540
aatggaaatt atcatggagt agtgagcaga gcttctttat tagttgaaat acatacatta 600
tttgaatccc aaagacagca ccataatttt ttggcatcga aagatttcga gctggaatat 660
gttaatattt ggtcttcaca acggcctgtt gcaacaaaag atcaaataga aaaaatgatt 720
ggtacatgta ctttcttacc gaaagaaaag agaactccaa aggcatcttg gcattttcaa 780
tattttatgc tcttgcaaac gatcaatcat atccgtataa caaatataca aggcacgaga 840
tcattgaata aagaagaaat tgaacaagtg gtcaatatgg cacttaccaa atcaaaggta 900
tcttaccatg atattagaaa agtccttgat ttatcagaag aatatcaatt tgttggcttg 960
gattatggaa aagagaatga aaaaaagaaa gttgaaagta aggaaacgat catcaaatta 1020
gatgattacc ataagttaaa taaaattttt aatgaagtgg aattagctaa aggagaagct 1080
tggaaagctg ttgattatga tacagtagcg tatgctctca ctttctttaa agatgatgaa 1140
gatattagag attatttgca aaataaatat aaagatagta aaaatcgtct tgttaagaac 1200
gtagctaata aagaatacac aaatgaattg gtagaaaaag tgagtacgct atcttttcgt 1260
aaagtggggc atttatcatt gaaagcctta cgaaagatca tcccgttttt ggagcaaggc 1320
atgacctatg ataaagcgtg ccaagcggct ggttttaact ttcaaggtat atcaaaaaag 1380
aagagatctg ttgtcttgcc ggtcattgac caaatttcca acccagtagt aaatcgtgct 1440
cttactcaaa cgcgtaaagt aattaatgct ctaatcaaaa aatatggttc acctgaaacc 1500
atacacattg aaacagcaag agagttatca aagagatttg atgaacgaaa gaatattact 1560
aaggattata aggagaatag agacaagaac gaacgtgcaa aaaaacactt atcggaatta 1620
gggattatta atcctactgg tcttgatata gtaaagtaca agctgtggtg tgagcaacaa 1680
gggcgctgta tgtacagcaa ccaacccatc tcctttgaaa gactgagaga atcaggttat 1740
accgaggtag atcacattat tccatacagc cgaagtatga atgatagtta taacaatcgc 1800
gtattggtta tgactaggga aaatcgtgaa aagagtaatc aaactcctta tgaatatatg 1860
ggtaatgata cgcaaagatg ggatgagttt gaacaaagag tcacaacaaa tccacaaata 1920
aaaaaggaga aacgacagaa cctcttgcta aaaggattca ctaatcgtcg ggaattagaa 1980
atgttagaaa gaaacttaaa tgatacacgt tatataacta agtatctttc acattttatt 2040
agtacgaatt tggaattctc tcctagtgat aaaaagaaaa aggtagttaa tacaagcggt 2100
cgtatcactt cccatttaag aagtagatgg ggattagaaa aaaatcgtga gcaaaatgat 2160
ttacaccatg caatggacgc ggttgtaatt gctgtcacct cagactcgtt tattcaacag 2220
gtaacgaact actataaacg gaaagaaaga agagagttga atagggatga taagttccct 2280
ctaccttgga agttttttag agaagaagtc atagcaagat taagccctaa tccaaaagaa 2340
caaattgaag cattgcccaa ccatttttat agcgaagatg aacttgctga tcttcaaccg 2400
atttttgtgt caagaatgcc caagcgtagt ataactggag aggcgcatca ggcacagttt 2460
cgccgagttg tgggtaaaac gaaagagggt aaaaatatta ctgcaaagaa aactgcttta 2520
gttgatatta gctatgataa aaatggtgat tttaatatgt atggaagaga aacagatcct 2580
gctacttatg aagcaattaa agaaagatat ctcgaatttg gaggaaatgc taaaaaagca 2640
ttttcaacgg atttacataa accgaaaaaa gatggcacca aagggccact gataaaatct 2700
gtgagaataa tggaaaataa aacattggta catcccgtaa ataaaggaaa aggtgttgtg 2760
tataacagct ctattgtaag gacagatgta tttcaaagaa aagagaaata ttatttacta 2820
cctgtgtacg taacagatgt aaccaaaggg aaactaccaa ataaggtgat tgttgctaaa 2880
aagggatacg atgattggat tgaggttgat gatagcttca catttttata cagtctatat 2940
ccgaatgatt taatattcat cagacaaaat ccaaaaaaga aaatatcatt aaaaaaacgt 3000

attgagagtc attctatttc tgatagtaaa gaggttcaag aaattcttgc ctattataaa 3060
ggggttgata gttcaactgc tgctatagag tttattactc atgatggtag ttactatgca 3120
aaaggtgttg gtgtccaaaa tctagattgt tttgaaaaat atcaagttga tactctgggt 3180
aattattcca aagtgaaagg agaaaaacga cttgagttgg agacatctga taataacgac 3240
aaaggcaaag atgtcaattc gattaaatca actagtcgtt aa 3282

<210> 25
<211> 3279
<212> DNA
<213> Brevibacillus laterosporus

<400> 25
atggcttaca caatgggact agatgtgggg attgcttcgt gtggatgggc tattgtagat 60
attgaaagac aacgaataat agatgtaggt gttcgaattt ttgagaaagc agagaatccg 120
aaaaatggag aggctcttgc tgtacctagg agagaagcta gatcgagccg taggagatta 180
cggagaaaaa aacatcgcat tgaaagattg aagcatatgt ttattcgtaa tagactggcg 240
gttgatattc agcatcttga gcagacgtta cgtagtcaaa atgaaataga tgtatggcaa 300
ttacgagtag atggtttgga tcgagtgtta actcaaaaag agtggcttcg tgtattaatt 360
catcttgcac aacgtcgtgg ttttcaatcc aatcgaaaaa cagatggttt aaatgaagat 420
ggacaggttc tcgtaaatgt atctgagaat gagagattaa tggaaaagaa agattatcga 480
accgtagcgg aaatgatggt aaaagatgga aaattttccg accataagcg aaataaaaat 540
ggaaattatc atggagtagt gagcagagct tctatattag ttgaaataca tgcattattt 600
gaatcccaaa gacagcacca taattttttt gcatcgaaag atttcgagct ggaatatgtt 660
aatatttggt cttcacaacg gcctgttgca acaaaagatc aaatagaaaa aatgattggt 720
acatgtactt tcttaccgaa agaaaagaga gctccaaagg catcttggca ttttcaatat 780
tttatgcttt tgcaaacgat caatcatatc cgtataacgt atttacaagt cacgagatca 840
ttgaataaag aagaaattga acaagtggtc aatatggcac ttaccaaatc aaaggtttct 900
taccatgata ttagaaaaat ccttgattta tcagaagagt atcaatttgt tggcttggat 960
tatggaaaag aggatgaaaa aaagaaagtt gaaagtaagg aaacgatcat caaattagat 1020
gattaccata agctaaataa gatttttaat gaagtggaat ttgctaaagg agaaacttgg 1080
gaagctgttg attatgatac agtagcgtat gccctcactt tctttaaaga tgatgaagac 1140
attagagatt atttgcaaaa taaatataaa gatagtaaaa atcggcttgt taagaactta 1200
gctaataaag aatacacaaa tgaattgata gaaaaagtga gtacgctatc ttttcgtaaa 1260
gtgggtcatt tatcattgaa agccttacga aagatcatcc cgtttttgga acaaggtatg 1320
acgtatgata aagcgtgcca agcggctggt tttgactttc aaggtatatc aaaaaagaag 1380
agatatgtcg tcttgccagt cattgaccaa atttccaacc cagtagtaaa tcgtgctctt 1440
actcaaacac gtaaagtaat taatgctcta atcaaaaaat atggttcacc tgaaaccata 1500
cacattgaaa cagcaagaga gttatcaaag acatttgatg aacgaaagaa tattaccaag 1560
gattataagg agaatagaga caagaacgaa catgcaaaga aacacttatc tgaattgggg 1620
attattaatc ctactggtct tgatatagta aagtacaagt tgtggtgtga gcaacaaggg 1680
cgctgtatgt acagcaatcg acctatctcc tttgaaagac tgaaagaatc aggttatacc 1740
gaggtagatc acattattcc atacagccga agtatgaatg atagttataa caatcgcgta 1800
ttggttatga ctagagaaaa tcgtgaaaag ggtaatcaaa ctccttttga atatatgggt 1860
aatgatactc aaagatggta tgagtttgaa cagagggtca caacaaatcc acaaataaaa 1920
aaggagaaac gacagaacct cttgctaaaa ggattcacta atcgtcggga actcgaaatg 1980
ttagaaagaa acttaaatga tacacgttat ataactaagt atctttcaca ttttattagt 2040
acgaatttgg aattctctcc tagtgataaa aagaaaaagg ttgttaatac aagcggtcgt 2100
atcacttccc atttaagaag tagatgggga ttagaaaaaa atcgtgagca aaatgatcta 2160
caccacgcaa tggatgcggt tgtaattgct gtcacctcag actcgtttat tcagcaggta 2220
acgaattact ataaacggaa agaaagaaga gagttgaatg gggatgataa gttccctcta 2280
ccttggaagt tttttagaga agaagtcata gcaagattaa gccctaaccc aaaagaacaa 2340
attgaagcat tgcccaacca tttttatagc gaagatgaac ttgctgatct tcaaccgatt 2400
tttgtgtcaa gaatgcccaa gcgtagtata actggagagg cgcatcaggc acagtttcgt 2460
cgagttgtgg gtagaacgaa agagggtaaa aatattactg caaagaaaac tgcgttagtt 2520
gatattggct atgataaaaa tggtgatttt aatatgtatg gaaaagaaac agatcctgct 2580
acttatgaag caattaaaca aagatatctc gaatttggag gaaatgctaa aaaagctttt 2640
tcgatggatt tacataaacc gaaaaaagat ggcgccaacg gtccactgat aaaatctgtg 2700
agaataatgg acaataaaac attggtacat cctgtaaata agggaaaagg cgttgtgtat 2760
aacagctcta ttgtaaggac agatgtatat caaagaaaag agaaatatta tttgttacct 2820
gtgtatgtaa cagatgtaac caaggcgaaa ctaccaaata aggtgatcgt tgccaaaaag 2880
ggatacgatg attggattga ggttgatgat agcttcacat ttttatttag tctatatccg 2940
aatgatttaa tattcgtcag acaaaatcaa aaaaagaaaa tatcattaaa aaaacgtatt 3000
gagagtcatt ttatttctga tagtaagaag gttcaagaaa ttctcgcata ttataaaggg 3060

gttgatagtg catctgctac tatagagttt attactcatg atggtagtta ctatgcaaaa 3120
agtgttggtg tccaaaatct agattgtttt gaaaaatatc aagttgatat tctgggcgat 3180
tattccaaag tgaaaggaga gaaacgactt gagttggaga catctgatag taaccacaaa 3240
ggcaaagatg tcaattcgat taaatcaact agtcgttaa 3279

<210> 26
<211> 3213
<212> DNA
<213> Artificial sequence

<220>
<223> Cas endonuclease of Locus 8 (Cas-Locus 8)

<400> 26
atgagagagt tggattatcg catcggatta gatattggaa cgaattctat tggctggggt 60
gttattgaat tatcttggaa caaagataga gaacaatatg agaaagcgag aattgtcgac 120
aagggtgttc gtatgtttga taaggctgaa atacctaaga ctggtgcttc tcttgctgaa 180
ccgaggcgta tagcgcgctc atcacgtaga aggttaaatc gcaaaagcca gagaaaaaaa 240
gatatacgta atttacttgt tcaacatgaa attattagcc aaaaagaatt agcttcgtta 300
tatcctctgt caaaaagttc aatggatatt tgggatattc gtttggatgg attagatcgc 360
ttgttagacc gttttgaatg ggctcgacta ttaattcatt tagcacaaaa acgcggtttt 420
aaatcaaatc gaaagtctga actgaaagat gtggagacag gaaaagtatt atcaagtatt 480
caagcaaatg aaaaacgatt atctctgtac cgtacagtgg gagagatgtg gatgaagaat 540
atggatttta gtaaatatgg caaaaggcgt aattctccta atgagtatgt attttcggtt 600
agccgtgcag atttagaaaa ggaaattgtg actctatttg aagcgcaaag aaaatttcat 660
tcatcatatg catcggttga tttacaaaaa acatatttac gaatttgggc acaccaactc 720
ccttttgctt ctgggaatgc aattgtaaat aaagtaggat attgttcatt attaaaaggt 780
aaagaaaaga gagttccgaa agcaacatat acttttcaat atttcaatac actagatcaa 840
ataaaccgaa cacgattagg gcctgatttc caaccattta cgaaggaaca gagagacatt 900
attttagata aaatgtttca acggacagat tattataaaa aaaagacaat acccgaagtt 960
acttactatg atattcggaa atggttagca ctagatgaaa caattcaatt taaaggactt 1020
acctatgacc caaacgaaga gctgaaaaaa atagaattga aaccctttat taatttaaag 1080
ccattttatg aaattaaaaa ggtagttact aattacgccc aaaaaacaaa tgaggtattc 1140
tcaacattag actatgatac agttgcatat gctttaacag tttataaaac ggacaaagat 1200
attagatctt atttgaagag atctaataat ttatcaaaac gttgctatga tgatcaatta 1260
atagaagagc tattaactct ctcctataca aagtttggtc atttatcatt taaagcaatt 1320
aatcatgtac tgccaattat gcaagaggga aggacttatc aggaagcaat aaaccaatta 1380
ggatatgata ccactaatct taaaaaagaa aacagaagta tgttcttgcc tcttattcca 1440
gatgagataa caaatccaat tgttaaaaga gcgctaactc aagcacgtaa agttgtaaat 1500
gctattatta gaagatatgg ttctccaaat tctgttcata ttgaactagc tcgtgagctt 1560
tctaaaagtc atgacgagag aaagaaaata atgacagctc atgatgaaaa ttataagaaa 1620
aataaaggag ctatatcaat tttgatcgag aatggaattt taaatccgac aggatatgat 1680
attgtgcgtt ataagttatg gaaagagcaa ggagaacgat gtgcttattc gttaaaaaag 1740
attcctgcta atacgttttt taatgaattg aaaaaagagc gaagtggccc tccggttcta 1800
gaggtagatc acattttacc gtatagtcag agttttattg atagttacca taataaagta 1860
ttagtttatg gggatgagaa tcaaaaaaag ggaaatcgaa ttccatatac ttttttttca 1920
gaagaaaata aggagtggga aagctttgaa agctacgtaa gatcgaatag ttttttttct 1980
aaaaagaagc gcggatattt attgaaaaaa gcttacttgc caagagagag taacctgatt 2040
aaggagcgtc atttaaatga tacccgatat gctagtagct atttgaaaaa tttcattgag 2100
aaaaatttaa aatttaaaga agctgaaggt attacacgaa aaaaacatgt acagacggtt 2160
aacggtgtaa ttacagccca tctgcgaaaa agatggggct tagaaaaaga taggcaggaa 2220
acatatttgc atcatgcaat ggacgctatt attgttgctt gtacagatca tcatatggtc 2280
actaaagtaa cggagtacta tcaaataaaa gaaagtaata agtcaataaa gaaaccgtat 2340
tttcctttgc catggatggg ctttagagag gaaattttat cacatttaga aagtcagcca 2400
attgctagaa aaattagtga ggaacttaaa attggatatc aatcatctga ttatatactt 2460
gtatcgcgaa tgccaaaaag atctgtcact ggatcagctc atgaccagac agtcatgaaa 2520
aaaggcggta tcgataaaaa aggaaagact attatcataa agcgtgtgca cttaaaggat 2580
attaagtttg atgagaatgg cgattttaaa atggttggaa aagaacaaga tttagcaact 2640
tacgaagcga taaagcaaag atatttagag tatggaaaag aatcgaaaaa agcatttgaa 2700
acacctttat acaagcctag taaaaaagga aaaggaaacc tcattaaaaa aattaaagta 2760
gaagtgcaaa ccaaatcttt tgttcgagaa gttaatgggg gcgtagctca aaatggtgat 2820
ttagtaagaa tagatctgtt tgaaaaggat aataggtatt atatggtacc tatttacgta 2880
gtagatactg ttcgttccga actaccaaat aaagctgtaa aaagcagtaa aggctatgag 2940

caatggctaa caatagataa cagctttacg ttcaaattca gtttatattc ttatgattta 3000
gtacgacttg taaaaggtga tgaagatcgt ttcctatact ttagtactct tgatattaat 3060
tcggaccgtc ttaattttaa agatgtaaat aagccatcaa agcaggctga atatcgttat 3120
agtcttaaaa caattgagaa tttagaaaaa tatgaggttg gtgttttagg tgatttaagg 3180
ttagtgagac aagaaacacg taaaattttt taa 3213

<210> 27
<211> 3246
<212> DNA
<213> Bacillus cereus

<400> 27
ttgaaatacg tattaggatt ggatattgga atcgcatctt gtggttgggc tgtaataaat 60
caagaaaaac atagaattga agatttaggg gtacgtattt ttgataaagc tgagcaccct 120
aaagatggaa aatccttagc agcaccaaga cgtgaatctc gctctactag gaaaacactt 180
cgtcgtaaaa aacatagaat gcaacgtatc aaagtacttt tagttaagta tggtttatta 240
tctacgactg agattgaaaa tttatatgaa agtgctagtg aaattgatgt ttggaaccta 300
cgtttagatg ctctagaacg caagttaaat caaaaggaat ttgcaagagt ccttattcac 360
cttgctaaaa gaagaggatt taaatctaac atgaaagaaa caattctctc agaaaacgga 420
caaatcttag aaagtatttc agaaaatcaa aagattatgg agcaacaaaa ttatagaact 480
gttggagaaa tgattgtaaa agataacaag tttgaaaacc ataaaagaaa caaagatggt 540
acttatatcg ggactgttac tcgtcaacaa cttcaagatg aaatacatat gattttcaat 600
gcacaacgtt tgtttataaa tgaccttgca acagaggaat ttgaaagtag ctacttagaa 660
atttgggcat ctcaacgtcc ctatgcttct aaagatcaaa ttgaaaaaat gattggttac 720
tgtaccttag aaccaaaaga aaaaagagtc cctaaagctt catggtcatt tcaatatttt 780
gtagcgctac aaaccataaa caacctacga ctgattagta aagacaggat agaagaactc 840
tcttttgaag aaaagaaaca aattatgaat ctagcacttg aaaaacctat tgttaaatac 900
attgatatcc gaaaattatt atccatttct aatgaacttc attttaacaa tctattatat 960
tcagctgata cagtagatac aacggtggaa aataaaaaat gtatagaact caaagaatat 1020
cactctatta ataaggtata taaacaaatt tatggaaaga acgccttaaa tttattacct 1080
atagattacg atacaattgc atacggacta actatcttca aagacgataa agatatccta 1140
gaatacttac aaaataaata tataaataca aaaggaaaaa ctataagtaa tttaacaaaa 1200
aagacatacg atgacacgtt tattcaagca ttattgactt tgaacttcac aaaaatggga 1260
catttatcat ttaaagcctt aaaaaatatc attccttttt tagaagaggg aatgtcatat 1320
gataaagctt gtgataaggc aggatataac tttaagggaa catcatttac aaaaaaaaca 1380
aagtacttgc ctgttattcc acagaataca aatcctgttg tacatcgtgc tctttcacaa 1440
acaagaaagg tcataaatgc aattataaat aaatacggtt ctccagtttc aattcatatt 1500
gaaaccgcaa gagagttatc taaaacattc caggaaagaa aagaaataga tagcatgtac 1560
caggataact ccaaaaagaa tgaacacgcc attaataagc taaaagaatt aggtaacatt 1620
aatccctcgg gaataaatat tgtcaaattt aagctttgga aggaacaaga cggtagatgt 1680
atgtattccg gagaatatat tgaaccacat cgattatttg aagagggata taccgaagta 1740
gatcacatac taccctatag tcgtagttta gatgacagct ataacaataa aacattagct 1800
ttaggaatag aaaatcaacg aaaagggaac aaaactcctt atgagtacat ggggaacacc 1860
tcaatttggt acaaatttga aacgagggta cagtcgaata aaaaaattaa caaaaagaaa 1920
caacaaaaat tgcttctaca atacttttcg catgcaagag agcaagaatt tatagagcga 1980
aatctaaatg atacccgtta cgccacaata tatatatcag cgtttattca acaacacctc 2040
attttctctg aaagcttcag taaaaaaaga gttcatacag ttagcggaat tataacatct 2100
catgtacgta gtagatgggg atttaacaag gatcgtaaag aaagtcatat ccatcacgca 2160
ttagatgcag tgattgttgc tgtcacatct gatcaaatga ttcaaagagt aacaaattac 2220
tataaactaa aagaattaaa tagaaagctt caagccaaag ggatactgtt tcccgaacct 2280
tgggaggggt ttcgtttaga attagaagca agaatttctc caaataccca gcaatattta 2340
aagggaattt gttctaaaaa ttacgccgat atcaatctat ccgaaatcaa gcctatcttt 2400
gtttcaagaa tgcctaagcg atctattact ggtgaattac atcaagagac aattcgaaaa 2460
tttattggtt ataacgaaaa agggaaagta ttaaccgcaa taaaaacaaa gttagaagat 2520
attccttttg atgccaatgg tgattttcct atgtatggaa aagaaactga tttgtatacg 2580
tataatgcca tcaaagaacg ttatttaagt cataaaaaag ataaacgtaa atcatttcaa 2640
gaatccttat acaaacccac aaaaacagga gaaattgggc cacttataaa atccattaaa 2700
ataattgata caagaaccat tgtcaatcct gttaaccaag gtaaaggtgt cgtatataat 2760
agtaaaattg ctcgtactga tgtattcaaa aaagatgata agtactattt aattcccatt 2820
tatacaattg accttatgaa aaatgtttta cctcaaaaag caattacagc aggaaaagga 2880
tatgaagatt ggataaccat tgatcatagc tttacttttt tatttagcct ttttcctaat 2940
gacctaataa aaattgtccc ctcaaaaaat aaagaaatta aagctaggtc aacttcatca 3000
aaaaaagaaa tattactacc atcactcaca ggatatttta agaaggttca tagtggaaca 3060

gcaggcatta ctgttgaatc ccacgatggt aggtttatag cagatatagg ctcaaagcaa 3120
ttattattat ttgaaaaaca ccaggtagat gtactgggac attacacaaa aataaaggag 3180
gaaaaacgta ttgggatgtc gacatgtaat gataacaaaa agagctcagc tttcggttcg 3240
ctataa 3246

<210> 28
<211> 4137
<212> DNA
<213> Lactobacillus fermentum

<400> 28
gtgaaagagt atcacattgg cttggacatt gggacaagct caattggctg ggcggtaacg 60
gatagccaat ttaaactgat gcgtatcaag gggaagacgg caatcggggt gcggttgttt 120
gaagaaggga agacggctgc agaacgtcga accttccgga caacgcggcg gcggttgaag 180
cggcgaaaat ggcgcttgca ctacttagat aaaatttttg ccccgcactt acaagaagtg 240
gatgaaaatt tcttacgccg tctgaagcaa tcaaatatcc acccggaaga tccagcgaag 300
aatcaagcct tcattggtaa gctcttattt ccggatctac tgaagaagaa tgaacgtggt 360
tacccaacac tgattaagat gcgcgatgaa ttgccagttg agcaacgcgc ccactaccca 420
gtaacgaaca tctacaagtt acgggaggcc atgattaacg aagatcgcca attcgatctg 480
cgggaagttt acctggccgt tcaccacatc gtgaagtacc ggggtcactt cttaaataac 540
gctagcgttg ataagttcaa agtggggaga atcgattttg acaaatcctt taacgttttg 600
aacgaagcct acgaggagct tcaaaacggc gaaggttcat tcacgattga gcctagcaag 660
gttgaaaaaa ttggacaact gttgctagat acgaagatgc gtaagttgga ccgccaaaaa 720
gcggttgcca aattgttaga ggtgaaggtc gctgacaaag aggagaccaa gcgcaacaag 780
caaattgcaa cggcgatgag taagttagtt cttggctaca aggcggactt tgcaacggta 840
gcgatggcga atggcaacga atggaagatc gatttaagtt ctgaaaccag tgaggacgag 900
attgaaaaat tccgggaaga attgagtgat gctcaaaatg atatcttaac ggaaatcact 960
agcctgttct cgcagatcat gctgaatgaa atcgtgccaa atgggatgtc gatttctgaa 1020
tcgatgatgg atcgttattg gatgcacgaa cgtcaattag cggaagttaa ggaatacttg 1080
gcgacccaac cagctagcgc gcggaaagat tttgaccaag tgtataacaa atacattggg 1140
caagcgccaa aggaaaaggg ctttgacctt gaaaagggac tcaagaagat tttatccaaa 1200
aaggaaaatt ggaaagaaat cgatgagtta ctcaaggcag gtgatttctt accaaagcaa 1260
cggacatcag ccaatggggt catcccacac cagatgcacc aacaagaatt ggaccgaatt 1320
attgaaaagc aagctaagta ctacccgtgg ctagcaacgg aaaacccagc cactggtgaa 1380
cgtgatcgtc accaagcgaa gtatgaactt gatcagctag tttccttccg gattccttac 1440
tatgttggac cgctagtaac gcctgaggtt caaaaggcga catctggcgc taagtttgct 1500
tgggcaaagc ggaaagaaga tggggagatt accccttgga acctgtggga taagattgac 1560
cgtgctgaat cagctgaagc ctttatcaag cggatgacgg ttaaggatac gtatctatta 1620
aacgaggatg tcctgcctgc taacagcttg ctataccaaa agtacaacgt cttaaacgaa 1680
ctaaacaacg ttcgagtgaa tggacgccgc ctatcagtcg gaattaaaca agatatttac 1740
actgagctct tcaagaagaa aaagacggtg aaggcaggtg acgtggccag cctagtgatg 1800
gcgaagacac gaggagttaa caagccaagt gttgaaggtt tatccgatcc gaagaagttc 1860
aattcaaatt tggcaaccta ccttgatcta aagtcaattg tgggtgacaa ggttgatgat 1920
aaccgttatc aaatggatct cgaaaacatt atcgagtggc gttccgtctt tgaagatggg 1980
gaaatttttg cggacaagtt aactgaagtg gaatggctga cggatgagca acgttctgcc 2040
cttgttaaga aacggtataa gggctgggga cgtttatcca agaagctact gacgggaatc 2100
gttgacgaga acggccaacg gatcattgat ctgatgtgga atactgatca aaacttcatg 2160
cagattgtca accagcctgt ctttaaagaa cagattgacc aactgaacca aaaggccatc 2220
accaatgatg ggatgacttt gcgggagcgg gtcgagagcg ttctggatga cgcctatact 2280
tcaccgcaga acaagaaggc catctggcaa gttgttcggg tggtggaaga tattgttaaa 2340
gcggtcggga atgcaccaaa gtcgatttca attgaatttg cccgtaatga aggcaacaag 2400
ggagaaataa cccggagccg gcgtactcaa ttgcagaaat tatttgaaga tcaggcccat 2460
gaattagtta aggatacgag cttgactgag gaattggaga aggcaccgga tctttccgat 2520
cgttattact tctacttcac ccaaggtggt aaggatatgt acaccggtga tccaatcaac 2580
ttcgatgaaa tttctaccaa gtatgatatt gaccacattt tgccacaatc cttcgtgaag 2640
gatgattcct tagataatcg ggtgttagta agtcgggcgg agaataataa gaagtctgac 2700
cgggtgccag ctaagcttta cgcagccaag atgaaaccat actggaacca acttttgaag 2760
cagggattga tcacgcaacg gaagtttgaa aacctgacga tggacgttga tcaaaatatt 2820
aagtatcggt cacttggatt cgtgaagcgc caattagttg aaacacgcca agttatcaag 2880
ttgacggcta acatcttggg tagtatgtac caagaagccg ggacggacat cattgagact 2940
cgggctggtt taactaagca attacgcgag gaattcgatt taccaaaggt tcgtgaagta 3000
aacgactatc accacgcagt tgatgcttac ctgacaacgt ttgctgggca atatttgaac 3060
cgccgttatc cgaaattacg ctccttcttc gtatacggtg agtacatgaa gttcaagcat 3120

gggtcagatc tgaagctacg taactttaac ttcttccatg aattgatgga aggcgacaag 3180
agccaaggta aggttgtcga tcaacaaact ggtgagctga ttacgacaag ggatgaagta 3240
gccaagagct ttgatcgatt attgaacatg aagtatatgc tggtatcgaa ggaagttcac 3300
gaccggagtg accaactgta tggagcaacg attgtaacag caaaggaatc tggtaagctc 3360
acttcaccaa ttgagatcaa gaaaaatcga cctgttgatt tgtatggtgc ttatacgaat 3420
ggaacgtcag ctttcatgac gatcattaag tttactggaa acaaacctaa gtataaggtg 3480
attggggttc caacaacatc ggcggtcggt ttaaaacgag ttgggaaacc aggcagtgaa 3540
tcatataacc aagaattaca tcgaatcatt aaatctaatc cgaaggtaaa gaaagatttt 3600
gagattgtag ttccacatgt ttcgtatagt cagttgatag ttgatggtga ctgcaaattt 3660
accctagcta gtgatactta tcagcatccg gcaactcagc tagttctttc taaagaatcg 3720
atggaaataa ttgcagatga ttttaaattt gtgaaagaaa atccggcaac tgcggatgaa 3780
caacttgtta gggtttttga tgaaattgtt aatcaaatga accattactt tactatcttt 3840
gaccaacgta gtaatcgtca aaaggtaacg aaggctcgag ataagtttgt atcgttacca 3900
acggaatcag agtatgaggg tgctaagaaa acgcaagtag ggaagacaga agtcattacg 3960
aacttactga tgggtctaca tgccaatgct gcacaaggtg atttgaaagt attaggatta 4020
tcaacctttg ggtttttcca gagttcaggt ggactgaatt taagtgaaga tgcaatgatc 4080
gtataccagt caccaaccgg tttgtttgaa cgtcgcattt gtttgaaaga catttaa 4137

<210> 29
<211> 4014
<212> DNA
<213> Enterococcus faecalis

<400> 29
atgaagaaag actacgttat cggtttggat atagggacta attctgtcgg ttgggcagtt 60
atgacagaag actatcagtt ggtgaagaaa aaaatgccta tttatggaaa cactgaaaaa 120
aagaaaatca agaaaaattt ttggggtgtg cgtttatttg aagaaggcca tacagccgaa 180
gatcgccgat taaaaagaac agcacgacga agaattagtc gtcgacggaa tcgtttacgc 240
tacttacaag ctttttttga agaagcgatg acagctttgg atgaaaactt ttttgctcgt 300
ttacaagaga gttttttagt gcctgaagat aagaagtggc acagacatcc gatttttgct 360
aagttggaag atgaagtagc ttaccatgaa acgtatccga caatctacca tttacgcaaa 420
aaattagcag attcatctga gcaagcagat ttacgactaa tttatttggc gttggcatat 480
attgtcaaat atcgtggaca ttttttaatt gaagggaaat taagtacaga aaatatttct 540
gttaaagaaa aatttcaaca atttatgatc atttataacc agacctttgt gaatggagag 600
ggtcgcttag ttagtgcgcc attacctgaa tctgtcttga ttgaggaaga gttgactgaa 660
aaagcttctc ggactaaaaa atctgaaaaa gtcttacaac aatttcctca agaaaaagct 720
aatggcttat ttggtcagtt cttaaagcta atggtaggga ataaggctga ctttaaaaaa 780
gtttttggtt tggaagaaga agccaaaata acctacgcta gtgaaagcta tgaagaagat 840
ttagaaggca ttttagcaaa ggttggggat gaatatagtg acgtgttttt agctgctaaa 900
aatgtgtacg atgcagttga attatcaacg attttagcag attcggataa aaaaagttac 960
gcgaaattat cttctagcat gattgtccgt tttacagaac atcaagaaga tttaaaaaaa 1020
ttcaaacgat ttattcgtga aaattgtcca gacgaatatg ataatctctt taaaaatgaa 1080
cagaaagacg gctatgcagg ctacattgca cacgcaggta aggtttcaca gcttaaattt 1140
taccagtatg ttaagaaaat cattcaagat attgctggag cagaatattt tttagaaaaa 1200
attgctcaag aaaacttttt aagaaagcaa cggacctttg ataatggggt gattcctcat 1260
caaattcatt tggctgagtt acaagcaatc attcatcgtc aagcggccta ttatccattt 1320
ttaaaagaaa atcaagaaaa aattgaacaa ctggttacat ttcgaattcc ttattatgtc 1380
ggaccattat caaaaggaga tgcaagtacc ttcgcttggc taaaacgtca aagtgaggaa 1440
ccaattcgac cttggaacct tcaagaaacc gttgatttgg accaatcagc gacagccttt 1500
attgaacgaa tgaccaattt tgatacgtat ttaccttctg aaaaagtttt accgaaacat 1560
agtttgttat atgaaaagtt tatggtattt aatgaattga ccaagatttc ttatacggat 1620
gatcgaggca tcaaagccaa tttttcaggt aaggaaaaag aaaaaatctt tgattatctg 1680
tttaagacgc gtcgcaaagt taagaaaaag gatatcattc aattctatcg aaacgaatat 1740
aatacggaga ttgtcacgct tagtggactt gaagaagacc aatttaatgc tagttttagc 1800
acgtatcagg atttactgaa atgtggtttg acacgagcag aattagacca cccagataat 1860
gccgaaaaat tggaagatat cataaaaatt ttaactattt ttgaagatcg gcaacgaatt 1920
cggacgcaac tttctacatt caaagggcag ttctcagcag aagtactaaa aaaattagaa 1980
cgcaagcact acactggctg gggaagattg tcgaagaaat taatcaatgg tatctacgat 2040
aaagaatcgg gtaaaacaat tttggactat ttagttaaag atgatggggt ctcaaaacac 2100
tataatcgca attttatgca actgattaat gattcacaat tatcttttaa aaatgctatt 2160
caaaaagcac agtccagtga acatgaagaa acattaagtg aaactgtcaa tgaattagct 2220
ggtagcccag caataaaaaa aggaatttat caaagtttaa aaattgtcga tgaactagtc 2280
gcgattatgg gttatgcgcc taagcggatt gttgtcgaaa tggcacgtga aaatcaaacg 2340

actagcactg gcaagagaag atccatccaa cgcttgaaaa tagttgaaaa agcaatggcc 2400
gaaatcggca gcaatttatt aaaagaacaa ccaacgacta atgaacagtt acgagatacc 2460
cgtcttttcc tttactatat gcaaaatggt aaagatatgt acacgggaga tgaattatcg 2520
cttcatcgtt tatctcacta tgatattgat catattatcc cacaaagttt tatgaaagac 2580
gattcattgg ataacctagt tttagtgggc tctactgaaa atcgagggaa atccgatgat 2640
gtacctagca aggaagttgt taaggatatg aaagcttatt gggagaaatt atatgctgct 2700
ggcttaatta gtcaacggaa attccaacgt ctgaccaaag ggagcaaggg tggcttgact 2760
ctcgaagaca aagcgcattt tatccaacga caattagtag aaactcgtca aattaccaaa 2820
aatgttgcag ggatcttaga tcaacgctac aatgccaagt caaaagagaa aaaagtccaa 2880
atcatcacct tgaaagctag tttaacgagc caatttcgtt cgatttttgg cttgtataaa 2940
gttcgtgaag tgaatgatta tcaccatgga caagatgctt atttaaactg tgtggtcgca 3000
accacgttat taaaagttta tcctaattta gcaccagaat ttgtttacgg agaatatcct 3060
aagttccaga cctttaaaga aaataaagcc acggcgaagg ctataattta tacaaatttg 3120
ctgcgctttt tcacggaaga tgaaccacgg ttcacgaagg atggcgaaat tctttggagt 3180
aacagttatt taaaaactat caaaaaagaa ttaaattacc atcaaatgaa tattgtcaaa 3240
aaagtagaag tgcaaaaagg cggtttttca aaagagtcga ttaaacccaa aggaccatcg 3300
aataaattga ttcctgtcaa aaatggtttg gatccccaaa aatatggcgg ttttgatagt 3360
cctatagttg catatacagt gctattcacg catgaaaaag gtaaaaaacc tcttattaaa 3420
caggagatac tgggcattac gattatggaa aaaacaaggt ttgaacaaaa tcctattctt 3480
tttttagagg agaaaggctt cctacggcct cgtgtattaa tgaaattgcc taagtataca 3540
ctgtatgaat ttccagaggg acgccggcgc ttattagcca gcgctaaaga agcgcaaaaa 3600
gggaaccaga tggttttacc tgaacattta cttacattac tgtaccatgc gaaacaatgt 3660
ttgttaccaa atcaatcaga aagtttggct tatgtagaac aacatcaacc agaatttcaa 3720
gagattttag agagagtggt tgactttgct gaagtgcata cattagccaa gtcaaaagtc 3780
cagcaaattg tgaaactttt tgaagcaaat caaacagcag atgtgaaaga gattgcggca 3840
tcctttatcc aacttatgca gtttaatgcg atgggcgcac caagtacgtt taaatttttc 3900
caaaaagaca ttgagcgagc acgctacacg tcaatcaaag aaatttttga tgccactatc 3960
atttaccaat cgactacggg actgtatgaa acacgcagaa aggttgtcga ctag 4014

<210> 30
<211> 4014
<212> DNA
<213> Bacillus cereus

<400> 30
atgaagaaag attacgttat tggtctggat ataggaacta attctgtcgg ctgggcagtt 60
atgacagaag actatcagtt ggtgaagaaa aaaatgccta tttatggaaa cactgaaaaa 120
aagaaaatta agaaaaattt ttggggtgtg cgtttatttg aagaagggca tacagccgaa 180
gatcgccgat taaaaagaac agcacgacga agaattagtc gtcgacggaa tcgtttacgc 240
tacttacaag ctttttttga agaagcgatg acagatttgg atgaaaactt ttttgctcgt 300
ttacatgaga gttttttagt gcctgaagat aagaagtggc acagacatcc gatttttgct 360
aagttggaag atgaagtagc ttaccatgaa acgtatccga caatctacca tttacgcaaa 420
aaattagcag attcatctga gcaagcagat ttacgactaa tttatttggc gttggcccat 480
attgtcaaat atcgtggaca ttttttaatt gaaggaaaac taagtacaga aaatacttct 540
gttaaagaac aatttcaaca atttatggtc atttataacc aaacctttgt gaatggggag 600
agtcgcttag ttagtgcgcc tttacctgaa tctgtcttga ttgaggaaga gttaactgaa 660
aaagcttctc ggactaaaaa atctgaaaaa gtcttacaac aatttcctca agaaaaagct 720
aatggcttat ttggtcagtt tttaaagcta atggtaggga ataaagctga ctttaaaaaa 780
gtttttgctt tggaagaaga agccaaaata acctacgcta gtgaaagtta tgaagaagat 840
ttagaaggca ttttagcaaa gattggggat gaatatagtg acgtgttttt agctgctaaa 900
aatgtgtacg atgcagttga attatcaacg attttagcag attcggataa aaaaagtcac 960
gcgaaattat cttctagcat gattgtccgt tttacagaac atcaagaaga tttaaaaaaa 1020
ttcaaacggt ttattcgtga aaattgtcca gacgaatatg ataatctctt taaaaatgaa 1080
cagaaagacg gctatgcagg ttacattgca cacgcaggta aggtttcaca gcttaaattt 1140
taccagtatg ttaagaaaat cattcaagat attgctggag cagaatattt tttagaaaaa 1200
attgctcaag aaaacttttt aagaaagcaa cggacctttg ataatggggt gattcctcat 1260
caaattcatt tggctgagtt acaagcaatc attcatcgtc aagcggccta ttatccattt 1320
ttaaaagaaa atcaagaaaa aattgaacaa ctggttacat ttcgaattcc ttattatgtc 1380
ggaccattat caaaaggaga tgcaagtacc ttcgcttggt taaaacgtca aaatgagaaa 1440
ccaattcgac cttggaacct tcaagaaacc gttgatttgg accaatcagc gacagccttt 1500
attgaacgaa tgaccaattt tgatacgtat ttaccttctg aaaaagtttt accgaaacat 1560
agtttgttat atgaaaagtt tatggtattt aatgaattga ccaagatttc ttatacggat 1620
gatcgaggaa tcaaagccaa tttttcaggt aaggaaaaag aaaaaatctt tgattatctg 1680

tttaagacgc gtcgaaaagt taagaaaaag gatataattc aattctatcg aaacgaatat 1740
aatacggaga ttgtcacgct tagtggactt gaagaagacc aatttaatgc tagttttagc 1800
acgtatcagg atttactgaa atgtggtttg acaagagcag aattagacca cccagataat 1860
gccgaaaaat tggaagatat cataaaaatt ttaactattt ttgaagatcg gcaacgaatt 1920
cggacgcaac tttccacatt caaagggcag ttctcagcag aagtactaaa aaaattagaa 1980
cgcaagcact acactggctg gggaagattg tcgaagaaat taatcaatgg tatctacgat 2040
aaagaatcgg gtaaaacaat tttggactat ttagttaaag atgatggggt ctcaaaacac 2100
tataatcgca attttatgca actgattaat gattcacaat tatcttttaa aaatgcgatt 2160
caaaaagcac agtccagtga acatgaagaa acattaagtg aaactgtcaa tgaattagct 2220
ggtagcccag caataaaaaa aggaatttat caaagtttaa aaattgtcga tgaactagtc 2280
gcgattatgg gttatgcgcc taagcggatt gttgtcgaaa tggcacgtga aaatcaaacg 2340
actagcactg gcaagagaag atccatccaa cgcttgaaaa tagttgaaaa agcaatggcc 2400
gaaatcggca gcaatttatt aaaagaacaa ccaacgacta atgaacagtt acgagatacc 2460
cgtcttttcc tttactatat gcaaaatggt aaagatatgt acacgggaga tgaattatcg 2520
cttcatcgtt tatctcacta tgatattgat catattatcc cacaaagttt tatgaaagac 2580
gattcattgg ataacctagt tttagtgggc tctactgaaa atcgagggaa atccgatgat 2640
gtacctagca aggaagttgt taaggatatg aaagcttatt gggagaaatt atatgctgct 2700
ggcttaatta gtcaacggaa attccaacgt ctgaccaagg gggagcaagg tggcttgact 2760
ctcgaagaca aagcgcattt tatccaacgg caattagtag aaactcgtca aattaccaaa 2820
aatgttgcag ggatcttaga tcaacgctac aatgccaagt caaaagagaa aaaagtccaa 2880
atcatcacct tgaaagctag tttaacgagc caatttcgtt cgatttttgg cttgtataaa 2940
gtccgtgaag tgaatgatta tcaccatgga caagatgctt atttaaactg tgtggtcgca 3000
accacgttat taaaagtttg tcctaattta gcaccagaat ttgtttacgg agaatatcct 3060
aagttccaga cctttaaaga aaataaagcc acggcgaagg ctataattta tacaaatttg 3120
ctgcgctttt tcacggaaga tgaaccacgg ttcacgaagg atggcgaaat tctttggagt 3180
aacagttatt taaaaactat caaaaaagaa ttaaattacc atcaaatgaa tattgtcaaa 3240
aaagtagaag tgcaaaaagg cggtttttca aaagagtcga ttaaacccaa aggaccatcg 3300
aataaattga ttcctgtcaa aaatggtttg gatccccaaa aatatggcgg ttttgatagt 3360
cctgtagttg cttatacagt gctattcacg catgaaaaag gtaaaaaacc tcttattaaa 3420
caggagatac tgggcattac gattatggaa aaaacaaggt ttgaacaaaa tcctattctt 3480
tttttagagg agaaaggctt cctgcggcct cgtgtattaa tgaaattgcc taagtataca 3540
ctgtatgaat ttccagaggg gcgccggcgc ttattagcta gtgctaaaga agcgcaaaaa 3600
gggaaccaga tggttttacc cgaacattta cttacattac tgtaccatgc gaaacaatgt 3660
ttgttaccaa atcaatcaga aagtttggct tatgtagaac aacatcaacc agaatttcaa 3720
gagattttag agagagtggt tgactttgct gaagtgcata cattagccaa gtcaaaagtc 3780
cagcaaattg tgaaactttt tgaagcaaat caaacagcag atgtgaaaga gattgcggca 3840
tcctttatcc aacttatgca gtttaatgcg atgggcgcac caagtacgtt taaatttttc 3900
caaaaagata ttgagcgagc acgctacacg tcaatcaaag aaatttttga tgccactatc 3960
atttaccaat cgactacggg cctgtatgaa acacgcagaa aggttgtcga ctag 4014

<210> 31
<211> 4014
<212> DNA
<213> Enterococcus faecalis

<400> 31
atgaagaaag actacgttat cggtttggat ataggactaa attctgtcgg ttgggcagtt 60
atgacagaag actatcagtt ggtgaagaaa aaaatgccta tttatggaaa cactgaaaaa 120
aagaaaatca agaaaaattt ttggggtgtg cgtttatttg aagaaggcca tacagctgaa 180
gatcgccgat taaaaagaac agcacgacga agaattagtc gtcgacggaa tcgtttacgc 240
tacttacaag ctttttttga agaagcgatg acagatttgg atgaaaactt ttttgctcgt 300
ttacaagaga gttttttagt gcctgaagat aagaagtggc acagacatcc gatttttgct 360
aagttggaag atgaagtagc ttaccatgaa acgtatccga caatctacca tttacgcaaa 420
aaattagcag attcatctga gcaagcagat ttacgactaa tttatttggc gttggcccat 480
attgtcaaat atcgtggaca ttttttaatt gaaggaaaac taagtacaga aaatacttct 540
gttaaagaac aatttcaaca atttatggtc atttataacc aaacctttgt gaatggagag 600
agtcgcttag ttagtgcgcc attacctgaa tctgtcttga ttgaggaaga gttaactgaa 660
aaagcttctc ggactaaaaa atctgaaaaa gtcttacaac aatttcctca agaaaaagct 720
aatggcttat ttggtcagtt cttaaagcta atggtaggga ataaagctga ctttaaaaaa 780
gtttttggtt tggaagaaga agccaaaata acctacgcta gtgaaagcta tgaagaagat 840
ttagaaggca ttttagcaaa ggttggggat gagtatagtg acgtgttttt agctgctaaa 900
aatgtgtacg atgcagttga attatcaacg attttagcag attcggataa aaaaagtcac 960
gcgaaattat cttctagcat gattgtccgt tttacagaac accaagaaga tttaaaaaaa 1020

ttcaaacgat ttattcgtga aaattgtcca gacgaatatg ataatctctt taaaaatgaa 1080
cagaaagacg gttatgcagg ctacattgcg cacgcaggta aggtttcaca gcttaaattt 1140
taccagtatg ttaagaaaat cattcaagat attgctggag cagaatattt tttagaaaaa 1200
attgctcaag aaaacttttt aagaaagcaa cggacctttg ataatggggt gattcctcat 1260
caaattcatt tggctgagtt acaagcaatc attcatcgtc aagcggccta ttatccattt 1320
ttaaaagaaa atcaagaaaa aattgaacaa ctggttacat ttcgaattcc ttattatgtc 1380
ggaccattat caaaaggaga tgcaagtacc ttcgcttggt taaaacgtca aagtgaggaa 1440
ccaattcgac cttggaacct tcaagaaacc gttgatttgg accaatcagc gacagccttt 1500
attgaacgaa tgaccaattt tgatacgtat ttaccttctg aaaaagtttt accgaaacat 1560
agtttgttat atgaaaagtt tatggtattt aatgaattga ccaagatttc ttatacggat 1620
gatcgaggaa tcaaagccaa tttttcaggt aaggaaaaag aaaaaatctt tgattatctg 1680
tttaagacgc gtcgaaaagt taagaaaaag gatataattc aattctatcg aaacgaatat 1740
aatacggaga ttgtcacgct tagtggactt gaagaagacc aatttaatgc tagttttagc 1800
acgtatcagg atttactgaa atgtggtttg acaagagcag aattagacca cccagataat 1860
gccgaaaaat tggaagatat cataaaaatt ttaactattt ttgaagatcg gcaacgaatt 1920
cggacgcaac tttccacatt caaagggcag ttctcagcag aagtactaaa aaaattagaa 1980
cgcaagcact acactggctg gggaagattg tcgaagaaat taatcaatgg tatctacgat 2040
aaagaatcgg gtaaaacaat tttggactat ttagttaaag atgatggggt ctcaaaacac 2100
tataatcgca attttatgca actgattaat gattcacaat tatcttttaa aaatgctatt 2160
caaaaagcac agtccagtga acatgaagaa acattaagtg aaactgtcaa tgaattagct 2220
ggtagcccag caataaaaaa aggaatttat caaagtttaa aaattgtcga tgaactagtc 2280
gcgattatgg gttatgcgcc taagcggatt gttgtcgaaa tggcacgtga aaatcaaacg 2340
actagcactg gcaaaagaag atccatccaa cgcttgaaaa tagttgaaaa agcaatggcc 2400
gaaatcggca gcaatttatt aaaagaacaa ccaacgacca atgaacagtt acgagatacc 2460
cgtcttttcc tttactatat gcaaaatggt aaagatatgt acacgggaga tgaattatcg 2520
cttcatcgtt tatctcacta tgatattgat catattatcc cacaaagttt tatgaaagac 2580
gattcattgg ataacctagt tttagtgggc tctactgaaa atcgagggaa atctgatgac 2640
gtcccaagta agaaagttgt taagaagatg aaagcttatt gggagaaatt atatgctgct 2700
ggcttaatta gtcaacggaa attccaacgt ctgaccaagg gggagcaagg tggcttgact 2760
ctcgaagaca aagcgcattt tatccaacgg caattagtag aaactcgtca aattaccaaa 2820
aatgttgcag ggatcttaga tcaacgctac aatgccaagt caaaagagaa aaaagtccaa 2880
atcatcacct tgaaagctag tttaacgagc caatttcgtt cgatttttgg cttgtataaa 2940
gtccgtgaag tgaatgatta tcaccatgga caagatgctt atttaaactg tgtggtcgca 3000
accacgttat taaaagttta ccctaattta gcaccagaat ttgtttacgg agaatatcct 3060
aaattccagg cctttaaaga aaataaagcc acggcgaaga ctataattta tacaaatttg 3120
atgcgctttt tcacggaaga tgaaccacgg ttcacgaagg atagcgaaat tctttggagt 3180
aacagttatt tgaaaactat caaaaaagaa ttaaattacc atcaaatgaa tattgtcaaa 3240
aaagtagaag tgcaaaaagg cggtttttca aaagagtcga ttaaacccaa aggaccatcg 3300
aataaattga ttcctgtcaa aaatggtttg gatccccaaa aatatggcgg ttttgatagt 3360
cctgtagttg cttatacagt gctattcacg catgaaaaag gtaaaaaacc tcttattaaa 3420
caggagatac tgggcattac gattatggaa aaaacaaggt ttgaacaaaa tcctattctt 3480
tttttagagg agaaaggctt cctgcggcct cgtgtattaa tgaaattgcc taagtataca 3540
ctgtatgaat ttccagaggg gcgccggcgc ttattagcta gtgctaaaga agcgcaaaaa 3600
gggaaccaga tggttttacc tgaacattta cttacattac tgtaccatgc gaaacaatgt 3660
ttattaccaa atcaatcaga aagtttggct tatgtagaac aacatcaacc agaatttcaa 3720
gagattttag agagagtggt tgactttgct gaagtgcata cattagccaa gtcaaaagtc 3780
cagcaaattg tgaaactttt tgaagcaaat caaacagcag atgtgaaaga gattgcggca 3840
tcctttatcc aacttatgca gtttaatgcg atgggcgcac caagtacgtt taaatttttc 3900
caaaaagata ttgagcgagc acgctacacg tcaatcaaag aaatttttga tgccactatc 3960
atttaccaat cgactacggg cctgtatgaa acacgcagaa aggttgtcga ctag 4014

<210> 32
<211> 3993
<212> DNA
<213> Artificial sequence

<220>
<223> Cas endonuclease of Locus 14

<400> 32
atggaaaaag agtatacgat tggattagat attgggacaa attcggttgg atgggcagtg 60
ttgacggatg attatcgatt agtcgcaaga aaaatgagta ttcaaggaga ctcgaataga 120
aagaaaataa aaaagaattt ttggggtgca cgtttattcg aagaaggtaa aactgcacaa 180

tttcgacgga tcaaacgaac gaaccgtcgt aggatcgcac gaagacgtca aagagtttta 240
gcattacaag atatttttgc agaagagata cataaaaaag atcccaattt ttttgctcgg 300
ctagaagaag gagatcgtgt ggaagcagac aaacgatttg ctaagtttcc tgtttttgct 360
acattgtcgg aggaaaaaaa ttatcatcgt caatatccga ccatctacca tttgaggcat 420
gatctagcaa actcaaaaga acaagcagat attcgtttag tgtacttagc gattgcccat 480
tgcttgaagt acagagggca ctttttgttt gaaggagaat tagatactga aaatacctcg 540
gtaaccgaaa attatcagca atttttacaa gcataccaac agttctttcc agaacctata 600
ggagatttgg atgatgctgt ccctattttg acagagcggc tatctaaggc aaaacgagtg 660
gaaaaagtat tagcttatta tccatctgaa aaaagtactg gaaattttgc tcaattcctt 720
aaactaatgg tcggaaatca agcaaatttt aaaaagacgt ttgatttaga agaagaaatg 780
aagttgaatt ttactcgtga ttgttatgaa gaggatttaa atgaactgct agaaaaaaca 840
agtgacgatt atgcagaatt atttttaaaa gcgaagggtg tgtatgatgc gattttactt 900
tcgcaaatcc tttctaagtc agatgatgaa acgaaagcta agttgtctgc gaatatgaag 960
ttgcgctttg aagagcacca acgagacctt aaacaattaa aagaactagt ccgtagagac 1020
ttacctaaaa agtacgatga tttctttaag aatcgcagta agaatggata cgctgggtat 1080
gtcaagggga aagcgacaca agaagatttt tataaatttc taagaacaga actagctgga 1140
ttagaggaaa gccaatcaat catggaaaaa attgatctag agatctatct attgaaacaa 1200
cgtacctttg caaatggggt gattcctcat caaatccatt tagttgaaat gagagaaatt 1260
atggatcggc agaaacgttt ttatcctttt ctaaaagggg cacaaggaaa aatcgagaaa 1320
ctactgacgt ttcgaatccc atattacgta ggaccgttgg cacaagaagg tcaaagtcct 1380
ttcgcttgga tcaaacgtaa gagtccgagc caaatcactc catggaattt tgcagaagtg 1440
gttgataaag aaaattcagc cattgaattt attgagcgaa tgacaaatca agatacgtat 1500
ttaccaaagg aaaaagtttt accaaaacaa agtttgattt atcaaagatt catgatcttc 1560
aatgaattga caaaagtcag ttatacggat gaacgtggca aatctcacta tttttcaagt 1620
gaacaaaaac gcaagatttt taacgagttg ttcaagcaac atccaagagt aacagaaaaa 1680
caattgagga aatttttaga gttgaacgag cagatcgatt caactgaaat caaaggaatt 1740
gaaacgtcat tcaatgcttc ttatagcacc tatcatgatt tgttgaaatt atcagatcag 1800
atggatacat tattagatga tcccgatatg acaacgatgt ttgaagaaat tatcaaaatt 1860
ttgacgattt ttgaagacag agaaatgatt agggaacaac tgaaaccgta cgaaactgtc 1920
ttaggtcttc cagctatcaa gaaattagca aaaaaacatt atactggttg gggacggcta 1980
tcggaaaaaa tgatccaagg aatgcgagag aagcagtccc ggaaaaccat tcttgattat 2040
ttgatcgatg atgatgattt tccttgcaat cgtaatcgaa atttcatgca actaatcaat 2100
gatgatcatc tttcgtttaa agaaacgatt gctaatgaat tgatcatgag cgactcaaac 2160
gtattgcttg atcaagtgaa ggctatccca ggaagtcctg cggtcaaaaa agggatttgg 2220
caaagtatca aaatcgtcga agaaatcatt ggtatcatcg gtaaggcacc taagaacata 2280
gtgattgaaa tggcacgtga gaatcaaaga acaagccgtt caagaccccg cttaaaagcc 2340
ttagaagaag cattgaagaa tatcgacagt ccattgttga aagactatcc aacagataat 2400
caagcgctac aaaaagatcg actgtattta tattatttgc aaaatggaaa agatatgtat 2460
acaggtgaac cactagagat tcatcgttta tcagaatatg atattgacca tattattcct 2520
agaagtttta ttgttgataa ttctttagat aataaggtcc tagtttcatc aaaagtcaat 2580
cgtggaaagt tagataatgc ccggacccca cttgttgtca aaagaatgcg atcacattgg 2640
gaaaagctac atcaagcaaa attgatcagt gacaaaaaac tagccaatct tacaaaacaa 2700
aatctgaccg aggcagacaa ggcaagattt atccaacgcc agttagtcga aacaagacag 2760
ataacgaaac atgttgccaa tctgttacat cagcatttta atttaccaga agaagtaagt 2820
gcgactgaga aaacaagtat cattaccttg aaatcaacgt tgacaagcca attccgtcag 2880
atgttcgata tttataaggt gagagagatc aatcatcacc accatgccca cgacgcttat 2940
ttaaacggag tagtcgcaat gactttacta aaaaaatatc ctaagttggc accagaattt 3000
gtctacggaa gttatatcaa aggggatatc aaccagatca ataaagcaac tgcaaaaaaa 3060
gaattctatt ctaatatcat gaaatttttt gaaagtgaag aaatcatttg tgatgagcaa 3120
ggtgaagtga tttggaataa aaaacgtgat ctctcaacaa tcaagaagac gattggcgca 3180
caccaagtga atatcgttaa gaaagtagaa aaacaaaaag gtggattcta taaggaaaca 3240
attaattcaa aagcgaatcc agaaaaacta attccaagaa aagcatcatt agatcctttg 3300
aaatatggtg gctatggtag cccacttgtg gcatatacgg tgatttttat ctttgaaaaa 3360
ggaaaacaga agaaggtaac caaaggaatc gaaggaatta ccgttatgga acaattgaga 3420
ttcgaacaag acccacgaga gtttctaaaa acaaaaggct atgagggcgt aaaacaatgg 3480
ctgatattgc ctaaatatat attattcgaa gcacaaggtg gatatcggag aatgattgcg 3540
agtcatcaag aaacgcaaaa agctaactca ttgattttac ctgaaaatct agttacattg 3600
ctttatcatg caagacatta cgatgaaatc aatcataaag tgagttttga ttatgtcaat 3660
gcgcataaag aagggtttaa tgacattttt gattttatct ctgactttgg ggtgcgttat 3720
attttggctc ctcaacatct tgaaaaaata aaagttgcct atgaaaaaaa taaagaggta 3780
gatctgaaag aaatgataga tgctatttta tctttattga aatttacgtt atttggtgct 3840
tctgttgagt ttaagttttt tgatattaag atattaaaaa gatataagag tttgaccgac 3900
atttgggaag caaccattat ctaccaatcg gttactggat tatatgaaag gcgagtggag 3960

gtaaggaagt tatgggatgg agaacgcttg taa                                    3993

<210>  33
<211>  3453
<212>  DNA
<213>  Enterococcus faecalis

<400>  33
atgtacagta taggtttaga tttagggatt agtagtgttg gttggtcagt aattgatgaa      60
cggacaggca atgttattga tttaggtgtc cgtttattta gtgcgaaaaa tagtgaaaaa     120
aatttagaac gacgcacaaa tcgaggtgga cgtcgtttaa ttagacggaa aacgaatcgc     180
ttaaaagatg caaaaaaaat acttgcggct gttggatttt atgaagataa atcattaaaa     240
aatagttgcc cgtaccaatt aagagtaaaa ggattgacag aaccattaag caaaggagaa     300
atttataaag tgactcttca cattttaaaa aaacgtggaa ttagttactt agacgaagat     360
gatacggaag cagcgaaaga aagtcaagat tataaggaac aagttaggga aaatgctcag     420
ttgttaacaa aatatacgcc gggtcaaatc caattacagc ggctgaaaga aaataatcga     480
gtgaaaacag gaatcaatgc tcaaggtaat tatcagttaa atgtttttaa agtttctgct     540
tatgcaaatg aattggcaac cattcttaaa acccaacaag ccttttatcc taacgaattg      600
acagatgatt ggattgcatt atttgtacag cctggtattg cagaggaagc tggattaatt     660
tatcgcaaac gaccgtatta tcatggacca ggaaatgaag ccaataacag tccttatggt     720
cgttggtctg attttaaaaa aacaggccaa ccagcaacaa atatttttga taaattgatt      780
ggcaaagact ttcaaggtga actacgagca agcggcttaa gtctatctgc acaacaatat     840
aatttactca atgatttaac caatttaaaa attgatggag aaataccact ttcgctagaa     900
caaaaagaat acattttagc agaattaatg accaaagaat ttactcgttt tggcgttaat     960
gatgtggtta aattgctggg tgtaaaaaaa gaacgattaa gtggttggcg tttggataaa    1020
aaaggtaaac cagaaattca tacattaaaa gcctatcgca attggcggaa aatttttgct     1080
gaagcaggga tcgatttggc tacgttacca acagaaacga ttgactgttt agcgaaagtt    1140
ttaaccttaa ataccgaacg cgaagggatt gaaaatacat tagcttttga actgcctaag    1200
ttagcagaac cagtaaagtc actagtttta gatcattaca aagaattaag ccaaagcatt    1260
agcacgcaag cgtggcatcg tttttcttta aaaacattac acttattaat tccagaacta    1320
atgaacgcta cctcagaaca aaatacactc ttagaacagt ttcagttaaa gtcaggtgtt    1380
agaacacggt atagtgaata taataaatta ccaacaaaag atgtcttggc agaaatttat    1440
aatccaacgg tgaacaaaac agtgagtcaa gccttcaaag ttatggatgc tttgttggaa    1500
aaatatggca aagagcaaat tcgttacatc actattgaaa tgcctaggga cgataacgaa    1560
gaagatgaaa aaaaacgaat aaaagaatta catgcaaaga atagtcaacg aaagaatgac    1620
agccaaagtt attttatgca aaagtctgga tggagccaag aaaaatttca aacgaccatt    1680
caaaaaaatc gtcgtttttt agctaagtta ttatactatt acgaacaaga tggcatatgt    1740
gcctatacag gactgtctat ttctcctgaa ttattggtta gtgattcaac agaaattgat    1800
catattattc caatttcgat tagcttagat gattctatta ataataaagt actggtttta    1860
agtaaagcta atcaagtcaa aggacaacaa acaccatatg atgcatggat ggacggttct    1920
ttcaaaaaaa ctaacggaaa atttagcaac tgggatgact atcaaaaatg ggtggaaagt    1980
cgccatttta gtcataaaaa agaaaataat ttattggaaa cgcggaatat ttttgacagt     2040
gagcaagtcg aaaaattttt agctagaaat ctgaatgata cgcgttatgc cagtcgttta    2100
gttttaaata ccttacagag tttttttgcg aatcagggaa cgaaagttcg agtagtgaac      2160
ggcagtttta ctcatacttt acgtaaaaaa tggggtgccg acttagataa aactcgtgaa    2220
acacatcatc atcatgccgt tgatgcaaca ttgtgtgcag tcactccgtt tgtcaaggtt    2280
agtcgttatc actatgcagt gaaggaagaa actggtgaaa aagtcatgcg tgaaatcgat    2340
ttcgaaactg gtgaaattgt cgatgaaatg tcttaccagg aattcaaaaa atcaaagaag    2400
tatgaacgaa aaacctatca ggtaaaatgg cctaattttc gtgagcaatt gaagccagta     2460
aacctccatc caagaattaa attttctcac caagtggatc gtaaagctaa ccgcaaactc    2520
agtgatgcca ctatttattc tgttcgagag aaaacggaag tcaaaacgct gaaaagtggt    2580
aaacaaaaaa taacgactga cgagtacaca atcggtaaaa tcaaagacat ttacacggta    2640
gatggttggg aagccttcaa gaaaaaacaa gataaacttt taatgaaaga tcttgacgaa    2700
aagacctatg aacgattgtt gtctattgca gaaacaactc ctgattttca agaagtggaa    2760
gaaaaaaatg gtaaggtaaa gcgtgttaaa cgttcaccgt ttgctgttta ctgtgaagaa    2820
aatgatattc cagcgattcg gaaatatgcg aaaaaaaaca atggaccact tattcgtagt    2880
ttgaagtatt atgacggtaa attgaataaa catattaata tcaccaagga ttcacaagga    2940
cgacctgttg aaaaaactaa aaatggtcgc aaagttacac tacaaagttt aaaaccatat     3000
cgctacgata tttatcaaga tttagaaact aaggcttatt atacagtaca actatattat    3060
tctgacttac gttttgtgga aggtaagtat ggaatcacag aaaaagaata catgaaaaaa    3120
gtcgccgaac agacaaaggg acaagtggtg cgtttctgtt tttctttaca aaaaaatgat    3180
ggtttggaaa ttgaatggaa agatagccaa cgctatgatg tgcgttttta taatttccaa    3240
agtgctaata gtattaactt taaaggttta gaacaagaaa tgatgccagc agaaaatcaa    3300

tttaagcaaa aaccttataa taatggagcc atcaatttaa atatcgctaa atatggcaaa 3360
gaaggcaaaa agttacgaaa attcaataca gatattttag gaaagaaaca ttatctgtat 3420
tatgaaaaag aaccaaagaa tattattaaa taa 3453

<210> 34
<211> 4371
<212> DNA
<213> Artificial sequence

<220>
<223> Cas endonuclease of Locus 16

<400> 34
atggtttccc ataaaatgat tacttttttt ctctttattt ttatgcttta tgaaaaccaa 60
atagcaatga ctaaaaatat actaggatta gacttaggaa cgaactcaat aggatgggca 120
ttgataaaac aggatttgga aaacaaacaa ggtgaaattc ttgggatggg aagcagaatt 180
atacctatgt ctcaggatat tttgggggaa tttggaaaag gaaattctgt ttctcaaact 240
gcttctcgga ctagctatag aggtgtgaga agattacgtg aaagatttct tttgcgaagg 300
gaaagattac atagagtttt aaatgttctg aactttttac ccgcacatta tgcttctcag 360
atagattttg aaaagaggtt tgggaaattc aaagaagaaa ctgagcctaa gttggtttat 420
gacgtgaatg aatttatttt taaaaattcg tttgaagaaa tgctttatga ttttagaaag 480
tatcagccac agcttttaga aaatgataaa aaagttccct atgactggac gatttattat 540
ttacgtaaca aagccttaac ccaaaaaata gagaaagaag aacttgcttg gattctttta 600
aattttaatc agaagcgtgg ttattatcaa ttacgtgggg aagattttga agaagaaaaa 660
gataaaactt ttgtacgcct aaaagttgat aaaattgttg attcgggaga cactataaag 720
ggcaaaatat tatatgatgt ttatttcgaa aatggctgga aatatgataa acagattgta 780
aaaacggaag attggataga acgtgtaaaa gaatttattg ttacagaatc attcttaaaa 840
aacggcgata taaaaagaac ttttaaagct gttgattctg aaaaggattg gattgctatc 900
aaaactaaaa cagaacaaga aattgataat tcgaataaaa ccgttgggac atacatttat 960
gaaacacttc ttcgaaatcc aaaacaaaaa attaaaggaa aattggttcg tacgattgaa 1020
agaaaattct ataaagatga gctgaaacag attctggaaa aacaaaaaga atttcatctt 1080
gaattaaaga atgatgattt atatagtgat tgtattcgtg aattgtatcg aaataatgaa 1140
gcgcatcaat tgacgttgag tagaaaagat tttgttcatc ttttgatgga ggatattatt 1200
ttttatcaaa gacctttaag aagtcataaa tcttctattt caaactgtac tttggaattt 1260
agaaaatata aagatgaaaa tggagcagag catattcaat atttaaaagc gattccaaaa 1320
tcaaatccat attatcagga attcagaatc tggcaatgga tttttaatct caatatttat 1380
aagagagatg acgagcaaaa tcctgttaca aaaaagtttt taaacaccac ccaagatttt 1440
gaaaatctgt ttgaatttct gaataacaga aaagaaattg accaaaaggc tttactcaaa 1500
catcttaaaa taaatgagaa aacgcatcgt tggaagtatg tagaagacaa aaagtatcct 1560
tgcaacgaaa ccaaaacaat gatttcatca aggttggata aagttgaaaa tatttctgaa 1620
aacttcctga caagagaaat tgaacagaag atttggcata ttatctattc tgtcaacgat 1680
aaaattgaat atgaaaaagc tttaaaatct tttgccaata aaaataatct ggacgaagtt 1740
tctttctttg aagcttttaa aaaattccca ccttttaaaa gcgaatatgg ttctttttct 1800
gaaaaagcaa taaagaaact gttgccttta atgcgattgg gaaaatattg gaattatgct 1860
gatattgata aatattcaaa agacagaatt cagaaaatta tcaatggaga atatgatgaa 1920
aatatcaaag acaaggtaag ggaaaaagca gttcatctta cgactgaaaa tgattttcaa 1980
ggtcagcaat tgtggctggc tcaatatatt gtttacggaa ggcattctga agcttcaatg 2040
attggaaaat ggaattctgc cgatgatttg gaagagtttt taaaagagtt taagcaacat 2100
tcgcttcgaa atccgattgt tgagcaggtc attaccgaaa ctttgcgcgt tgtaaaagat 2160
atttggttga aatacggaaa tggagcgaaa gatttcttta acgaaattca tattgagttg 2220
ggaagagaaa tgaaacttcc tgcagatgat agaaaaaaac taacgaacca aatttctgaa 2280
aacgaaaata ccaatcttcg tatcaaggct ttgctggctg aaatgataaa cgaccattct 2340
gttgaaaatg ttcgtccgtt ttcgcctatg caacaggaaa ttttgaaaat ttatgaagat 2400
ggagtattga attcaggtat tgaaattgaa gatgaatatt tgaaaatcag caaaaccgca 2460
caaccttctc cgtctgattt gaagagatac aaactttggt tggaacagaa atacaaatcg 2520
ccttacacag gacaaattat tcctttaaat aaattgttta cgccagaata tgaaattgaa 2580
catatcatac ctcaaagccg atattttgat gacagcttta gtaataaaat tatttgtgaa 2640
tcagccgtta ataaattgaa ggataattat atcggacttg gatttattaa gcaattcgga 2700
gggactatta ttgagcttgg ttttggaaaa aatgtaaagg tttttgaagt cgaagaatat 2760
gaagattttg tcaaaaaaca ttatgcaaat aatcgaagta agagaaataa acttttatta 2820
gaagaaattc ctgaaaaaat gattgaacgt cagttgaatg atacacgtca tatcagtaag 2880
tatatttcag gtgtgctttc caatattgta agagtagaag atggaacaga tgaaggtgta 2940
aattctaaaa atattgttcc cggaaacgga aaaatcacaa ctcaattaaa acaggattgg 3000

ggactgaatg atgtttggaa tgatttgata ctgccacgtt ttgaaagaat gaatcaacta 3060
accaattcaa cagattttac agcttggaat gaaaatcatc aaaagtattt gccaactgtt 3120
ccgattgagt tttcaaaagg attttcaaag aaaagaatcg accatcgtca tcacgctttg 3180
gatgctttgg tgattgcttg tgctacaaaa gaccacgtaa atttattgaa taatcaatct 3240
gcaaaatcgg agacgaaacg ttacgatttg aaaaagagat tgatgaagtt tgaaaaagtt 3300
gtttacaatc atacacaaac aggagagaaa atcgaaagag atgttccgaa aaagtttttg 3360
aaaccttggg aaaactttac ggttgaagct aaaaacagtt tggagaaaat aattgtaagc 3420
tttaagcaaa atctcagagt aatcaataaa gctacaaatt attatgaaaa atatgtggag 3480
aaagatggct tgaaaacgaa agaaagggta gagcagaccg gaacgaactg ggcgataaga 3540
aaatcattgc acgaagaaac cgtttcaggg aaaattaact tgccttgggt ggaaacaagc 3600
aatggagaat ttattactgc cacaagaaag tctttggaca caagcttcaa tttggaaaaa 3660
atcaaaaaaa ttacggatat cggaattcag aaaattctca gaaattatct tgaggcaaaa 3720
gatggcaatc ccgaattggc tttttcgcca gaaggaattg aagatctgaa taaaaatgtt 3780
gaaaagtata atgatggtaa atcacatcag ccaattatca aagttcgtgt ttatgaaaaa 3840
ggcaaaggaa ggtttgtctt agggcaaaca ggaaataaaa ttgataaata cgttcagggt 3900
tcccctaact tatttttcgc aatatataaa gatgataatg gtaaaaaaag ttatgaaagt 3960
attcgtttag atattgcgat tgaaagatta aagcaaggtt tgcaagcagt tccagaaaca 4020
aatcaaaatg gatttgcatt gtatcaaagc ctgtctcctt tagatttagt ttacattccc 4080
acagaggatg aagttgaatc accacatcta attgatttta gtaaattaga taagataaaa 4140
ttggaaagga tttataatgt aaatgacttc tcaggatata caatttactt cagccaaaat 4200
tcatttgcta aaaacattta tcctaaagag atggatatga cctggaacga aaaaaaacaa 4260
aaaccatcag gttcgtttga ttctaaaaca gcttcataca ataaaaaatc gattaaagat 4320
atttttatta aaatgaaaat tgaccgttta ggaaatattt caaaagcgta a 4371

<210> 35
<211> 4389
<212> DNA
<213> Artificial sequence

<220>
<223> Cas endonuclease of Locus 17

<400> 35
atgacaaata tagaagaaaa aatatttaat acaattatgg gaatccgtaa tttgaatgct 60
ttttttattt atattttagg tgatattcaa aacataactg tgatgattaa aaataaactt 120
ggcttagact tgggaacgaa ctccatagga tgggcattga taagacagga ttttgaaaac 180
aaatacggag aaattcttgg gatgggaagc agaattattc cgatgtcaca agatattctg 240
ggcgattttg gaaaagggaa ttctgtttcg caaaccgcag aacgaacaaa atatagaagt 300
gtgagaagat tacgtgaacg atttttattg aggagagaac gactacacag acttttacat 360
cttttaaatt ttcttccaca acattatgct tcacaaattg attttgaaaa aagattcgga 420
aagtttaaag tggaaaccga accaaaactg gcttggaaaa atattgaagg aaaattttca 480
ttccttttcc aaaactcttt caatgaaatg cttgaagatt ttaatgcaaa tggacaggat 540
ttaaaaattc cttacgactg gacaatttat tttctccgaa aaaaagcact ttcacaaaaa 600
attgaaaagg aagaattggc gtggattctt ttaaacttta atcaaaaacg tggatattat 660
caattgcgtg gtgaggaaga ggaagagaat cctaataaac aggttgagtt ttattcttta 720
aaagtcatag acgttttagc agatgaacct caaaaaggaa aatctgatat ttggtattct 780
ttgattttag agaatggatg gatttacaga cgttcaagta aaattccatt atttgactgg 840
aaagataaaa caagagattt tattgtaacc acggacttga atgatgatgg aagcattaaa 900
aaagataaag acggaaatga aaaaagaagt ttccgtgcgc cgggtgagaa tgattggact 960
ttggtaaaaa agaaaaccga gcaggaaatc gagcaatctc acaaaaccgt tggaacctac 1020
atttacgaag cacttcttga taatcccaaa caaaaaataa agggaaaact gattcgtacc 1080
attgaaagaa aattttacaa agaagaactc agacagattt tagaaaagca aaaggaattt 1140
catcaggaat tacaaagtga tgatttgtat aatgattgta ttcgtgagtt gtaccgaaac 1200
aacgaatcac atcaacttat tttgagcaag aaagattttg ttcatctttt gatggaggat 1260
cttattttct accaaagacc gctgagaagc cagaaatctt ctgtttctaa ctgtaccttg 1320
gagttcagaa agtataaaga tgaaaatgga gcagaacata tacaatattt aaaagccatt 1380
ccgaaatcta atccttatta tcaggaattc cgcctttggc aatggatcta caatttaaac 1440
atttatagaa aagatgatga agcgaatgtt accaaagatt ttttaaatac gacccaagat 1500
ttagaaaatc tgtttgagtt tttaaatatc agaaaagaaa ttgaccaaaa aggattacta 1560
aaacatttta aactcaatga aaaaaaacat cgttggaatt ttgtagaaga aaagaaatat 1620
ccttgcaacg aaactaaaac gatgatttct tctcgattgg ataaagtgga taatatttct 1680
gatgatttcc tgacaaggga cattgagcag aaaatttggc acatcattta ttccgttaat 1740
gataaagtag agtacgagaa agccttaaaa tcttttgcga gaaagtatca tctggatgaa 1800

agttcttttt ttgaggcttt cagaaaattt ccgccattta aaagtgaata tggttccttt 1860
tcggaaaaag caattaagaa attgttggct ttgatgcgat taggaaaata ttggaactat 1920
gcagatgttg ataaattttc aaaagacaga attcagaaaa tcattaacgg agaatatgat 1980
gaaaatatta aagataaagt gagagaaaaa gcaataaatc tcacatcaga aaatgatttt 2040
caggggttac aattgtggtt ggctcaatat ataatatatg gaaggcattc agaagcatct 2100
atgattggaa aatggaattc tgcggatgat ttagaaatgt ttttaaaaga ttttaaacaa 2160
cattcgcttc gcaatccgat tgtagaacag gtgattacag aaactcttcg tgtggtaaaa 2220
gatatttggt taaaatatgg aaatggagca aaagattttt tcaatgaaat tcatattgag 2280
ttgggaagag aaatgaagct tcctgcgggt gatcgaaaaa aactcacgaa tcaaattacc 2340
gaaaacgaaa acaccaatct ccgcatcaaa gctctattgg cagaaatgat gaatgatcat 2400
tcagtagaaa atgttcgtcc gttttcgcct atgcaacagg aaattttgaa aatctatgaa 2460
gacggcgttt taaattcagg tattgagatt gaagatgaat atttgaaaat aagcaaaacg 2520
gcacagcctt cttcttcaga tttaaaaaga tataaacttt ggctggaaca aaaatataaa 2580
tcaccttata ccgggcaaat tattccatta aataaattat tcacaccgga atacgaaatt 2640
gaacacatta ttccgcaaag ccgatatttt gatgacagtt ttagcaataa agtaatttgc 2700
gagtcggcgg ttaataaatt gaaagataat tatatcggac ttggatttat taagcagttt 2760
ggaggaacta ttattgagct tggtttcggg aaaagtgtaa aggtatttaa tatagaagag 2820
tatgaagatt ttgtaaagaa acattatgca aacaatagag gcaaaagaaa caagcttttg 2880
atggaagata ttcctggaaa aatgattgaa cggcaattga acgatacccg ctatatcagt 2940
aaatatgttt ccggaattct ttccaatatt gttcgggtag aagacggttc agacgaaggg 3000
gttaattcta aaaacatcgt tccgggaaat ggaaaaatca ctacccaact gaaacaagat 3060
tgggggttga atgatgtttg gaatgatata attttaccac gttttgaaag aatgaatcaa 3120
cttactaatt caaaagattt taccgcctgg aatgaaaatc atcaaaagtt tttacctacc 3180
gttccaattg agttttccaa aggattttca aagaaaagaa ttgaccatcg tcatcatgct 3240
ttagatgctt tggtgattgc ctgtgccaca aaagatcacg tgaatttatt aaataatcaa 3300
tcggcaaaat cggataccaa acgatacgat ttgaaaaaga aattgatgaa gtttgaaaaa 3360
gtagtttatt atcatacgca aacaggggag aaaatagaaa gggaaatacc gaaacaattt 3420
ttaaaaccgt gggaaaactt tacagttgac gcaaaacata atctggaatc tattattgta 3480
agttttaagc aaaaccttcg tgttattaat aaagcaacta attattacga aaagtatgtt 3540
gaaaaagacg gtacaaaaaa taaggaaaga gtagagcaga ccggaacaaa ctgggcgatt 3600
agaaagccaa tgcataagga tacggtttcc ggaaaagtag atttaccttg ggtgaaagtt 3660
ccaaaaggaa aaattttaac agcaacgagg aaaagccttg atatttcatt tgatttaaaa 3720
tcaatagcat ctattactga tacagggatt cagaaaattc tcagaaatta tctgaaattc 3780
aaaggcagtc cggaacttgc attttctcca gaaggaatcg aagatctgaa taagaatatt 3840
gagaagtaca atgatggaaa atctcatcaa ccgatcaata aagtaagagt tttgaactgg 3900
ggaagtaaat ttcaggtagg acaaaccgga aataaaaaag ataaatatgt agaagctgca 3960
aaaggaacca atctgttttt tgctgtttat gaaaatgaaa atagaaacag aagttatgaa 4020
accattcctt tgaatgaggt tatagaaaga caaaagcaag gtttatctgt ggttgactta 4080
aaaggaacaa ttgatttttt tctttgcccg aatgatttag tttatattcc ttcagaagat 4140
gaacttgaaa atagtagcaa cattgacttc aaaaatttta aagaagaaca aaacaaaaaa 4200
atttataaag tagttagttt tagtggaagt cagttgtttt ttataagaca ggaaattgcc 4260
atatcaattg taaataaagc agaattttcc accttgaata aaatggagcg tagtattgat 4320
gggttaatga ttaaggaaaa ttgtatcaaa ttaaaagtag accgtttggg aaatatttct 4380
aaagcatag 4389

<210> 36
<211> 4323
<212> DNA
<213> Artificial sequence

<220>
<223> Cas endonuclease of Locus 18

<400> 36
atgaaaaata tacttggact ggacttggga acaaactcta ttgggtgggc tttagtaaaa 60
caggattttg aaaacaaaca aggtgaaatt cttggaatgg ggaccaggat aattccgatg 120
tctcaagata ttttgggcga ttttggtaaa gggaattctg tttcgcaaac tgcggaacgt 180
acaaaataca gaagtgtaag aagattacgt gaacgatttc ttctacgaag ggagaggctt 240
cacagggttt taaacatttt gaattccctt ccagaacact atgcttcaca gattgatttt 300
gaaaaacgat ttgggaagtt caaagttgaa actgaaccaa aattggcctg gaaaaatatt 360
gaaggacatt tttcatttct gttccaaaac tctttcaacg agatgcttga agattttaaa 420
acgaacggac aggatttaaa aataccgtac gactggacga tttattatct ccgtaaaaag 480
gcactttctc aaaaaattga aaaagaggag ttggcctgga ttcttttgaa ttttaaccaa 540

aaacgtggat attatcaatt gcgtggggaa gaagaggaag aaaatcctaa taaaattgtt 600
gaattttatt ctttaaaagt tgtggatgtt ctagcagatg agccacaaaa aagcaaatca 660
gatatatggt attctttggt tttagaaaat ggatggattt acagaagggc aagtaaaata 720
tcgttgtttg attggaaaga taaaacaaga gattttattg taacaactga tttgaatgat 780
gatggaagtg ttaaaacaga caaagacgga aatgaaaaaa gaagtttccg ggcaccgggt 840
gagaacgatt ggactttggt aaagaagaaa acagagcagg aaatcgagca gtctcataaa 900
accgttggaa tatatattta cgaaactctc cttaataatc ccaaacaaaa aatcaaaggg 960
aaactagttc gtacgattga aagaaaattc tataaagaag aactcaggca gatattagaa 1020
aagcaaaagc agttccatca ggaattacaa aatgatgatt tatataattg ttgtattcgc 1080
gagttgtaca gaaataatga agcccatcaa ctgactttaa gtaaaaaaga ttttgtacat 1140
ctttttatgg aggatattgt tttctaccaa agaccgttga gaagccagaa gtcttctatt 1200
tccaactgta ctttggaatt cagaaaatac aaagatgaaa atggagcaga acatacacaa 1260
tatttaaaag ccattccaaa atcgaatcca tattatcagg agtttcgtct ctggcaatgg 1320
atttttaatc ttaatctata taaaaaagat aatgatgaaa acgttatcaa agaattttta 1380
accactaccc aggatatcga aaatctgttt gagtttttaa ataacagaaa agagattgat 1440
caaaaagctc tattgaaaca ttttaagctc aatgagaaaa cacatcgttg gaattttgta 1500
gaggataaaa aatacccctg caatgaaacg aagacgatga tttcaactcg attggaaaaa 1560
gtagaaaata tttccgatga ttttctgacc agggaaatcg aacaacagat ttggcatatt 1620
atctattctg tcaatgataa aatagaatac gaaaaagcct taaaaacctt tgcatcaaaa 1680
catcatctag acgaaagttc ttttttcgag gctttcagaa aatttccacc atttaaaagc 1740
gaatatggtt cctattcgga aaaagcaatt aagaaattgt tgcctataat gcgattgggc 1800
aaatattggg attatgccaa tattgatcag tcttcaaaag acaggattaa gaagatcatt 1860
aatggagaat atgatgaaaa tataaaagat aaggtaagag aaaaggcagt ccatcttacg 1920
aatgaaaatg atttccaggg tttacaattg tggctggctc aatatattgt atatggaaga 1980
cattcggaag catcaataac agcgaaatgg aattcagctg atgatttgga agaattttta 2040
aaagatttta aacaccactc gcttcgcaat ccaattgtag agcaggtcat tactgagact 2100
ttacgtgttg tgaaagatat ttggctaaaa tacggaaaag gagcaaaaga cttcttcaat 2160
gaaattcata tagagttggg aagggaaatg aaacttccgg ctgaagatag aaagaaactt 2220
acgagccaaa ttattgaaaa tgaaaatacg aatctccgca tcaaagctct attagctgaa 2280
atgatgaatg atcatgctgt agaaaatgtt cgtccgtttt caccccagca acaagaaatt 2340
ctgaaaattt atgaggacgg tgttttaaat tcaggcattg aaattgaaga tgaatattta 2400
aaaataagca aaacggcgca accttctcct tcagatttaa aacggtataa actttggctg 2460
gaacagaaat acaaatcgcc ttataccggg caaatgattc cactaaataa attgtttacg 2520
ccagaatatg aaattgaaca tattatccca caaagtcgat actttgatga tagttttagt 2580
aataaaatta tttgtgaatc ggcagttaac aaattgaagg ataattatat cggacttgga 2640
tttattaagc agtttggagg aactattatt gaacttggtt tgggtaaaag cgtgaaggtt 2700
tttgatattg atcaatacga agattttgtt aagaagcact acgccaataa ccgaagtaaa 2760
agaaataaac ttcttttaga agatattcct gaaaaaatga ttgaacgtca gatcaatgat 2820
actcgttata tcagtaaata tatttccggt atactttcta atattgttcg cttagaagat 2880
ggttcggatg aaggagtaaa ttctaaaaac attgttcccg gaaatggaaa aattactacc 2940
caattgaaaa aggactgggg gttgaatgat gtttggaatg acttgatttt accccgtttt 3000
aaaagaatga atcagttgac caattcgacg gattttaccg cctggaacga aaattttcaa 3060
aagtttttgc cgaccgttcc aattgagtat tcgaaaggtt tttctaaaaa aaggatcgac 3120
catcgtcatc atgctctaga cgctttggta attgcttgtg ctacaaagga tcacgtaaat 3180
ttattaaata atcaatcggc aaaatcggaa accaaacgct atgatttgaa aaagaaatta 3240
atgaagtttg aaagagttgt ttatttccat acgcaaacag gggagaagat cgaaagagaa 3300
gtgccaaaac aatttttaaa accctggaaa aattttactg ttgatgctat gcataatttg 3360
gaaacggtta ttgtaagttt taaacaaaac cttagggtca ttaataaagc aacgaattat 3420
tacgaaaaat atgtagagaa agatggtgta agaaataaga caagaataaa gcaaaaggga 3480
ataaattggg cgattagaaa accaatgcat aaagatacgg tttccggtaa agtagattta 3540
ccttgggtaa aagtcccaaa aggaaaaatt ttaacagcaa caaggaaaag ccttgacgct 3600
tcattcgatt taaaatcaat aggctccatt actgatacag ggattcaaaa aattctcaaa 3660
aattatttaa tgcataaaga cggaaatacg gaattggctt tttcacccga aggaattgaa 3720
gatttgaata aaagtattga gaagtataat gatggtaaac ctcatcagcc catcaacaaa 3780
gtaagggttt ttgaactggg tagtaaattt caggtaggac aagccggaaa taaaaaagat 3840
aaatacgtag aagccgcaaa aggaaccaat ttgttttttg gagtgtacga agataaaaac 3900
ggcaagagaa gctatgaaac cattcctttg aatgaggtga ttgaaaggca aaagcaaggt 3960
ttacctgtgg ttgacttaag aagtacaaat gatttctatt tatgcccgaa tgatttggtt 4020
tatattctat ctgatgatga gcggggtaat atgacaaata tcgattttga aagcctttct 4080
gatgaacaag ttaaaaggat atataaattt gttagttgta caggaggtga gggacatttt 4140
attccttacg caaatgcgac agaaataata aaaaatgaaa atggtacaaa tagtaaaagt 4200
gagagaatgc agaatttcta tgatgggagt tgtatgtttg ataaaaacgc caagcccatt 4260
atgataaaag aaaactgtgt aaaacttaaa gtcgaccgtt tagggaacat tgcaaagata 4320

taa                                                                 4323

<210>  37
<211>  4323
<212>  DNA
<213>  Artificial sequence

<220>
<223>  Cas endonuclease of Locus 19

<400>  37
atgaaaaata tacttggact ggacttggga acaaactcta ttgggtgggc gttggtaaaa      60
caggattttg aaaacaaaca aggtgaaatt cttggaatgg gagtcaggat aattccgatg     120
tctcaagata ttttgggcga ttttggtaaa gggaattctg tttcgcaaac cgcggaacgt     180
acaaaatata gaagtgtgag acgattacgt gaacgatttc ttctacgaag ggagaggctt     240
cacagggttt taaatatttt gaatttcctt ccagaacact atgcttcaca gattgatttt     300
gaaaaacgat ttgggaagtt caaagttgaa actgaaccaa aattggcctg gaaaaatatt     360
gaaggacagt tttcatttct gttccaaaac tctttcaacg agatgcttga agattttaaa     420
gcgaacggtc aggatttaag aataccgtac gactggacga tttattatct tcgtaaaaaa     480
gcactttatc aaaaaattga aaaagaggag ctggcctgga ttcttttgaa ttttaaccaa     540
aaacgtggat attatcaatt gcgtggggaa gaagaggaag aaaatcctaa taaacttgtt     600
gaattttatt ctttaaaagt tgtagatgtt cttgcagatg agccgcaaaa aagcaaatca     660
gatatttggt attctttggt tttagaaaat ggatggattt acagaagggc aagcaaaata     720
ttgttgtttg attggaaaga taaaacaaga gattttattg taacaactga tttgaatgat     780
gatggaagtg ttaaaacaga caaagaagga aatgaaaaaa gaagtttcag ggcaccgggt     840
gagaacgatt ggactttggt aaagaagaaa accgagcaga aaatcgagca gtctcataaa     900
accgttggaa tgtatatata cgaaacgcta cttgctaatc ctaaacaaaa aatcaaagga     960
aaattagttc gtacgattga aagaaaattt tacaaagaag agcttagaca gatattagag    1020
aagcaaaagg agtttcatca ggaattacaa aatgatgact tatataatga ttgtattcgt    1080
gagttgtaca gaaacaatga agctcaccaa ctgactttaa gcaaaaaaga ttttatacat    1140
ctttttataa aggatattgt tttctaccaa agaccgttga gaagccagaa gtcttctatt    1200
ttcaactgta ctttggaatt cagaaaatac aaagatgaaa atggagcaga acatacgcaa    1260
tatttaaaag caattccaaa atcgaatcca tattatcagg aatttcgtct ttggcaatgg    1320
atttttaatc ttaacctata taaaaaagat aatgatgaaa acgttatcaa agaattttta    1380
accactaccc aagatgtcga aaatctgttt gagtttttaa acaacagaaa agaagttgat    1440
caaaaaactt tattgaaaca ttttaagctc aatgagaaaa cacatcgctg gaattttgta    1500
gaggataaaa aatacccctg caacgaaaca aagacgatga tttcaacccg attgcagaaa    1560
gtagaaaata tttccgggga ttttctgacg agggaaatcg aacaaaagat ttggcatatt    1620
atatattctg tcaatgataa aattgaatac gaaaaagcct taaaatcctt tgcgttaaaa    1680
catcaacttg acaaaagttc ttttttcgag gctttcagaa aatttccacc atttaaaagc    1740
gaatatggtt ccttttcgga aaaagcaatt aagaaattgt tgcctttaat gcgattgggt    1800
aagtattggg attatgccaa tattgataag ttttcaaaag acaggattca gaaaatcatt    1860
aatggagaat atgatgaaaa tataaaagat agggtaagag aaaaggcggt ccatcttagg    1920
agtgaagatg atttccaggg tttacaattg tggctggccc aatatattgt atatggaaga    1980
cattcggaag catcaataac agggaaatgg aattcagctg atgacttgga agaatttttg    2040
aaagatttta aacaccactt gcttcgcaat ccaattgtag agcaggtcat tacagaaact    2100
ttacgtgttg taaaagatat ttggctaaaa tatggaaaag gagcaaaaga tttcttcaat    2160
gaaattcata tagagttggg aagggaaatg aaacttccag ctgacgatag aaagaaactc    2220
acaaaccaga ttactgaaaa cgaaaatacg aatctccgca ttaaagctct attaacagaa    2280
atgatgaatg atcatgctgt agaaaatgtt cgtccgtttt cgccccagca acaagaaatt    2340
ctgaaaattt atgaggacgg cgttttaaat tcaggcattg aaattgaaga tgaatattta    2400
aaaataagca aaacggcgca accttttcct tcagatttaa aacggtataa actttggctg    2460
gaacagaaat acaaatcgcc ttatactggg caaattattc cattaaataa attgtttaca    2520
ccagaatatg aaattgaaca cattattcca caaagccgat actttgatga cagttttagt    2580
aataaaatta tttgtgaatc ggcggttaac aaattgaaag ataattatat cggacttgga    2640
tttattaagc agtttggagg aactattatt gaacttggtt tcgggaaaag cgtaaaggtt    2700
tttgatattg atgaatatga agattttgtc aagaagcact acgccaataa ccgaagtaaa    2760
agaaataagc ttctcttaga agatattcct gaaaaaatga ttgaacgtca gatcaatgat    2820
actcgttata tcagtaaata tatttccggt atactttcta atattgttcg cttagaagat    2880
ggttcggatg aaggagtaaa ttctaaaaac attgttcccg gaaatggaaa aattactacc    2940
caattgaaaa aagactgggg gttaaatgat gtttggaatg acttgatttt accccgtttt    3000
aaaagaatga atcagctgac caattcgaca gattttaccg cctggaacga aaattttcaa    3060
aagtttttgc cgaccgttcc aattgaatat tccaaaggtt tttctaaaaa aagaatcgac    3120

catcgtcatc atgctttaga cgctttggta attgcttgtg ctacaaagga ccacgtaaat 3180
ttattaaata atcaatcggc aaaatcggaa accaaacgct acgatttgaa aaagaaatta 3240
atgaagtttg aaagagttgt ttatctccat atgcaaacag gggagaaaat cgaaagagaa 3300
gtgccaaaac aatttttaaa accttgggaa aattttactg tagatgttaa gtataatttg 3360
gacatggtta ttgtaagttt taagcaaaac cttagggtca ttaataaagc aacgaattat 3420
tacgaaaaat atgtagagaa ggatggtgca agaaataaga caagagtaga gcaaaaggga 3480
ataaattggg ctattagaaa accaatgcat aaagatacgg tttccggtaa agtagattta 3540
ccttgggtaa aagttccaaa aggaaaaatt ttaacagcga cgaggaaaag ccttgatgct 3600
tcattcgatt taaaatcaat tgcctccatt actgatacag ggattcaaaa aattctcaaa 3660
aattatttaa tgcttaaaga tggaaaccct gaattggctt tttcacccga aggaattgaa 3720
gatttgaata aaagtattga gaagtacaat gatggcaaac ctcatcaacc catcaataaa 3780
gtaagggttt ttgaactggg tagtaaattt cagataggac aagccggaaa taaaaaagat 3840
aaatacgtag aagccgcaaa aggaaccaat ttgttttttg cagtgtacga agatgaaaac 3900
ggcaagagaa gctatgaaac tattcctttg aatgaggtga ttgaaaggca aaagcacggt 3960
ttacctgtgg ttgatttaac aagtacaaat gatttctatt tatgcccgaa tgatttggtt 4020
tatattctat ctgatgatga gcgggataat atgacaaata acgattttga aaacctttct 4080
gacgaacaag ttaaaaggat atacaaattt gttagttgta caggaggtga ggggcatttt 4140
attccttacg caagtgcgac agaaataata aaaaatgaaa acggtacaaa tagtaaaagc 4200
gagagaatac agaatttcta tgatggtagt tgtatgcttg ataaaaacag caagcccatt 4260
atgataaagg aaaactgtgt aaaacttaaa gtcgaccgtt taggtaacat ttcaaagata 4320
taa 4323

<210> 38
<211> 4323
<212> DNA
<213> Artificial sequence

<220>
<223> Cas endonuclease of Locus 20

<400> 38
atgaaaaata tacttggact ggacttggga acaaactcta ttgggtgggc gttggtaaaa 60
caggattttg aaaacaaaca aggtgaaatt cttggaatgg gagccaggat aattccgatg 120
tctcaagata ttttgggcga ttttggtaaa gggaattctg tttcgcaaac cgcggaacgt 180
acaaaatata gaagtgtgag acgattacgt gaacgatttc ttctacgaag ggagaggctt 240
cacagggttt taaatatttt gaatttcctt ccagaacact atgcttcaca gattgatttt 300
gaaaaacgat ttgggaagtt caaagttgaa actgaaccaa aattggcatg gaaaaatatt 360
gaaggacagt tttcatttct gttccaaaac tctttcaacg agatgcttga agattttaaa 420
gcgaacggtc aggatttaag aataccgtac gactggacga tttattatct tcgtaaaaaa 480
gcactttatc aaaaaattga aaaagaggag ctggcctgga ttcttttgaa ttttaaccaa 540
aaacgtggat attatcaatt gcgtggggaa gaagaggaag aaaatcctaa taaacttgtt 600
gaattttatt ctttaaaagt tgtagatgtt cttgcagatg agccgcaaaa aagcaaatca 660
gatatttggt attctttggt tttagaaaat ggatggattt acagaagggc aagcaaaata 720
ttgttgtttg attggaaaga taaaacaaga gattttattg taacaactga tttgaatgat 780
gatggaagtg ttaaaacaga caaagaagga aatgaaaaaa gaagtttcag ggcaccgggt 840
gagaacgatt ggactttggt aaagaagaaa accgagcagg aaatcgagca gtctcataaa 900
accgttggaa tgtatatata cgaaacgcta cttgctaatc ctaaacaaaa aatcaaagga 960
aaattagttc gtacgattga aagaaaattt tacaaagaag agcttagaca gatattagag 1020
aagcaaaagg agtttcatca ggaattacaa aatgatgact tatataatga ttgtattcgt 1080
gagttgtaca gaaacaatga agctcaccaa ctgactttaa gcaaaaaaga ttttatacat 1140
ctttttataa aggatattgt tttctaccaa agaccgttga gaagccagaa gtcttctatt 1200
tccaactgta ctttggaatt cagaaaatac aaagatgaaa atagagcaga acatacgcaa 1260
tatttaaaag caattccaaa atcgaatcca tattatcagg aatttcgtct ttggcaatgg 1320
atttttaatc ttaacctata taaaaaagat aatgatgaaa acgttatcaa agaattttta 1380
accactaccc aagatgtcga aatctgtttt gagtttttaa acaatagaaa agaagttgat 1440
caaaaaactt tattgaaaca ttttaagctc aatgagaaaa cacatcgctg gaattttgta 1500
gaggataaaa aatacccctg caacgaaaca aagacgatga tttcaacccg attgcagaaa 1560
gtagaaaata tttccgggga ttttctgacg agggaaatcg aacaaaagat ttggcatatt 1620
atatattctg tcaatgataa aattgaatac gaaaaagcct taaaatcctt tgcgttaaaa 1680
catcaacttg acaaaagttc ttttttcgag gctttcagaa aatttccacc atttaaaagc 1740
gaatatggtt ccttttcgga aaaagcaatt aagaaattgt tgcctttaat gcgattgggt 1800
aagtattggg attatgccaa tattgataag ttttcaaaag acaggattca gaaaatcatt 1860
aatggagaat atgatgaaaa tataaaagat agggtaagag aaaaggcggt ccatcttagg 1920

agtgaagatg atttccaggg tttacaattg tggctggccc aatatattgt atatggaaga 1980
cattcggaag catcaataac agggaaatgg aattcagctg atgacttgga agaatttttg 2040
aaagatttta aacaccactt gcttcgcaat ccaattgtag agcaggtcat tacagaaact 2100
ttacgtgttg taaaagatat ttggctaaaa tatggaaaag gagcaaaaga tttcttcaat 2160
gaaattcata tagagttggg aagggaaatg aaacttccag ctgacgatag aaagaaactc 2220
acaaaccaga ttactgaaaa cgaaaatacg aatctccgca ttaaagctct attaacagaa 2280
atgatgaatg atcatgctgt agaaaatgtt cgtccgtttt cgccccagca acaagaaatt 2340
ctgaaaattt atgaggacgg cgttttaaat tcaggcattg aaattgaaga tgaatattta 2400
aaaataagca aaacggcgca accttctcct tcagatttaa aacggtataa actttggctg 2460
gaacagaaat acaaatcgcc ttatactggg caaattattc cattaaataa attgtttaca 2520
ccagaatatg aaattgaaca cattattcca caaagccgat actttgatga cagttttagt 2580
aataaaatta tttgtgaatc ggcggttaac aaattgaagg ataattatat cggacttgga 2640
tttattaagc agtttggagg aactattatt gaacttggtt tcgggaaaag cgtaaaggtt 2700
tttgatattg atgaatatga agattttgtc aagaagcact acgccaataa ccgaagtaaa 2760
agaaataagc ttctcttaga agatattcct gaaaaaatga ttgaacgtca gatcaatgat 2820
actcgttata tcagtaaata tatttccggt atactttcta atattgttcg cttagaagat 2880
ggttcggatg aaggagtaaa ttctaaaaac attgttcccg gaaatggaaa aattactacc 2940
caattgaaaa aagactgggg gttaaatgat gtttggaatg acttgatttt accccgtttt 3000
aaaagaatga atcagctgac caattcgaca gattttaccg cctggaacga aaattttcaa 3060
aagtttttgc cgaccgttcc aattgaatat tccaaaggtt tttctaaaaa aagaatcgac 3120
catcgtcatc atgctttaga cgctttggta attgcttgtg ctacaaagga ccacgtaaat 3180
ttattaaata atcaatcggc aaaatcggaa accaaacgct acgatttgaa aaagaaatta 3240
atgaagtttg aaagagttgt ttatctccat gtgcaaacag ggagaaaaat cgaaagagaa 3300
gtgccaaaac aatttttaaa accttgggaa aattttactg tagatgttaa gcataatttg 3360
gacacggtta ttgtaagttt taagcaaaac cttagggtta ttaataaagc aacgaattat 3420
tacgaaaaat atgtagagaa ggatggtgca agaaataaga ctagagtaga gcaaaaggga 3480
ataaattggg ctattagaaa accaatgcat aaagatacgg tttccggtaa agtagattta 3540
ccttgggtaa aagttccaaa aggaaaaatt ttaacagcga cgaggaaaag ccttgatgct 3600
tcattcgatt taaaatcaat tgcctccatt actgatacag ggattcaaaa aattatcaaa 3660
aattatttaa tgcttaaaga tggaaaccct gaattggctt tttcacccga aggaattgaa 3720
gatttgaata aaagtattga gaagtacaat gatggcaaac ctcatcaacc catcaataaa 3780
gtaagggttt ttgaactggg tagtaaattt cagataggac aagccggaaa taaaaaagat 3840
aaatacgtag aagccgcaaa aggaaccaat ttgttttttg cagtgtacga agatgaaaac 3900
ggcaagagaa gctatgaaac tattcctttg aatgaggtga ttgaaaggca aaagcacggt 3960
ttacctgtgg ttgatttaac aagtacaaat gatttctatt tatgcccgaa tgatttggtt 4020
tatattctat ctgatgatga gcgggataat atgacaaata acgattttga aagcctttct 4080
gacgaacaag ttaaaaggat atacaaattt gttagttgta caggaggtga ggggcatttt 4140
attccttacg caagtgcgac agaaataata aaaaatgaaa acggtacaaa tagtaaaagc 4200
gagagaatgc agaatttcta tgatggtagt tgtatgcttg ataaaaacag caagcccatt 4260
atgataaagg aaaactgtgt aaaacttaaa gtcgaccgtt taggtaacat ttcaaagata 4320
taa 4323

<210> 39
<211> 3702
<212> DNA
<213> Artificial sequence

<220>
<223> Cas endonuclease of Locus 21

<400> 39
   atgggttcac gtataattcc tttatcaaca agtgataaag aagaatttca aagaggtttg 60
   tcaatcacta agaatcaaag tagaacggta agccgtactc agagaaaagg gtatgaccgg 120
   aaacaattga agaaaagtaa tttaaagaaa attctaaata aatataatat cttcctttca 180
   gaagaattat taaaacttcc aatgcttgat ttgtggaaat tgagaagtga ttctgcaaac 240
   ccaaatgaaa atatttcgac tgaacaattg ggacgaattt tatatatgtt gaatcaaaag 300
   cgtggctata aatctgcaag aagtgaagca aatgccgata aaaaagatac agattatgtt 360
   caggcagtaa aagggcgcta cgcacagctg aaagaaagaa atcaaacaat cggacaatat 420
   tttcatgaag aattgcataa tgcaaatgca aacaattctt atttcagaat aaaagaagaa 480
   gtttatccga gagaagctta tatagaagag tttgatacga ttattaatgc tcaaaaagat 540
   aaacatgatt ttcttacaga cgaagtcgtt catcaattaa gaaatgaaat catttatttt 600
   caaagaaaac taaaatctca aaaaggattg atcaatattt gtgaatttga aggctttgag 660
   aaaacagttt ttgacaagga caaaaacaaa cacaaagtag tgtctgtcgg tccgaaagtt 720
   gcgccgaaaa catctccatt gtttcagctt tgtcgtattt gggaagttgt aaatacaatt 780
   accttaaaaa taaagaatcc tgaaggaagc aaatataaat ggggtgagaa aattccaaat 840
   ttaattgaga aagagatttt agctgattat ttacttaaaa atgagagttt aagttttacg 900
   aagcttcttg aaattttaga attaaataaa aatgatattt atgtaaataa gcaaatatta 960
   aaaggtataa aaggcaatga aacttatgct gttcttcaga atattttagg agataatgaa 1020
   cttttaaatt ttgatgtttc aattattcca tccaattaca cttcaatttt ggtggacaaa 1080
   aaaacgggag agatattgga agagagagca ggtctcgaat tagatgcttc attagaaaaa 1140
   gagcctttat accagctgtg gcacaccatt tattccctaa aagatttaga tgaatgcaaa 1200
   aatgctttaa ttaaaaggtt tggattcgat gatgagattg ccgaaaagtt gtctcgtatc 1260
   aatttcaaca aacaagcttt tagcaataaa tcgaacaaat cgatgagaaa aattcttcct 1320
   tttctgatgg aaggctacga ttattctcaa tcttgcagtt tggcggggta caatcattct 1380
   aattcgttga cgaaagatga aagagaacag caggtaactt taaacagatt agaacttctt 1440
   caaaaaaaca gtttacgaca gcctattgtt gagaaaattc tgaatcagat gataaatgtg 1500
   gtgaacgcaa ttcttgagca atacggaaaa ccaactgaaa ttagagttga gctcgcacga 1560
   gaccttaagc aaagcaaaga cgagcgcaac gatgccgatt tacaaaattc aaaaaataaa 1620
   aaactcaatg aagaaattgg taaacgattg gctgaattgg gactgcctgc tacgaaacga 1680
   tatattcaaa aatataaatt cattttccca tcgcttagca aaaatctaaa agatgctcgc 1740
   gttaacaatc aatgtattta ttgtggagaa acatttaatt taacggaagc tttaagtggt 1800
   gatgcttttg atgttgacca cattgtcccg aaagctttat tgtttgatga ttcacaaacc 1860
   aataaagtat tggttcatag aaaatgtaat tcgacaaaaa ccaatcaaac tgcgtatgat 1920
   tacattgcta caaaaggtga cgaacaagtt cgtatttatt ctgaacgaat tgatgattgg 1980
   tttaaaagag gaattatttc ttacagtaaa atgcaaagat taaaggtttc acataaggaa 2040
   tatcttgaaa gaaaaaaaat aggaaaagaa accgaaactg ataagaagct ttgggaaaat 2100
   tttatagaca gacagttgag agatactcaa tatatttccc gaaaatcaag agagattcta 2160
   cagaaagtat gcaacaatgt aactacaaca gaaggcggag ttacagcaaa acttcgcaat 2220
   ttgtggggtt gggatgatgt tttgatgaat ttgcaaatgc caaaatataa agaattaggg 2280
   caaactgtca tcaaagaatg gacaagcgac cacggaaaaa gaaaacatca aaaagaagaa 2340
   atagaaaatt ggacaaaaag agatgaccat cgtcatcacg ccattgatgc tttggtaatt 2400
   gcctgcacaa gacaaggttt tattcagcgg ataaatactt tgaattctag cgatactaaa 2460
   gatttaatga tgaaagaagt tgaaaaagca aaaatcgaat ttaatgaaaa gagtaccctt 2520
   ttagaaaaat atttaaagac tcaaaagccg tttactacag aacaagtaat gaaagaagct 2580
   gataaaattt tagtttcatt taaagcaggt aaaaaagtag caacaataac taaatttaaa 2640
   gctacaggaa aaaataaaga tacaggagta attgttccaa gaggagcgtt acatgaacaa 2700
   aatgtttatg gtaagataaa agctattgaa aaagataagt ctttaaagta tttgtttgaa 2760
   aattcaggga aaattgtaaa tcctaaaatt aaaaatttag ttgaaataag actttcggaa 2820
   aatgaaaata gttcaaaaat ggctttatca acattaaaga aaaatcccat tttcctaaat 2880
   gaagaaaaaa ctgatttact agaaaaggca tcatgttatg agtatgttac tgttagaaag 2940
   tatcctttga acacaataaa agcaagtcag gctgatgata ttgttgatga aagagtgaaa 3000
   tttttagtga aagaaagatt ggcccaattt ggaaataaag aaaaagaagc ttttaaagat 3060
   acactttggt tcaacgaaga aaaacaaatt ccgattatta ccgttcgttt atttgctcgt 3120
   cctgatgcta ataatttaca ggcaatgaaa aaggatgaaa acggtaaaga aataggattt 3180
   gtagtgttgg gcaacaatca tcatatcgcc atttatgagg ataaagacaa caagcttgtg 3240
   cagcacagtt gtactttttg gcatgcagtg gaaaggaaaa aatataacat tccaccagta 3300
   attaaaaatt cctcagaaat ttggaatcaa ttattaaata aagaattccc agaaactttt 3360
   ttggataaat tacctgcgga taatctcgaa ttaaaattta gtatgcaaca aaatgaaatg 3420
   tttattctgg gattaccaca agaagaattt gaagaagcaa taaaaaataa tgataaatct 3480
   gttttgagta aacacatgta tttagtttgg agtattgctg acggagatta ttggtttaga 3540
   catcatttag aaacaaaaaa tacagagtta aagaaaatca atggtgcaaa agaaagcaaa 3600
   agatttttca gattgcaaag tcttaaggct gtagaaaatt tgacaccaat caaggtaaga 3660
   ctgaatcatt taggagaaat aactaaaata ggagaatact aa 3702

<210> 40
<211> 3807
<212> DNA
<213> Artificial sequence

<220>
<223> Cas endonuclease of Locus 22

<400> 40
atgatgatta aaaatatact tggattagac ttaggaacca actcaattgg ttgggcatta 60
atagaagttg atgaaaataa tattcctatc cgaattgttg caatggggtc tagaataatt 120
cccttgtcaa caagtgataa agaggagttt caaagaggtt tatcaattac aaagaatcaa 180

agtagaacgg taagtcgtac ccagagaaaa ggttataata ggaaacaatt aaagaaaaat 240
gatctaaaga aaattctaaa taaatacaat atttttccat cagaagaatt gttaaaactt 300
ccaatgcttg atttgtggaa attaagaagt gactctgcta atcccaatga aaatatttct 360
gcagaacaat taggacgtat tctttatatg cttaatcaga aaagaggtta taaatctgct 420
agaagtgagg ctaatgctga taaaaaagat acagactatg ttcaggaggt aaaaggccgt 480
tatgctcagt tgaaagagag agagcagact attgggcagt atttttatga agggctttct 540
accgcttttt cagacaaaga taatcggcag tattttagaa tcaaagaaga agtttatcca 600
agagaagctt acgtagaaga gtttgatact atcattaatg ctcaaaaaga taagcatgat 660
tttcttacag ataaagtagt tcatcaatta agaaatgaaa tcatttattt tcaaaggaag 720
ctgaaatctc agaaaggatt agttagtatc tgtgaatttg aaggttttga gaaaactgtt 780
tttgataagg aaaaaaacaa aaataaagtg gtgtttattg ggccgaaagt agctcctaaa 840
acgtctccat tgtttcagct ttgccggatt tgggaagtcg tcaatacgat tactttaaaa 900
ataaaaaatc cagaaggaag taaatataaa tggggtgaaa aaattccgga tttaaaggag 960
aaagaaattt tagcagatta tttatttaaa aacgaaagtg taagttttag taaacttctc 1020
gaaattttag gattaaaaaa agaaaatgta tatgtgaata agcaaatcct gaaaggtatt 1080
aaaggaaacg aaacatatgc agctctttat aatgttttag gcgataatga acttttgaat 1140
tttaatgtat ctatcattac ttcaaatcat actgcgatat tgatagataa gaaaacaggc 1200
gaaattcttg aagaaagagc tggccttgaa cttgatgcat ttttggaaaa agagccgtta 1260
tatcaactgt ggcataccat ttattccctg aaagacttag atgaatgtaa aaatgcgtta 1320
attaaaaggt ttggtttcga agaagacatt gccgaaaaat tatcttgtat cgattttaat 1380
aaacaggctt tcggtaataa atcgaataaa tcgatgagaa aaattcttcc ttttctgatg 1440
gagggctatg attattctca atcctgcagc ttggcgggct ataatcattc taattcttta 1500
actaaagatg aaagagaaca acaaattact ttagaccgat tagaacttct ttccaaaaat 1560
agtttgagac aacctattgt tgagaaaatt ctgaaccaga tgattaatgt agttaatgca 1620
attcttgagc aatatggaaa accatctgaa attagagttg aacttgcccg tcaacttaaa 1680
caaagtaagg acgagcgaaa tgaaactgat ttgcaaaact ctaaaaataa aaagctcaat 1740
gaggaaattg gaaaacgatt agcggaattg ggactgcctg taaccaagcg ttatattcaa 1800
aaatataaat tcatttttcc atcacttagt aaaaacctga aagatgctca cgtcaataat 1860
caatgtattt attgtggaga aaccttcaat ttaacagaag ctttgagtgg ggatgcttat 1920
gatgttgacc atattgtacc gaaaacttta ttatttgatg actcacaaac caacaaagtt 1980
ttggttcatc gaaaatgtaa ctcgacaaaa actaatcaaa ctgcgtatga ttacattgct 2040
acaaaaggag atgaacaggt tagggtatac tcagaacggg tggatgactg gttcaagaga 2100
ggaattatct cttacagtaa aatgcaaagg ttgaaagttt cacacaaaga atatcttgaa 2160
agaaaaaaac ttggaaaaga aacagagacg gataagaagc tttgggaaaa cttcatcgac 2220
aggcagttga gagatactca atatatttct cgcaaatcaa gggaaatttt gcagaaagtg 2280
tgcaataatg taacaacaac agaaggtgga gtgactgcaa aactgcgtaa tgtatggggt 2340
tgggatgatg ttttaatgaa tcttcaactg cctaaatata aagaattagg gcaaactttc 2400
accaaagaat ggacaagcaa acatggaaaa agaaaacacc aaaaagaaga aatcgaaaac 2460
tggacaaaaa gagatgatca tcgccatcac gccattgatg ctttgataat agcttgtaca 2520
aaacaaggct ttattcaacg aataaatact ttgaattcga gtgagacaaa agatttgatg 2580
gtaaaagaag ttgagggagc taaaatagaa tttaatgaaa aggctacgct tttagaaaaa 2640
tacttaaaaa ctcagaaacc ttttgataca catgaagtaa tgaaagaagc tgataagatt 2700
ttaatttcat ttaaagcagg aaaaaaggta gcaacaataa ctaaatttaa agcaacagga 2760
aaaaataaag agacgggggt aattgttccg agaggagctt tacacgaaca atctgtttat 2820
ggtaaaataa aagtaattga gaatgataaa cctttaaaat acttgtttga aaactcagat 2880
aaaattgtaa atccaaaaat taagaatctt gttgaaacaa ggctcacaga aaatgaaaat 2940
aattttaaaa aggctttatc aacattaaag aaaaatccaa tctttttcaa tgaagaaaaa 3000
accgagatat tagaaaaagc atcttgctac aatgatgcca ctgttttgaa atataagtta 3060
caaaatataa aagcaagtca ggccgatgat attgttgatg aaagagtgaa atctttagta 3120
aaagaaagac ttgctcaatt tggaaataaa gaaaaagaag ctttcaagga tattctttgg 3180
cttaatgaag aaaaacaaat tccgattata acggttcgtt tgtttgcccg tccagatgca 3240
gataatttac aggctattaa aaaagataaa tatgggaatg agataggatt cgttgtgacg 3300
ggaaataatc atcatatcgc aatttatgaa gataaagaca ataagcttgt tcagcatact 3360
tgtacatttt ggcacgcaat agaaagaaaa aaatacaata ttcctgctgt tattggaaat 3420
tcatcagata tttggaacca attattagac aaagaacttc caatatcatt tttgaataaa 3480
ttaccgatgg ataagctcaa tttaaaattt agtgtgcagc aaaatgaaat gtttattctg 3540
gggttatcac aagacgaatt tgatgagaca attaaaaata atgataaatc tttattgagt 3600
aaatatttgt atttggtttg gagtattgca gaaggtgatt attggtttag acaccatctg 3660
gaaacaaaga acactgagtt aaagaaaact gatggagcaa aggaaagtaa aagatattat 3720
cgattaagtt caaaaggttt tattgactta aatccaataa aagtcagact gaatcattta 3780
ggagaaatta ccaaaatagg agaataa 3807

<210> 41

<211>  3795
<212>  DNA
<213>  Artificial sequence

<220>
<223>  Cas endonuclease of Locus 23

<400>  41
atgaaaaaaa tattaggatt ggacttgggg acaaatagta ttggatgggc tttgattgaa      60
attgataaaa ataatattcc tattagcatt attgcaatgg gttctcgaat aataccttta      120
tcatctagtg ataaagaaga gtttcaaaga ggtttgtcaa taacaaagaa tcagagtaga      180
acggttagcc gcactcaaag aaaaggatat gaccggaaac aattaaaaaa aagtgatcta      240
aagaaaattc taaataaata caacattttt ccttcagaag aattattaaa acttccaatg      300
cttgatttgt ggaaattaag gagtgattct gctaatccaa atgaagatat ttctgccgaa      360
caactaggcc gtatattgta tatgctaaat cagaaaagag gctataaatc tgctagaagt      420
gaagcgaatg ctgataaaaa agataccgat tatgtacaag ctgtaaaagg acgttatgct      480
catttgaagg ataggactca gactattggc caatacttct ttgaagaatt agacaatgca      540
aatactaata attcttattt tagaataaaa gaagaagttt atcccaggga agcttataca      600
gaagaatttg acgccataat taatgctcaa aaaggtaaac acgactttct taccgatgaa      660
gttgttcatc aattaaggaa tgaaatcatt tattttcaaa gaaaactgaa atctcaaaaa      720
ggattagtga gtatttgtga atttgaaggt tttgagaaaa ctattttcga taaggaaaaa      780
gacaaaaata aagtggtact tgttggtcca aaagttgctc caaaaacctc tcctctattt      840
caactttgcc gtatttggga ggttgtcaat acaattacat taaaaataaa aaatccagaa      900
ggaagcaaat acaaatgggg tgaaaaaatt ccaaatttaa aagagaaaga aattttagca      960
gattatttgt ttaaaaatga aagcttaagt tttagtaagc ttcttgaaat tttagaatta      1020
aaaaaagaaa atgtttatgt gaataagcaa atccttaaag gtcttaaagg aaacgaaact      1080
tattctacta tccatagtat tttaggagat aatgaacttt taaattttga tgtgtctatc      1140
attacttcaa atcatacatc aatgttggta gacaaaaaga cgggagaaat tttagaagaa      1200
agagcgggtc ttgaacttga tgcatcatta gagagagaac ccctatatca attgtggcat      1260
acaatctatt cattgaaaga tttagatgaa tgtaaaaatg ccttaatcaa aagatttggt      1320
tttgatgatg aaattgctaa aaaattatct cgtattgatt ttaataaaca ggctttcgga      1380
aataaatcga ataaatcgat gaggaaaatt ctaccttttc tgatggaagg ctacgattat      1440
tctcagtctt gcagtttggc tggttataat cattctaatt ctttaacgaa ggatgaacga      1500
gaacaactaa tcactttaga ccagttagca ctccttccta agaacagtct cagacaacca      1560
attgttgaga aaattctgaa tcagatgatt aatgttgtta acgcaattct tgaacaatat      1620
ggcaaaccct ctgaaattag agttgaactc gcacgtgaac ttaaacaaag taaggacgag      1680
cgaaacgata cagatttaca aaattcaaaa aataaaaaac tcaatgagga aatcagaaaa      1740
cgattggttg aactgggatt acctgtaact aaacgatata ttcaaaaata taaatttatt      1800
tttccttctc ttagtaaaaa tctgaaagat gctcacgtta acaatcaatg tatttattgt      1860
ggagaaacat ttaacttaac ggaagctttg agtggtgatg cctacgatgt tgaccatatt      1920
gtgccgaaag ctctattatt tgatgattct cagaccaata aagttttagt tcacagaaaa      1980
tgtaattcaa caaaagacaa attaacagca tacgattata tttctacaaa aggaatagag      2040
caagttagag tatattctga acgtgtggat gattggttca aaagaggtat tatttcttat      2100
agtaaaatgc aaaggttgaa agtttcacac gaagaatatc tggaaagaaa aaagatagga      2160
aaagaaacag agactgataa aaaactttgg gaaaacttca tcgagagaca attgagagat      2220
actcagtata tatctcgtaa agcaagagaa attttacata aattatgcaa caatgtgact      2280
acaacagaag gcggagttac agcaaaactt cgtaatttat ggggttggga tgatgtttta      2340
atgaatctac aaatgcctaa atacaaagaa ttaggacaaa ccgtcatcaa agaatgggca      2400
agcgatcatg gaaaacgaaa acaccaaaaa gaagaagtcg aaaactggac aaaaagggat      2460
gaccaccgcc atcacgccat tgatgctttg gtaattgctt gtacaaaaca aggttatatt      2520
cagcggataa atactttgaa ttcaagtgat accagagatt tgatgataaa agaagtggaa      2580
gaagcgaaaa tagaatttaa tgaaaagtca acacttttag aaaaatattt aaaaactcaa      2640
gagccgttta ctactcaaga agtgatgaaa gaggctgata agattttaat ttcttttaaa      2700
gctggcaaaa aagtagcaac aataactaaa tataaagcta caggcaaaaa caaaaatact      2760
ggtgttcttg tcccaagagg agctttgcac gaacaaaacg tttatggcaa aataaaagtg      2820
attgaaaagg ataaatcttt aaaataccta ttcgaaaatt cagataaaat agtaaactct      2880
agtattaaaa aacttataga agtaaggctt ttggataatg aaaataattc taaattggct      2940
ttatcctctt taaagaaaaa cccgattttt ctaaatgaag aaaaaaaaga agtattagaa      3000
aaagcgtctt gttatgagta tgtcactgtt aaaaaatatc ccttgaacac aataaaagcc      3060
aaccaagctg atgatattgt tgatgaaaga gtgaagtttt tagtaaaaga aagattatct      3120
caatttggaa ataaagaaaa agaagctttt aaagatacgc tttggtttaa tgaagaaaaa      3180
caaattccta ttattactgt tcgtttattt gctcgtccag atgcaaataa tttgcaggca      3240
ataaaaaaag ataagaacgg taaagaaata ggatttgtag tgacgggaaa caatcatcat      3300

attgcaattt atgaagacaa agacaataaa cttattcagc acagttgtac attttggcat 3360
gcagtagaaa gaaaaaaata caatgttcca accgttatta aaaattcatc tgatgtttgg 3420
aatcaattat tacacaaaga attaccagaa tcatttttaa ataaattacc tgctgattat 3480
ctcaatttaa aattcagtat gcaacaaaat gaaatgttta ttctgggctt atcacaagaa 3540
gaatttgatc aagcaattaa aaagaataat aagcccttat tgagtaaaca tttgtattta 3600
gtctggagta tcacagatgg agattacttt tttagacatc atttagaaac taaaaataca 3660
gaattgaaga aaacagacgg agcaaaggag ggtaaaagat attttcgatt aagtacaaaa 3720
gggcttacag atttaaatcc gataaaagtt agattaaatc atttaggaga aataactaaa 3780
ataggagaat attag 3795

<210> 42
<211> 4395
<212> DNA
<213> Artificial sequence

<220>
<223> Cas endonuclease of Locus 24

<400> 42
atggcgaaaa ttttaggatt ggatttaggg acaaactcga ttgggtgggc gattgttgat 60
aaagatggaa atgatttttc ccttgtagat aagggagtga gaatattttc cgagggagta 120
aaatctgaaa aaggaattga aagttctcgt gctgcggaac gaactggtta tagaagcgca 180
agaaaaatta aataccgcag aaaattaaga aagtacgaaa ccttaaaagt tctttcctca 240
aatggaatgt gtcctttgtc ggttgaagaa gttgaagact ggaaaaaatc cgggtttaaa 300
aaatatcctc ttaatcctga atttttgaaa tggttacgaa cggatgaata ccaacatata 360
aacccatact attttagaga tagagcaagc aagcaaaaag ttgccttatt tgaattggga 420
cgtgctttat atcatattgc tcaaagaaga ggttttttga gtaatcgtct agatcagtca 480
gctgaaggag tttttgaaga acataatcct caaatccaaa gtttgattga agatttggat 540
gattcgaaat taattcttga tgaattgaaa aattattatg ttgataaagg aataattgaa 600
gaagctgaaa aaggagtttt taaaaaagat ttagatgaag gtgagaaaaa actaaaatca 660
ctttataatt cgttagtagg aataattaag aagaatgaaa acaacgttga aaaatgcaaa 720
gaggaattta ttgcaaggct taataaaaaa gaagatttag gaaaagtaaa aggaaaaatc 780
aaagatattt cgcaagcaat gcttgatggt aattttaaaa ctttaggaca atattttttc 840
agtctttaca ataaaggaaa aataagaaat caatatactt cgagagaaga acattatttg 900
gaagagttta ttactatttg taaaattcag ggaatcgaag gaattgatga taaagaaaaa 960
ttacctgaaa agaagtttac aggcttagcc aaagatttgt acaaagccat tttctttcag 1020
cgtcccttaa aatctcaaaa aggactgatt ggaaagtgtt cctttgagaa aagcaaatca 1080
cgttgtgccg tttcgcatcc cgattttgag gagtacagaa tgtggactta cttaaacact 1140
atcaaaattg gaacacaaag cgaaaaaaca ttacgttttt taacgcaaga tgaaaaatta 1200
aaattagttc caaaattcta cagaaaaaat gattttaatt ttgaagtttt agccaaagaa 1260
cttgttgaaa aaggagcaag tttcgggttt tataaatctt ccaaaaagaa cgattttttt 1320
tattggttta attacaaacc aactgattcg gttgcagctt gtcaagtttc agcatcttta 1380
aaaaatgtga ttggtgaaga ctggaaaaca aaaatattta cttataaaac tttaaatgca 1440
aaagaagaag aagtaacaaa atctgttgat tataaagact tatggcactt actttccgta 1500
gcaacatcgg atatttattt ggaggaatat gctaaagata aattagggtt tgacgacaaa 1560
aatgccaaag ctttcagtaa aataaaactc aaaaaagatt tcgctagttt gagttcatca 1620
gcgatcacga aaattttgcc ttatgtaaaa gaaggacttt tgtattctca cgctgttttt 1680
atggcaaata ttgaaaatat tgttgatgaa aatatttgga ataatcttga ccaaagaaat 1740
tatattcagt ctaaaatagc cgaaattatt gagggctata cttttgaaaa aacaggtttg 1800
gaagtagtca acagccttat taaagaatat aaaacagaaa atgattacag caaaaaagta 1860
tattattcaa aagaagctga accagaattt gaaaaagatt taaagcaaaa attagtagtt 1920
ttttataaat caaatgacat tgaagatgaa aatataaaac aagctatttt taatgattta 1980
tattcagttt taattgacca acttaaaaaa tatgagtata tcaaaataca acgattggat 2040
gagaaagttt tagagttttt aaaaggtgaa aatgaagacg gagaagtttt ttgcaataat 2100
ccaaagcaat taaaaaagct gtatcatcca tcagatattg aaatttttaa aaagcaaaag 2160
gtcaaagata aagatggtaa agaaacggga cagcttattt taggtagccc tttaacttct 2220
tcaatcaaaa atccgatggc aatgcgtgct ttgcatcaat tgcgtaaagt tttaaatact 2280
ttgattttgg aaggtcaggt agatgaaagt acaaggattc atattgaaat ggctcgtgag 2340
ttaaacgatg ccaaccgaag aaaaggaatt caggattatc aaaaagagaa taaagatttc 2400
agagacaatg ctattaaaga aatcaaaaaa ctgtatttgg acgaatgtgg taaagaagta 2460
gaaccaacgg aagatgatat tttaagatat cagctttgga ttgaacagga caaatgtgag 2520
atttacgaac aaggcaagaa tattagtatt tgtgatataa taggaagtaa tccagcttat 2580
gatatagaac atacaattcc aagaagtatt tcgcaagata attctcaaat gaataaaacg 2640

ctatgcagtc agcgatttaa cagggatgtc aaaaagcaaa aaatgccaat tgaattgagc 2700
aatcatttgg aaattttgcc aagaattgcg cattggaaag aagaagccga caacttatca 2760
agggaaattg agataattag ccgttccata aaagccgcat cgaccaaaga aattaaagat 2820
aaaaaaatac gaagaagaca ttatctgact ttaaaacgtg attatcttca agggaaatac 2880
gatcgattta tttggaaaga gccaaaggtt gggttcaaaa acagtcaaat tccggataca 2940
ggaattatca ccaaatatgc gcaagcttat ttaaaatcgt attttaaaag agtagaaagt 3000
gtaaaaggcg gaatggttgc cgagttcaga aaacaatggg gaattcagga gagttttatt 3060
gacgaaaacg gatttaaaca atacaaagaa aaagatagaa gtaaacacac ccatcatacc 3120
attgatgcca ttactattgc ttgtatgacc aaagacaaat atgatgtttt ggctaaagcc 3180
tggacttctg aagatgaaca agataaacta aaggcaaaag ctctaatggc agatgccaag 3240
ccgtggaaaa cctttaaaga agatttactc aaaatagaag atgaaatttt agtttcacat 3300
tacacgcccg ataatgtaaa aaaacaatct aaaaagatta ttagggttcg tggcaaaaaa 3360
caatttattg cagaaattga gagagatgtc aaaggtaaag caatacaaaa aaaagatgct 3420
aatggaaaag tagtttacaa gttaaatgat aaaggagaaa agatcccaag attgcagcaa 3480
ggtgatacta ttcgaggctc gttacaccaa gatagtattt atggagcaat taaaaatcca 3540
ttaaatactg atgaaatacg ttatgtgatt cgtaaagatt tagaaagtct gaaagcaagt 3600
gatatcgaaa acattgtgga tgaaactgta aaagagaaag taaaacaagc cgttacaaat 3660
aaagttttgt tgctttcttc caatgcccaa caaaaaaata aaattgccga gaatgaaaag 3720
gtttggatga ataaagaaaa acaaatttcc attaacaaag ttagaattta tgccaattca 3780
gttaaaaacc cactggaaat aaaagagcat agtttattgt caaaatctcg tcacgaacac 3840
aagcaaagag tatatggtca gaatgatgag aattatgcaa tggcacttta tgagttagat 3900
gggaaaagag aatttgaatt gattaataat tttaatttgg ctaaactcat aaagaatgga 3960
caaggatttt atcctttaaa taaagaaaaa gaattaaaag gtaaaaaagt tttatatccg 4020
attgcaaaaa gtaacagcag agatgttgtt ttgaaacgag gacaacaagt tgtttttttt 4080
gataaagaag ttgaaaagcc aaaggatatt tctgaaatct tggattttaa aagaagggtt 4140
tatataattg aaggtttgtc cattcaaaga ataaaatcgg ggaataatga atttgaatat 4200
ggtgtaatta tgttaagata tttcaaagaa gcaaggaaat cagatgaaat caaaaaagat 4260
aattttaaac cagatggtgt ttttaaatta ggagaaaata aaccaacaag aaaaatgaat 4320
cacagccaat ttaccgcttt tgttgaaggt attgatttta aagttttacc atcaggaaag 4380
tttgtcaaaa tttga 4395

<210> 43
<211> 4377
<212> DNA
<213> Artificial sequence

<220>
<223> Cas endonuclease of Locus 25

<400> 43
atggcaaaga ttttaggatt agatttaggg acgaactcga ttggttgggc gattgtagat 60
aaagacgaaa ataatttttc attagtagat aaaggagtca gaatattttc tgaaggagtt 120
aaatctgaaa aaggaattga aagttctcgt gctgctgaac gaacaggatt tagaagtgca 180
aggaaaatta aatatcgaag aaagctaagg aaatacgaaa ctttaaaagt tctgtcatca 240
aatggaatgt gtccactttc aattgaagaa gtggaagact ggaaaaaatc aggttttaaa 300
caatatccac ttaatcccga attcctgaaa tggttaagaa ctgatgaggc tgaaaacatc 360
aatccttatt tttttagaga taaagccagc aaacaaaaag ttcctttgtt tgaattagga 420
cgagcattct atcatattgc tcagagaaga ggttttttga gtaatcgttt agatcaatct 480
gcagaaggaa ttttagagga acattgccct aaaattgaaa caattgtaga agatttgaat 540
tcttatgaag aagttttaga aaatttaaaa gattactttt tttttgaaac tggaattctt 600
gatgctaatg aaaaaagtgg ttatgcaaaa gatttagatg aaggtgataa aaaattggtt 660
tctttataca aatcacttca taccattgta aaaaagaatg atactaattt tgaaaaaggt 720
aaagcagaaa ttattgaacg actcaacaaa aaagatgttt taggaaaagt aaaaggaaaa 780
attaaagata tttctcaagc tatggtcgac ggaagtttta agactttagg gcaatacttt 840
ttcagtttat atagcaaaga aaaaattaga aatcaatata cttcaagaga agaacattat 900
ctcgaagaat tcataactat ttgtaaagtc caaggaatcg attccattaa cgagaaagaa 960
caattgccag agaagaaatc cgatggttta gccaaagatt tatacaaagc catattcttt 1020
caaagaccat taaaatctca aaaaggattg attggtaaat gttcttttga aaagagtaaa 1080
tcccgctgtt ccgtttcgca tcctgatttt gaagaataca gaatgtggaa ttacattaat 1140
acaattaaaa ttggaacaca aagcgaaaaa acattgcgat ttcttacgca agaagaaaag 1200
ttaaaattag ttccaaaatt ctacagaaaa aacgatttta attttgaagt tttagccaaa 1260
gaattaattg aaaaagggac gagctttgga ttttataaat cttctaaaaa gaacgattat 1320
ttttattggt tcaattttaa accaaccgat tcggtttctg cttgtcaggt ttctgcatca 1380

ttaaaaaata ttattggaga agattggaaa acaaaaacat tcacttacca aactttaaat 1440
gttaaaaaag aagaagttac aaaaacagtt gattataaag atttatggca cttactttca 1500
gtagcgactt ctgatattta tttgaataaa tatgcaaaag ataaattagg ttttgaggat 1560
aaaaacgcaa aagcattcag taaaatcaag ttaaaaaaag attttggaaa tttaagtttg 1620
tcggcaatta ctaaaattct gccttacata aaagaaggat tgctctattc tcacgctgtt 1680
tttatggcaa atatagaaaa cattgttgat gaggaaattt ggaaagatga acatcaaaga 1740
aaatatattc aaacccaaat agcagaaatt gttgaaaatt atacttttga gaaaagtcaa 1800
ttagaggtaa ttaatggact tataaaagat agtaaactaa aagaagaata ttattctaaa 1860
gaagcagaat caatttataa aaaagattta gctactaaat tagttttgtt ttttaaatac 1920
aatgttattg aaaatgaatc agaccaaaaa atcatttttg aaaacttatt tccaattttt 1980
attgagcaat taaaaaaata tgagttcatc aaaatccaga gattagatga aaaagtttta 2040
gaatttctta aaggagaaaa tcaagatggt gaaatatttt gcagcaatcc aaaacaatta 2100
aaaaaactct atcatccatc ggatattgaa gtttttaaaa agaaaatcat taaagacgaa 2160
tttgggaatg aaaaagtagt tttaggtact cctttaacat ctgctattaa aaacccaatg 2220
gcaatgaggg ctttgcatca attacgtaaa gttttaaata ctttgatttt agaaggtcta 2280
gtagatgaaa atacactaat tcatattgaa atggctcgcg aattaaacga tgctaacaaa 2340
cgaaaaggaa ttcaggatta tcaaaacgaa aacaaaaagt tcagagaaga tgcaataaaa 2400
gaaatcaaaa aactttattt ggatgaatgc aaaaaagaag tagaacccac agaagatgat 2460
attttaagat accaattgtg gattgaacaa aacagacaag agatttacga acaaggaaaa 2520
aatattagta tttgtgatat aattggaagt aaccctgctt atgatattga acatacaatt 2580
ccaagaagcc gctctcaaga caattctcag atgaataaaa ccttgtgtag tcaacgattt 2640
aatagagaaa ttaaaggaaa taaaatgcca attgaattga ataatcattt ggaaatcttg 2700
ccaagaattg cgcattggaa agaagaagcc agtaatttat caagagaaat tgaaattata 2760
agtcgttcca taaaagccgc tgcaaccaaa gaagtaaaag ataaaaaaat aagaaggaga 2820
cattatctaa cattaaagcg agattatctt caaggaaaat atgaccgatt tattttggaa 2880
gaaccaagag ttggatttaa aaacagtcaa attcctgata cgggaattat tactaaatat 2940
tctcaggctt acttaaaatc gtattttaaa agagtcgaaa gcgtaaaagg cggcatggtt 3000
gccgaattta gaaagcagtg gggaattcaa gaaagtttta ttgacgaaaa tggatttaag 3060
cattataaaa taaaagacag aagcaagcat acacatcaca caattgatgc cataacaatt 3120
gcttgtatga ccaaagataa atatgacgtt ttggctcacg cttgggcatt agaagatgaa 3180
caaaataaaa aagaagccaa agctattata gaaaaagcta aaccttggaa aacctttaaa 3240
gaagatttgt taaaaatcga agaagaaatt ttagtctcac attatacacc cgacaatgta 3300
aaaaaacaat ctaagaaagt tattagaatt cggggtaaaa aacaacacgt tgccgaaatt 3360
gaaagagatg aaaaaggaaa ggcaattcct aaaaaagatg ctaacggtaa aattctttac 3420
aagttagata aagaaggaaa aaaacttcca agatatcaac aaggcgatgc cattagaggt 3480
tctttgcatc aggatagtat ttatggagca attaaaaacc ctttaaatac ggaagaaatt 3540
cgttatgtta ttcgtaaaga tttagaaagt ttaaaagcat ctgatattga aaatattgtt 3600
gatgaaactg taaaagaaaa aattaaagaa gcagttgcaa ataaagtctt gttattttct 3660
tcaaatgccc aacaaaagaa taaaattaga gagaatgaaa aggtttggat gaacgaagaa 3720
aagcaagttc caataaacaa agttagaatt tatgctaatt ctgtaaaaaa tcctttggag 3780
gtaaaagaac atagtttatt gtcgaaatca cgtcatgagc ataaacaaaa ggtatatggg 3840
caaaatgatg agaattattg tatggcaatt tatgaattag aaggcaaaag ggaatttgaa 3900
ttaattaata attttaattt agcaaagttg attaaggagg gacaaggatt ttatcctttt 3960
tataaagaaa aggaggtaaa aggaaaagta gttcaagttc ctattgctaa aaggaatggt 4020
agagatttaa ttttaaaaag agggcaacaa gtaatttttt atgataaaga tgttgaaaat 4080
cccgatgatg tttttgattt agtggattta aaaggaagaa tttatattat tgaaggattg 4140
tcgattcaac gccaagttgt ttctggaaag ctttatgagt ttggtagtat tatgtttcgt 4200
tattttaagg aagccagaaa agcagatgag attaaaaaag atagttttaa acccgatggt 4260
acttttaaat caggagaaat aaaaccgact cgtaaaatga atcataatca atttaccgct 4320
tttgtagaag gaattgattt taaagttttg ccatcaggta agtttattaa aatttaa 4377

<210> 44
<211> 3384
<212> DNA
<213> Artificial sequence

<220>
<223> Cas endonuclease of Locus 26

<400> 44
atggcaaaga ttttaggatt agatttagga actaactcga taggttgggc attaattgac 60
gataaattaa ataaaatttt agggattgga agtagaattt ttccaatggg ggttgaaaat 120
cttggtgatg gcgataacga aatgtctaag aatgcaagtc gaacaggagc aagaggagtt 180

cgacgtcagt tttttagacg aagactaaga aagaaagtgt tactaaaatc actttctgaa 240
aataatatgt gtccaataaa caccaaagat tttgaagatt ggaaacaaac taaacagttc 300
cctaatgaaa aattagcaac ttggtttgct atgaatcctt atgaattgcg tcaaaaagca 360
ttgacggaaa aattatcttt agaagaaata ggtagaattt tttatcatct aattcaaaga 420
agaggatttt taagtaatag tagaaaagga ggaagcgatg atggagctat ttttaaagga 480
aatgctaaag aaggaaaaat aggaattgat tatactttag aaagtatcga aggaaaaaca 540
ttgggttcat atttatatga gatttatcca aaagaaaatc agccttttca atatggacta 600
gaaagaatca gaaacagata tacaactcgt agaatgtata ttgacgaatt tgaaatgatt 660
tggaataaac agtcagaatt tcattcggtt ttgaattatg atttaaaaac aattttaggg 720
ggtagaaaac aagataatta cacagaagac ggtattttat tttatcaaag acctttgcgt 780
tctcaaaaac atttagttgg aaactgttct tttgagcctt taaaaactaa atgccctata 840
agtgctattc ctgtagaaat gtttagaatt tggcaatggg ttaatacagt tgaatataat 900
gggaagaaaa tatctgaaga agaaaaaacc aaactagttg agcaattgct ttcattcgat 960
aaaattgaat ttaaaaaact ccgaaaagct attgggaaag aaagtgcgga gtttaagttt 1020
aattacaaag acgatgataa aattgctggt acacatacta tctctaattt atccaataga 1080
aaattctttg gcaaaagatg gtttgatttt tcagataaag agcaagaaga tatttggcat 1140
gttctgtatt tttttgatag caaatcaaac ctaaaagaat atgctgttaa gaattggaat 1200
tttagtgaag aacaagcaat tgcaatttca aggtttaatg taaaagacgg ctatgctagt 1260
ttgagtcgaa aagctattgg aaatattttg ccttttttgc agcaagggta tacttatgat 1320
attgcagttg ttatgggcgg aattaaaaat gcttttggtg acaaatggca aaatgacaca 1380
gtagaaaata agctaaaaca actagatttg attgataata ttgaaggtat agttaggtct 1440
aaaatttcag gaggatttat agatacaatt aaggatttat tacgtaaaga atttgaattt 1500
aatgacaaac aattaaaaaa actttaccat cattctgctg caattgatac ggtagcatta 1560
ttacgaaaac tgccaactgg gaaacaggca gataaggata ttcaagctat tagaaatcca 1620
attgtaatta cagctttatt cgaattacga aaattggtca atgaattaat cgaagagcat 1680
gggcagcttg acgaaataaa agtagaaatg gcccgtgacc tgaaaatatc aaaatcgcag 1740
cgaaataaga tacgcaaaga tcaaaaacga ttggagagag aaaatgacag agtaaaaaag 1800
gaagtagaaa agtatgtaag agtttctcat gataacattc taagatttaa gctttgggag 1860
gaatgcaagc agacttgtcc ttatacaggt gttgtaattc cagtcacaaa actttttaca 1920
ggggaaatac aaatagagca tatccatcct tggagtcgtt cgttaaacga tagtttcaat 1980
aataaaacat tgtgctatgc ggatgaaaat agacgaaaag gagacaaaac accttttgaa 2040
ttttatggaa gcgatgagct ggattggatt gctagaaaag aacgtgcttt aaaattgttt 2100
tctgatacta aagagtatcc caatgcctat caaaaattca agcgatttgt acagcaaaaa 2160
tttgacgatg atttttcttc aagacaatta aatgatacac gatttataag taaagaagca 2220
tcgtcttatt taaaacaaat atgtgaaaag gtaaatgttt ctccaggtca aatgacagct 2280
aacttacgtc aaaaatgggg tttgaatacg gttttgaatg atgaaaatgc taaaactcgg 2340
gaagaccatc gtcatcatgc tattgatgct ttggttatgg catgtgggaa gacaagttat 2400
ttacaggaat tgtcaaaatg gaatcgttac aatagaaatt atgatttaaa agattttcct 2460
atgccttggg aaactttccg atttgatgct gagaaagcaa ttgataaaat attgatttcg 2520
cataaacgta tttctaatga tattacaact cgatatacta aagtggaaaa aaacggaaag 2580
acatatataa ataagggagt tgcagcacga gggcaattgc ataaggaaac tgtttttggt 2640
aaacgaaatt ttaatggaga tgaagcattt catgttagga aatcaattga tagtttaaca 2700
actgagaaac aattagaaaa agtggttgat gaagttatta aaaaattaat tcataagaag 2760
attaaggaat tgggaggatt cgtaaaaggt actatccctg ctaatacttt tttcaatgtt 2820
gatgaaaatg gagttaaaca accacaaatt ttcctgccaa ataaaaatgg tagtccagtt 2880
ccaattttaa aagtccgaat gaaagaaaat attagtggag ctgaaaaatt aaaagacaat 2940
gtcaatcaat gggtaaaccc tagaaataat catcacgtac ttatttataa ggatgaaaaa 3000
ggaattttaa gggaagaagt ggtgactttc tggactgttg ttgaaagaaa aagtaaagga 3060
tttccagtgt atcaattacc agttgaagga aaagagatag tgacagcttt gcatattaat 3120
gatatgtttt tgttaggatt aaatgaagaa gaaatcaatt gggagaatcc agattatgag 3180
attctaagag atagtttata tagagttcaa agtttgtctt caaaatatta tgagtttagg 3240
cagtctatta atgcagaatc tcaaaataaa atttcaccta tttatgaaca aattagagga 3300
tttggttcgg gtaaaaccgg ttgggattct tttaatccaa taaaagtaaa aatttcagtt 3360
tcaggaaaaa tttcaaaaat ataa 3384

<210> 45
<211> 3327
<212> DNA
<213> Artificial sequence

<220>
<223> Cas endonuclease of Locus 27

<400>  45
atggcataca aattaggaat agatctgggc accgcgtcca ttggtgctgt cgccatccgc      60
atcgaaaaca acgaaccaca aagcattgac tggcatgcag tacgcattta cagcgaacca     120
gttgtaaaca ctccgaaagg gctgcaaccc aaaaaggcgg cccgccgtga cgcacggcaa     180
ttgcgcaaac agattgaaag gcgagcacga cggttgcgcc gcattgccaa tctgattcca     240
ttgctgggat tgcataaagc cgaagtgaaa ccgggcgccc gtcaggattt gaccacattg     300
cgcgccatcg cagcgcgcca gcaggtcgag ctaaacgagt ttgccctagt actgctgcgt     360
ctggccaaac gccgtggtta cgccggtgga tttcgcgatt ccaaagacgg tgtagtcaag     420
agctccagcg acaaactgag cgaggaaatg agacgtcttg ccgagacgca aaacctgcag     480
gaaataactc tgggtgaata cctgctgtac agacaggaac acggactgcc tgtctggctc     540
aaagccgaac gtccaggtct ggaaccgcgc tatgccctgc gcgaaatggt aaccgccgag     600
ttcgaacagc tctggacaac ccaggcccga taccacccga tcctggcctc taatcatctc     660
ggcaagccac ttaagcaatg gttcaatgaa gcgctgttct accagcggcc actgaagtct     720
cccgcccaca tggtcggcaa ttgcctgctt gagcccaatc tgccacgtgc cccacgcgct     780
cacatggcag cccaacactt cagaatcgat aaaacgctcc atgacatgcg ctggggtagt     840
gggcgacggg ctgaacgcct gagcaaggca caactcaacg tgctgcgtca gatgctgaat     900
gatccagcca atgccgatgg caaggtcagc ttcaaacacc tgtatcaagc tttgagcgag     960
acagggttga gcgatccacg aagtcgcact ttgaatctgg agcgcagcag ctgtgatcaa    1020
ctgtccggta acaacacggt tgctcgttgg cgtgcgttgg gactgtacga ccaatggctg    1080
acgctagccc ctcgcgacca gacaaccatc atcaacttgc tggcggactt gggcgggccg    1140
gaactgctgg ttccggacga ctggcaccag aacatccgca agagcagcgc caaagctggc    1200
gataccaacc tttatcgtcg attccggccc gaggtcgttg atttcatcaa tgcgcttcgc    1260
gagtgcaagg acaacaatgg gcagccagtt ttcaatcggc tgacatctat gaagttcgag    1320
ggcggacgca gcagctattc cgtcaaggcc ttggaaaaat tgaatacctg gttcaatgat    1380
ccttggtggc ccgacgaagg ctcaggggcc gagcgtggtg acgaaatcga tgaagacgca    1440
gcactacggg tctgctatcc acatcttttt acgcagcccc aagagctgct ccaacgactc    1500
gaggccgctc cgaccacggg caacgacgtg gttgacggag ccttgcgcga attacgcaag    1560
gtcatcaacc aatgcattga cgctttgggt gagaggccag aggaaatcgt cgtcgaaatg    1620
gcacgagaga tcggcaaggg cgtcgtttgg cggaaagagc aggaaaaccg catacggcag    1680
aatgaactcg cccgaaggaa aattgccagc gaactggaaa agcacggact gagcgccaac    1740
aacacccgga tactgcgcta tcaactctgg gaagagcagg atttgcactg gtgcccctac    1800
tgcgcccatc ccattaatct ggaagcagcc acctcgggca tgacacacat agagcacgtc    1860
ctgccacgct cgctcaccca gatcgggcgc aaacgcagcg aactggtgct ggcccacgcc    1920
ggttgcaaca acctgaaagc cgatcagaca ccttggcaag catttggcca tgatgtcgag    1980
cgctggtctg cagtagaacg cgctgccgcc cgttttcaag agctgggcaa gaagtcttac    2040
gccaaggacc gcatcaaggc gatgggcttc taccgcaagg cggaacttct cctgctgaag    2100
gattacgaaa gcgaagtact gagcgacgaa tccatagccg acttcgccga ccgccagtta    2160
catcagacct cctggatcgc caaggctgca gcccagtggt tgcgcaccat caccccgctg    2220
gtttccgtct ctcgcggtga attcaccgcc ctactacgcc atcaatggcg actcgacacc    2280
gtgatcgcag aagcgcgcct tgatgaaaaa cttcctgttc tggatatcga tgcgtgcccc    2340
gtatctccgg aagcattcca gcagttccgg aaacaatggg aagggcatcc accagacaac    2400
ctggccgaac ggaccgagcg caaactggaa aaacgcctcg accatcgcca ccacggcata    2460
gatgccttgg tcatcgcgct caccagccga agcctctaca tgtctctggc ccggcgctac    2520
gttgaagaaa gcgaaaggcg cgcccgcggc gagagagtcc ggcatgattg gtccatcgag    2580
ccgactatgc acaatctgcg caaccaagcg ctgggtatgg tccgtacttg caacctgacc    2640
cacaagcctg accgcaaccc agccggccga ctattcaagg acaccgccta tggcgtccac    2700
tttccagagg agggcgatcg ggtacagctg acgctgcgca aacctttgac cagcctggcc    2760
gacgaaaagt ctgccgagaa gactcgcaag aacctgcaca gcattgccag cagtgaggta    2820
cgcctactcg ttctgaaagc attcgaagcg cgcctcagtg tcgggcagaa tccgaaacag    2880
gcgctctccg aaccggtacc ctaccccgcc tatggcaccg caatctggcg agtccgctgc    2940
atgcaaggct acgcggacga tgccgtccgt attgtccata aaagccgtca gggcgaacac    3000
gaaaagcacc tgctcaacga tggctatgcc tgcttggaaa taaccgctgg ggaaaatggc    3060
aaacaccaag tacgccttgt cactcagcgt gaagcacaag gccgcggctt catggataac    3120
cggcagcagg cgaaacgcat attcaaggga gatacggtga tcgaccggcg cgacgaccgt    3180
aaatacctag tgggctactt caaggccgag ggctatattt tcctcctccc ccacacggac    3240
acccgcaact tcgacaagat caaggaggcg gactcgggca agaagaaagt ttccttcaac    3300
caagcgtacc aacttgagcg catctag                                        3327

<210>  46
<211>  3327
<212>  DNA
<213>  Artificial sequence

<220>
<223>  Cas endonuclease of Locus 28

<400>  46
atggcataca aattaggtat cgatctgggc accgcttcca tcggtgcggt tgccattcgt      60
attgaaaata aggtgcctat cagcgttgac tggcatgcag tgcgcatttt cagcgagccc     120
gtcgtaaaca ctcagaaagg gctcacgccc aaaaagacgg cccgtcgtga agcccggcaa     180
ttgcgtaaac aggtcgaacg acgagcccga cggttgcgcc gtattgccaa tctgattcca     240
ttgctggggt tgcagaaagc cgaagtaaag cttgggcgcc gtcaggactt gaccacgttg     300
cgagctattg ccgcgcgcga gaaggtcgaa ctacatgagt ttgccctggt actgctgcgt     360
attgccaaac gccgcggtta cgccggtgga tttcgcgatt ccgaaggcgg cgtggtcaag     420
actgccagtg acaaactgag cgtggaaatg agagctcttg ccgagacgcg aggcctgcaa     480
cagataactc tgggcgaata cttactgcat cgacagcaac gagggctgcc tatctggctc     540
aaagctgagc gtccaggctt ggaaccgctc tatgctctgc gggagatggt aaccgccgaa     600
ttcgaacaaa tctggacaat ccaagccaaa tgccatccga tcctgaacag ctgtcatctc     660
gaaaaaccac ttaagcaatg gttctatcaa gcactgttct atcagcgacc actgaaatct     720
cccgcccaca tggtcggcaa ttgcctgctt gaacccaatt tgccacgcgc cccacgcgct     780
caaatggcag cccaacgctt caggatagaa aaaacgcttc acgatttacg ctggggtggc      840
ggacgacagg ctgaacgcct gaacacagcg caactcgacc tactgcgtca gactctgagc     900
gatccaacca atgttgatag caaggtcagc tttaagcgcc tgtaccaagc cctgaacgag     960
gctgggttga acgatccgcg aaatcgcaca ttaaacctgg agcgcagcag tcgcgatcag    1020
ttacttggca atagcaccgt tgcccgttgg cgcgcgctgg gcctgcaaga tcaatggctg    1080
atgctggctc ctcgcgacca gacatctgtt atcaacctgc tggcagactt gggtggtccg    1140
gaaatactga ccccagacga ctggcaccag aacatccgta aaagcagtgc caaggacgac    1200
aacaccaaag cctatcgtcg attccggcag gaagttattg atttcatcga tgcgcttcgc    1260
cggtgcatag atagtaatgg ccagccggtt ttcaatcgtc tttccgctat gaagttcgac    1320
ggcggacgca gtagctactc ggtaaaggcc ttggaaaaga tgaacgaatg gttcaataac    1380
ccgtggtggc ccaatgaagg tccaggctca gagcgtcgcg gcgagatcga cgaagaagtg    1440
gcgatacggg tgtgttaccc acatctttat acgcaatctc aagagctgca cccgcgactc    1500
gaagccgctc cgaccaccgg caacgacgtt gtcgatggag cattacgcga actgcgcaag    1560
gtcatcaacc aatgcattga cgagcaaggt gagaaaccac aggaaatcat catcgaattg    1620
gcaagagagg tcgggaaagg tgtggctttg cggaatgaac aggaaagtcg cattcggaac    1680
aacgaactcg ccaggcggaa aattgccagc gaacttgaag ggcacggttt gattgccagc    1740
agcacccgga tactgcgata tcaactctgg gaggagcaag agcgacactg gtgtccttat    1800
tgcgcacttc ccatcagcct ggaagcagcc atctcaaaca tgacgcacat agaacacgtc    1860
ctgccccgtt cgctcaccca agtcggtcga aaacgcagtg aactggtgct agcccatgct    1920
ggttgcaatc atctgaaagc cgaccagacg ccttggcaag ccttcggcca cgatgccgag    1980
cgctggtctg cagtagaacg tgctgccgcc cgatttcagg agctgggtaa aaagtgctat    2040
ttcaaagaca gggcgaaggc gatgggttgc ttccgaaaat cagcccttct catgctgaag    2100
gattatgaaa gtgaagtatt aaacgacgaa tccatagccg gattcgccaa ccgtcagtta    2160
caccagacat cttggattgc caaggttgca gcccaatggt tacgtacgat ttcaccgctg    2220
atatccgttt cccgcggcga attcaccgcc ctgctgcgcc atcaatggcg actagacacc    2280
gttatcgcag aaacgcgcta tggtgaaaaa cttcccgttc tggatatcga tggcaatccc    2340
gtccatccgg aagaatttca gcacttccgg aaacaatggg aaggacatcc accggacaac    2400
cccgccgaaa gaaccgagcg caaactggaa aagcgcttcg accaccgcca ccatggcatt    2460
gatgccctgg tcatcgcgct caccagccgt agtttgtaca tgtctcttgc tcggcgctat    2520
gtagaagaaa gcaaaaagcg cgcccgcggc gagaaagtcc ggcgtgactg gtccattgag    2580
ccacctatgc acaatttgcg cggccaagct ctggatatga tccgcgcgtg caacctgacc    2640
ctcaagcccg accgcaacgc tgccggccga ctgtttgagg actacgccta tggcgtccac    2700
ttttctgacg caagcgagcg ggcacagctt acgcggcgca aaacattgat agatcttgca    2760
gacgaaaagt ccgcagagaa gactcgcaaa aatctccaga gcatcgcaag ccctgaggta    2820
cgccagatcg ttgtggaagc gctccaaatg cgcctaagtg ccgggcaaaa tccgaaacag    2880
gcgctctcca aacccgtact ctaccccgct tacggtacag aaatttggag ggtccgttgc    2940
ttacaaggct acgcggacca tgccgtgcga atcgttcaca atagccgcca gggtgaacac    3000
gaaaagtgtc tgctcaacga tggctatgcc tgcctggaga taagggccgg ggaaaatggc    3060
aaacaccaag cacgtctcgt cactcagcgg gaagcacagg gtcgtgactt catggaaacc    3120
cagcagcagg cgaagcgcct attcaaggga gatacggtga tcgacaggcg cgatgatcgt    3180
aaataccgag tgggttattt caccgccgag ggcaatattt tcctcatccc cttcgaggac    3240
actcgtaatt tcgataaaat caatgagcgg aactcgggca agaaaaaggt ttccttcaac    3300
caagcgcatc gacttgagcg agtatga                                        3327

<210>  47
<211>  1093

<212>  PRT
<213>  Bacillus cereus

<400>  47
Met Ala Tyr Thr Met Gly Ile Asp Val Gly Ile Ala Ser Cys Gly Trp
1               5                   10                  15
Ala Ile Val Asp Leu Glu Arg Gln Arg Ile Ile Asp Leu Gly Val Arg
            20                  25                  30
Thr Phe Glu Lys Ala Glu Asn Pro Lys Asn Gly Glu Ala Leu Ala Val
        35                  40                  45
Pro Arg Arg Glu Ala Arg Ser Ser Arg Arg Arg Leu Arg Arg Lys Lys
    50                  55                  60
His Arg Ile Glu Arg Leu Lys His Met Phe Ile Arg Asn Gly Leu Ala
65                  70                  75                  80
Val Asp Ile Gln His Leu Glu Gln Gln Thr Leu Arg Ser Gln Asn Glu
                85                  90                  95
Ile Asp Val Trp Gln Leu Arg Val Asp Gly Leu Asp Arg Met Leu Thr
            100                 105                 110
Gln Lys Glu Trp Leu Arg Val Leu Ile His Leu Ala Gln Arg Arg Gly
        115                 120                 125
Phe Gln Ser Asn Arg Lys Thr Asp Gly Ser Ser Glu Asp Gly Gln Val
    130                 135                 140
Leu Val Asn Val Thr Glu Asn Asp Arg Leu Met Glu Glu Lys Asp Tyr
145                 150                 155                 160
Arg Thr Val Ala Glu Met Met Val Lys Asp Glu Lys Phe Ser Asp His
                165                 170                 175
Lys Arg Asn Lys Asn Gly Asn Tyr His Gly Val Val Ser Arg Ala Ser
            180                 185                 190
Leu Leu Val Glu Ile His Thr Leu Phe Glu Ser Gln Arg Gln His His
        195                 200                 205
Asn Phe Leu Ala Ser Lys Asp Phe Glu Leu Glu Tyr Val Asn Ile Trp
    210                 215                 220
Ser Ser Gln Arg Pro Val Ala Thr Lys Asp Gln Ile Glu Lys Met Ile
225                 230                 235                 240
Gly Thr Cys Thr Phe Leu Pro Lys Glu Lys Arg Thr Pro Lys Ala Ser
                245                 250                 255
Trp His Phe Gln Tyr Phe Met Leu Leu Gln Thr Ile Asn His Ile Arg
            260                 265                 270
Ile Thr Asn Ile Gln Gly Thr Arg Ser Leu Asn Lys Glu Glu Ile Glu
        275                 280                 285
Gln Val Val Asn Met Ala Leu Thr Lys Ser Lys Val Ser Tyr His Asp
    290                 295                 300
Ile Arg Lys Val Leu Asp Leu Ser Glu Glu Tyr Gln Phe Val Gly Leu
305                 310                 315                 320
Asp Tyr Gly Lys Glu Asn Glu Lys Lys Lys Val Glu Ser Lys Glu Thr
                325                 330                 335
Ile Ile Lys Leu Asp Asp Tyr His Lys Leu Asn Lys Ile Phe Asn Glu
            340                 345                 350
Val Glu Leu Ala Lys Gly Glu Ala Trp Lys Ala Val Asp Tyr Asp Thr
        355                 360                 365
Val Ala Tyr Ala Leu Thr Phe Phe Lys Asp Asp Glu Asp Ile Arg Asp
    370                 375                 380
Tyr Leu Gln Asn Lys Tyr Lys Asp Ser Lys Asn Arg Leu Val Lys Asn
385                 390                 395                 400
Val Ala Asn Lys Glu Tyr Thr Asn Glu Leu Val Glu Lys Val Ser Thr
                405                 410                 415
Leu Ser Phe Arg Lys Val Gly His Leu Ser Leu Lys Ala Leu Arg Lys
            420                 425                 430
Ile Ile Pro Phe Leu Glu Gln Gly Met Thr Tyr Asp Lys Ala Cys Gln
        435                 440                 445
Ala Ala Gly Phe Asn Phe Gln Gly Ile Ser Lys Lys Lys Arg Ser Val
    450                 455                 460
Val Leu Pro Val Ile Asp Gln Ile Ser Asn Pro Val Val Asn Arg Ala

465                 470                 475                 480
Leu Thr Gln Thr Arg Lys Val Ile Asn Ala Leu Ile Lys Lys Tyr Gly
                485                 490                 495
Ser Pro Glu Thr Ile His Ile Glu Thr Ala Arg Glu Leu Ser Lys Arg
            500                 505                 510
Phe Asp Glu Arg Lys Asn Ile Thr Lys Asp Tyr Lys Glu Asn Arg Asp
        515                 520                 525
Lys Asn Glu Arg Ala Lys Lys His Leu Ser Glu Leu Gly Ile Ile Asn
    530                 535                 540
Pro Thr Gly Leu Asp Ile Val Lys Tyr Lys Leu Trp Cys Glu Gln Gln
545                 550                 555                 560
Gly Arg Cys Met Tyr Ser Asn Gln Pro Ile Ser Phe Glu Arg Leu Arg
                565                 570                 575
Glu Ser Gly Tyr Thr Glu Val Asp His Ile Ile Pro Tyr Ser Arg Ser
            580                 585                 590
Met Asn Asp Ser Tyr Asn Asn Arg Val Leu Val Met Thr Arg Glu Asn
        595                 600                 605
Arg Glu Lys Ser Asn Gln Thr Pro Tyr Glu Tyr Met Gly Asn Asp Thr
    610                 615                 620
Gln Arg Trp Asp Glu Phe Glu Gln Arg Val Thr Thr Asn Pro Gln Ile
625                 630                 635                 640
Lys Lys Glu Lys Arg Gln Asn Leu Leu Leu Lys Gly Phe Thr Asn Arg
                645                 650                 655
Arg Glu Leu Glu Met Leu Glu Arg Asn Leu Asn Asp Thr Arg Tyr Ile
            660                 665                 670
Thr Lys Tyr Leu Ser His Phe Ile Ser Thr Asn Leu Glu Phe Ser Pro
        675                 680                 685
Ser Asp Lys Lys Lys Lys Val Val Asn Thr Ser Gly Arg Ile Thr Ser
    690                 695                 700
His Leu Arg Ser Arg Trp Gly Leu Glu Lys Asn Arg Glu Gln Asn Asp
705                 710                 715                 720
Leu His His Ala Met Asp Ala Val Val Ile Ala Val Thr Ser Asp Ser
                725                 730                 735
Phe Ile Gln Gln Val Thr Asn Tyr Tyr Lys Arg Lys Glu Arg Arg Glu
            740                 745                 750
Leu Asn Arg Asp Asp Lys Phe Pro Leu Pro Trp Lys Phe Phe Arg Glu
        755                 760                 765
Glu Val Ile Ala Arg Leu Ser Pro Asn Pro Lys Glu Gln Ile Glu Ala
    770                 775                 780
Leu Pro Asn His Phe Tyr Ser Glu Asp Glu Leu Ala Asp Leu Gln Pro
785                 790                 795                 800
Ile Phe Val Ser Arg Met Pro Lys Arg Ser Ile Thr Gly Glu Ala His
                805                 810                 815
Gln Ala Gln Phe Arg Arg Val Val Gly Lys Thr Lys Glu Gly Lys Asn
            820                 825                 830
Ile Thr Ala Lys Lys Thr Ala Leu Val Asp Ile Ser Tyr Asp Lys Asn
        835                 840                 845
Gly Asp Phe Asn Met Tyr Gly Arg Glu Thr Asp Pro Ala Thr Tyr Glu
    850                 855                 860
Ala Ile Lys Glu Arg Tyr Leu Glu Phe Gly Gly Asn Ala Lys Lys Ala
865                 870                 875                 880
Phe Ser Thr Asp Leu His Lys Pro Lys Lys Asp Gly Thr Lys Gly Pro
                885                 890                 895
Leu Ile Lys Ser Val Arg Ile Met Glu Asn Lys Thr Leu Val His Pro
            900                 905                 910
Val Asn Lys Gly Lys Gly Val Val Tyr Asn Ser Ser Ile Val Arg Thr
        915                 920                 925
Asp Val Phe Gln Arg Lys Glu Lys Tyr Tyr Leu Leu Pro Val Tyr Val
    930                 935                 940
Thr Asp Val Thr Lys Gly Lys Leu Pro Asn Lys Val Ile Val Ala Lys
945                 950                 955                 960
Lys Gly Tyr Asp Asp Trp Ile Glu Val Asp Asp Ser Phe Thr Phe Leu
                965                 970                 975

Tyr Ser Leu Tyr Pro Asn Asp Leu Ile Phe Ile Arg Gln Asn Pro Lys
            980                 985                 990
Lys Lys Ile Ser Leu Lys Lys Arg  Ile Glu Ser His Ser  Ile Ser Asp
        995                 1000                1005
Ser Lys  Glu Val Gln Glu Ile  Leu Ala Tyr Tyr Lys  Gly Val Asp
    1010                1015                1020
Ser Ser  Thr Ala Ala Ile Glu  Phe Ile Thr His Asp  Gly Ser Tyr
    1025                1030                1035
Tyr Ala  Lys Gly Val Gly Val  Gln Asn Leu Asp Cys  Phe Glu Lys
    1040                1045                1050
Tyr Gln  Val Asp Thr Leu Gly  Asn Tyr Ser Lys Val  Lys Gly Glu
    1055                1060                1065
Lys Arg  Leu Glu Leu Glu Thr  Ser Asp Asn Asn Asp  Lys Gly Lys
    1070                1075                1080
Asp Val  Asn Ser Ile Lys Ser  Thr Ser Arg
    1085                1090

<210>  48
<211>  1092
<212>  PRT
<213>  Brevibacillus laterosporus

<400>  48
Met Ala Tyr Thr Met Gly Leu Asp Val Gly Ile Ala Ser Cys Gly Trp
1               5                   10                  15
Ala Ile Val Asp Ile Glu Arg Gln Arg Ile Ile Asp Val Gly Val Arg
            20                  25                  30
Ile Phe Glu Lys Ala Glu Asn Pro Lys Asn Gly Glu Ala Leu Ala Val
        35                  40                  45
Pro Arg Arg Glu Ala Arg Ser Ser Arg Arg Arg Leu Arg Arg Lys Lys
    50                  55                  60
His Arg Ile Glu Arg Leu Lys His Met Phe Ile Arg Asn Arg Leu Ala
65                  70                  75                  80
Val Asp Ile Gln His Leu Glu Gln Thr Leu Arg Ser Gln Asn Glu Ile
                85                  90                  95
Asp Val Trp Gln Leu Arg Val Asp Gly Leu Asp Arg Val Leu Thr Gln
            100                 105                 110
Lys Glu Trp Leu Arg Val Leu Ile His Leu Ala Gln Arg Arg Gly Phe
        115                 120                 125
Gln Ser Asn Arg Lys Thr Asp Gly Leu Asn Glu Asp Gly Gln Val Leu
    130                 135                 140
Val Asn Val Ser Glu Asn Glu Arg Leu Met Glu Lys Lys Asp Tyr Arg
145                 150                 155                 160
Thr Val Ala Glu Met Met Val Lys Asp Gly Lys Phe Ser Asp His Lys
                165                 170                 175
Arg Asn Lys Asn Gly Asn Tyr His Gly Val Val Ser Arg Ala Ser Ile
            180                 185                 190
Leu Val Glu Ile His Ala Leu Phe Glu Ser Gln Arg Gln His His Asn
        195                 200                 205
Phe Phe Ala Ser Lys Asp Phe Glu Leu Glu Tyr Val Asn Ile Trp Ser
    210                 215                 220
Ser Gln Arg Pro Val Ala Thr Lys Asp Gln Ile Glu Lys Met Ile Gly
225                 230                 235                 240
Thr Cys Thr Phe Leu Pro Lys Glu Lys Arg Ala Pro Lys Ala Ser Trp
                245                 250                 255
His Phe Gln Tyr Phe Met Leu Leu Gln Thr Ile Asn His Ile Arg Ile
            260                 265                 270
Thr Tyr Leu Gln Val Thr Arg Ser Leu Asn Lys Glu Glu Ile Glu Gln
        275                 280                 285
Val Val Asn Met Ala Leu Thr Lys Ser Lys Val Ser Tyr His Asp Ile
    290                 295                 300
Arg Lys Ile Leu Asp Leu Ser Glu Glu Tyr Gln Phe Val Gly Leu Asp
305                 310                 315                 320

Tyr Gly Lys Glu Asp Glu Lys Lys Lys Val Glu Ser Lys Glu Thr Ile
                325                 330                 335
Ile Lys Leu Asp Asp Tyr His Lys Leu Asn Lys Ile Phe Asn Glu Val
            340                 345                 350
Glu Phe Ala Lys Gly Glu Thr Trp Glu Ala Val Asp Tyr Asp Thr Val
        355                 360                 365
Ala Tyr Ala Leu Thr Phe Phe Lys Asp Asp Glu Asp Ile Arg Asp Tyr
    370                 375                 380
Leu Gln Asn Lys Tyr Lys Asp Ser Lys Asn Arg Leu Val Lys Asn Leu
385                 390                 395                 400
Ala Asn Lys Glu Tyr Thr Asn Glu Leu Ile Glu Lys Val Ser Thr Leu
                405                 410                 415
Ser Phe Arg Lys Val Gly His Leu Ser Leu Lys Ala Leu Arg Lys Ile
            420                 425                 430
Ile Pro Phe Leu Glu Gln Gly Met Thr Tyr Asp Lys Ala Cys Gln Ala
        435                 440                 445
Ala Gly Phe Asp Phe Gln Gly Ile Ser Lys Lys Lys Arg Tyr Val Val
    450                 455                 460
Leu Pro Val Ile Asp Gln Ile Ser Asn Pro Val Val Asn Arg Ala Leu
465                 470                 475                 480
Thr Gln Thr Arg Lys Val Ile Asn Ala Leu Ile Lys Lys Tyr Gly Ser
                485                 490                 495
Pro Glu Thr Ile His Ile Glu Thr Ala Arg Glu Leu Ser Lys Thr Phe
            500                 505                 510
Asp Glu Arg Lys Asn Ile Thr Lys Asp Tyr Lys Glu Asn Arg Asp Lys
        515                 520                 525
Asn Glu His Ala Lys Lys His Leu Ser Glu Leu Gly Ile Ile Asn Pro
    530                 535                 540
Thr Gly Leu Asp Ile Val Lys Tyr Lys Leu Trp Cys Glu Gln Gln Gly
545                 550                 555                 560
Arg Cys Met Tyr Ser Asn Arg Pro Ile Ser Phe Glu Arg Leu Lys Glu
                565                 570                 575
Ser Gly Tyr Thr Glu Val Asp His Ile Ile Pro Tyr Ser Arg Ser Met
            580                 585                 590
Asn Asp Ser Tyr Asn Asn Arg Val Leu Val Met Thr Arg Glu Asn Arg
        595                 600                 605
Glu Lys Gly Asn Gln Thr Pro Phe Glu Tyr Met Gly Asn Asp Thr Gln
    610                 615                 620
Arg Trp Tyr Glu Phe Glu Gln Arg Val Thr Thr Asn Pro Gln Ile Lys
625                 630                 635                 640
Lys Glu Lys Arg Gln Asn Leu Leu Leu Lys Gly Phe Thr Asn Arg Arg
                645                 650                 655
Glu Leu Glu Met Leu Glu Arg Asn Leu Asn Asp Thr Arg Tyr Ile Thr
            660                 665                 670
Lys Tyr Leu Ser His Phe Ile Ser Thr Asn Leu Glu Phe Ser Pro Ser
        675                 680                 685
Asp Lys Lys Lys Lys Val Val Asn Thr Ser Gly Arg Ile Thr Ser His
    690                 695                 700
Leu Arg Ser Arg Trp Gly Leu Glu Lys Asn Arg Glu Gln Asn Asp Leu
705                 710                 715                 720
His His Ala Met Asp Ala Val Val Ile Ala Val Thr Ser Asp Ser Phe
                725                 730                 735
Ile Gln Gln Val Thr Asn Tyr Tyr Lys Arg Lys Glu Arg Arg Glu Leu
            740                 745                 750
Asn Gly Asp Asp Lys Phe Pro Leu Pro Trp Lys Phe Phe Arg Glu Glu
        755                 760                 765
Val Ile Ala Arg Leu Ser Pro Asn Pro Lys Glu Gln Ile Glu Ala Leu
    770                 775                 780
Pro Asn His Phe Tyr Ser Glu Asp Glu Leu Ala Asp Leu Gln Pro Ile
785                 790                 795                 800
Phe Val Ser Arg Met Pro Lys Arg Ser Ile Thr Gly Glu Ala His Gln
                805                 810                 815
Ala Gln Phe Arg Arg Val Val Gly Arg Thr Lys Glu Gly Lys Asn Ile

            820                 825                 830
Thr Ala Lys Lys Thr Ala Leu Val Asp Ile Gly Tyr Asp Lys Asn Gly
        835                 840                 845
Asp Phe Asn Met Tyr Gly Lys Glu Thr Asp Pro Ala Thr Tyr Glu Ala
    850                 855                 860
Ile Lys Gln Arg Tyr Leu Glu Phe Gly Gly Asn Ala Lys Lys Ala Phe
865                 870                 875                 880
Ser Met Asp Leu His Lys Pro Lys Lys Asp Gly Ala Asn Gly Pro Leu
                885                 890                 895
Ile Lys Ser Val Arg Ile Met Asp Asn Lys Thr Leu Val His Pro Val
            900                 905                 910
Asn Lys Gly Lys Gly Val Val Tyr Asn Ser Ser Ile Val Arg Thr Asp
        915                 920                 925
Val Tyr Gln Arg Lys Glu Lys Tyr Tyr Leu Leu Pro Val Tyr Val Thr
    930                 935                 940
Asp Val Thr Lys Ala Lys Leu Pro Asn Lys Val Ile Val Ala Lys Lys
945                 950                 955                 960
Gly Tyr Asp Asp Trp Ile Glu Val Asp Asp Ser Phe Thr Phe Leu Phe
                965                 970                 975
Ser Leu Tyr Pro Asn Asp Leu Ile Phe Val Arg Gln Asn Gln Lys Lys
            980                 985                 990
Lys Ile Ser Leu Lys Lys Arg Ile Glu Ser His Phe Ile Ser Asp Ser
        995                 1000                1005
Lys Lys Val Gln Glu Ile Leu Ala Tyr Tyr Lys Gly Val Asp Ser
    1010                1015                1020
Ala Ser Ala Thr Ile Glu Phe Ile Thr His Asp Gly Ser Tyr Tyr
    1025                1030                1035
Ala Lys Ser Val Gly Val Gln Asn Leu Asp Cys Phe Glu Lys Tyr
    1040                1045                1050
Gln Val Asp Ile Leu Gly Asp Tyr Ser Lys Val Lys Gly Glu Lys
    1055                1060                1065
Arg Leu Glu Leu Glu Thr Ser Asp Ser Asn His Lys Gly Lys Asp
    1070                1075                1080
Val Asn Ser Ile Lys Ser Thr Ser Arg
    1085                1090

<210> 49
<211> 1070
<212> PRT
<213> Artificial sequence

<220>
<223> Cas-Locus 8

<400> 49
Met Arg Glu Leu Asp Tyr Arg Ile Gly Leu Asp Ile Gly Thr Asn Ser
1               5                   10                  15
Ile Gly Trp Gly Val Ile Glu Leu Ser Trp Asn Lys Asp Arg Glu Gln
            20                  25                  30
Tyr Glu Lys Ala Arg Ile Val Asp Lys Gly Val Arg Met Phe Asp Lys
        35                  40                  45
Ala Glu Ile Pro Lys Thr Gly Ala Ser Leu Ala Glu Pro Arg Arg Ile
    50                  55                  60
Ala Arg Ser Ser Arg Arg Arg Leu Asn Arg Lys Ser Gln Arg Lys Lys
65                  70                  75                  80
Asp Ile Arg Asn Leu Leu Val Gln His Glu Ile Ile Ser Gln Lys Glu
                85                  90                  95
Leu Ala Ser Leu Tyr Pro Leu Ser Lys Ser Ser Met Asp Ile Trp Asp
            100                 105                 110
Ile Arg Leu Asp Gly Leu Asp Arg Leu Leu Asp Arg Phe Glu Trp Ala
        115                 120                 125
Arg Leu Leu Ile His Leu Ala Gln Lys Arg Gly Phe Lys Ser Asn Arg
    130                 135                 140

Lys Ser Glu Leu Lys Asp Val Glu Thr Gly Lys Val Leu Ser Ser Ile
145                 150                 155                 160
Gln Ala Asn Glu Lys Arg Leu Ser Leu Tyr Arg Thr Val Gly Glu Met
                165                 170                 175
Trp Met Lys Asn Met Asp Phe Ser Lys Tyr Gly Lys Arg Arg Asn Ser
            180                 185                 190
Pro Asn Glu Tyr Val Phe Ser Val Ser Arg Ala Asp Leu Glu Lys Glu
        195                 200                 205
Ile Val Thr Leu Phe Glu Ala Gln Arg Lys Phe His Ser Ser Tyr Ala
    210                 215                 220
Ser Val Asp Leu Gln Lys Thr Tyr Leu Arg Ile Trp Ala His Gln Leu
225                 230                 235                 240
Pro Phe Ala Ser Gly Asn Ala Ile Val Asn Lys Val Gly Tyr Cys Ser
                245                 250                 255
Leu Leu Lys Gly Lys Glu Lys Arg Val Pro Lys Ala Thr Tyr Thr Phe
            260                 265                 270
Gln Tyr Phe Asn Thr Leu Asp Gln Ile Asn Arg Thr Arg Leu Gly Pro
        275                 280                 285
Asp Phe Gln Pro Phe Thr Lys Glu Gln Arg Asp Ile Ile Leu Asp Lys
    290                 295                 300
Met Phe Gln Arg Thr Asp Tyr Tyr Lys Lys Lys Thr Ile Pro Glu Val
305                 310                 315                 320
Thr Tyr Tyr Asp Ile Arg Lys Trp Leu Ala Leu Asp Glu Thr Ile Gln
                325                 330                 335
Phe Lys Gly Leu Thr Tyr Asp Pro Asn Glu Glu Leu Lys Lys Ile Glu
            340                 345                 350
Leu Lys Pro Phe Ile Asn Leu Lys Pro Phe Tyr Glu Ile Lys Lys Val
        355                 360                 365
Val Thr Asn Tyr Ala Gln Lys Thr Asn Glu Val Phe Ser Thr Leu Asp
    370                 375                 380
Tyr Asp Thr Val Ala Tyr Ala Leu Thr Val Tyr Lys Thr Asp Lys Asp
385                 390                 395                 400
Ile Arg Ser Tyr Leu Lys Arg Ser Asn Asn Leu Ser Lys Arg Cys Tyr
                405                 410                 415
Asp Asp Gln Leu Ile Glu Glu Leu Leu Thr Leu Ser Tyr Thr Lys Phe
            420                 425                 430
Gly His Leu Ser Phe Lys Ala Ile Asn His Val Leu Pro Ile Met Gln
        435                 440                 445
Glu Gly Arg Thr Tyr Gln Glu Ala Ile Asn Gln Leu Gly Tyr Asp Thr
    450                 455                 460
Thr Asn Leu Lys Lys Glu Asn Arg Ser Met Phe Leu Pro Leu Ile Pro
465                 470                 475                 480
Asp Glu Ile Thr Asn Pro Ile Val Lys Arg Ala Leu Thr Gln Ala Arg
                485                 490                 495
Lys Val Val Asn Ala Ile Ile Arg Arg Tyr Gly Ser Pro Asn Ser Val
            500                 505                 510
His Ile Glu Leu Ala Arg Glu Leu Ser Lys Ser His Asp Glu Arg Lys
        515                 520                 525
Lys Ile Met Thr Ala His Asp Glu Asn Tyr Lys Lys Asn Lys Gly Ala
    530                 535                 540
Ile Ser Ile Leu Ile Glu Asn Gly Ile Leu Asn Pro Thr Gly Tyr Asp
545                 550                 555                 560
Ile Val Arg Tyr Lys Leu Trp Lys Glu Gln Gly Glu Arg Cys Ala Tyr
                565                 570                 575
Ser Leu Lys Lys Ile Pro Ala Asn Thr Phe Phe Asn Glu Leu Lys Lys
            580                 585                 590
Glu Arg Ser Gly Pro Pro Val Leu Glu Val Asp His Ile Leu Pro Tyr
        595                 600                 605
Ser Gln Ser Phe Ile Asp Ser Tyr His Asn Lys Val Leu Val Tyr Gly
    610                 615                 620
Asp Glu Asn Gln Lys Lys Gly Asn Arg Ile Pro Tyr Thr Phe Phe Ser
625                 630                 635                 640
Glu Glu Asn Lys Glu Trp Glu Ser Phe Glu Ser Tyr Val Arg Ser Asn

                645                 650                 655
Ser Phe Phe Ser Lys Lys Lys Arg Gly Tyr Leu Leu Lys Lys Ala Tyr
            660                 665                 670
Leu Pro Arg Glu Ser Asn Leu Ile Lys Glu Arg His Leu Asn Asp Thr
        675                 680                 685
Arg Tyr Ala Ser Ser Tyr Leu Lys Asn Phe Ile Glu Lys Asn Leu Lys
    690                 695                 700
Phe Lys Glu Ala Glu Gly Ile Thr Arg Lys Lys His Val Gln Thr Val
705                 710                 715                 720
Asn Gly Val Ile Thr Ala His Leu Arg Lys Arg Trp Gly Leu Glu Lys
                725                 730                 735
Asp Arg Gln Glu Thr Tyr Leu His His Ala Met Asp Ala Ile Ile Val
            740                 745                 750
Ala Cys Thr Asp His His Met Val Thr Lys Val Thr Glu Tyr Tyr Gln
        755                 760                 765
Ile Lys Glu Ser Asn Lys Ser Ile Lys Lys Pro Tyr Phe Pro Leu Pro
    770                 775                 780
Trp Met Gly Phe Arg Glu Glu Ile Leu Ser His Leu Glu Ser Gln Pro
785                 790                 795                 800
Ile Ala Arg Lys Ile Ser Glu Glu Leu Lys Ile Gly Tyr Gln Ser Ser
                805                 810                 815
Asp Tyr Ile Leu Val Ser Arg Met Pro Lys Arg Ser Val Thr Gly Ser
            820                 825                 830
Ala His Asp Gln Thr Val Met Lys Lys Gly Gly Ile Asp Lys Lys Gly
        835                 840                 845
Lys Thr Ile Ile Ile Lys Arg Val His Leu Lys Asp Ile Lys Phe Asp
    850                 855                 860
Glu Asn Gly Asp Phe Lys Met Val Gly Lys Glu Gln Asp Leu Ala Thr
865                 870                 875                 880
Tyr Glu Ala Ile Lys Gln Arg Tyr Leu Glu Tyr Gly Lys Glu Ser Lys
                885                 890                 895
Lys Ala Phe Glu Thr Pro Leu Tyr Lys Pro Ser Lys Lys Gly Lys Gly
            900                 905                 910
Asn Leu Ile Lys Lys Ile Lys Val Glu Val Gln Thr Lys Ser Phe Val
        915                 920                 925
Arg Glu Val Asn Gly Gly Val Ala Gln Asn Gly Asp Leu Val Arg Ile
    930                 935                 940
Asp Leu Phe Glu Lys Asp Asn Arg Tyr Tyr Met Val Pro Ile Tyr Val
945                 950                 955                 960
Val Asp Thr Val Arg Ser Glu Leu Pro Asn Lys Ala Val Lys Ser Ser
                965                 970                 975
Lys Gly Tyr Glu Gln Trp Leu Thr Ile Asp Asn Ser Phe Thr Phe Lys
            980                 985                 990
Phe Ser Leu Tyr Ser Tyr Asp Leu Val Arg Leu Val Lys Gly Asp Glu
        995                 1000                1005
Asp Arg Phe Leu Tyr Phe Ser Thr Leu Asp Ile Asn Ser Asp Arg
    1010                1015                1020
Leu Asn Phe Lys Asp Val Asn Lys Pro Ser Lys Gln Ala Glu Tyr
    1025                1030                1035
Arg Tyr Ser Leu Lys Thr Ile Glu Asn Leu Glu Lys Tyr Glu Val
    1040                1045                1050
Gly Val Leu Gly Asp Leu Arg Leu Val Arg Gln Glu Thr Arg Lys
    1055                1060                1065
Ile Phe
    1070

<210> 50
<211> 1081
<212> PRT
<213> Bacillus cereus

<400> 50
Met Lys Tyr Val Leu Gly Leu Asp Ile Gly Ile Ala Ser Cys Gly Trp

1               5                   10                  15
Ala Val Ile Asn Gln Glu Lys His Arg Ile Glu Asp Leu Gly Val Arg
            20                  25                  30
Ile Phe Asp Lys Ala Glu His Pro Lys Asp Gly Lys Ser Leu Ala Ala
        35                  40                  45
Pro Arg Arg Glu Ser Arg Ser Thr Arg Lys Thr Leu Arg Arg Lys Lys
    50                  55                  60
His Arg Met Gln Arg Ile Lys Val Leu Leu Val Lys Tyr Gly Leu Leu
65                  70                  75                  80
Ser Thr Thr Glu Ile Glu Asn Leu Tyr Glu Ser Ala Ser Glu Ile Asp
                85                  90                  95
Val Trp Asn Leu Arg Leu Asp Ala Leu Glu Arg Lys Leu Asn Gln Lys
            100                 105                 110
Glu Phe Ala Arg Val Leu Ile His Leu Ala Lys Arg Arg Gly Phe Lys
        115                 120                 125
Ser Asn Met Lys Glu Thr Ile Leu Ser Glu Asn Gly Gln Ile Leu Glu
    130                 135                 140
Ser Ile Ser Glu Asn Gln Lys Ile Met Glu Gln Gln Asn Tyr Arg Thr
145                 150                 155                 160
Val Gly Glu Met Ile Val Lys Asp Asn Lys Phe Glu Asn His Lys Arg
                165                 170                 175
Asn Lys Asp Gly Thr Tyr Ile Gly Thr Val Thr Arg Gln Gln Leu Gln
            180                 185                 190
Asp Glu Ile His Met Ile Phe Asn Ala Gln Arg Leu Phe Ile Asn Asp
        195                 200                 205
Leu Ala Thr Glu Glu Phe Glu Ser Ser Tyr Leu Glu Ile Trp Ala Ser
    210                 215                 220
Gln Arg Pro Tyr Ala Ser Lys Asp Gln Ile Glu Lys Met Ile Gly Tyr
225                 230                 235                 240
Cys Thr Leu Glu Pro Lys Glu Lys Arg Val Pro Lys Ala Ser Trp Ser
                245                 250                 255
Phe Gln Tyr Phe Val Ala Leu Gln Thr Ile Asn Asn Leu Arg Leu Ile
            260                 265                 270
Ser Lys Asp Arg Ile Glu Glu Leu Ser Phe Glu Glu Lys Lys Gln Ile
        275                 280                 285
Met Asn Leu Ala Leu Glu Lys Pro Ile Val Lys Tyr Ile Asp Ile Arg
    290                 295                 300
Lys Leu Leu Ser Ile Ser Asn Glu Leu His Phe Asn Asn Leu Leu Tyr
305                 310                 315                 320
Ser Ala Asp Thr Val Asp Thr Thr Val Glu Asn Lys Lys Cys Ile Glu
                325                 330                 335
Leu Lys Glu Tyr His Ser Ile Asn Lys Val Tyr Lys Gln Ile Tyr Gly
            340                 345                 350
Lys Asn Ala Leu Asn Leu Leu Pro Ile Asp Tyr Asp Thr Ile Ala Tyr
        355                 360                 365
Gly Leu Thr Ile Phe Lys Asp Asp Lys Asp Ile Leu Glu Tyr Leu Gln
    370                 375                 380
Asn Lys Tyr Ile Asn Thr Lys Gly Lys Thr Ile Ser Asn Leu Thr Lys
385                 390                 395                 400
Lys Thr Tyr Asp Asp Thr Phe Ile Gln Ala Leu Leu Thr Leu Asn Phe
                405                 410                 415
Thr Lys Met Gly His Leu Ser Phe Lys Ala Leu Lys Asn Ile Ile Pro
            420                 425                 430
Phe Leu Glu Glu Gly Met Ser Tyr Asp Lys Ala Cys Asp Lys Ala Gly
        435                 440                 445
Tyr Asn Phe Lys Gly Thr Ser Phe Thr Lys Lys Thr Lys Tyr Leu Pro
    450                 455                 460
Val Ile Pro Gln Asn Thr Asn Pro Val Val His Arg Ala Leu Ser Gln
465                 470                 475                 480
Thr Arg Lys Val Ile Asn Ala Ile Ile Asn Lys Tyr Gly Ser Pro Val
                485                 490                 495
Ser Ile His Ile Glu Thr Ala Arg Glu Leu Ser Lys Thr Phe Gln Glu
            500                 505                 510

Arg Lys Glu Ile Asp Ser Met Tyr Gln Asp Asn Ser Lys Lys Asn Glu
        515                 520                 525
His Ala Ile Asn Lys Leu Lys Glu Leu Gly Asn Ile Asn Pro Ser Gly
    530                 535                 540
Ile Asn Ile Val Lys Phe Lys Leu Trp Lys Glu Gln Asp Gly Arg Cys
545                 550                 555                 560
Met Tyr Ser Gly Glu Tyr Ile Glu Pro His Arg Leu Phe Glu Glu Gly
                565                 570                 575
Tyr Thr Glu Val Asp His Ile Leu Pro Tyr Ser Arg Ser Leu Asp Asp
            580                 585                 590
Ser Tyr Asn Asn Lys Thr Leu Ala Leu Gly Ile Glu Asn Gln Arg Lys
        595                 600                 605
Gly Asn Lys Thr Pro Tyr Glu Tyr Met Gly Asn Thr Ser Ile Trp Tyr
    610                 615                 620
Lys Phe Glu Thr Arg Val Gln Ser Asn Lys Lys Ile Asn Lys Lys Lys
625                 630                 635                 640
Gln Gln Lys Leu Leu Leu Gln Tyr Phe Ser His Ala Arg Glu Gln Glu
                645                 650                 655
Phe Ile Glu Arg Asn Leu Asn Asp Thr Arg Tyr Ala Thr Ile Tyr Ile
            660                 665                 670
Ser Ala Phe Ile Gln Gln His Leu Ile Phe Ser Glu Ser Phe Ser Lys
        675                 680                 685
Lys Arg Val His Thr Val Ser Gly Ile Ile Thr Ser His Val Arg Ser
    690                 695                 700
Arg Trp Gly Phe Asn Lys Asp Arg Lys Glu Ser His Ile His His Ala
705                 710                 715                 720
Leu Asp Ala Val Ile Val Ala Val Thr Ser Asp Gln Met Ile Gln Arg
                725                 730                 735
Val Thr Asn Tyr Tyr Lys Leu Lys Glu Leu Asn Arg Lys Leu Gln Ala
            740                 745                 750
Lys Gly Ile Leu Phe Pro Glu Pro Trp Glu Gly Phe Arg Leu Glu Leu
        755                 760                 765
Glu Ala Arg Ile Ser Pro Asn Thr Gln Gln Tyr Leu Lys Gly Ile Cys
    770                 775                 780
Ser Lys Asn Tyr Ala Asp Ile Asn Leu Ser Glu Ile Lys Pro Ile Phe
785                 790                 795                 800
Val Ser Arg Met Pro Lys Arg Ser Ile Thr Gly Glu Leu His Gln Glu
                805                 810                 815
Thr Ile Arg Lys Phe Ile Gly Tyr Asn Glu Lys Gly Lys Val Leu Thr
            820                 825                 830
Ala Ile Lys Thr Lys Leu Glu Asp Ile Pro Phe Asp Ala Asn Gly Asp
        835                 840                 845
Phe Pro Met Tyr Gly Lys Glu Thr Asp Leu Tyr Thr Tyr Asn Ala Ile
    850                 855                 860
Lys Glu Arg Tyr Leu Ser His Lys Lys Asp Lys Arg Lys Ser Phe Gln
865                 870                 875                 880
Glu Ser Leu Tyr Lys Pro Thr Lys Thr Gly Glu Ile Gly Pro Leu Ile
                885                 890                 895
Lys Ser Ile Lys Ile Ile Asp Thr Arg Thr Ile Val Asn Pro Val Asn
            900                 905                 910
Gln Gly Lys Gly Val Val Tyr Asn Ser Lys Ile Ala Arg Thr Asp Val
        915                 920                 925
Phe Lys Lys Asp Asp Lys Tyr Tyr Leu Ile Pro Ile Tyr Thr Ile Asp
    930                 935                 940
Leu Met Lys Asn Val Leu Pro Gln Lys Ala Ile Thr Ala Gly Lys Gly
945                 950                 955                 960
Tyr Glu Asp Trp Ile Thr Ile Asp His Ser Phe Thr Phe Leu Phe Ser
                965                 970                 975
Leu Phe Pro Asn Asp Leu Ile Lys Ile Val Pro Ser Lys Asn Lys Glu
            980                 985                 990
Ile Lys Ala Arg Ser Thr Ser Ser  Lys Lys Glu Ile Leu  Leu Pro Ser
        995                 1000                1005
Leu Thr  Gly Tyr Phe Lys Lys  Val His Ser Gly Thr  Ala Gly Ile

1010                1015                1020
Thr Val  Glu Ser His Asp Gly  Arg Phe Ile Ala Asp  Ile Gly Ser
    1025                1030                1035
Lys Gln  Leu Leu Leu Phe Glu  Lys His Gln Val Asp  Val Leu Gly
    1040                1045                1050
His Tyr  Thr Lys Ile Lys Glu  Glu Lys Arg Ile Gly  Met Ser Thr
    1055                1060                1065
Cys Asn  Asp Asn Lys Lys Ser  Ser Ala Phe Gly Ser  Leu
    1070                1075                1080

<210>  51
<211>  1378
<212>  PRT
<213>  Lactobacillus fermentum

<400>  51
Met Lys Glu Tyr His Ile Gly Leu Asp Ile Gly Thr Ser Ser Ile Gly
1               5                   10                  15
Trp Ala Val Thr Asp Ser Gln Phe Lys Leu Met Arg Ile Lys Gly Lys
            20                  25                  30
Thr Ala Ile Gly Val Arg Leu Phe Glu Glu Gly Lys Thr Ala Ala Glu
        35                  40                  45
Arg Arg Thr Phe Arg Thr Thr Arg Arg Arg Leu Lys Arg Arg Lys Trp
    50                  55                  60
Arg Leu His Tyr Leu Asp Lys Ile Phe Ala Pro His Leu Gln Glu Val
65                  70                  75                  80
Asp Glu Asn Phe Leu Arg Arg Leu Lys Gln Ser Asn Ile His Pro Glu
                85                  90                  95
Asp Pro Ala Lys Asn Gln Ala Phe Ile Gly Lys Leu Leu Phe Pro Asp
            100                 105                 110
Leu Leu Lys Lys Asn Glu Arg Gly Tyr Pro Thr Leu Ile Lys Met Arg
        115                 120                 125
Asp Glu Leu Pro Val Glu Gln Arg Ala His Tyr Pro Val Thr Asn Ile
    130                 135                 140
Tyr Lys Leu Arg Glu Ala Met Ile Asn Glu Asp Arg Gln Phe Asp Leu
145                 150                 155                 160
Arg Glu Val Tyr Leu Ala Val His His Ile Val Lys Tyr Arg Gly His
                165                 170                 175
Phe Leu Asn Asn Ala Ser Val Asp Lys Phe Lys Val Gly Arg Ile Asp
            180                 185                 190
Phe Asp Lys Ser Phe Asn Val Leu Asn Glu Ala Tyr Glu Glu Leu Gln
        195                 200                 205
Asn Gly Glu Gly Ser Phe Thr Ile Glu Pro Ser Lys Val Glu Lys Ile
    210                 215                 220
Gly Gln Leu Leu Leu Asp Thr Lys Met Arg Lys Leu Asp Arg Gln Lys
225                 230                 235                 240
Ala Val Ala Lys Leu Leu Glu Val Lys Val Ala Asp Lys Glu Glu Thr
                245                 250                 255
Lys Arg Asn Lys Gln Ile Ala Thr Ala Met Ser Lys Leu Val Leu Gly
            260                 265                 270
Tyr Lys Ala Asp Phe Ala Thr Val Ala Met Ala Asn Gly Asn Glu Trp
        275                 280                 285
Lys Ile Asp Leu Ser Ser Glu Thr Ser Glu Asp Glu Ile Glu Lys Phe
    290                 295                 300
Arg Glu Glu Leu Ser Asp Ala Gln Asn Asp Ile Leu Thr Glu Ile Thr
305                 310                 315                 320
Ser Leu Phe Ser Gln Ile Met Leu Asn Glu Ile Val Pro Asn Gly Met
                325                 330                 335
Ser Ile Ser Glu Ser Met Met Asp Arg Tyr Trp Met His Glu Arg Gln
            340                 345                 350
Leu Ala Glu Val Lys Glu Tyr Leu Ala Thr Gln Pro Ala Ser Ala Arg
        355                 360                 365
Lys Asp Phe Asp Gln Val Tyr Asn Lys Tyr Ile Gly Gln Ala Pro Lys

370                 375                 380
Glu Lys Gly Phe Asp Leu Glu Lys Gly Leu Lys Lys Ile Leu Ser Lys
385                 390                 395                 400
Lys Glu Asn Trp Lys Glu Ile Asp Glu Leu Leu Lys Ala Gly Asp Phe
                405                 410                 415
Leu Pro Lys Gln Arg Thr Ser Ala Asn Gly Val Ile Pro His Gln Met
            420                 425                 430
His Gln Gln Glu Leu Asp Arg Ile Ile Glu Lys Gln Ala Lys Tyr Tyr
        435                 440                 445
Pro Trp Leu Ala Thr Glu Asn Pro Ala Thr Gly Glu Arg Asp Arg His
    450                 455                 460
Gln Ala Lys Tyr Glu Leu Asp Gln Leu Val Ser Phe Arg Ile Pro Tyr
465                 470                 475                 480
Tyr Val Gly Pro Leu Val Thr Pro Glu Val Gln Lys Ala Thr Ser Gly
                485                 490                 495
Ala Lys Phe Ala Trp Ala Lys Arg Lys Glu Asp Gly Glu Ile Thr Pro
            500                 505                 510
Trp Asn Leu Trp Asp Lys Ile Asp Arg Ala Glu Ser Ala Glu Ala Phe
        515                 520                 525
Ile Lys Arg Met Thr Val Lys Asp Thr Tyr Leu Leu Asn Glu Asp Val
    530                 535                 540
Leu Pro Ala Asn Ser Leu Leu Tyr Gln Lys Tyr Asn Val Leu Asn Glu
545                 550                 555                 560
Leu Asn Asn Val Arg Val Asn Gly Arg Arg Leu Ser Val Gly Ile Lys
                565                 570                 575
Gln Asp Ile Tyr Thr Glu Leu Phe Lys Lys Lys Lys Thr Val Lys Ala
            580                 585                 590
Gly Asp Val Ala Ser Leu Val Met Ala Lys Thr Arg Gly Val Asn Lys
        595                 600                 605
Pro Ser Val Glu Gly Leu Ser Asp Pro Lys Lys Phe Asn Ser Asn Leu
    610                 615                 620
Ala Thr Tyr Leu Asp Leu Lys Ser Ile Val Gly Asp Lys Val Asp Asp
625                 630                 635                 640
Asn Arg Tyr Gln Met Asp Leu Glu Asn Ile Ile Glu Trp Arg Ser Val
                645                 650                 655
Phe Glu Asp Gly Glu Ile Phe Ala Asp Lys Leu Thr Glu Val Glu Trp
            660                 665                 670
Leu Thr Asp Glu Gln Arg Ser Ala Leu Val Lys Lys Arg Tyr Lys Gly
        675                 680                 685
Trp Gly Arg Leu Ser Lys Lys Leu Leu Thr Gly Ile Val Asp Glu Asn
    690                 695                 700
Gly Gln Arg Ile Ile Asp Leu Met Trp Asn Thr Asp Gln Asn Phe Met
705                 710                 715                 720
Gln Ile Val Asn Gln Pro Val Phe Lys Glu Gln Ile Asp Gln Leu Asn
                725                 730                 735
Gln Lys Ala Ile Thr Asn Asp Gly Met Thr Leu Arg Glu Arg Val Glu
            740                 745                 750
Ser Val Leu Asp Asp Ala Tyr Thr Ser Pro Gln Asn Lys Lys Ala Ile
        755                 760                 765
Trp Gln Val Val Arg Val Val Glu Asp Ile Val Lys Ala Val Gly Asn
    770                 775                 780
Ala Pro Lys Ser Ile Ser Ile Glu Phe Ala Arg Asn Glu Gly Asn Lys
785                 790                 795                 800
Gly Glu Ile Thr Arg Ser Arg Arg Thr Gln Leu Gln Lys Leu Phe Glu
                805                 810                 815
Asp Gln Ala His Glu Leu Val Lys Asp Thr Ser Leu Thr Glu Glu Leu
            820                 825                 830
Glu Lys Ala Pro Asp Leu Ser Asp Arg Tyr Tyr Phe Tyr Phe Thr Gln
        835                 840                 845
Gly Gly Lys Asp Met Tyr Thr Gly Asp Pro Ile Asn Phe Asp Glu Ile
    850                 855                 860
Ser Thr Lys Tyr Asp Ile Asp His Ile Leu Pro Gln Ser Phe Val Lys
865                 870                 875                 880

Asp Asp Ser Leu Asp Asn Arg Val Leu Val Ser Arg Ala Glu Asn Asn
                885                 890                 895
Lys Lys Ser Asp Arg Val Pro Ala Lys Leu Tyr Ala Ala Lys Met Lys
            900                 905                 910
Pro Tyr Trp Asn Gln Leu Leu Lys Gln Gly Leu Ile Thr Gln Arg Lys
        915                 920                 925
Phe Glu Asn Leu Thr Met Asp Val Asp Gln Asn Ile Lys Tyr Arg Ser
    930                 935                 940
Leu Gly Phe Val Lys Arg Gln Leu Val Glu Thr Arg Gln Val Ile Lys
945                 950                 955                 960
Leu Thr Ala Asn Ile Leu Gly Ser Met Tyr Gln Glu Ala Gly Thr Asp
                965                 970                 975
Ile Ile Glu Thr Arg Ala Gly Leu Thr Lys Gln Leu Arg Glu Glu Phe
            980                 985                 990
Asp Leu Pro Lys Val Arg Glu Val  Asn Asp Tyr His His  Ala Val Asp
        995                 1000                1005
Ala Tyr  Leu Thr Thr Phe Ala  Gly Gln Tyr Leu Asn  Arg Arg Tyr
    1010                1015                1020
Pro Lys  Leu Arg Ser Phe Phe  Val Tyr Gly Glu Tyr  Met Lys Phe
    1025                1030                1035
Lys His  Gly Ser Asp Leu Lys  Leu Arg Asn Phe Asn  Phe Phe His
    1040                1045                1050
Glu Leu  Met Glu Gly Asp Lys  Ser Gln Gly Lys Val  Val Asp Gln
    1055                1060                1065
Gln Thr  Gly Glu Leu Ile Thr  Thr Arg Asp Glu Val  Ala Lys Ser
    1070                1075                1080
Phe Asp  Arg Leu Leu Asn Met  Lys Tyr Met Leu Val  Ser Lys Glu
    1085                1090                1095
Val His  Asp Arg Ser Asp Gln  Leu Tyr Gly Ala Thr  Ile Val Thr
    1100                1105                1110
Ala Lys  Glu Ser Gly Lys Leu  Thr Ser Pro Ile Glu  Ile Lys Lys
    1115                1120                1125
Asn Arg  Pro Val Asp Leu Tyr  Gly Ala Tyr Thr Asn  Gly Thr Ser
    1130                1135                1140
Ala Phe  Met Thr Ile Ile Lys  Phe Thr Gly Asn Lys  Pro Lys Tyr
    1145                1150                1155
Lys Val  Ile Gly Val Pro Thr  Thr Ser Ala Val Gly  Leu Lys Arg
    1160                1165                1170
Val Gly  Lys Pro Gly Ser Glu  Ser Tyr Asn Gln Glu  Leu His Arg
    1175                1180                1185
Ile Ile  Lys Ser Asn Pro Lys  Val Lys Lys Asp Phe  Glu Ile Val
    1190                1195                1200
Val Pro  His Val Ser Tyr Ser  Gln Leu Ile Val Asp  Gly Asp Cys
    1205                1210                1215
Lys Phe  Thr Leu Ala Ser Asp  Thr Tyr Gln His Pro  Ala Thr Gln
    1220                1225                1230
Leu Val  Leu Ser Lys Glu Ser  Met Glu Ile Ile Ala  Asp Asp Phe
    1235                1240                1245
Lys Phe  Val Lys Glu Asn Pro  Ala Thr Ala Asp Glu  Gln Leu Val
    1250                1255                1260
Arg Val  Phe Asp Glu Ile Val  Asn Gln Met Asn His  Tyr Phe Thr
    1265                1270                1275
Ile Phe  Asp Gln Arg Ser Asn  Arg Gln Lys Val Thr  Lys Ala Arg
    1280                1285                1290
Asp Lys  Phe Val Ser Leu Pro  Thr Glu Ser Glu Tyr  Glu Gly Ala
    1295                1300                1305
Lys Lys  Thr Gln Val Gly Lys  Thr Glu Val Ile Thr  Asn Leu Leu
    1310                1315                1320
Met Gly  Leu His Ala Asn Ala  Ala Gln Gly Asp Leu  Lys Val Leu
    1325                1330                1335
Gly Leu  Ser Thr Phe Gly Phe  Phe Gln Ser Ser Gly  Gly Leu Asn
    1340                1345                1350
Leu Ser  Glu Asp Ala Met Ile  Val Tyr Gln Ser Pro  Thr Gly Leu

1355                    1360                    1365
Phe Glu  Arg Arg Ile Cys Leu  Lys Asp Ile
    1370                    1375

<210>  52
<211>  1337
<212>  PRT
<213>  Enterococcus faecalis

<400>  52
Met Lys Lys Asp Tyr Val Ile Gly Leu Asp Ile Gly Thr Asn Ser Val
1               5                   10                  15
Gly Trp Ala Val Met Thr Glu Asp Tyr Gln Leu Val Lys Lys Lys Met
            20                  25                  30
Pro Ile Tyr Gly Asn Thr Glu Lys Lys Lys Ile Lys Lys Asn Phe Trp
        35                  40                  45
Gly Val Arg Leu Phe Glu Glu Gly His Thr Ala Glu Asp Arg Arg Leu
    50                  55                  60
Lys Arg Thr Ala Arg Arg Arg Ile Ser Arg Arg Arg Asn Arg Leu Arg
65                  70                  75                  80
Tyr Leu Gln Ala Phe Phe Glu Glu Ala Met Thr Ala Leu Asp Glu Asn
                85                  90                  95
Phe Phe Ala Arg Leu Gln Glu Ser Phe Leu Val Pro Glu Asp Lys Lys
            100                 105                 110
Trp His Arg His Pro Ile Phe Ala Lys Leu Glu Asp Glu Val Ala Tyr
        115                 120                 125
His Glu Thr Tyr Pro Thr Ile Tyr His Leu Arg Lys Lys Leu Ala Asp
    130                 135                 140
Ser Ser Glu Gln Ala Asp Leu Arg Leu Ile Tyr Leu Ala Leu Ala Tyr
145                 150                 155                 160
Ile Val Lys Tyr Arg Gly His Phe Leu Ile Glu Gly Lys Leu Ser Thr
                165                 170                 175
Glu Asn Ile Ser Val Lys Glu Lys Phe Gln Gln Phe Met Ile Ile Tyr
            180                 185                 190
Asn Gln Thr Phe Val Asn Gly Glu Gly Arg Leu Val Ser Ala Pro Leu
        195                 200                 205
Pro Glu Ser Val Leu Ile Glu Glu Glu Leu Thr Glu Lys Ala Ser Arg
    210                 215                 220
Thr Lys Lys Ser Glu Lys Val Leu Gln Gln Phe Pro Gln Glu Lys Ala
225                 230                 235                 240
Asn Gly Leu Phe Gly Gln Phe Leu Lys Leu Met Val Gly Asn Lys Ala
                245                 250                 255
Asp Phe Lys Lys Val Phe Gly Leu Glu Glu Glu Ala Lys Ile Thr Tyr
            260                 265                 270
Ala Ser Glu Ser Tyr Glu Glu Asp Leu Glu Gly Ile Leu Ala Lys Val
        275                 280                 285
Gly Asp Glu Tyr Ser Asp Val Phe Leu Ala Ala Lys Asn Val Tyr Asp
    290                 295                 300
Ala Val Glu Leu Ser Thr Ile Leu Ala Asp Ser Asp Lys Lys Ser Tyr
305                 310                 315                 320
Ala Lys Leu Ser Ser Ser Met Ile Val Arg Phe Thr Glu His Gln Glu
                325                 330                 335
Asp Leu Lys Lys Phe Lys Arg Phe Ile Arg Glu Asn Cys Pro Asp Glu
            340                 345                 350
Tyr Asp Asn Leu Phe Lys Asn Glu Gln Lys Asp Gly Tyr Ala Gly Tyr
        355                 360                 365
Ile Ala His Ala Gly Lys Val Ser Gln Leu Lys Phe Tyr Gln Tyr Val
    370                 375                 380
Lys Lys Ile Ile Gln Asp Ile Ala Gly Ala Glu Tyr Phe Leu Glu Lys
385                 390                 395                 400
Ile Ala Gln Glu Asn Phe Leu Arg Lys Gln Arg Thr Phe Asp Asn Gly
                405                 410                 415
Val Ile Pro His Gln Ile His Leu Ala Glu Leu Gln Ala Ile Ile His

420                 425                 430
Arg Gln Ala Ala Tyr Tyr Pro Phe Leu Lys Glu Asn Gln Glu Lys Ile
        435                 440                 445
Glu Gln Leu Val Thr Phe Arg Ile Pro Tyr Tyr Val Gly Pro Leu Ser
    450                 455                 460
Lys Gly Asp Ala Ser Thr Phe Ala Trp Leu Lys Arg Gln Ser Glu Glu
465                 470                 475                 480
Pro Ile Arg Pro Trp Asn Leu Gln Glu Thr Val Asp Leu Asp Gln Ser
                485                 490                 495
Ala Thr Ala Phe Ile Glu Arg Met Thr Asn Phe Asp Thr Tyr Leu Pro
            500                 505                 510
Ser Glu Lys Val Leu Pro Lys His Ser Leu Leu Tyr Glu Lys Phe Met
        515                 520                 525
Val Phe Asn Glu Leu Thr Lys Ile Ser Tyr Thr Asp Asp Arg Gly Ile
    530                 535                 540
Lys Ala Asn Phe Ser Gly Lys Glu Lys Glu Lys Ile Phe Asp Tyr Leu
545                 550                 555                 560
Phe Lys Thr Arg Arg Lys Val Lys Lys Lys Asp Ile Ile Gln Phe Tyr
                565                 570                 575
Arg Asn Glu Tyr Asn Thr Glu Ile Val Thr Leu Ser Gly Leu Glu Glu
            580                 585                 590
Asp Gln Phe Asn Ala Ser Phe Ser Thr Tyr Gln Asp Leu Leu Lys Cys
        595                 600                 605
Gly Leu Thr Arg Ala Glu Leu Asp His Pro Asp Asn Ala Glu Lys Leu
    610                 615                 620
Glu Asp Ile Ile Lys Ile Leu Thr Ile Phe Glu Asp Arg Gln Arg Ile
625                 630                 635                 640
Arg Thr Gln Leu Ser Thr Phe Lys Gly Gln Phe Ser Ala Glu Val Leu
                645                 650                 655
Lys Lys Leu Glu Arg Lys His Tyr Thr Gly Trp Gly Arg Leu Ser Lys
            660                 665                 670
Lys Leu Ile Asn Gly Ile Tyr Asp Lys Glu Ser Gly Lys Thr Ile Leu
        675                 680                 685
Asp Tyr Leu Val Lys Asp Asp Gly Val Ser Lys His Tyr Asn Arg Asn
    690                 695                 700
Phe Met Gln Leu Ile Asn Asp Ser Gln Leu Ser Phe Lys Asn Ala Ile
705                 710                 715                 720
Gln Lys Ala Gln Ser Ser Glu His Glu Glu Thr Leu Ser Glu Thr Val
                725                 730                 735
Asn Glu Leu Ala Gly Ser Pro Ala Ile Lys Lys Gly Ile Tyr Gln Ser
            740                 745                 750
Leu Lys Ile Val Asp Glu Leu Val Ala Ile Met Gly Tyr Ala Pro Lys
        755                 760                 765
Arg Ile Val Val Glu Met Ala Arg Glu Asn Gln Thr Thr Ser Thr Gly
    770                 775                 780
Lys Arg Arg Ser Ile Gln Arg Leu Lys Ile Val Glu Lys Ala Met Ala
785                 790                 795                 800
Glu Ile Gly Ser Asn Leu Leu Lys Glu Gln Pro Thr Thr Asn Glu Gln
                805                 810                 815
Leu Arg Asp Thr Arg Leu Phe Leu Tyr Tyr Met Gln Asn Gly Lys Asp
            820                 825                 830
Met Tyr Thr Gly Asp Glu Leu Ser Leu His Arg Leu Ser His Tyr Asp
        835                 840                 845
Ile Asp His Ile Ile Pro Gln Ser Phe Met Lys Asp Asp Ser Leu Asp
    850                 855                 860
Asn Leu Val Leu Val Gly Ser Thr Glu Asn Arg Gly Lys Ser Asp Asp
865                 870                 875                 880
Val Pro Ser Lys Glu Val Val Lys Asp Met Lys Ala Tyr Trp Glu Lys
                885                 890                 895
Leu Tyr Ala Ala Gly Leu Ile Ser Gln Arg Lys Phe Gln Arg Leu Thr
            900                 905                 910
Lys Gly Glu Gln Gly Gly Leu Thr Leu Glu Asp Lys Ala His Phe Ile
        915                 920                 925

Gln Arg Gln Leu Val Glu Thr Arg Gln Ile Thr Lys Asn Val Ala Gly
930                 935                 940
Ile Leu Asp Gln Arg Tyr Asn Ala Lys Ser Lys Glu Lys Lys Val Gln
945                 950                 955                 960
Ile Ile Thr Leu Lys Ala Ser Leu Thr Ser Gln Phe Arg Ser Ile Phe
                965                 970                 975
Gly Leu Tyr Lys Val Arg Glu Val Asn Asp Tyr His His Gly Gln Asp
            980                 985                 990
Ala Tyr Leu Asn Cys Val Val Ala  Thr Thr Leu Leu Lys  Val Tyr Pro
        995                 1000                1005
Asn Leu  Ala Pro Glu Phe Val  Tyr Gly Glu Tyr Pro  Lys Phe Gln
    1010                1015                1020
Thr Phe  Lys Glu Asn Lys Ala  Thr Ala Lys Ala Ile  Ile Tyr Thr
    1025                1030                1035
Asn Leu  Leu Arg Phe Phe Thr  Glu Asp Glu Pro Arg  Phe Thr Lys
    1040                1045                1050
Asp Gly  Glu Ile Leu Trp Ser  Asn Ser Tyr Leu Lys  Thr Ile Lys
    1055                1060                1065
Lys Glu  Leu Asn Tyr His Gln  Met Asn Ile Val Lys  Lys Val Glu
    1070                1075                1080
Val Gln  Lys Gly Gly Phe Ser  Lys Glu Ser Ile Lys  Pro Lys Gly
    1085                1090                1095
Pro Ser  Asn Lys Leu Ile Pro  Val Lys Asn Gly Leu  Asp Pro Gln
    1100                1105                1110
Lys Tyr  Gly Gly Phe Asp Ser  Pro Ile Val Ala Tyr  Thr Val Leu
    1115                1120                1125
Phe Thr  His Glu Lys Gly Lys  Lys Pro Leu Ile Lys  Gln Glu Ile
    1130                1135                1140
Leu Gly  Ile Thr Ile Met Glu  Lys Thr Arg Phe Glu  Gln Asn Pro
    1145                1150                1155
Ile Leu  Phe Leu Glu Glu Lys  Gly Phe Leu Arg Pro  Arg Val Leu
    1160                1165                1170
Met Lys  Leu Pro Lys Tyr Thr  Leu Tyr Glu Phe Pro  Glu Gly Arg
    1175                1180                1185
Arg Arg  Leu Leu Ala Ser Ala  Lys Glu Ala Gln Lys  Gly Asn Gln
    1190                1195                1200
Met Val  Leu Pro Glu His Leu  Leu Thr Leu Leu Tyr  His Ala Lys
    1205                1210                1215
Gln Cys  Leu Leu Pro Asn Gln  Ser Glu Ser Leu Ala  Tyr Val Glu
    1220                1225                1230
Gln His  Gln Pro Glu Phe Gln  Glu Ile Leu Glu Arg  Val Val Asp
    1235                1240                1245
Phe Ala  Glu Val His Thr Leu  Ala Lys Ser Lys Val  Gln Gln Ile
    1250                1255                1260
Val Lys  Leu Phe Glu Ala Asn  Gln Thr Ala Asp Val  Lys Glu Ile
    1265                1270                1275
Ala Ala  Ser Phe Ile Gln Leu  Met Gln Phe Asn Ala  Met Gly Ala
    1280                1285                1290
Pro Ser  Thr Phe Lys Phe Phe  Gln Lys Asp Ile Glu  Arg Ala Arg
    1295                1300                1305
Tyr Thr  Ser Ile Lys Glu Ile  Phe Asp Ala Thr Ile  Ile Tyr Gln
    1310                1315                1320
Ser Thr  Thr Gly Leu Tyr Glu  Thr Arg Arg Lys Val  Val Asp
    1325                1330                1335

<210>  53
<211>  1337
<212>  PRT
<213>  Bacillus cereus

<400>  53
Met Lys Lys Asp Tyr Val Ile Gly Leu Asp Ile Gly Thr Asn Ser Val
1               5                   10                  15

Gly Trp Ala Val Met Thr Glu Asp Tyr Gln Leu Val Lys Lys Lys Met
                20                  25                  30
Pro Ile Tyr Gly Asn Thr Glu Lys Lys Lys Ile Lys Lys Asn Phe Trp
            35                  40                  45
Gly Val Arg Leu Phe Glu Glu Gly His Thr Ala Glu Asp Arg Arg Leu
    50                  55                  60
Lys Arg Thr Ala Arg Arg Arg Ile Ser Arg Arg Arg Asn Arg Leu Arg
65                  70                  75                  80
Tyr Leu Gln Ala Phe Phe Glu Glu Ala Met Thr Asp Leu Asp Glu Asn
                85                  90                  95
Phe Phe Ala Arg Leu His Glu Ser Phe Leu Val Pro Glu Asp Lys Lys
            100                 105                 110
Trp His Arg His Pro Ile Phe Ala Lys Leu Glu Asp Glu Val Ala Tyr
        115                 120                 125
His Glu Thr Tyr Pro Thr Ile Tyr His Leu Arg Lys Lys Leu Ala Asp
    130                 135                 140
Ser Ser Glu Gln Ala Asp Leu Arg Leu Ile Tyr Leu Ala Leu Ala His
145                 150                 155                 160
Ile Val Lys Tyr Arg Gly His Phe Leu Ile Glu Gly Lys Leu Ser Thr
                165                 170                 175
Glu Asn Thr Ser Val Lys Glu Gln Phe Gln Gln Phe Met Val Ile Tyr
            180                 185                 190
Asn Gln Thr Phe Val Asn Gly Glu Ser Arg Leu Val Ser Ala Pro Leu
        195                 200                 205
Pro Glu Ser Val Leu Ile Glu Glu Glu Leu Thr Glu Lys Ala Ser Arg
    210                 215                 220
Thr Lys Lys Ser Glu Lys Val Leu Gln Gln Phe Pro Gln Glu Lys Ala
225                 230                 235                 240
Asn Gly Leu Phe Gly Gln Phe Leu Lys Leu Met Val Gly Asn Lys Ala
                245                 250                 255
Asp Phe Lys Lys Val Phe Ala Leu Glu Glu Glu Ala Lys Ile Thr Tyr
            260                 265                 270
Ala Ser Glu Ser Tyr Glu Glu Asp Leu Glu Gly Ile Leu Ala Lys Ile
        275                 280                 285
Gly Asp Glu Tyr Ser Asp Val Phe Leu Ala Ala Lys Asn Val Tyr Asp
    290                 295                 300
Ala Val Glu Leu Ser Thr Ile Leu Ala Asp Ser Asp Lys Lys Ser His
305                 310                 315                 320
Ala Lys Leu Ser Ser Ser Met Ile Val Arg Phe Thr Glu His Gln Glu
                325                 330                 335
Asp Leu Lys Lys Phe Lys Arg Phe Ile Arg Glu Asn Cys Pro Asp Glu
            340                 345                 350
Tyr Asp Asn Leu Phe Lys Asn Glu Gln Lys Asp Gly Tyr Ala Gly Tyr
        355                 360                 365
Ile Ala His Ala Gly Lys Val Ser Gln Leu Lys Phe Tyr Gln Tyr Val
    370                 375                 380
Lys Lys Ile Ile Gln Asp Ile Ala Gly Ala Glu Tyr Phe Leu Glu Lys
385                 390                 395                 400
Ile Ala Gln Glu Asn Phe Leu Arg Lys Gln Arg Thr Phe Asp Asn Gly
                405                 410                 415
Val Ile Pro His Gln Ile His Leu Ala Glu Leu Gln Ala Ile Ile His
            420                 425                 430
Arg Gln Ala Ala Tyr Tyr Pro Phe Leu Lys Glu Asn Gln Glu Lys Ile
        435                 440                 445
Glu Gln Leu Val Thr Phe Arg Ile Pro Tyr Tyr Val Gly Pro Leu Ser
    450                 455                 460
Lys Gly Asp Ala Ser Thr Phe Ala Trp Leu Lys Arg Gln Asn Glu Lys
465                 470                 475                 480
Pro Ile Arg Pro Trp Asn Leu Gln Glu Thr Val Asp Leu Asp Gln Ser
                485                 490                 495
Ala Thr Ala Phe Ile Glu Arg Met Thr Asn Phe Asp Thr Tyr Leu Pro
            500                 505                 510
Ser Glu Lys Val Leu Pro Lys His Ser Leu Leu Tyr Glu Lys Phe Met

        515                 520                 525
Val Phe Asn Glu Leu Thr Lys Ile Ser Tyr Thr Asp Asp Arg Gly Ile
    530                 535                 540
Lys Ala Asn Phe Ser Gly Lys Glu Lys Glu Lys Ile Phe Asp Tyr Leu
545                 550                 555                 560
Phe Lys Thr Arg Arg Lys Val Lys Lys Lys Asp Ile Ile Gln Phe Tyr
                565                 570                 575
Arg Asn Glu Tyr Asn Thr Glu Ile Val Thr Leu Ser Gly Leu Glu Glu
            580                 585                 590
Asp Gln Phe Asn Ala Ser Phe Ser Thr Tyr Gln Asp Leu Leu Lys Cys
        595                 600                 605
Gly Leu Thr Arg Ala Glu Leu Asp His Pro Asp Asn Ala Glu Lys Leu
    610                 615                 620
Glu Asp Ile Ile Lys Ile Leu Thr Ile Phe Glu Asp Arg Gln Arg Ile
625                 630                 635                 640
Arg Thr Gln Leu Ser Thr Phe Lys Gly Gln Phe Ser Ala Glu Val Leu
                645                 650                 655
Lys Lys Leu Glu Arg Lys His Tyr Thr Gly Trp Gly Arg Leu Ser Lys
            660                 665                 670
Lys Leu Ile Asn Gly Ile Tyr Asp Lys Glu Ser Gly Lys Thr Ile Leu
        675                 680                 685
Asp Tyr Leu Val Lys Asp Asp Gly Val Ser Lys His Tyr Asn Arg Asn
    690                 695                 700
Phe Met Gln Leu Ile Asn Asp Ser Gln Leu Ser Phe Lys Asn Ala Ile
705                 710                 715                 720
Gln Lys Ala Gln Ser Ser Glu His Glu Glu Thr Leu Ser Glu Thr Val
                725                 730                 735
Asn Glu Leu Ala Gly Ser Pro Ala Ile Lys Lys Gly Ile Tyr Gln Ser
            740                 745                 750
Leu Lys Ile Val Asp Glu Leu Val Ala Ile Met Gly Tyr Ala Pro Lys
        755                 760                 765
Arg Ile Val Val Glu Met Ala Arg Glu Asn Gln Thr Thr Ser Thr Gly
    770                 775                 780
Lys Arg Arg Ser Ile Gln Arg Leu Lys Ile Val Glu Lys Ala Met Ala
785                 790                 795                 800
Glu Ile Gly Ser Asn Leu Leu Lys Glu Gln Pro Thr Thr Asn Glu Gln
                805                 810                 815
Leu Arg Asp Thr Arg Leu Phe Leu Tyr Tyr Met Gln Asn Gly Lys Asp
            820                 825                 830
Met Tyr Thr Gly Asp Glu Leu Ser Leu His Arg Leu Ser His Tyr Asp
        835                 840                 845
Ile Asp His Ile Ile Pro Gln Ser Phe Met Lys Asp Asp Ser Leu Asp
    850                 855                 860
Asn Leu Val Leu Val Gly Ser Thr Glu Asn Arg Gly Lys Ser Asp Asp
865                 870                 875                 880
Val Pro Ser Lys Glu Val Val Lys Asp Met Lys Ala Tyr Trp Glu Lys
                885                 890                 895
Leu Tyr Ala Ala Gly Leu Ile Ser Gln Arg Lys Phe Gln Arg Leu Thr
            900                 905                 910
Lys Gly Glu Gln Gly Gly Leu Thr Leu Glu Asp Lys Ala His Phe Ile
        915                 920                 925
Gln Arg Gln Leu Val Glu Thr Arg Gln Ile Thr Lys Asn Val Ala Gly
    930                 935                 940
Ile Leu Asp Gln Arg Tyr Asn Ala Lys Ser Lys Glu Lys Lys Val Gln
945                 950                 955                 960
Ile Ile Thr Leu Lys Ala Ser Leu Thr Ser Gln Phe Arg Ser Ile Phe
                965                 970                 975
Gly Leu Tyr Lys Val Arg Glu Val Asn Asp Tyr His His Gly Gln Asp
            980                 985                 990
Ala Tyr Leu Asn Cys Val Val Ala Thr Thr Leu Leu Lys Val Cys Pro
        995                 1000                1005
Asn Leu Ala Pro Glu Phe Val Tyr Gly Glu Tyr Pro Lys Phe Gln
    1010                1015                1020

Thr Phe Lys Glu Asn Lys Ala Thr Ala Lys Ala Ile Ile Tyr Thr
    1025                1030                1035
Asn Leu Leu Arg Phe Phe Thr Glu Asp Glu Pro Arg Phe Thr Lys
    1040                1045                1050
Asp Gly Glu Ile Leu Trp Ser Asn Ser Tyr Leu Lys Thr Ile Lys
    1055                1060                1065
Lys Glu Leu Asn Tyr His Gln Met Asn Ile Val Lys Lys Val Glu
    1070                1075                1080
Val Gln Lys Gly Gly Phe Ser Lys Glu Ser Ile Lys Pro Lys Gly
    1085                1090                1095
Pro Ser Asn Lys Leu Ile Pro Val Lys Asn Gly Leu Asp Pro Gln
    1100                1105                1110
Lys Tyr Gly Gly Phe Asp Ser Pro Val Val Ala Tyr Thr Val Leu
    1115                1120                1125
Phe Thr His Glu Lys Gly Lys Lys Pro Leu Ile Lys Gln Glu Ile
    1130                1135                1140
Leu Gly Ile Thr Ile Met Glu Lys Thr Arg Phe Glu Gln Asn Pro
    1145                1150                1155
Ile Leu Phe Leu Glu Glu Lys Gly Phe Leu Arg Pro Arg Val Leu
    1160                1165                1170
Met Lys Leu Pro Lys Tyr Thr Leu Tyr Glu Phe Pro Glu Gly Arg
    1175                1180                1185
Arg Arg Leu Leu Ala Ser Ala Lys Glu Ala Gln Lys Gly Asn Gln
    1190                1195                1200
Met Val Leu Pro Glu His Leu Leu Thr Leu Leu Tyr His Ala Lys
    1205                1210                1215
Gln Cys Leu Leu Pro Asn Gln Ser Glu Ser Leu Ala Tyr Val Glu
    1220                1225                1230
Gln His Gln Pro Glu Phe Gln Glu Ile Leu Glu Arg Val Val Asp
    1235                1240                1245
Phe Ala Glu Val His Thr Leu Ala Lys Ser Lys Val Gln Gln Ile
    1250                1255                1260
Val Lys Leu Phe Glu Ala Asn Gln Thr Ala Asp Val Lys Glu Ile
    1265                1270                1275
Ala Ala Ser Phe Ile Gln Leu Met Gln Phe Asn Ala Met Gly Ala
    1280                1285                1290
Pro Ser Thr Phe Lys Phe Phe Gln Lys Asp Ile Glu Arg Ala Arg
    1295                1300                1305
Tyr Thr Ser Ile Lys Glu Ile Phe Asp Ala Thr Ile Ile Tyr Gln
    1310                1315                1320
Ser Thr Thr Gly Leu Tyr Glu Thr Arg Arg Lys Val Val Asp
    1325                1330                1335

<210> 54
<211> 1337
<212> PRT
<213> Enterococcus faecalis

<400> 54
Met Lys Lys Asp Tyr Val Ile Gly Leu Asp Ile Gly Thr Asn Ser Val
1               5                   10                  15
Gly Trp Ala Val Met Thr Glu Asp Tyr Gln Leu Val Lys Lys Lys Met
            20                  25                  30
Pro Ile Tyr Gly Asn Thr Glu Lys Lys Lys Ile Lys Lys Asn Phe Trp
        35                  40                  45
Gly Val Arg Leu Phe Glu Glu Gly His Thr Ala Glu Asp Arg Arg Leu
    50                  55                  60
Lys Arg Thr Ala Arg Arg Arg Ile Ser Arg Arg Arg Asn Arg Leu Arg
65                  70                  75                  80
Tyr Leu Gln Ala Phe Phe Glu Glu Ala Met Thr Asp Leu Asp Glu Asn
                85                  90                  95
Phe Phe Ala Arg Leu Gln Glu Ser Phe Leu Val Pro Glu Asp Lys Lys
            100                 105                 110

Trp His Arg His Pro Ile Phe Ala Lys Leu Glu Asp Glu Val Ala Tyr
        115                 120                 125
His Glu Thr Tyr Pro Thr Ile Tyr His Leu Arg Lys Lys Leu Ala Asp
    130                 135                 140
Ser Ser Glu Gln Ala Asp Leu Arg Leu Ile Tyr Leu Ala Leu Ala His
145                 150                 155                 160
Ile Val Lys Tyr Arg Gly His Phe Leu Ile Glu Gly Lys Leu Ser Thr
                165                 170                 175
Glu Asn Thr Ser Val Lys Glu Gln Phe Gln Gln Phe Met Val Ile Tyr
            180                 185                 190
Asn Gln Thr Phe Val Asn Gly Glu Ser Arg Leu Val Ser Ala Pro Leu
        195                 200                 205
Pro Glu Ser Val Leu Ile Glu Glu Glu Leu Thr Glu Lys Ala Ser Arg
    210                 215                 220
Thr Lys Lys Ser Glu Lys Val Leu Gln Gln Phe Pro Gln Glu Lys Ala
225                 230                 235                 240
Asn Gly Leu Phe Gly Gln Phe Leu Lys Leu Met Val Gly Asn Lys Ala
                245                 250                 255
Asp Phe Lys Lys Val Phe Gly Leu Glu Glu Glu Ala Lys Ile Thr Tyr
            260                 265                 270
Ala Ser Glu Ser Tyr Glu Glu Asp Leu Glu Gly Ile Leu Ala Lys Val
        275                 280                 285
Gly Asp Glu Tyr Ser Asp Val Phe Leu Ala Ala Lys Asn Val Tyr Asp
    290                 295                 300
Ala Val Glu Leu Ser Thr Ile Leu Ala Asp Ser Asp Lys Lys Ser His
305                 310                 315                 320
Ala Lys Leu Ser Ser Ser Met Ile Val Arg Phe Thr Glu His Gln Glu
                325                 330                 335
Asp Leu Lys Lys Phe Lys Arg Phe Ile Arg Glu Asn Cys Pro Asp Glu
            340                 345                 350
Tyr Asp Asn Leu Phe Lys Asn Glu Gln Lys Asp Gly Tyr Ala Gly Tyr
        355                 360                 365
Ile Ala His Ala Gly Lys Val Ser Gln Leu Lys Phe Tyr Gln Tyr Val
    370                 375                 380
Lys Lys Ile Ile Gln Asp Ile Ala Gly Ala Glu Tyr Phe Leu Glu Lys
385                 390                 395                 400
Ile Ala Gln Glu Asn Phe Leu Arg Lys Gln Arg Thr Phe Asp Asn Gly
                405                 410                 415
Val Ile Pro His Gln Ile His Leu Ala Glu Leu Gln Ala Ile Ile His
            420                 425                 430
Arg Gln Ala Ala Tyr Tyr Pro Phe Leu Lys Glu Asn Gln Glu Lys Ile
        435                 440                 445
Glu Gln Leu Val Thr Phe Arg Ile Pro Tyr Tyr Val Gly Pro Leu Ser
    450                 455                 460
Lys Gly Asp Ala Ser Thr Phe Ala Trp Leu Lys Arg Gln Ser Glu Glu
465                 470                 475                 480
Pro Ile Arg Pro Trp Asn Leu Gln Glu Thr Val Asp Leu Asp Gln Ser
                485                 490                 495
Ala Thr Ala Phe Ile Glu Arg Met Thr Asn Phe Asp Thr Tyr Leu Pro
            500                 505                 510
Ser Glu Lys Val Leu Pro Lys His Ser Leu Leu Tyr Glu Lys Phe Met
        515                 520                 525
Val Phe Asn Glu Leu Thr Lys Ile Ser Tyr Thr Asp Asp Arg Gly Ile
    530                 535                 540
Lys Ala Asn Phe Ser Gly Lys Glu Lys Glu Lys Ile Phe Asp Tyr Leu
545                 550                 555                 560
Phe Lys Thr Arg Arg Lys Val Lys Lys Lys Asp Ile Ile Gln Phe Tyr
                565                 570                 575
Arg Asn Glu Tyr Asn Thr Glu Ile Val Thr Leu Ser Gly Leu Glu Glu
            580                 585                 590
Asp Gln Phe Asn Ala Ser Phe Ser Thr Tyr Gln Asp Leu Leu Lys Cys
        595                 600                 605
Gly Leu Thr Arg Ala Glu Leu Asp His Pro Asp Asn Ala Glu Lys Leu

610                 615                 620
Glu Asp Ile Ile Lys Ile Leu Thr Ile Phe Glu Asp Arg Gln Arg Ile
625                 630                 635                 640
Arg Thr Gln Leu Ser Thr Phe Lys Gly Gln Phe Ser Ala Glu Val Leu
                645                 650                 655
Lys Lys Leu Glu Arg Lys His Tyr Thr Gly Trp Gly Arg Leu Ser Lys
            660                 665                 670
Lys Leu Ile Asn Gly Ile Tyr Asp Lys Glu Ser Gly Lys Thr Ile Leu
        675                 680                 685
Asp Tyr Leu Val Lys Asp Asp Gly Val Ser Lys His Tyr Asn Arg Asn
    690                 695                 700
Phe Met Gln Leu Ile Asn Asp Ser Gln Leu Ser Phe Lys Asn Ala Ile
705                 710                 715                 720
Gln Lys Ala Gln Ser Ser Glu His Glu Glu Thr Leu Ser Glu Thr Val
                725                 730                 735
Asn Glu Leu Ala Gly Ser Pro Ala Ile Lys Lys Gly Ile Tyr Gln Ser
            740                 745                 750
Leu Lys Ile Val Asp Glu Leu Val Ala Ile Met Gly Tyr Ala Pro Lys
        755                 760                 765
Arg Ile Val Val Glu Met Ala Arg Glu Asn Gln Thr Thr Ser Thr Gly
    770                 775                 780
Lys Arg Arg Ser Ile Gln Arg Leu Lys Ile Val Glu Lys Ala Met Ala
785                 790                 795                 800
Glu Ile Gly Ser Asn Leu Leu Lys Glu Gln Pro Thr Thr Asn Glu Gln
                805                 810                 815
Leu Arg Asp Thr Arg Leu Phe Leu Tyr Tyr Met Gln Asn Gly Lys Asp
            820                 825                 830
Met Tyr Thr Gly Asp Glu Leu Ser Leu His Arg Leu Ser His Tyr Asp
        835                 840                 845
Ile Asp His Ile Ile Pro Gln Ser Phe Met Lys Asp Asp Ser Leu Asp
    850                 855                 860
Asn Leu Val Leu Val Gly Ser Thr Glu Asn Arg Gly Lys Ser Asp Asp
865                 870                 875                 880
Val Pro Ser Lys Lys Val Val Lys Lys Met Lys Ala Tyr Trp Glu Lys
                885                 890                 895
Leu Tyr Ala Ala Gly Leu Ile Ser Gln Arg Lys Phe Gln Arg Leu Thr
            900                 905                 910
Lys Gly Glu Gln Gly Gly Leu Thr Leu Glu Asp Lys Ala His Phe Ile
        915                 920                 925
Gln Arg Gln Leu Val Glu Thr Arg Gln Ile Thr Lys Asn Val Ala Gly
    930                 935                 940
Ile Leu Asp Gln Arg Tyr Asn Ala Lys Ser Lys Glu Lys Lys Val Gln
945                 950                 955                 960
Ile Ile Thr Leu Lys Ala Ser Leu Thr Ser Gln Phe Arg Ser Ile Phe
                965                 970                 975
Gly Leu Tyr Lys Val Arg Glu Val Asn Asp Tyr His His Gly Gln Asp
            980                 985                 990
Ala Tyr Leu Asn Cys Val Val Ala  Thr Thr Leu Leu Lys  Val Tyr Pro
        995                 1000                1005
Asn Leu  Ala Pro Glu Phe Val  Tyr Gly Glu Tyr Pro  Lys Phe Gln
    1010                1015                1020
Ala Phe  Lys Glu Asn Lys Ala  Thr Ala Lys Thr Ile  Ile Tyr Thr
    1025                1030                1035
Asn Leu  Met Arg Phe Phe Thr  Glu Asp Glu Pro Arg  Phe Thr Lys
    1040                1045                1050
Asp Ser  Glu Ile Leu Trp Ser  Asn Ser Tyr Leu Lys  Thr Ile Lys
    1055                1060                1065
Lys Glu  Leu Asn Tyr His Gln  Met Asn Ile Val Lys  Lys Val Glu
    1070                1075                1080
Val Gln  Lys Gly Gly Phe Ser  Lys Glu Ser Ile Lys  Pro Lys Gly
    1085                1090                1095
Pro Ser  Asn Lys Leu Ile Pro  Val Lys Asn Gly Leu  Asp Pro Gln
    1100                1105                1110

Lys Tyr Gly Gly Phe Asp Ser Pro Val Val Ala Tyr Thr Val Leu
    1115                1120                1125
Phe Thr His Glu Lys Gly Lys Lys Pro Leu Ile Lys Gln Glu Ile
    1130                1135                1140
Leu Gly Ile Thr Ile Met Glu Lys Thr Arg Phe Glu Gln Asn Pro
    1145                1150                1155
Ile Leu Phe Leu Glu Glu Lys Gly Phe Leu Arg Pro Arg Val Leu
    1160                1165                1170
Met Lys Leu Pro Lys Tyr Thr Leu Tyr Glu Phe Pro Glu Gly Arg
    1175                1180                1185
Arg Arg Leu Leu Ala Ser Ala Lys Glu Ala Gln Lys Gly Asn Gln
    1190                1195                1200
Met Val Leu Pro Glu His Leu Leu Thr Leu Leu Tyr His Ala Lys
    1205                1210                1215
Gln Cys Leu Leu Pro Asn Gln Ser Glu Ser Leu Ala Tyr Val Glu
    1220                1225                1230
Gln His Gln Pro Glu Phe Gln Glu Ile Leu Glu Arg Val Val Asp
    1235                1240                1245
Phe Ala Glu Val His Thr Leu Ala Lys Ser Lys Val Gln Gln Ile
    1250                1255                1260
Val Lys Leu Phe Glu Ala Asn Gln Thr Ala Asp Val Lys Glu Ile
    1265                1270                1275
Ala Ala Ser Phe Ile Gln Leu Met Gln Phe Asn Ala Met Gly Ala
    1280                1285                1290
Pro Ser Thr Phe Lys Phe Phe Gln Lys Asp Ile Glu Arg Ala Arg
    1295                1300                1305
Tyr Thr Ser Ile Lys Glu Ile Phe Asp Ala Thr Ile Ile Tyr Gln
    1310                1315                1320
Ser Thr Thr Gly Leu Tyr Glu Thr Arg Arg Lys Val Val Asp
    1325                1330                1335

<210> 55
<211> 1330
<212> PRT
<213> Artificial sequence

<220>
<223> Cas-Locus 14

<400> 55
Met Glu Lys Glu Tyr Thr Ile Gly Leu Asp Ile Gly Thr Asn Ser Val
1               5                   10                  15
Gly Trp Ala Val Leu Thr Asp Asp Tyr Arg Leu Val Ala Arg Lys Met
            20                  25                  30
Ser Ile Gln Gly Asp Ser Asn Arg Lys Lys Ile Lys Lys Asn Phe Trp
        35                  40                  45
Gly Ala Arg Leu Phe Glu Glu Gly Lys Thr Ala Gln Phe Arg Arg Ile
    50                  55                  60
Lys Arg Thr Asn Arg Arg Arg Ile Ala Arg Arg Arg Gln Arg Val Leu
65                  70                  75                  80
Ala Leu Gln Asp Ile Phe Ala Glu Glu Ile His Lys Lys Asp Pro Asn
                85                  90                  95
Phe Phe Ala Arg Leu Glu Glu Gly Asp Arg Val Glu Ala Asp Lys Arg
            100                 105                 110
Phe Ala Lys Phe Pro Val Phe Ala Thr Leu Ser Glu Glu Lys Asn Tyr
        115                 120                 125
His Arg Gln Tyr Pro Thr Ile Tyr His Leu Arg His Asp Leu Ala Asn
    130                 135                 140
Ser Lys Glu Gln Ala Asp Ile Arg Leu Val Tyr Leu Ala Ile Ala His
145                 150                 155                 160
Cys Leu Lys Tyr Arg Gly His Phe Leu Phe Glu Gly Glu Leu Asp Thr
                165                 170                 175
Glu Asn Thr Ser Val Thr Glu Asn Tyr Gln Gln Phe Leu Gln Ala Tyr

            180                 185                 190
Gln Gln Phe Phe Pro Glu Pro Ile Gly Asp Leu Asp Asp Ala Val Pro
        195                 200                 205
Ile Leu Thr Glu Arg Leu Ser Lys Ala Lys Arg Val Glu Lys Val Leu
    210                 215                 220
Ala Tyr Tyr Pro Ser Glu Lys Ser Thr Gly Asn Phe Ala Gln Phe Leu
225                 230                 235                 240
Lys Leu Met Val Gly Asn Gln Ala Asn Phe Lys Lys Thr Phe Asp Leu
                245                 250                 255
Glu Glu Glu Met Lys Leu Asn Phe Thr Arg Asp Cys Tyr Glu Glu Asp
            260                 265                 270
Leu Asn Glu Leu Leu Glu Lys Thr Ser Asp Asp Tyr Ala Glu Leu Phe
        275                 280                 285
Leu Lys Ala Lys Gly Val Tyr Asp Ala Ile Leu Leu Ser Gln Ile Leu
    290                 295                 300
Ser Lys Ser Asp Asp Glu Thr Lys Ala Lys Leu Ser Ala Asn Met Lys
305                 310                 315                 320
Leu Arg Phe Glu Glu His Gln Arg Asp Leu Lys Gln Leu Lys Glu Leu
                325                 330                 335
Val Arg Arg Asp Leu Pro Lys Lys Tyr Asp Asp Phe Phe Lys Asn Arg
            340                 345                 350
Ser Lys Asn Gly Tyr Ala Gly Tyr Val Lys Gly Lys Ala Thr Gln Glu
        355                 360                 365
Asp Phe Tyr Lys Phe Leu Arg Thr Glu Leu Ala Gly Leu Glu Glu Ser
    370                 375                 380
Gln Ser Ile Met Glu Lys Ile Asp Leu Glu Ile Tyr Leu Leu Lys Gln
385                 390                 395                 400
Arg Thr Phe Ala Asn Gly Val Ile Pro His Gln Ile His Leu Val Glu
                405                 410                 415
Met Arg Glu Ile Met Asp Arg Gln Lys Arg Phe Tyr Pro Phe Leu Lys
            420                 425                 430
Gly Ala Gln Gly Lys Ile Glu Lys Leu Leu Thr Phe Arg Ile Pro Tyr
        435                 440                 445
Tyr Val Gly Pro Leu Ala Gln Glu Gly Gln Ser Pro Phe Ala Trp Ile
    450                 455                 460
Lys Arg Lys Ser Pro Ser Gln Ile Thr Pro Trp Asn Phe Ala Glu Val
465                 470                 475                 480
Val Asp Lys Glu Asn Ser Ala Ile Glu Phe Ile Glu Arg Met Thr Asn
                485                 490                 495
Gln Asp Thr Tyr Leu Pro Lys Glu Lys Val Leu Pro Lys Gln Ser Leu
            500                 505                 510
Ile Tyr Gln Arg Phe Met Ile Phe Asn Glu Leu Thr Lys Val Ser Tyr
        515                 520                 525
Thr Asp Glu Arg Gly Lys Ser His Tyr Phe Ser Ser Glu Gln Lys Arg
    530                 535                 540
Lys Ile Phe Asn Glu Leu Phe Lys Gln His Pro Arg Val Thr Glu Lys
545                 550                 555                 560
Gln Leu Arg Lys Phe Leu Glu Leu Asn Glu Gln Ile Asp Ser Thr Glu
                565                 570                 575
Ile Lys Gly Ile Glu Thr Ser Phe Asn Ala Ser Tyr Ser Thr Tyr His
            580                 585                 590
Asp Leu Leu Lys Leu Ser Asp Gln Met Asp Thr Leu Leu Asp Asp Pro
        595                 600                 605
Asp Met Thr Thr Met Phe Glu Glu Ile Ile Lys Ile Leu Thr Ile Phe
    610                 615                 620
Glu Asp Arg Glu Met Ile Arg Glu Gln Leu Lys Pro Tyr Glu Thr Val
625                 630                 635                 640
Leu Gly Leu Pro Ala Ile Lys Lys Leu Ala Lys Lys His Tyr Thr Gly
                645                 650                 655
Trp Gly Arg Leu Ser Glu Lys Met Ile Gln Gly Met Arg Glu Lys Gln
            660                 665                 670
Ser Arg Lys Thr Ile Leu Asp Tyr Leu Ile Asp Asp Asp Phe Pro
        675                 680                 685

Cys Asn Arg Asn Arg Asn Phe Met Gln Leu Ile Asn Asp Asp His Leu
        690                 695                 700
Ser Phe Lys Glu Thr Ile Ala Asn Glu Leu Ile Met Ser Asp Ser Asn
705 710 715 720
Val Leu Leu Asp Gln Val Lys Ala Ile Pro Gly Ser Pro Ala Val Lys
725 730 735
Lys Gly Ile Trp Gln Ser Ile Lys Ile Val Glu Glu Ile Ile Gly Ile
                740                 745                 750
Ile Gly Lys Ala Pro Lys Asn Ile Val Ile Glu Met Ala Arg Glu Asn
            755                 760                 765
Gln Arg Thr Ser Arg Ser Arg Pro Arg Leu Lys Ala Leu Glu Glu Ala
        770                 775                 780
Leu Lys Asn Ile Asp Ser Pro Leu Leu Lys Asp Tyr Pro Thr Asp Asn
785 790 795 800
Gln Ala Leu Gln Lys Asp Arg Leu Tyr Leu Tyr Tyr Leu Gln Asn Gly
805 810 815
Lys Asp Met Tyr Thr Gly Glu Pro Leu Glu Ile His Arg Leu Ser Glu
                820                 825                 830
Tyr Asp Ile Asp His Ile Ile Pro Arg Ser Phe Ile Val Asp Asn Ser
            835                 840                 845
Leu Asp Asn Lys Val Leu Val Ser Ser Lys Val Asn Arg Gly Lys Leu
        850                 855                 860
Asp Asn Ala Pro Asp Pro Leu Val Val Lys Arg Met Arg Ser His Trp
865 870 875 880
Glu Lys Leu His Gln Ala Lys Leu Ile Ser Asp Lys Lys Leu Ala Asn
885 890 895
Leu Thr Lys Gln Asn Leu Thr Glu Ala Asp Lys Ala Arg Phe Ile Gln
                900                 905                 910
Arg Gln Leu Val Glu Thr Arg Gln Ile Thr Lys His Val Ala Asn Leu
            915                 920                 925
Leu His Gln His Phe Asn Leu Pro Glu Glu Val Ser Ala Thr Glu Lys
        930                 935                 940
Thr Ser Ile Ile Thr Leu Lys Ser Thr Leu Thr Ser Gln Phe Arg Gln
945 950 955 960
Met Phe Asp Ile Tyr Lys Val Arg Glu Ile Asn His His His His Ala
965 970 975
His Asp Ala Tyr Leu Asn Gly Val Val Ala Met Thr Leu Leu Lys Lys
                980                 985                 990
Tyr Pro Lys Leu Ala Pro Glu Phe Val Tyr Gly Ser Tyr Ile Lys Gly
            995                 1000                1005
Asp Ile Asn Gln Ile Asn Lys Ala Thr Ala Lys Lys Glu Phe Tyr
        1010                1015                1020
Ser Asn Ile Met Lys Phe Phe Glu Ser Glu Glu Ile Ile Cys Asp
        1025                1030                1035
Glu Gln Gly Glu Val Ile Trp Asn Lys Lys Arg Asp Leu Ser Thr
        1040                1045                1050
Ile Lys Lys Thr Ile Gly Ala His Gln Val Asn Ile Val Lys Lys
        1055                1060                1065
Val Glu Lys Gln Lys Gly Gly Phe Tyr Lys Glu Thr Ile Asn Ser
        1070                1075                1080
Lys Ala Asn Pro Glu Lys Leu Ile Pro Arg Lys Ala Ser Leu Asp
        1085                1090                1095
Pro Leu Lys Tyr Gly Gly Tyr Gly Ser Pro Leu Val Ala Tyr Thr
        1100                1105                1110
Val Ile Phe Ile Phe Glu Lys Gly Lys Gln Lys Lys Val Thr Lys
        1115                1120                1125
Gly Ile Glu Gly Ile Thr Val Met Glu Gln Leu Arg Phe Glu Gln
        1130                1135                1140
Asp Pro Arg Glu Phe Leu Lys Thr Lys Gly Tyr Glu Gly Val Lys
        1145                1150                1155
Gln Trp Leu Ile Leu Pro Lys Tyr Ile Leu Phe Glu Ala Gln Gly
        1160                1165                1170
Gly Tyr Arg Arg Met Ile Ala Ser His Gln Glu Thr Gln Lys Ala

1175                    1180                    1185
Asn Ser Leu Ile Leu Pro Glu Asn Leu Val Thr Leu Leu Tyr His
    1190                    1195                    1200
Ala Arg His Tyr Asp Glu Ile Asn His Lys Val Ser Phe Asp Tyr
    1205                    1210                    1215
Val Asn Ala His Lys Glu Gly Phe Asn Asp Ile Phe Asp Phe Ile
    1220                    1225                    1230
Ser Asp Phe Gly Val Arg Tyr Ile Leu Ala Pro Gln His Leu Glu
    1235                    1240                    1245
Lys Ile Lys Val Ala Tyr Glu Lys Asn Lys Glu Val Asp Leu Lys
    1250                    1255                    1260
Glu Met Ile Asp Ala Ile Leu Ser Leu Leu Lys Phe Thr Leu Phe
    1265                    1270                    1275
Gly Ala Ser Val Glu Phe Lys Phe Phe Asp Ile Lys Ile Leu Lys
    1280                    1285                    1290
Arg Tyr Lys Ser Leu Thr Asp Ile Trp Glu Ala Thr Ile Ile Tyr
    1295                    1300                    1305
Gln Ser Val Thr Gly Leu Tyr Glu Arg Arg Val Glu Val Arg Lys
    1310                    1315                    1320
Leu Trp Asp Gly Glu Arg Leu
    1325                    1330

<210>  56
<211>  1150
<212>  PRT
<213>  Enterococcus faecalis

<400>  56
Met Tyr Ser Ile Gly Leu Asp Leu Gly Ile Ser Ser Val Gly Trp Ser
1               5                   10                  15
Val Ile Asp Glu Arg Thr Gly Asn Val Ile Asp Leu Gly Val Arg Leu
            20                  25                  30
Phe Ser Ala Lys Asn Ser Glu Lys Asn Leu Glu Arg Arg Thr Asn Arg
        35                  40                  45
Gly Gly Arg Arg Leu Ile Arg Arg Lys Thr Asn Arg Leu Lys Asp Ala
    50                  55                  60
Lys Lys Ile Leu Ala Ala Val Gly Phe Tyr Glu Asp Lys Ser Leu Lys
65                  70                  75                  80
Asn Ser Cys Pro Tyr Gln Leu Arg Val Lys Gly Leu Thr Glu Pro Leu
                85                  90                  95
Ser Lys Gly Glu Ile Tyr Lys Val Thr Leu His Ile Leu Lys Lys Arg
            100                 105                 110
Gly Ile Ser Tyr Leu Asp Glu Asp Asp Thr Glu Ala Ala Lys Glu Ser
        115                 120                 125
Gln Asp Tyr Lys Glu Gln Val Arg Glu Asn Ala Gln Leu Leu Thr Lys
    130                 135                 140
Tyr Thr Pro Gly Gln Ile Gln Leu Gln Arg Leu Lys Glu Asn Asn Arg
145                 150                 155                 160
Val Lys Thr Gly Ile Asn Ala Gln Gly Asn Tyr Gln Leu Asn Val Phe
                165                 170                 175
Lys Val Ser Ala Tyr Ala Asn Glu Leu Ala Thr Ile Leu Lys Thr Gln
            180                 185                 190
Gln Ala Phe Tyr Pro Asn Glu Leu Thr Asp Asp Trp Ile Ala Leu Phe
        195                 200                 205
Val Gln Pro Gly Ile Ala Glu Glu Ala Gly Leu Ile Tyr Arg Lys Arg
    210                 215                 220
Pro Tyr Tyr His Gly Pro Gly Asn Glu Ala Asn Asn Ser Pro Tyr Gly
225                 230                 235                 240
Arg Trp Ser Asp Phe Lys Lys Thr Gly Gln Pro Ala Thr Asn Ile Phe
                245                 250                 255
Asp Lys Leu Ile Gly Lys Asp Phe Gln Gly Glu Leu Arg Ala Ser Gly
            260                 265                 270
Leu Ser Leu Ser Ala Gln Gln Tyr Asn Leu Leu Asn Asp Leu Thr Asn

            275                 280                 285
Leu Lys Ile Asp Gly Glu Ile Pro Leu Ser Leu Glu Gln Lys Glu Tyr
    290                 295                 300
Ile Leu Ala Glu Leu Met Thr Lys Glu Phe Thr Arg Phe Gly Val Asn
305                 310                 315                 320
Asp Val Val Lys Leu Leu Gly Val Lys Lys Glu Arg Leu Ser Gly Trp
                325                 330                 335
Arg Leu Asp Lys Lys Gly Lys Pro Glu Ile His Thr Leu Lys Ala Tyr
            340                 345                 350
Arg Asn Trp Arg Lys Ile Phe Ala Glu Ala Gly Ile Asp Leu Ala Thr
        355                 360                 365
Leu Pro Thr Glu Thr Ile Asp Cys Leu Ala Lys Val Leu Thr Leu Asn
    370                 375                 380
Thr Glu Arg Glu Gly Ile Glu Asn Thr Leu Ala Phe Glu Leu Pro Lys
385                 390                 395                 400
Leu Ala Glu Pro Val Lys Ser Leu Val Leu Asp His Tyr Lys Glu Leu
                405                 410                 415
Ser Gln Ser Ile Ser Thr Gln Ala Trp His Arg Phe Ser Leu Lys Thr
            420                 425                 430
Leu His Leu Leu Ile Pro Glu Leu Met Asn Ala Thr Ser Glu Gln Asn
        435                 440                 445
Thr Leu Leu Glu Gln Phe Gln Leu Lys Ser Gly Val Arg Thr Arg Tyr
    450                 455                 460
Ser Glu Tyr Asn Lys Leu Pro Thr Lys Asp Val Leu Ala Glu Ile Tyr
465                 470                 475                 480
Asn Pro Thr Val Asn Lys Thr Val Ser Gln Ala Phe Lys Val Met Asp
                485                 490                 495
Ala Leu Leu Glu Lys Tyr Gly Lys Glu Gln Ile Arg Tyr Ile Thr Ile
            500                 505                 510
Glu Met Pro Arg Asp Asp Asn Glu Glu Asp Glu Lys Lys Arg Ile Lys
        515                 520                 525
Glu Leu His Ala Lys Asn Ser Gln Arg Lys Asn Asp Ser Gln Ser Tyr
    530                 535                 540
Phe Met Gln Lys Ser Gly Trp Ser Gln Glu Lys Phe Gln Thr Thr Ile
545                 550                 555                 560
Gln Lys Asn Arg Arg Phe Leu Ala Lys Leu Leu Tyr Tyr Tyr Glu Gln
                565                 570                 575
Asp Gly Ile Cys Ala Tyr Thr Gly Leu Ser Ile Ser Pro Glu Leu Leu
            580                 585                 590
Val Ser Asp Ser Thr Glu Ile Asp His Ile Ile Pro Ile Ser Ile Ser
        595                 600                 605
Leu Asp Asp Ser Ile Asn Asn Lys Val Leu Val Leu Ser Lys Ala Asn
    610                 615                 620
Gln Val Lys Gly Gln Gln Thr Pro Tyr Asp Ala Trp Met Asp Gly Ser
625                 630                 635                 640
Phe Lys Lys Thr Asn Gly Lys Phe Ser Asn Trp Asp Asp Tyr Gln Lys
                645                 650                 655
Trp Val Glu Ser Arg His Phe Ser His Lys Lys Glu Asn Asn Leu Leu
            660                 665                 670
Glu Thr Arg Asn Ile Phe Asp Ser Glu Gln Val Glu Lys Phe Leu Ala
        675                 680                 685
Arg Asn Leu Asn Asp Thr Arg Tyr Ala Ser Arg Leu Val Leu Asn Thr
    690                 695                 700
Leu Gln Ser Phe Phe Ala Asn Gln Gly Thr Lys Val Arg Val Val Asn
705                 710                 715                 720
Gly Ser Phe Thr His Thr Leu Arg Lys Lys Trp Gly Ala Asp Leu Asp
                725                 730                 735
Lys Thr Arg Glu Thr His His His His Ala Val Asp Ala Thr Leu Cys
            740                 745                 750
Ala Val Thr Pro Phe Val Lys Val Ser Arg Tyr His Tyr Ala Val Lys
        755                 760                 765
Glu Glu Thr Gly Glu Lys Val Met Arg Glu Ile Asp Phe Glu Thr Gly
    770                 775                 780

Glu Ile Val Asp Glu Met Ser Tyr Gln Glu Phe Lys Lys Ser Lys Lys
785                 790                 795                 800
Tyr Glu Arg Lys Thr Tyr Gln Val Lys Trp Pro Asn Phe Arg Glu Gln
                805                 810                 815
Leu Lys Pro Val Asn Leu His Pro Arg Ile Lys Phe Ser His Gln Val
            820                 825                 830
Asp Arg Lys Ala Asn Arg Lys Leu Ser Asp Ala Thr Ile Tyr Ser Val
        835                 840                 845
Arg Glu Lys Thr Glu Val Lys Thr Leu Lys Ser Gly Lys Gln Lys Ile
    850                 855                 860
Thr Thr Asp Glu Tyr Thr Ile Gly Lys Ile Lys Asp Ile Tyr Thr Val
865                 870                 875                 880
Asp Gly Trp Glu Ala Phe Lys Lys Lys Gln Asp Lys Leu Leu Met Lys
                885                 890                 895
Asp Leu Asp Glu Lys Thr Tyr Glu Arg Leu Leu Ser Ile Ala Glu Thr
            900                 905                 910
Thr Pro Asp Phe Gln Glu Val Glu Glu Lys Asn Gly Lys Val Lys Arg
        915                 920                 925
Val Lys Arg Ser Pro Phe Ala Val Tyr Cys Glu Glu Asn Asp Ile Pro
    930                 935                 940
Ala Ile Arg Lys Tyr Ala Lys Lys Asn Asn Gly Pro Leu Ile Arg Ser
945                 950                 955                 960
Leu Lys Tyr Tyr Asp Gly Lys Leu Asn Lys His Ile Asn Ile Thr Lys
                965                 970                 975
Asp Ser Gln Gly Arg Pro Val Glu Lys Thr Lys Asn Gly Arg Lys Val
            980                 985                 990
Thr Leu Gln Ser Leu Lys Pro Tyr  Arg Tyr Asp Ile Tyr  Gln Asp Leu
        995                 1000                1005
Glu Thr  Lys Ala Tyr Tyr Thr  Val Gln Leu Tyr Tyr  Ser Asp Leu
    1010                1015                1020
Arg Phe  Val Glu Gly Lys Tyr  Gly Ile Thr Glu Lys  Glu Tyr Met
    1025                1030                1035
Lys Lys  Val Ala Glu Gln Thr  Lys Gly Gln Val Val  Arg Phe Cys
    1040                1045                1050
Phe Ser  Leu Gln Lys Asn Asp  Gly Leu Glu Ile Glu  Trp Lys Asp
    1055                1060                1065
Ser Gln  Arg Tyr Asp Val Arg  Phe Tyr Asn Phe Gln  Ser Ala Asn
    1070                1075                1080
Ser Ile  Asn Phe Lys Gly Leu  Glu Gln Glu Met Met  Pro Ala Glu
    1085                1090                1095
Asn Gln  Phe Lys Gln Lys Pro  Tyr Asn Asn Gly Ala  Ile Asn Leu
    1100                1105                1110
Asn Ile  Ala Lys Tyr Gly Lys  Glu Gly Lys Lys Leu  Arg Lys Phe
    1115                1120                1125
Asn Thr  Asp Ile Leu Gly Lys  Lys His Tyr Leu Tyr  Tyr Glu Lys
    1130                1135                1140
Glu Pro  Lys Asn Ile Ile Lys
    1145                1150

<210>  57
<211>  1456
<212>  PRT
<213>  Artificial sequence

<220>
<223>  Cas-Locus 16

<400>  57
Met Val Ser His Lys Met Ile Thr Phe Phe Leu Phe Ile Phe Met Leu
1               5                   10                  15
Tyr Glu Asn Gln Ile Ala Met Thr Lys Asn Ile Leu Gly Leu Asp Leu
            20                  25                  30
Gly Thr Asn Ser Ile Gly Trp Ala Leu Ile Lys Gln Asp Phe Glu Asn

35                  40                  45
Lys Gln Gly Glu Ile Leu Gly Met Gly Ser Arg Ile Ile Pro Met Ser
    50                  55                  60
Gln Asp Ile Leu Gly Glu Phe Gly Lys Gly Asn Ser Val Ser Gln Thr
65                  70                  75                  80
Ala Ser Arg Thr Ser Tyr Arg Gly Val Arg Arg Leu Arg Glu Arg Phe
                85                  90                  95
Leu Leu Arg Arg Glu Arg Leu His Arg Val Leu Asn Val Leu Asn Phe
            100                 105                 110
Leu Pro Ala His Tyr Ala Ser Gln Ile Asp Phe Glu Lys Arg Phe Gly
        115                 120                 125
Lys Phe Lys Glu Glu Thr Glu Pro Lys Leu Val Tyr Asp Val Asn Glu
    130                 135                 140
Phe Ile Phe Lys Asn Ser Phe Glu Glu Met Leu Tyr Asp Phe Arg Lys
145                 150                 155                 160
Tyr Gln Pro Gln Leu Leu Glu Asn Asp Lys Lys Val Pro Tyr Asp Trp
                165                 170                 175
Thr Ile Tyr Tyr Leu Arg Asn Lys Ala Leu Thr Gln Lys Ile Glu Lys
            180                 185                 190
Glu Glu Leu Ala Trp Ile Leu Leu Asn Phe Asn Gln Lys Arg Gly Tyr
        195                 200                 205
Tyr Gln Leu Arg Gly Glu Asp Phe Glu Glu Glu Lys Asp Lys Thr Phe
    210                 215                 220
Val Arg Leu Lys Val Asp Lys Ile Val Asp Ser Gly Asp Thr Ile Lys
225                 230                 235                 240
Gly Lys Ile Leu Tyr Asp Val Tyr Phe Glu Asn Gly Trp Lys Tyr Asp
                245                 250                 255
Lys Gln Ile Val Lys Thr Glu Asp Trp Ile Glu Arg Val Lys Glu Phe
            260                 265                 270
Ile Val Thr Glu Ser Phe Leu Lys Asn Gly Asp Ile Lys Arg Thr Phe
        275                 280                 285
Lys Ala Val Asp Ser Glu Lys Asp Trp Ile Ala Ile Lys Thr Lys Thr
    290                 295                 300
Glu Gln Glu Ile Asp Asn Ser Asn Lys Thr Val Gly Thr Tyr Ile Tyr
305                 310                 315                 320
Glu Thr Leu Leu Arg Asn Pro Lys Gln Lys Ile Lys Gly Lys Leu Val
                325                 330                 335
Arg Thr Ile Glu Arg Lys Phe Tyr Lys Asp Glu Leu Lys Gln Ile Leu
            340                 345                 350
Glu Lys Gln Lys Glu Phe His Leu Glu Leu Lys Asn Asp Asp Leu Tyr
        355                 360                 365
Ser Asp Cys Ile Arg Glu Leu Tyr Arg Asn Asn Glu Ala His Gln Leu
    370                 375                 380
Thr Leu Ser Arg Lys Asp Phe Val His Leu Leu Met Glu Asp Ile Ile
385                 390                 395                 400
Phe Tyr Gln Arg Pro Leu Arg Ser His Lys Ser Ser Ile Ser Asn Cys
                405                 410                 415
Thr Leu Glu Phe Arg Lys Tyr Lys Asp Glu Asn Gly Ala Glu His Ile
            420                 425                 430
Gln Tyr Leu Lys Ala Ile Pro Lys Ser Asn Pro Tyr Tyr Gln Glu Phe
        435                 440                 445
Arg Ile Trp Gln Trp Ile Phe Asn Leu Asn Ile Tyr Lys Arg Asp Asp
    450                 455                 460
Glu Gln Asn Pro Val Thr Lys Lys Phe Leu Asn Thr Thr Gln Asp Phe
465                 470                 475                 480
Glu Asn Leu Phe Glu Phe Leu Asn Asn Arg Lys Glu Ile Asp Gln Lys
                485                 490                 495
Ala Leu Leu Lys His Leu Lys Ile Asn Glu Lys Thr His Arg Trp Lys
            500                 505                 510
Tyr Val Glu Asp Lys Lys Tyr Pro Cys Asn Glu Thr Lys Thr Met Ile
        515                 520                 525
Ser Ser Arg Leu Asp Lys Val Glu Asn Ile Ser Glu Asn Phe Leu Thr
    530                 535                 540

Arg Glu Ile Glu Gln Lys Ile Trp His Ile Ile Tyr Ser Val Asn Asp
545                 550                 555                 560
Lys Ile Glu Tyr Glu Lys Ala Leu Lys Ser Phe Ala Asn Lys Asn Asn
                565                 570                 575
Leu Asp Glu Val Ser Phe Phe Glu Ala Phe Lys Lys Phe Pro Pro Phe
            580                 585                 590
Lys Ser Glu Tyr Gly Ser Phe Ser Glu Lys Ala Ile Lys Lys Leu Leu
        595                 600                 605
Pro Leu Met Arg Leu Gly Lys Tyr Trp Asn Tyr Ala Asp Ile Asp Lys
    610                 615                 620
Tyr Ser Lys Asp Arg Ile Gln Lys Ile Ile Asn Gly Glu Tyr Asp Glu
625                 630                 635                 640
Asn Ile Lys Asp Lys Val Arg Glu Lys Ala Val His Leu Thr Thr Glu
                645                 650                 655
Asn Asp Phe Gln Gly Gln Gln Leu Trp Leu Ala Gln Tyr Ile Val Tyr
            660                 665                 670
Gly Arg His Ser Glu Ala Ser Met Ile Gly Lys Trp Asn Ser Ala Asp
        675                 680                 685
Asp Leu Glu Glu Phe Leu Lys Glu Phe Lys Gln His Ser Leu Arg Asn
    690                 695                 700
Pro Ile Val Glu Gln Val Ile Thr Glu Thr Leu Arg Val Val Lys Asp
705                 710                 715                 720
Ile Trp Leu Lys Tyr Gly Asn Gly Ala Lys Asp Phe Phe Asn Glu Ile
                725                 730                 735
His Ile Glu Leu Gly Arg Glu Met Lys Leu Pro Ala Asp Asp Arg Lys
            740                 745                 750
Lys Leu Thr Asn Gln Ile Ser Glu Asn Glu Asn Thr Asn Leu Arg Ile
        755                 760                 765
Lys Ala Leu Leu Ala Glu Met Ile Asn Asp His Ser Val Glu Asn Val
    770                 775                 780
Arg Pro Phe Ser Pro Met Gln Gln Glu Ile Leu Lys Ile Tyr Glu Asp
785                 790                 795                 800
Gly Val Leu Asn Ser Gly Ile Glu Ile Glu Asp Glu Tyr Leu Lys Ile
                805                 810                 815
Ser Lys Thr Ala Gln Pro Ser Pro Ser Asp Leu Lys Arg Tyr Lys Leu
            820                 825                 830
Trp Leu Glu Gln Lys Tyr Lys Ser Pro Tyr Thr Gly Gln Ile Ile Pro
        835                 840                 845
Leu Asn Lys Leu Phe Thr Pro Glu Tyr Glu Ile Glu His Ile Ile Pro
    850                 855                 860
Gln Ser Arg Tyr Phe Asp Asp Ser Phe Ser Asn Lys Ile Ile Cys Glu
865                 870                 875                 880
Ser Ala Val Asn Lys Leu Lys Asp Asn Tyr Ile Gly Leu Gly Phe Ile
                885                 890                 895
Lys Gln Phe Gly Gly Thr Ile Ile Glu Leu Gly Phe Gly Lys Asn Val
            900                 905                 910
Lys Val Phe Glu Val Glu Glu Tyr Glu Asp Phe Val Lys Lys His Tyr
        915                 920                 925
Ala Asn Asn Arg Ser Lys Arg Asn Lys Leu Leu Leu Glu Glu Ile Pro
    930                 935                 940
Glu Lys Met Ile Glu Arg Gln Leu Asn Asp Thr Arg His Ile Ser Lys
945                 950                 955                 960
Tyr Ile Ser Gly Val Leu Ser Asn Ile Val Arg Val Glu Asp Gly Thr
                965                 970                 975
Asp Glu Gly Val Asn Ser Lys Asn Ile Val Pro Gly Asn Gly Lys Ile
            980                 985                 990
Thr Thr Gln Leu Lys Gln Asp Trp  Gly Leu Asn Asp Val  Trp Asn Asp
        995                 1000                 1005
Leu Ile  Leu Pro Arg Phe Glu  Arg Met Asn Gln Leu  Thr Asn Ser
    1010                 1015                 1020
Thr Asp  Phe Thr Ala Trp Asn  Glu Asn His Gln Lys  Tyr Leu Pro
    1025                 1030                 1035
Thr Val  Pro Ile Glu Phe Ser  Lys Gly Phe Ser Lys  Lys Arg Ile

1040                    1045                    1050
Asp His Arg His His Ala Leu Asp Ala Leu Val Ile Ala Cys Ala
    1055                    1060                    1065
Thr Lys Asp His Val Asn Leu Leu Asn Asn Gln Ser Ala Lys Ser
    1070                    1075                    1080
Glu Thr Lys Arg Tyr Asp Leu Lys Lys Arg Leu Met Lys Phe Glu
    1085                    1090                    1095
Lys Val Val Tyr Asn His Thr Gln Thr Gly Glu Lys Ile Glu Arg
    1100                    1105                    1110
Asp Val Pro Lys Lys Phe Leu Lys Pro Trp Glu Asn Phe Thr Val
    1115                    1120                    1125
Glu Ala Lys Asn Ser Leu Glu Lys Ile Ile Val Ser Phe Lys Gln
    1130                    1135                    1140
Asn Leu Arg Val Ile Asn Lys Ala Thr Asn Tyr Tyr Glu Lys Tyr
    1145                    1150                    1155
Val Glu Lys Asp Gly Leu Lys Thr Lys Glu Arg Val Glu Gln Thr
    1160                    1165                    1170
Gly Thr Asn Trp Ala Ile Arg Lys Ser Leu His Glu Glu Thr Val
    1175                    1180                    1185
Ser Gly Lys Ile Asn Leu Pro Trp Val Glu Thr Ser Asn Gly Glu
    1190                    1195                    1200
Phe Ile Thr Ala Thr Arg Lys Ser Leu Asp Thr Ser Phe Asn Leu
    1205                    1210                    1215
Glu Lys Ile Lys Lys Ile Thr Asp Ile Gly Ile Gln Lys Ile Leu
    1220                    1225                    1230
Arg Asn Tyr Leu Glu Ala Lys Asp Gly Asn Pro Glu Leu Ala Phe
    1235                    1240                    1245
Ser Pro Glu Gly Ile Glu Asp Leu Asn Lys Asn Val Glu Lys Tyr
    1250                    1255                    1260
Asn Asp Gly Lys Ser His Gln Pro Ile Ile Lys Val Arg Val Tyr
    1265                    1270                    1275
Glu Lys Gly Lys Gly Arg Phe Val Leu Gly Gln Thr Gly Asn Lys
    1280                    1285                    1290
Ile Asp Lys Tyr Val Gln Gly Ser Pro Asn Leu Phe Phe Ala Ile
    1295                    1300                    1305
Tyr Lys Asp Asp Asn Gly Lys Lys Ser Tyr Glu Ser Ile Arg Leu
    1310                    1315                    1320
Asp Ile Ala Ile Glu Arg Leu Lys Gln Gly Leu Gln Ala Val Pro
    1325                    1330                    1335
Glu Thr Asn Gln Asn Gly Phe Ala Leu Tyr Gln Ser Leu Ser Pro
    1340                    1345                    1350
Leu Asp Leu Val Tyr Ile Pro Thr Glu Asp Glu Val Glu Ser Pro
    1355                    1360                    1365
His Leu Ile Asp Phe Ser Lys Leu Asp Lys Ile Lys Leu Glu Arg
    1370                    1375                    1380
Ile Tyr Asn Val Asn Asp Phe Ser Gly Tyr Thr Ile Tyr Phe Ser
    1385                    1390                    1395
Gln Asn Ser Phe Ala Lys Asn Ile Tyr Pro Lys Glu Met Asp Met
    1400                    1405                    1410
Thr Trp Asn Glu Lys Lys Gln Lys Pro Ser Gly Ser Phe Asp Ser
    1415                    1420                    1425
Lys Thr Ala Ser Tyr Asn Lys Lys Ser Ile Lys Asp Ile Phe Ile
    1430                    1435                    1440
Lys Met Lys Ile Asp Arg Leu Gly Asn Ile Ser Lys Ala
    1445                    1450                    1455

<210>  58
<211>  1462
<212>  PRT
<213>  Artificial sequence

<220>
<223>  Cas-Locus 17

<400>  58
Met Thr Asn Ile Glu Glu Lys Ile Phe Asn Thr Ile Met Gly Ile Arg
1               5                   10                  15
Asn Leu Asn Ala Phe Phe Ile Tyr Ile Leu Gly Asp Ile Gln Asn Ile
            20                  25                  30
Thr Val Met Ile Lys Asn Lys Leu Gly Leu Asp Leu Gly Thr Asn Ser
        35                  40                  45
Ile Gly Trp Ala Leu Ile Arg Gln Asp Phe Glu Asn Lys Tyr Gly Glu
    50                  55                  60
Ile Leu Gly Met Gly Ser Arg Ile Ile Pro Met Ser Gln Asp Ile Leu
65                  70                  75                  80
Gly Asp Phe Gly Lys Gly Asn Ser Val Ser Gln Thr Ala Glu Arg Thr
                85                  90                  95
Lys Tyr Arg Ser Val Arg Arg Leu Arg Glu Arg Phe Leu Leu Arg Arg
            100                 105                 110
Glu Arg Leu His Arg Leu Leu His Leu Leu Asn Phe Leu Pro Gln His
        115                 120                 125
Tyr Ala Ser Gln Ile Asp Phe Glu Lys Arg Phe Gly Lys Phe Lys Val
    130                 135                 140
Glu Thr Glu Pro Lys Leu Ala Trp Lys Asn Ile Glu Gly Lys Phe Ser
145                 150                 155                 160
Phe Leu Phe Gln Asn Ser Phe Asn Glu Met Leu Glu Asp Phe Asn Ala
                165                 170                 175
Asn Gly Gln Asp Leu Lys Ile Pro Tyr Asp Trp Thr Ile Tyr Phe Leu
            180                 185                 190
Arg Lys Lys Ala Leu Ser Gln Lys Ile Glu Lys Glu Glu Leu Ala Trp
        195                 200                 205
Ile Leu Leu Asn Phe Asn Gln Lys Arg Gly Tyr Tyr Gln Leu Arg Gly
    210                 215                 220
Glu Glu Glu Glu Glu Asn Pro Asn Lys Gln Val Glu Phe Tyr Ser Leu
225                 230                 235                 240
Lys Val Ile Asp Val Leu Ala Asp Glu Pro Gln Lys Gly Lys Ser Asp
                245                 250                 255
Ile Trp Tyr Ser Leu Ile Leu Glu Asn Gly Trp Ile Tyr Arg Arg Ser
            260                 265                 270
Ser Lys Ile Pro Leu Phe Asp Trp Lys Asp Lys Thr Arg Asp Phe Ile
        275                 280                 285
Val Thr Thr Asp Leu Asn Asp Asp Gly Ser Ile Lys Lys Asp Lys Asp
    290                 295                 300
Gly Asn Glu Lys Arg Ser Phe Arg Ala Pro Gly Glu Asn Asp Trp Thr
305                 310                 315                 320
Leu Val Lys Lys Lys Thr Glu Gln Glu Ile Glu Gln Ser His Lys Thr
                325                 330                 335
Val Gly Thr Tyr Ile Tyr Glu Ala Leu Leu Asp Asn Pro Lys Gln Lys
            340                 345                 350
Ile Lys Gly Lys Leu Ile Arg Thr Ile Glu Arg Lys Phe Tyr Lys Glu
        355                 360                 365
Glu Leu Arg Gln Ile Leu Glu Lys Gln Lys Glu Phe His Gln Glu Leu
    370                 375                 380
Gln Ser Asp Asp Leu Tyr Asn Asp Cys Ile Arg Glu Leu Tyr Arg Asn
385                 390                 395                 400
Asn Glu Ser His Gln Leu Ile Leu Ser Lys Lys Asp Phe Val His Leu
                405                 410                 415
Leu Met Glu Asp Leu Ile Phe Tyr Gln Arg Pro Leu Arg Ser Gln Lys
            420                 425                 430
Ser Ser Val Ser Asn Cys Thr Leu Glu Phe Arg Lys Tyr Lys Asp Glu
        435                 440                 445
Asn Gly Ala Glu His Ile Gln Tyr Leu Lys Ala Ile Pro Lys Ser Asn
    450                 455                 460
Pro Tyr Tyr Gln Glu Phe Arg Leu Trp Gln Trp Ile Tyr Asn Leu Asn
465                 470                 475                 480
Ile Tyr Arg Lys Asp Asp Glu Ala Asn Val Thr Lys Asp Phe Leu Asn

                485                 490                 495
Thr Thr Gln Asp Leu Glu Asn Leu Phe Glu Phe Leu Asn Ile Arg Lys
            500                 505                 510
Glu Ile Asp Gln Lys Gly Leu Leu Lys His Phe Lys Leu Asn Glu Lys
        515                 520                 525
Lys His Arg Trp Asn Phe Val Glu Glu Lys Lys Tyr Pro Cys Asn Glu
    530                 535                 540
Thr Lys Thr Met Ile Ser Ser Arg Leu Asp Lys Val Asp Asn Ile Ser
545                 550                 555                 560
Asp Asp Phe Leu Thr Arg Asp Ile Glu Gln Lys Ile Trp His Ile Ile
                565                 570                 575
Tyr Ser Val Asn Asp Lys Val Glu Tyr Glu Lys Ala Leu Lys Ser Phe
            580                 585                 590
Ala Arg Lys Tyr His Leu Asp Glu Ser Ser Phe Phe Glu Ala Phe Arg
        595                 600                 605
Lys Phe Pro Pro Phe Lys Ser Glu Tyr Gly Ser Phe Ser Glu Lys Ala
    610                 615                 620
Ile Lys Lys Leu Leu Ala Leu Met Arg Leu Gly Lys Tyr Trp Asn Tyr
625                 630                 635                 640
Ala Asp Val Asp Lys Phe Ser Lys Asp Arg Ile Gln Lys Ile Ile Asn
                645                 650                 655
Gly Glu Tyr Asp Glu Asn Ile Lys Asp Lys Val Arg Glu Lys Ala Ile
            660                 665                 670
Asn Leu Thr Ser Glu Asn Asp Phe Gln Gly Leu Gln Leu Trp Leu Ala
        675                 680                 685
Gln Tyr Ile Ile Tyr Gly Arg His Ser Glu Ala Ser Met Ile Gly Lys
    690                 695                 700
Trp Asn Ser Ala Asp Asp Leu Glu Met Phe Leu Lys Asp Phe Lys Gln
705                 710                 715                 720
His Ser Leu Arg Asn Pro Ile Val Glu Gln Val Ile Thr Glu Thr Leu
                725                 730                 735
Arg Val Val Lys Asp Ile Trp Leu Lys Tyr Gly Asn Gly Ala Lys Asp
            740                 745                 750
Phe Phe Asn Glu Ile His Ile Glu Leu Gly Arg Glu Met Lys Leu Pro
        755                 760                 765
Ala Gly Asp Arg Lys Lys Leu Thr Asn Gln Ile Thr Glu Asn Glu Asn
    770                 775                 780
Thr Asn Leu Arg Ile Lys Ala Leu Leu Ala Glu Met Met Asn Asp His
785                 790                 795                 800
Ser Val Glu Asn Val Arg Pro Phe Ser Pro Met Gln Gln Glu Ile Leu
                805                 810                 815
Lys Ile Tyr Glu Asp Gly Val Leu Asn Ser Gly Ile Glu Ile Glu Asp
            820                 825                 830
Glu Tyr Leu Lys Ile Ser Lys Thr Ala Gln Pro Ser Ser Ser Asp Leu
        835                 840                 845
Lys Arg Tyr Lys Leu Trp Leu Glu Gln Lys Tyr Lys Ser Pro Tyr Thr
    850                 855                 860
Gly Gln Ile Ile Pro Leu Asn Lys Leu Phe Thr Pro Glu Tyr Glu Ile
865                 870                 875                 880
Glu His Ile Ile Pro Gln Ser Arg Tyr Phe Asp Asp Ser Phe Ser Asn
                885                 890                 895
Lys Val Ile Cys Glu Ser Ala Val Asn Lys Leu Lys Asp Asn Tyr Ile
            900                 905                 910
Gly Leu Gly Phe Ile Lys Gln Phe Gly Gly Thr Ile Ile Glu Leu Gly
        915                 920                 925
Phe Gly Lys Ser Val Lys Val Phe Asn Ile Glu Glu Tyr Glu Asp Phe
    930                 935                 940
Val Lys Lys His Tyr Ala Asn Asn Arg Gly Lys Arg Asn Lys Leu Leu
945                 950                 955                 960
Met Glu Asp Ile Pro Gly Lys Met Ile Glu Arg Gln Leu Asn Asp Thr
                965                 970                 975
Arg Tyr Ile Ser Lys Tyr Val Ser Gly Ile Leu Ser Asn Ile Val Arg
            980                 985                 990

Val Glu Asp Gly Ser Asp Glu Gly Val Asn Ser Lys Asn Ile Val Pro
        995                 1000                1005
Gly Asn Gly Lys Ile Thr Thr Gln Leu Lys Gln Asp Trp Gly Leu
    1010                1015                1020
Asn Asp Val Trp Asn Asp Ile Ile Leu Pro Arg Phe Glu Arg Met
    1025                1030                1035
Asn Gln Leu Thr Asn Ser Lys Asp Phe Thr Ala Trp Asn Glu Asn
    1040                1045                1050
His Gln Lys Phe Leu Pro Thr Val Pro Ile Glu Phe Ser Lys Gly
    1055                1060                1065
Phe Ser Lys Lys Arg Ile Asp His Arg His His Ala Leu Asp Ala
    1070                1075                1080
Leu Val Ile Ala Cys Ala Thr Lys Asp His Val Asn Leu Leu Asn
    1085                1090                1095
Asn Gln Ser Ala Lys Ser Asp Thr Lys Arg Tyr Asp Leu Lys Lys
    1100                1105                1110
Lys Leu Met Lys Phe Glu Lys Val Val Tyr Tyr His Thr Gln Thr
    1115                1120                1125
Gly Glu Lys Ile Glu Arg Glu Ile Pro Lys Gln Phe Leu Lys Pro
    1130                1135                1140
Trp Glu Asn Phe Thr Val Asp Ala Lys His Asn Leu Glu Ser Ile
    1145                1150                1155
Ile Val Ser Phe Lys Gln Asn Leu Arg Val Ile Asn Lys Ala Thr
    1160                1165                1170
Asn Tyr Tyr Glu Lys Tyr Val Glu Lys Asp Gly Thr Lys Asn Lys
    1175                1180                1185
Glu Arg Val Glu Gln Thr Gly Thr Asn Trp Ala Ile Arg Lys Pro
    1190                1195                1200
Met His Lys Asp Thr Val Ser Gly Lys Val Asp Leu Pro Trp Val
    1205                1210                1215
Lys Val Pro Lys Gly Lys Ile Leu Thr Ala Thr Arg Lys Ser Leu
    1220                1225                1230
Asp Ile Ser Phe Asp Leu Lys Ser Ile Ala Ser Ile Thr Asp Thr
    1235                1240                1245
Gly Ile Gln Lys Ile Leu Arg Asn Tyr Leu Lys Phe Lys Gly Ser
    1250                1255                1260
Pro Glu Leu Ala Phe Ser Pro Glu Gly Ile Glu Asp Leu Asn Lys
    1265                1270                1275
Asn Ile Glu Lys Tyr Asn Asp Gly Lys Ser His Gln Pro Ile Asn
    1280                1285                1290
Lys Val Arg Val Phe Glu Leu Gly Ser Lys Phe Gln Val Gly Gln
    1295                1300                1305
Thr Gly Asn Lys Lys Asp Lys Tyr Val Glu Ala Ala Lys Gly Thr
    1310                1315                1320
Asn Leu Phe Phe Ala Val Tyr Glu Asn Glu Asn Arg Asn Arg Ser
    1325                1330                1335
Tyr Glu Thr Ile Pro Leu Asn Glu Val Ile Glu Arg Gln Lys Gln
    1340                1345                1350
Gly Leu Ser Val Val Asp Leu Lys Gly Thr Ile Asp Phe Phe Leu
    1355                1360                1365
Cys Pro Asn Asp Leu Val Tyr Ile Pro Ser Glu Asp Glu Leu Glu
    1370                1375                1380
Asn Ser Ser Asn Ile Asp Phe Lys Asn Phe Lys Glu Glu Gln Asn
    1385                1390                1395
Lys Lys Ile Tyr Lys Val Val Ser Phe Ser Gly Ser Gln Leu Phe
    1400                1405                1410
Phe Ile Arg Gln Glu Ile Ala Ile Ser Ile Val Asn Lys Ala Glu
    1415                1420                1425
Phe Ser Thr Leu Asn Lys Met Glu Arg Ser Ile Asp Gly Leu Met
    1430                1435                1440
Ile Lys Glu Asn Cys Ile Lys Leu Lys Val Asp Arg Leu Gly Asn
    1445                1450                1455
Ile Ser Lys Ala

    1460

<210> 59
<211> 1440
<212> PRT
<213> Artificial sequence

<220>
<223> Cas-Locus 18

<400> 59
Met Lys Asn Ile Leu Gly Leu Asp Leu Gly Thr Asn Ser Ile Gly Trp
1               5                   10                  15
Ala Leu Val Lys Gln Asp Phe Glu Asn Lys Gln Gly Glu Ile Leu Gly
            20                  25                  30
Met Gly Thr Arg Ile Ile Pro Met Ser Gln Asp Ile Leu Gly Asp Phe
        35                  40                  45
Gly Lys Gly Asn Ser Val Ser Gln Thr Ala Glu Arg Thr Lys Tyr Arg
    50                  55                  60
Ser Val Arg Arg Leu Arg Glu Arg Phe Leu Leu Arg Arg Glu Arg Leu
65                  70                  75                  80
His Arg Val Leu Asn Ile Leu Asn Ser Leu Pro Glu His Tyr Ala Ser
                85                  90                  95
Gln Ile Asp Phe Glu Lys Arg Phe Gly Lys Phe Lys Val Glu Thr Glu
            100                 105                 110
Pro Lys Leu Ala Trp Lys Asn Ile Glu Gly His Phe Ser Phe Leu Phe
        115                 120                 125
Gln Asn Ser Phe Asn Glu Met Leu Glu Asp Phe Lys Thr Asn Gly Gln
    130                 135                 140
Asp Leu Lys Ile Pro Tyr Asp Trp Thr Ile Tyr Tyr Leu Arg Lys Lys
145                 150                 155                 160
Ala Leu Ser Gln Lys Ile Glu Lys Glu Glu Leu Ala Trp Ile Leu Leu
                165                 170                 175
Asn Phe Asn Gln Lys Arg Gly Tyr Tyr Gln Leu Arg Gly Glu Glu Glu
            180                 185                 190
Glu Glu Asn Pro Asn Lys Ile Val Glu Phe Tyr Ser Leu Lys Val Val
        195                 200                 205
Asp Val Leu Ala Asp Glu Pro Gln Lys Ser Lys Ser Asp Ile Trp Tyr
    210                 215                 220
Ser Leu Val Leu Glu Asn Gly Trp Ile Tyr Arg Arg Ala Ser Lys Ile
225                 230                 235                 240
Ser Leu Phe Asp Trp Lys Asp Lys Thr Arg Asp Phe Ile Val Thr Thr
                245                 250                 255
Asp Leu Asn Asp Asp Gly Ser Val Lys Thr Asp Lys Asp Gly Asn Glu
            260                 265                 270
Lys Arg Ser Phe Arg Ala Pro Gly Glu Asn Asp Trp Thr Leu Val Lys
        275                 280                 285
Lys Lys Thr Glu Gln Glu Ile Glu Gln Ser His Lys Thr Val Gly Ile
    290                 295                 300
Tyr Ile Tyr Glu Thr Leu Leu Asn Asn Pro Lys Gln Lys Ile Lys Gly
305                 310                 315                 320
Lys Leu Val Arg Thr Ile Glu Arg Lys Phe Tyr Lys Glu Glu Leu Arg
                325                 330                 335
Gln Ile Leu Glu Lys Gln Lys Gln Phe His Gln Glu Leu Gln Asn Asp
            340                 345                 350
Asp Leu Tyr Asn Cys Cys Ile Arg Glu Leu Tyr Arg Asn Asn Glu Ala
        355                 360                 365
His Gln Leu Thr Leu Ser Lys Lys Asp Phe Val His Leu Phe Met Glu
    370                 375                 380
Asp Ile Val Phe Tyr Gln Arg Pro Leu Arg Ser Gln Lys Ser Ser Ile
385                 390                 395                 400
Ser Asn Cys Thr Leu Glu Phe Arg Lys Tyr Lys Asp Glu Asn Gly Ala
                405                 410                 415

Glu His Thr Gln Tyr Leu Lys Ala Ile Pro Lys Ser Asn Pro Tyr Tyr
            420                 425                 430
Gln Glu Phe Arg Leu Trp Gln Trp Ile Phe Asn Leu Asn Leu Tyr Lys
        435                 440                 445
Lys Asp Asn Asp Glu Asn Val Ile Lys Glu Phe Leu Thr Thr Thr Gln
    450                 455                 460
Asp Ile Glu Asn Leu Phe Glu Phe Leu Asn Asn Arg Lys Glu Ile Asp
465                 470                 475                 480
Gln Lys Ala Leu Leu Lys His Phe Lys Leu Asn Glu Lys Thr His Arg
                485                 490                 495
Trp Asn Phe Val Glu Asp Lys Lys Tyr Pro Cys Asn Glu Thr Lys Thr
            500                 505                 510
Met Ile Ser Thr Arg Leu Glu Lys Val Glu Asn Ile Ser Asp Asp Phe
        515                 520                 525
Leu Thr Arg Glu Ile Glu Gln Gln Ile Trp His Ile Ile Tyr Ser Val
    530                 535                 540
Asn Asp Lys Ile Glu Tyr Glu Lys Ala Leu Lys Thr Phe Ala Ser Lys
545                 550                 555                 560
His His Leu Asp Glu Ser Ser Phe Phe Glu Ala Phe Arg Lys Phe Pro
                565                 570                 575
Pro Phe Lys Ser Glu Tyr Gly Ser Tyr Ser Glu Lys Ala Ile Lys Lys
            580                 585                 590
Leu Leu Pro Ile Met Arg Leu Gly Lys Tyr Trp Asp Tyr Ala Asn Ile
        595                 600                 605
Asp Gln Ser Ser Lys Asp Arg Ile Lys Lys Ile Ile Asn Gly Glu Tyr
    610                 615                 620
Asp Glu Asn Ile Lys Asp Lys Val Arg Glu Lys Ala Val His Leu Thr
625                 630                 635                 640
Asn Glu Asn Asp Phe Gln Gly Leu Gln Leu Trp Leu Ala Gln Tyr Ile
                645                 650                 655
Val Tyr Gly Arg His Ser Glu Ala Ser Ile Thr Ala Lys Trp Asn Ser
            660                 665                 670
Ala Asp Asp Leu Glu Glu Phe Leu Lys Asp Phe Lys His His Ser Leu
        675                 680                 685
Arg Asn Pro Ile Val Glu Gln Val Ile Thr Glu Thr Leu Arg Val Val
    690                 695                 700
Lys Asp Ile Trp Leu Lys Tyr Gly Lys Gly Ala Lys Asp Phe Phe Asn
705                 710                 715                 720
Glu Ile His Ile Glu Leu Gly Arg Glu Met Lys Leu Pro Ala Glu Asp
                725                 730                 735
Arg Lys Lys Leu Thr Ser Gln Ile Ile Glu Asn Glu Asn Thr Asn Leu
            740                 745                 750
Arg Ile Lys Ala Leu Leu Ala Glu Met Met Asn Asp His Ala Val Glu
        755                 760                 765
Asn Val Arg Pro Phe Ser Pro Gln Gln Gln Glu Ile Leu Lys Ile Tyr
    770                 775                 780
Glu Asp Gly Val Leu Asn Ser Gly Ile Glu Ile Glu Asp Glu Tyr Leu
785                 790                 795                 800
Lys Ile Ser Lys Thr Ala Gln Pro Ser Pro Ser Asp Leu Lys Arg Tyr
                805                 810                 815
Lys Leu Trp Leu Glu Gln Lys Tyr Lys Ser Pro Tyr Thr Gly Gln Met
            820                 825                 830
Ile Pro Leu Asn Lys Leu Phe Thr Pro Glu Tyr Glu Ile Glu His Ile
        835                 840                 845
Ile Pro Gln Ser Arg Tyr Phe Asp Asp Ser Phe Ser Asn Lys Ile Ile
    850                 855                 860
Cys Glu Ser Ala Val Asn Lys Leu Lys Asp Asn Tyr Ile Gly Leu Gly
865                 870                 875                 880
Phe Ile Lys Gln Phe Gly Gly Thr Ile Ile Glu Leu Gly Leu Gly Lys
                885                 890                 895
Ser Val Lys Val Phe Asp Ile Asp Gln Tyr Glu Asp Phe Val Lys Lys
            900                 905                 910
His Tyr Ala Asn Asn Arg Ser Lys Arg Asn Lys Leu Leu Leu Glu Asp

915                 920                 925
Ile Pro Glu Lys Met Ile Glu Arg Gln Ile Asn Asp Thr Arg Tyr Ile
    930                 935                 940
Ser Lys Tyr Ile Ser Gly Ile Leu Ser Asn Ile Val Arg Leu Glu Asp
945                 950                 955                 960
Gly Ser Asp Glu Gly Val Asn Ser Lys Asn Ile Val Pro Gly Asn Gly
                965                 970                 975
Lys Ile Thr Thr Gln Leu Lys Lys Asp Trp Gly Leu Asn Asp Val Trp
            980                 985                 990
Asn Asp Leu Ile Leu Pro Arg Phe  Lys Arg Met Asn Gln  Leu Thr Asn
        995                 1000                1005
Ser Thr  Asp Phe Thr Ala Trp  Asn Glu Asn Phe Gln  Lys Phe Leu
    1010                1015                1020
Pro Thr  Val Pro Ile Glu Tyr  Ser Lys Gly Phe Ser  Lys Lys Arg
    1025                1030                1035
Ile Asp  His Arg His His Ala  Leu Asp Ala Leu Val  Ile Ala Cys
    1040                1045                1050
Ala Thr  Lys Asp His Val Asn  Leu Leu Asn Asn Gln  Ser Ala Lys
    1055                1060                1065
Ser Glu  Thr Lys Arg Tyr Asp  Leu Lys Lys Lys Leu  Met Lys Phe
    1070                1075                1080
Glu Arg  Val Val Tyr Phe His  Thr Gln Thr Gly Glu  Lys Ile Glu
    1085                1090                1095
Arg Glu  Val Pro Lys Gln Phe  Leu Lys Pro Trp Lys  Asn Phe Thr
    1100                1105                1110
Val Asp  Ala Met His Asn Leu  Glu Thr Val Ile Val  Ser Phe Lys
    1115                1120                1125
Gln Asn  Leu Arg Val Ile Asn  Lys Ala Thr Asn Tyr  Tyr Glu Lys
    1130                1135                1140
Tyr Val  Glu Lys Asp Gly Val  Arg Asn Lys Thr Arg  Ile Lys Gln
    1145                1150                1155
Lys Gly  Ile Asn Trp Ala Ile  Arg Lys Pro Met His  Lys Asp Thr
    1160                1165                1170
Val Ser  Gly Lys Val Asp Leu  Pro Trp Val Lys Val  Pro Lys Gly
    1175                1180                1185
Lys Ile  Leu Thr Ala Thr Arg  Lys Ser Leu Asp Ala  Ser Phe Asp
    1190                1195                1200
Leu Lys  Ser Ile Gly Ser Ile  Thr Asp Thr Gly Ile  Gln Lys Ile
    1205                1210                1215
Leu Lys  Asn Tyr Leu Met His  Lys Asp Gly Asn Thr  Glu Leu Ala
    1220                1225                1230
Phe Ser  Pro Glu Gly Ile Glu  Asp Leu Asn Lys Ser  Ile Glu Lys
    1235                1240                1245
Tyr Asn  Asp Gly Lys Pro His  Gln Pro Ile Asn Lys  Val Arg Val
    1250                1255                1260
Phe Glu  Leu Gly Ser Lys Phe  Gln Val Gly Gln Ala  Gly Asn Lys
    1265                1270                1275
Lys Asp  Lys Tyr Val Glu Ala  Ala Lys Gly Thr Asn  Leu Phe Phe
    1280                1285                1290
Gly Val  Tyr Glu Asp Lys Asn  Gly Lys Arg Ser Tyr  Glu Thr Ile
    1295                1300                1305
Pro Leu  Asn Glu Val Ile Glu  Arg Gln Lys Gln Gly  Leu Pro Val
    1310                1315                1320
Val Asp  Leu Arg Ser Thr Asn  Asp Phe Tyr Leu Cys  Pro Asn Asp
    1325                1330                1335
Leu Val  Tyr Ile Leu Ser Asp  Asp Glu Arg Gly Asn  Met Thr Asn
    1340                1345                1350
Ile Asp  Phe Glu Ser Leu Ser  Asp Glu Gln Val Lys  Arg Ile Tyr
    1355                1360                1365
Lys Phe  Val Ser Cys Thr Gly  Gly Glu Gly His Phe  Ile Pro Tyr
    1370                1375                1380
Ala Asn  Ala Thr Glu Ile Ile  Lys Asn Glu Asn Gly  Thr Asn Ser
    1385                1390                1395

Lys Ser Glu Arg Met Gln Asn Phe Tyr Asp Gly Ser Cys Met Phe
    1400                1405                1410
Asp Lys Asn Ala Lys Pro Ile Met Ile Lys Glu Asn Cys Val Lys
    1415                1420                1425
Leu Lys Val Asp Arg Leu Gly Asn Ile Ala Lys Ile
    1430                1435                1440

<210> 60
<211> 1440
<212> PRT
<213> Artificial sequence

<220>
<223> Cas-Locus 19

<400> 60
Met Lys Asn Ile Leu Gly Leu Asp Leu Gly Thr Asn Ser Ile Gly Trp
1               5                   10                  15
Ala Leu Val Lys Gln Asp Phe Glu Asn Lys Gln Gly Glu Ile Leu Gly
            20                  25                  30
Met Gly Val Arg Ile Ile Pro Met Ser Gln Asp Ile Leu Gly Asp Phe
        35                  40                  45
Gly Lys Gly Asn Ser Val Ser Gln Thr Ala Glu Arg Thr Lys Tyr Arg
    50                  55                  60
Ser Val Arg Arg Leu Arg Glu Arg Phe Leu Leu Arg Arg Glu Arg Leu
65                  70                  75                  80
His Arg Val Leu Asn Ile Leu Asn Phe Leu Pro Glu His Tyr Ala Ser
                85                  90                  95
Gln Ile Asp Phe Glu Lys Arg Phe Gly Lys Phe Lys Val Glu Thr Glu
            100                 105                 110
Pro Lys Leu Ala Trp Lys Asn Ile Glu Gly Gln Phe Ser Phe Leu Phe
        115                 120                 125
Gln Asn Ser Phe Asn Glu Met Leu Glu Asp Phe Lys Ala Asn Gly Gln
    130                 135                 140
Asp Leu Arg Ile Pro Tyr Asp Trp Thr Ile Tyr Tyr Leu Arg Lys Lys
145                 150                 155                 160
Ala Leu Tyr Gln Lys Ile Glu Lys Glu Glu Leu Ala Trp Ile Leu Leu
                165                 170                 175
Asn Phe Asn Gln Lys Arg Gly Tyr Tyr Gln Leu Arg Gly Glu Glu Glu
            180                 185                 190
Glu Glu Asn Pro Asn Lys Leu Val Glu Phe Tyr Ser Leu Lys Val Val
        195                 200                 205
Asp Val Leu Ala Asp Glu Pro Gln Lys Ser Lys Ser Asp Ile Trp Tyr
    210                 215                 220
Ser Leu Val Leu Glu Asn Gly Trp Ile Tyr Arg Arg Ala Ser Lys Ile
225                 230                 235                 240
Leu Leu Phe Asp Trp Lys Asp Lys Thr Arg Asp Phe Ile Val Thr Thr
                245                 250                 255
Asp Leu Asn Asp Asp Gly Ser Val Lys Thr Asp Lys Glu Gly Asn Glu
            260                 265                 270
Lys Arg Ser Phe Arg Ala Pro Gly Glu Asn Asp Trp Thr Leu Val Lys
        275                 280                 285
Lys Lys Thr Glu Gln Lys Ile Glu Gln Ser His Lys Thr Val Gly Met
    290                 295                 300
Tyr Ile Tyr Glu Thr Leu Leu Ala Asn Pro Lys Gln Lys Ile Lys Gly
305                 310                 315                 320
Lys Leu Val Arg Thr Ile Glu Arg Lys Phe Tyr Lys Glu Glu Leu Arg
                325                 330                 335
Gln Ile Leu Glu Lys Gln Lys Glu Phe His Gln Glu Leu Gln Asn Asp
            340                 345                 350
Asp Leu Tyr Asn Asp Cys Ile Arg Glu Leu Tyr Arg Asn Asn Glu Ala
        355                 360                 365
His Gln Leu Thr Leu Ser Lys Lys Asp Phe Ile His Leu Phe Ile Lys

    370                 375                 380
Asp Ile Val Phe Tyr Gln Arg Pro Leu Arg Ser Gln Lys Ser Ser Ile
385                 390                 395                 400
Phe Asn Cys Thr Leu Glu Phe Arg Lys Tyr Lys Asp Glu Asn Gly Ala
                405                 410                 415
Glu His Thr Gln Tyr Leu Lys Ala Ile Pro Lys Ser Asn Pro Tyr Tyr
            420                 425                 430
Gln Glu Phe Arg Leu Trp Gln Trp Ile Phe Asn Leu Asn Leu Tyr Lys
        435                 440                 445
Lys Asp Asn Asp Glu Asn Val Ile Lys Glu Phe Leu Thr Thr Thr Gln
    450                 455                 460
Asp Val Glu Asn Leu Phe Glu Phe Leu Asn Asn Arg Lys Glu Val Asp
465                 470                 475                 480
Gln Lys Thr Leu Leu Lys His Phe Lys Leu Asn Glu Lys Thr His Arg
                485                 490                 495
Trp Asn Phe Val Glu Asp Lys Lys Tyr Pro Cys Asn Glu Thr Lys Thr
            500                 505                 510
Met Ile Ser Thr Arg Leu Gln Lys Val Glu Asn Ile Ser Gly Asp Phe
        515                 520                 525
Leu Thr Arg Glu Ile Glu Gln Lys Ile Trp His Ile Ile Tyr Ser Val
    530                 535                 540
Asn Asp Lys Ile Glu Tyr Glu Lys Ala Leu Lys Ser Phe Ala Leu Lys
545                 550                 555                 560
His Gln Leu Asp Lys Ser Ser Phe Phe Glu Ala Phe Arg Lys Phe Pro
                565                 570                 575
Pro Phe Lys Ser Glu Tyr Gly Ser Phe Ser Glu Lys Ala Ile Lys Lys
            580                 585                 590
Leu Leu Pro Leu Met Arg Leu Gly Lys Tyr Trp Asp Tyr Ala Asn Ile
        595                 600                 605
Asp Lys Phe Ser Lys Asp Arg Ile Gln Lys Ile Ile Asn Gly Glu Tyr
    610                 615                 620
Asp Glu Asn Ile Lys Asp Arg Val Arg Glu Lys Ala Val His Leu Arg
625                 630                 635                 640
Ser Glu Asp Asp Phe Gln Gly Leu Gln Leu Trp Leu Ala Gln Tyr Ile
                645                 650                 655
Val Tyr Gly Arg His Ser Glu Ala Ser Ile Thr Gly Lys Trp Asn Ser
            660                 665                 670
Ala Asp Asp Leu Glu Glu Phe Leu Lys Asp Phe Lys His His Leu Leu
        675                 680                 685
Arg Asn Pro Ile Val Glu Gln Val Ile Thr Glu Thr Leu Arg Val Val
    690                 695                 700
Lys Asp Ile Trp Leu Lys Tyr Gly Lys Gly Ala Lys Asp Phe Phe Asn
705                 710                 715                 720
Glu Ile His Ile Glu Leu Gly Arg Glu Met Lys Leu Pro Ala Asp Asp
                725                 730                 735
Arg Lys Lys Leu Thr Asn Gln Ile Thr Glu Asn Glu Asn Thr Asn Leu
            740                 745                 750
Arg Ile Lys Ala Leu Leu Thr Glu Met Met Asn Asp His Ala Val Glu
        755                 760                 765
Asn Val Arg Pro Phe Ser Pro Gln Gln Gln Glu Ile Leu Lys Ile Tyr
    770                 775                 780
Glu Asp Gly Val Leu Asn Ser Gly Ile Glu Ile Glu Asp Glu Tyr Leu
785                 790                 795                 800
Lys Ile Ser Lys Thr Ala Gln Pro Phe Pro Ser Asp Leu Lys Arg Tyr
                805                 810                 815
Lys Leu Trp Leu Glu Gln Lys Tyr Lys Ser Pro Tyr Thr Gly Gln Ile
            820                 825                 830
Ile Pro Leu Asn Lys Leu Phe Thr Pro Glu Tyr Glu Ile Glu His Ile
        835                 840                 845
Ile Pro Gln Ser Arg Tyr Phe Asp Asp Ser Phe Ser Asn Lys Ile Ile
    850                 855                 860
Cys Glu Ser Ala Val Asn Lys Leu Lys Asp Asn Tyr Ile Gly Leu Gly
865                 870                 875                 880

Phe Ile Lys Gln Phe Gly Gly Thr Ile Ile Glu Leu Gly Phe Gly Lys
                885                 890                 895
Ser Val Lys Val Phe Asp Ile Asp Glu Tyr Glu Asp Phe Val Lys Lys
            900                 905                 910
His Tyr Ala Asn Asn Arg Ser Lys Arg Asn Lys Leu Leu Leu Glu Asp
        915                 920                 925
Ile Pro Glu Lys Met Ile Glu Arg Gln Ile Asn Asp Thr Arg Tyr Ile
    930                 935                 940
Ser Lys Tyr Ile Ser Gly Ile Leu Ser Asn Ile Val Arg Leu Glu Asp
945                 950                 955                 960
Gly Ser Asp Glu Gly Val Asn Ser Lys Asn Ile Val Pro Gly Asn Gly
                965                 970                 975
Lys Ile Thr Thr Gln Leu Lys Lys Asp Trp Gly Leu Asn Asp Val Trp
            980                 985                 990
Asn Asp Leu Ile Leu Pro Arg Phe  Lys Arg Met Asn Gln  Leu Thr Asn
        995                 1000                1005
Ser Thr  Asp Phe Thr Ala Trp  Asn Glu Asn Phe Gln  Lys Phe Leu
    1010                1015                1020
Pro Thr  Val Pro Ile Glu Tyr  Ser Lys Gly Phe Ser  Lys Lys Arg
    1025                1030                1035
Ile Asp  His Arg His His Ala  Leu Asp Ala Leu Val  Ile Ala Cys
    1040                1045                1050
Ala Thr  Lys Asp His Val Asn  Leu Leu Asn Asn Gln  Ser Ala Lys
    1055                1060                1065
Ser Glu  Thr Lys Arg Tyr Asp  Leu Lys Lys Lys Leu  Met Lys Phe
    1070                1075                1080
Glu Arg  Val Val Tyr Leu His  Met Gln Thr Gly Glu  Lys Ile Glu
    1085                1090                1095
Arg Glu  Val Pro Lys Gln Phe  Leu Lys Pro Trp Glu  Asn Phe Thr
    1100                1105                1110
Val Asp  Val Lys Tyr Asn Leu  Asp Met Val Ile Val  Ser Phe Lys
    1115                1120                1125
Gln Asn  Leu Arg Val Ile Asn  Lys Ala Thr Asn Tyr  Tyr Glu Lys
    1130                1135                1140
Tyr Val  Glu Lys Asp Gly Ala  Arg Asn Lys Thr Arg  Val Glu Gln
    1145                1150                1155
Lys Gly  Ile Asn Trp Ala Ile  Arg Lys Pro Met His  Lys Asp Thr
    1160                1165                1170
Val Ser  Gly Lys Val Asp Leu  Pro Trp Val Lys Val  Pro Lys Gly
    1175                1180                1185
Lys Ile  Leu Thr Ala Thr Arg  Lys Ser Leu Asp Ala  Ser Phe Asp
    1190                1195                1200
Leu Lys  Ser Ile Ala Ser Ile  Thr Asp Thr Gly Ile  Gln Lys Ile
    1205                1210                1215
Leu Lys  Asn Tyr Leu Met Leu  Lys Asp Gly Asn Pro  Glu Leu Ala
    1220                1225                1230
Phe Ser  Pro Glu Gly Ile Glu  Asp Leu Asn Lys Ser  Ile Glu Lys
    1235                1240                1245
Tyr Asn  Asp Gly Lys Pro His  Gln Pro Ile Asn Lys  Val Arg Val
    1250                1255                1260
Phe Glu  Leu Gly Ser Lys Phe  Gln Ile Gly Gln Ala  Gly Asn Lys
    1265                1270                1275
Lys Asp  Lys Tyr Val Glu Ala  Ala Lys Gly Thr Asn  Leu Phe Phe
    1280                1285                1290
Ala Val  Tyr Glu Asp Glu Asn  Gly Lys Arg Ser Tyr  Glu Thr Ile
    1295                1300                1305
Pro Leu  Asn Glu Val Ile Glu  Arg Gln Lys His Gly  Leu Pro Val
    1310                1315                1320
Val Asp  Leu Thr Ser Thr Asn  Asp Phe Tyr Leu Cys  Pro Asn Asp
    1325                1330                1335
Leu Val  Tyr Ile Leu Ser Asp  Asp Glu Arg Asp Asn  Met Thr Asn
    1340                1345                1350
Asn Asp  Phe Glu Asn Leu Ser  Asp Glu Gln Val Lys  Arg Ile Tyr

1355                    1360                    1365
Lys Phe Val Ser Cys Thr Gly Gly Glu Gly His Phe Ile Pro Tyr
    1370                    1375                    1380
Ala Ser Ala Thr Glu Ile Ile Lys Asn Glu Asn Gly Thr Asn Ser
    1385                    1390                    1395
Lys Ser Glu Arg Ile Gln Asn Phe Tyr Asp Gly Ser Cys Met Leu
    1400                    1405                    1410
Asp Lys Asn Ser Lys Pro Ile Met Ile Lys Glu Asn Cys Val Lys
    1415                    1420                    1425
Leu Lys Val Asp Arg Leu Gly Asn Ile Ser Lys Ile
    1430                    1435                    1440

<210>  61
<211>  1440
<212>  PRT
<213>  Artificial sequence

<220>
<223>  Cas-Locus 20

<400>  61
Met Lys Asn Ile Leu Gly Leu Asp Leu Gly Thr Asn Ser Ile Gly Trp
1               5                   10                  15
Ala Leu Val Lys Gln Asp Phe Glu Asn Lys Gln Gly Glu Ile Leu Gly
            20                  25                  30
Met Gly Ala Arg Ile Ile Pro Met Ser Gln Asp Ile Leu Gly Asp Phe
        35                  40                  45
Gly Lys Gly Asn Ser Val Ser Gln Thr Ala Glu Arg Thr Lys Tyr Arg
    50                  55                  60
Ser Val Arg Arg Leu Arg Glu Arg Phe Leu Leu Arg Arg Glu Arg Leu
65                  70                  75                  80
His Arg Val Leu Asn Ile Leu Asn Phe Leu Pro Glu His Tyr Ala Ser
                85                  90                  95
Gln Ile Asp Phe Glu Lys Arg Phe Gly Lys Phe Lys Val Glu Thr Glu
            100                 105                 110
Pro Lys Leu Ala Trp Lys Asn Ile Glu Gly Gln Phe Ser Phe Leu Phe
        115                 120                 125
Gln Asn Ser Phe Asn Glu Met Leu Glu Asp Phe Lys Ala Asn Gly Gln
    130                 135                 140
Asp Leu Arg Ile Pro Tyr Asp Trp Thr Ile Tyr Tyr Leu Arg Lys Lys
145                 150                 155                 160
Ala Leu Tyr Gln Lys Ile Glu Lys Glu Glu Leu Ala Trp Ile Leu Leu
                165                 170                 175
Asn Phe Asn Gln Lys Arg Gly Tyr Tyr Gln Leu Arg Gly Glu Glu Glu
            180                 185                 190
Glu Glu Asn Pro Asn Lys Leu Val Glu Phe Tyr Ser Leu Lys Val Val
        195                 200                 205
Asp Val Leu Ala Asp Glu Pro Gln Lys Ser Lys Ser Asp Ile Trp Tyr
    210                 215                 220
Ser Leu Val Leu Glu Asn Gly Trp Ile Tyr Arg Arg Ala Ser Lys Ile
225                 230                 235                 240
Leu Leu Phe Asp Trp Lys Asp Lys Thr Arg Asp Phe Ile Val Thr Thr
                245                 250                 255
Asp Leu Asn Asp Asp Gly Ser Val Lys Thr Asp Lys Glu Gly Asn Glu
            260                 265                 270
Lys Arg Ser Phe Arg Ala Pro Gly Glu Asn Asp Trp Thr Leu Val Lys
        275                 280                 285
Lys Lys Thr Glu Gln Glu Ile Glu Gln Ser His Lys Thr Val Gly Met
    290                 295                 300
Tyr Ile Tyr Glu Thr Leu Leu Ala Asn Pro Lys Gln Lys Ile Lys Gly
305                 310                 315                 320
Lys Leu Val Arg Thr Ile Glu Arg Lys Phe Tyr Lys Glu Glu Leu Arg
                325                 330                 335

Gln Ile Leu Glu Lys Gln Lys Glu Phe His Gln Glu Leu Gln Asn Asp
                340                 345                 350
Asp Leu Tyr Asn Asp Cys Ile Arg Glu Leu Tyr Arg Asn Asn Glu Ala
            355                 360                 365
His Gln Leu Thr Leu Ser Lys Lys Asp Phe Ile His Leu Phe Ile Lys
        370                 375                 380
Asp Ile Val Phe Tyr Gln Arg Pro Leu Arg Ser Gln Lys Ser Ser Ile
385                 390                 395                 400
Ser Asn Cys Thr Leu Glu Phe Arg Lys Tyr Lys Asp Glu Asn Arg Ala
                405                 410                 415
Glu His Thr Gln Tyr Leu Lys Ala Ile Pro Lys Ser Asn Pro Tyr Tyr
            420                 425                 430
Gln Glu Phe Arg Leu Trp Gln Trp Ile Phe Asn Leu Asn Leu Tyr Lys
        435                 440                 445
Lys Asp Asn Asp Glu Asn Val Ile Lys Glu Phe Leu Thr Thr Thr Gln
    450                 455                 460
Asp Val Glu Asn Leu Phe Glu Phe Leu Asn Asn Arg Lys Glu Val Asp
465                 470                 475                 480
Gln Lys Thr Leu Leu Lys His Phe Lys Leu Asn Glu Lys Thr His Arg
                485                 490                 495
Trp Asn Phe Val Glu Asp Lys Lys Tyr Pro Cys Asn Glu Thr Lys Thr
            500                 505                 510
Met Ile Ser Thr Arg Leu Gln Lys Val Glu Asn Ile Ser Gly Asp Phe
        515                 520                 525
Leu Thr Arg Glu Ile Glu Gln Lys Ile Trp His Ile Ile Tyr Ser Val
    530                 535                 540
Asn Asp Lys Ile Glu Tyr Glu Lys Ala Leu Lys Ser Phe Ala Leu Lys
545                 550                 555                 560
His Gln Leu Asp Lys Ser Ser Phe Phe Glu Ala Phe Arg Lys Phe Pro
                565                 570                 575
Pro Phe Lys Ser Glu Tyr Gly Ser Phe Ser Glu Lys Ala Ile Lys Lys
            580                 585                 590
Leu Leu Pro Leu Met Arg Leu Gly Lys Tyr Trp Asp Tyr Ala Asn Ile
        595                 600                 605
Asp Lys Phe Ser Lys Asp Arg Ile Gln Lys Ile Ile Asn Gly Glu Tyr
    610                 615                 620
Asp Glu Asn Ile Lys Asp Arg Val Arg Glu Lys Ala Val His Leu Arg
625                 630                 635                 640
Ser Glu Asp Asp Phe Gln Gly Leu Gln Leu Trp Leu Ala Gln Tyr Ile
                645                 650                 655
Val Tyr Gly Arg His Ser Glu Ala Ser Ile Thr Gly Lys Trp Asn Ser
            660                 665                 670
Ala Asp Asp Leu Glu Glu Phe Leu Lys Asp Phe Lys His His Leu Leu
        675                 680                 685
Arg Asn Pro Ile Val Glu Gln Val Ile Thr Glu Thr Leu Arg Val Val
    690                 695                 700
Lys Asp Ile Trp Leu Lys Tyr Gly Lys Gly Ala Lys Asp Phe Phe Asn
705                 710                 715                 720
Glu Ile His Ile Glu Leu Gly Arg Glu Met Lys Leu Pro Ala Asp Asp
                725                 730                 735
Arg Lys Lys Leu Thr Asn Gln Ile Thr Glu Asn Glu Asn Thr Asn Leu
            740                 745                 750
Arg Ile Lys Ala Leu Leu Thr Glu Met Met Asn Asp His Ala Val Glu
        755                 760                 765
Asn Val Arg Pro Phe Ser Pro Gln Gln Gln Glu Ile Leu Lys Ile Tyr
    770                 775                 780
Glu Asp Gly Val Leu Asn Ser Gly Ile Glu Ile Glu Asp Glu Tyr Leu
785                 790                 795                 800
Lys Ile Ser Lys Thr Ala Gln Pro Ser Pro Ser Asp Leu Lys Arg Tyr
                805                 810                 815
Lys Leu Trp Leu Glu Gln Lys Tyr Lys Ser Pro Tyr Thr Gly Gln Ile
            820                 825                 830
Ile Pro Leu Asn Lys Leu Phe Thr Pro Glu Tyr Glu Ile Glu His Ile

        835                 840                 845
Ile Pro Gln Ser Arg Tyr Phe Asp Asp Ser Phe Ser Asn Lys Ile Ile
    850                 855                 860
Cys Glu Ser Ala Val Asn Lys Leu Lys Asp Asn Tyr Ile Gly Leu Gly
865                 870                 875                 880
Phe Ile Lys Gln Phe Gly Gly Thr Ile Ile Glu Leu Gly Phe Gly Lys
                885                 890                 895
Ser Val Lys Val Phe Asp Ile Asp Glu Tyr Glu Asp Phe Val Lys Lys
            900                 905                 910
His Tyr Ala Asn Asn Arg Ser Lys Arg Asn Lys Leu Leu Leu Glu Asp
        915                 920                 925
Ile Pro Glu Lys Met Ile Glu Arg Gln Ile Asn Asp Thr Arg Tyr Ile
    930                 935                 940
Ser Lys Tyr Ile Ser Gly Ile Leu Ser Asn Ile Val Arg Leu Glu Asp
945                 950                 955                 960
Gly Ser Asp Glu Gly Val Asn Ser Lys Asn Ile Val Pro Gly Asn Gly
                965                 970                 975
Lys Ile Thr Thr Gln Leu Lys Lys Asp Trp Gly Leu Asn Asp Val Trp
            980                 985                 990
Asn Asp Leu Ile Leu Pro Arg Phe Lys Arg Met Asn Gln Leu Thr Asn
        995                 1000                1005
Ser Thr Asp Phe Thr Ala Trp Asn Glu Asn Phe Gln Lys Phe Leu
    1010                1015                1020
Pro Thr Val Pro Ile Glu Tyr Ser Lys Gly Phe Ser Lys Lys Arg
    1025                1030                1035
Ile Asp His Arg His His Ala Leu Asp Ala Leu Val Ile Ala Cys
    1040                1045                1050
Ala Thr Lys Asp His Val Asn Leu Leu Asn Asn Gln Ser Ala Lys
    1055                1060                1065
Ser Glu Thr Lys Arg Tyr Asp Leu Lys Lys Lys Leu Met Lys Phe
    1070                1075                1080
Glu Arg Val Val Tyr Leu His Val Gln Thr Gly Glu Lys Ile Glu
    1085                1090                1095
Arg Glu Val Pro Lys Gln Phe Leu Lys Pro Trp Glu Asn Phe Thr
    1100                1105                1110
Val Asp Val Lys His Asn Leu Asp Thr Val Ile Val Ser Phe Lys
    1115                1120                1125
Gln Asn Leu Arg Val Ile Asn Lys Ala Thr Asn Tyr Tyr Glu Lys
    1130                1135                1140
Tyr Val Glu Lys Asp Gly Ala Arg Asn Lys Thr Arg Val Glu Gln
    1145                1150                1155
Lys Gly Ile Asn Trp Ala Ile Arg Lys Pro Met His Lys Asp Thr
    1160                1165                1170
Val Ser Gly Lys Val Asp Leu Pro Trp Val Lys Val Pro Lys Gly
    1175                1180                1185
Lys Ile Leu Thr Ala Thr Arg Lys Ser Leu Asp Ala Ser Phe Asp
    1190                1195                1200
Leu Lys Ser Ile Ala Ser Ile Thr Asp Thr Gly Ile Gln Lys Ile
    1205                1210                1215
Ile Lys Asn Tyr Leu Met Leu Lys Asp Gly Asn Pro Glu Leu Ala
    1220                1225                1230
Phe Ser Pro Glu Gly Ile Glu Asp Leu Asn Lys Ser Ile Glu Lys
    1235                1240                1245
Tyr Asn Asp Gly Lys Pro His Gln Pro Ile Asn Lys Val Arg Val
    1250                1255                1260
Phe Glu Leu Gly Ser Lys Phe Gln Ile Gly Gln Ala Gly Asn Lys
    1265                1270                1275
Lys Asp Lys Tyr Val Glu Ala Ala Lys Gly Thr Asn Leu Phe Phe
    1280                1285                1290
Ala Val Tyr Glu Asp Glu Asn Gly Lys Arg Ser Tyr Glu Thr Ile
    1295                1300                1305
Pro Leu Asn Glu Val Ile Glu Arg Gln Lys His Gly Leu Pro Val
    1310                1315                1320

Val Asp Leu Thr Ser Thr Asn Asp Phe Tyr Leu Cys Pro Asn Asp
    1325                1330                1335
Leu Val Tyr Ile Leu Ser Asp Asp Glu Arg Asp Asn Met Thr Asn
    1340                1345                1350
Asn Asp Phe Glu Ser Leu Ser Asp Glu Gln Val Lys Arg Ile Tyr
    1355                1360                1365
Lys Phe Val Ser Cys Thr Gly Gly Glu Gly His Phe Ile Pro Tyr
    1370                1375                1380
Ala Ser Ala Thr Glu Ile Ile Lys Asn Glu Asn Gly Thr Asn Ser
    1385                1390                1395
Lys Ser Glu Arg Met Gln Asn Phe Tyr Asp Gly Ser Cys Met Leu
    1400                1405                1410
Asp Lys Asn Ser Lys Pro Ile Met Ile Lys Glu Asn Cys Val Lys
    1415                1420                1425
Leu Lys Val Asp Arg Leu Gly Asn Ile Ser Lys Ile
    1430                1435                1440

<210> 62
<211> 1233
<212> PRT
<213> Artificial sequence

<220>
<223> Cas-Locus 21

<400> 62
Met Gly Ser Arg Ile Ile Pro Leu Ser Thr Ser Asp Lys Glu Glu Phe
1               5                   10                  15
Gln Arg Gly Leu Ser Ile Thr Lys Asn Gln Ser Arg Thr Val Ser Arg
            20                  25                  30
Thr Gln Arg Lys Gly Tyr Asp Arg Lys Gln Leu Lys Lys Ser Asn Leu
        35                  40                  45
Lys Lys Ile Leu Asn Lys Tyr Asn Ile Phe Leu Ser Glu Glu Leu Leu
    50                  55                  60
Lys Leu Pro Met Leu Asp Leu Trp Lys Leu Arg Ser Asp Ser Ala Asn
65                  70                  75                  80
Pro Asn Glu Asn Ile Ser Thr Glu Gln Leu Gly Arg Ile Leu Tyr Met
                85                  90                  95
Leu Asn Gln Lys Arg Gly Tyr Lys Ser Ala Arg Ser Glu Ala Asn Ala
            100                 105                 110
Asp Lys Lys Asp Thr Asp Tyr Val Gln Ala Val Lys Gly Arg Tyr Ala
        115                 120                 125
Gln Leu Lys Glu Arg Asn Gln Thr Ile Gly Gln Tyr Phe His Glu Glu
    130                 135                 140
Leu His Asn Ala Asn Ala Asn Asn Ser Tyr Phe Arg Ile Lys Glu Glu
145                 150                 155                 160
Val Tyr Pro Arg Glu Ala Tyr Ile Glu Glu Phe Asp Thr Ile Ile Asn
                165                 170                 175
Ala Gln Lys Asp Lys His Asp Phe Leu Thr Asp Glu Val Val His Gln
            180                 185                 190
Leu Arg Asn Glu Ile Ile Tyr Phe Gln Arg Lys Leu Lys Ser Gln Lys
        195                 200                 205
Gly Leu Ile Asn Ile Cys Glu Phe Glu Gly Phe Glu Lys Thr Val Phe
    210                 215                 220
Asp Lys Asp Lys Asn Lys His Lys Val Val Ser Val Gly Pro Lys Val
225                 230                 235                 240
Ala Pro Lys Thr Ser Pro Leu Phe Gln Leu Cys Arg Ile Trp Glu Val
                245                 250                 255
Val Asn Thr Ile Thr Leu Lys Ile Lys Asn Pro Glu Gly Ser Lys Tyr
            260                 265                 270
Lys Trp Gly Glu Lys Ile Pro Asn Leu Ile Glu Lys Glu Ile Leu Ala
        275                 280                 285
Asp Tyr Leu Leu Lys Asn Glu Ser Leu Ser Phe Thr Lys Leu Leu Glu

    290                 295                 300
Ile Leu Glu Leu Asn Lys Asn Asp Ile Tyr Val Asn Lys Gln Ile Leu
305                 310                 315                 320
Lys Gly Ile Lys Gly Asn Glu Thr Tyr Ala Val Leu Gln Asn Ile Leu
                325                 330                 335
Gly Asp Asn Glu Leu Leu Asn Phe Asp Val Ser Ile Ile Pro Ser Asn
            340                 345                 350
Tyr Thr Ser Ile Leu Val Asp Lys Lys Thr Gly Glu Ile Leu Glu Glu
        355                 360                 365
Arg Ala Gly Leu Glu Leu Asp Ala Ser Leu Glu Lys Glu Pro Leu Tyr
    370                 375                 380
Gln Leu Trp His Thr Ile Tyr Ser Leu Lys Asp Leu Asp Glu Cys Lys
385                 390                 395                 400
Asn Ala Leu Ile Lys Arg Phe Gly Phe Asp Asp Glu Ile Ala Glu Lys
                405                 410                 415
Leu Ser Arg Ile Asn Phe Asn Lys Gln Ala Phe Ser Asn Lys Ser Asn
            420                 425                 430
Lys Ser Met Arg Lys Ile Leu Pro Phe Leu Met Glu Gly Tyr Asp Tyr
        435                 440                 445
Ser Gln Ser Cys Ser Leu Ala Gly Tyr Asn His Ser Asn Ser Leu Thr
    450                 455                 460
Lys Asp Glu Arg Glu Gln Gln Val Thr Leu Asn Arg Leu Glu Leu Leu
465                 470                 475                 480
Gln Lys Asn Ser Leu Arg Gln Pro Ile Val Glu Lys Ile Leu Asn Gln
                485                 490                 495
Met Ile Asn Val Val Asn Ala Ile Leu Glu Gln Tyr Gly Lys Pro Thr
            500                 505                 510
Glu Ile Arg Val Glu Leu Ala Arg Asp Leu Lys Gln Ser Lys Asp Glu
        515                 520                 525
Arg Asn Asp Ala Asp Leu Gln Asn Ser Lys Asn Lys Lys Leu Asn Glu
    530                 535                 540
Glu Ile Gly Lys Arg Leu Ala Glu Leu Gly Leu Pro Ala Thr Lys Arg
545                 550                 555                 560
Tyr Ile Gln Lys Tyr Lys Phe Ile Phe Pro Ser Leu Ser Lys Asn Leu
                565                 570                 575
Lys Asp Ala Arg Val Asn Asn Gln Cys Ile Tyr Cys Gly Glu Thr Phe
            580                 585                 590
Asn Leu Thr Glu Ala Leu Ser Gly Asp Ala Phe Asp Val Asp His Ile
        595                 600                 605
Val Pro Lys Ala Leu Leu Phe Asp Asp Ser Gln Thr Asn Lys Val Leu
    610                 615                 620
Val His Arg Lys Cys Asn Ser Thr Lys Thr Asn Gln Thr Ala Tyr Asp
625                 630                 635                 640
Tyr Ile Ala Thr Lys Gly Asp Glu Gln Val Arg Ile Tyr Ser Glu Arg
                645                 650                 655
Ile Asp Asp Trp Phe Lys Arg Gly Ile Ile Ser Tyr Ser Lys Met Gln
            660                 665                 670
Arg Leu Lys Val Ser His Lys Glu Tyr Leu Glu Arg Lys Lys Ile Gly
        675                 680                 685
Lys Glu Thr Glu Thr Asp Lys Lys Leu Trp Glu Asn Phe Ile Asp Arg
    690                 695                 700
Gln Leu Arg Asp Thr Gln Tyr Ile Ser Arg Lys Ser Arg Glu Ile Leu
705                 710                 715                 720
Gln Lys Val Cys Asn Asn Val Thr Thr Thr Glu Gly Gly Val Thr Ala
                725                 730                 735
Lys Leu Arg Asn Leu Trp Gly Trp Asp Asp Val Leu Met Asn Leu Gln
            740                 745                 750
Met Pro Lys Tyr Lys Glu Leu Gly Gln Thr Val Ile Lys Glu Trp Thr
        755                 760                 765
Ser Asp His Gly Lys Arg Lys His Gln Lys Glu Glu Ile Glu Asn Trp
    770                 775                 780
Thr Lys Arg Asp Asp His Arg His His Ala Ile Asp Ala Leu Val Ile
785                 790                 795                 800

Ala Cys Thr Arg Gln Gly Phe Ile Gln Arg Ile Asn Thr Leu Asn Ser
                805                 810                 815
Ser Asp Thr Lys Asp Leu Met Met Lys Glu Val Glu Lys Ala Lys Ile
            820                 825                 830
Glu Phe Asn Glu Lys Ser Thr Leu Leu Glu Lys Tyr Leu Lys Thr Gln
        835                 840                 845
Lys Pro Phe Thr Thr Glu Gln Val Met Lys Glu Ala Asp Lys Ile Leu
    850                 855                 860
Val Ser Phe Lys Ala Gly Lys Lys Val Ala Thr Ile Thr Lys Phe Lys
865                 870                 875                 880
Ala Thr Gly Lys Asn Lys Asp Thr Gly Val Ile Val Pro Arg Gly Ala
                885                 890                 895
Leu His Glu Gln Asn Val Tyr Gly Lys Ile Lys Ala Ile Glu Lys Asp
            900                 905                 910
Lys Ser Leu Lys Tyr Leu Phe Glu Asn Ser Gly Lys Ile Val Asn Pro
        915                 920                 925
Lys Ile Lys Asn Leu Val Glu Ile Arg Leu Ser Glu Asn Glu Asn Ser
    930                 935                 940
Ser Lys Met Ala Leu Ser Thr Leu Lys Lys Asn Pro Ile Phe Leu Asn
945                 950                 955                 960
Glu Glu Lys Thr Asp Leu Leu Glu Lys Ala Ser Cys Tyr Glu Tyr Val
                965                 970                 975
Thr Val Arg Lys Tyr Pro Leu Asn Thr Ile Lys Ala Ser Gln Ala Asp
            980                 985                 990
Asp Ile Val Asp Glu Arg Val Lys  Phe Leu Val Lys Glu  Arg Leu Ala
        995                 1000                1005
Gln Phe  Gly Asn Lys Glu Lys  Glu Ala Phe Lys Asp  Thr Leu Trp
    1010                1015                1020
Phe Asn  Glu Glu Lys Gln Ile  Pro Ile Ile Thr Val  Arg Leu Phe
    1025                1030                1035
Ala Arg  Pro Asp Ala Asn Asn  Leu Gln Ala Met Lys  Lys Asp Glu
    1040                1045                1050
Asn Gly  Lys Glu Ile Gly Phe  Val Val Leu Gly Asn  Asn His His
    1055                1060                1065
Ile Ala  Ile Tyr Glu Asp Lys  Asp Asn Lys Leu Val  Gln His Ser
    1070                1075                1080
Cys Thr  Phe Trp His Ala Val  Glu Arg Lys Lys Tyr  Asn Ile Pro
    1085                1090                1095
Pro Val  Ile Lys Asn Ser Ser  Glu Ile Trp Asn Gln  Leu Leu Asn
    1100                1105                1110
Lys Glu  Phe Pro Glu Thr Phe  Leu Asp Lys Leu Pro  Ala Asp Asn
    1115                1120                1125
Leu Glu  Leu Lys Phe Ser Met  Gln Gln Asn Glu Met  Phe Ile Leu
    1130                1135                1140
Gly Leu  Pro Gln Glu Glu Phe  Glu Glu Ala Ile Lys  Asn Asn Asp
    1145                1150                1155
Lys Ser  Val Leu Ser Lys His  Met Tyr Leu Val Trp  Ser Ile Ala
    1160                1165                1170
Asp Gly  Asp Tyr Trp Phe Arg  His His Leu Glu Thr  Lys Asn Thr
    1175                1180                1185
Glu Leu  Lys Lys Ile Asn Gly  Ala Lys Glu Ser Lys  Arg Phe Phe
    1190                1195                1200
Arg Leu  Gln Ser Leu Lys Ala  Val Glu Asn Leu Thr  Pro Ile Lys
    1205                1210                1215
Val Arg  Leu Asn His Leu Gly  Glu Ile Thr Lys Ile  Gly Glu Tyr
    1220                1225                1230

<210>  63
<211>  1268
<212>  PRT
<213>  Artificial sequence

<220>

<223>  Cas-Locus 22

<400>  63
Met Met Ile Lys Asn Ile Leu Gly Leu Asp Leu Gly Thr Asn Ser Ile
1               5                   10                  15
Gly Trp Ala Leu Ile Glu Val Asp Glu Asn Asn Ile Pro Ile Arg Ile
            20                  25                  30
Val Ala Met Gly Ser Arg Ile Ile Pro Leu Ser Thr Ser Asp Lys Glu
        35                  40                  45
Glu Phe Gln Arg Gly Leu Ser Ile Thr Lys Asn Gln Ser Arg Thr Val
    50                  55                  60
Ser Arg Thr Gln Arg Lys Gly Tyr Asn Arg Lys Gln Leu Lys Lys Asn
65                  70                  75                  80
Asp Leu Lys Lys Ile Leu Asn Lys Tyr Asn Ile Phe Pro Ser Glu Glu
                85                  90                  95
Leu Leu Lys Leu Pro Met Leu Asp Leu Trp Lys Leu Arg Ser Asp Ser
            100                 105                 110
Ala Asn Pro Asn Glu Asn Ile Ser Ala Glu Gln Leu Gly Arg Ile Leu
        115                 120                 125
Tyr Met Leu Asn Gln Lys Arg Gly Tyr Lys Ser Ala Arg Ser Glu Ala
    130                 135                 140
Asn Ala Asp Lys Lys Asp Thr Asp Tyr Val Gln Glu Val Lys Gly Arg
145                 150                 155                 160
Tyr Ala Gln Leu Lys Glu Arg Glu Gln Thr Ile Gly Gln Tyr Phe Tyr
                165                 170                 175
Glu Gly Leu Ser Thr Ala Phe Ser Asp Lys Asp Asn Arg Gln Tyr Phe
            180                 185                 190
Arg Ile Lys Glu Glu Val Tyr Pro Arg Glu Ala Tyr Val Glu Glu Phe
        195                 200                 205
Asp Thr Ile Ile Asn Ala Gln Lys Asp Lys His Asp Phe Leu Thr Asp
    210                 215                 220
Lys Val Val His Gln Leu Arg Asn Glu Ile Ile Tyr Phe Gln Arg Lys
225                 230                 235                 240
Leu Lys Ser Gln Lys Gly Leu Val Ser Ile Cys Glu Phe Glu Gly Phe
                245                 250                 255
Glu Lys Thr Val Phe Asp Lys Glu Lys Asn Lys Asn Lys Val Val Phe
            260                 265                 270
Ile Gly Pro Lys Val Ala Pro Lys Thr Ser Pro Leu Phe Gln Leu Cys
        275                 280                 285
Arg Ile Trp Glu Val Val Asn Thr Ile Thr Leu Lys Ile Lys Asn Pro
    290                 295                 300
Glu Gly Ser Lys Tyr Lys Trp Gly Glu Lys Ile Pro Asp Leu Lys Glu
305                 310                 315                 320
Lys Glu Ile Leu Ala Asp Tyr Leu Phe Lys Asn Glu Ser Val Ser Phe
                325                 330                 335
Ser Lys Leu Leu Glu Ile Leu Gly Leu Lys Lys Glu Asn Val Tyr Val
            340                 345                 350
Asn Lys Gln Ile Leu Lys Gly Ile Lys Gly Asn Glu Thr Tyr Ala Ala
        355                 360                 365
Leu Tyr Asn Val Leu Gly Asp Asn Glu Leu Leu Asn Phe Asn Val Ser
    370                 375                 380
Ile Ile Thr Ser Asn His Thr Ala Ile Leu Ile Asp Lys Lys Thr Gly
385                 390                 395                 400
Glu Ile Leu Glu Glu Arg Ala Gly Leu Glu Leu Asp Ala Phe Leu Glu
                405                 410                 415
Lys Glu Pro Leu Tyr Gln Leu Trp His Thr Ile Tyr Ser Leu Lys Asp
            420                 425                 430
Leu Asp Glu Cys Lys Asn Ala Leu Ile Lys Arg Phe Gly Phe Glu Glu
        435                 440                 445
Asp Ile Ala Glu Lys Leu Ser Cys Ile Asp Phe Asn Lys Gln Ala Phe
    450                 455                 460
Gly Asn Lys Ser Asn Lys Ser Met Arg Lys Ile Leu Pro Phe Leu Met
465                 470                 475                 480

Glu Gly Tyr Asp Tyr Ser Gln Ser Cys Ser Leu Ala Gly Tyr Asn His
                485                 490                 495
Ser Asn Ser Leu Thr Lys Asp Glu Arg Glu Gln Gln Ile Thr Leu Asp
            500                 505                 510
Arg Leu Glu Leu Leu Ser Lys Asn Ser Leu Arg Gln Pro Ile Val Glu
        515                 520                 525
Lys Ile Leu Asn Gln Met Ile Asn Val Val Asn Ala Ile Leu Glu Gln
    530                 535                 540
Tyr Gly Lys Pro Ser Glu Ile Arg Val Glu Leu Ala Arg Gln Leu Lys
545                 550                 555                 560
Gln Ser Lys Asp Glu Arg Asn Glu Thr Asp Leu Gln Asn Ser Lys Asn
                565                 570                 575
Lys Lys Leu Asn Glu Glu Ile Gly Lys Arg Leu Ala Glu Leu Gly Leu
            580                 585                 590
Pro Val Thr Lys Arg Tyr Ile Gln Lys Tyr Lys Phe Ile Phe Pro Ser
        595                 600                 605
Leu Ser Lys Asn Leu Lys Asp Ala His Val Asn Asn Gln Cys Ile Tyr
    610                 615                 620
Cys Gly Glu Thr Phe Asn Leu Thr Glu Ala Leu Ser Gly Asp Ala Tyr
625                 630                 635                 640
Asp Val Asp His Ile Val Pro Lys Thr Leu Leu Phe Asp Asp Ser Gln
                645                 650                 655
Thr Asn Lys Val Leu Val His Arg Lys Cys Asn Ser Thr Lys Thr Asn
            660                 665                 670
Gln Thr Ala Tyr Asp Tyr Ile Ala Thr Lys Gly Asp Glu Gln Val Arg
        675                 680                 685
Val Tyr Ser Glu Arg Val Asp Asp Trp Phe Lys Arg Gly Ile Ile Ser
    690                 695                 700
Tyr Ser Lys Met Gln Arg Leu Lys Val Ser His Lys Glu Tyr Leu Glu
705                 710                 715                 720
Arg Lys Lys Leu Gly Lys Glu Thr Glu Thr Asp Lys Lys Leu Trp Glu
                725                 730                 735
Asn Phe Ile Asp Arg Gln Leu Arg Asp Thr Gln Tyr Ile Ser Arg Lys
            740                 745                 750
Ser Arg Glu Ile Leu Gln Lys Val Cys Asn Asn Val Thr Thr Thr Glu
        755                 760                 765
Gly Gly Val Thr Ala Lys Leu Arg Asn Val Trp Gly Trp Asp Asp Val
    770                 775                 780
Leu Met Asn Leu Gln Leu Pro Lys Tyr Lys Glu Leu Gly Gln Thr Phe
785                 790                 795                 800
Thr Lys Glu Trp Thr Ser Lys His Gly Lys Arg Lys His Gln Lys Glu
                805                 810                 815
Glu Ile Glu Asn Trp Thr Lys Arg Asp Asp His Arg His His Ala Ile
            820                 825                 830
Asp Ala Leu Ile Ile Ala Cys Thr Lys Gln Gly Phe Ile Gln Arg Ile
        835                 840                 845
Asn Thr Leu Asn Ser Ser Glu Thr Lys Asp Leu Met Val Lys Glu Val
    850                 855                 860
Glu Gly Ala Lys Ile Glu Phe Asn Glu Lys Ala Thr Leu Leu Glu Lys
865                 870                 875                 880
Tyr Leu Lys Thr Gln Lys Pro Phe Asp Thr His Glu Val Met Lys Glu
                885                 890                 895
Ala Asp Lys Ile Leu Ile Ser Phe Lys Ala Gly Lys Lys Val Ala Thr
            900                 905                 910
Ile Thr Lys Phe Lys Ala Thr Gly Lys Asn Lys Glu Thr Gly Val Ile
        915                 920                 925
Val Pro Arg Gly Ala Leu His Glu Gln Ser Val Tyr Gly Lys Ile Lys
    930                 935                 940
Val Ile Glu Asn Asp Lys Pro Leu Lys Tyr Leu Phe Glu Asn Ser Asp
945                 950                 955                 960
Lys Ile Val Asn Pro Lys Ile Lys Asn Leu Val Glu Thr Arg Leu Thr
                965                 970                 975
Glu Asn Glu Asn Asn Phe Lys Lys Ala Leu Ser Thr Leu Lys Lys Asn

980                 985                 990
Pro Ile Phe Phe Asn Glu Glu Lys  Thr Glu Ile Leu Glu  Lys Ala Ser
        995                 1000                1005
Cys Tyr  Asn Asp Ala Thr Val  Leu Lys Tyr Lys Leu  Gln Asn Ile
    1010                1015                1020
Lys Ala  Ser Gln Ala Asp Asp  Ile Val Asp Glu Arg  Val Lys Ser
    1025                1030                1035
Leu Val  Lys Glu Arg Leu Ala  Gln Phe Gly Asn Lys  Glu Lys Glu
    1040                1045                1050
Ala Phe  Lys Asp Ile Leu Trp  Leu Asn Glu Glu Lys  Gln Ile Pro
    1055                1060                1065
Ile Ile  Thr Val Arg Leu Phe  Ala Arg Pro Asp Ala  Asp Asn Leu
    1070                1075                1080
Gln Ala  Ile Lys Lys Asp Lys  Tyr Gly Asn Glu Ile  Gly Phe Val
    1085                1090                1095
Val Thr  Gly Asn Asn His His  Ile Ala Ile Tyr Glu  Asp Lys Asp
    1100                1105                1110
Asn Lys  Leu Val Gln His Thr  Cys Thr Phe Trp His  Ala Ile Glu
    1115                1120                1125
Arg Lys  Lys Tyr Asn Ile Pro  Ala Val Ile Gly Asn  Ser Ser Asp
    1130                1135                1140
Ile Trp  Asn Gln Leu Leu Asp  Lys Glu Leu Pro Ile  Ser Phe Leu
    1145                1150                1155
Asn Lys  Leu Pro Met Asp Lys  Leu Asn Leu Lys Phe  Ser Val Gln
    1160                1165                1170
Gln Asn  Glu Met Phe Ile Leu  Gly Leu Ser Gln Asp  Glu Phe Asp
    1175                1180                1185
Glu Thr  Ile Lys Asn Asn Asp  Lys Ser Leu Leu Ser  Lys Tyr Leu
    1190                1195                1200
Tyr Leu  Val Trp Ser Ile Ala  Glu Gly Asp Tyr Trp  Phe Arg His
    1205                1210                1215
His Leu  Glu Thr Lys Asn Thr  Glu Leu Lys Lys Thr  Asp Gly Ala
    1220                1225                1230
Lys Glu  Ser Lys Arg Tyr Tyr  Arg Leu Ser Ser Lys  Gly Phe Ile
    1235                1240                1245
Asp Leu  Asn Pro Ile Lys Val  Arg Leu Asn His Leu  Gly Glu Ile
    1250                1255                1260
Thr Lys  Ile Gly Glu
    1265

<210>  64
<211>  1264
<212>  PRT
<213>  Artificial sequence

<220>
<223>  Cas-Locus 23

<400>  64
Met Lys Lys Ile Leu Gly Leu Asp Leu Gly Thr Asn Ser Ile Gly Trp
1               5                   10                  15
Ala Leu Ile Glu Ile Asp Lys Asn Asn Ile Pro Ile Ser Ile Ile Ala
            20                  25                  30
Met Gly Ser Arg Ile Ile Pro Leu Ser Ser Ser Asp Lys Glu Glu Phe
        35                  40                  45
Gln Arg Gly Leu Ser Ile Thr Lys Asn Gln Ser Arg Thr Val Ser Arg
    50                  55                  60
Thr Gln Arg Lys Gly Tyr Asp Arg Lys Gln Leu Lys Lys Ser Asp Leu
65                  70                  75                  80
Lys Lys Ile Leu Asn Lys Tyr Asn Ile Phe Pro Ser Glu Glu Leu Leu
                85                  90                  95
Lys Leu Pro Met Leu Asp Leu Trp Lys Leu Arg Ser Asp Ser Ala Asn
            100                 105                 110

Pro Asn Glu Asp Ile Ser Ala Glu Gln Leu Gly Arg Ile Leu Tyr Met
        115                 120                 125
Leu Asn Gln Lys Arg Gly Tyr Lys Ser Ala Arg Ser Glu Ala Asn Ala
    130                 135                 140
Asp Lys Lys Asp Thr Asp Tyr Val Gln Ala Val Lys Gly Arg Tyr Ala
145                 150                 155                 160
His Leu Lys Asp Arg Thr Gln Thr Ile Gly Gln Tyr Phe Phe Glu Glu
                165                 170                 175
Leu Asp Asn Ala Asn Thr Asn Asn Ser Tyr Phe Arg Ile Lys Glu Glu
            180                 185                 190
Val Tyr Pro Arg Glu Ala Tyr Thr Glu Glu Phe Asp Ala Ile Ile Asn
        195                 200                 205
Ala Gln Lys Gly Lys His Asp Phe Leu Thr Asp Glu Val Val His Gln
    210                 215                 220
Leu Arg Asn Glu Ile Ile Tyr Phe Gln Arg Lys Leu Lys Ser Gln Lys
225                 230                 235                 240
Gly Leu Val Ser Ile Cys Glu Phe Glu Gly Phe Glu Lys Thr Ile Phe
                245                 250                 255
Asp Lys Glu Lys Asp Lys Asn Lys Val Val Leu Val Gly Pro Lys Val
            260                 265                 270
Ala Pro Lys Thr Ser Pro Leu Phe Gln Leu Cys Arg Ile Trp Glu Val
        275                 280                 285
Val Asn Thr Ile Thr Leu Lys Ile Lys Asn Pro Glu Gly Ser Lys Tyr
    290                 295                 300
Lys Trp Gly Glu Lys Ile Pro Asn Leu Lys Glu Lys Glu Ile Leu Ala
305                 310                 315                 320
Asp Tyr Leu Phe Lys Asn Glu Ser Leu Ser Phe Ser Lys Leu Leu Glu
                325                 330                 335
Ile Leu Glu Leu Lys Lys Glu Asn Val Tyr Val Asn Lys Gln Ile Leu
            340                 345                 350
Lys Gly Leu Lys Gly Asn Glu Thr Tyr Ser Thr Ile His Ser Ile Leu
        355                 360                 365
Gly Asp Asn Glu Leu Leu Asn Phe Asp Val Ser Ile Ile Thr Ser Asn
    370                 375                 380
His Thr Ser Met Leu Val Asp Lys Lys Thr Gly Glu Ile Leu Glu Glu
385                 390                 395                 400
Arg Ala Gly Leu Glu Leu Asp Ala Ser Leu Glu Arg Glu Pro Leu Tyr
                405                 410                 415
Gln Leu Trp His Thr Ile Tyr Ser Leu Lys Asp Leu Asp Glu Cys Lys
            420                 425                 430
Asn Ala Leu Ile Lys Arg Phe Gly Phe Asp Asp Glu Ile Ala Lys Lys
        435                 440                 445
Leu Ser Arg Ile Asp Phe Asn Lys Gln Ala Phe Gly Asn Lys Ser Asn
    450                 455                 460
Lys Ser Met Arg Lys Ile Leu Pro Phe Leu Met Glu Gly Tyr Asp Tyr
465                 470                 475                 480
Ser Gln Ser Cys Ser Leu Ala Gly Tyr Asn His Ser Asn Ser Leu Thr
                485                 490                 495
Lys Asp Glu Arg Glu Gln Leu Ile Thr Leu Asp Gln Leu Ala Leu Leu
            500                 505                 510
Pro Lys Asn Ser Leu Arg Gln Pro Ile Val Glu Lys Ile Leu Asn Gln
        515                 520                 525
Met Ile Asn Val Val Asn Ala Ile Leu Glu Gln Tyr Gly Lys Pro Ser
    530                 535                 540
Glu Ile Arg Val Glu Leu Ala Arg Glu Leu Lys Gln Ser Lys Asp Glu
545                 550                 555                 560
Arg Asn Asp Thr Asp Leu Gln Asn Ser Lys Asn Lys Lys Leu Asn Glu
                565                 570                 575
Glu Ile Arg Lys Arg Leu Val Glu Leu Gly Leu Pro Val Thr Lys Arg
            580                 585                 590
Tyr Ile Gln Lys Tyr Lys Phe Ile Phe Pro Ser Leu Ser Lys Asn Leu
        595                 600                 605
Lys Asp Ala His Val Asn Asn Gln Cys Ile Tyr Cys Gly Glu Thr Phe

610                 615                 620
Asn Leu Thr Glu Ala Leu Ser Gly Asp Ala Tyr Asp Val Asp His Ile
625                 630                 635                 640
Val Pro Lys Ala Leu Leu Phe Asp Asp Ser Gln Thr Asn Lys Val Leu
                645                 650                 655
Val His Arg Lys Cys Asn Ser Thr Lys Asp Lys Leu Thr Ala Tyr Asp
            660                 665                 670
Tyr Ile Ser Thr Lys Gly Ile Glu Gln Val Arg Val Tyr Ser Glu Arg
        675                 680                 685
Val Asp Asp Trp Phe Lys Arg Gly Ile Ile Ser Tyr Ser Lys Met Gln
    690                 695                 700
Arg Leu Lys Val Ser His Glu Glu Tyr Leu Glu Arg Lys Lys Ile Gly
705                 710                 715                 720
Lys Glu Thr Glu Thr Asp Lys Lys Leu Trp Glu Asn Phe Ile Glu Arg
                725                 730                 735
Gln Leu Arg Asp Thr Gln Tyr Ile Ser Arg Lys Ala Arg Glu Ile Leu
            740                 745                 750
His Lys Leu Cys Asn Asn Val Thr Thr Thr Glu Gly Gly Val Thr Ala
        755                 760                 765
Lys Leu Arg Asn Leu Trp Gly Trp Asp Asp Val Leu Met Asn Leu Gln
    770                 775                 780
Met Pro Lys Tyr Lys Glu Leu Gly Gln Thr Val Ile Lys Glu Trp Ala
785                 790                 795                 800
Ser Asp His Gly Lys Arg Lys His Gln Lys Glu Glu Val Glu Asn Trp
                805                 810                 815
Thr Lys Arg Asp Asp His Arg His His Ala Ile Asp Ala Leu Val Ile
            820                 825                 830
Ala Cys Thr Lys Gln Gly Tyr Ile Gln Arg Ile Asn Thr Leu Asn Ser
        835                 840                 845
Ser Asp Thr Arg Asp Leu Met Ile Lys Glu Val Glu Glu Ala Lys Ile
    850                 855                 860
Glu Phe Asn Glu Lys Ser Thr Leu Leu Glu Lys Tyr Leu Lys Thr Gln
865                 870                 875                 880
Glu Pro Phe Thr Thr Gln Glu Val Met Lys Glu Ala Asp Lys Ile Leu
                885                 890                 895
Ile Ser Phe Lys Ala Gly Lys Lys Val Ala Thr Ile Thr Lys Tyr Lys
            900                 905                 910
Ala Thr Gly Lys Asn Lys Asn Thr Gly Val Leu Val Pro Arg Gly Ala
        915                 920                 925
Leu His Glu Gln Asn Val Tyr Gly Lys Ile Lys Val Ile Glu Lys Asp
    930                 935                 940
Lys Ser Leu Lys Tyr Leu Phe Glu Asn Ser Asp Lys Ile Val Asn Ser
945                 950                 955                 960
Ser Ile Lys Lys Leu Ile Glu Val Arg Leu Leu Asp Asn Glu Asn Asn
                965                 970                 975
Ser Lys Leu Ala Leu Ser Ser Leu Lys Lys Asn Pro Ile Phe Leu Asn
            980                 985                 990
Glu Glu Lys Lys Glu Val Leu Glu  Lys Ala Ser Cys Tyr  Glu Tyr Val
        995                 1000                 1005
Thr Val  Lys Lys Tyr Pro Leu  Asn Thr Ile Lys Ala  Asn Gln Ala
    1010                 1015                 1020
Asp Asp  Ile Val Asp Glu Arg  Val Lys Phe Leu Val  Lys Glu Arg
    1025                 1030                 1035
Leu Ser  Gln Phe Gly Asn Lys  Glu Lys Glu Ala Phe  Lys Asp Thr
    1040                 1045                 1050
Leu Trp  Phe Asn Glu Glu Lys  Gln Ile Pro Ile Ile  Thr Val Arg
    1055                 1060                 1065
Leu Phe  Ala Arg Pro Asp Ala  Asn Asn Leu Gln Ala  Ile Lys Lys
    1070                 1075                 1080
Asp Lys  Asn Gly Lys Glu Ile  Gly Phe Val Val Thr  Gly Asn Asn
    1085                 1090                 1095
His His  Ile Ala Ile Tyr Glu  Asp Lys Asp Asn Lys  Leu Ile Gln
    1100                 1105                 1110

His Ser Cys Thr Phe Trp His Ala Val Glu Arg Lys Lys Tyr Asn
    1115                1120                1125
Val Pro Thr Val Ile Lys Asn Ser Ser Asp Val Trp Asn Gln Leu
    1130                1135                1140
Leu His Lys Glu Leu Pro Glu Ser Phe Leu Asn Lys Leu Pro Ala
    1145                1150                1155
Asp Tyr Leu Asn Leu Lys Phe Ser Met Gln Gln Asn Glu Met Phe
    1160                1165                1170
Ile Leu Gly Leu Ser Gln Glu Glu Phe Asp Gln Ala Ile Lys Lys
    1175                1180                1185
Asn Asn Lys Pro Leu Leu Ser Lys His Leu Tyr Leu Val Trp Ser
    1190                1195                1200
Ile Thr Asp Gly Asp Tyr Phe Phe Arg His His Leu Glu Thr Lys
    1205                1210                1215
Asn Thr Glu Leu Lys Lys Thr Asp Gly Ala Lys Glu Gly Lys Arg
    1220                1225                1230
Tyr Phe Arg Leu Ser Thr Lys Gly Leu Thr Asp Leu Asn Pro Ile
    1235                1240                1245
Lys Val Arg Leu Asn His Leu Gly Glu Ile Thr Lys Ile Gly Glu
    1250                1255                1260
Tyr

<210>  65
<211>  1464
<212>  PRT
<213>  Artificial sequence

<220>
<223>  Cas-Locus 24

<400>  65
Met Ala Lys Ile Leu Gly Leu Asp Leu Gly Thr Asn Ser Ile Gly Trp
1               5                   10                  15
Ala Ile Val Asp Lys Asp Gly Asn Asp Phe Ser Leu Val Asp Lys Gly
            20                  25                  30
Val Arg Ile Phe Ser Glu Gly Val Lys Ser Glu Lys Gly Ile Glu Ser
        35                  40                  45
Ser Arg Ala Ala Glu Arg Thr Gly Tyr Arg Ser Ala Arg Lys Ile Lys
    50                  55                  60
Tyr Arg Arg Lys Leu Arg Lys Tyr Glu Thr Leu Lys Val Leu Ser Ser
65                  70                  75                  80
Asn Gly Met Cys Pro Leu Ser Val Glu Glu Val Glu Asp Trp Lys Lys
                85                  90                  95
Ser Gly Phe Lys Lys Tyr Pro Leu Asn Pro Glu Phe Leu Lys Trp Leu
            100                 105                 110
Arg Thr Asp Glu Tyr Gln His Ile Asn Pro Tyr Tyr Phe Arg Asp Arg
        115                 120                 125
Ala Ser Lys Gln Lys Val Ala Leu Phe Glu Leu Gly Arg Ala Leu Tyr
    130                 135                 140
His Ile Ala Gln Arg Arg Gly Phe Leu Ser Asn Arg Leu Asp Gln Ser
145                 150                 155                 160
Ala Glu Gly Val Phe Glu Glu His Asn Pro Gln Ile Gln Ser Leu Ile
                165                 170                 175
Glu Asp Leu Asp Asp Ser Lys Leu Ile Leu Asp Glu Leu Lys Asn Tyr
            180                 185                 190
Tyr Val Asp Lys Gly Ile Ile Glu Glu Ala Glu Lys Gly Val Phe Lys
        195                 200                 205
Lys Asp Leu Asp Glu Gly Glu Lys Lys Leu Lys Ser Leu Tyr Asn Ser
    210                 215                 220
Leu Val Gly Ile Ile Lys Lys Asn Glu Asn Asn Val Glu Lys Cys Lys
225                 230                 235                 240
Glu Glu Phe Ile Ala Arg Leu Asn Lys Lys Glu Asp Leu Gly Lys Val

245                 250                 255
Lys Gly Lys Ile Lys Asp Ile Ser Gln Ala Met Leu Asp Gly Asn Phe
            260                 265                 270
Lys Thr Leu Gly Gln Tyr Phe Phe Ser Leu Tyr Asn Lys Gly Lys Ile
        275                 280                 285
Arg Asn Gln Tyr Thr Ser Arg Glu Glu His Tyr Leu Glu Glu Phe Ile
    290                 295                 300
Thr Ile Cys Lys Ile Gln Gly Ile Glu Gly Ile Asp Asp Lys Glu Lys
305                 310                 315                 320
Leu Pro Glu Lys Lys Phe Thr Gly Leu Ala Lys Asp Leu Tyr Lys Ala
                325                 330                 335
Ile Phe Phe Gln Arg Pro Leu Lys Ser Gln Lys Gly Leu Ile Gly Lys
            340                 345                 350
Cys Ser Phe Glu Lys Ser Lys Ser Arg Cys Ala Val Ser His Pro Asp
        355                 360                 365
Phe Glu Glu Tyr Arg Met Trp Thr Tyr Leu Asn Thr Ile Lys Ile Gly
    370                 375                 380
Thr Gln Ser Glu Lys Thr Leu Arg Phe Leu Thr Gln Asp Glu Lys Leu
385                 390                 395                 400
Lys Leu Val Pro Lys Phe Tyr Arg Lys Asn Asp Phe Asn Phe Glu Val
                405                 410                 415
Leu Ala Lys Glu Leu Val Glu Lys Gly Ala Ser Phe Gly Phe Tyr Lys
            420                 425                 430
Ser Ser Lys Lys Asn Asp Phe Phe Tyr Trp Phe Asn Tyr Lys Pro Thr
        435                 440                 445
Asp Ser Val Ala Ala Cys Gln Val Ser Ala Ser Leu Lys Asn Val Ile
    450                 455                 460
Gly Glu Asp Trp Lys Thr Lys Ile Phe Thr Tyr Lys Thr Leu Asn Ala
465                 470                 475                 480
Lys Glu Glu Glu Val Thr Lys Ser Val Asp Tyr Lys Asp Leu Trp His
                485                 490                 495
Leu Leu Ser Val Ala Thr Ser Asp Ile Tyr Leu Glu Glu Tyr Ala Lys
            500                 505                 510
Asp Lys Leu Gly Phe Asp Asp Lys Asn Ala Lys Ala Phe Ser Lys Ile
        515                 520                 525
Lys Leu Lys Lys Asp Phe Ala Ser Leu Ser Ser Ser Ala Ile Thr Lys
    530                 535                 540
Ile Leu Pro Tyr Val Lys Glu Gly Leu Leu Tyr Ser His Ala Val Phe
545                 550                 555                 560
Met Ala Asn Ile Glu Asn Ile Val Asp Glu Asn Ile Trp Asn Asn Leu
                565                 570                 575
Asp Gln Arg Asn Tyr Ile Gln Ser Lys Ile Ala Glu Ile Ile Glu Gly
            580                 585                 590
Tyr Thr Phe Glu Lys Thr Gly Leu Glu Val Val Asn Ser Leu Ile Lys
        595                 600                 605
Glu Tyr Lys Thr Glu Asn Asp Tyr Ser Lys Lys Val Tyr Tyr Ser Lys
    610                 615                 620
Glu Ala Glu Pro Glu Phe Glu Lys Asp Leu Lys Gln Lys Leu Val Val
625                 630                 635                 640
Phe Tyr Lys Ser Asn Asp Ile Glu Asp Glu Asn Ile Lys Gln Ala Ile
                645                 650                 655
Phe Asn Asp Leu Tyr Ser Val Leu Ile Asp Gln Leu Lys Lys Tyr Glu
            660                 665                 670
Tyr Ile Lys Ile Gln Arg Leu Asp Glu Lys Val Leu Glu Phe Leu Lys
        675                 680                 685
Gly Glu Asn Glu Asp Gly Glu Val Phe Cys Asn Asn Pro Lys Gln Leu
    690                 695                 700
Lys Lys Leu Tyr His Pro Ser Asp Ile Glu Ile Phe Lys Lys Gln Lys
705                 710                 715                 720
Val Lys Asp Lys Asp Gly Lys Glu Thr Gly Gln Leu Ile Leu Gly Ser
                725                 730                 735
Pro Leu Thr Ser Ser Ile Lys Asn Pro Met Ala Met Arg Ala Leu His
            740                 745                 750

Gln Leu Arg Lys Val Leu Asn Thr Leu Ile Leu Glu Gly Gln Val Asp
        755                 760                 765
Glu Ser Thr Arg Ile His Ile Glu Met Ala Arg Glu Leu Asn Asp Ala
    770                 775                 780
Asn Arg Arg Lys Gly Ile Gln Asp Tyr Gln Lys Glu Asn Lys Asp Phe
785                 790                 795                 800
Arg Asp Asn Ala Ile Lys Glu Ile Lys Lys Leu Tyr Leu Asp Glu Cys
                805                 810                 815
Gly Lys Glu Val Glu Pro Thr Glu Asp Asp Ile Leu Arg Tyr Gln Leu
            820                 825                 830
Trp Ile Glu Gln Asp Lys Cys Glu Ile Tyr Glu Gln Gly Lys Asn Ile
        835                 840                 845
Ser Ile Cys Asp Ile Ile Gly Ser Asn Pro Ala Tyr Asp Ile Glu His
    850                 855                 860
Thr Ile Pro Arg Ser Ile Ser Gln Asp Asn Ser Gln Met Asn Lys Thr
865                 870                 875                 880
Leu Cys Ser Gln Arg Phe Asn Arg Asp Val Lys Lys Gln Lys Met Pro
                885                 890                 895
Ile Glu Leu Ser Asn His Leu Glu Ile Leu Pro Arg Ile Ala His Trp
            900                 905                 910
Lys Glu Glu Ala Asp Asn Leu Ser Arg Glu Ile Glu Ile Ile Ser Arg
        915                 920                 925
Ser Ile Lys Ala Ala Ser Thr Lys Glu Ile Lys Asp Lys Lys Ile Arg
    930                 935                 940
Arg Arg His Tyr Leu Thr Leu Lys Arg Asp Tyr Leu Gln Gly Lys Tyr
945                 950                 955                 960
Asp Arg Phe Ile Trp Lys Glu Pro Lys Val Gly Phe Lys Asn Ser Gln
                965                 970                 975
Ile Pro Asp Thr Gly Ile Ile Thr Lys Tyr Ala Gln Ala Tyr Leu Lys
            980                 985                 990
Ser Tyr Phe Lys Arg Val Glu Ser Val Lys Gly Gly Met Val Ala Glu
        995                 1000                1005
Phe Arg Lys Gln Trp Gly Ile Gln Glu Ser Phe Ile Asp Glu Asn
    1010                1015                1020
Gly Phe Lys Gln Tyr Lys Glu Lys Asp Arg Ser Lys His Thr His
    1025                1030                1035
His Thr Ile Asp Ala Ile Thr Ile Ala Cys Met Thr Lys Asp Lys
    1040                1045                1050
Tyr Asp Val Leu Ala Lys Ala Trp Thr Ser Glu Asp Glu Gln Asp
    1055                1060                1065
Lys Leu Lys Ala Lys Ala Leu Met Ala Asp Ala Lys Pro Trp Lys
    1070                1075                1080
Thr Phe Lys Glu Asp Leu Leu Lys Ile Glu Asp Glu Ile Leu Val
    1085                1090                1095
Ser His Tyr Thr Pro Asp Asn Val Lys Lys Gln Ser Lys Lys Ile
    1100                1105                1110
Ile Arg Val Arg Gly Lys Lys Gln Phe Ile Ala Glu Ile Glu Arg
    1115                1120                1125
Asp Val Lys Gly Lys Ala Ile Gln Lys Lys Asp Ala Asn Gly Lys
    1130                1135                1140
Val Val Tyr Lys Leu Asn Asp Lys Gly Glu Lys Ile Pro Arg Leu
    1145                1150                1155
Gln Gln Gly Asp Thr Ile Arg Gly Ser Leu His Gln Asp Ser Ile
    1160                1165                1170
Tyr Gly Ala Ile Lys Asn Pro Leu Asn Thr Asp Glu Ile Arg Tyr
    1175                1180                1185
Val Ile Arg Lys Asp Leu Glu Ser Leu Lys Ala Ser Asp Ile Glu
    1190                1195                1200
Asn Ile Val Asp Glu Thr Val Lys Glu Lys Val Lys Gln Ala Val
    1205                1210                1215
Thr Asn Lys Val Leu Leu Leu Ser Ser Asn Ala Gln Gln Lys Asn
    1220                1225                1230
Lys Ile Ala Glu Asn Glu Lys Val Trp Met Asn Lys Glu Lys Gln

1235                    1240                    1245
Ile Ser Ile Asn Lys Val Arg Ile Tyr Ala Asn Ser Val Lys Asn
    1250                    1255                    1260
Pro Leu Glu Ile Lys Glu His Ser Leu Leu Ser Lys Ser Arg His
    1265                    1270                    1275
Glu His Lys Gln Arg Val Tyr Gly Gln Asn Asp Glu Asn Tyr Ala
    1280                    1285                    1290
Met Ala Leu Tyr Glu Leu Asp Gly Lys Arg Glu Phe Glu Leu Ile
    1295                    1300                    1305
Asn Asn Phe Asn Leu Ala Lys Leu Ile Lys Asn Gly Gln Gly Phe
    1310                    1315                    1320
Tyr Pro Leu Asn Lys Glu Lys Glu Leu Lys Gly Lys Lys Val Leu
    1325                    1330                    1335
Tyr Pro Ile Ala Lys Ser Asn Ser Arg Asp Val Val Leu Lys Arg
    1340                    1345                    1350
Gly Gln Gln Val Val Phe Phe Asp Lys Glu Val Glu Lys Pro Lys
    1355                    1360                    1365
Asp Ile Ser Glu Ile Leu Asp Phe Lys Arg Arg Val Tyr Ile Ile
    1370                    1375                    1380
Glu Gly Leu Ser Ile Gln Arg Ile Lys Ser Gly Asn Asn Glu Phe
    1385                    1390                    1395
Glu Tyr Gly Val Ile Met Leu Arg Tyr Phe Lys Glu Ala Arg Lys
    1400                    1405                    1410
Ser Asp Glu Ile Lys Lys Asp Asn Phe Lys Pro Asp Gly Val Phe
    1415                    1420                    1425
Lys Leu Gly Glu Asn Lys Pro Thr Arg Lys Met Asn His Ser Gln
    1430                    1435                    1440
Phe Thr Ala Phe Val Glu Gly Ile Asp Phe Lys Val Leu Pro Ser
    1445                    1450                    1455
Gly Lys Phe Val Lys Ile
    1460

<210>  66
<211>  1458
<212>  PRT
<213>  Artificial sequence

<220>
<223>  Cas-Locus 25

<400>  66
Met Ala Lys Ile Leu Gly Leu Asp Leu Gly Thr Asn Ser Ile Gly Trp
1               5                   10                  15
Ala Ile Val Asp Lys Asp Glu Asn Asn Phe Ser Leu Val Asp Lys Gly
            20                  25                  30
Val Arg Ile Phe Ser Glu Gly Val Lys Ser Glu Lys Gly Ile Glu Ser
        35                  40                  45
Ser Arg Ala Ala Glu Arg Thr Gly Phe Arg Ser Ala Arg Lys Ile Lys
    50                  55                  60
Tyr Arg Arg Lys Leu Arg Lys Tyr Glu Thr Leu Lys Val Leu Ser Ser
65                  70                  75                  80
Asn Gly Met Cys Pro Leu Ser Ile Glu Glu Val Glu Asp Trp Lys Lys
                85                  90                  95
Ser Gly Phe Lys Gln Tyr Pro Leu Asn Pro Glu Phe Leu Lys Trp Leu
            100                 105                 110
Arg Thr Asp Glu Ala Glu Asn Ile Asn Pro Tyr Phe Phe Arg Asp Lys
        115                 120                 125
Ala Ser Lys Gln Lys Val Pro Leu Phe Glu Leu Gly Arg Ala Phe Tyr
    130                 135                 140
His Ile Ala Gln Arg Arg Gly Phe Leu Ser Asn Arg Leu Asp Gln Ser
145                 150                 155                 160
Ala Glu Gly Ile Leu Glu Glu His Cys Pro Lys Ile Glu Thr Ile Val
                165                 170                 175

Glu Asp Leu Asn Ser Tyr Glu Glu Val Leu Glu Asn Leu Lys Asp Tyr
            180                 185                 190
Phe Phe Phe Glu Thr Gly Ile Leu Asp Ala Asn Glu Lys Ser Gly Tyr
        195                 200                 205
Ala Lys Asp Leu Asp Glu Gly Asp Lys Lys Leu Val Ser Leu Tyr Lys
    210                 215                 220
Ser Leu His Thr Ile Val Lys Lys Asn Asp Thr Asn Phe Glu Lys Gly
225                 230                 235                 240
Lys Ala Glu Ile Ile Glu Arg Leu Asn Lys Lys Asp Val Leu Gly Lys
                245                 250                 255
Val Lys Gly Lys Ile Lys Asp Ile Ser Gln Ala Met Val Asp Gly Ser
            260                 265                 270
Phe Lys Thr Leu Gly Gln Tyr Phe Phe Ser Leu Tyr Ser Lys Glu Lys
        275                 280                 285
Ile Arg Asn Gln Tyr Thr Ser Arg Glu Glu His Tyr Leu Glu Glu Phe
    290                 295                 300
Ile Thr Ile Cys Lys Val Gln Gly Ile Asp Ser Ile Asn Glu Lys Glu
305                 310                 315                 320
Gln Leu Pro Glu Lys Lys Ser Asp Gly Leu Ala Lys Asp Leu Tyr Lys
                325                 330                 335
Ala Ile Phe Phe Gln Arg Pro Leu Lys Ser Gln Lys Gly Leu Ile Gly
            340                 345                 350
Lys Cys Ser Phe Glu Lys Ser Lys Ser Arg Cys Ser Val Ser His Pro
        355                 360                 365
Asp Phe Glu Glu Tyr Arg Met Trp Asn Tyr Ile Asn Thr Ile Lys Ile
    370                 375                 380
Gly Thr Gln Ser Glu Lys Thr Leu Arg Phe Leu Thr Gln Glu Glu Lys
385                 390                 395                 400
Leu Lys Leu Val Pro Lys Phe Tyr Arg Lys Asn Asp Phe Asn Phe Glu
                405                 410                 415
Val Leu Ala Lys Glu Leu Ile Glu Lys Gly Thr Ser Phe Gly Phe Tyr
            420                 425                 430
Lys Ser Ser Lys Lys Asn Asp Tyr Phe Tyr Trp Phe Asn Phe Lys Pro
        435                 440                 445
Thr Asp Ser Val Ser Ala Cys Gln Val Ser Ala Ser Leu Lys Asn Ile
    450                 455                 460
Ile Gly Glu Asp Trp Lys Thr Lys Thr Phe Thr Tyr Gln Thr Leu Asn
465                 470                 475                 480
Val Lys Lys Glu Glu Val Thr Lys Thr Val Asp Tyr Lys Asp Leu Trp
                485                 490                 495
His Leu Leu Ser Val Ala Thr Ser Asp Ile Tyr Leu Asn Lys Tyr Ala
            500                 505                 510
Lys Asp Lys Leu Gly Phe Glu Asp Lys Asn Ala Lys Ala Phe Ser Lys
        515                 520                 525
Ile Lys Leu Lys Lys Asp Phe Gly Asn Leu Ser Leu Ser Ala Ile Thr
    530                 535                 540
Lys Ile Leu Pro Tyr Ile Lys Glu Gly Leu Leu Tyr Ser His Ala Val
545                 550                 555                 560
Phe Met Ala Asn Ile Glu Asn Ile Val Asp Glu Glu Ile Trp Lys Asp
                565                 570                 575
Glu His Gln Arg Lys Tyr Ile Gln Thr Gln Ile Ala Glu Ile Val Glu
            580                 585                 590
Asn Tyr Thr Phe Glu Lys Ser Gln Leu Glu Val Ile Asn Gly Leu Ile
        595                 600                 605
Lys Asp Ser Lys Leu Lys Glu Glu Tyr Tyr Ser Lys Glu Ala Glu Ser
    610                 615                 620
Ile Tyr Lys Lys Asp Leu Ala Thr Lys Leu Val Leu Phe Phe Lys Tyr
625                 630                 635                 640
Asn Val Ile Glu Asn Glu Ser Asp Gln Lys Ile Ile Phe Glu Asn Leu
                645                 650                 655
Phe Pro Ile Phe Ile Glu Gln Leu Lys Lys Tyr Glu Phe Ile Lys Ile
            660                 665                 670
Gln Arg Leu Asp Glu Lys Val Leu Glu Phe Leu Lys Gly Glu Asn Gln

        675                 680                 685
Asp Gly Glu Ile Phe Cys Ser Asn Pro Lys Gln Leu Lys Lys Leu Tyr
    690                 695                 700
His Pro Ser Asp Ile Glu Val Phe Lys Lys Lys Ile Ile Lys Asp Glu
705                 710                 715                 720
Phe Gly Asn Glu Lys Val Val Leu Gly Thr Pro Leu Thr Ser Ala Ile
                725                 730                 735
Lys Asn Pro Met Ala Met Arg Ala Leu His Gln Leu Arg Lys Val Leu
            740                 745                 750
Asn Thr Leu Ile Leu Glu Gly Leu Val Asp Glu Asn Thr Leu Ile His
        755                 760                 765
Ile Glu Met Ala Arg Glu Leu Asn Asp Ala Asn Lys Arg Lys Gly Ile
    770                 775                 780
Gln Asp Tyr Gln Asn Glu Asn Lys Lys Phe Arg Glu Asp Ala Ile Lys
785                 790                 795                 800
Glu Ile Lys Lys Leu Tyr Leu Asp Glu Cys Lys Lys Glu Val Glu Pro
                805                 810                 815
Thr Glu Asp Asp Ile Leu Arg Tyr Gln Leu Trp Ile Glu Gln Asn Arg
            820                 825                 830
Gln Glu Ile Tyr Glu Gln Gly Lys Asn Ile Ser Ile Cys Asp Ile Ile
        835                 840                 845
Gly Ser Asn Pro Ala Tyr Asp Ile Glu His Thr Ile Pro Arg Ser Arg
    850                 855                 860
Ser Gln Asp Asn Ser Gln Met Asn Lys Thr Leu Cys Ser Gln Arg Phe
865                 870                 875                 880
Asn Arg Glu Ile Lys Gly Asn Lys Met Pro Ile Glu Leu Asn Asn His
                885                 890                 895
Leu Glu Ile Leu Pro Arg Ile Ala His Trp Lys Glu Glu Ala Ser Asn
            900                 905                 910
Leu Ser Arg Glu Ile Glu Ile Ile Ser Arg Ser Ile Lys Ala Ala Ala
        915                 920                 925
Thr Lys Glu Val Lys Asp Lys Lys Ile Arg Arg Arg His Tyr Leu Thr
    930                 935                 940
Leu Lys Arg Asp Tyr Leu Gln Gly Lys Tyr Asp Arg Phe Ile Leu Glu
945                 950                 955                 960
Glu Pro Arg Val Gly Phe Lys Asn Ser Gln Ile Pro Asp Thr Gly Ile
                965                 970                 975
Ile Thr Lys Tyr Ser Gln Ala Tyr Leu Lys Ser Tyr Phe Lys Arg Val
            980                 985                 990
Glu Ser Val Lys Gly Gly Met Val Ala Glu Phe Arg Lys Gln Trp Gly
        995                 1000                1005
Ile Gln Glu Ser Phe Ile Asp Glu Asn Gly Phe Lys His Tyr Lys
    1010                1015                1020
Ile Lys Asp Arg Ser Lys His Thr His His Thr Ile Asp Ala Ile
    1025                1030                1035
Thr Ile Ala Cys Met Thr Lys Asp Lys Tyr Asp Val Leu Ala His
    1040                1045                1050
Ala Trp Ala Leu Glu Asp Glu Gln Asn Lys Lys Glu Ala Lys Ala
    1055                1060                1065
Ile Ile Glu Lys Ala Lys Pro Trp Lys Thr Phe Lys Glu Asp Leu
    1070                1075                1080
Leu Lys Ile Glu Glu Glu Ile Leu Val Ser His Tyr Thr Pro Asp
    1085                1090                1095
Asn Val Lys Lys Gln Ser Lys Lys Val Ile Arg Ile Arg Gly Lys
    1100                1105                1110
Lys Gln His Val Ala Glu Ile Glu Arg Asp Glu Lys Gly Lys Ala
    1115                1120                1125
Ile Pro Lys Lys Asp Ala Asn Gly Lys Ile Leu Tyr Lys Leu Asp
    1130                1135                1140
Lys Glu Gly Lys Lys Leu Pro Arg Tyr Gln Gln Gly Asp Ala Ile
    1145                1150                1155
Arg Gly Ser Leu His Gln Asp Ser Ile Tyr Gly Ala Ile Lys Asn
    1160                1165                1170

Pro Leu Asn Thr Glu Glu Ile Arg Tyr Val Ile Arg Lys Asp Leu
    1175                1180                1185
Glu Ser Leu Lys Ala Ser Asp Ile Glu Asn Ile Val Asp Glu Thr
    1190                1195                1200
Val Lys Glu Lys Ile Lys Glu Ala Val Ala Asn Lys Val Leu Leu
    1205                1210                1215
Phe Ser Ser Asn Ala Gln Gln Lys Asn Lys Ile Arg Glu Asn Glu
    1220                1225                1230
Lys Val Trp Met Asn Glu Glu Lys Gln Val Pro Ile Asn Lys Val
    1235                1240                1245
Arg Ile Tyr Ala Asn Ser Val Lys Asn Pro Leu Glu Val Lys Glu
    1250                1255                1260
His Ser Leu Leu Ser Lys Ser Arg His Glu His Lys Gln Lys Val
    1265                1270                1275
Tyr Gly Gln Asn Asp Glu Asn Tyr Cys Met Ala Ile Tyr Glu Leu
    1280                1285                1290
Glu Gly Lys Arg Glu Phe Glu Leu Ile Asn Asn Phe Asn Leu Ala
    1295                1300                1305
Lys Leu Ile Lys Gly Gly Gln Gly Phe Tyr Pro Phe Tyr Lys Glu
    1310                1315                1320
Lys Glu Val Lys Gly Lys Val Val Gln Val Pro Ile Ala Lys Arg
    1325                1330                1335
Asn Gly Arg Asp Leu Ile Leu Lys Arg Gly Gln Gln Val Ile Phe
    1340                1345                1350
Tyr Asp Lys Asp Val Glu Asn Pro Asp Asp Val Phe Asp Leu Val
    1355                1360                1365
Asp Leu Lys Gly Arg Ile Tyr Ile Ile Glu Gly Leu Ser Ile Gln
    1370                1375                1380
Arg Gln Val Val Ser Gly Lys Leu Tyr Glu Phe Gly Ser Ile Met
    1385                1390                1395
Phe Arg Tyr Phe Lys Glu Ala Arg Lys Ala Asp Glu Ile Lys Lys
    1400                1405                1410
Asp Ser Phe Lys Pro Asp Gly Thr Phe Lys Ser Gly Glu Ile Lys
    1415                1420                1425
Pro Thr Arg Lys Met Asn His Asn Gln Phe Thr Ala Phe Val Glu
    1430                1435                1440
Gly Ile Asp Phe Lys Val Leu Pro Ser Gly Lys Phe Ile Lys Ile
    1445                1450                1455

<210>  67
<211>  1127
<212>  PRT
<213>  Artificial sequence

<220>
<223>  Cas-Locus 26

<400>  67
Met Ala Lys Ile Leu Gly Leu Asp Leu Gly Thr Asn Ser Ile Gly Trp
1               5                   10                  15
Ala Leu Ile Asp Asp Lys Leu Asn Lys Ile Leu Gly Ile Gly Ser Arg
            20                  25                  30
Ile Phe Pro Met Gly Val Glu Asn Leu Gly Asp Gly Asp Asn Glu Met
        35                  40                  45
Ser Lys Asn Ala Ser Arg Thr Gly Ala Arg Gly Val Arg Arg Gln Phe
    50                  55                  60
Phe Arg Arg Arg Leu Arg Lys Lys Val Leu Leu Lys Ser Leu Ser Glu
65                  70                  75                  80
Asn Asn Met Cys Pro Ile Asn Thr Lys Asp Phe Glu Asp Trp Lys Gln
                85                  90                  95
Thr Lys Gln Phe Pro Asn Glu Lys Leu Ala Thr Trp Phe Ala Met Asn
            100                 105                 110
Pro Tyr Glu Leu Arg Gln Lys Ala Leu Thr Glu Lys Leu Ser Leu Glu

115                 120                 125
Glu Ile Gly Arg Ile Phe Tyr His Leu Ile Gln Arg Arg Gly Phe Leu
    130                 135                 140
Ser Asn Ser Arg Lys Gly Gly Ser Asp Asp Gly Ala Ile Phe Lys Gly
145                 150                 155                 160
Asn Ala Lys Glu Gly Lys Ile Gly Ile Asp Tyr Thr Leu Glu Ser Ile
                165                 170                 175
Glu Gly Lys Thr Leu Gly Ser Tyr Leu Tyr Glu Ile Tyr Pro Lys Glu
            180                 185                 190
Asn Gln Pro Phe Gln Tyr Gly Leu Glu Arg Ile Arg Asn Arg Tyr Thr
        195                 200                 205
Thr Arg Arg Met Tyr Ile Asp Glu Phe Glu Met Ile Trp Asn Lys Gln
    210                 215                 220
Ser Glu Phe His Ser Val Leu Asn Tyr Asp Leu Lys Thr Ile Leu Gly
225                 230                 235                 240
Gly Arg Lys Gln Asp Asn Tyr Thr Glu Asp Gly Ile Leu Phe Tyr Gln
                245                 250                 255
Arg Pro Leu Arg Ser Gln Lys His Leu Val Gly Asn Cys Ser Phe Glu
            260                 265                 270
Pro Leu Lys Thr Lys Cys Pro Ile Ser Ala Ile Pro Val Glu Met Phe
        275                 280                 285
Arg Ile Trp Gln Trp Val Asn Thr Val Glu Tyr Asn Gly Lys Lys Ile
    290                 295                 300
Ser Glu Glu Glu Lys Thr Lys Leu Val Glu Gln Leu Leu Ser Phe Asp
305                 310                 315                 320
Lys Ile Glu Phe Lys Lys Leu Arg Lys Ala Ile Gly Lys Glu Ser Ala
                325                 330                 335
Glu Phe Lys Phe Asn Tyr Lys Asp Asp Asp Lys Ile Ala Gly Thr His
            340                 345                 350
Thr Ile Ser Asn Leu Ser Asn Arg Lys Phe Phe Gly Lys Arg Trp Phe
        355                 360                 365
Asp Phe Ser Asp Lys Glu Gln Glu Asp Ile Trp His Val Leu Tyr Phe
    370                 375                 380
Phe Asp Ser Lys Ser Asn Leu Lys Glu Tyr Ala Val Lys Asn Trp Asn
385                 390                 395                 400
Phe Ser Glu Glu Gln Ala Ile Ala Ile Ser Arg Phe Asn Val Lys Asp
                405                 410                 415
Gly Tyr Ala Ser Leu Ser Arg Lys Ala Ile Gly Asn Ile Leu Pro Phe
            420                 425                 430
Leu Gln Gln Gly Tyr Thr Tyr Asp Ile Ala Val Val Met Gly Gly Ile
        435                 440                 445
Lys Asn Ala Phe Gly Asp Lys Trp Gln Asn Asp Thr Val Glu Asn Lys
    450                 455                 460
Leu Lys Gln Leu Asp Leu Ile Asp Asn Ile Glu Gly Ile Val Arg Ser
465                 470                 475                 480
Lys Ile Ser Gly Gly Phe Ile Asp Thr Ile Lys Asp Leu Leu Arg Lys
                485                 490                 495
Glu Phe Glu Phe Asn Asp Lys Gln Leu Lys Lys Leu Tyr His His Ser
            500                 505                 510
Ala Ala Ile Asp Thr Val Ala Leu Leu Arg Lys Leu Pro Thr Gly Lys
        515                 520                 525
Gln Ala Asp Lys Asp Ile Gln Ala Ile Arg Asn Pro Ile Val Ile Thr
    530                 535                 540
Ala Leu Phe Glu Leu Arg Lys Leu Val Asn Glu Leu Ile Glu Glu His
545                 550                 555                 560
Gly Gln Leu Asp Glu Ile Lys Val Glu Met Ala Arg Asp Leu Lys Ile
                565                 570                 575
Ser Lys Ser Gln Arg Asn Lys Ile Arg Lys Asp Gln Lys Arg Leu Glu
            580                 585                 590
Arg Glu Asn Asp Arg Val Lys Lys Glu Val Glu Lys Tyr Val Arg Val
        595                 600                 605
Ser His Asp Asn Ile Leu Arg Phe Lys Leu Trp Glu Glu Cys Lys Gln
    610                 615                 620

Thr Cys Pro Tyr Thr Gly Val Val Ile Pro Val Thr Lys Leu Phe Thr
625                 630                 635                 640
Gly Glu Ile Gln Ile Glu His Ile His Pro Trp Ser Arg Ser Leu Asn
                645                 650                 655
Asp Ser Phe Asn Asn Lys Thr Leu Cys Tyr Ala Asp Glu Asn Arg Arg
            660                 665                 670
Lys Gly Asp Lys Thr Pro Phe Glu Phe Tyr Gly Ser Asp Glu Leu Asp
        675                 680                 685
Trp Ile Ala Arg Lys Glu Arg Ala Leu Lys Leu Phe Ser Asp Thr Lys
    690                 695                 700
Glu Tyr Pro Asn Ala Tyr Gln Lys Phe Lys Arg Phe Val Gln Gln Lys
705                 710                 715                 720
Phe Asp Asp Asp Phe Ser Ser Arg Gln Leu Asn Asp Thr Arg Phe Ile
                725                 730                 735
Ser Lys Glu Ala Ser Ser Tyr Leu Lys Gln Ile Cys Glu Lys Val Asn
            740                 745                 750
Val Ser Pro Gly Gln Met Thr Ala Asn Leu Arg Gln Lys Trp Gly Leu
        755                 760                 765
Asn Thr Val Leu Asn Asp Glu Asn Ala Lys Thr Arg Glu Asp His Arg
    770                 775                 780
His His Ala Ile Asp Ala Leu Val Met Ala Cys Gly Lys Thr Ser Tyr
785                 790                 795                 800
Leu Gln Glu Leu Ser Lys Trp Asn Arg Tyr Asn Arg Asn Tyr Asp Leu
                805                 810                 815
Lys Asp Phe Pro Met Pro Trp Glu Thr Phe Arg Phe Asp Ala Glu Lys
            820                 825                 830
Ala Ile Asp Lys Ile Leu Ile Ser His Lys Arg Ile Ser Asn Asp Ile
        835                 840                 845
Thr Thr Arg Tyr Thr Lys Val Glu Lys Asn Gly Lys Thr Tyr Ile Asn
    850                 855                 860
Lys Gly Val Ala Ala Arg Gly Gln Leu His Lys Glu Thr Val Phe Gly
865                 870                 875                 880
Lys Arg Asn Phe Asn Gly Asp Glu Ala Phe His Val Arg Lys Ser Ile
                885                 890                 895
Asp Ser Leu Thr Thr Glu Lys Gln Leu Glu Lys Val Val Asp Glu Val
            900                 905                 910
Ile Lys Lys Leu Ile His Lys Lys Ile Lys Glu Leu Gly Gly Phe Val
        915                 920                 925
Lys Gly Thr Ile Pro Ala Asn Thr Phe Phe Asn Val Asp Glu Asn Gly
    930                 935                 940
Val Lys Gln Pro Gln Ile Phe Leu Pro Asn Lys Asn Gly Ser Pro Val
945                 950                 955                 960
Pro Ile Leu Lys Val Arg Met Lys Glu Asn Ile Ser Gly Ala Glu Lys
                965                 970                 975
Leu Lys Asp Asn Val Asn Gln Trp Val Asn Pro Arg Asn Asn His His
            980                 985                 990
Val Leu Ile Tyr Lys Asp Glu Lys  Gly Ile Leu Arg Glu  Glu Val Val
        995                 1000                1005
Thr Phe  Trp Thr Val Val Glu  Arg Lys Ser Lys Gly  Phe Pro Val
    1010                1015                1020
Tyr Gln  Leu Pro Val Glu Gly  Lys Glu Ile Val Thr  Ala Leu His
    1025                1030                1035
Ile Asn  Asp Met Phe Leu Leu  Gly Leu Asn Glu Glu  Glu Ile Asn
    1040                1045                1050
Trp Glu  Asn Pro Asp Tyr Glu  Ile Leu Arg Asp Ser  Leu Tyr Arg
    1055                1060                1065
Val Gln  Ser Leu Ser Ser Lys  Tyr Tyr Glu Phe Arg  Gln Ser Ile
    1070                1075                1080
Asn Ala  Glu Ser Gln Asn Lys  Ile Ser Pro Ile Tyr  Glu Gln Ile
    1085                1090                1095
Arg Gly  Phe Gly Ser Gly Lys  Thr Gly Trp Asp Ser  Phe Asn Pro
    1100                1105                1110
Ile Lys  Val Lys Ile Ser Val  Ser Gly Lys Ile Ser  Lys Ile

1115                1120                1125

<210>  68
<211>  1108
<212>  PRT
<213>  Artificial sequence

<220>
<223>  Cas-Locus 27

<400>  68
Met Ala Tyr Lys Leu Gly Ile Asp Leu Gly Thr Ala Ser Ile Gly Ala
1               5                   10                  15
Val Ala Ile Arg Ile Glu Asn Asn Glu Pro Gln Ser Ile Asp Trp His
            20                  25                  30
Ala Val Arg Ile Tyr Ser Glu Pro Val Val Asn Thr Pro Lys Gly Leu
        35                  40                  45
Gln Pro Lys Lys Ala Ala Arg Arg Asp Ala Arg Gln Leu Arg Lys Gln
    50                  55                  60
Ile Glu Arg Arg Ala Arg Arg Leu Arg Arg Ile Ala Asn Leu Ile Pro
65                  70                  75                  80
Leu Leu Gly Leu His Lys Ala Glu Val Lys Pro Gly Arg Arg Gln Asp
                85                  90                  95
Leu Thr Thr Leu Arg Ala Ile Ala Ala Arg Gln Gln Val Glu Leu Asn
            100                 105                 110
Glu Phe Ala Leu Val Leu Leu Arg Leu Ala Lys Arg Arg Gly Tyr Ala
        115                 120                 125
Gly Gly Phe Arg Asp Ser Lys Asp Gly Val Val Lys Ser Ser Ser Asp
    130                 135                 140
Lys Leu Ser Glu Glu Met Arg Arg Leu Ala Glu Thr Gln Asn Leu Gln
145                 150                 155                 160
Glu Ile Thr Leu Gly Glu Tyr Leu Leu Tyr Arg Gln Glu His Gly Leu
                165                 170                 175
Pro Val Trp Leu Lys Ala Glu Arg Pro Gly Leu Glu Pro Arg Tyr Ala
            180                 185                 190
Leu Arg Glu Met Val Thr Ala Glu Phe Glu Gln Leu Trp Thr Thr Gln
        195                 200                 205
Ala Arg Tyr His Pro Ile Leu Ala Ser Asn His Leu Gly Lys Pro Leu
    210                 215                 220
Lys Gln Trp Phe Asn Glu Ala Leu Phe Tyr Gln Arg Pro Leu Lys Ser
225                 230                 235                 240
Pro Ala His Met Val Gly Asn Cys Leu Leu Glu Pro Asn Leu Pro Arg
                245                 250                 255
Ala Pro Arg Ala His Met Ala Ala Gln His Phe Arg Ile Asp Lys Thr
            260                 265                 270
Leu His Asp Met Arg Trp Gly Ser Gly Arg Arg Ala Glu Arg Leu Ser
        275                 280                 285
Lys Ala Gln Leu Asn Val Leu Arg Gln Met Leu Asn Asp Pro Ala Asn
    290                 295                 300
Ala Asp Gly Lys Val Ser Phe Lys His Leu Tyr Gln Ala Leu Ser Glu
305                 310                 315                 320
Thr Gly Leu Ser Asp Pro Arg Ser Arg Thr Leu Asn Leu Glu Arg Ser
                325                 330                 335
Ser Cys Asp Gln Leu Ser Gly Asn Asn Thr Val Ala Arg Trp Arg Ala
            340                 345                 350
Leu Gly Leu Tyr Asp Gln Trp Leu Thr Leu Ala Pro Arg Asp Gln Thr
        355                 360                 365
Thr Ile Ile Asn Leu Leu Ala Asp Leu Gly Gly Pro Glu Leu Leu Val
    370                 375                 380
Pro Asp Asp Trp His Gln Asn Ile Arg Lys Ser Ser Ala Lys Ala Gly
385                 390                 395                 400
Asp Thr Asn Leu Tyr Arg Arg Phe Arg Pro Glu Val Val Asp Phe Ile
                405                 410                 415

Asn Ala Leu Arg Glu Cys Lys Asp Asn Asn Gly Gln Pro Val Phe Asn
            420                 425                 430
Arg Leu Thr Ser Met Lys Phe Glu Gly Gly Arg Ser Ser Tyr Ser Val
        435                 440                 445
Lys Ala Leu Glu Lys Leu Asn Thr Trp Phe Asn Asp Pro Trp Trp Pro
    450                 455                 460
Asp Glu Gly Ser Gly Ala Glu Arg Gly Asp Glu Ile Asp Glu Asp Ala
465                 470                 475                 480
Ala Leu Arg Val Cys Tyr Pro His Leu Phe Thr Gln Pro Gln Glu Leu
                485                 490                 495
Leu Gln Arg Leu Glu Ala Ala Pro Thr Thr Gly Asn Asp Val Val Asp
            500                 505                 510
Gly Ala Leu Arg Glu Leu Arg Lys Val Ile Asn Gln Cys Ile Asp Ala
        515                 520                 525
Leu Gly Glu Arg Pro Glu Glu Ile Val Val Glu Met Ala Arg Glu Ile
    530                 535                 540
Gly Lys Gly Val Val Trp Arg Lys Glu Gln Glu Asn Arg Ile Arg Gln
545                 550                 555                 560
Asn Glu Leu Ala Arg Arg Lys Ile Ala Ser Glu Leu Glu Lys His Gly
                565                 570                 575
Leu Ser Ala Asn Asn Thr Arg Ile Leu Arg Tyr Gln Leu Trp Glu Glu
            580                 585                 590
Gln Asp Leu His Trp Cys Pro Tyr Cys Ala His Pro Ile Asn Leu Glu
        595                 600                 605
Ala Ala Thr Ser Gly Met Thr His Ile Glu His Val Leu Pro Arg Ser
    610                 615                 620
Leu Thr Gln Ile Gly Arg Lys Arg Ser Glu Leu Val Leu Ala His Ala
625                 630                 635                 640
Gly Cys Asn Asn Leu Lys Ala Asp Gln Thr Pro Trp Gln Ala Phe Gly
                645                 650                 655
His Asp Val Glu Arg Trp Ser Ala Val Glu Arg Ala Ala Ala Arg Phe
            660                 665                 670
Gln Glu Leu Gly Lys Lys Ser Tyr Ala Lys Asp Arg Ile Lys Ala Met
        675                 680                 685
Gly Phe Tyr Arg Lys Ala Glu Leu Leu Leu Leu Lys Asp Tyr Glu Ser
    690                 695                 700
Glu Val Leu Ser Asp Glu Ser Ile Ala Asp Phe Ala Asp Arg Gln Leu
705                 710                 715                 720
His Gln Thr Ser Trp Ile Ala Lys Ala Ala Ala Gln Trp Leu Arg Thr
                725                 730                 735
Ile Thr Pro Leu Val Ser Val Ser Arg Gly Glu Phe Thr Ala Leu Leu
            740                 745                 750
Arg His Gln Trp Arg Leu Asp Thr Val Ile Ala Glu Ala Arg Leu Asp
        755                 760                 765
Glu Lys Leu Pro Val Leu Asp Ile Asp Ala Cys Pro Val Ser Pro Glu
    770                 775                 780
Ala Phe Gln Gln Phe Arg Lys Gln Trp Glu Gly His Pro Pro Asp Asn
785                 790                 795                 800
Leu Ala Glu Arg Thr Glu Arg Lys Leu Glu Lys Arg Leu Asp His Arg
                805                 810                 815
His His Gly Ile Asp Ala Leu Val Ile Ala Leu Thr Ser Arg Ser Leu
            820                 825                 830
Tyr Met Ser Leu Ala Arg Arg Tyr Val Glu Glu Ser Glu Arg Arg Ala
        835                 840                 845
Arg Gly Glu Arg Val Arg His Asp Trp Ser Ile Glu Pro Thr Met His
    850                 855                 860
Asn Leu Arg Asn Gln Ala Leu Gly Met Val Arg Thr Cys Asn Leu Thr
865                 870                 875                 880
His Lys Pro Asp Arg Asn Pro Ala Gly Arg Leu Phe Lys Asp Thr Ala
                885                 890                 895
Tyr Gly Val His Phe Pro Glu Glu Gly Asp Arg Val Gln Leu Thr Leu
            900                 905                 910
Arg Lys Pro Leu Thr Ser Leu Ala Asp Glu Lys Ser Ala Glu Lys Thr

915                 920                 925
Arg Lys Asn Leu His Ser Ile Ala Ser Ser Glu Val Arg Leu Leu Val
    930                 935                 940
Leu Lys Ala Phe Glu Ala Arg Leu Ser Val Gly Gln Asn Pro Lys Gln
945                 950                 955                 960
Ala Leu Ser Glu Pro Val Pro Tyr Pro Ala Tyr Gly Thr Ala Ile Trp
                965                 970                 975
Arg Val Arg Cys Met Gln Gly Tyr Ala Asp Asp Ala Val Arg Ile Val
            980                 985                 990
His Lys Ser Arg Gln Gly Glu His  Glu Lys His Leu Leu  Asn Asp Gly
    995                 1000                1005
Tyr Ala  Cys Leu Glu Ile Thr  Ala Gly Glu Asn Gly  Lys His Gln
    1010                1015                1020
Val Arg  Leu Val Thr Gln Arg  Glu Ala Gln Gly Arg  Gly Phe Met
    1025                1030                1035
Asp Asn  Arg Gln Gln Ala Lys  Arg Ile Phe Lys Gly  Asp Thr Val
    1040                1045                1050
Ile Asp  Arg Arg Asp Asp Arg  Lys Tyr Leu Val Gly  Tyr Phe Lys
    1055                1060                1065
Ala Glu  Gly Tyr Ile Phe Leu  Leu Pro His Thr Asp  Thr Arg Asn
    1070                1075                1080
Phe Asp  Lys Ile Lys Glu Ala  Asp Ser Gly Lys Lys  Lys Val Ser
    1085                1090                1095
Phe Asn  Gln Ala Tyr Gln Leu  Glu Arg Ile
    1100                1105

<210>  69
<211>  1108
<212>  PRT
<213>  Artificial sequence

<220>
<223>  Cas-Locus 28

<400>  69
Met Ala Tyr Lys Leu Gly Ile Asp Leu Gly Thr Ala Ser Ile Gly Ala
1               5                   10                  15
Val Ala Ile Arg Ile Glu Asn Lys Val Pro Ile Ser Val Asp Trp His
            20                  25                  30
Ala Val Arg Ile Phe Ser Glu Pro Val Val Asn Thr Gln Lys Gly Leu
        35                  40                  45
Thr Pro Lys Lys Thr Ala Arg Arg Glu Ala Arg Gln Leu Arg Lys Gln
    50                  55                  60
Val Glu Arg Arg Ala Arg Arg Leu Arg Arg Ile Ala Asn Leu Ile Pro
65                  70                  75                  80
Leu Leu Gly Leu Gln Lys Ala Glu Val Lys Leu Gly Arg Arg Gln Asp
                85                  90                  95
Leu Thr Thr Leu Arg Ala Ile Ala Ala Arg Glu Lys Val Glu Leu His
            100                 105                 110
Glu Phe Ala Leu Val Leu Leu Arg Ile Ala Lys Arg Arg Gly Tyr Ala
        115                 120                 125
Gly Gly Phe Arg Asp Ser Glu Gly Gly Val Val Lys Thr Ala Ser Asp
    130                 135                 140
Lys Leu Ser Val Glu Met Arg Ala Leu Ala Glu Thr Arg Gly Leu Gln
145                 150                 155                 160
Gln Ile Thr Leu Gly Glu Tyr Leu Leu His Arg Gln Gln Arg Gly Leu
                165                 170                 175
Pro Ile Trp Leu Lys Ala Glu Arg Pro Gly Leu Glu Pro Leu Tyr Ala
            180                 185                 190
Leu Arg Glu Met Val Thr Ala Glu Phe Glu Gln Ile Trp Thr Ile Gln
        195                 200                 205
Ala Lys Cys His Pro Ile Leu Asn Ser Cys His Leu Glu Lys Pro Leu
    210                 215                 220

Lys Gln Trp Phe Tyr Gln Ala Leu Phe Tyr Gln Arg Pro Leu Lys Ser
225                 230                 235                 240
Pro Ala His Met Val Gly Asn Cys Leu Leu Glu Pro Asn Leu Pro Arg
                245                 250                 255
Ala Pro Arg Ala Gln Met Ala Ala Gln Arg Phe Arg Ile Glu Lys Thr
            260                 265                 270
Leu His Asp Leu Arg Trp Gly Gly Gly Arg Gln Ala Glu Arg Leu Asn
        275                 280                 285
Thr Ala Gln Leu Asp Leu Leu Arg Gln Thr Leu Ser Asp Pro Thr Asn
    290                 295                 300
Val Asp Ser Lys Val Ser Phe Lys Arg Leu Tyr Gln Ala Leu Asn Glu
305                 310                 315                 320
Ala Gly Leu Asn Asp Pro Arg Asn Arg Thr Leu Asn Leu Glu Arg Ser
                325                 330                 335
Ser Arg Asp Gln Leu Leu Gly Asn Ser Thr Val Ala Arg Trp Arg Ala
            340                 345                 350
Leu Gly Leu Gln Asp Gln Trp Leu Met Leu Ala Pro Arg Asp Gln Thr
        355                 360                 365
Ser Val Ile Asn Leu Leu Ala Asp Leu Gly Gly Pro Glu Ile Leu Thr
    370                 375                 380
Pro Asp Asp Trp His Gln Asn Ile Arg Lys Ser Ser Ala Lys Asp Asp
385                 390                 395                 400
Asn Thr Lys Ala Tyr Arg Arg Phe Arg Gln Glu Val Ile Asp Phe Ile
                405                 410                 415
Asp Ala Leu Arg Arg Cys Ile Asp Ser Asn Gly Gln Pro Val Phe Asn
            420                 425                 430
Arg Leu Ser Ala Met Lys Phe Asp Gly Gly Arg Ser Ser Tyr Ser Val
        435                 440                 445
Lys Ala Leu Glu Lys Met Asn Glu Trp Phe Asn Asn Pro Trp Trp Pro
    450                 455                 460
Asn Glu Gly Pro Gly Ser Glu Arg Arg Gly Glu Ile Asp Glu Glu Val
465                 470                 475                 480
Ala Ile Arg Val Cys Tyr Pro His Leu Tyr Thr Gln Ser Gln Glu Leu
                485                 490                 495
His Pro Arg Leu Glu Ala Ala Pro Thr Thr Gly Asn Asp Val Val Asp
            500                 505                 510
Gly Ala Leu Arg Glu Leu Arg Lys Val Ile Asn Gln Cys Ile Asp Glu
        515                 520                 525
Gln Gly Glu Lys Pro Gln Glu Ile Ile Ile Glu Leu Ala Arg Glu Val
    530                 535                 540
Gly Lys Gly Val Ala Leu Arg Asn Glu Gln Glu Ser Arg Ile Arg Asn
545                 550                 555                 560
Asn Glu Leu Ala Arg Arg Lys Ile Ala Ser Glu Leu Glu Gly His Gly
                565                 570                 575
Leu Ile Ala Ser Ser Thr Arg Ile Leu Arg Tyr Gln Leu Trp Glu Glu
            580                 585                 590
Gln Glu Arg His Trp Cys Pro Tyr Cys Ala Leu Pro Ile Ser Leu Glu
        595                 600                 605
Ala Ala Ile Ser Asn Met Thr His Ile Glu His Val Leu Pro Arg Ser
    610                 615                 620
Leu Thr Gln Val Gly Arg Lys Arg Ser Glu Leu Val Leu Ala His Ala
625                 630                 635                 640
Gly Cys Asn His Leu Lys Ala Asp Gln Thr Pro Trp Gln Ala Phe Gly
                645                 650                 655
His Asp Ala Glu Arg Trp Ser Ala Val Glu Arg Ala Ala Ala Arg Phe
            660                 665                 670
Gln Glu Leu Gly Lys Lys Cys Tyr Phe Lys Asp Arg Ala Lys Ala Met
        675                 680                 685
Gly Cys Phe Arg Lys Ser Ala Leu Leu Met Leu Lys Asp Tyr Glu Ser
    690                 695                 700
Glu Val Leu Asn Asp Glu Ser Ile Ala Gly Phe Ala Asn Arg Gln Leu
705                 710                 715                 720
His Gln Thr Ser Trp Ile Ala Lys Val Ala Ala Gln Trp Leu Arg Thr

                725                 730                 735
Ile Ser Pro Leu Ile Ser Val Ser Arg Gly Glu Phe Thr Ala Leu Leu
            740                 745                 750
Arg His Gln Trp Arg Leu Asp Thr Val Ile Ala Glu Thr Arg Tyr Gly
        755                 760                 765
Glu Lys Leu Pro Val Leu Asp Ile Asp Gly Asn Pro Val His Pro Glu
    770                 775                 780
Glu Phe Gln His Phe Arg Lys Gln Trp Glu Gly His Pro Pro Asp Asn
785                 790                 795                 800
Pro Ala Glu Arg Thr Glu Arg Lys Leu Glu Lys Arg Phe Asp His Arg
                805                 810                 815
His His Gly Ile Asp Ala Leu Val Ile Ala Leu Thr Ser Arg Ser Leu
            820                 825                 830
Tyr Met Ser Leu Ala Arg Arg Tyr Val Glu Glu Ser Lys Lys Arg Ala
        835                 840                 845
Arg Gly Glu Lys Val Arg Arg Asp Trp Ser Ile Glu Pro Pro Met His
    850                 855                 860
Asn Leu Arg Gly Gln Ala Leu Asp Met Ile Arg Ala Cys Asn Leu Thr
865                 870                 875                 880
Leu Lys Pro Asp Arg Asn Ala Ala Gly Arg Leu Phe Glu Asp Tyr Ala
                885                 890                 895
Tyr Gly Val His Phe Ser Asp Ala Ser Glu Arg Ala Gln Leu Thr Arg
            900                 905                 910
Arg Lys Thr Leu Ile Asp Leu Ala Asp Glu Lys Ser Ala Glu Lys Thr
        915                 920                 925
Arg Lys Asn Leu Gln Ser Ile Ala Ser Pro Glu Val Arg Gln Ile Val
    930                 935                 940
Val Glu Ala Leu Gln Met Arg Leu Ser Ala Gly Gln Asn Pro Lys Gln
945                 950                 955                 960
Ala Leu Ser Lys Pro Val Leu Tyr Pro Ala Tyr Gly Thr Glu Ile Trp
                965                 970                 975
Arg Val Arg Cys Leu Gln Gly Tyr Ala Asp His Ala Val Arg Ile Val
            980                 985                 990
His Asn Ser Arg Gln Gly Glu His Glu Lys Cys Leu Leu Asn Asp Gly
        995                 1000                1005
Tyr Ala Cys Leu Glu Ile Arg Ala Gly Glu Asn Gly Lys His Gln
    1010                1015                1020
Ala Arg Leu Val Thr Gln Arg Glu Ala Gln Gly Arg Asp Phe Met
    1025                1030                1035
Glu Thr Gln Gln Gln Ala Lys Arg Leu Phe Lys Gly Asp Thr Val
    1040                1045                1050
Ile Asp Arg Arg Asp Asp Arg Lys Tyr Arg Val Gly Tyr Phe Thr
    1055                1060                1065
Ala Glu Gly Asn Ile Phe Leu Ile Pro Phe Glu Asp Thr Arg Asn
    1070                1075                1080
Phe Asp Lys Ile Asn Glu Arg Asn Ser Gly Lys Lys Lys Val Ser
    1085                1090                1095
Phe Asn Gln Ala His Arg Leu Glu Arg Val
    1100                1105

<210> 70
<211> 36
<212> DNA
<213> Bacillus cereus

<400> 70
atcatatcat atcgagtttt agtaaggaac tatagc 36

<210> 71
<211> 36
<212> DNA
<213> Brevibacillus laterosporus

<400>  71
atcatatcat atcgagtttt ggtaaggaac tatagc                                36

<210>  72
<211>  36
<212>  DNA
<213>  Artificial sequence

<220>
<223>  CRISPR repeat consensus Locus 8

<400>  72
atcataatac agcaatggct ttaatggaac tatgac                                36

<210>  73
<211>  36
<212>  DNA
<213>  Bacillus cereus

<400>  73
atcatagcat agcgatgttt cttttggaac tatagc                                36

<210>  74
<211>  36
<212>  DNA
<213>  Lactobacillus fermentum

<400>  74
gtcttggatg agtgtcagat cagtagttcc gagtac                                36

<210>  75
<211>  36
<212>  DNA
<213>  Enterococcus faecalis

<400>  75
gttttagagt catgttgttt agaatggtac caaaac                                36

<210>  76
<211>  36
<212>  DNA
<213>  Bacillus cereus

<400>  76
gttttagagt catgttgttt agaatggtac caaaac                                36

<210>  77
<211>  36
<212>  DNA
<213>  Enterococcus faecalis

<400>  77
gttttagagt catgttgttt agaatggtac caaaac                                36

<210>  78
<211>  36
<212>  DNA
<213>  Artificial sequence

<220>
<223>  CRISPR repeat consensus Locus 14

<400>  78

gttttagagc tatgttgaat tgaatgcttc caaaac                                    36

<210>  79
<211>  36
<212>  DNA
<213>  Enterococcus faecalis

<400>  79
gtttttgtac tctcaataat ttcttatcag taaaac                                    36

<210>  80
<211>  47
<212>  DNA
<213>  Artificial sequence

<220>
<223>  CRISPR repeat consensus Locus 16

<400>  80
gttgtgctgt atctcaaaga tacggataaa tgaaagcaat tcacaac                        47

<210>  81
<211>  47
<212>  DNA
<213>  Artificial sequence

<220>
<223>  CRISPR repeat consensus Locus 17

<400>  81
gttgtgacta atcacgaaga taataaattt tgaaagcaat tcacaac                        47

<210>  82
<211>  47
<212>  DNA
<213>  Artificial sequence

<220>
<223>  CRISPR repeat consensus Locus 18

<400>  82
gctgtgacta atcacgaaga taataatttt tgaaagcaat tcacaac                        47

<210>  83
<211>  47
<212>  DNA
<213>  Artificial sequence

<220>
<223>  CRISPR repeat consensus Locus 19

<400>  83
gctgtgacta atcacgaaga taataatttt tgaaagcaat tcacaac                        47

<210>  84
<211>  47
<212>  DNA
<213>  Artificial sequence

<220>
<223>  CRISPR repeat consensus Locus 20

<400>  84

gctgtgacta atcacgaaga taataatttt tgaaagcaat tcacaac 47

<210> 85
<211> 47
<212> DNA
<213> Artificial sequence

<220>
<223> CRISPR repeat consensus Locus 21

<400> 85
gttgtgttat atctcaaaga tactcaaaaa tgaaagcaat tcacaac 47

<210> 86
<211> 47
<212> DNA
<213> Artificial sequence

<220>
<223> CRISPR repeat consensus Locus 22

<400> 86
gctgtgacta atcacgaaga taataatttt tgaaagcaat tcacaac 47

<210> 87
<211> 46
<212> DNA
<213> Artificial sequence

<220>
<223> CRISPR repeat consensus Locus 23

<400> 87
gttgggaatc ataagtaaaa atacaatttt gaaagcaatt cacaac 46

<210> 88
<211> 36
<212> DNA
<213> Artificial sequence

<220>
<223> CRISPR repeat consensus Locus 24

<400> 88
gttgtggttt gattaaagat tagaaaacac gatatt 36

<210> 89
<211> 36
<212> DNA
<213> Artificial sequence

<220>
<223> CRISPR repeat consensus Locus 25

<400> 89
gttgtggttt gattaaagat tagaaaacac gatatc 36

<210> 90
<211> 36
<212> DNA
<213> Artificial sequence

<220>

<223>  CRISPR repeat consensus Locus 26

<400>  90
gttgtggttt gattaaagat tagaaaacac gatatt                                36

<210>  91
<211>  36
<212>  DNA
<213>  Artificial sequence

<220>
<223>  CRISPR repeat consensus Locus 27

<400>  91
aggttatcag tcattccgat ggtggggtat cggaac                                36

<210>  92
<211>  36
<212>  DNA
<213>  Artificial sequence

<220>
<223>  CRISPR repeat consensus Locus 28

<400>  92
aggttatcag tcattccgat ggtggggtat cggaac                                36

<210>  93
<211>  36
<212>  DNA
<213>  Bacillus cereus

<400>  93
atcatatcat atcaagtttt ggtaagttgc tatagt                                36

<210>  94
<211>  36
<212>  DNA
<213>  Brevibacillus laterosporus

<400>  94
atcatatcat atcaagtttt ggtaagttgc tatagt                                36

<210>  95
<211>  36
<212>  DNA
<213>  Artificial Sequence

<220>
<223>  anti repeat Locus 8

<400>  95
atcatggtac agcaatggct ttgatgtttc tatgat                                36

<210>  96
<211>  36
<212>  DNA
<213>  Bacillus cereus

<400>  96
atcatatcat agcgatgttt cttaagttat tatagt                                36

<210>  97

<211>  38
<212>  DNA
<213>  Lactobacillus fermentum

<400>  97
atcttgacta tgagtgttag atcagtagtc tcgaatgt                              38

<210>  98
<211>  38
<212>  DNA
<213>  Enterococcus faecalis

<400>  98
attttaactt gctatgttgt ttagaatagt cccaaaag                              38

<210>  99
<211>  38
<212>  DNA
<213>  Bacillus cereus

<400>  99
attttaactt gctatgttgt ttagaatagt cccaaaag                              38

<210>  100
<211>  38
<212>  DNA
<213>  Enterococcus faecalis

<400>  100
attttaactt gctatgttgt ttagaatagt cccaaaag                              38

<210>  101
<211>  38
<212>  DNA
<213>  Artificial Sequence

<220>
<223>  anti repeat Locus 14

<400>  101
attttaactt gctatattga attgaatgct accaatgg                              38

<210>  102
<211>  38
<212>  DNA
<213>  Enterococcus faecalis

<400>  102
attttactga taagaaatta ttgagaatct acaaaaat                              38

<210>  103
<211>  47
<212>  DNA
<213>  Artificial sequence

<220>
<223>  anti repeat Locus 16

<400>  103
attgtgaatt gctttcggtt acaaatctaa aaattatttt tttaact                    47

<210>  104
<211>  47

<212>  DNA
<213>  Artificial sequence

<220>
<223>  anti repeat Locus 17

<400>  104
attgtgaatt gctttcaaaa tttatatgac aaatatagaa gaaaaaa                47

<210>  105
<211>  47
<212>  DNA
<213>  Artificial sequence

<220>
<223>  anti repeat Locus 18

<400>  105
attgtgaatt gctttcaaaa ttattgatac aaatataaaa gaaaaaa                47

<210>  106
<211>  47
<212>  DNA
<213>  Artificial sequence

<220>
<223>  anti repeat Locus 19

<400>  106
attgtgaatt gctttcaaaa ttattgatac aaatataaaa gaaaaaa                47

<210>  107
<211>  47
<212>  DNA
<213>  Artificial sequence

<220>
<223>  anti repeat Locus 20

<400>  107
attgtgaatt gctttcaaaa ttattgatac aaatataaaa gaaaaaa                47

<210>  108
<211>  47
<212>  DNA
<213>  Artificial sequence

<220>
<223>  anti repeat Locus 21

<400>  108
attgtgaatt gctttcggat acaaatctaa aaataatatt tttaatc                47

<210>  109
<211>  47
<212>  DNA
<213>  Artificial sequence

<220>
<223>  anti repeat Locus 22

<400>  109
attgtgaatt gcttttcaaa attattaata caaatataga agaaaaa                47

<210>  110
<211>  45
<212>  DNA
<213>  Artificial sequence

<220>
<223>  anti repeat Locus 23

<400>  110
attgtgaatt gcttcaaaat tgtattgcaa taatacaata aaata                45

<210>  111
<211>  36
<212>  DNA
<213>  Artificial sequence

<220>
<223>  anti repeat Locus 24

<400>  111
attgtgataa gattaaagat tagaaatttt aatcag                          36

<210>  112
<211>  36
<212>  DNA
<213>  Artificial sequence

<220>
<223>  anti repeat Locus 25

<400>  112
attgtgataa gattaaagat tagaaatttt aatcag                          36

<210>  113
<211>  36
<212>  DNA
<213>  Artificial sequence

<220>
<223>  anti repeat Locus 26

<400>  113
attgtgataa gattaaagat tagaaatttt aatcag                          36

<210>  114
<211>  39
<212>  DNA
<213>  Artificial sequence

<220>
<223>  anti repeat Locus 27

<400>  114
gttgccgata gtccccacca tcggaatgac tgatacgct                       39

<210>  115
<211>  39
<212>  DNA
<213>  Artificial sequence

<220>
<223>  anti repeat Locus 28

<400>  115
gttgccgata gtccccacca tcggaataac tgatacgct                              39

<210>  116
<211>  16
<212>  RNA
<213>  Bacillus cereus

<400>  116
gcuauaguuc cuuacu                                                       16

<210>  117
<211>  16
<212>  RNA
<213>  Brevibacillus laterosporus

<400>  117
gcuauaguuc cuuacc                                                       16

<210>  118
<211>  19
<212>  RNA
<213>  Artificial sequence

<220>
<223>  sgRNA repeat region for Cas-Locus 8

<400>  118
gucauaguuc cauuaaagc                                                    19

<210>  119
<211>  20
<212>  RNA
<213>  Bacillus cereus

<400>  119
gcuauaguuc caaaagaaac                                                   20

<210>  120
<211>  21
<212>  RNA
<213>  Lactobacillus fermentum

<400>  120
gucuuggaug agugucagau c                                                 21

<210>  121
<211>  17
<212>  RNA
<213>  Enterococcus faecalis

<400>  121
guuuuagagu cauguug                                                      17

<210>  122
<211>  17
<212>  RNA
<213>  Bacillus cereus

<400>  122
guuuuagagu cauguug                                                      17

<210>  123
<211>  17
<212>  RNA
<213>  Enterococcus faecalis

<400>  123
guuuuagagu cauguug                                                    17

<210>  124
<211>  17
<212>  RNA
<213>  Artificial sequence

<220>
<223>  sgRNA repeat region for Cas-Locus 14

<400>  124
guuuuagagc uauguug                                                    17

<210>  125
<211>  16
<212>  RNA
<213>  Enterococcus faecalis

<400>  125
guuuuuguac ucucaa                                                     16

<210>  126
<211>  22
<212>  RNA
<213>  Artificial sequence

<220>
<223>  sgRNA repeat region for Cas-Locus 16

<400>  126
guugugaauu gcuuucauuu au                                              22

<210>  127
<211>  25
<212>  RNA
<213>  Artificial sequence

<220>
<223>  sgRNA repeat region for Cas-Locus 17

<400>  127
guugugaauu gcuuucaaaa uuuau                                           25

<210>  128
<211>  26
<212>  RNA
<213>  Artificial sequence

<220>
<223>  sgRNA repeat region for Cas-Locus 18

<400>  128
guugugaauu gcuuucaaaa auuauu                                          26

<210>  129
<211>  26
<212>  RNA

<213>  Artificial sequence

<220>
<223>  sgRNA repeat region for Cas-Locus 19

<400>  129
guugugaauu gcuuucaaaa auuauu                                          26

<210>  130
<211>  26
<212>  RNA
<213>  Unknown

<220>
<223>  sgRNA repeat region for Cas-Locus 20

<400>  130
guugugaauu gcuuucaaaa auuauu                                          26

<210>  131
<211>  26
<212>  RNA
<213>  Artificial sequence

<220>
<223>  sgRNA repeat region for Cas-Locus 21

<400>  131
guugugaauu gcuuucauuu uugagu                                          26

<210>  132
<211>  23
<212>  RNA
<213>  Artificial sequence

<220>
<223>  sgRNA repeat region for Cas-Locus 22

<400>  132
guugugaauu gcuuucaaaa auu                                             23

<210>  133
<211>  22
<212>  RNA
<213>  Artificial sequence

<220>
<223>  sgRNA repeat region for Cas-Locus 23

<400>  133
guugugaauu gcuuucaaaa uu                                              22

<210>  134
<211>  16
<212>  RNA
<213>  Artificial sequence

<220>
<223>  sgRNA repeat region for Cas-Locus 24

<400>  134
guugugguuu gauuaa                                                     16

<210>  135
<211>  16
<212>  RNA
<213>  Artificial sequence

<220>
<223>  sgRNA repeat region for Cas-Locus 25

<400>  135
guugugguuu gauuaa                                                    16

<210>  136
<211>  16
<212>  RNA
<213>  Artificial sequence

<220>
<223>  sgRNA repeat region for Cas-Locus 26

<400>  136
guugugguuu gauuaa                                                    16

<210>  137
<211>  15
<212>  RNA
<213>  Artificial sequence

<220>
<223>  sgRNA repeat region for Cas-Locus 27

<400>  137
guuccgauac cccac                                                     15

<210>  138
<211>  15
<212>  RNA
<213>  Artificial sequence

<220>
<223>  sgRNA repeat region for Cas-Locus 28

<400>  138
guuccgauac cccac                                                     15

<210>  139
<211>  16
<212>  RNA
<213>  Bacillus cereus

<400>  139
gguaaguugc uauagu                                                    16

<210>  140
<211>  16
<212>  RNA
<213>  Brevibacillus laterosporus

<400>  140
gguaaguugc uauagu                                                    16

<210>  141
<211>  19
<212>  RNA

<213>  Artificial sequence

<220>
<223>  sgRNA anti-repeat region for Cas-Locus 8

<400>  141
gcuuugaugu uucuaugau                                                    19

<210>  142
<211>  20
<212>  RNA
<213>  Bacillus cereus

<400>  142
guuucuuaag uuauuauagu                                                   20

<210>  143
<211>  23
<212>  RNA
<213>  Lactobacillus fermentum

<400>  143
gaucuaacac ucauagucaa gau                                               23

<210>  144
<211>  19
<212>  RNA
<213>  Enterococcus faecalis

<400>  144
caacauagca aguuaaaau                                                    19

<210>  145
<211>  19
<212>  RNA
<213>  Bacillus cereus

<400>  145
caacauagca aguuaaaau                                                    19

<210>  146
<211>  19
<212>  RNA
<213>  Enterococcus faecalis

<400>  146
caacauagca aguuaaaau                                                    19

<210>  147
<211>  19
<212>  RNA
<213>  Artificial sequence

<220>
<223>  sgRNA anti-repeat region for Cas-Locus 14

<400>  147
caauauagca aguuaaaau                                                    19

<210>  148
<211>  18
<212>  RNA
<213>  Enterococcus faecalis

<400>  148
uugagaaucu acaaaaau                                                    18

<210>  149
<211>  22
<212>  RNA
<213>  Artificial sequence

<220>
<223>  sgRNA anti-repeat region for Cas-Locus 16

<400>  149
guaaccgaaa gcaauucaca au                                               22

<210>  150
<211>  25
<212>  RNA
<213>  Artificial Sequence

<220>
<223>  sgRNA anti-repeat region for Cas-Locus 17

<400>  150
auaaauuuug aaagcaauuc acaau                                            25

<210>  151
<211>  25
<212>  RNA
<213>  Artificial sequence

<220>
<223>  sgRNA anti-repeat region for Cas-Locus 18

<400>  151
aauaauuuug aaagcaauuc acaau                                            25

<210>  152
<211>  25
<212>  RNA
<213>  Artificial sequence

<220>
<223>  sgRNA anti-repeat region for Cas-Locus 19

<400>  152
aauaauuuug aaagcaauuc acaau                                            25

<210>  153
<211>  25
<212>  RNA
<213>  Unknown

<220>
<223>  sgRNA anti-repeat region for Cas-Locus 20

<400>  153
aauaauuuug aaagcaauuc acaau                                            25

<210>  154
<211>  26
<212>  RNA
<213>  Artificial sequence

<220>
<223>  sgRNA anti-repeat region for Cas-Locus 21

<400>  154
auuuguaucc gaaagcaauu cacaau                                          26

<210>  155
<211>  23
<212>  RNA
<213>  Artificial sequence

<220>
<223>  sgRNA anti-repeat region for Cas-Locus 22

<400>  155
aauuuugaaa agcaauucac aau                                             23

<210>  156
<211>  21
<212>  RNA
<213>  Artificial sequence

<220>
<223>  sgRNA anti-repeat region for Cas-Locus 23

<400>  156
aauuuugaag caauucacaa u                                               21

<210>  157
<211>  16
<212>  RNA
<213>  Artificial sequence

<220>
<223>  sgRNA anti-repeat region for Cas-Locus 24

<400>  157
uuaaucuuau cacaau                                                     16

<210>  158
<211>  16
<212>  RNA
<213>  Artificial sequence

<220>
<223>  sgRNA anti-repeat region for Cas-Locus 25

<400>  158
uuaaucuuau cacaau                                                     16

<210>  159
<211>  16
<212>  RNA
<213>  Artificial sequence

<220>
<223>  sgRNA anti-repeat region for Cas-Locus 26

<400>  159
uuaaucuuau cacaau                                                     16

<210>  160

<211>  18
<212>  RNA
<213>  Artificial sequence

<220>
<223>  sgRNA anti-repeat region for Cas-Locus 27

<400>  160
guggggacua ucggcaac                                                    18

<210>  161
<211>  18
<212>  RNA
<213>  Artificial sequence

<220>
<223>  sgRNA anti-repeat region for Cas-Locus 28

<400>  161
guggggacua ucggcaac                                                    18

<210>  162
<211>  118
<212>  RNA
<213>  Bacillus cereus

<400>  162
aagggcaaca gacccgaggc guuggggauc gccuagcccg uuuuuacggg cucucccuau      60
auucaaaaua augacaaacg agcaccuugg agaauuuauu uccgaggugc uuuuuuuu        118

<210>  163
<211>  118
<212>  RNA
<213>  Brevibacillus laterosporus

<400>  163
aagggcaaca gacccgaggc guuggggauc gccuagcccg uucuuacggg cucuccccac      60
aaucaaaaua augacaaacg agcaccucgg agcauuuauu uccgaggugc uuuuuuuu        118

<210>  164
<211>  114
<212>  RNA
<213>  Artificial sequence

<220>
<223>  sgRNA 3 prime tracrRNA for Cas-Locus 8

<400>  164
aagggcuuug gcccguggcg uaggggaucg ccugcccauu uuaaugggcu ucuccccauc      60
uauuuaauga gaguuuuaca accuuggcua uucuuaaaua gcuaagguuu uuuu            114

<210>  165
<211>  100
<212>  RNA
<213>  Bacillus cereus

<400>  165
aagggcaaug acccguggcg uuuggggauc gccuuauccu guauggauau ucuccccaug      60
uuaaaagcac cuacgcauaa cgcuauggug cuuuuauuuu                            100

<210>  166
<211>  99
<212>  RNA

<213>  Lactobacillus fermentum

<400>  166
caaacgagug guuuuccacg aguuauuacu uuugagccau guuauucugg ccauacauau      60
aauaaaaagg agucgauuua aucggcuccu uuuuacuuu                             99

<210>  167
<211>  53
<212>  RNA
<213>  Enterococcus faecalis

<400>  167
aagguuuuaa ccguaaucaa cuguaaagug gcgcuguuuc ggcgcuuuuu uuu             53

<210>  168
<211>  53
<212>  RNA
<213>  Bacillus cereus

<400>  168
aagguuuuaa ccguaaucaa cuguaaagug gcgcuguuuc ggcgcuuuuu uug             53

<210>  169
<211>  56
<212>  RNA
<213>  Enterococcus faecalis

<400>  169
aagguuucaa ccguaaucaa cuguaaagug gcgcuguuuc ggcgcuuuuu uuguuu          56

<210>  170
<211>  55
<212>  RNA
<213>  Artificial sequence

<220>
<223>  sgRNA 3 prime tracrRNA for Cas-Locus 14

<400>  170
aaggcuuugu ccgucaucag cuucuaggag caagcgcugu cucggcgcuu uuuuu           55

<210>  171
<211>  49
<212>  RNA
<213>  Enterococcus faecalis

<400>  171
aaggcauuuu gccgaauuua ccgcccuaca uauguagggc gguuuuuuu                  49

<210>  172
<211>  51
<212>  RNA
<213>  Artificial sequence

<220>
<223>  sgRNA 3 prime tracrRNA for Cas-Locus 16

<400>  172
aaggauuauu ccguugugaa aacauucaag gcgggguaac ucgccuuuuc c               51

<210>  173
<211>  51
<212>  RNA

<213>  Artificial sequence

<220>
<223>  sgRNA 3 prime tracrRNA for Cas-Locus 17

<400>  173
aaggauuauu ccguugugaa aacauucaag gcggggcaac ucgccuuuuu u                51

<210>  174
<211>  59
<212>  RNA
<213>  Artificial sequence

<220>
<223>  sgRNA 3 prime tracrRNA for Cas-Locus 18

<400>  174
aaggauuauu ccguugugaa aacauuuagc gccucgacua ccuucggggc auuuuuauu        59

<210>  175
<211>  60
<212>  RNA
<213>  Artificial sequence

<220>
<223>  sgRNA 3 prime tracrRNA for Cas-Locus 19

<400>  175
aaggauuauu ccguugugaa aacauuuagc gccucgacua ccuucggggc auuuuuauua       60

<210>  176
<211>  60
<212>  RNA
<213>  Unknown

<220>
<223>  sgRNA 3 prime tracrRNA for Cas-Locus 20

<400>  176
aaggauuauu ccguugugaa aacauuuaga gccucgacua ccuucggggc auuuuuauuu       60

<210>  177
<211>  60
<212>  RNA
<213>  Artificial sequence

<220>
<223>  sgRNA 3 prime tracrRNA for Cas-Locus 21

<400>  177
aaggauuauu ccguugugaa aacauuuagc gccucgccua ucugcggggc auuuuuauuu       60

<210>  178
<211>  57
<212>  RNA
<213>  Artificial sequence

<220>
<223>  sgRNA 3 prime tracrRNA for Cas-Locus 22

<400>  178
aaggauuauu ccguugugaa aacauucaag gcggggcaac ucgucuuuuu ucuuuuu          57

<210>  179
<211>  52
<212>  RNA
<213>  Artificial sequence

<220>
<223>  sgRNA 3 prime tracrRNA for Cas-Locus 23

<400>  179
aaggauuauu ccguugugaa aacauuuaag gcggggagac ucgccuuuuu cu          52

<210>  180
<211>  55
<212>  RNA
<213>  Artificial sequence

<220>
<223>  sgRNA 3 prime tracrRNA for Cas-Locus 24

<400>  180
aaggcuauau gccguagaug aaaaucuuua guccugcuuc ggugggacuu uucau          55

<210>  181
<211>  55
<212>  RNA
<213>  Artificial sequence

<220>
<223>  sgRNA 3 prime tracrRNA for Cas-Locus 25

<400>  181
aaggcuacau gccguagaug aaaaucuuua guccugcuuc ggugggacuu uuuuu          55

<210>  182
<211>  55
<212>  RNA
<213>  Artificial sequence

<220>
<223>  sgRNA 3 prime tracrRNA for Cas-Locus 26

<400>  182
aaggcuauau gccguagaua aaaaucuuua guccugcuuc ggugggacuu uuuuu          55

<210>  183
<211>  66
<212>  RNA
<213>  Artificial sequence

<220>
<223>  sgRNA 3 prime tracrRNA for Cas-Locus 27

<400>  183
aaggcuaagu accucgguac gaagccaacc gauacaaacg gucaucucgg ugaccguuuu          60
ucuuuu                                                                  66

<210>  184
<211>  66
<212>  RNA
<213>  Artificial sequence

<220>
<223>  sgRNA 3 prime tracrRNA for Cas-Locus 28

<400>  184
aaggcugagu acuucgguac gaagccaacc gauacaaacg gccaccucgg uggccguuuu      60
uguuuu                                                                  66

<210>  185
<211>  174
<212>  RNA
<213>  Artificial Sequence

<220>
<223>  sgRNA for Cas-Locus 6

<220>
<221>  misc_feature
<222>  (1)..(20)
<223>  n is a, c, g, or u

<400>  185
nnnnnnnnnn nnnnnnnnnn gcuauaguuc cuuacugaaa gguaaguugc uauaguaagg      60
gcaacagacc cgaggcguug gggaucgccu agcccguuuu uacgggcucu cccuauauuc      120
aaaauaauga caaacgagca ccuuggagaa uuuauuuccg aggugcuuuu uuuu            174

<210>  186
<211>  174
<212>  RNA
<213>  Artificial Sequence

<220>
<223>  sgRNA for Cas-Locus 7

<220>
<221>  misc_feature
<222>  (1)..(20)
<223>  n is a, c, g, or u

<400>  186
nnnnnnnnnn nnnnnnnnnn gcuauaguuc cuuaccgaaa gguaaguugc uauaguaagg      60
gcaacagacc cgaggcguug gggaucgccu agcccguucu uacgggcucu ccccacaauc      120
aaaauaauga caaacgagca ccucggagca uuuauuuccg aggugcuuuu uuuu            174

<210>  187
<211>  176
<212>  RNA
<213>  Artificial Sequence

<220>
<223>  sgRNA for Cas-Locus 8

<220>
<221>  misc_feature
<222>  (1)..(20)
<223>  n is a, c, g, or u

<400>  187
nnnnnnnnnn nnnnnnnnnn gucauaguuc cauuaaagcg aaagcuuuga uguuucuaug      60
auaagggcuu uggcccgugg cguaggggau cgccugccca uuuuaauggg cuucucccca      120
ucuauuuaau gagaguuuua caaccuuggc uauucuuaaa uagcuaaggu uuuuuu          176

<210>  188
<211>  164
<212>  RNA
<213>  Artificial Sequence

<220>
<223>  sgRNA for Cas-Locus 9

<220>
<221>  misc_feature
<222>  (1)..(20)
<223>  n is a, c, g, or u

<400>  188
nnnnnnnnnn nnnnnnnnnn gcuauaguuc caaaagaaac gaaaguuucu uaaguuauua      60
uaguaagggc aaugacccgu ggcguuuggg gaucgccuua uccuguaugg auauucuccc     120
cauguuaaaa gcaccuacgc auaacgcuau ggugcuuuua uuuu                      164

<210>  189
<211>  167
<212>  RNA
<213>  Artificial Sequence

<220>
<223>  sgRNA for Cas-Locus 10

<220>
<221>  misc_feature
<222>  (1)..(20)
<223>  n is a, c, g, or u

<400>  189
nnnnnnnnnn nnnnnnnnnn gucuuggaug agugucagau cgaaagaucu aacacucaua      60
gucaagauca aacgaguggu uuuccacgag uuauuacuuu ugagccaugu uauucuggcc     120
auacauauaa uaaaaaggag ucgauuuaau cggcuccuuu uuacuuu                   167

<210>  190
<211>  113
<212>  RNA
<213>  Artificial Sequence

<220>
<223>  sgRNA for Cas-Locus 11

<220>
<221>  misc_feature
<222>  (1)..(20)
<223>  n is a, c, g, or u

<400>  190
nnnnnnnnnn nnnnnnnnnn guuuuagagu cauguuggaa acaacauagc aaguuaaaau      60
aagguuuuaa ccguaaucaa cuguaaagug gcgcuguuuc ggcgcuuuuu uuu            113

<210>  191
<211>  113
<212>  RNA
<213>  Artificial Sequence

<220>
<223>  sgRNA for Cas-Locus 12

<220>
<221>  misc_feature
<222>  (1)..(20)
<223>  n is a, c, g, or u

<400>  191

nnnnnnnnnn nnnnnnnnnn guuuuagagu cauguuggaa acaacauagc aaguuaaaau 60
aagguuuuaa ccguaaucaa cuguaaagug gcgcuguuuc ggcgcuuuuu uug 113

<210> 192
<211> 116
<212> RNA
<213> Artificial Sequence

<220>
<223> sgRNA for Cas-Locus 13

<220>
<221> misc_feature
<222> (1)..(20)
<223> n is a, c, g, or u

<400> 192
nnnnnnnnnn nnnnnnnnnn guuuuagagu cauguuggaa acaacauagc aaguuaaaau 60
aagguuucaa ccguaaucaa cuguaaagug gcgcuguuuc ggcgcuuuuu uuguuu 116

<210> 193
<211> 115
<212> RNA
<213> Artificial Sequence

<220>
<223> sgRNA for Cas-Locus 14

<220>
<221> misc_feature
<222> (1)..(20)
<223> n is a, c, g, or u

<400> 193
nnnnnnnnnn nnnnnnnnnn guuuuagagc uauguuggaa acaauauagc aaguuaaaau 60
aaggcuuugu ccgucaucag cuucuaggag caagcgcugu cucggcgcuu uuuuu 115

<210> 194
<211> 107
<212> RNA
<213> Artificial Sequence

<220>
<223> sgRNA for Cas-Locus 15

<220>
<221> misc_feature
<222> (1)..(20)
<223> n is a, c, g, or u

<400> 194
nnnnnnnnnn nnnnnnnnnn guuuuuguac ucucaagaaa uugagaaucu acaaaaauaa 60
ggcauuuugc cgaauuuacc gcccuacaua uguagggcgg uuuuuuu 107

<210> 195
<211> 119
<212> RNA
<213> Artificial Sequence

<220>
<223> sgRNA for Cas-Locus 16

<220>

<221>  misc_feature
<222>  (1)..(20)
<223>  n is a, c, g, or u

<400>  195
nnnnnnnnnn nnnnnnnnnn guugugaauu gcuuucauuu augaaaguaa ccgaaagcaa      60
uucacaauaa ggauuauucc guugugaaaa cauucaaggc gggguaacuc gccuuuucc      119

<210>  196
<211>  125
<212>  RNA
<213>  Artificial Sequence

<220>
<223>  sgRNA for Cas-Locus 17

<220>
<221>  misc_feature
<222>  (1)..(20)
<223>  n is a, c, g, or u

<400>  196
nnnnnnnnnn nnnnnnnnnn guugugaauu gcuuucaaaa uuuaugaaaa uaaauuuuga      60
aagcaauuca caauaaggau uauuccguug ugaaaacauu caaggcgggg caacucgccu     120
uuuuu                                                                 125

<210>  197
<211>  134
<212>  RNA
<213>  Artificial Sequence

<220>
<223>  sgRNA for Cas-Locus 18

<220>
<221>  misc_feature
<222>  (1)..(20)
<223>  n is a, c, g, or u

<400>  197
nnnnnnnnnn nnnnnnnnnn guugugaauu gcuuucaaaa auuauugaaa aauaauuuug      60
aaagcaauuc acaauaagga uuauuccguu gugaaaacau uuagcgccuc gacuaccuuc     120
ggggcauuuu uauu                                                       134

<210>  198
<211>  135
<212>  RNA
<213>  Artificial Sequence

<220>
<223>  sgRNA for Cas-Locus 19

<220>
<221>  misc_feature
<222>  (1)..(20)
<223>  n is a, c, g, or u

<400>  198
nnnnnnnnnn nnnnnnnnnn guugugaauu gcuuucaaaa auuauugaaa aauaauuuug      60
aaagcaauuc acaauaagga uuauuccguu gugaaaacau uuagcgccuc gacuaccuuc     120
ggggcauuuu uauua                                                      135

<210>  199

<211>  135
<212>  RNA
<213>  Artificial Sequence

<220>
<223>  sgRNA for Cas-Locus 20

<220>
<221>  misc_feature
<222>  (1)..(20)
<223>  n is a, c, g, or u

<400>  199
nnnnnnnnnn nnnnnnnnnn guugugaauu gcuuucaaaa auuauugaaa aauaauuuug      60
aaagcaauuc acaauaagga uuauuccguu gugaaaacau uuagagccuc gacuaccuuc     120
ggggcauuuu uauuu                                                      135

<210>  200
<211>  136
<212>  RNA
<213>  Artificial Sequence

<220>
<223>  sgRNA for Cas-Locus 21

<220>
<221>  misc_feature
<222>  (1)..(20)
<223>  n is a, c, g, or u

<400>  200
nnnnnnnnnn nnnnnnnnnn guugugaauu gcuuucauuu uugagugaaa auuuguaucc      60
gaaagcaauu cacaauaagg auuauuccgu ugugaaaaca uuuagcgccu cgccuaucug     120
cggggcauuu uuauuu                                                     136

<210>  201
<211>  127
<212>  RNA
<213>  Artificial Sequence

<220>
<223>  sgRNA for Cas-Locus 22

<220>
<221>  misc_feature
<222>  (1)..(20)
<223>  n is a, c, g, or u

<400>  201
nnnnnnnnnn nnnnnnnnnn guugugaauu gcuuucaaaa auugaaaaau uuugaaaagc      60
aauucacaau aaggauuauu ccguugugaa aacauucaag gcggggcaac ucgucuuuuu     120
ucuuuuu                                                               127

<210>  202
<211>  119
<212>  RNA
<213>  Artificial Sequence

<220>
<223>  sgRNA for Cas-Locus 23

<220>
<221>  misc_feature

<222>  (1)..(20)
<223>  n is a, c, g, or u

<400>  202
nnnnnnnnnn nnnnnnnnnn guugugaauu gcuuucaaaa uugaaaaauu uugaagcaau      60
ucacaauaag gauuauuccg uugugaaaac auuuaaggcg gggagacucg ccuuuuucu      119

<210>  203
<211>  111
<212>  RNA
<213>  Artificial Sequence

<220>
<223>  sgRNA for Cas-Locus 24

<220>
<221>  misc_feature
<222>  (1)..(20)
<223>  n is a, c, g, or u

<400>  203
nnnnnnnnnn nnnnnnnnnn guugugguuu gauuaagaaa uuaaucuuau cacaauaagg      60
cuauaugccg uagaugaaaa ucuuuagucc ugcuucggug ggacuuuuca u              111

<210>  204
<211>  111
<212>  RNA
<213>  Artificial Sequence

<220>
<223>  sgRNA for Cas-Locus 25

<220>
<221>  misc_feature
<222>  (1)..(20)
<223>  n is a, c, g, or u

<400>  204
nnnnnnnnnn nnnnnnnnnn guugugguuu gauuaagaaa uuaaucuuau cacaauaagg      60
cuacaugccg uagaugaaaa ucuuuagucc ugcuucggug ggacuuuuuu u              111

<210>  205
<211>  111
<212>  RNA
<213>  Artificial Sequence

<220>
<223>  sgRNA for Cas-Locus 26

<220>
<221>  misc_feature
<222>  (1)..(20)
<223>  n is a, c, g, or u

<400>  205
nnnnnnnnnn nnnnnnnnnn guugugguuu gauuaagaaa uuaaucuuau cacaauaagg      60
cuauaugccg uagauaaaaa ucuuuagucc ugcuucggug ggacuuuuuu u              111

<210>  206
<211>  123
<212>  RNA
<213>  Artificial Sequence

<220>
<223>  sgRNA for Cas-Locus 27

<220>
<221>  misc_feature
<222>  (1)..(20)
<223>  n is a, c, g, or u

<400>  206
nnnnnnnnnn nnnnnnnnnn guuccgauac cccacgaaag uggggacuau cggcaacaag      60
gcuaaguacc ucgguacgaa gccaaccgau acaaacgguc aucucgguga ccguuuuucu     120
uuu                                                                   123

<210>  207
<211>  123
<212>  RNA
<213>  Artificial Sequence

<220>
<223>  sgRNA for Cas-Locus 28

<220>
<221>  misc_feature
<222>  (1)..(20)
<223>  n is a, c, g, or u

<400>  207
nnnnnnnnnn nnnnnnnnnn guuccgauac cccacgaaag uggggacuau cggcaacaag      60
gcugaguacu ucgguacgaa gccaaccgau acaaacggcc accucggugg ccguuuuugu     120
uuu                                                                   123

## Description

This application claims the benefit of U.S. Provisional Application No. 62/306904, filed March 11, 2016, which is incorporated herein in its entirety by reference.

### FIELD

The disclosure relates to the field of plant molecular biology, in particular, to compositions for novel guided Cas9 endonuclease systems and compositions and methods for altering the genome of a cell.

### REFERENCE TO SEQUENCE LISTING SUBMITTED ELECTRONICALLY

The official copy of the sequence listing is submitted electronically via EFS-Web as an ASCII formatted sequence listing with a file named 20170223_BB2398PCT_ST25.txt created on February 23, 2017 and having a size of 605 kilobytes and is filed concurrently with the specification. The sequence listing contained in this ASCII formatted document is part of the specification and is herein incorporated by reference in its entirety.

### BACKGROUND

Recombinant DNA technology has made it possible to modify (edit) specific endogenous chromosomal sequences and/or insert DNA sequences at targeted genomic locations thus altering the organism's phenotype. Site-specific integration techniques, which employ site-specific recombination systems, as well as other types of recombination technologies, have been used to generate targeted insertions of genes of interest in a variety of organism. Genome-editing techniques such as designer zinc finger nucleases (ZFNs) or transcription activator-like effector nucleases (TALENs), or homing meganucleases, are available for producing targeted genome perturbations, but these systems tends to have a low specificity and employ designed nucleases that need to be redesigned for each target site, which renders them costly and time-consuming to prepare.

Although several approaches have been developed to target a specific site for modification in the genome of an organism, there still remains a need for new genome engineering technologies that are affordable, easy to set up, scalable, and amenable to targeting multiple positions within the genome of an organism.

### BRIEF SUMMARY

Compositions and methods are provided for novel Cas9 systems and elements comprising such systems, including, but not limiting to, novel guide polynucleotide/Cas9 endonucleases complexes, single guide RNAs, guide RNA elements, and Cas9 endonucleases.

In one embodiment of the disclosure, the guide RNA is a single guide RNA capable of forming a guide RNA/Cas9 endonuclease complex, wherein said guide RNA/Cas9 endonuclease complex can recognize, bind to, and optionally nick or cleave a target sequence, wherein said single guide RNA is selected from the group consisting of SEQ ID NOs: 185-207, a functional fragment of SEQ ID NOs: 185-207, and a functional variant of SEQ ID NOs: 185-207.

In one embodiment of the disclosure, the guide RNA is single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of SEQ ID NOs: 139-184, a functional fragment of SEQ ID NOs: 139-184, and a functional variant of SEQ ID NOs: 139-184.

In one embodiment of the disclosure, the guide RNA is a single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of SEQ ID NOs: 116-138, a functional fragment of SEQ ID NOs: 116-138, and a functional variant of SEQ ID NOs: 116-138.

The guide RNA can also be a dual molecule comprising a chimeric non-naturally occurring crRNA linked to a tracrRNA, wherein said chimeric non-naturally occurring crRNA comprises a nucleotide sequence selected from the group consisting of SEQ ID NOs: 116-138, a functional fragment of SEQ ID NOs: 116-138, and a functional variant of SEQ ID NOs: 116-138, and/or wherein said tracrRNA comprises a nucleotide sequence selected from the group consisting of SEQ ID NOs: 139-184, a functional fragment of SEQ ID NOs: 139-184, and a functional variant of SEQ ID NOs: 139-184 .

In one embodiment of the disclosure, the guide RNA/Cas9 endonuclease complex is a guide RNA/Cas9 endonuclease complex comprising at least one guide RNA and a Cas9 endonuclease, wherein said Cas9 endonuclease is encoded by a DNA sequence selected from the group consisting of SEQ ID NOs: 24-46, wherein said guide RNA/Cas9 endonuclease complex is capable of recognizing, binding to, and optionally nicking or cleaving all or part of a target sequence.

In one embodiment of the disclosure, the method is a method for modifying a target site in the genome of a cell, the method comprising introducing into said cell at least one guide RNA and at least one Cas9 endonuclease selected from the group consisting of SEQ ID NOs: 47-69, a functional fragment of SEQ ID NOs: 47-69, and a functional variant of SEQ ID NOs: 47-69 , wherein said guide RNA and Cas9 endonuclease can form a complex that is capable of recognizing, binding to, and optionally nicking or cleaving all or part of said target site. The method can further comprise identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii).

In one embodiment of the disclosure, the method is a method for editing a nucleotide sequence in the genome of a cell, the method comprising introducing into said cell a polynucleotide modification template, at least one guide RNA and at least one Cas9 endonuclease selected from the group consisting of SEQ ID NOs: 47-69, a functional fragment of SEQ ID NOs: 47-69, and a functional variant of SEQ ID NOs: 47-69, wherein said polynucleotide modification template comprises at least one nucleotide modification of said nucleotide sequence, wherein said guide RNA and Cas9 endonuclease can form a complex that is capable of recognizing, binding to, and optionally nicking or cleaving all or part of said target site.

In one embodiment of the disclosure, the method is a method for modifying a target site in the genome of a cell, the method comprising introducing into said cell at least one guide RNA, at least one donor DNA, and at least one Cas9 endonuclease selected from the group consisting of SEQ ID NOs: 47-69, a functional fragment of SEQ ID NOs: 47-69, and a functional variant of SEQ ID NOs: 47-69, wherein said at least one guide RNA and at least one Cas9 endonuclease can form a complex that is capable of recognizing, binding to, and optionally nicking or cleaving all or part of said target site, wherein said donor DNA comprises a polynucleotide of interest.

Also provided are nucleic acid constructs, plants, plant cells, explants, seeds and grain having an altered target site or altered polynucleotide of interest produced by the methods described herein. Additional embodiments of the methods and compositions of the present disclosure are shown herein.

### BRIEF DESCRIPTION OF THE DRAWINGS AND THE SEQUENCE LISTING

The disclosure can be more fully understood from the following detailed description and the accompanying drawings and Sequence Listing, which form a part of this application. The sequence descriptions and sequence listing attached hereto comply with the rules governing nucleotide and amino acid sequence disclosures in patent applications as set forth in 37 C.F.R. §§1.821-1.825. The sequence descriptions contain the three letter codes for amino acids as defined in 37 C.F.R. §§ 1.821-1.825, which are incorporated herein by reference.

### Figures

Figure 1 shows a diagram of a genomic DNA region from *Bacillus cereus* representing a CRISPR-Cas locus (referred to as Locus 6) described herein. Arrows indicate the transcriptional directional of the anti-repeat and the CRISPR repeats within the CRISPR array (CRISPR array). Cas9 Gene ORF refers to the open reading frame of the Cas9 endonuclease. Cas1 and Cas2 refer to the open reading frame of the Cas1 and Cas2 protein, respectively.
Figure 2 shows a diagram of a genomic DNA region from *Brevibacillus laterosporus* representing a CRISPR-Cas locus (referred to as Locus 7) described herein.
Figure 3 shows a diagram of a genomic DNA region from *Bacillus* species representing a CRISPR-Cas locus (referred to as Locus 8) described herein.
Figure 4 shows a diagram of a genomic DNA region from *Bacillus cereus* representing a CRISPR-Cas locus (referred to as Locus 9) described herein.
Figure 5 shows a diagram of a genomic DNA region from *Lactobacillus fermentum* representing a CRISPR-Cas locus (referred to as Locus 10) described herein. Arrows indicate the transcriptional directional of the anti-repeat and the CRISPR repeat within the CRISPR array. Cas9 Gene ORF refers to the open reading frame of the Cas9 endonuclease. Cas1, Cas2, Csn2 refer to the open reading frame of the Cas1, Cas2 and Csn2 protein, respectively.
Figure 6 shows a diagram of a genomic DNA region from *Enterococcus faecalis* representing a CRISPR-Cas locus (referred to as Locus 11) described herein.
Figure 7 shows a diagram of a genomic DNA region from *Bacillus cereus* representing a CRISPR-Cas locus (referred to as Locus 12) described herein.
Figure 8 shows a diagram of a genomic DNA region from *Enterococcus faecalis* representing a CRISPR-Cas locus (referred to as Locus 13) described herein.
Figure 9 shows a diagram of a genomic DNA region from an unknown organism representing a CRISPR-Cas locus (referred to as Locus 14) described herein.
Figure 10 shows a diagram of a genomic DNA region from *Enterococcus faecalis* representing a CRISPR-Cas locus (referred to as Locus 15) described herein. Arrows indicate the transcriptional directional of the anti-repeat and the CRISPR repeats within the CRISPR array (CRISPR array). Cas9 Gene ORF refers to the open reading frame of the Cas9 endonuclease. Cas1, Cas2 and Cas7-Like protein refer to the open reading frame of the Cas1, Cas2 and Cas7-Like protein, respectively.
Figure 11 shows a diagram of a genomic DNA region from metagenomic material representing a CRISPR-Cas locus (referred to as Locus 16) described herein.
Figure 12 shows a diagram of a genomic DNA region from *Chryseobacterium* species representing a CRISPR-Cas locus (referred to as Locus 17) described herein.
Figure 13 shows a diagram of a genomic DNA region from metagenomic material representing a CRISPR-Cas locus (referred to as Locus 18) described herein.
Figure 14 shows a diagram of a genomic DNA region from metagenomic material representing a CRISPR-Cas locus (referred to as Locus 19) described herein.
Figure 15 shows a diagram of a genomic DNA region from an unknown organism representing a CRISPR-Cas locus (referred to as Locus 20) described herein.
Figure 16 shows a diagram of a genomic DNA region from metagenomic material representing a CRISPR-Cas locus (referred to as Locus 21) described herein.
Figure 17 shows a diagram of a genomic DNA region from metagenomic material representing a CRISPR-Cas locus (referred to as Locus 22) described herein.
Figure 18 shows a diagram of a genomic DNA region from metagenomic material representing a CRISPR-Cas locus (referred to as Locus 23) described herein.
Figure 19 shows a diagram of a genomic DNA region from metagenomic material representing a CRISPR-Cas locus (referred to as Locus 24) described herein.
Figure 20 shows a diagram of a genomic DNA region from metagenomic material representing a CRISPR-Cas locus (referred to as Locus 25) described herein.
Figure 21 shows a diagram of a genomic DNA region from metagenomic material representing a CRISPR-Cas locus (referred to as Locus 26) described herein.
Figure 22 shows a diagram of a genomic DNA region from metagenomic material representing a CRISPR-Cas locus (referred to as Locus 27) described herein.
Figure 23 shows a diagram of a genomic DNA region from metagenomic material representing a CRISPR-Cas locus (referred to as Locus 28) described herein.

### Sequences

**Table 1. Summary of Nucleic Acid and Protein SEQ ID Numbers**

| Description | Nucleic acid SEQ ID NO: | Protein SEQ ID NO: |
|---|---|---|
| Locus 6 | 1 | |
| Locus 7 | 2 | |
| Locus 8 | 3 | |
| Locus 9 | 4 | |
| Locus 10 | 5 | |
| Locus 11 | 6 | |
| Locus 12 | 7 | |
| Locus 13 | 8 | |
| Locus 14 | 9 | |
| Locus 15 | 10 | |
| Locus 16 | 11 | |
| Locus 17 | 12 | |
| Locus 18 | 13 | |
| Locus 19 | 14 | |
| Locus 20 | 15 | |
| Locus 21 | 16 | |
| Locus 22 | 17 | |
| Locus 23 | 18 | |
| Locus 24 | 19 | |
| Locus 25 | 20 | |
| Locus 26 | 21 | |
| Locus 27 | 22 | |
| Locus 28 | 23 | |
| Cas9 endonuclease from Locus 6 to Locus 28, respectively | 24-46 | 47-69 |
| CRISPR repeat consensus from Locus 6 to Locus 28, respectively | 70-92 | |
| Anti-Repeat from Locus 6 to Locus 28, respectively | 93-115 | |
| sgRNA repeat region ( Locus 6 to Locus 28, respectively) | 116-138 | |
| sgRNA anti Repeat region (Locus 6 to Locus 28, respectively) | 139-161 | |
| 3' tracrRNA in gRNA ( Locus 6 to Locus 28, respectively) | 162-184 | |
| sgRNAs | 185-207 | |

### DETAILED DESCRIPTION

Compositions are provided for novel Cas9 systems and elements comprising such systems, including, but not limiting to, novel guide polynucleotide/Cas9 endonucleases complexes, single guide RNAs, guide RNA elements, and Cas9 endonucleases. The present disclosure further includes compositions and methods for genome modification of a target sequence in the genome of a cell, for gene editing, and for inserting a polynucleotide of interest into the genome of a cell.

CRISPR (clustered regularly interspaced short palindromic repeats) loci refers to certain genetic loci encoding factors of DNA cleavage systems, for example, used by bacterial and archaeal cells to destroy foreign DNA (Horvath and Barrangou, 2010, Science 327:167-170). A CRISPR locus can consist of a CRISPR array, comprising short direct repeats (CRISPR repeats) separated by short variable DNA sequences (called 'spacers'), which can be flanked by diverse Cas (CRISPR-associated) genes. Multiple CRISPR-Cas systems have been described including Class 1 systems, with multisubunit effector complexes, and Class 2 systems, with single protein effectors (such as but not limiting to Cas9, Cpf1 ,C2c1,C2c2, C2c3). (Zetsche et al., 2015, Cell 163, 1-13; Shmakov et al., 2015, Molecular_Cell 60, 1-13; Makarova et al. 2015, Nature Reviews Microbiology Vol. 13:1-15, WO 2013/176772 A1 published on November 23, 2013 and incorporated by its entirety by reference herein). CRISPR systems belong to different classes, with different repeat patterns, sets of genes, and species ranges.

The type II CRISPR/Cas system from bacteria employs a crRNA (CRISPR RNA) and tracrRNA (trans-activating CRISPR RNA) to guide a Cas9 endonuclease (encoded by a *cas9* gene) to its DNA target. The crRNA contains a spacer region complementary to one strand of the double strand DNA target and a region that base pairs with the tracrRNA (trans-activating CRISPR RNA) forming a RNA duplex that directs the Cas9 endonuclease to cleave the DNA target. Spacers are acquired through a not fully understood process involving Cas1 and Cas2 proteins. All type II CRISPR-Cas loci contain *cas1* and *cas2* genes in addition to the *cas9* gene (Makarova et al. 2015, Nature Reviews Microbiology Vol. 13:1-15). Type II CRISPR-Cas loci can encode a tracrRNA, which is partially complementary to the repeats within the respective CRISPR array, and can comprise other proteins such as Csn1 and Csn2. The presence of *cas9* in the vicinity of *cas1* and *cas2* genes is the hallmark of type II loci (Makarova et al. 2015, Nature Reviews Microbiology Vol. 13:1-15).

The number of CRISPR-associated genes at a given CRISPR locus can vary between species (Haft et al., 2005, Computational Biology, PLoS Comput Biol 1(6): e60. doi:10.1371/journal.pcbi.0010060; Makarova et al. 2015, Nature Reviews Microbiology Vol. 13 :1-15; WO 2013/176772 A1 published on November 23, 2013 and incorporated by its entirety by reference herein).

"Cas9" (formerly referred to as Cas5, Csn1, or Csx12) herein refers to a Cas (CRISPR-associated) endonuclease that when in complex with a suitable polynucleotide component (such as crNucleotide and a tracrNucleotide, or a single guide polynucleotide) is capable of recognizing , binding to, and optionally nicking or cleaving all or part of a DNA target sequence. A Cas9 protein comprises a HNH domain and a RuvC nuclease domain, each of which can cleave a single DNA strand at a target sequence (the concerted action of both domains leads to DNA double-strand cleavage, whereas activity of one domain leads to a nick). In general, the RuvC domain comprises subdomains I, II and III, where domain I is located near the N-terminus of Cas9 and subdomains II and III are located in the middle of the protein, flanking the HNH domain (Hsu et al, Cell 157:1262-1278). Cas9 endonucleases are typically derived from a type II CRISPR system, which includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA (Makarova et al. 2015, Nature Reviews Microbiology Vol. 13:1-15).

The term "*cas9* gene" herein refers to a gene encoding a Cas9 endonuclease.

As used herein, the terms "guide polynucleotide/Cas9 endonuclease complex", "guide polynucleotide/Cas9 endonuclease system", " guide polynucleotide/Cas9 complex", "guide polynucleotide/Cas9 system", "Polynucleotide-guided endonuclease", "PGEN" , "guided Cas system" are used interchangeably herein and refer to at least one guide polynucleotide and at least one Cas9 endonuclease that are capable of forming a complex, wherein said guide polynucleotide/Cas9 endonuclease complex can direct the Cas9 endonuclease to a DNA target site, enabling the Cas9 endonuclease to recognize, bind to, and optionally nick or cleave (introduce a single or double strand break) the DNA target site. A guide polynucleotide/Cas9 endonuclease complex herein can comprise Cas9 protein(s) and suitable polynucleotide component(s) of any of the known CRISPR systems (Horvath and Barrangou, 2010, Science 327:167-170; Makarova et al. 2015, Nature Reviews Microbiology Vol. 13:1-15). A Cas9 endonuclease unwinds the DNA duplex at the target sequence and optionally cleaves at least one DNA strand, as mediated by recognition of the target sequence by a polynucleotide (such as, but not limited to, a crRNA or guide RNA) that is in complex with the Cas9 protein. Such recognition and cutting of a target sequence by a Cas9 endonuclease typically occurs if the correct protospacer-adjacent motif (PAM) is located at or adjacent to the 3' end of the DNA target sequence. Alternatively, a Cas9 protein herein may lack DNA cleavage or nicking activity, but can still specifically bind to a DNA target sequence when complexed with a suitable RNA component. (See also U.S. Patent Application US 2015-0082478 A1, published on March 19, 2015 and US 2015-0059010 A1, published on February 26, 2015, both are incorporated in their entirety by reference herein).

A guide polynucleotide/Cas9 endonuclease complex can cleave one or both strands of a DNA target sequence. A guide polynucleotide/Cas9 endonuclease complex that can cleave both strands of a DNA target sequence typically comprises a Cas9 protein that has all of its endonuclease domains in a functional state (e.g., wild type endonuclease domains or variants thereof retaining some or all activity in each endonuclease domain). Thus, a wild type Cas9 protein, or a variant thereof retaining some or all activity in each endonuclease domain of the Cas9 protein, is a suitable example of a Cas9 endonuclease that can cleave both strands of a DNA target sequence. A Cas9 protein comprising functional RuvC and HNH nuclease domains is an example of a Cas9 protein that can cleave both strands of a DNA target sequence. A guide polynucleotide/Cas9 endonuclease complex that can cleave one strand of a DNA target sequence can be characterized herein as having nickase activity (e.g., partial cleaving capability). A Cas9 nickase typically comprises one functional endonuclease domain that allows the Cas9 to cleave only one strand (i.e., make a nick) of a DNA target sequence. For example, a Cas9 nickase may comprise (i) a mutant, dysfunctional RuvC domain and (ii) a functional HNH domain (e.g., wild type HNH domain). As another example, a Cas9 nickase may comprise (i) a functional RuvC domain (e.g., wild type RuvC domain) and (ii) a mutant, dysfunctional HNH domain. Non-limiting examples of Cas9 nickases suitable for use herein are disclosed in U.S. Patent Appl. Publ. No. 2014/0189896, which is incorporated herein by reference.

A pair of Cas9 nickases can be used to increase the specificity of DNA targeting. In general, this can be done by providing two Cas9 nickases that, by virtue of being associated with RNA components with different guide sequences, target and nick nearby DNA sequences on opposite strands in the region for desired targeting. Such nearby cleavage of each DNA strand creates a double strand break (i.e., a DSB with single-stranded overhangs), which is then recognized as a substrate for non-homologous-end-joining, NHEJ (prone to imperfect repair leading to mutations) or homologous recombination, HR. Each nick can be at least about 5, 10, 15, 20, 30, 40, 50, 60, 70, 80, 90, or 100 (or any integer between 5 and 100) bases apart from each other, for example. One or two Cas9 nickase proteins herein can be used in a Cas9 nickase pair. For example, a Cas9 nickase with a mutant RuvC domain, but functioning HNH domain (i.e., Cas9 HNH+/RuvC-), could be used (e.g., *Streptococcus pyogenes* Cas9 HNH+/RuvC-). Each Cas9 nickase (e.g., Cas9 HNH+/RuvC-) would be directed to specific DNA sites nearby each other (up to 100 base pairs apart) by using suitable RNA components herein with guide RNA sequences targeting each nickase to each specific DNA site.

A Cas9 protein can be part of a fusion protein comprising one or more heterologous protein domains (e.g., 1, 2, 3, or more domains in addition to the Cas9 protein). Such a fusion protein may comprise any additional protein sequence, and optionally a linker sequence between any two domains, such as between Cas9 and a first heterologous domain. Examples of protein domains that may be fused to a Cas9 protein herein include, without limitation, epitope tags (e.g., histidine [His], V5, FLAG, influenza hemagglutinin [HA], myc, VSV-G, thioredoxin [Trx]), reporters (e.g., glutathione-5-transferase [GST], horseradish peroxidase [HRP], chloramphenicol acetyltransferase [CAT], beta-galactosidase, beta-glucuronidase [GUS], luciferase, green fluorescent protein [GFP], HcRed, DsRed, cyan fluorescent protein [CFP], yellow fluorescent protein [YFP], blue fluorescent protein [BFP]), and domains having one or more of the following activities: methylase activity, demethylase activity, transcription activation activity (e.g., VP16 or VP64), transcription repression activity, transcription release factor activity, histone modification activity, RNA cleavage activity and nucleic acid binding activity. A Cas9 protein can also be in fusion with a protein that binds DNA molecules or other molecules, such as maltose binding protein (MBP), S-tag, Lex A DNA binding domain (DBD), GAL4A DNA binding domain, and herpes simplex virus (HSV) VP16.

A guide polynucleotide/Cas9 endonuclease complex in certain embodiments can bind to a DNA target site sequence, but does not cleave any strand at the target site sequence. Such a complex may comprise a Cas9 protein in which all of its nuclease domains are mutant, dysfunctional. For example, a Cas9 protein herein that can bind to a DNA target site sequence, but does not cleave any strand at the target site sequence, may comprise both a mutant, dysfunctional RuvC domain and a mutant, dysfunctional HNH domain. A Cas9 protein herein that binds, but does not cleave, a target DNA sequence can be used to modulate gene expression, for example, in which case the Cas protein could be fused with a transcription factor (or portion thereof) (e.g., a repressor or activator, such as any of those disclosed herein).

The Cas9 endonuclease gene herein can be a plant, microbial, animal or mammalian codon optimized Cas9 endonuclease gene. The Cas9 endonuclease gene can be operably linked to a SV40 nuclear targeting signal upstream of the Cas codon region and a bipartite VirD2 nuclear localization signal (Tinland et al. (1992) Proc. Natl. Acad. Sci. USA 89:7442-6) downstream of the Cas9 codon region.

Cas9 endonucleases are typically derived from a type II CRISPR system, which includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA (Makarova et al. 2015, Nature Reviews Microbiology Vol. 13:1-15).

In one embodiment of the disclosure, the composition comprises at least one Cas9 endonuclease selected from the group consisting of SEQ ID NOs: 47-69, a functional fragment of SEQ ID NOs: 47-69, and a functional variant of SEQ ID NOs: 47-69.

In one embodiment of the disclosure, the composition comprises at least one recombinant DNA (such as a vector) encoding the Cas9 endonuclease selected from the group consisting of SEQ ID NOs: 47-69, a functional fragment of SEQ ID NOs: 47-69, and a functional variant of SEQ ID NOs: 47-69 (such as a recombinant DNA comprising the DNA sequences form SEQ ID NO: 24-46, a functional fragment of SEQ ID NOs: 24-46, and a functional variant of SEQ ID NOs: 24-46), or mRNA encoding Cas9 endonuclease selected from the group consisting of SEQ ID NOs: 47-69, a functional fragment of SEQ ID NOs: 47-69, and a functional variant of SEQ ID NOs: 47-69. The Cas9 endonuclease selected from the group consisting of SEQ ID NOs: 47-69, a functional fragment of SEQ ID NOs: 47-69, and a functional variant of SEQ ID NOs: 47-69 can form a (Ribonucleotide Protein -RNP) complex with at least one guide RNA, wherein said complex is capable of recognizing, binding to, and optionally nicking or cleaving all or part of a target site.

Recombinant DNA expressing the Cas9 endonucleases described herein (including, variants, functional fragments thereof, plant -, microbe -,or mammalian-codon optimized Cas9 endonuclease) can be stably integrated into the genome of an organism. For example, plants can be produced that comprise a cas9 gene stably integrated in the plant's genome. Plants expressing a stably integrated Cas endonuclease can be exposed to at least one guide RNA and/or a polynucleotide modification templates and/or donor DNAs to enable genome modifications such as gene knockout, gene editing or DNA insertions.

The terms "functional fragment ", "fragment that is functionally equivalent" and "functionally equivalent fragment" of a Cas9 endonuclease are used interchangeably herein, and refer to a portion or subsequence of the Cas9 endonuclease sequence of the present disclosure in which the ability to recognize, bind to, and optionally nick or cleave (introduce a single or double strand break in) the target site is retained.

Functional fragments of a Cas9 endonuclease of the present disclosure include proteins comprising at least one domain selected from the group consisting of a guide polynucleotide binding domain (an amino acid domain that can bind to or hybridize to a guide RNA), a crRNA binding domain (an amino acid domain that can bind to or hybridize to a crRNA), a tracrRNA binding domain (an amino acid domain that can bind to or hybridize to a tracrRNA), a DNA binding domain (an amino acid domain that can bind to DNA target sequence), a DNA cleavage domain (such as an HNH or RuvC domain) and any combination thereof.

Functional fragments of Cas9 endonucleases of the present disclosure include fragments comprising 50-100, 100-200, 100-300, 100-400, 100-500, 100-600, 100-700, 100-800, 100-900, 100-1000, 200-300, 200-400, 200-500, 200-600, 200-700, 200-800, 200-900, 200-1000, 300-400, 300-500, 300-600, 300-700, 300-800, 300-900, 300-1000, 400-500, 400-600, 400-700, 400-800, 400-900, 400-1000, 500-600, 500-700, 500-800, 500-900, 500-1000, 600-700, 600-800, 600-900, 600-1000, 700-800, 700-900, 700-1000, 800-900, 800-1000, or 900-1000 amino acids of a reference Cas9 protein, such as the reference Cas9 endonucleases of the present disclosure of SEQ ID NOs:46-69.

Functional fragments of the Cas9 endonucleases of the present disclosure include a protein comprising one or more protein domains of the Cas9 endonuclease of SEQ ID NOs: 46-69, wherein said protein retains specific binding activity, and optionally endonucleolytic activity, towards a target DNA when associated with a polynucleotide component.

The terms "functional variant", "Variant that is functionally equivalent" and "functionally equivalent variant" of a Cas9 endonuclease are used interchangeably herein, and refer to a variant of the Cas9 endonuclease of the present disclosure in which the ability to recognize, bind to, and optionally nick or cleave (introduce a single or double strand break in) the target site is retained.

A functional variant of a Cas9 protein sequence may be used, but should have specific binding activity, and optionally endonucleolytic activity, toward DNA when associated with a polynucleotide component herein. Such a functional variant of Cas9 may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of the reference Cas9, such as the reference Cas9 endonucleases described herein, including the Cas9 endonucleases of SEQ ID NOs: 47-69. Such a variant Cas9 protein can have specific binding activity, and optionally cleavage or nicking activity, toward DNA when associated with an RNA component herein. Cas9 variants include Cas9 endonuclease proteins wherein the HNH domain and/ or the RuvC domain contains at least one amino acid change (e.g., deletion, insertion, or substitution). In some embodiments, the amino acid variation resulting in at least about 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of the reference Cas9 protein, is located inside the HNH domain, or inside the RuvC domain, or inside both the HNH and RuvC domain. In some embodiments, the amino acid variation resulting in at least about 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of the reference Cas9 protein, is located outside of the HNH domain, or outside the RuvC domain, or outside both the HNH and RuvC domain.

Multiple functional domains and conserved elements were determined for each of the novel Cas9 endonuclease protein of the present disclosure (see Example 2, Tables 13-14). The novel Cas9 endonucleases of the present disclosure comprised an HNH domain, an RuvC domain that included three subdomains (RuvC-I, Ruvc-II and RuvC-II), a Bridge-Helix domain a PAM interacting domain and DNA/RNA recognition regions including REC1 and REC1'. The REC1 binds to repeat::anti-repeat RNA duplex of the guide RNA while REC1' mainly interacts with targetDNA::guide RNA hybrid duplex. The REC2 domain is a conserved element.

In some aspects the RuvC-I domain of a Cas9 endonuclease can be 40, 41, 42, 43, 44, 45, 46, 47, 47 , 48, 49, 50, 51, 52, 53, 54, 55, 56, 57, 58 ,59, 60, 61, 62, 63, 64, 65, 66, 67, 68, 69, 70, 71, 72, 73, 74, 75, 76, 77, 78, 79 or 80 amino acids in length. The RuvC-I domain can be located next to anyone of the amino acid domains selected from the group consisting of HNH, RuvC-I, RuvC-II, RuvC-III, REC1, REC1', REC-2, Bridge Helix (BH) and PAM interacting (PI) domain. Tables 13-14 described herein the location of the RuvC-I domain of each of the Cas9 endonucleases of the present disclosure and based on this information one can design novel Cas9 endonucleases comprising any one of the RuvC-I domain selected from the group consisting of the RuvC-I domain of Cas-Locus-6, the RuvC-I domain of Cas-Locus 7, the RuvC-I domain of Cas-locus-8, the RuvC-I domain of Cas-Locus-9, the RuvC-I domain of Cas-locus-10, the RuvC-I domain of Cas-Locus-11, Cas-Locus-12, the RuvC-I domain of Cas-Locus 13, the RuvC-I domain of Cas-locus-14, the RuvC-I domain of Cas-Locus-15, the RuvC-I domain of Cas-locus-16, the RuvC-I domain of Cas-Locus-17, Cas-Locus-18, the RuvC-I domain of Cas-Locus 19, the RuvC-I domain of Cas-locus-20, the RuvC-I domain of Cas-Locus-21, the RuvC-I domain of Cas-locus-22, the RuvC-I domain of Cas-Locus-23, the RuvC-I domain of Cas-locus-24, the RuvC-I domain of Cas-Locus-25, the RuvC-I domain of Cas-locus-26, the RuvC-I domain of Cas-Locus-27, the RuvC-I domain of Cas-locus-28, a function fragment thereof, and a functional variant thereof. (A functional fragment or functional variant of a RuvC-I domain is a fragment or variant in which the ability to function as a RuvC-I domain is retained).

In some aspects the Bridge-Helix (BH) domain of a Cas9 endonuclease can be 35, 36, 37, 38, 39, 40, 41, 42, 43, 44, 45, 46 or 47 amino acids in length. The BH domain can be located next to anyone of the amino acid domains selected from the group consisting of HNH, RuvC-I, RuvC-II, RuvC-III, REC1, REC1', REC-2, Bridge Helix (BH) and PAM interacting (PI) domain. Tables 13-14 described herein the location of the BH domain of each of the Cas9 endonucleases of the present disclosure and based on this information one can design novel Cas9 endonucleases comprising any one of the BH domain selected from the group consisting of the BH domain of Cas-Locus-6, the BH domain of Cas-Locus 7, the BH domain of Cas-locus-8, the BH domain of Cas-Locus-9, the BH domain of Cas-locus-10, the BH domain of Cas-Locus-11, Cas-Locus-12, the BH domain of Cas-Locus 13, the BH domain of Cas-locus-14, the BH domain of Cas-Locus-15, the BH domain of Cas-locus-16, the BH domain of Cas-Locus-17, Cas-Locus-18, the BH domain of Cas-Locus 19, the BH domain of Cas-locus-20, the BH domain of Cas-Locus-21, the BH domain of Cas-locus-22, the BH domain of Cas-Locus-23, the BH domain of Cas-locus-24, the BH domain of Cas-Locus-25, the BH domain of Cas-locus-26, the BH domain of Cas-Locus-27, the BH domain of Cas-locus-28, a function fragment thereof, and a functional variant thereof. (A functional fragment or functional variant of a BH domain is a fragment or variant in which the ability to function as a BH domain is retained).

In some aspects the REC1 domain of a Cas9 endonuclease can be 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113,114, 115,116, 117, 118, 119, 120, 121,122, 123, 124, 125,126, 127, 128, 129, 130, 131, 132, 133, 134, 135, 136, 137,138,139, 140, 141,142, 143, 144,145,146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221,222, 223, 224, 225, 226, 227, 228, 229, 230, 231, 232, 233, 234, 235, 236, 237, 238, 239, 240, 241,242, 243, 244,245,246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304 or 305 amino acids in length. The REC1 domain can be located next to anyone of the amino acid domains selected from the group consisting of HNH, RuvC-I, RuvC-II, RuvC-III, REC1, REC1', REC-2, Bridge Helix (BH) and PAM interacting (PI) domain. Tables 13-14 described herein the location of the REC1 domain of each of the Cas9 endonucleases of the present disclosure and based on this information one can design novel Cas9 endonucleases comprising any one of the REC1 domain selected from the group consisting of the REC1 domain of Cas-Locus-6, the REC1 domain of Cas-Locus 7, the REC1 domain of Cas-locus-8, the REC1 domain of Cas-Locus-9, the REC1 domain of Cas-locus-10, the REC1 domain of Cas-Locus-11, Cas-Locus-12, the REC1 domain of Cas-Locus 13, the REC1 domain of Cas-locus-14, the REC1 domain of Cas-Locus-15, the REC1 domain of Cas-locus-16, the REC1 domain of Cas-Locus-17, Cas-Locus-18, the REC1 domain of Cas-Locus 19, the REC1 domain of Cas-locus-20, the REC1 domain of Cas-Locus-21, the REC1 domain of Cas-locus-22, the REC1 domain of Cas-Locus-23, the REC1 domain of Cas-locus-24, the REC1 domain of Cas-Locus-25, the REC1 domain of Cas-locus-26, the REC1 domain of Cas-Locus-27, the REC1 domain of Cas-locus-28, a function fragment thereof, and a functional variant thereof. (A functional fragment or functional variant of a REC1 domain is a fragment or variant in which the ability to function as a REC1 domain is retained).

In some aspects the REC2 domain of a Cas9 endonuclease can be 130, 131, 132, 133, 134, 135, 136, 137,138,139, 140, 141,142, 143, 144,145,146, 147, 148, 149, 150, 151, 152, 153, 154, 155, or 156 amino acids in length. The REC2 domain can be located next to anyone of the amino acid domains selected from the group consisting of HNH, RuvC-I, RuvC-II, RuvC-III, REC1, REC1', REC-2, Bridge Helix (BH) and PAM interacting (PI) domain. Tables 13-14 described herein the location of the REC2 domain of each of the Cas9 endonucleases of the present disclosure and based on this information one can design novel Cas9 endonucleases comprising any one of the REC2 domain selected from the group consisting of the REC2 domain of Cas-Locus-6, the REC2 domain of Cas-Locus 7, the REC2 domain of Cas-locus-8, the REC2 domain of Cas-Locus-9, the REC2 domain of Cas-locus-10, the REC2 domain of Cas-Locus-11, Cas-Locus-12, the REC2 domain of Cas-Locus 13, the REC2 domain of Cas-locus-14, the REC2 domain of Cas-Locus-15, the REC2 domain of Cas-locus-16, the REC2 domain of Cas-Locus-17, Cas-Locus-18, the REC2 domain of Cas-Locus 19, the REC2 domain of Cas-locus-20, the REC2 domain of Cas-Locus-21, the REC2 domain of Cas-locus-22, the REC2 domain of Cas-Locus-23, the REC2 domain of Cas-locus-24, the REC2 domain of Cas-Locus-25, the REC2 domain of Cas-locus-26, the REC2 domain of Cas-Locus-27, the REC2 domain of Cas-locus-28, a function fragment thereof, and a functional variant thereof. (A functional fragment or functional variant of a REC2 domain is a fragment or variant in which the ability to function as a REC2 domain is retained).

In some aspects the REC1' domain of a Cas9 endonuclease can be 213, 214, 215, 216, 217, 218, 219, 220, 221,222, 223, 224, 225, 226, 227, 228, 229, 230, 231, 232, 233, 234, 235, 236, 237, 238, 239, 240, 241,242, 243, 244,245,246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321,322, 323, 324, 325, 326, 327, 328, 329, 330, 331, 332, 333, 334, 335, 336, 337, 338, 339, 340, 341,342, 343, 344, 345, 346, 347, 348, 349, 350, 351, 352, 353, 354, 355, 356, 357, 358, 359, 360, 361, 362, 363, 364, 365, 366, 367, 368, 369, 360, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 405, 406, 407, 408, 409, 410, 411, 412, 413, 414, 415, 416 or 417 amino acids in length. The REC1' domain can be located next to anyone of the amino acid domains selected from the group consisting of HNH, RuvC-I, RuvC-II, RuvC-III, REC1, REC1', REC-2, Bridge Helix (BH) and PAM interacting (PI) domain. Tables 13-14 described herein the location of the REC1' domain of each of the Cas9 endonucleases of the present disclosure and based on this information one can design novel Cas9 endonucleases comprising any one of the REC1' domain selected from the group consisting of the REC1' domain of Cas-Locus-6, the REC1' domain of Cas-Locus 7, the REC1' domain of Cas-locus-8, the REC1' domain of Cas-Locus-9, the REC1' domain of Cas-locus-10, the REC1' domain of Cas-Locus-11, Cas-Locus-12, the REC1' domain of Cas-Locus 13, the REC1' domain of Cas-locus-14, the REC1' domain of Cas-Locus-15, the REC1' domain of Cas-locus-16, the REC1' domain of Cas-Locus-17, Cas-Locus-18, the REC1' domain of Cas-Locus 19, the REC1' domain of Cas-locus-20, the REC1' domain of Cas-Locus-21, the REC1' domain of Cas-locus-22, the REC1' domain of Cas-Locus-23, the REC1' domain of Cas-locus-24, the REC1' domain of Cas-Locus-25, the REC1' domain of Cas-locus-26, the REC1' domain of Cas-Locus-27, the REC1' domain of Cas-locus-28, a function fragment thereof, and a functional variant thereof. (A functional fragment or functional variant of a REC1' domain is a fragment or variant in which the ability to function as a REC1' domain is retained).

In some aspects the RuvC-II domain of a Cas9 endonuclease can be 45, 46, 47, 47 , 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113,114, 115,116, 117, 118, 119, 120, 121,122, 123, 124, 125,126, 127, 128, 129, 130, 131, 132, 133, 134, 135, 136, 137,138,139, 140, 141,142, 143, 144,145,146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221,222, 223, 224, 225, 226, 227, 228, 229, 230, 231, 232, 233, 234, 235, 236, 237, 238, 239, 240, 241,242, 243, 244 or 245 amino acids in length. The RuvC-II domain can be located next to anyone of the amino acid domains selected from the group consisting of HNH, RuvC-I, RuvC-II, RuvC-III, REC1, REC1', REC-2, Bridge Helix (BH) and PAM interacting (PI) domain. Tables 13-14 described herein the location of the RuvC-II domain of each of the Cas9 endonucleases of the present disclosure and based on this information one can design novel Cas9 endonucleases comprising any one of the RuvC-II domain selected from the group consisting of the RuvC-II domain of Cas-Locus-6, the RuvC-II domain of Cas-Locus 7, the RuvC-II domain of Cas-locus-8, the RuvC-II domain of Cas-Locus-9, the RuvC-II domain of Cas-locus-10, the RuvC-II domain of Cas-Locus-11, Cas-Locus-12, the RuvC-II domain of Cas-Locus 13, the RuvC-II domain of Cas-locus-14, the RuvC-II domain of Cas-Locus-15, the RuvC-II domain of Cas-locus-16, the RuvC-II domain of Cas-Locus-17, Cas-Locus-18, the RuvC-II domain of Cas-Locus 19, the RuvC-II domain of Cas-locus-20, the RuvC-II domain of Cas-Locus-21, the RuvC-II domain of Cas-locus-22, the RuvC-II domain of Cas-Locus-23, the RuvC-II domain of Cas-locus-24, the RuvC-II domain of Cas-Locus-25, the RuvC-II domain of Cas-locus-26, the RuvC-II domain of Cas-Locus-27, the RuvC-II domain of Cas-locus-28, a function fragment thereof, and a functional variant thereof. (A functional fragment or functional variant of a RuvC-II domain is a fragment or variant in which the ability to function as a RuvC-II domain is retained).

In some aspects the HNH domain of a Cas9 endonuclease can be 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216 or 217 amino acids in length. The HNH domain can be located next to anyone of the amino acid domains selected from the group consisting of HNH, RuvC-I, RuvC-II, RuvC-III, REC1, REC1', REC-2, Bridge Helix (BH) and PAM interacting (PI) domain. Tables 13-14 described herein the location of the HNH domain of each of the Cas9 endonucleases of the present disclosure and based on this information one can design novel Cas9 endonucleases comprising any one of the HNH domain selected from the group consisting of the HNH domain of Cas-Locus-6, the HNH domain of Cas-Locus 7, the HNH domain of Cas-locus-8, the HNH domain of Cas-Locus-9, the HNH domain of Cas-locus-10, the HNH domain of Cas-Locus-11, Cas-Locus-12, the HNH domain of Cas-Locus 13, the HNH domain of Cas-locus-14, the HNH domain of Cas-Locus-15, the HNH domain of Cas-locus-16, the HNH domain of Cas-Locus-17, Cas-Locus-18, the HNH domain of Cas-Locus 19, the HNH domain of Cas-locus-20, the HNH domain of Cas-Locus-21, the HNH domain of Cas-locus-22, the HNH domain of Cas-Locus-23, the HNH domain of Cas-locus-24, the HNH domain of Cas-Locus-25, the HNH domain of Cas-locus-26, the HNH domain of Cas-Locus-27, the HNH domain of Cas-locus-28, a function fragment thereof, and a functional variant thereof. (A functional fragment or functional variant of a HNH domain is a fragment or variant in which the ability to function as a HNH domain is retained).

In some aspects the RuvC-III domain of a Cas9 endonuclease can be 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257 or 258 amino acids in length. The RuvC-III domain can be located next to anyone of the amino acid domains selected from the group consisting of HNH, RuvC-I, RuvC-II, RuvC-III, REC1, REC1', REC-2, Bridge Helix (BH) and PAM interacting (PI) domain. Tables 13-14 described herein the location of the RuvC-III domain of each of the Cas9 endonucleases of the present disclosure and based on this information one can design novel Cas9 endonucleases comprising any one of the RuvC-III domain selected from the group consisting of the RuvC-III domain of Cas-Locus-6, the RuvC-III domain of Cas-Locus 7, the RuvC-III domain of Cas-locus-8, the RuvC-III domain of Cas-Locus-9, the RuvC-III domain of Cas-locus-10, the RuvC-III domain of Cas-Locus-11, Cas-Locus-12, the RuvC-III domain of Cas-Locus 13, the RuvC-III domain of Cas-locus-14, the RuvC-III domain of Cas-Locus-15, the RuvC-III domain of Cas-locus-16, the RuvC-III domain of Cas-Locus-17, Cas-Locus-18, the RuvC-III domain of Cas-Locus 19, the RuvC-III domain of Cas-locus-20, the RuvC-III domain of Cas-Locus-21, the RuvC-III domain of Cas-locus-22, the RuvC-III domain of Cas-Locus-23, the RuvC-III domain of Cas-locus-24, the RuvC-III domain of Cas-Locus-25, the RuvC-III domain of Cas-locus-26, the RuvC-III domain of Cas-Locus-27, the RuvC-III domain of Cas-locus-28, a function fragment thereof, and a functional variant thereof.

In some aspects the PI domain of a Cas9 endonuclease can be 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321,322, 323, 324, 325, 326, 327, 328, 329, 330, 331, 332, 333, 334, 335, 336, 337, 338, 339, 340, or 341 amino acids in length. The PI domain can be located next to anyone of the amino acid domains selected from the group consisting of HNH, RuvC-I, RuvC-II, RuvC-III, REC1, REC1', REC-2, Bridge Helix (BH) and PAM interacting (PI) domain. Tables 13-14 described herein the location of the PI domain of each of the Cas9 endonucleases of the present disclosure and based on this information one can design novel Cas9 endonucleases comprising any one of the PI domain selected from the group consisting of the PI domain of Cas-Locus-6, the PI domain of Cas-Locus 7, the PI domain of Cas-locus-8, the PI domain of Cas-Locus-9, the PI domain of Cas-locus-10, the PI domain of Cas-Locus-11, Cas-Locus-12, the PI domain of Cas-Locus 13, the PI domain of Cas-locus-14, the PI domain of Cas-Locus-15, the PI domain of Cas-locus-16, the PI domain of Cas-Locus-17, Cas-Locus-18, the PI domain of Cas-Locus 19, the PI domain of Cas-locus-20, the PI domain of Cas-Locus-21, the PI domain of Cas-locus-22, the PI domain of Cas-Locus-23, the PI domain of Cas-locus-24, the PI domain of Cas-Locus-25, the PI domain of Cas-locus-26, the PI domain of Cas-Locus-27, the PI domain of Cas-locus-28, a function fragment thereof, and a functional variant thereof. (A functional fragment or functional variant of a PI domain is a fragment or variant in which the ability to function as a PI domain is retained).

Cas9 endonuclease functional fragments and Cas9 endonuclease variants can be obtained via methods such as site-directed mutagenesis and synthetic construction

Methods for determining if fragments and/or variants of a Cas9 endonuclease of the present disclosure are functional include methods that measure the endonuclease activity of the fragment or variant when in complex with a suitable polynucleotide. Methods that measure Cas9 endonuclease activity are well known in the art such as, but not limiting to, PCT/US13/39011, filed May 1, 2013, PCT/US16/32073 filed May 12, 2016, PCT/US16/32028 filed May 12, 2016, incorporated by reference herein). Methods for measuring Cas9 endonuclease activity include methods that measure the mutation frequency at a target site after a double strand break has occurred (see also, Example 3).

Methods for measuring Cas9 endonuclease activity include methods that measure the mutation frequency at a target site after a double strand break has occurred (see also, Example 3). Methods for measuring if a functional fragment or functional variant of a Cas9 endonuclease of the present disclosure can make a double strand break include the following method: The cellular repair of chromosomal double-strand breaks (DSBs) induced by CRISPR-Cas9 in plant cells results in the production of insertion or deletion (indel) mutagenesis (Svitashev *et al.* et al. (2015)). This outcome can be used to detect and monitor the production of DSBs generated by functional fragments of functional variant of the Cas9 endonucleases of the present disclosure (see also Karvelis et al. (2015). Briefly, appropriate CRISPR-Cas9 maize genomic DNA target sites can be selected, a guide RNA transcriptional cassette (recombinant DNA that expresses a guide RNA) and a DNA recombinant construct expressing the Cas9 endonuclease of the present disclosure (or a functional fragment of the Cas9 endonuclease of the present disclosure, or a functional variant of the Cas9 endonuclease variant of the present disclosure endonuclease (such as an expression cassette described in Example 2) can be constructed and can be co-delivered by biolistic transformation into Hi-Type II 10-day-old immature maize embryos (IMEs) in the presence of BBM and WUS2 genes as described in Svitashev *et al.* (2015). A visual marker DNA expression cassette encoding a yellow fluorescent protein can also be co-delivered with the guide RNA transcriptional cassette and the Cas9 endonuclease expression cassette (recombinant DNA construct) to aid in the selection of evenly transformed IMEs . After 2 days, the 20-30 most evenly transformed IMEs can be harvested based on their fluorescence. Total genomic DNA is extracted and the DNA region surrounding the intended target site is PCR amplified with Phusion® HighFidelity PCR Master Mix (New England Biolabs, M0531L) adding on the sequences necessary for amplicon-specific barcodes and Illumnia sequencing and deep sequenced. The resulting reads are then examined for the presence of mutations at the expected site of cleavage by comparison to control experiments where the guide RNA transcriptional cassette was omitted from the transformation. If mutations are observed at the intended target sites when using a fragment or variant of the Cas9 endonuclease of the present disclosure, in complex with a suitable guide polynucleotide, the fragments or variants are functional.

Methods for measuring if a functional fragment of functional variant of a Cas9 endonuclease of the present disclosure can make a single strand break (also referred to as a nick; hence acts as a nickase) in the double stranded DNA target site include the following method: The cellular repair of chromosomal single-strand breaks (SSBs) in a double-stranded DNA target may be typically repaired seamlessly in plant cells such as maize. Therefore to examine a functional Cas9 fragment or functional variant of a Cas9 for nicking activity, two chromosomal DNA target sites in close proximity (0-200 bp), each targeting a different strand (sense and anti-sense DNA strands) of the double-stranded DNA, can be targeted. If SSB activity is present, the SSB activity from both target sites will result in a DNA double-strand break (DSB) that will result in the production of insertion or deletion (indel) mutagenesis in maize cells. This outcome can then be used to detect and monitor the activity of the Cas9 nickase similar to that described in Karvelis et al. (2015). Briefly, appropriate CRISPR-Cas9 maize genomic DNA target sites are selected, guide RNA transcription cassettes and functional fragment Cas9 nicking expression cassettes are constructed and co-delivered by biolistic transformation into Hi-Type II 10-day-old immature maize embryos (IMEs) in the presence of BBM and WUS2 genes as described in Svitashev et al. (2015). Since particle gun transformation can be highly variable, a visual marker DNA expression cassette encoding a yellow fluorescent protein can also be co-delivered to aid in the selection of evenly transformed IMEs [immature maize embryos]. After 2 days, the 20-30 most evenly transformed IMEs are harvested based on their fluorescence, total genomic DNA extracted, the region surrounding the intended target site PCR amplified with Phusion® HighFidelity PCR Master Mix (New England Biolabs, M0531L) adding on the sequences necessary for amplicon-specific barcodes and Illumnia sequencing and deep sequenced. The resulting reads are then examined for the presence of mutations at the expected site of cleavage by comparison to control experiments where the small RNA transcriptional cassette was omitted from the transformation.

Methods for measuring if a functional fragment of functional variant of a Cas9 endonuclease of the present disclosure can bind to the intended DNA target site include the following method: The binding of a maize chromosomal DNA target site does not result in either a single-stranded break (SSB) or a double-stranded break (DSB) in the double-stranded DNA target site. Therefore to examine a functional Cas9 fragment for binding activity in maize cells, another nuclease domain (e.g. Fokl) may be attached to the functional Cas9 fragment with binding activity. If binding activity is present, the added nuclease domain may be used to produce a DSB that will result in the production of insertion or deletion (indel) mutagenesis in maize cells. This outcome may then be used to detect and monitor the binding activity of a Cas9 similar to that described in Karvelis et al. (2015). Briefly, appropriate CRISPR-Cas9 maize genomic DNA target sites can be selected, guide RNA transcription cassettes and functional fragment Cas9 binding and nuclease attached expression cassettes can be constructed and co-delivered by biolistic transformation into Hi-Type II 10-day-old immature maize embryos (IMEs) in the presence of BBM and WUS2 genes as described in Svitashev et al. (2015). A visual marker DNA expression cassette encoding a yellow fluorescent protein can also be co-delivered to aid in the selection of evenly transformed IMEs [immature maize embryos]. After 2 days, the 20-30 most evenly transformed IMEs can be harvested based on their fluorescence, total genomic DNA extracted, the region surrounding the intended target site PCR amplified with Phusion® HighFidelity PCR Master Mix (New England Biolabs, M0531L) adding on the sequences necessary for amplicon-specific barcodes and Illumnia sequencing and deep sequenced. The resulting reads can then be examined for the presence of mutations at the expected site of cleavage by comparison to control experiments where the small RNA transcriptional cassette was omitted from the transformation.

Alternatively, the binding activity of maize chromosomal DNA target sites can be monitored by the transcriptional induction or repression of a gene. This can be accomplished by attaching a transcriptional activation or repression domain to the functional Cas9 binding fragment and targeting it to the promoter region of a gene and binding monitored through an increase in accumulation of the gene transcript or protein. The gene targeted for either activation or repression can be any naturally occurring maize gene or engineered gene (e.g. a gene encoded a red fluorescent protein) introduced into the maize genome by methods known in the art (e.g. particle gun or agrobacterium transformation).

The Cas9 endonuclease can comprise a modified form of the Cas9 polypeptide. The modified form of the Cas9 polypeptide can include an amino acid change (e.g., deletion, insertion, or substitution) that reduces the naturally-occurring nuclease activity of the Cas9 protein. For example, in some instances, the modified form of the Cas9 protein has less than 50%, less than 40%, less than 30%, less than 20%, less than 10%, less than 5%, or less than 1% of the nuclease activity of the corresponding wild-type Cas9 polypeptide (US patent application US20140068797 A1, published on March 6, 2014). In some cases, the modified form of the Cas9 polypeptide has no substantial nuclease activity and is referred to as catalytically "inactivated Cas9" or "deactivated cas9 (dCas9)." Catalytically inactivated Cas9 variants include Cas9 variants that contain mutations in the HNH and RuvC nuclease domains. These catalytically inactivated Cas9 variants are capable of interacting with sgRNA and binding to the target site in vivo but cannot cleave either strand of the target DNA.

A catalytically inactive Cas9 can be fused to a heterologous sequence (US patent application US20140068797 A1, published on March 6, 2014). Suitable fusion partners include, but are not limited to, a polypeptide that provides an activity that indirectly increases transcription by acting directly on the target DNA or on a polypeptide (e.g., a histone or other DNA-binding protein) associated with the target DNA. Additional suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity. Further suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.). A catalytically inactive Cas9 can also be fused to a Fokl nuclease to generate double strand breaks (Guilinger et al. Nature biotechnology, volume 32, number 6, June 2014).

A Cas9 protein herein can comprise a heterologous nuclear localization sequence (NLS). A heterologous NLS amino acid sequence herein may be of sufficient strength to drive accumulation of a Cas9 protein in a detectable amount in the nucleus of a yeast cell herein, for example. An NLS may comprise one (monopartite) or more (e.g., bipartite) short sequences (e.g., 2 to 20 residues) of basic, positively charged residues (e.g., lysine and/or arginine), and can be located anywhere in a Cas9 amino acid sequence but such that it is exposed on the protein surface. An NLS may be operably linked to the N-terminus or C-terminus of a Cas9 protein herein, for example. Two or more NLS sequences can be linked to a Cas9 protein, for example, such as on both the N- and C-termini of a Cas9 protein. The Cas9 endonuclease gene can be operably linked to a SV40 nuclear targeting signal upstream of the Cas9 codon region and a bipartite VirD2 nuclear localization signal (Tinland et al. (1992) Proc. Natl. Acad. Sci. USA 89:7442-6) downstream of the Cas9 codon region. Non-limiting examples of suitable NLS sequences herein include those disclosed in U.S. Patent No. 7309576, which is incorporated herein by reference.

Cas9 endonucleases described herein can be used for targeted genome editing (via simplex and multiplex double-strand breaks and nicks) and targeted genome regulation (via tethering of epigenetic effector domains to either the Cas9 protein or guide polynucleotide (sgRNA or combination of crRNA+tracrRNA). A Cas9 endonuclease can also be engineered to function as an RNA-guided recombinase, and via RNA tethers could serve as a scaffold for the assembly of multiprotein and nucleic acid complexes (Mali et al., 2013, Nature Methods Vol. 10: 957-963).

The Cas9 protein, or functional fragment thereof, for use in the disclosed methods, can be isolated from a recombinant source where the genetically modified host cell (e.g. an insect cell or a yeast cell or human-derived cell line) is modified to express the nucleic acid sequence encoding the Cas9 protein. Alternatively, the Cas9 protein can be produced using cell free protein expression systems or be synthetically produced.

The term "plant-optimized Cas9 endonuclease" herein refers to a Cas9 protein encoded by a nucleotide sequence that has been optimized for expression in a plant cell or plant, and optionally for increased expression in a plant. A "plant-optimized nucleotide sequence encoding a Cas9 endonuclease", "plant-optimized construct encoding a Cas9 endonuclease" and a "plant-optimized polynucleotide encoding a Cas9" are used interchangeably herein and refer to a nucleotide sequence encoding an Cas9 protein, or a variant or functional fragment thereof, that has been optimized for expression in a plant cell or plant. A plant comprising a plant-optimized Cas9 endonuclease includes a plant comprising the nucleotide sequence encoding for the Cas9 sequence and/or a plant comprising the Cas9 endonuclease protein. In one aspect, the plant-optimized Cas9 endonuclease nucleotide sequence is a maize-optimized, rice-optimized, wheat-optimized or soybean-optimized Cas9 endonuclease.

A plant-optimized nucleotide sequence, such as a plant-optimized Cas9 endonuclease DNA sequence, can be synthesized by modifying a nucleotide sequence using one or more plant-preferred codons for improved expression. *See*, for example, Campbell and Gowri (1990) Plant Physiol. 92:1-11 for a discussion of host-preferred codon usage.

The term "mammalian-optimized Cas9 endonuclease sequence" herein refers to a nucleotide sequence encoding a Cas9 endonuclease that has been optimized for expression in mammalian cells, particularly for increased expression in mammalian cells.

The Cas9 endonuclease described herein can be introduced into a cell by any method known in the art, for example, but not limited to transient introduction methods (such as *Agrobacterium*-mediated transformation, or particle mediated delivery such as biolistic particle bombardment), transfection, microinjection, and/or topical application or indirectly via recombination constructs. The Cas9 endonuclease can be introduced as a protein or as a guided polynucleotide complex (ribonucleotide complex, RNP complex) directly to a cell or indirectly via recombination constructs. The endonuclease can be introduced into a cell transiently or can be incorporated into the genome of the host cell using any method known in the art. Uptake of the endonuclease and/or the guided polynucleotide into the cell can be facilitated with a Cell Penetrating Peptide (CPP) as described in US application 62/075999, filed November 06, 2014.

As used herein, the term "guide polynucleotide", relates to a polynucleotide sequence that can form a complex with a Cas9 endonuclease and enables the Cas9 endonuclease to recognize, bind to, and optionally cleave a DNA target site. The guide polynucleotide can be a single molecule or a double molecule. The guide polynucleotide sequence can be a RNA sequence, a DNA sequence, or a combination thereof (a RNA-DNA combination sequence). Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA" or "gRNA" (See also U.S. Patent Application US 2015-0082478 A1, published on March 19, 2015 and US 2015-0059010 A1, published on February 26, 2015, both are hereby incorporated in its entirety by reference).

The guide polynucleotide can be a double molecule (also referred to as duplex guide polynucleotide) comprising a crNucleotide sequence and a tracrNucleotide sequence. The crNucleotide includes a first nucleotide sequence domain (referred to as Variable Targeting domain or VT domain) that can hybridize to a nucleotide sequence in a target DNA and a second nucleotide sequence (also referred to as a tracr mate sequence) that is part of a Cas endonuclease recognition (CER) domain. The tracr mate sequence can hybridized to a tracrNucleotide along a region of complementarity and together form the Cas endonuclease recognition domain or CER domain. The CER domain is capable of interacting with a Cas9 endonuclease polypeptide. The crNucleotide and the tracrNucleotide of the duplex guide polynucleotide can be RNA, DNA, and/or RNA-DNA- combination sequences. In some embodiments, the crNucleotide molecule of the duplex guide polynucleotide is referred to as "crDNA" (when composed of a contiguous stretch of DNA nucleotides) or "crRNA" (when composed of a contiguous stretch of RNA nucleotides), or "crDNA-RNA" (when composed of a combination of DNA and RNA nucleotides). The crNucleotide can comprise a fragment of the crRNA naturally occurring in Bacteria and Archaea. The size of the fragment of the crRNA naturally occurring in Bacteria and Archaea that can be present in a crNucleotide disclosed herein can range from, but is not limited to, 2, 3, 4, 5, 6, 7, 8, 9,10, 11, 12, 13, 14, 15, 16, 17, 18, 19, 20 or more nucleotides. In some embodiments the tracrNucleotide is referred to as "tracrRNA" (when composed of a contiguous stretch of RNA nucleotides) or "tracrDNA" (when composed of a contiguous stretch of DNA nucleotides) or "tracrDNA-RNA" (when composed of a combination of DNA and RNA nucleotides. In one embodiment, the RNA that guides the RNA/ Cas9 endonuclease complex is a duplexed RNA comprising a duplex crRNA-tracrRNA.

The tracrRNA (trans-activating CRISPR RNA, Deltcheva et al., Nature 471:602-607) contains, in the 5'-to-3' direction, (i) a sequence that anneals with the repeat region of CRISPR type II crRNA (described herein as the anti-repeat region, such as but not limiting to SEQ ID NOs: 138-161), and (ii) a stem loop-containing portion (described herein as a 3' tracrRNA such as but not limiting to SEQ ID NOs: 162-184)

The tracrRNA component of the single or duplex guide RNA for the Cas9 endonuclease systems described herein can comprise an anti-repeat fragment (including any one of SEQ ID NOs: 139-161) and a 3' tracrRNA component (including any one of SEQ ID NO: 162-184). For example the tracrRNA can comprise SEQ ID NOs: 139 and 162, or SEQ ID NOs: 140 and 163, or SEQ ID NOs: 141 and 164, or SEQ ID NOs: 142 and 165, or SEQ ID NOs: 143 and 166, or SEQ ID NOs: 144 and 167, or SEQ ID NOs: 145 and 168, or SEQ ID NOs: 146 and 169, or SEQ ID NOs: 147 and 170, or SEQ ID NOs: 148 and 171, or SEQ ID NOs: 149 and 172, or SEQ ID NOs: 150 and 173, or SEQ ID NOs: 151 and 174, or SEQ ID NOs: 152 and 175, or SEQ ID NOs: 153 and 176, or SEQ ID NOs: 154 and 177, or SEQ ID NOs: 155 and 178, or SEQ ID NOs: 156 and 179, or SEQ ID NOs: 157 and 180, or SEQ ID NOs: 158 and 181, or SEQ ID NOs: 159 and 182, or SEQ ID NOs: 160 and 183, or SEQ ID NOs: 161 and 184.

The duplex guide polynucleotide can form a complex with a Cas9 endonuclease, wherein said guide polynucleotide/Cas9 endonuclease complex (also referred to as a guide polynucleotide/Cas9 endonuclease system) can direct the Cas9 endonuclease to a genomic target site, enabling the Cas9 endonuclease to recognize, bind to, and optionally nick or cleave (introduce a single or double strand break) into the target site. (See also U.S. Patent Application US 2015-0082478 A1, published on March 19, 2015 and US 2015-0059010 A1, published on February 26, 2015, both are hereby incorporated in its entirety by reference.)

A chimeric non-naturally occurring crRNA includes a crRNA that comprises regions that are not found together in nature (i.e., they are heterologous with each other. For example, a crRNA comprising a first nucleotide sequence domain (referred to as Variable Targeting domain or VT domain) that can hybridize to a nucleotide sequence in a target DNA, linked to a second nucleotide sequence (also referred to as a tracr mate sequence) such that the first and second sequence are not found linked together in nature. In one such example, a chimeric non-naturally occurring crRNA includes a VT domain that is capable of recognizing (or binding to) a target sequence in a eukaryotic genome.

The guide polynucleotide can also be a single molecule (also referred to as single guide polynucleotide) comprising a crNucleotide sequence linked to a tracrNucleotide sequence. The single guide polynucleotide comprises a first nucleotide sequence domain (referred to as Variable Targeting domain or VT domain) that can hybridize to a nucleotide sequence in a target DNA and a Cas9 endonuclease recognition domain (CER domain), that interacts with a Cas9 endonuclease polypeptide. By "domain" it is meant a contiguous stretch of nucleotides that can be RNA, DNA, and/or RNA-DNA-combination sequence. The VT domain and /or the CER domain of a single guide polynucleotide can comprise a RNA sequence, a DNA sequence, or a RNA-DNA-combination sequence. The single guide polynucleotide being comprised of sequences from the crNucleotide and the tracrNucleotide may be referred to as "single guide RNA" (when composed of a contiguous stretch of RNA nucleotides) or "single guide DNA" (when composed of a contiguous stretch of DNA nucleotides) or "single guide RNA-DNA" (when composed of a combination of RNA and DNA nucleotides). The single guide polynucleotide can form a complex with a Cas9 endonuclease, wherein said guide polynucleotide/Cas9 endonuclease complex (also referred to as a guide polynucleotide/Cas9 endonuclease system) can direct the Cas9 endonuclease to a genomic target site, enabling the Cas9 endonuclease to recognize, bind to, and optionally nick or cleave (introduce a single or double strand break) the target site. (See also U.S. Patent Application US 2015-0082478 A1, published on March 19, 2015 and US 2015-0059010 A1, published on February 26, 2015, both are hereby incorporated in its entirety by reference.)

A chimeric non-naturally occurring single guide RNA (sgRNA) includes a sgRNA that comprises regions that are not found together in nature (i.e., they are heterologous with each other). For example, a sgRNA comprising a first nucleotide sequence domain (referred to as Variable Targeting domain or VT domain) that can hybridize to a nucleotide sequence in a target DNA linked to a second nucleotide sequence (also referred to as a tracr mate sequence) that are not found linked together in nature.

The term "variable targeting domain" or "VT domain" is used interchangeably herein and includes a nucleotide sequence that can hybridize (is complementary) to one strand (nucleotide sequence) of a double strand DNA target site. The % complementation between the first nucleotide sequence domain (VT domain) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable targeting domain can be at least 12, 13, 14, 15, 16, 17, 18, 19, 20, 21, 22, 23, 24, 25, 26, 27, 28, 29 or 30 nucleotides in length.

In some embodiments, the variable targeting domain comprises a contiguous stretch of 12 to 30, 12 to 29, 12 to 28, 12 to 27, 12 to 26, 12 to 25, 12 to 26, 12 to 25, 12 to 24, 12 to 23, 12 to 22, 12 to 21, 12 to 20, 12 to 19, 12 to 18, 12 to 17, 12 to 16, 12 to 15, 12 to 14, 12 to 13, 13 to 30, 13 to 29, 13 to 28, 13 to 27, 13 to 26, 13 to 25, 13 to 26, 13 to 25, 13 to 24, 13 to 23, 13 to 22, 13 to 21, 13 to 20, 13 to 19, 13 to 18, 13 to 17, 13 to 16, 13 to 15, 13 to 14, 14 to 30, 14 to 29, 14 to 28, 14 to 27, 14 to 26, 14 to 25, 14 to 26, 14 to 25, 14 to 24, 14 to 23, 14 to 22, 14 to 21, 14 to 20, 14 to 19, 14 to 18, 14 to 17, 14 to 16, 14 to 15, 15 to 30, 15 to 29, 15 to 28, 15 to 27, 15 to 26, 15 to 25, 15 to 26, 15 to 25, 15 to 24, 15 to 23, 15 to 22, 15 to 21, 15 to 20, 15 to 19, 15 to 18, 15 to 17, 15 to 16, 16 to 30, 16 to 29, 16 to 28, 16 to 27, 16 to 26, 16 to 25, 16 to 24, 16 to 23, 16 to 22, 16 to 21, 16 to 20, 16 to 19, 16 to 18, 16 to 17, 17 to 30, 17 to 29, 17 to 28, 17 to 27, 17 to 26, 17 to 25, 17 to 24, 17 to 23, 17 to 22, 17 to 21, 17 to 20, 17 to 19, 17 to 18, 18 to 30, 18 to 29, 18 to 28, 18 to 27, 18 to 26, 18 to 25, 18 to 24, 18 to 23, 18 to 22, 18 to 21, 18 to 20, 18 to 19, 19 to 30, 19 to 29, 19 to 28, 19 to 27, 19 to 26, 19 to 25, 19 to 24, 19 to 23, 19 to 22, 19 to 21, 19 to 20, 20 to 30, 20 to 29, 20 to 28, 20 to 27, 20 to 26, 20 to 25, 20 to 24, 20 to 23, 20 to 22, 20 to 21, 21 to 30, 21 to 29, 21 to 28, 21 to 27, 21 to 26, 21 to 25, 21 to 24, 21 to 23, 21 to 22, 22 to 30, 22 to 29, 22 to 28, 22 to 27, 22 to 26, 22 to 25, 22 to 24, 22 to 23, 23 to 30, 23 to 29, 23 to 28, 23 to 27, 23 to 26, 23 to 25, 23 to 24, 24 to 30, 24 to 29, 24 to 28, 24 to 27, 24 to 26, 24 to 25, 25 to 30, 25 to 29, 25 to 28, 25 to 27, 25 to 26, 26 to 30, 26 to 29, 26 to 28 nucleotides.

The variable targeting domain can be composed of a DNA sequence, a RNA sequence, a modified DNA sequence, a modified RNA sequence, or any combination thereof.

The term "Cas endonuclease recognition domain" or "CER domain" (of a guide polynucleotide) is used interchangeably herein and includes a nucleotide sequence that interacts with a Cas9 endonuclease polypeptide. A CER domain comprises a tracrNucleotide mate sequence followed by a tracrNucleotide sequence. The CER domain can be composed of a DNA sequence, a RNA sequence, a modified DNA sequence, a modified RNA sequence (see for example US 2015-0059010 A1, published on February 26, 2015, incorporated in its entirety by reference herein), or any combination thereof.

The nucleotide sequence linking the crNucleotide and the tracrNucleotide of a single guide polynucleotide can comprise a RNA sequence, a DNA sequence, or a RNA-DNA combination sequence. In one embodiment, the nucleotide sequence linking the crNucleotide and the tracrNucleotide of a single guide polynucleotide (also referred to as loop) can be at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 or 100 nucleotides in length. In some embodiments, the loop can be 3-4, 3-5, 3-6, 3-7, 3-8, 3-9, 3-10, 3-11, 3-12, 3-13, 3-14, 3-15, 3-20, 3-30, 3-40, 3-50, 3-60, 3-70, 3-80, 3-90, 3-100, 4-5, 4-6, 4-7, 4-8, 4-9, 4-10, 4-11, 4-12, 4-13, 4-14, 4-15, 4-20, 4-30, 4-40, 4-50, 4-60, 4-70, 4-80, 4-90, 4-100, 5-6, 5-7, 5-8, 5-9, 5-10, 5-11, 5-12, 5-13, 5-14, 5-15, 5-20, 5-30, 5-40, 5-50, 5-60, 5-70, 5-80, 5-90, 5-100, 6-7, 6-8, 6-9, 6-10, 6-11, 6-12, 6-13, 6-14, 6-15, 6-20, 6-30, 6-40, 6-50, 6-60, 6-70, 6-80, 6-90, 6-100, 7-8, 7-9, 7-10, 7-11, 7-12, 7-13, 7-14, 7-15, 7-20, 7-30, 7-40, 7-50, 7-60, 7-70, 7-80, 7-90, 7-100, 8-9, 8-10, 8-11, 8-12, 8-13, 8-14, 8-15, 8-20, 8-30, 8-40, 8-50, 8-60, 8-70, 8-80, 8-90, 8-100, 9-10, 9-11, 9-12, 9-13, 9-14, 9-15, 9-20, 9-30, 9-40, 9-50, 9-60, 9-70, 9-80, 9-90, 9-100, 10-20, 20-30, 30-40, 40-50, 50-60, 70-80, 80-90 or 90-100 nucleotides in length.

In one embodiment, the nucleotide sequence linking the crNucleotide and the tracrNucleotide of a single guide polynucleotide can comprise a tetraloop sequence, such as, but not limiting to a GAAA tetraloop sequence.

The guide polynucleotide can be produced by any method known in the art, including chemically synthesizing guide polynucleotides (such as but not limiting to Hendel et al. 2015, Nature Biotechnology 33, 985-989), in vitro generated guide polynucleotides, and/or self-splicing guide RNAs (such as but not limiting to Xie et al. 2015, PNAS 112:3570-3575).

A method of expressing RNA components such as gRNA in eukaryotic cells for performing Cas9-mediated DNA targeting has been to use RNA polymerase III (Pol III) promoters, which allow for transcription of RNA with precisely defined, unmodified, 5'- and 3'-ends (DiCarlo et al., Nucleic Acids Res. 41: 4336-4343; Ma et al., Mol. Ther. Nucleic Acids 3:e161). This strategy has been successfully applied in cells of several different species including maize and soybean (US 20150082478, published on March 19, 2015). Methods for expressing RNA components that do not have a 5' cap have been described (WO 2016/025131, published on February 18, 2016).

In some embodiments, a subject nucleic acid (e.g., a guide polynucleotide, a nucleic acid comprising a nucleotide sequence encoding a guide polynucleotide; a nucleic acid encoding Cas9 endonuclease of the present disclosure; a crRNA or a nucleotide encoding a crRNA, a tracrRNA or a nucleotide encoding a tracrRNA, a nucleotide encoding a VT domain, a nucleotide encoding a CER domain, etc.) comprises a modification or sequence that provides for an additional desirable feature (e.g., modified or regulated stability; subcellular targeting; tracking, e.g., a fluorescent label; a binding site for a protein or protein complex; etc.). Nucleotide sequence modification of the guide polynucleotide, VT domain and/or CER domain can be selected from, but not limited to , the group consisting of a 5' cap, a 3' polyadenylated tail, a riboswitch sequence, a stability control sequence, a sequence that forms a dsRNA duplex, a modification or sequence that targets the guide poly nucleotide to a subcellular location, a modification or sequence that provides for tracking , a modification or sequence that provides a binding site for proteins , a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

In one embodiment of the disclosure, the composition comprises at least one a single guide RNA capable of forming a guide RNA/Cas9 endonuclease complex, wherein said guide RNA/Cas9 endonuclease complex can recognize, bind to, and optionally nick or cleave a target sequence, wherein said single guide RNA is selected from the group consisting of SEQ ID NOs: 185-207, a functional fragment of SEQ ID NOs: 185-207, and a functional variant of SEQ ID NOs: 185-207.

In one embodiment of the disclosure, the composition is a single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of SEQ ID NOs: 139-184, a functional fragment of SEQ ID NOs: 139-184, and a functional variant of SEQ ID NOs: 139-184, .

The guide RNA can also be a dual molecule comprising a chimeric non-naturally occurring crRNA linked to a tracrRNA, wherein said chimeric non-naturally occurring crRNA comprises a nucleotide sequence selected from the group consisting of SEQ ID NOs: 116-138, a functional fragment of SEQ ID NOs: 116-138, and a functional variant of SEQ ID NOs: 116-138, and/or wherein said tracrRNA comprises a nucleotide sequence selected from the group consisting of SEQ ID NOs: 139-184, a functional fragment of SEQ ID NOs: 139-184, and a functional variant of SEQ ID NOs: 139-184, .

In one embodiment of the disclosure, the composition is a single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of SEQ ID NOs: 116-138, a functional fragment of SEQ ID NOs: 116-138, and a functional variant of SEQ ID NOs: 116-138.

Single guide RNAs targeting a target site in the genome of an organism can be designed by changing Variable Targeting Domain (VT) of any one of SEQ ID NOs: 185-207 (or a functional fragment of SEQ ID NOs: 185-207, or a functional variant of SEQ ID NOs: 185-207) with any random nucleotide that can hybridize to any desired target sequence. In SEQ ID NOs: 185-207 the sgRNA comprises a VT domain of 20 Ns. As described herein, the variable targeting domain can be at least 12, 13, 14, 15, 16, 17, 18, 19, 20, 21, 22, 23, 24, 25, 26, 27, 28, 29 or 30 nucleotides in length. The VT domain of these sgRNAs can include

The terms "functional fragment ", "fragment that is functionally equivalent" and "functionally equivalent fragment" of a single guide RNA, crRNA or tracrRNA are used interchangeably herein, and refer to a portion or subsequence of the single guide RNA, crRNA or tracrRNA, respectively, of the present disclosure in which the ability to function as a guide RNA, crRNA or tracrRNA, respectively, is retained.

A functional fragments of a guide RNA (guide polynucleotide) of the present disclosure include a fragment of 20-40, 20-45, 20-50, 20-55, 20-60, 20-65, 20-70, 20-75, 20-80, 25-40, 25-45, 25-50, 25-55, 25-60, 25-65, 25-70, 25-75, 25-80, 30-40, 30-45, 30-50, 30-55, 30-60, 30-65, 30-70, 30-75, 30-80, 35-40, 35-45, 35-50, 35-55, 35-60, 35-65, 35-70, 35-75, 35-80, 40-45, 40-50, 40-55, 40-60, 40-65, 40-70, 40-75, 40-80, 45-50, 45-55, 45-60, 45-65, 45-70, 45-75, 45-80, 50-55, 50-60, 50-65, 50-70, 50-75, 50-80, 55-55, 55-60, 55-65, 55-70, 55-75, 55-80, 60-65, 60-70, 60-75, 60-80, 65-70, 65-75, 65-80, 70-75, 70-80 or 75-80 nucleotides of a reference guide RNA, such as the reference guide RNAs of SEQ ID NOs: 185-207.

Functional fragments of a crRNA of the present disclosure include a fragment of 5-30, 10-30, 15-30, 20-30, 25-30, 5-25, 10-25, 15-25, 20-25, 5-20, 10-20, 15-20, 5-15, 10-15, nucleotides of a reference crRNA, such as the reference crRNAs of SEQ ID NOs: 116-138.

The terms "functional variant", "Variant that is functionally equivalent" and "functionally equivalent variant" of a guide RNA, crRNA or tracrRNA (respectively) are used interchangeably herein, and refer to a variant of the guide RNA, crRNA or tracrRNA, respectively, of the present disclosure in which the ability to function as a guide RNA, crRNA or tracrRNA, respectively, is retained. A functional variant of a single guide RNA may comprise a nucleotide sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to reference single guide RNA, such as the reference single guide RNA of SEQ ID NOs:185-207, described herein. In some embodiments, a functional variant of a single guide RNA comprises a nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8, 9, 10, 11, 12, 13, 14, 15, 16, 17, 18, 19, 20, 21, 22, 23, 24, 25, 26, 27, 28, 29, 30 contiguous nucleotides to any one of the nucleotide sequences set forth in SEQ ID NOs: 185-207.

Functional variants of a guide polynucleotide of the present disclosure can comprise a modified guide polynucleotide wherein the modification comprises an engineered secondary structure, and/or an artificial loop, and/or a reduction in the length and/or degree of complementation in a region of hybridization compared to a region of hybridization of a reference guide polynucleotide, including the guide polynucleotides of SEQ ID NOs: 185-207, and/or a reduction in the length and/or degree of complementation in the portion of the protein-binding segment that forms a double stranded RNA duplex.

Functional variants of a guide polynucleotide of the present disclosure can comprise a modified guide polynucleotide wherein the modification comprises adding, removing, or otherwise altering loops and/or hairpins in the single guide RNA.

Functional variants of a guide polynucleotide of the present disclosure can comprise a modified guide polynucleotide wherein the modification comprises one or more modified nucleotides in the nucleotide sequence, wherein the one or more modified nucleotides comprises at least one non-naturally-occurring nucleotide, nucleotide mimetic (as described in US application US2014/0068797, published March 6, 2014), or analog thereof, or wherein the one or more modified nucleotides are selected from the group consisting of 2'-0-methylanalogs, 2'-fluoro analogs 2-aminopurine, 5-bromo-uridine, pseudouridine, and 7 -methylguanosine.

In one aspect, the functional variant of the guide RNA can form a guide RNA/Cas9 endonuclease complex that can recognize, bind to, and optionally nick or cleave a target sequence.

A functional variant of a crRNA may comprise an nucleotide sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to a reference crRNA, such as the reference crRNA of SEQ ID NOs:116-138, described herein. In one aspect, the functional variant of the crRNA can bind to a Cas9 endonuclease described herein and together with a tracrRNA, or as part of a guide RNA, can form a guide RNA/Cas9 endonuclease complex that can recognize, bind to, and optionally nick or cleave a target sequence.

A functional variant of a tracrRNA may comprise an nucleotide sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to a reference tracrRNA, such as the reference tracrRNA comprising a nucleotide sequence selected from the group consisting of SEQ ID NOs: 139-184, described herein. In one aspect, the functional variant of the tracrRNA can bind to a Cas9 endonuclease described herein and together with a crRNA, or as part of a guide RNA, can form a guide RNA/Cas9 endonuclease complex that can recognize, bind to, and optionally nick or cleave a target sequence.

Methods for determining if fragments or variants of a guide RNA, crRNA or tracrRNA are functional include methods that measure the Cas9 endonuclease activity when in complex with said fragment/variant guide RNA, crRNA and/or tracrRNA, as described herein. Methods for measuring Cas9 endonuclease activity (either double strand breaks or single strand breaks) include methods that measure the mutation frequency at a target site, as described herein. If mutations are observed at the intended target sites when using a fragment or variant of guide RNA, crRNA and/or tracrRNA of the present disclosure, in complex with a cas9 endonuclease of the present disclosure, the fragments or variants are functional.

The terms "single guide RNA" and "sgRNA" are used interchangeably herein and relate to a synthetic fusion of two RNA molecules, a crRNA (CRISPR RNA) comprising a variable targeting domain (linked to a tracr mate sequence that hybridizes to a tracrRNA), fused to a tracrRNA (trans-activating CRISPR RNA). The single guide RNA can comprise a crRNA or crRNA fragment and a tracrRNA or tracrRNA fragment of the type II CRISPR/Cas9 system that can form a complex with a type II Cas9 endonuclease, wherein said guide RNA/Cas9 endonuclease complex can direct the Cas9 endonuclease to a DNA target site, enabling the Cas9 endonuclease to recognize, bind to, and optionally nick or cleave (introduce a single or double strand break) the DNA target site.

Single guide RNAs targeting a target site in the genome of an organism can be designed by changing the Variable Targeting Domain (VT) of anyone of SEQ ID NOs: 185-207 (or a functional fragment or functional variant of SEQ ID NOs: 185-207) with any random nucleotide that can hybridize to any desired target sequence.

The terms "guide RNA/Cas9 endonuclease complex", "guide RNA/Cas9 endonuclease system", " guide RNA/Cas9 complex", "guide RNA/Cas9 system", "gRNA/Cas9 complex", "gRNA/Cas9 system", "RNA-guided endonuclease" , "RGEN" are used interchangeably herein and refer to at least one RNA component and at least one Cas9 endonuclease that are capable of forming a complex , wherein said guide RNA/Cas9 endonuclease complex can direct the Cas9 endonuclease to a DNA target site, enabling the Cas9 endonuclease to recognize, bind to, and optionally nick or cleave (introduce a single or double strand break) the DNA target site. A guide RNA/Cas9 endonuclease complex herein can comprise Cas9 protein(s) and suitable RNA component(s) of any of the four known CRISPR systems (Horvath and Barrangou, 2010, Science 327:167-170) such as a type I, II, or III CRISPR system. A guide RNA/Cas9 endonuclease complex can comprise a Type II Cas9 endonuclease and at least one RNA component (e.g., a crRNA and tracrRNA, or a gRNA). (See also U.S. Patent Application US 2015-0082478 A1, published on March 19, 2015 and US 2015-0059010 A1, published on February 26, 2015, both are hereby incorporated in its entirety by reference).

The guide polynucleotide can be introduced into a cell transiently, as single stranded polynucleotide or a double stranded polynucleotide, using any method known in the art such as, but not limited to, particle bombardment, *Agrobacterium transformation* or topical applications. The guide polynucleotide can also be introduced indirectly into a cell by introducing a recombinant DNA molecule (via methods such as, but not limited to, particle bombardment or *Agrobacterium transformation*) comprising a heterologous nucleic acid fragment encoding a guide polynucleotide, operably linked to a specific promoter that is capable of transcribing the guide RNA in said cell. The specific promoter can be, but is not limited to, a RNA polymerase III promoter, which allow for transcription of RNA with precisely defined, unmodified, 5'- and 3'-ends (DiCarlo et al., Nucleic Acids Res. 41: 4336-4343; Ma et al., Mol. Ther. Nucleic Acids 3:e161) as described in US application 62/036652, filed on August 13, 2014, incorporated herein in its entirety by reference.

Direct delivery of a polynucleotide modification template into plant cells can be achieved through particle mediated delivery, and any other direct method of delivery, such as but not limiting to, polyethylene glycol (PEG)-mediated transfection to protoplasts, whiskers mediated transformation, electroporation, particle bombardment, cell-penetrating peptides, or mesoporous silica nanoparticle (MSN)-mediated direct protein delivery can be successfully used for delivering a polynucleotide modification template in eukaryotic cells, such as plant cells.

The donor DNA can be introduced by any means known in the art. The donor DNA may be provided by any transformation method known in the art including, for example, *Agrobacterium*-mediated transformation or biolistic particle bombardment. The donor DNA may be present transiently in the cell or it could be introduced via a viral replicon. In the presence of the Cas9 endonuclease and the target site, the donor DNA is inserted into the transformed plant's genome.

Direct delivery of any one of the guided Cas9 system components can be accompanied by direct delivery (co-delivery) of other mRNAs that can promote the enrichment and/or visualization of cells receiving the guide polynucleotide/Cas9 endonuclease complex components. For example, direct co-delivery of the guide polynucleotide/Cas9 endonuclease components (and/or guide polynucleotide/Cas9 endonuclease complex itself) together with mRNA encoding phenotypic markers (such as but not limiting to transcriptional activators such as CRC (Bruce et al. 2000 The Plant Cell 12:65-79) can enable the selection and enrichment of cells without the use of an exogenous selectable marker by restoring function to a non-functional gene product as described in PCT/US16/57272 filed October 17, 2016 and PCT/US16/57279, filed October 17, 2016, both incorporated herein by reference.

In one aspect, the guide polynucleotide/Cas9 endonuclease complex of the present disclosure comprises a guide RNA of the present disclosure (such as a single guide RNA selected from the group consisting of SEQ ID NOs: 185-207, a functional fragment of SEQ ID NOs: 185-207, and a functional variant of SEQ ID NOs: 185-207) in complex with a Cas9 endonuclease of the present disclosure (such as a Cas9 endonuclease selected from the group consisting of SEQ ID NOs: 47-69, a functional fragment of SEQ ID NOs: 47-69, and a functional variant of SEQ ID NOs: 47-69).

The terms "target site", "target sequence", "target site sequence, "target DNA", "target locus", "genomic target site", "genomic target sequence", "genomic target locus" and "protospacer", are used interchangeably herein and refer to a polynucleotide sequence such as, but not limited to, a nucleotide sequence on a chromosome, episome, or any other DNA molecule in the genome (including chromosomal, choloroplastic, mitochondrial DNA, plasmid DNA) of a cell, at which a guide polynucleotide/Cas9 endonuclease complex can recognize, bind to, and optionally nick or cleave . The target site can be an endogenous site in the genome of a cell, or alternatively, the target site can be heterologous to the cell and thereby not be naturally occurring in the genome of the cell, or the target site can be found in a heterologous genomic location compared to where it occurs in nature. As used herein, terms "endogenous target sequence" and "native target sequence" are used interchangeable herein to refer to a target sequence that is endogenous or native to the genome of a cell and is at the endogenous or native position of that target sequence in the genome of the cell. Cells include, but are not limited to, human, non-human, animal, bacterial, fungal, insect, yeast, non-conventional yeast, and plant cells as well as plants and seeds produced by the methods described herein. An "artificial target site" or "artificial target sequence" are used interchangeably herein and refer to a target sequence that has been introduced into the genome of a cell. Such an artificial target sequence can be identical in sequence to an endogenous or native target sequence in the genome of a cell but be located in a different position (*i.e*., a non-endogenous or non-native position) in the genome of a cell.

An "altered target site", "altered target sequence", "modified target site", "modified target sequence" are used interchangeably herein and refer to a target sequence as disclosed herein that comprises at least one alteration when compared to non-altered target sequence. Such "alterations" include, for example:
(i) replacement of at least one nucleotide,
(ii) a deletion of at least one nucleotide,
(iii) an insertion of at least one nucleotide, or
(iv) any combination of (i) - (iii).

Methods for "modifying a target site" and "altering a target site" are used interchangeably herein and refer to methods for producing an altered target site.

The length of the target DNA sequence (target site) can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic, that is, the sequence on one strand reads the same in the opposite direction on the complementary strand. The nick/cleavage site can be within the target sequence or the nick/cleavage site could be outside of the target sequence. In another variation, the cleavage could occur at nucleotide positions immediately opposite each other to produce a blunt end cut or, in other cases, the incisions could be staggered to produce single-stranded overhangs, also called "sticky ends", which can be either 5' overhangs, or 3' overhangs. Active variants of genomic target sites can also be used. Such active variants can comprise at least 65%, 70%, 75%, 80%, 85%, 90%, 91%, 92%, 93%, 94%, 95%, 96%, 97%, 98%, 99% or more sequence identity to the given target site, wherein the active variants retain biological activity and hence are capable of being recognized and cleaved by an Cas9 endonuclease. Assays to measure the single or double-strand break of a target site by an endonuclease are known in the art and generally measure the overall activity and specificity of the agent on DNA substrates containing recognition sites.

A "protospacer adjacent motif" (PAM) herein refers to a short nucleotide sequence adjacent to a target sequence (protospacer) that is recognized (targeted) by a guide polynucleotide/Cas9 endonuclease system described herein. The Cas9 endonuclease may not successfully recognize a target DNA sequence if the target DNA sequence is not followed by a PAM sequence. The sequence and length of a PAM herein can differ depending on the Cas9 protein or Cas9 protein complex used. The PAM sequence can be of any length but is typically 1, 2, 3, 4, 5, 6, 7, 8, 9, 10, 11, 12, 13, 14, 15, 16, 17, 18, 19 or 20 nucleotides long.

A "randomized PAM" and "randomized protospacer adjacent motif" are used interchangeably herein, and refer to a random DNA sequence adjacent to a target sequence (protospacer) that is recognized (targeted) by a guide polynucleotide/Cas9 endonuclease system described herein. The randomized PAM sequence can be of any length but is typically 1, 2, 3, 4, 5, 6, 7, 8, 9, 10, 11, 12, 13, 14, 15, 16, 17, 18, 19 or 20 nucleotides long. A randomized nucleotide includes anyone of the nucleotides A, C, G or T.

Given the diversity of Type II CRISPR-Cas systems (Fonfara et al. (2014) Nucleic Acids Res. 42:2577-2590), it is plausible that many of the Cas9 endonucleases and cognate guide RNAs may have unique sequence recognition and enzymatic properties different from those previously described or characterized. For example, cleavage activity and specificity may be enhanced or proto-spacer adjacent motif (PAM) sequence may be different leading to increased genomic target site density. To tap into this vast unexplored diversity and expand the repertoire of Cas9 endonucleases and cognate guide RNAs available for genome targeting, the components of Cas9 target site recognition, the PAM sequence and the guide RNA (either duplexed CRISPR RNA (crRNA) and trans-activating CRISPR RNA (tracrRNA) or chimeric fusion of crRNA and tracrRNA (single guide RNA (sgRNA), need to be established for each new system. As described herein, CRISPR-Cas loci (including Cas9 genes and open reading frames, CRISPR array and anti-repeats) from uncharacterized CRISPR-Cas systems were identified by searching internal Pioneer-DuPont databases consisting of microbial genomes. The Cas9 endonuclease described herein can be expressed and purified by methods known in the art. As described herein, the transcriptional direction of the tracrRNA for all the CRISPR-Cas systems can be deduced (as described in PCT/US16/32028 filed May 12, 2016, and PCT/US16/32073 filed May 12, 2016), and examples of sgRNAs (described herein, see SEQ ID NOs: 185-207) and its components (VT, crRNA repeat, loop, anti-repeat and 3'tracrRNA) were identified for each new CRISPR-Cas endonuclease described herein.

The terms "targeting", "gene targeting" and "DNA targeting" are used interchangeably herein. DNA targeting herein may be the specific introduction of a knock-out, edit, or knock-in at a particular DNA sequence, such as in a chromosome or plasmid of a cell. In general, DNA targeting can be performed herein by cleaving one or both strands at a specific DNA sequence in a cell with an endonuclease associated with a suitable polynucleotide component. Such DNA cleavage, if a double-strand break (DSB) can prompt NHEJ or HDR processes which can lead to modifications at the target site.

A targeting method herein can be performed in such a way that two or more DNA target sites are targeted in the method, for example. Such a method can optionally be characterized as a multiplex method. Two, three, four, five, six, seven, eight, nine, ten, or more target sites can be targeted at the same time in certain embodiments. A multiplex method is typically performed by a targeting method herein in which multiple different RNA components are provided, each designed to guide a guide polynucleotide/Cas9 endonuclease complex to a unique DNA target site.

The terms "knock-out", "gene knock-out" and "genetic knock-out" are used interchangeably herein. A knock-out represents a DNA sequence of a cell that has been rendered partially or completely inoperative by targeting with a Cas9 protein; such a DNA sequence prior to knock-out could have encoded an amino acid sequence, or could have had a regulatory function (e.g., promoter), for example. A knock-out may be produced by an indel (insertion or deletion of nucleotide bases in a target DNA sequence through NHEJ), or by specific removal of sequence that reduces or completely destroys the function of sequence at or near the targeting site.

In one embodiment of the disclosure, the method comprises a method for modifying a target site in the genome of a cell, the method comprising introducing into said cell at least one guide RNA and at least one Cas9 endonuclease selected from the group consisting of SEQ ID NOs: 47-69, a functional fragment of SEQ ID NOs: 47-69, and a functional variant of SEQ ID NOs: 47-69 , wherein said guide RNA and Cas9 endonuclease can form a complex that is capable of recognizing, binding to, and optionally nicking or cleaving all or part of said target site.. The method can further comprise identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii).

The guide polynucleotide/Cas9 endonuclease system can be used in combination with a co-delivered polynucleotide modification template to allow for editing (modification) of a genomic nucleotide sequence of interest. (See also U.S. Patent Application US 2015-0082478 A1, published on March 19, 2015 and WO2015/026886 A1, published on February 26, 2015, both are hereby incorporated in its entirety by reference.)

A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, or (iv) any combination of (i) - (iii).

The term "polynucleotide modification template" includes a polynucleotide that comprises at least one nucleotide modification when compared to the nucleotide sequence to be edited. A nucleotide modification can be at least one nucleotide substitution, addition or deletion. Optionally, the polynucleotide modification template can further comprise homologous nucleotide sequences flanking the at least one nucleotide modification, wherein the flanking homologous nucleotide sequences provide sufficient homology to the desired nucleotide sequence to be edited.

In one embodiment, the disclosure describes a method for editing a nucleotide sequence in the genome of a cell, the method comprising introducing into said cell a polynucleotide modification template, at least one guide RNA and at least one Cas9 endonuclease selected from the group consisting of SEQ ID NOs: 47-69, a functional fragment of SEQ ID NOs: 47-69, and a functional variant of SEQ ID NOs: 47-69, wherein said polynucleotide modification template comprises at least one nucleotide modification of said nucleotide sequence, wherein said guide RNA and Cas9 endonuclease can form a complex that is capable of recognizing, binding to, and optionally nicking or cleaving all or part of said target site.

Cells include, but are not limited to, human, non-human, animal, bacterial, fungal, insect, yeast, non-conventional yeast, and plant cells as well as plants and seeds produced by the methods described herein. Plant cells include cells selected from the group consisting of maize, rice, sorghum, rye, barley, wheat, millet, oats, sugarcane, turfgrass, or switchgrass, soybean, canola, alfalfa, sunflower, cotton, tobacco, peanut, potato, tobacco, *Arabidopsis*, and safflower cells. The nucleotide to be edited can be located within or outside a target site recognized and cleaved by a Cas9 endonuclease.

In one embodiment, the at least one nucleotide modification is not a modification at a target site recognized and cleaved by a Cas9 endonuclease. In another embodiment, the nucleotide modification is located in close proximity to the target site. In another embodiment, there are at least 1, 2, 3, 4, 5, 6, 7, 8, 9, 10, 11, 12, 13, 14, 15, 16, 17, 18, 19, 20, 21, 22, 23, 24, 25, 26, 27, 30, 40, 50, 100, 200, 300, 400, 500, 600, 700, 900 or 1000 nucleotides between the at least one nucleotide to be edited and the genomic target site.

Genome editing can be accomplished using any method of gene editing available. For example, gene editing can be accomplished through the introduction into a host cell of a polynucleotide modification template (sometimes also referred to as a gene repair oligonucleotide) containing a targeted modification to a gene within the genome of the host cell. The polynucleotide modification template for use in such methods can be either single-stranded or double-stranded. Examples of such methods are generally described, for example, in US Publication No. 2013/0019349.

In some embodiments, gene editing may be facilitated through the induction of a double-stranded break (DSB) in a defined position in the genome near the desired alteration. DSBs can be induced using any DSB-inducing agent available, including, but not limited to, TALENs, meganucleases, zinc finger nucleases, Cas9-gRNA systems (based on bacterial CRISPR-Cas systems), and the like. In some embodiments, the introduction of a DSB can be combined with the introduction of a polynucleotide modification template.

The process for editing a genomic sequence combining DSB and modification templates generally comprises: introducing into a host cell, a DSB-inducing agent, or a nucleic acid encoding a DSB-inducing agent, that recognizes a target sequence in the chromosomal sequence and is able to induce a DSB in the genomic sequence, and at least one polynucleotide modification template comprising at least one nucleotide alteration when compared to the nucleotide sequence to be edited. The polynucleotide modification template can further comprise nucleotide sequences flanking the at least one nucleotide alteration, in which the flanking sequences are substantially homologous to the chromosomal region flanking the DSB. Genome editing using DSB-inducing agents, such as Cas9-gRNA complexes, has been described, for example in U.S. Patent Application US 2015-0082478 A1, published on March 19, 2015, WO2015/026886 A1, published on February 26, 2015, US application 62/023246, filed on July 07, 2014, and US application 62/036,652, filed on August 13, 2014, all of which are incorporated by reference herein.

The terms "knock-in", "gene knock-in, "gene insertion" and "genetic knock-in" are used interchangeably herein. A knock-in represents the replacement or insertion of a DNA sequence at a specific DNA sequence in cell by targeting with a Cas9 protein (by HR, wherein a suitable donor DNA polynucleotide is also used). Examples of knock-ins are a specific insertion of a heterologous amino acid coding sequence in a coding region of a gene, or a specific insertion of a transcriptional regulatory element in a genetic locus.

Various methods and compositions can be employed to obtain a cell or organism having a polynucleotide of interest inserted in a target site for a Cas9 endonuclease. Such methods can employ homologous recombination to provide integration of the polynucleotide of Interest at the target site. In one method provided, a polynucleotide of interest is introduced into the organism cell in a donor DNA construct. As used herein, "donor DNA" is a DNA construct that comprises a polynucleotide of Interest to be inserted into the target site of a Cas9 endonuclease. The donor DNA construct further comprises a first and a second region of homology that flank the polynucleotide of Interest. The first and second regions of homology of the donor DNA share homology to a first and a second genomic region, respectively, present in or flanking the target site of the cell or organism genome. By "homology" is meant DNA sequences that are similar. For example, a "region of homology to a genomic region" that is found on the donor DNA is a region of DNA that has a similar sequence to a given "genomic region" in the cell or organism genome. A region of homology can be of any length that is sufficient to promote homologous recombination at the cleaved target site. For example, the region of homology can comprise at least 5-10, 5-15, 5-20, 5-25, 5-30, 5-35, 5-40, 5-45, 5-50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 or more bases in length such that the region of homology has sufficient homology to undergo homologous recombination with the corresponding genomic region. "Sufficient homology" indicates that two polynucleotide sequences have sufficient structural similarity to act as substrates for a homologous recombination reaction. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of the sequences.

The amount of homology or sequence identity shared by a target and a donor polynucleotide can vary and includes total lengths and/or regions having unit integral values in the ranges of about 1-20 bp, 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kb, 1.5-3 kb, 2-4 kb, 2.5-5 kb, 3-6 kb, 3.5-7 kb, 4-8 kb, 5-10 kb, or up to and including the total length of the target site. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bps. The amount of homology can also described by percent sequence identity over the full aligned length of the two polynucleotides which includes percent sequence identity of about at least 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Sufficient homology includes any combination of polynucleotide length, global percent sequence identity, and optionally conserved regions of contiguous nucleotides or local percent sequence identity, for example sufficient homology can be described as a region of 75-150 bp having at least 80% sequence identity to a region of the target locus. Sufficient homology can also be described by the predicted ability of two polynucleotides to specifically hybridize under high stringency conditions, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular BiologyHybridization with Nucleic Acid Probes, (Elsevier, New York).

In one embodiment of the disclosure, the method comprises a method for modifying a target site in the genome of a cell, the method comprising introducing into said cell at least one guide RNA, at least one donor DNA, and at least one Cas9 endonuclease selected from the group consisting of SEQ ID NOs: 47-69, a functional fragment of SEQ ID NOs: 47-69, and a functional variant of SEQ ID NOs: 47-69, wherein said at least one guide RNA and at least one Cas9 endonuclease can form a complex that is capable of recognizing, binding to, and optionally nicking or cleaving all or part of said target site, wherein said donor DNA comprises a polynucleotide of interest.

The guide polynucleotide/Cas9 endonuclease systems described herein can be used for introducing one or more polynucleotides of interest or one or more traits of interest into one or more target sites by introducing one or more guide polynucleotides, one Cas endonuclease, and optionally one or more donor DNAs into a plant cell (as described in US patent application No. 14/463,687, file August 20, 2014, incorporated by reference herein). A fertile plant can be produced from that plant cell that comprises an alteration at said one or more target sites, wherein the alteration is selected from the group consisting of (i) replacement of at least one nucleotide, (ii) a deletion of at least one nucleotide, (iii) an insertion of at least one nucleotide, and (iv) any combination of (i) - (iii). Plants comprising these altered target sites can be crossed with plants comprising at least one gene or trait of interest in the same complex trait locus; thereby further stacking traits in said complex trait locus (see also US-2013-0263324-A1, published 03 Oct 2013 and in PCT/US13/22891, published January 24, 2013).

As used herein, a "genomic region" is a segment of a chromosome in the genome of a cell that is present on either side of the target site or, alternatively, also comprises a portion of the target site. The genomic region can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800. 5-2900, 5-3000, 5-3100 or more bases such that the genomic region has sufficient homology to undergo homologous recombination with the corresponding region of homology.

Polynucleotides of interest and/or traits can be stacked together in a complex trait locus as described in US 2013/0263324-A1, published October 3, 2013 and in PCT/US13/22891, published January 24, 2013, both applications are hereby incorporated by reference. The guide polynucleotide/Cas9 endonuclease system described herein provides for an efficient system to generate double strand breaks and allows for traits to be stacked in a complex trait locus.

The structural similarity between a given genomic region and the corresponding region of homology found on the donor DNA can be any degree of sequence identity that allows for homologous recombination to occur. For example, the amount of homology or sequence identity shared by the "region of homology" of the donor DNA and the "genomic region" of the organism genome can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination

The region of homology on the donor DNA can have homology to any sequence flanking the target site. While in some embodiments the regions of homology share significant sequence homology to the genomic sequence immediately flanking the target site, it is recognized that the regions of homology can be designed to have sufficient homology to regions that may be further 5' or 3' to the target site. In still other embodiments, the regions of homology can also have homology with a fragment of the target site along with downstream genomic regions. In one embodiment, the first region of homology further comprises a first fragment of the target site and the second region of homology comprises a second fragment of the target site, wherein the first and second fragments are dissimilar.

As used herein, "homologous recombination" includes the exchange of DNA fragments between two DNA molecules at the sites of homology. The frequency of homologous recombination is influenced by a number of factors. Different organisms vary with respect to the amount of homologous recombination and the relative proportion of homologous to non-homologous recombination. Generally, the length of the region of homology affects the frequency of homologous recombination events: the longer the region of homology, the greater the frequency. The length of the homology region needed to observe homologous recombination is also species-variable. In many cases, at least 5 kb of homology has been utilized, but homologous recombination has been observed with as little as 25-50 bp of homology. See, for example, Singer et al., (1982) Cell 31:25-33; Shen and Huang, (1986) Genetics 112:441-57; Watt et al., (1985) Proc. Natl. Acad. Sci. USA 82:4768-72, Sugawara and Haber, (1992) Mol Cell Biol 12:563-75, Rubnitz and Subramani, (1984) Mol Cell Biol 4:2253-8; Ayares et al., (1986) Proc. Natl. Acad. Sci. USA 83:5199-203; Liskay et al., (1987) Genetics 115:161-7.

Homology-directed repair (HDR) is a mechanism in cells to repair double-stranded and single stranded DNA breaks. Homology-directed repair includes homologous recombination (HR) and single-strand annealing (SSA) (Lieber. 2010 Annu. Rev. Biochem. 79:181-211). The most common form of HDR is called homologous recombination (HR), which has the longest sequence homology requirements, between the donor and acceptor DNA. Other forms of HDR include single-stranded annealing (SSA) and breakage-induced replication, and these require shorter sequence homology relative to HR. Homology-directed repair at nicks (single-stranded breaks) can occur via a mechanism distinct from HDR at double-strand breaks (Davis and Maizels. (2014) PNAS (0027-8424), 111 (10), p. E924-E932).

Alteration of the genome of a plant cell, for example, through homologous recombination (HR), is a powerful tool for genetic engineering. Homologous recombination has been demonstrated in plants (Halfter et al., (1992) Mol Gen Genet 231:186-93) and insects (Dray and Gloor, 1997, Genetics 147:689-99). Homologous recombination has also been accomplished in other organisms. For example, at least 150-200 bp of homology was required for homologous recombination in the parasitic protozoan Leishmania (Papadopoulou and Dumas, (1997) Nucleic Acids Res 25:4278-86). In the filamentous fungus Aspergillus *nidulans*, gene replacement has been accomplished with as little as 50 bp flanking homology (Chaveroche et al., (2000) Nucleic Acids Res 28:e97). Targeted gene replacement has also been demonstrated in the ciliate *Tetrahymena thermophila* (Gaertig et al., (1994) Nucleic Acids Res 22:5391-8). In mammals, homologous recombination has been most successful in the mouse using pluripotent embryonic stem cell lines (ES) that can be grown in culture, transformed, selected and introduced into a mouse embryo (Watson et al., 1992, Recombinant DNA, 2nd Ed., (Scientific American Books distributed by WH Freeman & Co.).

Error-prone DNA repair mechanisms can produce mutations at double-strand break sites. The Non-Homologous-End-Joining (NHEJ) pathways are the most common repair mechanism to bring the broken ends together (Bleuyard et al., (2006) DNA Repair 5:1-12). The structural integrity of chromosomes is typically preserved by the repair, but deletions, insertions, or other rearrangements are possible. The two ends of one double-strand break are the most prevalent substrates of NHEJ (Kirik et al., (2000) EMBO J 19:5562-6), however if two different double-strand breaks occur, the free ends from different breaks can be ligated and result in chromosomal deletions (Siebert and Puchta, (2002) Plant Cell 14:1121-31), or chromosomal translocations between different chromosomes (Pacher et al., (2007) Genetics 175:21-9).

Episomal DNA molecules can also be ligated into the double-strand break, for example, integration of T-DNAs into chromosomal double-strand breaks (Chilton and Que, (2003) Plant Physiol 133:956-65; Salomon and Puchta, (1998) EMBO J 17:6086-95). Once the sequence around the double-strand breaks is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

Once a double-strand break is induced in the DNA, the cell's DNA repair mechanism is activated to repair the break. Error-prone DNA repair mechanisms can produce mutations at double-strand break sites. The most common repair mechanism to bring the broken ends together is the nonhomologous end-joining (NHEJ) pathway (Bleuyard et al., (2006) DNA Repair 5:1-12). The structural integrity of chromosomes is typically preserved by the repair, but deletions, insertions, or other rearrangements are possible (Siebert and Puchta, (2002) Plant Cell 14:1121-31; Pacher et al., (2007) Genetics 175:21-9).

Alternatively, the double-strand break can be repaired by homologous recombination between homologous DNA sequences. Once the sequence around the double-strand break is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

The donor DNA may be introduced by any means known in the art. The donor DNA may be provided by any transformation method known in the art including, for example, Agrobacterium-mediated transformation or biolistic particle bombardment. The donor DNA may be present transiently in the cell or it could be introduced via a viral replicon. In the presence of the Cas9 endonuclease and the target site, the donor DNA is inserted into the transformed plant's genome. (see guide language)

Further uses for guide RNA/Cas9 endonuclease systems have been described (See U.S. Patent Application US 2015-0082478 A1, published on March 19, 2015, WO2015/026886 A1, published on February 26, 2015, US 2015-0059010 A1, published on February 26, 2015, US application 62/023246, filed on July 07, 2014, and US application 62/036,652, filed on August 13, 2014, all of which are incorporated by reference herein) and include but are not limited to modifying or replacing nucleotide sequences of interest (such as a regulatory elements), insertion of polynucleotides of interest, gene knock-out, gene-knock in, modification of splicing sites and/or introducing alternate splicing sites, modifications of nucleotide sequences encoding a protein of interest, amino acid and/or protein fusions, and gene silencing by expressing an inverted repeat into a gene of interest.

Given the diversity of Type II CRISPR-Cas systems (Fonfara et al. (2014) Nucleic Acids Res. 42:2577-2590), it is plausible that many of the Cas9 endonucleases and cognate guide RNAs may have unique sequence recognition and enzymatic properties different from those previously described or characterized. For example, cleavage activity and specificity may be enhanced or proto-spacer adjacent motif (PAM) sequence may be different leading to increased genomic target site density. To tap into this vast unexplored diversity and expand the repertoire of Cas9 endonucleases and cognate guide RNAs available for genome targeting, the two components of Cas9 target site recognition, the PAM sequence and the guide RNA (either duplexed CRISPR RNA (crRNA) and trans-activating CRISPR RNA (tracrRNA) or chimeric fusion of crRNA and tracrRNA (single guide RNA (sgRNA), need to be established for each new system.

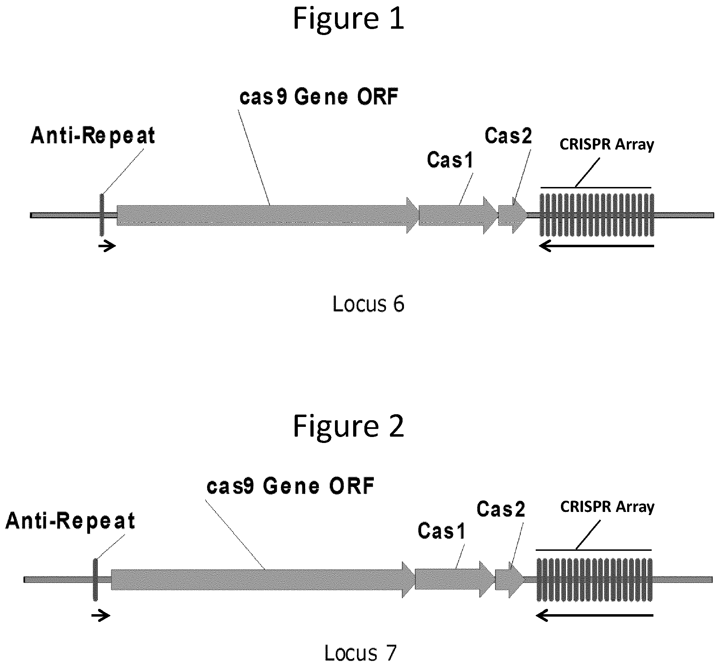

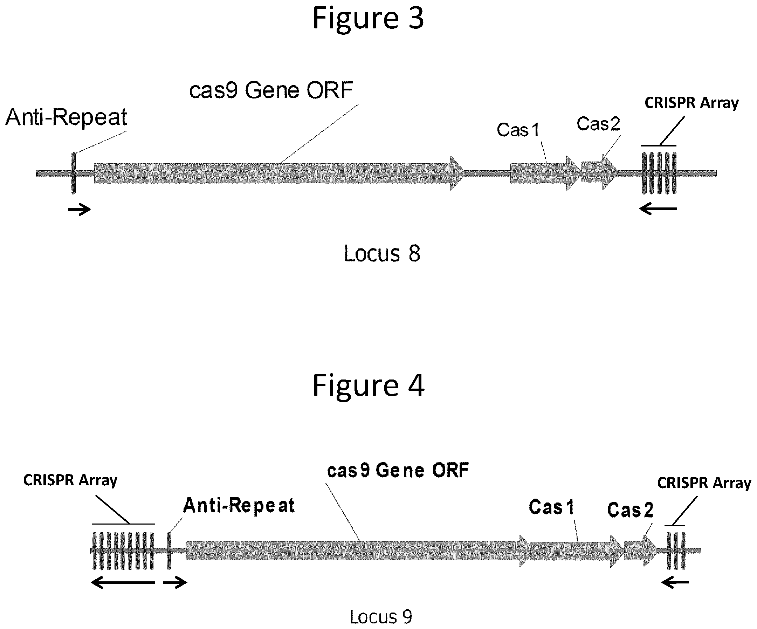

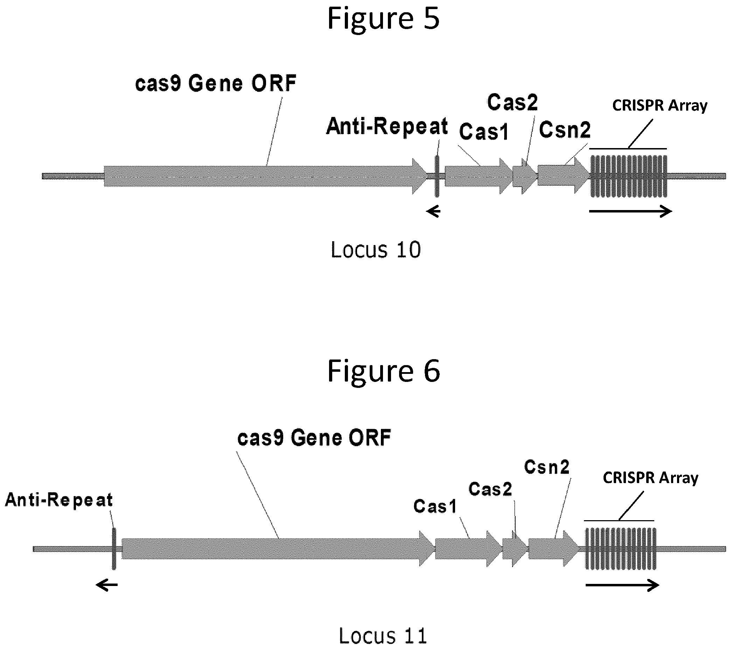

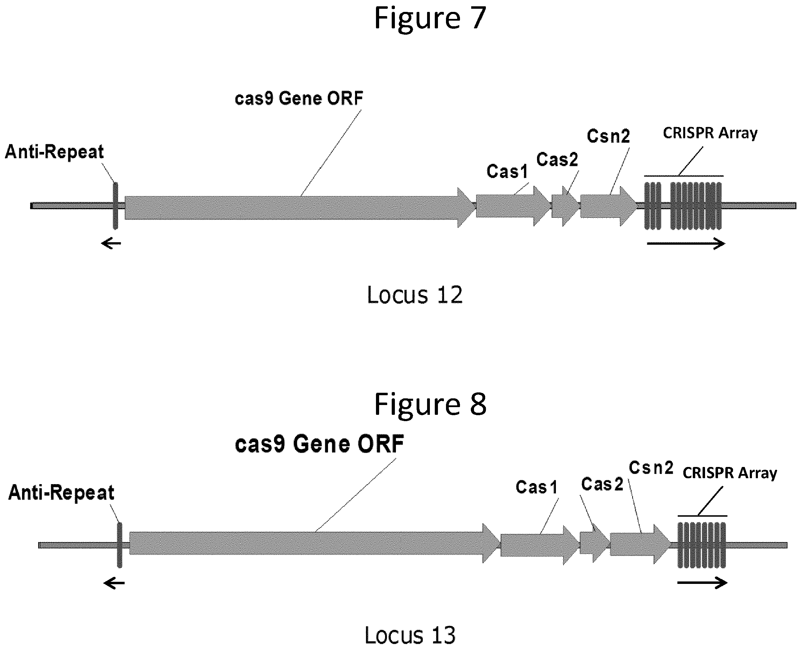

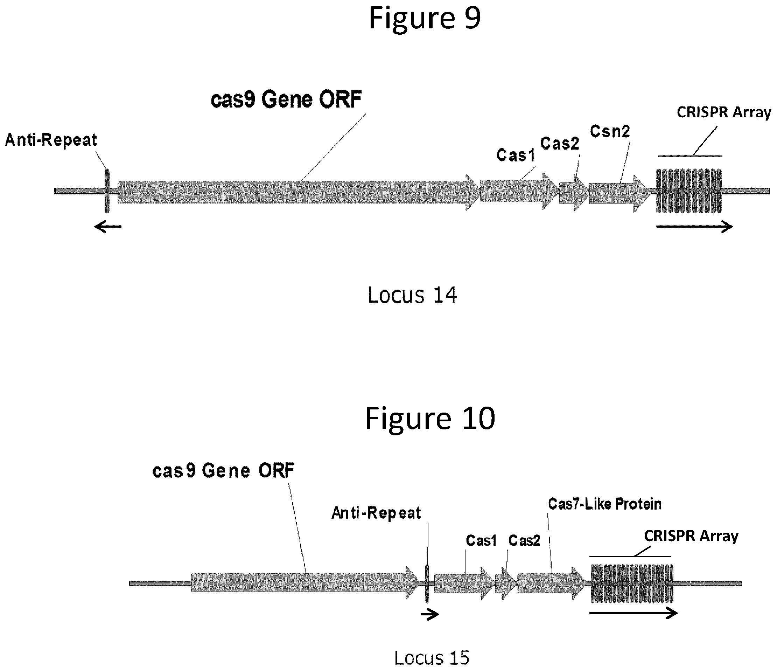

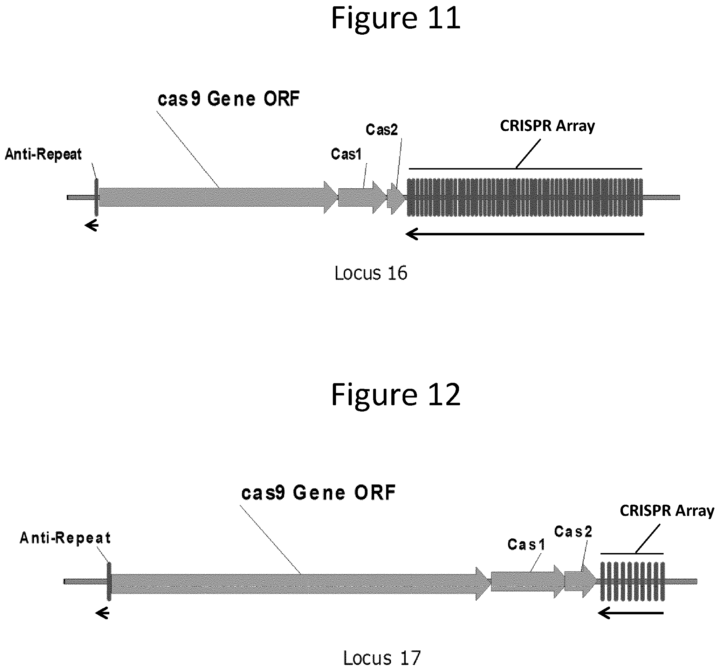

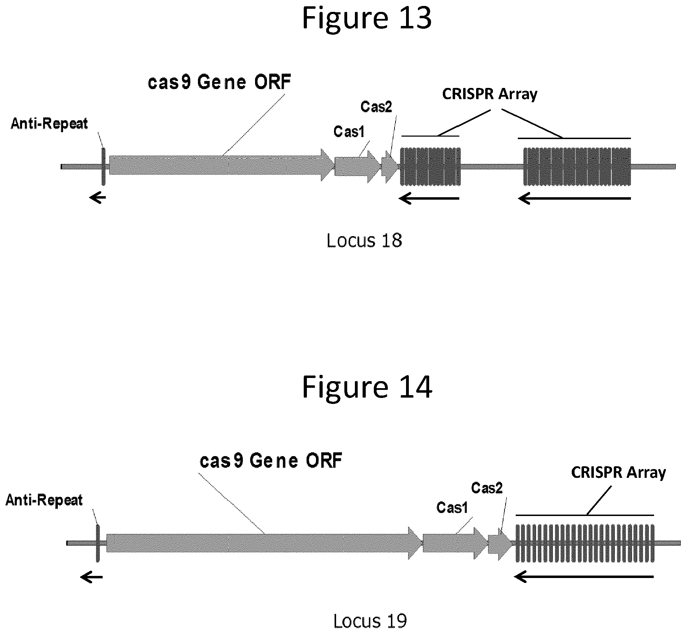

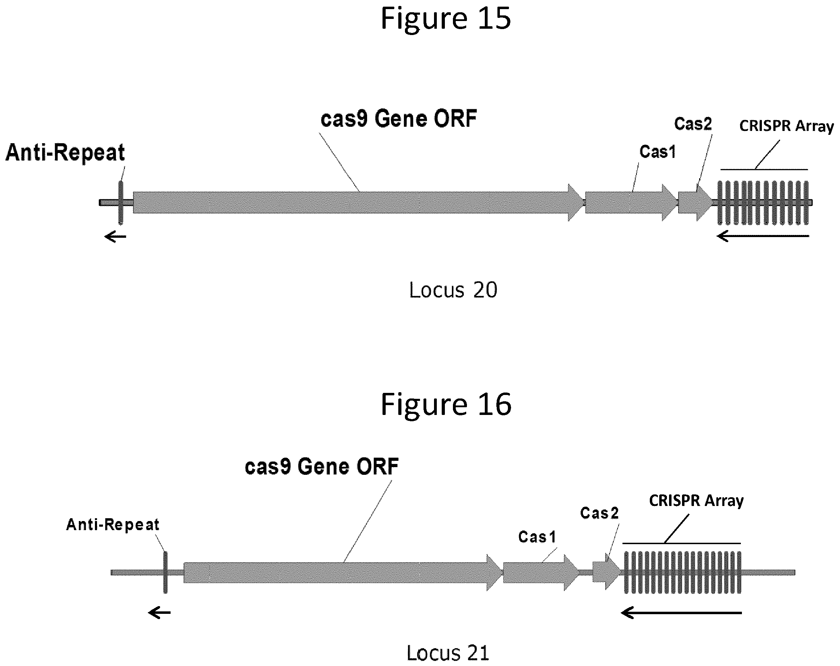

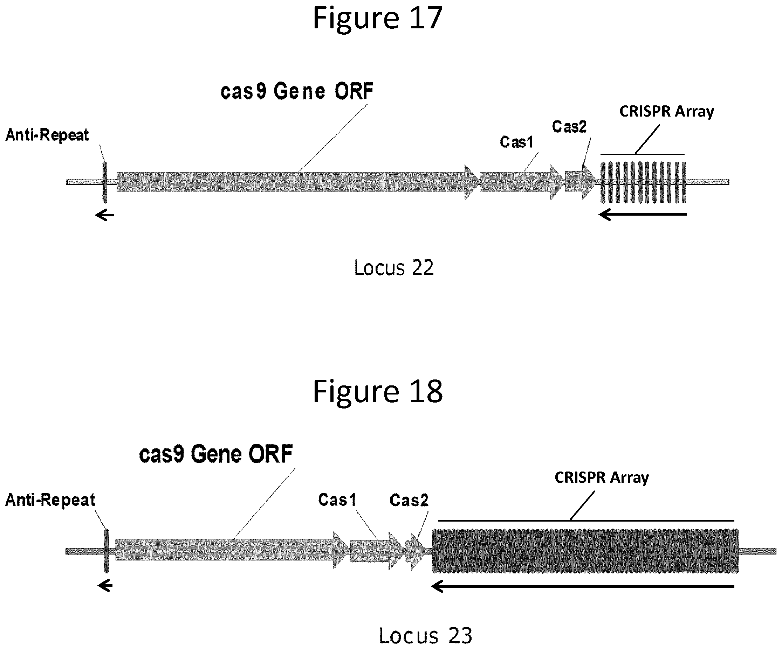

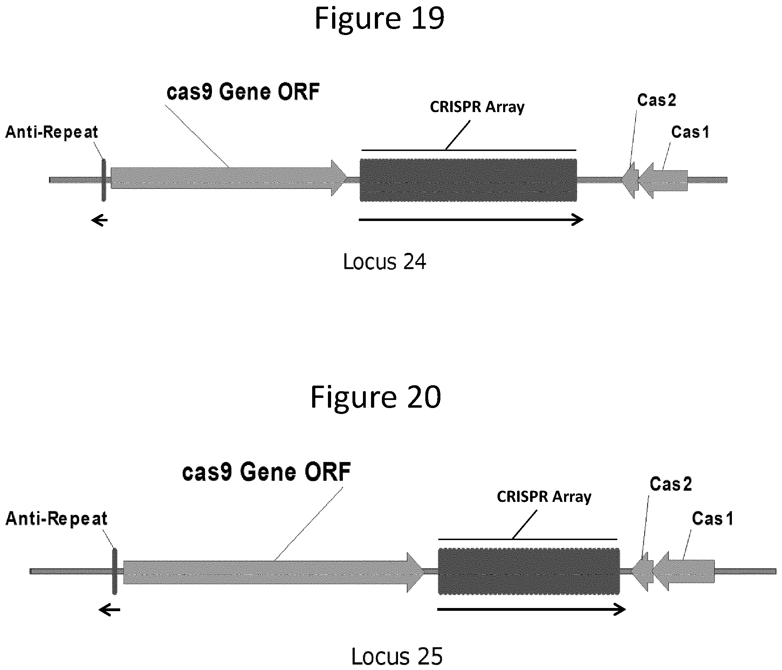

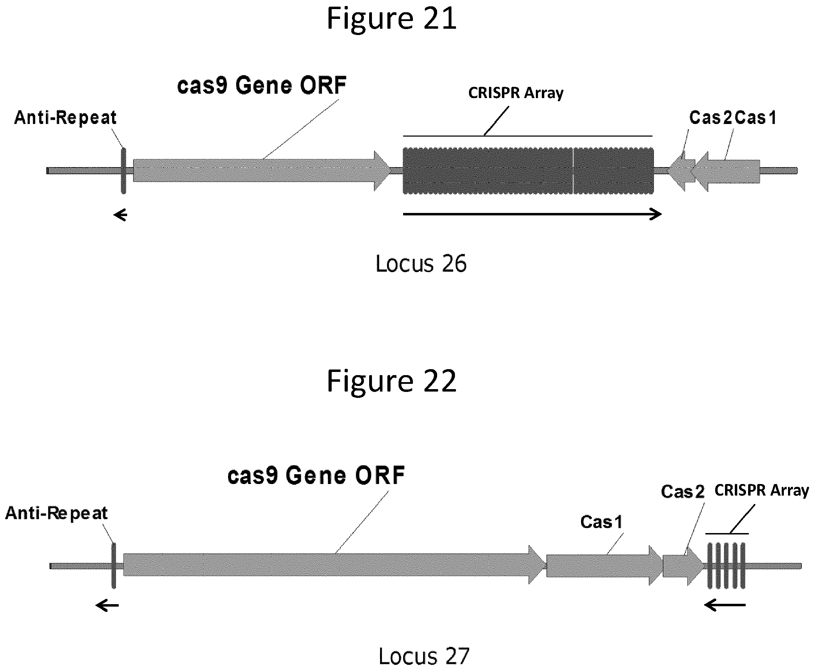

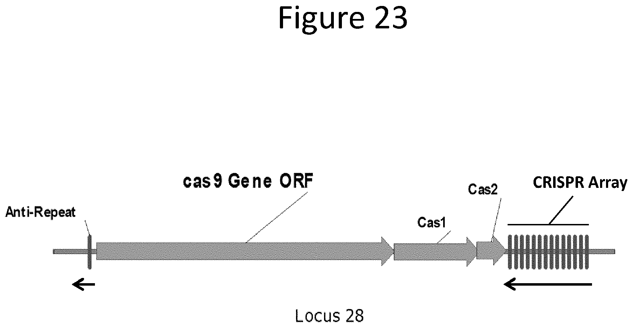

As described herein, CRISPR-Cas loci (including Cas9 genes and open reading frames, CRISPR array and anti-repeats) from uncharacterized CRISPR-Cas systems (Figures 1-23) were identified by searching internal Pioneer-DuPont databases consisting of microbial genomes. The Cas9 endonuclease described herein can be expressed and purified by methods known in the art (such as those described in Example 2 of US patent applications 62/162,377 filed May 15, 2015, incorporated herein by reference). As described herein (Example 1), the transcriptional direction of the tracrRNA for all the CRISPR-Cas systems can be deduced and examples of sgRNAs (SEQ ID NOs:) and its components (VT, crRNA repeat, loop, anti-repeat and 3'tracrRNA) were identified for each new diverse CRISPR-Cas endonuclease described herein.

Polynucleotides of interest are further described herein and include polynucleotides reflective of the commercial markets and interests of those involved in the development of the crop. Crops and markets of interest change, and as developing nations open up world markets, new crops and technologies will emerge also. In addition, as our understanding of agronomic traits and characteristics such as yield and heterosis increase, the choice of genes for genetic engineering will change accordingly.

Further provided are methods for identifying at least one plant cell, comprising in its genome, a polynucleotide of interest integrated at the target site. A variety of methods are available for identifying those plant cells with insertion into the genome at or near to the target site without using a screenable marker phenotype. Such methods can be viewed as directly analyzing a target sequence to detect any change in the target sequence, including but not limited to PCR methods, sequencing methods, nuclease digestion, Southern blots, and any combination thereof. See, for example, US Patent Application 12/147,834, herein incorporated by reference to the extent necessary for the methods described herein. The method also comprises recovering a plant from the plant cell comprising a polynucleotide of Interest integrated into its genome. The plant may be sterile or fertile. It is recognized that any polynucleotide of interest can be provided, integrated into the plant genome at the target site, and expressed in a plant.

Polynucleotides/polypeptides of interest include, but are not limited to, herbicide-resistance coding sequences, insecticidal coding sequences, nematicidal coding sequences, antimicrobial coding sequences, antifungal coding sequences, antiviral coding sequences, abiotic and biotic stress tolerance coding sequences, or sequences modifying plant traits such as yield, grain quality, nutrient content, starch quality and quantity, nitrogen fixation and/or utilization, fatty acids, and oil content and/or composition. More specific polynucleotides of interest include, but are not limited to, genes that improve crop yield, polypeptides that improve desirability of crops, genes encoding proteins conferring resistance to abiotic stress, such as drought, nitrogen, temperature, salinity, toxic metals or trace elements, or those conferring resistance to toxins such as pesticides and herbicides, or to biotic stress, such as attacks by fungi, viruses, bacteria, insects, and nematodes, and development of diseases associated with these organisms. General categories of genes of interest include, for example, those genes involved in information, such as zinc fingers, those involved in communication, such as kinases, and those involved in housekeeping, such as heat shock proteins. More specific categories of transgenes, for example, include genes encoding important traits for agronomics, insect resistance, disease resistance, herbicide resistance, fertility or sterility, grain characteristics, and commercial products. Genes of interest include, generally, those involved in oil, starch, carbohydrate, or nutrient metabolism as well as those affecting kernel size, sucrose loading, and the like that can be stacked or used in combination with other traits, such as but not limited to herbicide resistance, described herein.

Agronomically important traits such as oil, starch, and protein content can be genetically altered in addition to using traditional breeding methods. Modifications include increasing content of oleic acid, saturated and unsaturated oils, increasing levels of lysine and sulfur, providing essential amino acids, and also modification of starch. Hordothionin protein modifications are described in U.S. Patent Nos. 5,703,049, 5,885,801, 5,885,802, and 5,990,389, herein incorporated by reference.

Polynucleotide sequences of interest may encode proteins involved in providing disease or pest resistance. By "disease resistance" or "pest resistance" is intended that the plants avoid the harmful symptoms that are the outcome of the plant-pathogen interactions. Pest resistance genes may encode resistance to pests that have great yield drag such as rootworm, cutworm, European Corn Borer, and the like. Disease resistance and insect resistance genes such as lysozymes or cecropins for antibacterial protection, or proteins such as defensins, glucanases or chitinases for antifungal protection, or Bacillus thuringiensis endotoxins, protease inhibitors, collagenases, lectins, or glycosidases for controlling nematodes or insects are all examples of useful gene products. Genes encoding disease resistance traits include detoxification genes, such as against fumonisin (U.S. Patent No. 5,792,931); avirulence (avr) and disease resistance (R) genes (Jones et al. (1994) Science 266:789; Martin et al. (1993) Science 262:1432; and Mindrinos et al. (1994) Cell 78:1089); and the like. Insect resistance genes may encode resistance to pests that have great yield drag such as rootworm, cutworm, European Corn Borer, and the like. Such genes include, for example, *Bacillus thuringiensis* toxic protein genes (U.S. Patent Nos. 5,366,892; 5,747,450; 5,736,514; 5,723,756; 5,593,881; and Geiser et al. (1986) Gene 48:109); and the like.

An "herbicide resistance protein" or a protein resulting from expression of an "herbicide resistance-encoding nucleic acid molecule" includes proteins that confer upon a cell the ability to tolerate a higher concentration of an herbicide than cells that do not express the protein, or to tolerate a certain concentration of an herbicide for a longer period of time than cells that do not express the protein. Herbicide resistance traits may be introduced into plants by genes coding for resistance to herbicides that act to inhibit the action of acetolactate synthase (ALS), in particular the sulfonylurea-type herbicides, genes coding for resistance to herbicides that act to inhibit the action of glutamine synthase, such as phosphinothricin or basta (e.g., the bar gene), glyphosate (e.g., the EPSP synthase gene and the GAT gene), HPPD inhibitors (e.g, the HPPD gene) or other such genes known in the art. See, for example, US Patent Nos. 7,626,077, 5,310,667, 5,866,775, 6,225,114, 6,248,876, 7,169,970, 6,867,293, and US Provisional Application No. 61/401,456, each of which is herein incorporated by reference. The *bar* gene encodes resistance to the herbicide basta, the *nptll* gene encodes resistance to the antibiotics kanamycin and geneticin, and the ALS-gene mutants encode resistance to the herbicide chlorsulfuron.

Furthermore, it is recognized that the polynucleotide of interest may also comprise antisense sequences complementary to at least a portion of the messenger RNA (mRNA) for a targeted gene sequence of interest. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, 80%, or 85% sequence identity to the corresponding antisense sequences may be used. Furthermore, portions of the antisense nucleotides may be used to disrupt the expression of the target gene. Generally, sequences of at least 50 nucleotides, 100 nucleotides, 200 nucleotides, or greater may be used.

In addition, the polynucleotide of interest may also be used in the sense orientation to suppress the expression of endogenous genes in plants. Methods for suppressing gene expression in plants using polynucleotides in the sense orientation are known in the art. The methods generally involve transforming plants with a DNA construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, generally greater than about 65% sequence identity, about 85% sequence identity, or greater than about 95% sequence identity. See, U.S. Patent Nos. 5,283,184 and 5,034,323; herein incorporated by reference.

The polynucleotide of interest can also be a phenotypic marker. A phenotypic marker is screenable or a selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, inorganic and organic compounds or compositions and the like.

Examples of selectable markers include, but are not limited to, DNA segments that comprise restriction enzyme sites; DNA segments that encode products which provide resistance against otherwise toxic compounds including antibiotics, such as, spectinomycin, ampicillin, kanamycin, tetracycline, Basta, neomycin phosphotransferase II (NEO) and hygromycin phosphotransferase (HPT)); DNA segments that encode products which are otherwise lacking in the recipient cell (e.g., tRNA genes, auxotrophic markers); DNA segments that encode products which can be readily identified (e.g., phenotypic markers such as β-galactosidase, GUS; fluorescent proteins such as green fluorescent protein (GFP), cyan (CFP), yellow (YFP), red (RFP), and cell surface proteins); the generation of new primer sites for PCR (e.g., the juxtaposition of two DNA sequence not previously juxtaposed), the inclusion of DNA sequences not acted upon or acted upon by a restriction endonuclease or other DNA modifying enzyme, chemical, etc.; and, the inclusion of a DNA sequences required for a specific modification (e.g., methylation) that allows its identification.

Additional selectable markers include genes that confer resistance to herbicidal compounds, such as glufosinate ammonium, bromoxynil, imidazolinones, and 2,4-dichlorophenoxyacetate (2,4-D). Commercial traits can also be encoded on a gene or genes that could increase for example, starch for ethanol production, or provide expression of proteins. Exogenous products include plant enzymes and products as well as those from other sources including prokaryotes and other eukaryotes. Such products include enzymes, cofactors, hormones, and the like. The level of proteins, particularly modified proteins having improved amino acid distribution to improve the nutrient value of the plant, can be increased. This is achieved by the expression of such proteins having enhanced amino acid content.

The transgenes, recombinant DNA molecules, DNA sequences of interest, and polynucleotides of interest can be comprise one or more DNA sequences for gene silencing. Methods for gene silencing involving the expression of DNA sequences in plant are known in the art include, but are not limited to, cosuppression, antisense suppression, double-stranded RNA (dsRNA) interference, hairpin RNA (hpRNA) interference, intron-containing hairpin RNA (ihpRNA) interference, transcriptional gene silencing, and micro RNA (miRNA) interference

As used herein, "nucleic acid" means a polynucleotide and includes a single or a double-stranded polymer of deoxyribonucleotide or ribonucleotide bases. Nucleic acids may also include fragments and modified nucleotides. Thus, the terms "polynucleotide", "nucleic acid sequence", "nucleotide sequence" and "nucleic acid fragment" are used interchangeably to denote a polymer of RNA and/or DNA that is single- or double-stranded, optionally containing synthetic, non-natural, or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

"Open reading frame" is abbreviated ORF.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of genes to produce the desired phenotype in a transformed plant. Genes can be designed for use in suppression by linking a nucleic acid fragment or subfragment thereof, whether or not it encodes an active enzyme, in the sense or antisense orientation relative to a plant promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential to the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

Polynucleotide and polypeptide sequences, variants thereof, and the structural relationships of these sequences can be described by the terms "homology", "homologous", "substantially identical", "substantially similar" and "corresponding substantially" which are used interchangeably herein. These refer to polypeptide or nucleic acid fragments wherein changes in one or more amino acids or nucleotide bases do not affect the function of the molecule, such as the ability to mediate gene expression or to produce a certain phenotype. These terms also refer to modification(s) of nucleic acid fragments that do not substantially alter the functional properties of the resulting nucleic acid fragment relative to the initial, unmodified fragment. These modifications include deletion, substitution, and/or insertion of one or more nucleotides in the nucleic acid fragment.

Substantially similar nucleic acid sequences encompassed may be defined by their ability to hybridize (under moderately stringent conditions, e.g., 0.5X SSC, 0.1% SDS, 60°C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence in an *in vitro* hybridization assay. Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salt(s)) at pH 7.0 to 8.3, and at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1 X SSC at 60 to 65°C.

"Sequence identity" or "identity" in the context of nucleic acid or polypeptide sequences refers to the nucleic acid bases or amino acid residues in two sequences that are the same when aligned for maximum correspondence over a specified comparison window.

The term "percentage of sequence identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90% or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign™ program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

The "Clustal V method of alignment" corresponds to the alignment method labeled Clustal V (described by Higgins and Sharp, (1989) CABIOS 5:151-153; Higgins et al., (1992) Comput Appl Biosci 8:189-191) and found in the MegAlign™ program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

The "Clustal W method of alignment" corresponds to the alignment method labeled Clustal W (described by Higgins and Sharp, (1989) CABIOS 5:151-153; Higgins et al., (1992) Comput Appl Biosci 8:189-191) and found in the MegAlign™ v6.1 program of the LASERGENE bioinformatics computing suite (DNASTAR Inc., Madison, WI). Default parameters for multiple alignment (GAP PENALTY=10, GAP LENGTH PENALTY=0.2, Delay Divergen Seqs (%)=30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

Unless otherwise stated, sequence identity/similarity values provided herein refer to the value obtained using GAP Version 10 (GCG, Accelrys, San Diego, CA) using the following parameters: % identity and % similarity for a nucleotide sequence using a gap creation penalty weight of 50 and a gap length extension penalty weight of 3, and the nwsgapdna.cmp scoring matrix; % identity and % similarity for an amino acid sequence using a GAP creation penalty weight of 8 and a gap length extension penalty of 2, and the BLOSUM62 scoring matrix (Henikoff and Henikoff, (1989) Proc. Natl. Acad. Sci. USA 89:10915). GAP uses the algorithm of Needleman and Wunsch, (1970) J Mol Biol 48:443-53, to find an alignment of two complete sequences that maximizes the number of matches and minimizes the number of gaps. GAP considers all possible alignments and gap positions and creates the alignment with the largest number of matched bases and the fewest gaps, using a gap creation penalty and a gap extension penalty in units of matched bases.

"BLAST" is a searching algorithm provided by the National Center for Biotechnology Information (NCBI) used to find regions of similarity between biological sequences. The program compares nucleotide or protein sequences to sequence databases and calculates the statistical significance of matches to identify sequences having sufficient similarity to a query sequence such that the similarity would not be predicted to have occurred randomly. BLAST reports the identified sequences and their local alignment to the query sequence.

It is well understood by one skilled in the art that many levels of sequence identity are useful in identifying polypeptides from other species or modified naturally or synthetically wherein such polypeptides have the same or similar function or activity. Useful examples of percent identities include, but are not limited to, 50%, 55%, 60%, 65%, 70%, 75%, 80%, 85%, 90% or 95%, or any integer percentage from 50% to 100%. Indeed, any integer amino acid identity from 50% to 100% may be useful in describing the present disclosure, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

"Gene" includes a nucleic acid fragment that expresses a functional molecule such as, but not limited to, a specific protein, including regulatory sequences preceding (5' non-coding sequences) and following (3' non-coding sequences) the coding sequence. "Native gene" refers to a gene as found in nature with its own regulatory sequences.

A "mutated gene" is a gene that has been altered through human intervention. Such a "mutated gene" has a sequence that differs from the sequence of the corresponding non-mutated gene by at least one nucleotide addition, deletion, or substitution. In certain embodiments of the disclosure, the mutated gene comprises an alteration that results from a guide polynucleotide/Cas9 endonuclease system as disclosed herein. A mutated plant is a plant comprising a mutated gene.

As used herein, a "targeted mutation" is a mutation in a gene, such as a native gene, that was made by altering a target sequence within that gene using a method involving a double-strand-break-inducing agent that is capable of inducing a double-strand break in the DNA of the target sequence as disclosed herein or known in the art.

The guide RNA/Cas9 endonuclease induced targeted mutation can occur in a nucleotide sequence that is located within or outside a genomic target site that is recognized and cleaved by a Cas9 endonuclease.

The term "genome" as it applies to a plant cells encompasses not only chromosomal DNA found within the nucleus, but organelle DNA found within subcellular components (e.g., mitochondria, or plastid) of the cell.

A "codon-modified gene" or "codon-preferred gene" or "codon-optimized gene" is a gene having its frequency of codon usage designed to mimic the frequency of preferred codon usage of the host cell.

An "allele" is one of several alternative forms of a gene occupying a given locus on a chromosome. When all the alleles present at a given locus on a chromosome are the same, that plant is homozygous at that locus. If the alleles present at a given locus on a chromosome differ, that plant is heterozygous at that locus.

"Coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, 5' untranslated sequences, 3' untranslated sequences, introns, polyadenylation target sequences, RNA processing sites, effector binding sites, and stem-loop structures.

Methods are available in the art for synthesizing plant-preferred genes. See, for example, U.S. Patent Nos. 5,380,831, and 5,436,391, and Murray et al. (1989) Nucleic Acids Res. 17:477-498, herein incorporated by reference. Additional sequence modifications are known to enhance gene expression in a plant host. These include, for example, elimination of: one or more sequences encoding spurious polyadenylation signals, one or more exon-intron splice site signals, one or more transposon-like repeats, and other such well-characterized sequences that may be deleterious to gene expression. The G-C content of the sequence may be adjusted to levels average for a given plant host, as calculated by reference to known genes expressed in the host plant cell. When possible, the sequence is modified to avoid one or more predicted hairpin secondary mRNA structures. Thus, "a plant-optimized nucleotide sequence" of the present disclosure comprises one or more of such sequence modifications.

A promoter is a region of DNA involved in recognition and binding of RNA polymerase and other proteins to initiate transcription. The promoter sequence consists of proximal and more distal upstream elements, the latter elements often referred to as enhancers. An "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, and/or comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters".

It has been shown that certain promoters are able to direct RNA synthesis at a higher rate than others. These are called "strong promoters". Certain other promoters have been shown to direct RNA synthesis at higher levels only in particular types of cells or tissues and are often referred to as "tissue specific promoters", or "tissue-preferred promoters" if the promoters direct RNA synthesis preferably in certain tissues but also in other tissues at reduced levels. Since patterns of expression of a chimeric gene (or genes) introduced into a plant are controlled using promoters, there is an ongoing interest in the isolation of novel promoters which are capable of controlling the expression of a chimeric gene or (genes) at certain levels in specific tissue types or at specific plant developmental stages.

A "chimeric gene" refers to any gene that is not a native gene, comprising regulatory and coding sequences that are not found together in nature (i.e., the regulatory and coding regions are heterologous with each other). Accordingly, a chimeric gene may comprise regulatory sequences and coding sequences that are derived from different sources, or regulatory sequences and coding sequences derived from the same source, but arranged in a manner different than that found in nature. A "foreign" or "heterologous" gene refers to a gene that is introduced into the host organism by gene transfer. Foreign genes can comprise native genes inserted into a non-native organism, native genes introduced into a new location within the native host, or chimeric genes. The polynucleotide sequences in certain embodiments disclosed herein are heterologous. A "transgene" is a gene that has been introduced into the genome by a transformation procedure. A "codon-optimized" open reading frame has its frequency of codon usage designed to mimic the frequency of preferred codon usage of the host cell. A plant promoter can include a promoter capable of initiating transcription in a plant cell, for a review of plant promoters, see, Potenza et al., (2004) In Vitro Cell Dev Biol 40:1-22. Constitutive promoters include, for example, the core promoter of the Rsyn7 promoter and other constitutive promoters disclosed in WO99/43838 and U.S. Patent No. 6,072,050; the core CaMV 35S promoter (Odell et al., (1985) Nature 313:810-2); rice actin (McElroy et al., (1990) Plant Cell 2:163-71); ubiquitin (Christensen et al., (1989) Plant MolBiol 12:619-32; Christensen et al., (1992) Plant Mol Biol 18:675-89); pEMU (Last et al., (1991) Theor Appl Genet 81:581-8); MAS (Velten et al., (1984) EMBO J 3:2723-30); ALS promoter (U.S. Patent No. 5,659,026), and the like. Other constitutive promoters are described in, for example, U.S. Patent Nos. 5,608,149; 5,608,144; 5,604,121; 5,569,597; 5,466,785; 5,399,680; 5,268,463; 5,608,142 and 6,177,611. In some examples an inducible promoter may be used. Pathogen-inducible promoters induced following infection by a pathogen include, but are not limited to those regulating expression of PR proteins, SAR proteins, beta-1,3-glucanase, chitinase, *etc.*

Chemical-regulated promoters can be used to modulate the expression of a gene in a plant through the application of an exogenous chemical regulator. The promoter may be a chemical-inducible promoter, where application of the chemical induces gene expression, or a chemical-repressible promoter, where application of the chemical represses gene expression. Chemical-inducible promoters include, but are not limited to, the maize In2-2 promoter, activated by benzene sulfonamide herbicide safeners (De Veylder et al., (1997) Plant Cell Physiol 38:568-77), the maize GST promoter (GST-II-27, WO93/01294), activated by hydrophobic electrophilic compounds used as pre-emergent herbicides, and the tobacco PR-1a promoter (Ono et al., (2004) Biosci Biotechnol Biochem 68:803-7) activated by salicylic acid. Other chemical-regulated promoters include steroid-responsive promoters (see, for example, the glucocorticoid-inducible promoter (Schena et al., (1991) Proc. Natl. Acad. Sci. USA 88:10421-5; McNellis et al., (1998) Plant J 14:247-257); tetracycline-inducible and tetracycline-repressible promoters (Gatz et al., (1991) Mol Gen Genet 227:229-37; U.S. Patent Nos. 5,814,618 and 5,789,156).

Tissue-preferred promoters can be utilized to target enhanced expression within a particular plant tissue. Tissue-preferred promoters include, for example, Kawamata et al., (1997) Plant Cell Physiol 38:792-803; Hansen et al., (1997) Mol Gen Genet 254:337-43; Russell et al., (1997) Transgenic Res 6:157-68; Rinehart et al., (1996) Plant Physiol 112:1331-41; Van Camp et al., (1996) Plant Physiol 112:525-35; Canevascini et al., (1996) Plant Physiol 112:513-524; Lam, (1994) Results Probl Cell Differ 20:181-96; and Guevara-Garcia et al., (1993) Plant J 4:495-505. Leaf-preferred promoters include, for example, Yamamoto et al., (1997) Plant J 12:255-65; Kwon et al., (1994) Plant Physiol 105:357-67; Yamamoto et al., (1994) Plant Cell Physiol 35:773-8; Gotor et a/., (1993) Plant J 3:509-18; Orozco et a/., (1993) Plant MolBiol 23:1129-38; Matsuoka et al., (1993) Proc. Natl. Acad. Sci. USA 90:9586-90; Simpson et al., (1958) EMBO J 4:2723-9; Timko et al., (1988) Nature 318:57-8. Root-preferred promoters include, for example, Hire et al., (1992) Plant MolBiol 20:207-18 (soybean root-specific glutamine synthase gene); Miao et al., (1991) Plant Cell 3:11-22 (cytosolic glutamine synthase (GS)); Keller and Baumgartner, (1991) Plant Cell 3:1051-61 (root-specific control element in the GRP 1.8 gene of French bean); Sanger et al., (1990) Plant MolBiol 14:433-43 (root-specific promoter of *A. tumefaciens* mannopine synthase (MAS)); Bogusz et al., (1990) Plant Cell 2:633-41 (root-specific promoters isolated from *Parasponia andersonii* and *Trema tomentosa*); Leach and Aoyagi, (1991) Plant Sci 79:69-76 (*A*. *rhizogenes* rolC and rolD root-inducing genes); Teeri et al., (1989) EMBO J 8:343-50 (*Agrobacterium* wound-induced TR1' and TR2' genes); VfENOD-GRP3 gene promoter (Kuster et al., (1995) Plant Mol Biol 29:759-72); and rolB promoter (Capana et al., (1994) Plant Mo/ Bio/ 25:681-91; phaseolin gene (Murai et al., (1983) Science 23:476-82; Sengopta-Gopalen et al., (1988) Proc. Natl. Acad. Sci. USA 82:3320-4). See also, U.S. Patent Nos. 5,837,876; 5,750,386; 5,633,363; 5,459,252; 5,401,836; 5,110,732 and 5,023,179.

Seed-preferred promoters include both seed-specific promoters active during seed development, as well as seed-germinating promoters active during seed germination. See, Thompson et al., (1989) BioEssays 10:108. Seed-preferred promoters include, but are not limited to, Cim1 (cytokinin-induced message); cZ19B1 (maize 19 kDa zein); and milps (myo-inositol-1-phosphate synthase); (WO00/11177; and U.S. Patent 6,225,529). For dicots, seed-preferred promoters include, but are not limited to, bean β-phaseoiin, napin, β-conglycinin, soybean lectin, cruciferin, and the like. For monocots, seed-preferred promoters include, but are not limited to, maize 15 kDa zein, 22 kDa zein, 27 kDa gamma zein, waxy, shrunken 1, shrunken 2, globulin 1, oleosin, and nuc1. See also, WO00/12733, where seed-preferred promoters from *END1* and *END2* genes are disclosed.

The term "inducible promoter" refers to promoters that selectively express a coding sequence or functional RNA in response to the presence of an endogenous or exogenous stimulus, for example by chemical compounds (chemical inducers) or in response to environmental, hormonal, chemical, and/or developmental signals. Inducible or regulated promoters include, for example, promoters induced or regulated by light, heat, stress, flooding or drought, salt stress, osmotic stress, phytohormones, wounding, or chemicals such as ethanol, abscisic acid (ABA), jasmonate, salicylic acid, or safeners.

An example of a stress-inducible is RD29A promoter (Kasuga et al. (1999) Nature Biotechnol. 17:287-91). One of ordinary skill in the art is familiar with protocols for simulating drought conditions and for evaluating drought tolerance of plants that have been subjected to simulated or naturally-occurring drought conditions. For example, one can simulate drought conditions by giving plants less water than normally required or no water over a period of time, and one can evaluate drought tolerance by looking for differences in physiological and/or physical condition, including (but not limited to) vigor, growth, size, or root length, or in particular, leaf color or leaf area size. Other techniques for evaluating drought tolerance include measuring chlorophyll fluorescence, photosynthetic rates and gas exchange rates. Also, one of ordinary skill in the art is familiar with protocols for simulating stress conditions such as osmotic stress, salt stress and temperature stress and for evaluating stress tolerance of plants that have been subjected to simulated or naturally-occurring stress conditions.

Another example of an inducible promoter useful in plant cells has been described in US patent application, US 2013-0312137A1, published on November 21, 2013, incorporated by reference herein. US patent application US 2013-0312137A1 describes a ZmCAS1 promoter from a CBSU-Anther_Subtraction library (CAS1) gene encoding a mannitol dehydrogenase from maize, and functional fragments thereof. The ZmCAS1 promoter (also referred to as "CAS1 promoter", "mannitol dehydrogenase promoter " , "mdh promoter") can be induced by a chemical or stress treatment. The chemical can be a safener such as, but not limited to, N-(aminocarbonyl)-2-chlorobenzenesulfonamide (2-CBSU). The stress treatment can be a heat treatment such as, but not limited to, a heat shock treatment (see also US provisional patent application,62/120421, filed on February 25, 2015, and incorporated by reference herein.

New promoters of various types useful in plant cells are constantly being discovered; numerous examples may be found in the compilation by Okamuro and Goldberg, (1989) In The Biochemistry of Plants, Vol. 115, Stumpf and Conn, eds (New York, NY: Academic Press), pp. 1-82.

"Translation leader sequence" refers to a polynucleotide sequence located between the promoter sequence of a gene and the coding sequence. The translation leader sequence is present in the mRNA upstream of the translation start sequence. The translation leader sequence may affect processing of the primary transcript to mRNA, mRNA stability or translation efficiency. Examples of translation leader sequences have been described (e.g., Turner and Foster, (1995) Mol Biotechnol 3:225-236).

"3' non-coding sequences", "transcription terminator" or "termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et a/., (1989) Plant Cell 1:671-680.

"RNA transcript" refers to the product resulting from RNA polymerase-catalyzed transcription of a DNA sequence. When the RNA transcript is a perfect complimentary copy of the DNA sequence, it is referred to as the primary transcript or pre-mRNA. A RNA transcript is referred to as the mature RNA or mRNA when it is a RNA sequence derived from post-transcriptional processing of the primary transcript pre mRNA. "Messenger RNA" or "mRNA" refers to the RNA that is without introns and that can be translated into protein by the cell. "cDNA" refers to a DNA that is complementary to, and synthesized from, an mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks the expression of a target gene (see, e.g., U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., the coding sequence is under the transcriptional control of the promoter). Coding sequences can be operably linked to regulatory sequences in a sense or antisense orientation. In another example, the complementary RNA regions can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook et al., Molecular Cloning: A Laboratory Manual; Cold Spring Harbor Laboratory: Cold Spring Harbor, NY (1989). Transformation methods are well known to those skilled in the art and are described *infra.*

The term "recombinant" refers to an artificial combination of two otherwise separated segments of sequence, e.g., by chemical synthesis, or manipulation of isolated segments of nucleic acids by genetic engineering techniques.

The terms "plasmid", "vector" and "cassette" refer to an extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. "Expression cassette" refers to a specific vector containing a gene and having elements in addition to the gene that allow for expression of that gene in a host.

The terms "recombinant DNA molecule", "recombinant construct", "expression construct", " construct", "construct", and "recombinant DNA construct" are used interchangeably herein. A recombinant construct comprises an artificial combination of nucleic acid fragments, e.g., regulatory and coding sequences that are not all found together in nature. For example, a construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "expression", as used herein, refers to the production of a functional end-product (e.g., an mRNA, guide RNA, or a protein) in either precursor or mature form.

The term "introducing" includes providing a nucleic acid (e.g., expression construct) or protein into a cell. "Introducing" is intended to mean presenting to the organism, such as a cell or organism, the polynucleotide or polypeptide or polynucleotide-protein complex (also referred to as ribonucleotide protein complex or RNP), in such a manner that the component(s) gains access to the interior of a cell of the organism or to the cell itself. The methods and compositions do not depend on a particular method for introducing a sequence into an organism or cell, only that the polynucleotide or polypeptide gains access to the interior of at least one cell of the organism. Introducing includes reference to the incorporation of a nucleic acid into a eukaryotic or prokaryotic cell where the nucleic acid may be incorporated into the genome of the cell, and includes reference to the transient (direct) provision of a nucleic acid, protein or polynucleotide-protein complex (PGEN, RGEN) to the cell.

Introduced includes reference to stable or transient transformation methods, as well as sexually crossing. Thus, "introducing" in the context of inserting a nucleic acid fragment (e.g., a recombinant DNA construct/expression construct) into a cell, includes "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid, or mitochondrial DNA), converted into an autonomous replicon, or transiently expressed (e.g., transfected mRNA).

"Mature" protein refers to a post-translationally processed polypeptide (i.e., one from which any pre- or propeptides present in the primary translation product have been removed). "Precursor" protein refers to the primary product of translation of mRNA (i.e., with pre- and propeptides still present). Pre- and propeptides may be but are not limited to intracellular localization signals.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or other DNA-containing organelle, of a host organism resulting in gene expression without integration or stable inheritance. Host organisms containing the transformed nucleic acid fragments are referred to as "transgenic" organisms.

The commercial development of genetically improved germplasm has also advanced to the stage of introducing multiple traits into crop plants, often referred to as a gene stacking approach. In this approach, multiple genes conferring different characteristics of interest can be introduced into a plant. Gene stacking can be accomplished by many means including but not limited to co-transformation, retransformation, and crossing lines with different genes of interest.

The term "plant" refers to whole plants, plant organs, plant tissues, seeds, plant cells, seeds and progeny of the same. Plant cells include, without limitation, cells from seeds, suspension cultures, embryos, meristematic regions, callus tissue, leaves, roots, shoots, gametophytes, sporophytes, pollen and microspores. Plant parts include differentiated and undifferentiated tissues including, but not limited to roots, stems, shoots, leaves, pollens, seeds, tumor tissue and various forms of cells and culture (e.g., single cells, protoplasts, embryos, and callus tissue). The plant tissue may be in plant or in a plant organ, tissue or cell culture. The term "plant organ" refers to plant tissue or a group of tissues that constitute a morphologically and functionally distinct part of a plant. The term "genome" refers to the entire complement of genetic material (genes and non-coding sequences) that is present in each cell of an organism, or virus or organelle; and/or a complete set of chromosomes inherited as a (haploid) unit from one parent. "Progeny" comprises any subsequent generation of a plant.

A transgenic plant includes, for example, a plant which comprises within its genome a heterologous polynucleotide introduced by a transformation step. The heterologous polynucleotide can be stably integrated within the genome such that the polynucleotide is passed on to successive generations. The heterologous polynucleotide may be integrated into the genome alone or as part of a recombinant DNA construct. A transgenic plant can also comprise more than one heterologous polynucleotide within its genome. Each heterologous polynucleotide may confer a different trait to the transgenic plant. A heterologous polynucleotide can include a sequence that originates from a foreign species, or, if from the same species, can be substantially modified from its native form. Transgenic can include any cell, cell line, callus, tissue, plant part or plant, the genotype of which has been altered by the presence of heterologous nucleic acid including those transgenics initially so altered as well as those created by sexual crosses or asexual propagation from the initial transgenic. The alterations of the genome (chromosomal or extra-chromosomal) by conventional plant breeding methods, by the genome editing procedure described herein that does not result in an insertion of a foreign polynucleotide, or by naturally occurring events such as random cross-fertilization, non-recombinant viral infection, non-recombinant bacterial transformation, non-recombinant transposition, or spontaneous mutation are not intended to be regarded as transgenic.

In certain embodiments of the disclosure, a fertile plant is a plant that produces viable male and female gametes and is self-fertile. Such a self-fertile plant can produce a progeny plant without the contribution from any other plant of a gamete and the genetic material contained therein. Other embodiments of the disclosure can involve the use of a plant that is not self-fertile because the plant does not produce male gametes, or female gametes, or both, that are viable or otherwise capable of fertilization. As used herein, a "male sterile plant" is a plant that does not produce male gametes that are viable or otherwise capable of fertilization. As used herein, a "female sterile plant" is a plant that does not produce female gametes that are viable or otherwise capable of fertilization. It is recognized that male-sterile and female-sterile plants can be female-fertile and male- fertile, respectively. It is further recognized that a male fertile (but female sterile) plant can produce viable progeny when crossed with a female fertile plant and that a female fertile (but male sterile) plant can produce viable progeny when crossed with a male fertile plant.

The guided Cas9 endonuclease systems of the present disclosure can be used in any prokaryotic or eukaryotic organism including non-conventional yeast and yeast or any fungal species that predominantly exist in unicellular form .

A "centimorgan" (cM) or "map unit" is the distance between two linked genes, markers, target sites, loci, or any pair thereof, wherein 1% of the products of meiosis are recombinant. Thus, a centimorgan is equivalent to a distance equal to a 1% average recombination frequency between the two linked genes, markers, target sites, loci, or any pair thereof.

The present disclosure finds use in the breeding of plants comprising one or more transgenic traits. Most commonly, transgenic traits are randomly inserted throughout the plant genome as a consequence of transformation systems based on *Agrobacterium,* biolistics, or other commonly used procedures. More recently, gene targeting protocols have been developed that enable directed transgene insertion. One important technology, site-specific integration (SSI) enables the targeting of a transgene to the same chromosomal location as a previously inserted transgene. Custom-designed meganucleases and custom-designed zinc finger meganucleases allow researchers to design nucleases to target specific chromosomal locations, and these reagents allow the targeting of transgenes at the chromosomal site cleaved by these nucleases.

The currently used systems for precision genetic engineering of eukaryotic genomes, e.g. plant genomes, rely upon homing endonucleases, meganucleases, zinc finger nucleases, and transcription activator-like effector nucleases (TALENs), which require de novo protein engineering for every new target locus. The highly specific, RNA-directed DNA nuclease, guide RNA/ Cas9 endonuclease system described herein, is more easily customizable and therefore more useful when modification of many different target sequences is the goal. This disclosure takes further advantage of the two component nature of the guide RNA/ Cas9 system, with its constant protein component, the Cas9 endonuclease, and its variable and easily reprogrammable targeting component, the guide RNA or the crRNA.

The guide RNA/Cas9 system described herein is especially useful for genome engineering, especially plant genome engineering, in circumstances where nuclease off-target cutting can be toxic to the targeted cells. In one embodiment of the guide RNA/Cas9 system described herein, the constant component, in the form of an expression-optimized Cas9 gene, is stably integrated into the target genome, e.g. plant genome. Expression of the Cas9 gene is under control of a promoter, e.g. plant promoter, which can be a constitutive promoter, tissue-specific promoter or inducible promoter, e.g. temperature-inducible, stress-inducible, developmental stage inducible, or chemically inducible promoter. In the absence of the variable component, i.e. the guide RNA or crRNA, the Cas9 protein is not able to cut DNA and therefore its presence in the plant cell should have little or no consequence. Hence a key advantage of the guide RNA/Cas9 system described herein is the ability to create and maintain a cell line or transgenic organism capable of efficient expression of the Cas9 protein with little or no consequence to cell viability. In order to induce cutting at desired genomic sites to achieve targeted genetic modifications, guide RNAs or crRNAs can be introduced by a variety of methods into cells containing the stably-integrated and expressed cas9 gene. For example, guide RNAs or crRNAs can be chemically or enzymatically synthesized, and introduced into the Cas9 expressing cells via direct delivery methods such a particle bombardment or electroporation.

Alternatively, genes capable of efficiently expressing guide RNAs or crRNAs in the target cells can be synthesized chemically, enzymatically or in a biological system, and these genes can be introduced into the Cas9 expressing cells via direct delivery methods such a particle bombardment, electroporation or biological delivery methods such as Agrobacterium mediated DNA delivery.

A guide RNA/Cas9 system mediating gene targeting can be used in methods for directing transgene insertion and / or for producing complex transgenic trait loci comprising multiple transgenes in a fashion similar as disclosed in WO2013/0198888 (published August 1, 2013) where instead of using a double strand break inducing agent to introduce a gene of interest, a guide RNA/Cas9 system as disclosed herein is used. In one embodiment, a complex transgenic trait locus is a genomic locus that has multiple transgenes genetically linked to each other. By inserting independent transgenes within 0.1, 0.2, 0.3, 0.4, 0.5 , 1.0, 2, or even 5 centimorgans (cM) from each other, the transgenes can be bred as a single genetic locus (see, for example, U.S. patent application 13/427,138) or PCT application PCT/US2012/030061. After selecting a plant comprising a transgene, plants containing (at least) one transgenes can be crossed to form an F1 that contains both transgenes. In progeny from these F1 (F2 or BC1) 1/500 progeny would have the two different transgenes recombined onto the same chromosome. The complex locus can then be bred as single genetic locus with both transgene traits. This process can be repeated to stack as many traits as desired.

Chromosomal intervals that correlate with a phenotype or trait of interest can be identified. A variety of methods well known in the art are available for identifying chromosomal intervals. The boundaries of such chromosomal intervals are drawn to encompass markers that will be linked to the gene controlling the trait of interest. In other words, the chromosomal interval is drawn such that any marker that lies within that interval (including the terminal markers that define the boundaries of the interval) can be used as a marker for northern leaf blight resistance. In one embodiment, the chromosomal interval comprises at least one QTL, and furthermore, may indeed comprise more than one QTL. Close proximity of multiple QTLs in the same interval may obfuscate the correlation of a particular marker with a particular QTL, as one marker may demonstrate linkage to more than one QTL. Conversely, e.g., if two markers in close proximity show co-segregation with the desired phenotypic trait, it is sometimes unclear if each of those markers identifies the same QTL or two different QTL. The term "quantitative trait locus" or "QTL" refers to a region of DNA that is associated with the differential expression of a quantitative phenotypic trait in at least one genetic background, e.g., in at least one breeding population. The region of the QTL encompasses or is closely linked to the gene or genes that affect the trait in question. An "allele of a QTL" can comprise multiple genes or other genetic factors within a contiguous genomic region or linkage group, such as a haplotype. An allele of a QTL can denote a haplotype within a specified window wherein said window is a contiguous genomic region that can be defined, and tracked, with a set of one or more polymorphic markers. A haplotype can be defined by the unique fingerprint of alleles at each marker within the specified window.

A variety of methods are available to identify those cells having an altered genome at or near a target site without using a screenable marker phenotype. Such methods can be viewed as directly analyzing a target sequence to detect any change in the target sequence, including but not limited to PCR methods, sequencing methods, nuclease digestion, Southern blots, and any combination thereof.

Proteins may be altered in various ways including amino acid substitutions, deletions, truncations, and insertions. Methods for such manipulations are generally known. For example, amino acid sequence variants of the protein(s) can be prepared by mutations in the DNA. Methods for mutagenesis and nucleotide sequence alterations include, for example, Kunkel, (1985) Proc. Natl. Acad. Sci. USA 82:488-92; Kunkel et al., (1987) Meth Enzymol 154:367-82; U.S. Patent No. 4,873,192; Walker and Gaastra, eds. (1983) Techniques in Molecular Biology (MacMillan Publishing Company, New York) and the references cited therein. Guidance regarding amino acid substitutions not likely to affect biological activity of the protein is found, for example, in the model of Dayhoff et al., (1978) Atlas of Protein Sequence and Structure (Natl Biomed Res Found, Washington, D.C.). Conservative substitutions, such as exchanging one amino acid with another having similar properties, may be preferable. Conservative deletions, insertions, and amino acid substitutions are not expected to produce radical changes in the characteristics of the protein, and the effect of any substitution, deletion, insertion, or combination thereof can be evaluated by routine screening assays. Assays for double-strand-break-inducing activity are known and generally measure the overall activity and specificity of the agent on DNA substrates containing target sites.

A variety of methods are known for the introduction of nucleotide sequences and polypeptides into an organism, including, for example, transformation, sexual crossing, and the introduction of the polypeptide, DNA, or mRNA into the cell.

Methods for contacting, providing, and/or introducing a composition into various organisms are known and include but are not limited to, stable transformation methods, transient transformation methods, virus-mediated methods, and sexual breeding. Stable transformation indicates that the introduced polynucleotide integrates into the genome of the organism and is capable of being inherited by progeny thereof. Transient transformation indicates that the introduced composition is only temporarily expressed or present in the organism.

Protocols for introducing polynucleotides and polypeptides into plants may vary depending on the type of plant or plant cell targeted for transformation, such as monocot or dicot. Protocols for introducing polynucleotides, polypeptides or polynucleotide-protein complexes (PGEN, RGEN) into eukaryotic cells, such as plants or plant cells are known and include microinjection (Crossway et al., (1986) Biotechniques 4:320-34 and U.S. Patent No. 6,300,543), meristem transformation (U.S. Patent No. 5,736,369), electroporation (Riggs et al., (1986) Proc. Natl. Acad. Sci. USA 83:5602-6, *Agrobacterium*-mediated transformation (U.S. Patent Nos. 5,563,055 and 5,981,840), direct gene transfer (Paszkowski et al., (1984) EMBO J 3:2717-22), and ballistic particle acceleration (U.S. Patent Nos. 4,945,050; 5,879,918; 5,886,244; 5,932,782; Tomes et al., (1995) "Direct DNA Transfer into Intact Plant Cells via Microprojectile Bombardment" in Plant Cell, Tissue, and Organ Culture: Fundamental Methods, ed. Gamborg & Phillips (Springer-Verlag, Berlin); McCabe et al., (1988) Biotechnology 6:923-6; Weissinger et al., (1988) Ann Rev Genet 22:421-77; Sanford et al., (1987) Particulate Science and Technology 5:27-37 (onion); Christou et al., (1988) Plant Physiol 87:671-4 (soybean); Finer and McMullen, (1991) In Vitro Cell Dev Biol 27P: 175-82 (soybean); Singh et al., (1998) Theor Appl Genet 96:319-24 (soybean); Datta et al., (1990) Biotechnology 8:736-40 (rice); Klein et al., (1988) Proc. Natl. Acad. Sci. USA 85:4305-9 (maize); Klein et al., (1988) Biotechnology 6:559-63 (maize); U.S. Patent Nos. 5,240,855; 5,322,783 and 5,324,646; Klein et al., (1988) Plant Physiol 91:440-4 (maize); Fromm et al., (1990) Biotechnology 8:833-9 (maize); Hooykaas-Van Slogteren et al., (1984) Nature 311:763-4; U.S. Patent No. 5,736,369 (cereals); Bytebier et al., (1987) Proc. Natl. Acad. Sci. USA 84:5345-9 (Liliaceae); De Wet et al., (1985) in The Experimental Manipulation of Ovule Tissues, ed. Chapman et al., (Longman, New York), pp. 197-209 (pollen); Kaeppler et al., (1990) Plant Cell Rep 9:415-8) and Kaeppler et al., (1992) Theor Appl Genet 84:560-6 (whisker-mediated transformation); D'Halluin et al., (1992) Plant Cell 4:1495-505 (electroporation); Li et al., (1993) Plant Cell Rep 12:250-5; Christou and Ford (1995) Annals Botany 75:407-13 (rice) and Osjoda et al., (1996) Nat Biotechnol 14:745-50 (maize via Agrobacterium tumefaciens).

Alternatively, polynucleotides may be introduced into plants by contacting plants with a virus or viral nucleic acids. Generally, such methods involve incorporating a polynucleotide within a viral DNA or RNA molecule. In some examples a polypeptide of interest may be initially synthesized as part of a viral polyprotein, which is later processed by proteolysis *in vivo* or *in vitro* to produce the desired recombinant protein. Methods for introducing polynucleotides into plants and expressing a protein encoded therein, involving viral DNA or RNA molecules, are known, see, for example, U.S. Patent Nos. 5,889,191, 5,889,190, 5,866,785, 5,589,367 and 5,316,931. Transient transformation methods include, but are not limited to, the introduction of polypeptides, such as a double-strand break inducing agent, directly into the organism, the introduction of polynucleotides such as DNA and/or RNA polynucleotides, and the introduction of the RNA transcript, such as an mRNA encoding a double-strand break inducing agent, into the organism. Such methods include, for example, microinjection or particle bombardment. See, for example Crossway et al., (1986) Mol Gen Genet 202:179-85; Nomura et al., (1986) Plant Sci 44:53-8; Hepler et al., (1994) Proc. Natl. Acad. Sci. USA 91:2176-80; and, Hush et al., (1994) J Cell Sci 107:775-84.

The term "dicot" refers to the subclass of angiosperm plants also knows as "dicotyledoneae" and includes reference to whole plants, plant organs (e.g., leaves, stems, roots, etc.), seeds, plant cells, and progeny of the same. Plant cell, as used herein includes, without limitation, seeds, suspension cultures, embryos, meristematic regions, callus tissue, leaves, roots, shoots, gametophytes, sporophytes, pollen, and microspores.

The term "crossed" or "cross" or "crossing" in the context of this disclosure means the fusion of gametes via pollination to produce progeny (i.e., cells, seeds, or plants). The term encompasses both sexual crosses (the pollination of one plant by another) and selfing (self-pollination, i.e., when the pollen and ovule (or microspores and megaspores) are from the same plant or genetically identical plants).

The term "introgression" refers to the transmission of a desired allele of a genetic locus from one genetic background to another. For example, introgression of a desired allele at a specified locus can be transmitted to at least one progeny plant via a sexual cross between two parent plants, where at least one of the parent plants has the desired allele within its genome. Alternatively, for example, transmission of an allele can occur by recombination between two donor genomes, e.g., in a fused protoplast, where at least one of the donor protoplasts has the desired allele in its genome. The desired allele can be, e.g., a transgene, a modified (mutated or edited) native allele, or a selected allele of a marker or QTL.

Standard DNA isolation, purification, molecular cloning, vector construction, and verification/characterization methods are well established, see, for example Sambrook et al., (1989) Molecular Cloning: A Laboratory Manual, (Cold Spring Harbor Laboratory Press, NY). Vectors and constructs include circular plasmids, and linear polynucleotides, comprising a polynucleotide of interest and optionally other components including linkers, adapters, regulatory or analysis. In some examples a recognition site and/or target site can be contained within an intron, coding sequence, 5' UTRs, 3' UTRs, and/or regulatory regions.

The present disclosure further provides expression constructs for expressing in a plant, plant cell, or plant part a guide RNA/Cas9 system that is capable of binding to and creating a double strand break in a target site. In one embodiment, the expression constructs of the disclosure comprise a promoter operably linked to a nucleotide sequence encoding a Cas9 gene and a promoter operably linked to a guide RNA of the present disclosure. The promoter is capable of driving expression of an operably linked nucleotide sequence in a plant cell.

A phenotypic marker is a screenable or selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, inorganic and organic compounds or compositions and the like.

Examples of selectable markers include, but are not limited to, DNA segments that comprise restriction enzyme sites; DNA segments that encode products which provide resistance against otherwise toxic compounds including antibiotics, such as, spectinomycin, ampicillin, kanamycin, tetracycline, Basta, neomycin phosphotransferase II (NEO) and hygromycin phosphotransferase (HPT)); DNA segments that encode products which are otherwise lacking in the recipient cell (e.g., tRNA genes, auxotrophic markers); DNA segments that encode products which can be readily identified (e.g., phenotypic markers such as β-galactosidase, GUS; fluorescent proteins such as green fluorescent protein (GFP), cyan (CFP), yellow (YFP), red (RFP), and cell surface proteins); the generation of new primer sites for PCR (e.g., the juxtaposition of two DNA sequence not previously juxtaposed), the inclusion of DNA sequences not acted upon or acted upon by a restriction endonuclease or other DNA modifying enzyme, chemical, etc.; and, the inclusion of a DNA sequences required for a specific modification (e.g., methylation) that allows its identification.

Additional selectable markers include genes that confer resistance to herbicidal compounds, such as glufosinate ammonium, bromoxynil, imidazolinones, and 2,4-dichlorophenoxyacetate (2,4-D). See for example, Yarranton, (1992) Curr Opin Biotech 3:506-11)

The cells having the introduced sequence may be grown or regenerated into plants using conventional conditions, see for example, McCormick et al., (1986) Plant Cell Rep 5:81-4. These plants may then be grown, and either pollinated with the same transformed strain or with a different transformed or untransformed strain, and the resulting progeny having the desired characteristic and/or comprising the introduced polynucleotide or polypeptide identified. Two or more generations may be grown to ensure that the polynucleotide is stably maintained and inherited, and seeds harvested.

Any plant can be used, including monocot and dicot plants. Examples of monocot plants that can be used include, but are not limited to, corn (*Zea mays*), rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*)), wheat (*Triticum aestivum*), sugarcane (*Saccharum spp*.), oats (*Avena*), barley (*Hordeum*), switchgrass (*Panicum virgatum*), pineapple (*Ananas comosus*), banana (*Musa spp.*), palm, ornamentals, turfgrasses, and other grasses. Examples of dicot plants that can be used include, but are not limited to, soybean (*Glycine max*), canola (*Brassica napus* and *B*. *campestris*), alfalfa (*Medicago sativa*), tobacco (*Nicotiana tabacum*), *Arabidopsis* (*Arabidopsis thaliana*), sunflower (*Helianthus annuus*), cotton (*Gossypium arboreum*), and peanut (*Arachis hypogaea*), tomato (*Solanum lycopersicum*), potato (*Solanum tuberosum*) *etc.*

The transgenes, recombinant DNA molecules, DNA sequences of interest, and polynucleotides of interest can comprise one or more genes of interest. Such genes of interest can encode, for example, a protein that provides agronomic advantage to the plant.

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kb" means kilobase(s).

Non-limiting examples of compositions and methods disclosed herein are as follows:
1. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA is selected from the group consisting of SEQ ID NOs: 185-207, a functional fragment of SEQ ID NOs: 185-207, and a functional variant of SEQ ID NOs: 185-207.
2. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of SEQ ID NOs: 139-184, a functional fragment of SEQ ID NOs: 139-184, and a functional variant of SEQ ID NOs: 139-184.
3. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of SEQ ID NOs: 116-138, a functional fragment of SEQ ID NOs: 116-138, and a functional variant of SEQ ID NOs: 116-138.
4. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a variable targeting domain capable of hybridizing to said target sequence, wherein said tracrRNA comprises a nucleotide sequence selected from the group consisting of SEQ ID NOs: 139-184, a functional fragment of SEQ ID NOs: 139-184, and a functional variant of SEQ ID NOs: 139-184, wherein said chimeric non-naturally occurring crRNA comprises a variable targeting domain capable of hybridizing to said target sequence.
5. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a nucleotide sequence selected from the group consisting of SEQ ID NOs: 116-138, a functional fragment of SEQ ID NOs: 116-138, and a functional variant of SEQ ID NOs: 116-138, wherein said chimeric non-naturally occurring crRNA comprises a variable targeting domain capable of hybridizing to said target sequence.
6. A guide RNA/Cas9 endonuclease complex comprising a Cas9 endonuclease selected from the group consisting of SEQ ID NOs: 47-69, a functional fragment of SEQ ID NOs: 47-69, and a functional variant of SEQ ID NOs: 47-69, and at least one guide RNA, wherein said guide RNA/Cas9 endonuclease complex is capable of recognizing, binding to, and optionally nicking or cleaving all or part of a target sequence.
7. A guide RNA/Cas9 endonuclease complex comprising at least one guide RNA and a Cas9 endonuclease, wherein said Cas9 endonuclease is encoded by a DNA sequence selected from the group consisting of SEQ ID NOs: 24-46, a functional fragment of SEQ ID NOs: 24-46, and a functional variant of SEQ ID NOs: 24-46, wherein said guide RNA/Cas9 endonuclease complex is capable of recognizing, binding to, and optionally nicking or cleaving all or part of a target sequence.
8. The guide RNA/Cas9 endonuclease complex of any of embodiments 6-7 comprising at least one guide RNA of any one of embodiments 1-5.
9. The guide RNA/Cas9 endonuclease complex of any of embodiments 6-7, wherein said target sequence is located in the genome of a cell.
10. A method for modifying a target site in the genome of a cell, the method comprising introducing into said cell at least one guide RNA and at least one Cas9 endonuclease selected from the group consisting of SEQ ID NOs: 47-69, a functional fragment of SEQ ID NOs: 47-69, and a functional variant of SEQ ID NOs: 47-69 , wherein said guide RNA and Cas9 endonuclease can form a complex that is capable of recognizing, binding to, and optionally nicking or cleaving all or part of said target site.
11. The method of embodiment 10, further comprising identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii).
12. A method for editing a nucleotide sequence in the genome of a cell, the method comprising introducing into said cell a polynucleotide modification template, at least one guide RNA and at least one Cas9 endonuclease selected from the group consisting of SEQ ID NOs: 47-69, a functional fragment of SEQ ID NOs: 47-69, and a functional variant of SEQ ID NOs: 47-69, wherein said polynucleotide modification template comprises at least one nucleotide modification of said nucleotide sequence, wherein said guide RNA and Cas9 endonuclease can form a complex that is capable of recognizing, binding to, and optionally nicking or cleaving all or part of said target site.
13. A method for modifying a target site in the genome of a cell, the method comprising introducing into said cell at least one guide RNA, at least one donor DNA, and at least one Cas9 endonuclease selected from the group consisting of SEQ ID NOs: 47-69, a functional fragment of SEQ ID NOs: 47-69, and a functional variant of SEQ ID NOs: 47-69, wherein said at least one guide RNA and at least one Cas9 endonuclease can form a complex that is capable of recognizing, binding to, and optionally nicking or cleaving all or part of said target site, wherein said donor DNA comprises a polynucleotide of interest.
14. The method of embodiment 13, further comprising identifying at least one cell that has said polynucleotide of interest integrated in or near said target site.
15. The method of any one of embodiments 10-14, wherein the cell is selected from the group consisting of a human, non-human, animal, bacterial, fungal, insect,
   yeast, non-conventional yeast, and plant cell.
16. The method of embodiment 15, wherein the plant cell is selected from the group consisting of a monocot and dicot cell.
17. The method of embodiment 16, wherein the plant cell is selected from the group consisting of maize, rice, sorghum, rye, barley, wheat, millet, oats, sugarcane, turfgrass, or switchgrass, soybean, canola, alfalfa, sunflower, cotton, tobacco, peanut, potato, tobacco, Arabidopsis, and safflower cell.
18. A plant comprising a modified target site, wherein said plant originates from a plant cell comprising a modified target site produced by the method of any of embodiments 10-17.
19. A plant comprising an edited nucleotide, wherein said plant originates from a plant cell comprising an edited nucleotide produced by the method of embodiment 12.

### EXAMPLES

In the following Examples, unless otherwise stated, parts and percentages are by weight and degrees are Celsius. It should be understood that these Examples, while indicating embodiments of the disclosure, are given by way of illustration only. From the above discussion and these Examples, one skilled in the art can make various changes and modifications of the disclosure to adapt it to various usages and conditions. Such modifications are also intended to fall within the scope of the appended claims.

### EXAMPLE 1

### Characterization of new Cas9 endonucleases and cognate guide RNAs

CRISPR-Cas loci from uncharacterized Type II CRISPR-Cas systems were identified by searching internal Pioneer-DuPont databases consisting of microbial genomes as described below. First, multiple sequence alignment of protein sequences from a diverse collection of Cas9 endonucleases was performed using MUSCLE (Edgar R. (2004) Nucleic Acids Res. 32(5): 1792-97). The alignments were examined and curated and were used to build profile hidden Markov models (HMM) for Cas9 sub-families using HMMER (Eddy S.R. (1998) Bioinformatics 14:755-763; Eddy S.R. (2011) PLoS Comp. Biol., 7:e1002195). The resulting HMM models were then utilized to search protein sequences translated from DNA sequence collections for the presence of cas9-like genes. The resulting genes were further validated as encoding a Cas9 protein by examining the translated amino acid sequence for the presence of HNH and RuvC cleavage domains. To further validate the gene as encoding a Cas9 protein, the other structural components of a Type II CRISPR-Cas system (Makarova et al. (2015) Nat. Rev. Microbiol. 13:722-736) (cas1 gene, cas2 gene, CRISPR array and tracrRNA encoding region) were identified in the DNA locus. Cas1 and cas2 genes were identified by examining the protein translations of open-reading-frames (ORFs) ≥ 201 nucleotides within the CRISPR-Cas locus against the NCBI protein database for those matching known Cas1 and Cas2 proteins using the PSI-BLAST program (Altschul, S. F. et al. (2005) FEBS J. 272:5101-5109.). The CRISPR array was detected using the PILER-CR program (Edgar R. (2007) BMC Bioinformatics 8:18. Additional CRISPR array repeats not detected by PILER-CR were identified by performing pairwise alignments of the locus with the PILER-CR identified repeats using the blastn program (Altschul, S. F. et al. (1997) Nucleic Acids Res. 25:3389-3402). The tracrRNA encoding region, termed the anti-repeat, was established by searching the locus for regions (distinct from the CRISPR array) with complete to partial homology to the repeats in the CRISPR array. In total, 23 DNA regions (Locus 6 to Locus 28, respectively) were selected (Table 2).

**Table 2. List of sequence for Type II CRISPR-Cas loci identified from Pioneer-Dupont databases.**

| CRISPR-Cas locus name | Genus/species of Origin | SEQ ID NO: |
|---|---|---|
| Locus 6 | Bacillus cereus | 1 |
| Locus 7 | Brevibacillus laterosporus | 2 |
| Locus 8 | Bacillus species | 3 |
| Locus 9 | Bacillus cereus | 4 |
| Locus 10 | Lactobacillus fermentum | 5 |
| Locus 11 | Enterococcus faecalis | 6 |
| Locus 12 | Bacillus cereus | 7 |
| Locus 13 | Enterococcus faecalis | 8 |
| Locus 14 | Unknown | 9 |
| Locus 15 | Enterococcus faecalis | 10 |
| Locus 16 | Metagenomic | 11 |
| Locus 17 | Chryseobacterium species | 12 |
| Locus 18 | Metagenomic | 13 |
| Locus 19 | Metagenomic | 14 |
| Locus 20 | Unknown | 15 |
| Locus 21 | Metagenomic | 16 |
| Locus 22 | Metagenomic | 17 |
| Locus 23 | Metagenomic | 18 |
| Locus 24 | Metagenomic | 19 |
| Locus 25 | Metagenomic | 20 |
| Locus 26 | Metagenomic | 21 |
| Locus 27 | Metagenomic | 22 |
| Locus 28 | Metagenomic | 23 |

A schematic of the DNA locus for each system is depicted in Figures 1-23. The cas9 gene open-reading-frame (cas9 gene ORF), accessory protein gene ORF (e.g. Cas1, Cas2, and when present Csn2), CRISPR array with CRISPR repeats, and anti-repeat (the genomic DNA region demonstrating partial homology to the CRISPR array repeat that indicates the location of the encoded tracrRNA) are indicated.

The genomic DNA sequence and length of each cas9 gene ORF and cas9 gene translation (not including the stop codon) are referenced in Table 3 for each system. Table 4 lists the consensus sequence of the CRISPR array repeats from the DNA locus of each system and the sequences of the anti-repeat for each system (as DNA sequence on the same strand as the cas9 gene ORF).

**Table 3. Sequence and length of the cas9 gene ORF and cas9 gene translation from each Type II CRISPR-Cas system identified as described herein.**

| Cas9 endonuclease name | *cas9* Gene ORF (SEQ ID NO:) | Length of cas9 Gene ORF (bp) | Translation of cas9 Gene ORF (not including the stop codon) (SEQ ID NO) | Length of cas9 Gene Translation (No. of Amino Acids) |
|---|---|---|---|---|
| Cas-Locus 6 | 24 | 3282 | 47 | 1093 |
| Cas-Locus 7 | 25 | 3279 | 48 | 1092 |
| Cas-Locus 8 | 26 | 3213 | 49 | 1070 |
| Cas-Locus 9 | 27 | 3246 | 50 | 1081 |
| Cas-Locus 10 | 28 | 4137 | 51 | 1378 |
| Cas-Locus 11 | 29 | 4014 | 52 | 1337 |
| Cas-Locus 12 | 30 | 4014 | 53 | 1337 |
| Cas-Locus 13 | 31 | 4014 | 54 | 1337 |
| Cas-Locus 14 | 32 | 3993 | 55 | 1330 |
| Cas-Locus 15 | 33 | 3453 | 56 | 1150 |
| Cas-Locus 16 | 34 | 4371 | 57 | 1456 |
| Cas-Locus 17 | 35 | 4389 | 58 | 1462 |
| Cas-Locus 18 | 36 | 4323 | 59 | 1440 |
| Cas-Locus 19 | 37 | 4323 | 60 | 1440 |
| Cas-Locus 20 | 38 | 4323 | 61 | 1440 |
| Cas-Locus 21 | 39 | 3702 | 62 | 1233 |
| Cas-Locus 22 | 40 | 3807 | 63 | 1268 |
| Cas-Locus 23 | 41 | 3795 | 64 | 1264 |
| Cas-Locus 24 | 42 | 4395 | 65 | 1464 |
| Cas-Locus 25 | 43 | 4377 | 66 | 1458 |
| Cas-Locus 26 | 44 | 3384 | 67 | 1127 |
| Cas-Locus 27 | 45 | 3327 | 68 | 1108 |
| Cas-Locus 28 | 46 | 3327 | 69 | 1108 |

**Table 4. CRISPR repeat consensus and anti-repeat (putative tracrRNA coding region) for diverse Type II CRISPR-Cas systems described herein.**

| CRISPR-Cas locus name | CRISPR repeat consensus (SEQ ID NO) | CRISPR repeat length | CRISPR Array Transcriptional Direction | Anti-Repeat Consensus (SEQ ID NO) | Anti-Repeat Direction |
|---|---|---|---|---|---|
| Locus 6 | 70 | 36 | Anti-sense | 93 | Sense |
| Locus 7 | 71 | 36 | Anti-sense | 94 | Sense |
| Locus 8 | 72 | 36 | Anti-sense | 95 | Sense |
| Locus 9 | 73 | 36 | Anti-sense | 96 | Sense |
| Locus 10 | 74 | 36 | Sense | 97 | Anti-sense |
| Locus 11 | 75 | 36 | Sense | 98 | Anti-sense |
| Locus 12 | 76 | 36 | Sense | 99 | Anti-sense |
| Locus 13 | 77 | 36 | Sense | 100 | Anti-sense |
| Locus 14 | 78 | 36 | Sense | 101 | Anti-sense |
| Locus 15 | 79 | 36 | Sense | 102 | Sense |
| Locus 16 | 80 | 47 | Anti-sense | 103 | Anti-sense |
| Locus 17 | 81 | 47 | Anti-sense | 104 | Anti-sense |
| Locus 18 | 82 | 47 | Anti-sense | 105 | Anti-sense |
| Locus 19 | 83 | 47 | Anti-sense | 106 | Anti-sense |
| Locus 20 | 84 | 47 | Anti-sense | 107 | Anti-sense |
| Locus 21 | 85 | 47 | Anti-sense | 108 | Anti-sense |
| Locus 22 | 86 | 47 | Anti-sense | 109 | Anti-sense |
| Locus 23 | 87 | 46 | Anti-sense | 110 | Anti-sense |
| Locus 24 | 88 | 36 | Sense | 111 | Anti-sense |
| Locus 25 | 89 | 36 | Sense | 112 | Anti-sense |
| Locus 26 | 90 | 36 | Sense | 113 | Anti-sense |
| Locus 27 | 91 | 36 | Anti-sense | 114 | Anti-sense |
| Locus 28 | 92 | 36 | Anti-sense | 115 | Anti-sense |

The possible transcriptional directions of the putative tracrRNAs for each new system were considered by examining the secondary structures and possible termination signals present in a RNA version of the sense and anti-sense genomic DNA sequences surrounding the anti-repeat (as described in US patent applications 62/162,377 filed May 15, 2015, 62/162,353 filed May 15, 2015 and 62/196,535 filed July 24, 2015, all three applications incorporated in their entirety herein by reference). Based on the hairpin-like secondary structures and termination signals present for each system, the transcriptional direction of the tracrRNA for all the Type II CRISPR-Cas systems can be deduced. Because the anti-repeat in the tracrRNA can hybridize to the crRNA derived from the CRISPR array to form a duplexed RNA capable of guiding the Cas9 endonuclease to cleave invading DNA the transcriptional direction of the CRISPR array may also be determined based on the direction of tracrRNA transcription (since double-stranded RNA hybridizes with 5' to 3' directionality). The transcriptional directions of both the tracrRNA and CRISPR array were deduced for each system as described above and are listed in Table 4 and depicted in Figures 1-23. Based on the likely transcriptional direction of the tracrRNA and CRISPR array, single guide RNAs (sgRNAs, SEQ ID NOs: 185-207) were designed and are shown in Table 5.

**Table 5. Examples of sgRNAs (SEQ ID NOs: 185-207) and its components (VT, crRNA repeat, loop, anti-repeat and 3'tracrRNA) for each new diverse Type II CRISPR-Cas endonuclease described herein.**

| Cas endonuclease name | Variable targeting domain (VT) | crRNA repeat (SEQ ID NO) | Loop | Anti-Repeat (SEQ ID NO) | 3'tracrRNA (SEQ ID NO) | Single guide RNA (sgRNA) SEQ ID NO: |
|---|---|---|---|---|---|---|
| Cas-Locus 6 | N_{20 (*)} | 116 | N_{4 (**)} | 139 | 162 | 185 |
| Cas-Locus 7 | N_{20 (*)} | 117 | N_{4 (**)} | 140 | 163 | 186 |
| Cas-Locus 8 | N_{20 (*)} | 118 | N_{4 (**)} | 141 | 164 | 187 |
| Cas-Locus 9 | N_{20 (*)} | 119 | N_{4 (**)} | 142 | 165 | 188 |
| Cas-Locus 10 | N_{20 (*)} | 120 | N₄ (_{**)} | 143 | 166 | 189 |
| Cas-Locus 11 | N_{20 (*)} | 121 | N₄ (_{**)} | 144 | 167 | 190 |
| Cas-Locus 12 | N_{20 (*)} | 122 | N₄ (_{**)} | 145 | 168 | 191 |
| Cas-Locus 13 | N_{20 (*)} | 123 | N₄ (_{**)} | 146 | 169 | 192 |
| Cas-Locus 14 | N_{20 (*)} | 124 | N₄ (_{**)} | 147 | 170 | 193 |
| Cas-Locus 15 | N_{20 (*)} | 125 | N₄ (_{**)} | 148 | 171 | 194 |
| Cas-Locus 16 | N_{20 (*)} | 126 | N_{4 (**)} | 149 | 172 | 195 |
| Cas-Locus 17 | N_{20 (*)} | 127 | N_{4 (**)} | 150 | 173 | 196 |
| Cas-Locus 18 | N_{20 (*)} | 128 | N_{4 (**)} | 151 | 174 | 197 |
| Cas-Locus 19 | N_{20 (*)} | 129 | N_{4 (**)} | 152 | 175 | 198 |
| Cas-Locus 20 | N_{20 (*)} | 130 | N_{4 (**)} | 153 | 176 | 199 |
| Cas-Locus 21 | N_{20 (*)} | 131 | N_{4 (**)} | 154 | 177 | 200 |
| Cas-Locus 22 | N_{20 (*)} | 132 | N_{4 (**)} | 155 | 178 | 201 |
| Cas-Locus 23 | N_{20 (*)} | 133 | N_{4 (**)} | 156 | 179 | 202 |
| Cas-Locus 24 | N_{20 (*)} | 134 | N_{4 (**)} | 157 | 180 | 203 |
| Cas-Locus 25 | N_{20 (*)} | 135 | N_{4 (**)} | 158 | 181 | 204 |
| Cas-Locus 26 | N_{20 (*)} | 136 | N_{4 (**)} | 159 | 182 | 205 |
| Cas-Locus 27 | N_{20 (*)} | 137 | N_{4 (**)} | 160 | 183 | 206 |
| Cas-Locus 28 | N_{20 (*)} | 138 | N_{4 (**)} | 161 | 184 | 207 |

| | | | | | | |
|---|---|---|---|---|---|---|
| N_{20 (*)} indicates a series of 20 nucleotides as one example of a sgRNA variable targeting domain. As described herein, the variable targeting domain of a sgRNA can vary for example, but not limiting from at least 12 to 30 nucleotides. N_{4 (**)} indicates a loop of 4 nucleotides such as but not limiting to GAAA. As described herein, the length of the loop can vary from at least 3 nucleotides to 100 nucleotides. | | | | | | |

Rapid in vitro methods to characterize the protospacer adjacent motif (PAM) specificity of Type II Cas9 proteins have been described (see US patent applications 62/162,377 filed May 15, 2015, 62/162,353 filed May 15, 2015 and 62/196,535 filed July 24, 2015, incorporated in their entirety herein by reference) and can be used to characterize the PAM preference of the novel CRISPR-Cas systems described herein.

The single guide RNAs described herein (Table 5) can be complexed with the respective purified Cas9 protein (for example SEQ ID NO: 185 -Table 5- can be complexed with the Cas-Locus 6 endonuclease protein of SEQ ID NO: 47 - Table 3) and assayed for their ability to support cleavage of a randomized PAM plasmid DNA library (as described in Example 7 of US patent applications 62/162,377 filed May 15, 2015). If the sgRNA does not support cleavage activity, new guide RNA designs (either sgRNA or duplexed crRNA and tracrRNA; in both possible transcriptional directions of the CRISPR array and anti-repeat region) will be tested for their ability to support cleavage.

Once a guide RNA that supports Cas9 cleavage has been established, the PAM specificity of each Cas9 endonuclease can be assayed (as described in Examples 4, 8, 14 and 15 US patent applications 62/162,377 filed May 15, 2015). PAM preferences which extend past the randomized PAM region may also be examined (as described in Example 11 US patent applications 62/162,377 filed May 15, 2015). After PAM preferences have been determined, the sgRNAs may be further refined for maximal activity or cellular transcription by either increasing or decreasing the tracrRNA 3' end tail length, increasing or decreasing crRNA repeat and tracrRNA anti-repeat length, modifying the 4 nt self-folding loop or altering the sequence composition. The guide RNA solutions provided in Table 5 supported target recognition and cleavage for all of the Type II Cas9s examined (Cas-Locus 9, Cas-Locus 14, Cas-Locus 15, Cas-Locus 24, Cas-Locus 26, Cas-Locus 27 and Cas-Locus 28). Digestion of randomized PAM libraries followed by the capture and analysis of the PAM sequences which supported cleavage activity as described previously (see Examples 4, 8, 14 and 15 from US patent application 62/162,377 filed May 15) yielded the PAM recognition profiles shown in Tables 6-12.

Taken together, the Type II Cas9 proteins combined with the guide polynucleotide solutions listed in Table 5 were capable of programmable RNA directed DNA target recognition and cleavage.

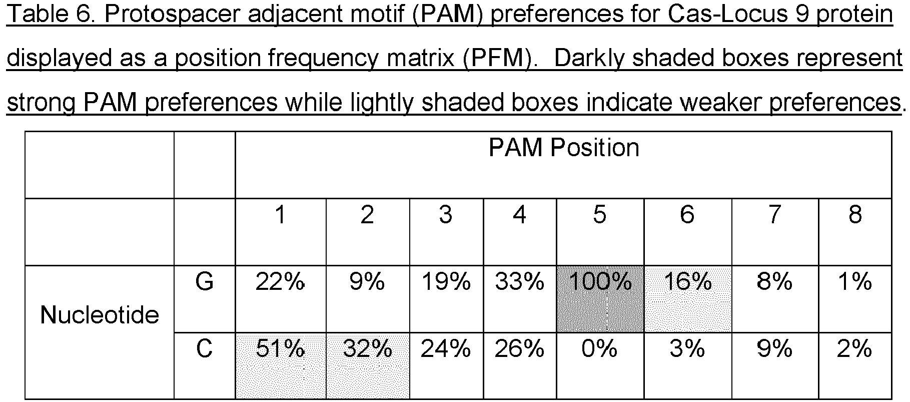

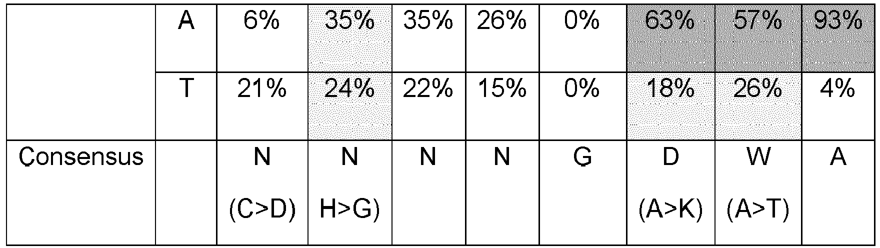

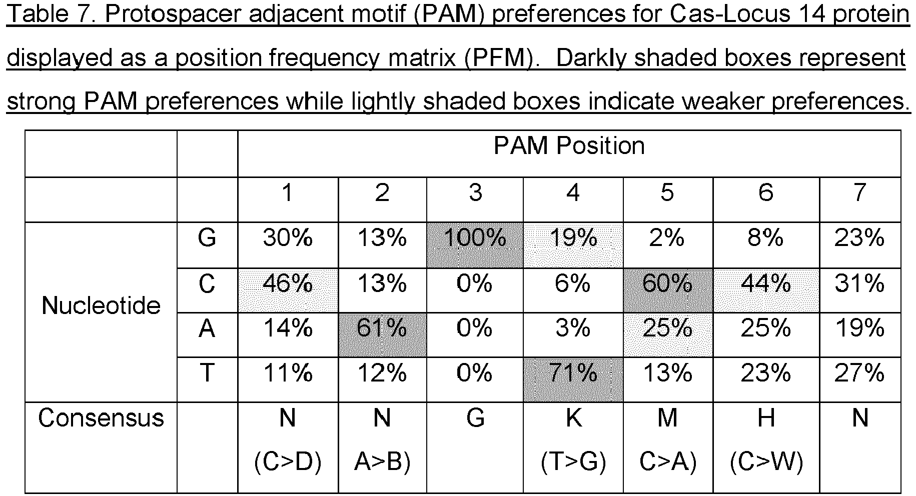

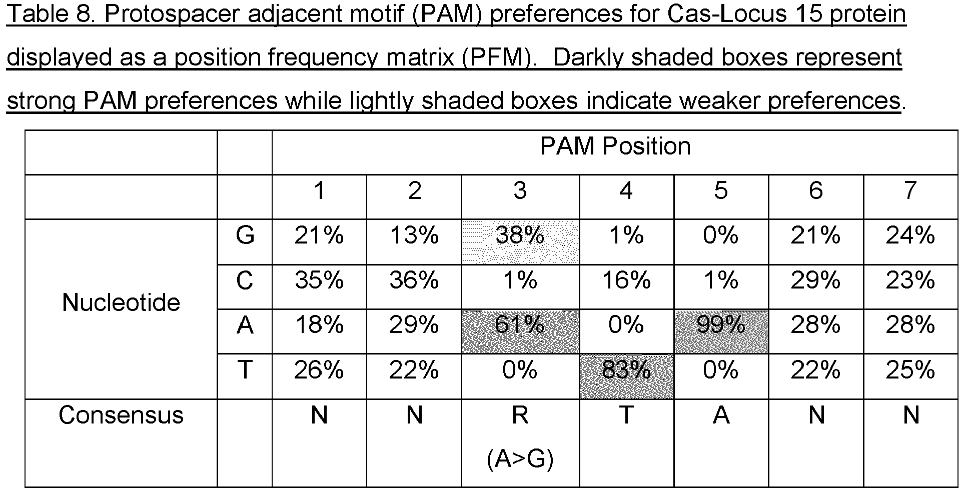

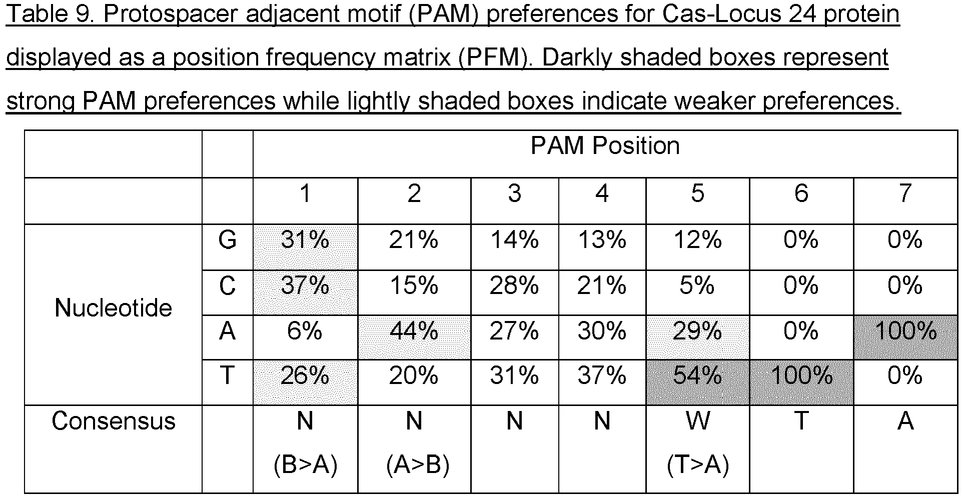

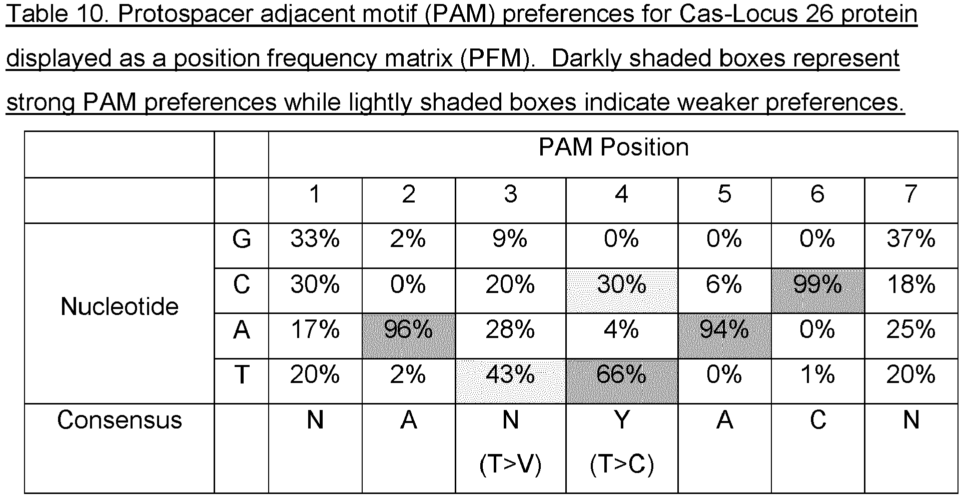

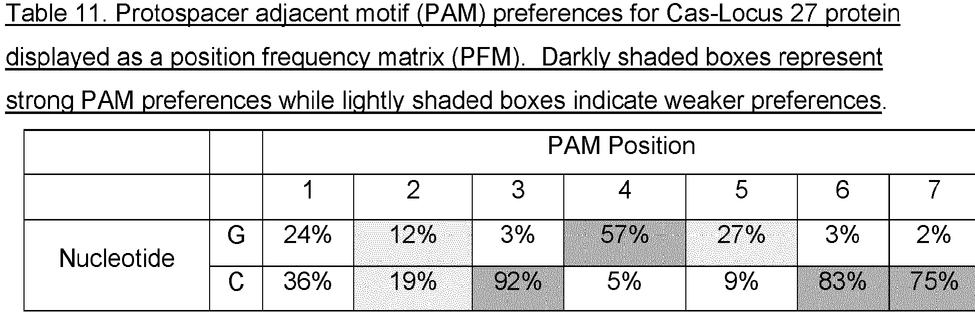

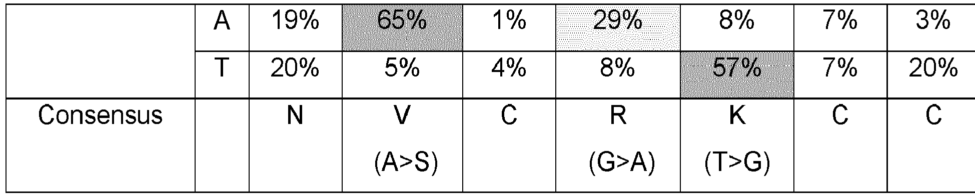

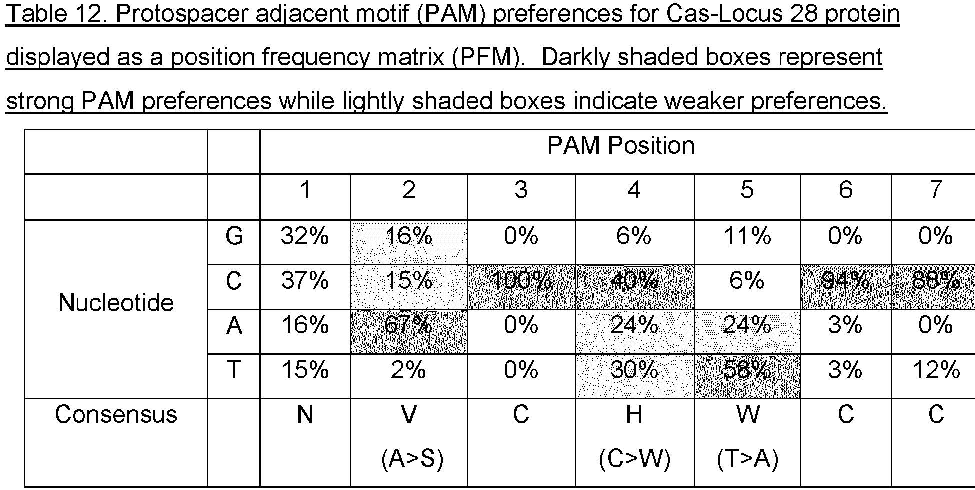

### EXAMPLE 2

### Identification of amino acid domains of novel Cas9 systems of the present disclosure.

Multiple functional domains and conserved elements were determined for each of the novel Cas9 endonuclease proteins of the present disclosure. Tables 13-14 show the domain location of the HNH, RuvC-I, RuvC-II, RuvC-III, REC1, REC1', REC-2, Bridge-Helix (BH) and PAM interacting (PI) domains along the amino acid sequence of each Cas9 endonuclease.

The novel Cas9 endonucleases of the present disclosure comprised an HNH domain, an RuvC domain that included three subdomains (RuvC-I, Ruvc-II and RuvC-II), a Brige Helicx domain a PAM interacting domain and DNA/RNA recognition regions including REC1 and REC1'. The REC1 binds to repeat::anti-repeat RNA duplex of the guide RNA while REC1' mainly interacts with targetDNA::guide RNA hybrid duplex. The REC2 domain is a conserved element.

**Table 13. Location of RuvC-I, BH, REC1 and REC2 domains of novel Cas9 endonucleases of the present disclosure relative to their respective Cas9 amino acid sequence.**

| Cas9 | Length of AA sequence | RuvC-I | BH | REC1 | REC2 |
|---|---|---|---|---|---|
| Locus-6 (SEQ ID NO: 47) | 1093 | 1-41 | 42-81 | 82-233 | None |
| Locus-7 (SEQ ID NO: 48) | 1092 | 1-41 | 42-81 | 82-232 | None |
| Locus-8 (SEQ ID NO: 49) | 1070 | 1-53 | 54-93 | 94-245 | None |
| Locus-9 (SEQ ID NO: 50) | 1081 | 1-41 | 42-81 | 82-231 | None |
| Locus-10 (SEQ ID NO: 51) | 1378 | 1-45 | 46-80 | 81-176 | 177-332 |
| Locus-11 (SEQ ID NO: 52) | 1337 | 1-58 | 59-93 | 94-176 | 177-312 |
| Locus-12 (SEQ ID NO: 53) | 1337 | 1-58 | 59-93 | 94-176 | 177-312 |
| Locus-13 (SEQ ID NO: 54) | 1337 | 1-58 | 59-93 | 94-176 | 177-312 |
| Locus-14 (SEQ ID NO: 55) | 1330 | 1-58 | 59-97 | 98-180 | 181-310 |
| Locus-15 (SEQ ID NO: 56) | 1150 | 1-40 | 41-75 | 76-257 | None |
| Locus-16 (SEQ ID NO: 57) | 1456 | 1-68 | 69-114 | 115-409 | undefined |
| Locus-17 (SEQ ID NO: 58) | 1462 | 1-80 | 81-126 | 127-431 | undefined |
| Locus-18 (SEQ ID NO: 59) | 1440 | 1-45 | 46-91 | 92-396 | undefined |
| Locus-19 (SEQ ID NO: 60) | 1440 | 1-45 | 46-91 | 92-396 | undefined |
| Locus-20 (SEQ ID NO: 61) | 1440 | 1-45 | 46-91 | 92-396 | undefined |
| Locus-21 (SEQ ID NO: 62) | 1233 | 1-13* | 14-59 | 60-207 | None |
| Locus-22 (SEQ ID NO: 63) | 1268 | 1-47 | 47-93 | 94-244 | None |
| Locus-23 (SEQ ID NO: 64) | 1264 | 1-45 | 46-91 | 92-239 | None |
| Locus-24 (SEQ ID NO: 65) | 1464 | 1-43 | 44-85 | 86-346 | undefined |
| Locus-25 (SEQ ID NO: 66) | 1458 | 1-43 | 44-85 | 86-347 | undefined |
| Locus-26 (SEQ ID NO: 67) | 1127 | 1-44 | 42-85 | 86-262 | None |
| Locus-27 (SEQ ID NO: 68) | 1108 | 1-41 | 45-86 | 87-241 | None |
| Locus-28 (SEQ ID NO: 69) | 1108 | 1-44 | 45-86 | 87-241 | None |

| | | | | | |
|---|---|---|---|---|---|
| *This RuvC domain is missing a N-terminal fragment | | | | | |

**Table 14. Location of REC1', RuvC-II, HNH, RuvC-III and PAM interacting (PI) domains of novel Cas9 endonucleases of the present disclosure relative to their respective Cas9 amino acid sequence.**

| Cas9 | REC1' | RuvC-II | HNH | RuvC-III | PI |
|---|---|---|---|---|---|
| Locus-6 (SEQ ID NO: 47) | 234-463 | 464-508 | 509-664 | 665-810 | 811-1093 |
| Locus-7 (SEQ ID NO: 48) | 233-462 | 463-707 | 508-663 | 664-809 | 810-1092 |
| Locus-8 (SEQ ID NO: 49) | 246-473 | 474-519 | 520-683 | 684-808 | 809-1070 |
| Locus-9 (SEQ ID NO: 50) | 231-460 | 461-505 | 506-660 | 661-808 | 809-1081 |
| Locus-10 (SEQ ID NO: 51) | 333-748 | 749-796 | 750-944 | 945-1101 | 1102-1378 |
| Locus-11 (SEQ ID NO: 52) | 313-729 | 730-777 | 778-930 | 931-1084 | 1085-1337 |
| Locus-12 (SEQ ID NO: 53) | 313-729 | 730-777 | 778-930 | 931-1084 | 1085-1337 |
| Locus-13 (SEQ ID NO: 54) | 313-729 | 730-777 | 778-930 | 931-1084 | 1085-1337 |
| Locus-14 (SEQ ID NO: 55) | 311-719 | 720-770 | 771-922 | 923-1104 | 1105-1330 |
| Locus-15 (SEQ ID NO: 56) | 258-470 | 471-517 | 517-690 | 691-848 | 849-1150 |
| Locus-16 (SEQ ID NO: 57) | undefined | 691-743 | 744-950 | 921-1178 | 1179-1456 |
| Locus-17 (SEQ ID NO: 58) | undefined | 711-764 | 765-971 | 972-1199 | 1200-1462 |
| Locus-18 (SEQ ID NO: 59) | undefined | 677-729 | 720-936 | 936-1164 | 1165-1440 |
| Locus-19 (SEQ ID NO: 60) | undefined | 677-729 | 720-936 | 936-1164 | 1165-1440 |
| Locus-20 (SEQ ID NO: 61) | undefined | 677-729 | 720-936 | 936-1164 | 1165-1440 |
| Locus-21 (SEQ ID NO: 62) | 208-474 | 475-521 | 522-704 | 705-892 | 893-1233 |
| Locus-22 (SEQ ID NO: 63) | 245-511 | 512-558 | 559-741 | 742-929 | 930-1268 |
| Locus-23 (SEQ ID NO: 64) | 240-506 | 507-553 | 554-736 | 737-924 | 925-1264 |
| Locus-24 (SEQ ID NO: 65) | undefined | 734-780 | 781-965 | 966-1163 | 1164-1464 |
| Locus-25 (SEQ ID NO: 66) | undefined | 728-774 | 775-969 | 970-1157 | 1158-1458 |
| Locus-26 (SEQ ID NO: 67) | 262-515 | 516-573 | 574-728 | 729-868 | 869-1127 |
| Locus-27 (SEQ ID NO: 68) | 242-497 | 498-543 | 543-718 | 719-886 | 887-1108 |
| Locus-28 (SEQ ID NO: 69) | 242-497 | 498-543 | 544-718 | 719-886 | 887-1108 |

| | | | | | |
|---|---|---|---|---|---|
| Length refers to the total amino acids of each Cas9 endonuclease protein. | | | | | |

The number range shown for each Cas9 endonuclease domain (RuvC-I, Bridge Helix (BH), REC1, REC2, REC1', RuvC-II, HNH, RuvC-III and PAM interacting (PI)) indicates the location of the first amino acid and last amino acid of that domain relative to the amino acid sequence of its respective Cas9 endonuclease. For example, the RuvCI domain of Cas-Locus-6 (1-41) comprises 41 amino acids spanning from the first amino acid (amino acid 1) to the 41^{st} amino acid of the Cas9 endonuclease (Cas-Locus6) of SEQ ID NO: 11. None indicates that no REC2 domain is present is said Cas9 endonuclease.

### EXAMPLE 3

### Transformation of Maize Immature Embryos

Transformation can be accomplished by various methods known to be effective in plants, including particle-mediated delivery, *Agrobacterium*-mediated transformation, PEG-mediated delivery, and electroporation.

### a. Particle-mediated delivery

Transformation of maize immature embryos using particle delivery is performed as follows. Media recipes follow below.

The ears are husked and surface sterilized in 30% Clorox bleach plus 0.5% Micro detergent for 20 minutes, and rinsed two times with sterile water. The immature embryos are isolated and placed embryo axis side down (scutellum side up), 25 embryos per plate, on 560Y medium for 4 hours and then aligned within the 2.5-cm target zone in preparation for bombardment. Alternatively, isolated embryos are placed on 560L (Initiation medium) and placed in the dark at temperatures ranging from 26°C to 37°C for 8 to 24 hours prior to placing on 560Y for 4 hours at 26°C prior to bombardment as described above.

Plasmids containing the double strand brake inducing agent and donor DNA are constructed using standard molecular biology techniques and co-bombarded with plasmids containing the developmental genes ODP2 (AP2 domain transcription factor ODP2 (Ovule development protein 2); US20090328252 A1) and Wushel (US2011/0167516).

The plasmids and DNA of interest are precipitated onto 0.6 µm (average diameter) gold pellets using a water-soluble cationic lipid transfection reagent as follows. DNA solution is prepared on ice using 1 µg of plasmid DNA and optionally other constructs for co-bombardment such as 50 ng (0.5 µl) of each plasmid containing the developmental genes ODP2 (AP2 domain transcription factor ODP2 (Ovule development protein 2); US20090328252 A1) and Wushel. To the pre-mixed DNA, 20 µl of prepared gold particles (15 mg/ml) and 5 µl of a water-soluble cationic lipid transfection reagent is added in water and mixed carefully. Gold particles are pelleted in a microfuge at 10,000 rpm for 1 min and supernatant is removed. The resulting pellet is carefully rinsed with 100 ml of 100% EtOH without resuspending the pellet and the EtOH rinse is carefully removed. 105 µl of 100% EtOH is added and the particles are resuspended by brief sonication. Then, 10 µl is spotted onto the center of each macrocarrier and allowed to dry about 2 minutes before bombardment.

Alternatively, the plasm ids and DNA of interest are precipitated onto 1.1 µm (average diameter) tungsten pellets using a calcium chloride (CaCl₂) precipitation procedure by mixing 100 µl prepared tungsten particles in water, 10 µl (1 µg) DNA in Tris EDTA buffer (1 µg total DNA), 100 µl 2.5 M CaC12, and 10 µl 0.1 M spermidine. Each reagent is added sequentially to the tungsten particle suspension, with mixing. The final mixture is sonicated briefly and allowed to incubate under constant vortexing for 10 minutes. After the precipitation period, the tubes are centrifuged briefly, liquid is removed, and the particles are washed with 500 ml 100% ethanol, followed by a 30 second centrifugation. Again, the liquid is removed, and 105 µl of 100% ethanol is added to the final tungsten particle pellet. For particle gun bombardment, the tungsten/DNA particles are briefly sonicated. 10 µl of the tungsten/DNA particles is spotted onto the center of each macrocarrier, after which the spotted particles are allowed to dry about 2 minutes before bombardment.

The sample plates are bombarded at level #4 with a Biorad Helium Gun. All samples receive a single shot at 450 PSI, with a total of ten aliquots taken from each tube of prepared particles/DNA.

Following bombardment, the embryos are incubated on 560P (maintenance medium) for 12 to 48 hours at temperatures ranging from 26C to 37C, and then placed at 26C. After 5 to 7 days the embryos are transferred to 560R selection medium containing 3 mg/liter Bialaphos, and subcultured every 2 weeks at 26C. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to a lighted culture room. Approximately 7-10 days later, developing plantlets are transferred to 272V hormone-free medium in tubes for 7-10 days until plantlets are well established. Plants are then transferred to inserts in flats (equivalent to a 2.5" pot) containing potting soil and grown for 1 week in a growth chamber, subsequently grown an additional 1-2 weeks in the greenhouse, then transferred to Classic 600 pots (1.6 gallon) and grown to maturity. Plants are monitored and scored for transformation efficiency, and/or modification of regenerative capabilities.

Initiation medium (560L) comprises 4.0 g/l N6 basal salts (SIGMA C-1416), 1.0 ml/l Eriksson's Vitamin Mix (1000X SIGMA-1511), 0.5 mg/l thiamine HCl, 20.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature).

Maintenance medium (560P) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, 2.0 mg/l 2,4-D, and 0.69 g/l L-proline (brought to volume with D-I H2O following adjustment to pH 5.8 with KOH); 3.0 g/l Gelrite (added after bringing to volume with D-I H2O); and 0.85 mg/l silver nitrate (added after sterilizing the medium and cooling to room temperature).

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCI, 120.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature).

Selection medium (560R) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, and 2.0 mg/l 2,4-D (brought to volume with D-I H2O following adjustment to pH 5.8 with KOH); 3.0 g/l Gelrite (added after bringing to volume with D-I H2O); and 0.85 mg/l silver nitrate and 3.0 mg/l bialaphos (both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCL, 0.10 g/l pyridoxine HCL, and 0.40 g/l glycine brought to volume with polished D-I H2O) (Murashige and Skoog (1962) Physiol. Plant. 15:473), 100 mg/l myo-inositol, 0.5 mg/l zeatin, 60 g/l sucrose, and 1.0 ml/l of 0.1 mM abscisic acid (brought to volume with polished D-I H2O after adjusting to pH 5.6); 3.0 g/l Gelrite (added after bringing to volume with D-I H2O); and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCL, 0.10 g/l pyridoxine HCL, and 0.40 g/l glycine brought to volume with polished D-I H2O), 0.1 g/l myo-inositol, and 40.0 g/l sucrose (brought to volume with polished D-I H2O after adjusting pH to 5.6); and 6 g/l bacto-agar (added after bringing to volume with polished D-I H2O), sterilized and cooled to 60°C.

### b. Agrobacterium-mediated transformation

*Agrobacterium*-mediated transformation was performed essentially as described in Djukanovic et al. (2006) Plant Biotech J 4:345-57. Briefly, 10-12 day old immature embryos (0.8 -2.5 mm in size) were dissected from sterilized kernels and placed into liquid medium (4.0 g/L N6 Basal Salts (Sigma C-1416), 1.0 ml/L Eriksson's Vitamin Mix (Sigma E-1511), 1.0 mg/L thiamine HCI, 1.5 mg/L 2, 4-D, 0.690 g/L L-proline, 68.5 g/L sucrose, 36.0 g/L glucose, pH 5.2). After embryo collection, the medium was replaced with 1 ml *Agrobacterium* at a concentration of 0.35-0.45 OD550. Maize embryos were incubated with *Agrobacterium* for 5 min at room temperature, then the mixture was poured onto a media plate containing 4.0 g/L N6 Basal Salts (Sigma C-1416), 1.0 ml/L Eriksson's Vitamin Mix (Sigma E-1511), 1.0 mg/L thiamine HCI, 1.5 mg/L 2, 4-D, 0.690 g/L L-proline, 30.0 g/L sucrose, 0.85 mg/L silver nitrate, 0.1 nM acetosyringone, and 3.0 g/L Gelrite, pH 5.8. Embryos were incubated axis down, in the dark for 3 days at 20°C, then incubated 4 days in the dark at 28°C, then transferred onto new media plates containing 4.0 g/L N6 Basal Salts (Sigma C-1416), 1.0 ml/L Eriksson's Vitamin Mix (Sigma E-1511), 1.0 mg/L thiamine HCI, 1.5 mg/L 2, 4-D, 0.69 g/L L-proline, 30.0 g/L sucrose, 0.5 g/L MES buffer, 0.85 mg/L silver nitrate, 3.0 mg/L Bialaphos, 100 mg/L carbenicillin, and 6.0 g/L agar, pH 5.8. Embryos were subcultured every three weeks until transgenic events were identified. Somatic embryogenesis was induced by transferring a small amount of tissue onto regeneration medium (4.3 g/L MS salts (Gibco 11117), 5.0 ml/L MS Vitamins Stock Solution, 100 mg/L myo-inositol, 0.1 µM ABA, 1 mg/L IAA, 0.5 mg/L zeatin, 60.0 g/L sucrose, 1.5 mg/L Bialaphos, 100 mg/L carbenicillin, 3.0 g/L Gelrite, pH 5.6) and incubation in the dark for two weeks at 28°C. All material with visible shoots and roots were transferred onto media containing 4.3 g/L MS salts (Gibco 11117), 5.0 ml/L MS Vitamins Stock Solution, 100 mg/L myo-inositol, 40.0 g/L sucrose, 1.5 g/L Gelrite, pH 5.6, and incubated under artificial light at 28°C. One week later, plantlets were moved into glass tubes containing the same medium and grown until they were sampled and/or transplanted into soil.

### EXAMPLE 4

### Transient Expression of BBM Enhances Transformation

Parameters of the transformation protocol can be modified to ensure that the BBM activity is transient. One such method involves precipitating the BBM-containing plasmid in a manner that allows for transcription and expression, but precludes subsequent release of the DNA, for example, by using the chemical PEI. In one example, the BBM plasmid is precipitated onto gold particles with PEI, while the transgenic expression cassette (UBI::moPAT∼GFPm::PinII; moPAT is the maize optimized PAT gene) to be integrated is precipitated onto gold particles using the standard calcium chloride method.

Briefly, gold particles were coated with PEI as follows. First, the gold particles were washed. Thirty-five mg of gold particles, 1.0 in average diameter (A.S.I. #162-0010), were weighed out in a microcentrifuge tube, and 1.2 ml absolute EtOH was added and vortexed for one minute. The tube was incubated for 15 minutes at room temperature and then centrifuged at high speed using a microfuge for 15 minutes at 4oC. The supernatant was discarded and a fresh 1.2 ml aliquot of ethanol (EtOH) was added, vortexed for one minute, centrifuged for one minute, and the supernatant again discarded (this is repeated twice). A fresh 1.2 ml aliquot of EtOH was added, and this suspension (gold particles in EtOH) was stored at -20oC for weeks. To coat particles with polyethylimine (PEI; Sigma #P3143), 250 µl of the washed gold particle/EtOH mix was centrifuged and the EtOH discarded. The particles were washed once in 100 µl ddH2O to remove residual ethanol, 250 µl of 0.25 mM PEI was added, followed by a pulse-sonication to suspend the particles and then the tube was plunged into a dry ice/EtOH bath to flash-freeze the suspension, which was then lyophilized overnight. At this point, dry, coated particles could be stored at -80oC for at least 3 weeks. Before use, the particles were rinsed 3 times with 250 µl aliquots of 2.5 mM HEPES buffer, pH 7.1, with 1x pulse-sonication, and then a quick vortex before each centrifugation. The particles were then suspended in a final volume of 250 µl HEPES buffer. A 25 µl aliquot of the particles was added to fresh tubes before attaching DNA. To attach uncoated DNA, the particles were pulse-sonicated, then 1 µg of DNA (in 5 µl water) was added, followed by mixing by pipetting up and down a few times with a Pipetteman and incubated for 10 minutes. The particles were spun briefly (i.e. 10 seconds), the supernatant removed, and 60 µl EtOH added. The particles with PEI-precipitated DNA-1 were washed twice in 60 µl of EtOH. The particles were centrifuged, the supernatant discarded, and the particles were resuspended in 45 µl water. To attach the second DNA (DNA-2), precipitation using a water-soluble cationic lipid transfection reagent was used. The 45 µl of particles/DNA-1 suspension was briefly sonicated, and then 5 µl of 100 ng/µl of DNA-2 and 2.5 µl of the water-soluble cationic lipid transfection reagent were added. The solution was placed on a rotary shaker for 10 minutes, centrifuged at 10,000g for 1 minute. The supernatant was removed, and the particles resuspended in 60 µl of EtOH. The solution was spotted onto macrocarriers and the gold particles onto which DNA-1 and DNA-2 had been sequentially attached were delivered into scutellar cells of 10 DAP Hi-II immature embryos using a standard protocol for the PDS-1000. For this experiment, the DNA-1 plasmid contained a UBI::RFP::pinII expression cassette, and DNA-2 contained a UBI::CFP::pinll expression cassette. Two days after bombardment, transient expression of both the CFP and RFP fluorescent markers was observed as numerous red & blue cells on the surface of the immature embryo. The embryos were then placed on non-selective culture medium and allowed to grow for 3 weeks before scoring for stable colonies. After this 3-week period, 10 multicellular, stably-expressing blue colonies were observed, in comparison to only one red colony. This demonstrated that PEI-precipitation could be used to effectively introduce DNA for transient expression while dramatically reducing integration of the PEI-introduced DNA and thus reducing the recovery of RFP-expressing transgenic events. In this manner, PEI-precipitation can be used to deliver transient expression of BBM and/or WUS2.

For example, the particles are first coated with UBI::BBM::pinll using PEI, then coated with UBI::moPAT∼YFP using a water-soluble cationic lipid transfection reagent, and then bombarded into scutellar cells on the surface of immature embryos. PEI-mediated precipitation results in a high frequency of transiently expressing cells on the surface of the immature embryo and extremely low frequencies of recovery of stable transformants Thus, it is expected that the PEI-precipitated BBM cassette expresses transiently and stimulates a burst of embryogenic growth on the bombarded surface of the tissue (i.e. the scutellar surface), but this plasmid will not integrate. The PAT∼GFP plasmid released from the Ca++/gold particles is expected to integrate and express the selectable marker at a frequency that results in substantially improved recovery of transgenic events. As a control treatment, PEI-precipitated particles containing a UBI::GUS::pinII (instead of BBM) are mixed with the PAT∼GFP/Ca++ particles. Immature embryos from both treatments are moved onto culture medium containing 3mg/l bialaphos. After 6-8 weeks, it is expected that GFP+, bialaphos-resistant calli will be observed in the PEI/BBM treatment at a much higher frequency relative to the control treatment (PEI/GUS).

As an alternative method, the BBM plasmid is precipitated onto gold particles with PEI, and then introduced into scutellar cells on the surface of immature embryos, and subsequent transient expression of the BBM gene elicits a rapid proliferation of embryogenic growth. During this period of induced growth, the explants are treated with Agrobacterium using standard methods for maize (see Example 1), with T-DNA delivery into the cell introducing a transgenic expression cassette such as UBI::moPAT∼GFPm::pinII. After co-cultivation, explants are allowed to recover on normal culture medium, and then are moved onto culture medium containing 3 mg/l bialaphos. After 6-8 weeks, it is expected that GFP+, bialaphos-resistant calli will be observed in the PEI/BBM treatment at a much higher frequency relative to the control treatment (PEI/GUS).

It may be desirable to "kick start" callus growth by transiently expressing the BBM and/or WUS2 polynucleotide products. This can be done by delivering BBM and WUS2 5'-capped polyadenylated RNA, expression cassettes containing BBM and WUS2 DNA, or BBM and/or WUS2 proteins. All of these molecules can be delivered using a biolistics particle gun. For example 5'-capped polyadenylated BBM and/or WUS2 RNA can easily be made in vitro using Ambion's mMessage mMachine kit. RNA is co-delivered along with DNA containing a polynucleotide of interest and a marker used for selection/screening such as Ubi::moPAT∼GFPm::PinII. It is expected that the cells receiving the RNA will immediately begin dividing more rapidly and a large portion of these will have integrated the agronomic gene. These events can further be validated as being transgenic clonal colonies because they will also express the PAT∼GFP fusion protein (and thus will display green fluorescence under appropriate illumination). Plants regenerated from these embryos can then be screened for the presence of the polynucleotide of interest.

### ASPECTS OF THE INVENTION

1. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA is selected from the group consisting of SEQ ID NOs: 185-207, a functional fragment of SEQ ID NOs: 185-207, and a functional variant of SEQ ID NOs: 185-207.
2. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of SEQ ID NOs: 139-184, a functional fragment of SEQ ID NOs: 139-184, and a functional variant of SEQ ID NOs: 139-184.
3. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of SEQ ID NOs: 116-138, a functional fragment of SEQ ID NOs: 116-138, and a functional variant of SEQ ID NOs: 116-138.
4. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a variable targeting domain capable of hybridizing to said target sequence, wherein said tracrRNA comprises a nucleotide sequence selected from the group consisting of SEQ ID NOs: 139-184, a functional fragment of SEQ ID NOs: 139-184, and a functional variant of SEQ ID NOs: 139-184, wherein said chimeric non-naturally occurring crRNA comprises a variable targeting domain capable of hybridizing to said target sequence.
5. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a nucleotide sequence selected from the group consisting of SEQ ID NOs: 116-138, a functional fragment of SEQ ID NOs: 116-138, and a functional variant of SEQ ID NOs: 116-138, wherein said chimeric non-naturally occurring crRNA comprises a variable targeting domain capable of hybridizing to said target sequence.
6. A guide RNA/Cas9 endonuclease complex comprising a Cas9 endonuclease selected from the group consisting of SEQ ID NOs: 47-69, a functional fragment of SEQ ID NOs: 47-69, and a functional variant of SEQ ID NOs: 47-69 , and at least one guide RNA, wherein said guide RNA/Cas9 endonuclease complex is capable of recognizing, binding to, and optionally nicking or cleaving all or part of a target sequence.
7. A guide RNA/Cas9 endonuclease complex comprising at least one guide RNA and a Cas9 endonuclease, wherein said Cas9 endonuclease is encoded by a DNA sequence selected from the group consisting of SEQ ID NOs: 24-46, a functional fragment of SEQ ID NOs: 24-46, and a functional variant of SEQ ID NOs: 24-46, wherein said guide RNA/Cas9 endonuclease complex is capable of recognizing, binding to, and optionally nicking or cleaving all or part of a target sequence.
8. The guide RNA/Cas9 endonuclease complex of any of aspects 6-7 comprising at least one guide RNA of any one of aspects 1-5.
9. The guide RNA/Cas9 endonuclease complex of any of aspects 6-7, wherein said target sequence is located in the genome of a cell.
10. A method for modifying a target site in the genome of a cell, the method comprising introducing into said cell at least one guide RNA and at least one Cas9 endonuclease selected from the group consisting of SEQ ID NOs: 47-69, a functional fragment of SEQ ID NOs: 47-69, and a functional variant of SEQ ID NOs: 47-69 , wherein said guide RNA and Cas9 endonuclease can form a complex that is capable of recognizing, binding to, and optionally nicking or cleaving all or part of said target site.
11. The method of aspect 10, further comprising identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii).
12. A method for editing a nucleotide sequence in the genome of a cell, the method comprising introducing into said cell a polynucleotide modification template, at least one guide RNA and at least one Cas9 endonuclease selected from the group consisting of SEQ ID NOs: 47-69, a functional fragment of SEQ ID NOs: 47-69, and a functional variant of SEQ ID NOs: 47-69, wherein said polynucleotide modification template comprises at least one nucleotide modification of said nucleotide sequence, wherein said guide RNA and Cas9 endonuclease can form a complex that is capable of recognizing, binding to, and optionally nicking or cleaving all or part of said target site.
13. A method for modifying a target site in the genome of a cell, the method comprising introducing into said cell at least one guide RNA, at least one donor DNA, and at least one Cas9 endonuclease selected from the group consisting of SEQ ID NOs: 47-69, a functional fragment of SEQ ID NOs: 47-69, and a functional variant of SEQ ID NOs: 47-69, wherein said at least one guide RNA and at least one Cas9 endonuclease can form a complex that is capable of recognizing, binding to, and optionally nicking or cleaving all or part of said target site, wherein said donor DNA comprises a polynucleotide of interest.
14. The method of aspect 13, further comprising identifying at least one cell that has said polynucleotide of interest integrated in or near said target site.
15. The method of any one of aspects 10-14, wherein the cell is selected from the group consisting of a human, non-human, animal, bacterial, fungal, insect, yeast, non-conventional yeast, and plant cell.
16. The method of aspect 15, wherein the plant cell is selected from the group consisting of a monocot and dicot cell.
17. The method of aspect 16, wherein the plant cell is selected from the group consisting of maize, rice, sorghum, rye, barley, wheat, millet, oats, sugarcane, turfgrass, or switchgrass, soybean, canola, alfalfa, sunflower, cotton, tobacco, peanut, potato, tobacco, Arabidopsis, and safflower cell.
18. A plant comprising a modified target site, wherein said plant originates from a plant cell comprising a modified target site produced by the method of any of aspects 10-17.
19. A plant comprising an edited nucleotide, wherein said plant originates from a plant cell comprising an edited nucleotide produced by the method of aspect 12.

## Claims

1. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA is selected from the group consisting of:
(a) SEQ ID NOs: 193, 185-187, 189-192, and 194-207; and
(b) a sequence sharing at least 90% sequence identity with any one of SEQ ID NOs: 193, 185-187, 189-192, and 194-207 and retaining the ability to function as a guide RNA.

2. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of:
(a) SEQ ID NOs: 170, 139-164, 166-169, and 171-184; and
(b) a sequence sharing at least 90% sequence identity with any one of SEQ ID NOs: 170, 139-164, 166-169, and 171-184 and retaining the ability to function as a tracrRNA.

3. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of:
(a) SEQ ID NOs: 124, 116-118, 120-123, and 125-138; and
(b) a sequence sharing at least 90% sequence identity with any one of SEQ ID NOs: 124, 116-118, 120-123, and 125-138 and retaining the ability to function as a crRNA.

4. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a variable targeting domain capable of hybridizing to said target sequence, wherein said tracrRNA comprises a nucleotide sequence selected from the group consisting of:
(a) SEQ ID NOs: 170, 139-164, 166-169, and 171-184; and
(b) a sequence sharing at least 90% sequence identity with any one of SEQ ID NOs: 170, 139-164, 166-169, and 171-184 and retaining the ability to function as a tracrRNA;
wherein said chimeric non-naturally occurring crRNA comprises a variable targeting domain capable of hybridizing to said target sequence.

5. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a nucleotide sequence selected from the group consisting of:
(a) SEQ ID NOs: 124, 116-118, 120-123, and 125-138; and
(b) a sequence sharing at least 90% sequence identity with any one of SEQ ID NOs: 124, 116-118, 120-123, and 125-138 and retaining the ability to function as a crRNA;
wherein said chimeric non-naturally occurring crRNA comprises a variable targeting domain capable of hybridizing to said target sequence.

6. A guide RNA/Cas9 endonuclease complex, wherein the guide RNA/Cas9 endonuclease complex comprises a guide RNA of any one of claims 1 to 5:
(a) further comprising a Cas9 endonuclease selected from the group consisting of:
(i) SEQ ID NOs: 55, 47-49, 51-54, and 56-69; and
(ii) a sequence sharing at least 90% sequence identity with any one of SEQ ID NOs: 55, 47-49, 51-54, and 56-69 and retaining the ability to recognise, bind to and optionally nick or cleave a target site; or
(b) further comprising a Cas9 endonuclease encoded by a DNA sequence consisting of any one of SEQ ID NOs: 32, 24-26, 28-31, and 33-46;
wherein said guide RNA/Cas9 endonuclease complex is capable of recognizing, binding to, and optionally nicking or cleaving all or part of a target sequence.

7. The guide RNA/Cas9 endonuclease complex of claim 6, wherein said target sequence is located in the genome of a cell.

8. A method for modifying a target site in the genome of a cell, the method comprising introducing into said cell:
(a) at least one guide RNA comprising:
(i) any one of SEQ ID NOs: 124, 116-118, 120-123, and 125-138, any one of SEQ ID NOs: 170, 139-164, 166-169, and 171-184, or any one of SEQ ID NOs: 193, 185-187, 189-192, and 194-207; or
(ii) a sequence sharing at least 90% sequence identity with any one of SEQ ID NOs: 124, 116-118, 120-123, and 125-138, any one of SEQ ID NOs: 170, 139-164, 166-169, and 171-184, or any one of SEQ ID NOs: 193, 185-187, 189-192, and 194-207 and retaining the ability to function as a guide RNA; and
(b) at least one Cas9 endonuclease selected from the group consisting of:
(i) SEQ ID NOs: 55, 47-49, 51-54, and 56-69; and
(ii) a sequence sharing at least 90% sequence identity with any one of SEQ ID NOs: 55, 47-49, 51-54, and 56-69 and retaining the ability to recognise, bind to and optionally nick or cleave a target site;
wherein said guide RNA and Cas9 endonuclease can form a complex that is capable of recognizing, binding to, and optionally nicking or cleaving all or part of said target site; and wherein the cell is selected from the group consisting of a bacterial, fungal, insect, yeast, non-conventional yeast, and plant cell.

9. The method of claim 8, further comprising:
(a) identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii); or
(b) introducing into said cell a polynucleotide modification template, wherein said polynucleotide modification template comprises at least one nucleotide modification as compared to said nucleotide sequence, wherein said guide RNA and Cas9 endonuclease can form a complex that is capable of recognizing, binding to, and optionally nicking or cleaving all or part of said target site.

10. The method of claim 8, further comprising introducing into said cell at least one donor DNA, wherein said donor DNA comprises a polynucleotide of interest.

11. The method of claim 10, further comprising identifying at least one cell that has said polynucleotide of interest integrated in or near said target site.

12. The method of any one of claims 8 to 11, wherein the plant cell is selected from the group consisting of a monocot and dicot cell;
preferably wherein the plant cell is selected from the group consisting of maize, rice, sorghum, rye, barley, wheat, millet, oats, sugarcane, turfgrass, switchgrass, soybean, canola, alfalfa, sunflower, cotton, peanut, potato, tobacco, Arabidopsis, and safflower cell.

13. A cell comprising:
(a) at least one Cas9 endonuclease comprising any one of SEQ ID NOs: 55, 47-49, 51-54, and 56-69 or sharing at least 90% sequence identity with any one of SEQ ID NOs: 55, 47-49, 51-54, and 56-69; and
(b) at least one guide RNA comprising any one of SEQ ID NOs: 124, 116-118, 120-123, and 125-138, any one of SEQ ID NOs: 170, 139-164, 166-169, and 171-184, or any one of SEQ ID NOs: 193, 185-187, 189-192, and 194-207, or a sequence sharing at least 90% sequence identity with any one of SEQ ID NOs: 124, 116-118, 120-123, and 125-138, with any one of SEQ ID NOs: 170, 139-164, 166-169, and 171-184, or with any one of SEQ ID NOs: 193, 185-187, 189-192, and 194-207;
preferably wherein the cell is selected from the group consisting of a human, non-human, animal, bacterial, fungal, insect, yeast, non-conventional yeast, and plant cell.

14. An *ex vivo* method for modifying a target site in the genome of a cell, the method comprising introducing into said cell:
(a) at least one guide RNA comprising:
(i) any one of SEQ ID NO: 124, 116-118, 120-123, and 125-138, any one of SEQ ID NOs: 170, 139-164, 166-169, and 171-184, or any one of SEQ ID NOs: 193, 185-187, 189-192, and 194-207; or
(ii) a sequence sharing at least 90% sequence identity with any one of SEQ ID NOs: 124, 116-118, 120-123, and 125-138, with any one of SEQ ID NOs: 170, 139-164, 166-169, and 171-184, or with any one of SEQ ID NOs: 193, 185-187, 189-192, and 194-207 and retaining the ability to function as a guide RNA; and
(b) at least one Cas9 endonuclease selected from the group consisting of:
(i) SEQ ID NOs: 55, 47-49, 51-54, and 56-69; and
(ii) a sequence sharing at least 90% sequence identity with any one of SEQ ID NOs: 55, 47-49, 51-54, and 56-69 and retaining the ability to recognise, bind to and optionally nick or cleave a target site;
wherein said guide RNA and Cas9 endonuclease can form a complex that is capable of recognizing, binding to, and optionally nicking or cleaving all or part of said target site.

15. At least:
(a) one guide RNA comprising:
(i) any one of SEQ ID NOs: 124, 116-118, 120-123, and 125-138, any one of SEQ ID NOs: 170, 139-164, 166-169, and 171-184, or any one of SEQ ID NOs: 193, 185-187, 189-192, and 194-207; or
(ii) a sequence sharing at least 90% sequence identity with any one of SEQ ID NOs: 124, 116-118, 120-123, and 125-138, with any one of SEQ ID NOs: 170, 139-164, 166-169, and 171-184, or with any one of SEQ ID NOs: 193, 185-187, 189-192, and 194-207 and retaining the ability to function as a guide RNA; and
(b) one Cas9 endonuclease selected from the group consisting of:
(i) SEQ ID NOs: 55, 47-49, 51-54, and 56-69; and
(ii) a sequence sharing at least 90% sequence identity with any one of SEQ ID NOs: 55, 47-49, 51-54, and 56-69 and retaining the ability to recognise, bind to and optionally nick or cleave a target site;
wherein said guide RNA and Cas9 endonuclease can form a complex that is capable of recognizing, binding to, and optionally nicking or cleaving all or part of said target site;
for use in a method of treatment of the human or animal body, said treatment comprising modifying a target site in the genome of a cell;
wherein the cell is selected from a human and an animal cell.
